# EUROPEAN PATENT APPLICATION

(11) **EP 1 284 297 A2**
(43) Date of publication of application: **19.02.2003**
(21) Application number: 02255228.5
(22) Date of filing: 26.07.2002
(51) Int. Cl.: C12Q 1/68, A01K 67/027

(54) **Identification and use of molecules implicated in pain**

(30) Priority: 27.07.2001 GB 0118354; 07.02.2002 GB 0202880
(71) Applicant: WARNER-LAMBERT COMPANY, Morris Plains, New Jersey 07950 (US)
(72) Inventor: Brooksbank, Robert Alan, Cambridgeshire CB1 3AW (GB); Dixon, Alistair Kerr, Cambridgeshire CB1 2LL (GB); Lee, Kevin, c/o Pfizer Limited, Sandwich, Kent CT13 9NJ (GB); Pinnock, Robert Denham, Pfizer Global Res. & Dev., Ann Arbor, MI 48105 (US)
(74) Representative: Hayles, James Richard

(57) **Abstract**

The invention relates to the use of:
(a) an isolated gene sequence that is down-regulated in the spinal cord in response to streptozocin-induced diabetes;
(b) an isolated gene sequence comprising a nucleic acid sequence of Tables I to X.
(c) an isolated gene sequence having at least 80% sequence identity with a nucleic acid sequence of Tables I to X;
(d) an isolated nucleic acid sequence that is hybridizable to any of the gene sequences according to (a), (b) or (c) under stringent hybridisation conditions;
(e) a recombinant vector comprising a gene sequence or nucleic acid sequence according to any one of (a) to (d);
(f) a host cell containing the vector according to (e);
(g) a non-human animal having in its genome an introduced gene sequence or nucleic acid sequence or a removed or down-regulated gene sequence or nucleic acid sequence according to any one of (a) to (d);
(h) an isolated polypeptide comprising an amino acid sequence at least 90% identical to an amino acid sequence encoded by a nucleotide sequence according to any one of (a) to (d), or a polypeptide variant thereof with sequential amino acid deletions from the C terminus and/or the N-terminus; or
(i) an isolated polypeptide encoded by a nucleotide sequence according to any one of (a) to (d); or
(k) an isolated antibody that binds specifically to a polypeptide according to (h) or (i);
in the screening of compounds for the treatment of pain, or for the diagnosis of pain.

The invention also relates to the use of naturally occurring compounds such as peptide ligands of the expression products of the above gene sequences and their associated signal transduction pathways for use in the treatment of pain.

SEQUENCE LISTING

<110> Warner-Lambert Company
      Lee, Kevin
      Dixon, Alistair
      Brooksbank, Robert
      Pinnock, Robert

<120> Identification and Use of Molecules Implicated in Pain

<130> WL-A-018201

<140>
<141>

<150> GB 0118354.0
<151> 2001-07-27

<160> 177

<170> PatentIn Ver. 2.1

<210> 1
<211> 4128
<212> PRT
<213> Mus musculus

<220>
<223> DNA-PKcs

<400> 1
Met Ala Glu Glu Gly Thr Gly Val Arg Cys Trp Leu Leu Gln Leu Gln
  1               5                  10                  15

Glu Phe Leu Ser Ala Ala Asp Arg Cys Ser Ala Ala Gly Ala Ser Tyr
             20                  25                  30

Gln Leu Ile Arg Ser Leu Gly Gln Glu Cys Val Leu Ser Thr Ser Ser
         35                  40                  45

Ala Val Gln Ala Leu Gln Ile Ser Leu Val Phe Ser Arg Asp Phe Gly
     50                  55                  60

Leu Leu Val Phe Ile Arg Lys Ser Leu Ser Ile Glu Asp Phe Arg Asp
 65                  70                  75                  80

Cys Arg Glu Glu Ala Leu Lys Phe Leu Cys Val Phe Leu Glu Lys Ile
                 85                  90                  95

Asp Gln Lys Val Met His Tyr Ser Leu Asp Ile Lys Asn Thr Cys Thr
            100                 105                 110

Ser Val Tyr Thr Lys Asp Arg Thr Ala Lys Cys Lys Ile Pro Ala Leu
        115                 120                 125

Asp Leu Leu Ile Lys Leu Leu Gln Ile Leu Arg Ser Thr Arg Leu Met
    130                 135                 140

Asp Glu Phe Lys Ile Gly Glu Leu Phe Asn Lys Phe Tyr Gly Glu Leu
145                 150                 155                 160

Ala Ser Lys Ser Lys Leu Pro Asp Thr Val Leu Glu Lys Val Tyr Glu
                165                 170                 175

Leu Leu Gly Val Leu Gly Glu Val His Pro Ser Glu Met Ile Asn His
            180                 185                 190

Ser Glu Asn Leu Phe Arg Ala Phe Leu Gly Glu Leu Lys Thr Gln Met
        195                 200                 205

Thr Ser Thr Val Arg Glu Pro Lys Phe Pro Val Leu Ala Gly Cys Leu
    210                 215                 220

Lys Gly Leu Ser Ser Leu Leu Cys Asn Phe Thr Lys Ser Met Glu Glu
225                 230                 235                 240

Asp Pro Gln Thr Ser Lys Glu Ile Phe Gly Phe Thr Phe Lys Ala Ile
                245                 250                 255

Arg Pro Gln Ile Glu Met Lys Arg Tyr Ala Val Pro Leu Ala Gly Leu
            260                 265                 270

Arg Leu Leu Thr Leu His Ala Ser Gln Phe Thr Ala Cys Leu Leu Asp
        275                 280                 285

Asn Tyr Ile Thr Leu Phe Glu Val Leu Ser Lys Trp Cys Ser His Thr
    290                 295                 300

Asn Val Glu Leu Lys Lys Ala Ala His Ser Ala Leu Glu Ser Phe Leu
305                 310                 315                 320

Arg Gln Ile Ser Phe Thr Val Ala Glu Asp Ala Glu Leu His Lys Ser
                325                 330                 335

Arg Leu Lys Tyr Phe Met Glu Gln Phe Tyr Gly Ile Ile Arg Asn Thr
            340                 345                 350

Asp Ser Asn Asn Lys Glu Leu Ala Ile Ala Ile Arg Gly Tyr Gly Leu
        355                 360                 365

Phe Ala Gly Pro Cys Lys Val Ile Asn Ala Lys Asp Val Asp Phe Met
    370                 375                 380

Tyr Val Glu Leu Ile Gln Arg Cys Lys Gln Met Phe Leu Thr His Ala
385                 390                 395                 400

Asp Ala Ser Glu Asp His Val Tyr Gln Met Pro Ser Phe Leu Gln Ser
                405                 410                 415

Ile Ala Ser Val Leu Leu Tyr Leu Asp Thr Val Pro Glu Val Tyr Thr
            420                 425                 430

Pro Val Leu Glu His Leu Met Val Val Gln Ile Asp Ser Phe Pro Gln
        435                 440                 445

Tyr Ser Pro Lys Met Gln Leu Val Cys Cys Lys Ala Ile Ile Lys Leu
    450                 455                 460

Phe Leu Ala Leu Ser Glu Lys Gly Pro Val His Trp Asn Cys Ile Ser
465                 470                 475                 480

Ala Val Val His Gln Gly Leu Ile Arg Ile Cys Ser Lys Pro Val Val
                485                 490                 495

Leu Gln Lys Asp Val Glu Ser Arg Ser Asp Asn Arg Ser Ala Ser Glu
            500                 505                 510

Glu Val Arg Thr Gly Arg Trp Lys Val Pro Thr Tyr Lys Asp Tyr Val
        515                 520                 525

Asp Leu Phe Gln His Leu Leu Gly Cys Asp Gln Met Glu Asp Phe Ile
    530                 535                 540

Leu Gly Asp Glu Thr Phe Leu Phe Val Asn Ser Ser Leu Lys Ser Leu
545                 550                 555                 560

Asn His Leu Leu Tyr Asp Glu Phe Ile Arg Ser Val Leu Lys Ile Val
                565                 570                 575

Glu Lys Leu Asp Leu Thr Leu Glu Lys Gln Thr Val Gly Glu Gln Glu
            580                 585                 590

Asp Gly Ser Thr Ala Asp Val Trp Val Ile Pro Thr Ser Asp Pro Ala
        595                 600                 605

Ala Asn Leu His Pro Ala Lys Pro Ser Asp Phe Ser Ala Leu Ile Asn
    610                 615                 620

Leu Val Glu Phe Cys Arg Glu Ile Leu Pro Arg Lys His Val Gly Phe
625                 630                 635                 640

Phe Glu Pro Trp Val Tyr Ser Phe Ala Tyr Glu Leu Ile Leu Gln Ser
                645                 650                 655

Thr Arg Leu Pro Leu Ile Ser Gly Phe Tyr Lys Leu Leu Ser Ile Ala
            660                 665                 670

Val Lys Asn Ala Arg Lys Ile Lys Tyr Phe Glu Gly Ile Ser Pro Lys
        675                 680                 685

Ser Leu Lys His Ser Pro Glu Asp Thr Glu Lys Tyr Ser Cys Phe Ala
    690                 695                 700

Leu Phe Ala Lys Phe Gly Lys Glu Val Ser Val Lys Met Lys Gln Tyr
705                 710                 715                 720

Lys Asp Glu Leu Leu Ala Ser Cys Leu Thr Phe Val Leu Ser Leu Pro
                725                 730                 735

His Asp Ile Ile Glu Leu Asp Val Arg Ala Tyr Val Pro Ala Leu Gln
            740                 745                 750

Met Ala Phe Lys Leu Gly Leu Ser His Met Pro Leu Ala Glu Ile Gly
        755                 760                 765

Leu His Ala Leu Lys Glu Trp Ser Val His Ile Asp Lys Ser Ile Leu
    770                 775                 780

Gln Pro Tyr Tyr Lys Asp Ile Leu Pro Cys Leu Asp Gly Tyr Leu Asn
785                 790                 795                 800

Thr Ser Thr Leu Ser Asp Glu Thr Lys Ser His Trp Gly Leu Ser Ala

805                 810                 815

Leu Ser Arg Ala Ala Gln Lys Gly Phe Asn Arg His Val Val Lys His
            820                 825                 830

Leu Lys Arg Thr Arg Asn Ser Ser Pro Asp Glu Ala Leu Ser Leu Glu
        835                 840                 845

Glu Ile Arg Ile Lys Val Val Gln Ile Leu Gly Ser Leu Gly Gly Gln
    850                 855                 860

Ile Asn Lys Ser Leu Val Thr Ala Thr Ser Gly Glu Arg Met Lys Lys
865                 870                 875                 880

Tyr Val Ala Trp Asp Ala Glu Arg Arg Leu Ser Phe Ala Val Pro Phe
                885                 890                 895

Arg Glu Met Lys Pro Val Ile Tyr Leu Asp Val Phe Leu Pro Arg Val
            900                 905                 910

Thr Glu Leu Ala Leu Ser Ala Ser Asp Arg Gln Thr Lys Val Ala Ala
        915                 920                 925

Cys Glu Leu Leu His Ser Met Val Met Phe Met Leu Gly Arg Ala Thr
    930                 935                 940

Gln Met Pro Glu Gly Gln Gly Leu Pro Pro Met Tyr Gln Leu Tyr Lys
945                 950                 955                 960

His Thr Phe Pro Val Leu Leu Gln Leu Ala Cys Asp Val Asp Gln Val
                965                 970                 975

Thr Arg Gln Leu Tyr Glu Pro Leu Val Met Gln Leu Ile His Trp Leu
            980                 985                 990

Thr Asn Asn Lys Lys Phe Glu Ser Gln Asp Thr Val Ala Leu Leu Glu
        995                 1000                1005

Ala Ile Leu Asp Gly Ile Val Asp Pro Val Asp Ser Thr Leu Arg Asp
    1010                1015                1020

Phe Cys Gly Arg Cys Val Gln Glu Phe Leu Lys Trp Ser Ile Lys Gln
1025                1030                1035                1040

Thr Thr Pro Gln Gln Gln Glu Lys Ser Pro Val Asn Ser Lys Ser Leu
                1045                1050                1055

Phe Lys Arg Leu Tyr Ser Leu Ala Leu His Pro Asn Ala Phe Lys Arg
            1060                1065                1070

Leu Gly Ala Ala Leu Ala Phe Asn His Ile Tyr Lys Glu Phe Arg Glu
        1075                1080                1085

Glu Gly Ser Leu Val Glu Gln Phe Val Phe Glu Ala Leu Val Thr Tyr
    1090                1095                1100

Met Glu Ser Leu Ala Leu Ala His Glu Asp Glu Lys Ser Leu Gly Thr
1105                1110                1115                1120

Val Gln Gln Cys Cys Asp Ala Ile Asp His Leu Arg Arg Ile Ile Glu
                1125                1130                1135

Lys Lys His Val Ser Leu Asn Lys Ala Lys Lys Arg Arg Leu Pro Gln
            1140                1145                1150

Gly Phe Pro Pro Leu Thr Ser Leu Cys Leu Leu Asp Leu Val Glu Trp
        1155                1160                1165

Leu Leu Ala His Cys Gly Arg Pro Gln Thr Glu Cys Arg His Lys Ser
    1170                1175                1180

Met Glu Leu Phe Tyr Lys Phe Val Pro Leu Leu Pro Gly Asn Lys Ser
1185                1190                1195                1200

Pro Ser Leu Trp Leu Lys Asp Leu Ile Lys Lys Lys Gly Ile Ser Phe
                1205                1210                1215

Leu Ile Asn Thr Phe Glu Gly Gly Ala Ser Ser Ser Asp Gln Pro Ala
            1220                1225                1230

Gly Ile Leu Ala Gln Pro Thr Leu Val Tyr Leu Gln Gly Pro Ile Ser
        1235                1240                1245

Leu Arg Gly Val Leu Gln Trp Leu Asp Leu Leu Leu Ala Ala Leu Glu
    1250                1255                1260

Cys Tyr Asn Thr Phe Ile Glu Lys Glu Thr Val Gln Gly Gln Glu Val
1265                1270                1275                1280

Leu Gly Ala Glu Val Gln Ser Ser Leu Leu Lys Ser Val Ala Phe Phe
                1285                1290                1295

Leu Glu Ser Ile Ala Thr His Ser Ala Arg Ala Val Glu Gln Arg Phe
            1300                1305                1310

Gly Ser Gly Ala Pro Gly Pro Pro Ser Leu His Glu Glu Glu Lys Tyr
        1315                1320                1325

Asn Tyr Ser Lys Cys Thr Val Leu Val Arg Ile Met Glu Phe Thr Thr
    1330                1335                1340

Thr Leu Leu Ile Ala Ser Pro Glu Asp Cys Lys Leu Leu Glu Lys Asp
1345                1350                1355                1360

Leu Cys Asn Thr Asn Leu Met Gln Val Leu Val Lys Met Ile Cys Glu
                1365                1370                1375

Pro Met Ser Leu Gly Phe Asn Ile Gly Asp Val Gln Val Met Asn His
            1380                1385                1390

Leu Pro Ser Ile Cys Val Asn Leu Leu Lys Ala Leu Arg Lys Ser Pro
        1395                1400                1405

Tyr Arg Asp Met Leu Glu Thr His Leu Lys Glu Lys Val Thr Val Gln
    1410                1415                1420

Ser Val Glu Glu Leu Cys Ser Ile Asn Leu Cys Ser Ser Gly Ala Arg
1425                1430                1435                1440

Gln Glu Arg Ser Lys Leu Leu Ser Ile Leu Ser Ala Cys Lys Gln Leu
                1445                1450                1455

His Lys Ala Gly Phe Ser His Val Ile Ser Pro Ser Gln Ser Thr Ala
            1460                1465                1470

Leu Asn His Ser Val Gly Met Arg Leu Leu Ser Leu Val Tyr Lys Gly
        1475                1480                1485

Ile Val Pro Ala Glu Glu Arg Gln Cys Leu Gln Ser Leu Asp Pro Ser
    1490                1495                1500

Cys Lys Ser Leu Ala Asn Gly Leu Leu Glu Leu Ala Phe Gly Phe Gly
1505                1510                1515                1520

Gly Leu Cys Asp His Leu Val Ser Leu Leu Leu Asn Ser Ala Met Leu
                1525                1530                1535

Ser Thr Gln Tyr Leu Gly Ser Ser Gln Arg Asn Ile Ser Phe Ser His
            1540                1545                1550

Gly Glu Tyr Phe Tyr Ser Leu Phe Ser Glu Val Ile Asn Ser Glu Leu
        1555                1560                1565

Leu Lys Asn Leu Asp Ile Ala Val Ser Arg Leu Met Glu Ser Ser Ser
    1570                1575                1580

Asp Asn Pro Lys Met Val Ser Thr Val Leu Asn Gly Met Leu Asp Thr
1585                1590                1595                1600

Ser Phe Arg Asp Arg Ala Val Gln Lys His Gln Gly Leu Lys Leu Ala
                1605                1610                1615

Thr Ala Ile Leu Gln Asn Trp Arg Lys Cys Asp Ser Trp Trp Ala Pro
            1620                1625                1630

Asp Ser Ala Pro Glu Ser Lys Thr Thr Val Leu Ser Leu Leu Ala Lys
        1635                1640                1645

Met Leu Gln Ile Asp Ser Ala Leu Ser Phe Asp Thr Asn His Ser Ser
    1650                1655                1660

Phe Ser Glu Ile Phe Thr Thr Tyr Ala Ser Leu Leu Ala Asp Thr Lys
1665                1670                1675                1680

Leu Gly Leu His Leu Lys Gly Gln Ala Ile Ile Leu Leu Pro Phe Phe
                1685                1690                1695

Thr Ser Leu Arg Glu Gly Ser Leu Glu Asn Leu Lys His Ile Leu Glu
            1700                1705                1710

Lys Leu Ile Val Cys Asn Phe Pro Met Lys Ser Asp Glu Phe Pro Pro
        1715                1720                1725

Asp Ser Leu Lys Tyr Asn Asn Tyr Val Asp Cys Met Lys Lys Phe Leu
    1730                1735                1740

Asp Ala Leu Glu Leu Ser Gln Ser Pro Met Leu Phe Gln Leu Met Thr
1745                1750                1755                1760

Asp Ile Leu Cys Arg Glu Gln Arg His Ile Met Glu Glu Leu Phe Gln
                1765                1770                1775

Thr Thr Phe Lys Arg Ile Ala Arg Gln Ser Pro Cys Val Thr Gln Leu

1780                1785                1790

Asn Leu Leu Glu Ser Val Tyr Thr Met Phe Arg Lys Ala Asp Leu Pro
    1795                1800                1805

Ser Asn Val Thr Arg Gln Ala Phe Val Asp Arg Ser Leu Leu Thr Leu
  1810                1815                1820

Leu Trp His Cys Asp Leu Asp Thr Leu Lys Glu Phe Phe Ser Arg Ile
1825                1830                1835                1840

Val Val Asp Ala Ile Asp Val Leu Lys Ser Arg Phe Thr Lys Leu Asn
                1845                1850                1855

Glu Phe Thr Phe Asp Thr Gln Ile Thr Lys Lys Met Cys Tyr Tyr Lys
            1860                1865                1870

Met Leu Ala Val Met Tyr Ser Arg Leu Leu Lys Asp Asp Val His Ser
        1875                1880                1885

Lys Glu Ala Lys Ile Asn Gln Ala Phe His Gly Ser Arg Val Ala Glu
    1890                1895                1900

Gly Asn Glu Leu Thr Lys Thr Leu Leu Lys Leu Cys His Asp Ala Phe
1905                1910                1915                1920

Thr Glu Asn Met Val Gly Glu Ser Gln Leu Leu Glu Lys Arg Arg Leu
                1925                1930                1935

Tyr His Cys Ala Ala Tyr Asn Cys Ala Ile Ser Leu Ile Ser Cys Val
            1940                1945                1950

Phe Asn Glu Leu Lys Phe Tyr Gln Gly Phe Leu Phe Asn Glu Lys Pro
        1955                1960                1965

Glu Lys Asn Leu Phe Ile Phe Glu Asn Leu Ile Asp Leu Lys Arg Cys
    1970                1975                1980

Tyr Thr Phe Pro Ile Glu Val Glu Val Pro Met Glu Arg Lys Lys Lys
1985                1990                1995                2000

Tyr Ile Glu Ile Arg Lys Glu Ala Arg Asp Ala Ala Asn Gly Ala Ser
                2005                2010                2015

Gly Ser Pro His Tyr Met Ser Ser Leu Ser Tyr Leu Thr Asp Ser Ser
            2020                2025                2030

Leu Ser Glu Glu Met Ser Gln Phe Asp Phe Ser Thr Gly Val Gln Ser
        2035                2040                2045

Tyr Ser Tyr Ser Ser Gln Asp Arg Lys Pro Thr Thr Gly His Phe Gln
    2050                2055                2060

Arg Arg Glu His Gln Asp Ser Met Thr Gln Asp Asp Ile Met Glu Leu
2065                2070                2075                2080

Glu Met Asp Glu Leu Asn Gln His Glu Cys Met Ala Pro Met Ile Ala
                2085                2090                2095

Leu Ile Lys His Met Gln Arg Asn Val Ile Ala Pro Lys Gly Glu Glu
            2100                2105                2110

Gly Ser Ile Pro Lys Asp Leu Pro Pro Trp Met Lys Phe Leu His Asp
        2115                2120                2125

Lys Leu Gly Asn Ala Ser Val Ser Leu Asn Ile Cys Leu Phe Leu Ala
    2130                2135                2140

Lys Leu Val Ile Asn Thr Glu Glu Val Phe Arg Pro Tyr Ala Lys His
2145                2150                2155                2160

Trp Leu Ser Pro Leu Leu Gln Leu Ala Val Cys Glu Asn Asn Arg Glu
                2165                2170                2175

Gly Ile His Tyr Met Met Val Glu Ile Val Ala Thr Ile Leu Ser Trp
            2180                2185                2190

Thr Gly Leu Ala Thr Pro Thr Gly Val Pro Lys Asp Glu Val Leu Ala
        2195                2200                2205

Asn Arg Leu Leu Arg Phe Leu Met Lys His Val Phe His Pro Lys Arg
    2210                2215                2220

Ala Val Phe Arg His Asn Leu Glu Ile Ile Lys Thr Leu Val Glu Cys
2225                2230                2235                2240

Trp Lys Glu Cys Leu Ser Ile Pro Tyr Arg Leu Ile Phe Glu Lys Phe
                2245                2250                2255

Ser His Lys Asp Pro Asn Ser Lys Asp Asn Ser Val Gly Ile Gln Leu
            2260                2265                2270

Leu Gly Ile Val Ile Ala Asn Asn Leu Pro Pro Tyr Asp Pro Asn Cys
        2275                2280                2285

Asp Ile Thr Ser Ala Met Tyr Phe Glu Ala Leu Val Asn Asn Met Ser
    2290                2295                2300

Phe Val Lys Tyr Lys Glu Val Tyr Ala Ala Ala Ala Glu Val Leu Gly
2305                2310                2315                2320

Leu Ile Leu Gln Tyr Ile Thr Glu Arg Lys His Val Ile Ala Glu Leu
                2325                2330                2335

Val Cys Glu Leu Val Ile Lys Gln Leu Lys Gln His Gln Asn Thr Met
            2340                2345                2350

Glu Asp Lys Phe Ile Val Cys Leu Asn Lys Ile Ala Lys Gly Phe Pro
        2355                2360                2365

Pro Leu Ala Asp Arg Phe Leu Asn Ala Leu Phe Phe Leu Leu Pro Lys
    2370                2375                2380

Phe His Gly Val Met Lys Thr Leu Cys Leu Glu Val Val Leu Cys Arg
2385                2390                2395                2400

Ala Glu Glu Ile Thr Gly Leu Tyr Leu Gln Leu Lys Ser Lys Asp Phe
                2405                2410                2415

Leu Gln Val Met Arg His Arg Asp Asp Glu Arg Gln Lys Val Cys Leu
            2420                2425                2430

Asp Ile Val Tyr Lys Met Val Ala Lys Leu Lys Pro Ile Glu Leu Arg
        2435                2440                2445

Glu Leu Leu Asn Pro Val Val Glu Phe Val Ser His Pro Ser Pro Thr
    2450                2455                2460

Cys Arg Glu Gln Met Tyr Asn Ile Leu Met Trp Ile His Asp Asn Tyr
2465                2470                2475                2480

Arg Asp Gln Glu Ser Gln Asn Asp Glu Asp Ser Gln Glu Ile Phe Lys
                2485                2490                2495

Leu Ala Lys Asp Val Leu Ile Gln Gly Leu Ile Asp Glu Asn Val Gly
            2500                2505                2510

Leu Gln Leu Ile Ile Arg Asn Phe Trp Ser His Glu Thr Arg Leu Pro
        2515                2520                2525

Ser Asn Thr Leu Asp Arg Leu Leu Ala Leu Asn Ser Leu Tyr Ser Pro
    2530                2535                2540

Lys Ile Glu Val His Phe Leu Ser Leu Ala Thr Asn Phe Leu Leu Glu
2545                2550                2555                2560

Met Thr Arg Met Ser Pro Asp Tyr Leu Asn Pro Ile Phe Glu His Pro
                2565                2570                2575

Leu Ser Glu Cys Glu Phe Gln Glu Tyr Thr Ile Asp Pro Asp Trp Arg
            2580                2585                2590

Phe Arg Ser Thr Val Leu Thr Pro Met Phe Ile Glu Thr Gln Ala Ser
        2595                2600                2605

Pro Ser Ile Leu His Thr Gln Thr Gln Glu Gly Pro Leu Ser Asp Gln
    2610                2615                2620

Arg Gln Lys Pro Gly Gln Val Arg Ala Thr Gln Gln Gln Tyr Asp Phe
2625                2630                2635                2640

Thr Pro Thr Gln Ala Ser Val Glu Arg Ser Ser Phe Asp Trp Leu Thr
                2645                2650                2655

Gly Ser Ser Ile Asp Leu Leu Ala Asp His Thr Val Phe Ser Ser Glu
            2660                2665                2670

Thr Leu Ser Ser Ser Leu Leu Phe Ser His Lys Arg Thr Glu Lys Ser
        2675                2680                2685

Gln Arg Met Ser Cys Lys Ser Val Gly Pro Asp Phe Gly Thr Lys Lys
    2690                2695                2700

Leu Gly Leu Pro Asp Asp Glu Val Asp Asn Gln Val Lys Ser Gly Thr
2705                2710                2715                2720

Pro Ser Gln Ala Asp Ile Leu Arg Leu Arg Arg Arg Phe Leu Lys Asp
                2725                2730                2735

Arg Glu Lys Leu Ser Leu Leu Tyr Ala Lys Arg Gly Leu Met Glu Gln
            2740                2745                2750

Lys Leu Glu Lys Asp Ile Lys Ser Glu Phe Lys Met Lys Gln Asp Ala

2755                    2760                    2765

Gln Val Val Leu Tyr Arg Ser Tyr Arg His Gly Asp Leu Pro Asp Ile
    2770                    2775                    2780

Gln Ile Gln His Ser Gly Leu Ile Thr Pro Leu Gln Ala Val Ala Gln
2785                    2790                    2795                    2800

Lys Asp Pro Ile Ile Ala Lys Gln Leu Phe Ser Ser Leu Phe Ser Gly
                2805                    2810                    2815

Ile Leu Lys Glu Met Asn Lys Phe Lys Thr Thr Ser Glu Lys Asn Ile
            2820                    2825                    2830

Ile Thr Gln Asn Leu Leu Gln Asp Phe Asn Arg Phe Leu Asn Thr Thr
        2835                    2840                    2845

Phe Leu Phe Phe Pro Pro Phe Val Ser Cys Ile Gln Glu Ile Ser Cys
    2850                    2855                    2860

Gln His Pro Asp Phe Leu Thr Leu Asp Pro Ala Ser Val Arg Val Gly
2865                    2870                    2875                    2880

Cys Leu Ala Ser Leu Gln Gln Pro Gly Gly Ile Arg Leu Leu Glu Glu
                2885                    2890                    2895

Ala Leu Leu Arg Leu Met Pro Lys Glu Pro Pro Thr Lys Arg Val Arg
            2900                    2905                    2910

Gly Lys Thr Cys Leu Pro Pro Asp Val Leu Arg Trp Met Glu Leu Ala
        2915                    2920                    2925

Lys Leu Tyr Arg Ser Ile Gly Glu Tyr Asp Val Leu Arg Gly Ile Phe
    2930                    2935                    2940

Ser Ser Glu Leu Gly Thr Thr Gln Asp Thr Gln Asn Ala Leu Leu Ala
2945                    2950                    2955                    2960

Glu Ala Arg Ser Asp Tyr Cys Gln Ala Ala Lys Leu Tyr Asp Glu Ala
                2965                    2970                    2975

Leu Asn Lys Leu Glu Trp Val Asp Gly Glu Pro Thr Glu Ala Glu Lys
            2980                    2985                    2990

Glu Phe Trp Glu Leu Ala Ser Leu Asp Cys Tyr Asn Asn Leu Ser Lys
        2995                    3000                    3005

Trp Lys Glu Leu Glu Tyr Cys Ser Thr Val Asn Ile Val Ser Glu Asn
    3010                    3015                    3020

Ser Leu Asp Leu Ser Lys Met Trp Ser Glu Pro Phe Tyr Gln Glu Thr
3025                    3030                    3035                    3040

Tyr Leu Pro Tyr Val Ile Arg Ser Lys Leu Lys Leu Leu Leu Gln Gly
                3045                    3050                    3055

Glu Gly Asn Gln Ser Leu Leu Thr Phe Val Asp Glu Ala Met Asn Lys
            3060                    3065                    3070

Glu Leu Gln Lys Thr Val Leu Glu Leu Gln Tyr Ser Gln Glu Leu Ser
        3075                    3080                    3085

Leu Leu Tyr Ile Leu Gln Asp Asp Ile Asp Arg Ala Thr Tyr Tyr Ile
    3090                3095                3100

Lys Asn Gly Ile Gln Ile Phe Met Gln Asn Tyr Ser Ser Ile Asp Val
3105                3110                3115                3120

Leu Leu Tyr Arg Ser Arg Leu Ala Lys Leu Gln Ser Val Gln Thr Leu
                3125                3130                3135

Ala Glu Ile Glu Glu Phe Leu Ser Phe Ile Cys Lys His Gly Asp Leu
            3140                3145                3150

Ser Ser Leu Gly Pro Leu Arg Arg Leu Leu Lys Thr Trp Thr Ser Arg
        3155                3160                3165

Tyr Pro Asp Val Val Thr Asp Pro Met His Ile Trp Asp Asp Ile Ile
    3170                3175                3180

Thr Asn Arg Cys Phe Phe Leu Ser Lys Ile Glu Glu Arg Leu Thr Ala
3185                3190                3195                3200

Pro Ser Gly Asp His Ser Met Ser Val Asp Glu Asp Glu Glu Ser Ile
                3205                3210                3215

Asp Arg Glu Val Tyr Glu Pro Lys Glu Asp Val Arg Cys Met Leu Gln
            3220                3225                3230

Ser Cys Arg Phe Thr Met Lys Met Lys Met Ile Glu Ser Ala Trp Lys
        3235                3240                3245

Gln Ser Asn Phe Ser Leu Ser Met Lys Leu Leu Lys Glu Met His Lys
    3250                3255                3260

Glu Ser Lys Thr Arg Glu Ile Trp Arg Val Gln Trp Leu His Ser Tyr
3265                3270                3275                3280

Ser Gln Leu Asn His Cys Arg Ser His Thr Gln Ser Pro Arg Glu Gln
                3285                3290                3295

Val Leu Asn Thr Leu Lys Thr Ile Thr Leu Leu Asp Glu Ser Asp Ile
            3300                3305                3310

Ser Asn Tyr Leu Asn Lys Asn Ile Gln Ala Ser Cys Asp Gln Ser Ile
        3315                3320                3325

Leu Leu Gly Thr Thr Cys Arg Ile Met Ala Asp Ala Leu Ser Arg Glu
    3330                3335                3340

Pro Ala Cys Leu Ser Asp Leu Glu Glu Asn Lys Val Asn Ser Ile Leu
3345                3350                3355                3360

Thr Leu Ser Gly Ser Asn Ala Glu Asn Thr Glu Thr Val Ile Thr Gly
                3365                3370                3375

Leu Tyr Gln Arg Ala Phe His His Leu Ser Lys Ala Val Gln Ser Ala
            3380                3385                3390

Glu Glu Glu Thr Gln Leu Ser Cys Trp Gly His Glu Ala Ala Ala Glu
        3395                3400                3405

Arg Ala His Ala Tyr Met Thr Leu Val Gly Phe Cys Asp Gln Gln Leu
    3410                3415                3420

Arg Lys Val Glu Glu Ser Ala Ser Gln Lys Thr Ser Ala Glu Met Glu
3425                3430                3435                3440

Ala Tyr Pro Ala Leu Val Val Glu Lys Met Leu Arg Ala Leu Lys Leu
                3445                3450                3455

Asn Ser Ser Glu Ala Arg Leu Lys Phe Pro Arg Leu Leu Gln Ile Ile
            3460                3465                3470

Glu Gln Tyr Ser Glu Glu Thr Leu Asn Ile Met Thr Lys Glu Ile Ser
        3475                3480                3485

Ser Ile Pro Cys Trp Gln Phe Ile Gly Trp Ile Ser His Met Met Ala
    3490                3495                3500

Leu Leu Asp Lys Glu Glu Ala Ile Ala Val Gln His Thr Val Glu Glu
3505                3510                3515                3520

Ile Ala Asp Asn Tyr Pro Gln Ala Ile Ile Tyr Pro Phe Ile Ile Ser
                3525                3530                3535

Ser Glu Ser Tyr Ser Phe Lys Asn Thr Ser Ser Gly His Asn Asn Lys
            3540                3545                3550

Ala Phe Val Glu Arg Ile Lys Ser Lys Leu Asp His Gly Glu Val Ile
        3555                3560                3565

His Ser Phe Ile Asn Ala Leu Asp Gln Leu Ser Asn Pro Asp Leu Leu
    3570                3575                3580

Phe Lys Asp Trp Val Ser Asp Thr Lys Asp Glu Leu Gly Lys Asn Pro
3585                3590                3595                3600

Val Asn Lys Lys Asn Ile Glu Lys Leu Tyr Glu Arg Met Tyr Ala Ala
                3605                3610                3615

Leu Gly Asp Leu Arg Ala Pro Gly Leu Gly Pro Phe Arg Arg Arg Phe
            3620                3625                3630

Ile Gln Ala Phe Gly Lys Glu Phe Val Lys Ser Phe Gly Asn Gly Gly
        3635                3640                3645

Ser Lys Leu Leu Thr Met Lys Val Asp Asp Phe Cys Lys Ile Thr Gly
    3650                3655                3660

Ser Leu Leu Val Arg Met Lys Lys Asp Ser Lys Leu Pro Gly Asn Leu
3665                3670                3675                3680

Lys Glu Tyr Ser Pro Trp Met Ser Glu Phe Lys Ala Gln Phe Leu Lys
                3685                3690                3695

Asn Glu Leu Glu Ile Pro Gly Gln Tyr Asp Gly Lys Ser Lys Pro Leu
            3700                3705                3710

Pro Glu Tyr His Val Arg Ile Ser Gly Phe Asp Glu Arg Val Lys Val
        3715                3720                3725

Met Leu Ser Leu Arg Lys Pro Lys Arg Ile Val Ile Arg Gly His Asp

3730                3735                3740

Glu Lys Glu Tyr Pro Phe Leu Val Lys Gly Gly Glu Asp Leu Arg Gln
3745                3750                3755                3760

Asp Gln Arg Ile Glu Gln Ile Phe Glu Val Met Asn Ala Ile Leu Ser
                3765                3770                3775

Gln Asp Ala Ala Cys Ser Gln Arg Asn Met Gln Leu Arg Thr Tyr Arg
            3780                3785                3790

Val Val Pro Met Thr Ser Arg Leu Gly Leu Ile Glu Trp Ile Glu Asn
        3795                3800                3805

Thr Met Thr Leu Lys Asp Leu Leu Leu Ser Asn Met Ser Gln Glu Glu
    3810                3815                3820

Lys Val Ala Asn Asn Ser Asp Pro Lys Ala Pro Ile Arg Asp Tyr Lys
3825                3830                3835                3840

Asp Trp Leu Met Lys Val Ser Gly Lys Ser Asp Ala Gly Ala Tyr Val
                3845                3850                3855

Leu Met Tyr Ser Arg Ala Asn Arg Thr Glu Thr Val Val Ala Phe Arg
            3860                3865                3870

Arg Arg Glu Ser Gln Val Pro Pro Asp Leu Leu Lys Arg Ala Phe Val
        3875                3880                3885

Lys Met Ser Thr Ser Pro Glu Ala Phe Leu Ala Leu Arg Ser His Phe
    3890                3895                3900

Ala Ser Ser His Ala Leu Leu Cys Ile Ser His Trp Leu Leu Gly Ile
3905                3910                3915                3920

Gly Asp Arg His Leu Asn Asn Phe Met Val Ala Met Glu Thr Gly Ser
                3925                3930                3935

Val Ile Gly Ile Asp Phe Gly His Ala Phe Gly Ser Ala Thr Gln Phe
            3940                3945                3950

Leu Pro Val Pro Glu Leu Met Pro Phe Arg Leu Thr Arg Gln Phe Val
        3955                3960                3965

Ser Leu Met Leu Pro Met Lys Glu Thr Gly Leu Met Cys Thr Val Met
    3970                3975                3980

Val His Ala Leu Arg Ala Phe Arg Ser Cys Ala Gly Leu Leu Thr Asp
3985                3990                3995                4000

Thr Met Glu Ile Phe Val Lys Glu Pro Ser Phe Asp Trp Lys Ser Phe
                4005                4010                4015

Glu Gln Thr Met Leu Arg Lys Gly Gly Ser Trp Ile Gln Glu Ile Asn
            4020                4025                4030

Val Thr Glu Lys Asn Trp Tyr Pro Gln His Lys Ile Arg Tyr Ala Lys
        4035                4040                4045

Arg Lys Leu Ala Gly Ala Asn Pro Ala Val Ile Thr Cys Asp Glu Leu
    4050                4055                4060

Tyr Leu Gly His Glu Ala Ser Ser Ala Phe Arg Ser Tyr Thr Ala Val
4065                4070                4075                4080

Ala Arg Gly Asn Arg Asp Tyr Asn Ile Arg Ala Gln Glu Pro Glu Ser
                4085                4090                4095

Gly Leu Ser Glu Glu Thr Gln Val Lys Cys Leu Val Asp Gln Ala Thr
            4100                4105                4110

Asp Pro Asn Ile Leu Gly Arg Thr Trp Glu Gly Trp Glu Pro Trp Met
        4115                4120                4125

<210> 2
<211> 12653
<212> DNA
<213> Mus musculus

<220>
<223> DNA-PKcs

<400> 2
atggcggagg agggaaccgg cgtacggtgt tggctactgc agctgcagga gttcctgtcc 60
gcagcagacc gctgcagtgc tgccggggcc agttaccagc tgattcgtag tctggggcag 120
gagtgcgtgc tgagcactag ctctgcagtg caggcattgc agatttcctt agttttttcc 180
agagactttg gtttgcttgt gtttatccgg aagtcgctta gcattgagga ctttcgtgat 240
tgtagagaag aagccctaaa gtttttatgt gttttcttag agaaaattga ccagaaggtt 300
atgcattact ctcttgatat taagaatact tgtaccagtg tttacacaaa agatagaact 360
gctaagtgta aaattccagc cttagacctt ctgattaagt tacttcagat actaagaagt 420
accagactca tggatgaatt taagattgga gaattattta acaaattcta tggagaactt 480
gcatcaaaat caaaactacc tgatacagtt ttagaaaaag tctatgagct cctgggagta 540
ttaggtgaag ttcatcctag tgagatgata aaccattcag aaaacctgtt ccgagctttt 600
ctgggagaac ttaagaccca gatgacatcc acagtaagag aacctaaatt tcctgtctta 660
gctggctgtc tgaagggact atcctcactt ctgtgcaact tcactaagtc catggaagaa 720
gatccccaga cttcaaagga aatttttggt tttacattta aagcaattcg tcctcagatt 780
gagatgaagc gatatgctgt gcccttagct ggcctacgat tacttaccct gcatgcatct 840
caattcactg cctgccttct ggacaactat attactttat ttgaagtact gtctaagtgg 900
tgtagccata caaatgtgga attaaaaaaa gctgcacatt cagccctgga gtccttcctg 960
agacagattt cgtttactgt ggcagaagat gcagagttgc ataagagcag gctgaagtac 1020
tttatggaac agttctatgg aatcatcaga aatacagact cgaacaacaa ggaattagcc 1080
attgctattc gcggatatgg actatttgca ggaccttgca aggttataaa tgcaaaagat 1140
gttgacttca tgtacgtgga gctcattcag cgctgtaagc agatgttcct cacccacgca 1200
gatgcttctg aggatcatgt ttaccagatg ccaagtttcc ttcagtctat tgcaagtgtc 1260
ttgctttacc ttgacacggt tccggaggta tatactccag tactggaaca tctcatggtg 1320
gtacagatag acagcttccc tcagtatagt cccaaaatgc agttggtgtg ctgtaaagca 1380
ataataaaac ttttcctagc cttatcagag aaaggaccag ttcactggaa ttgcattagt 1440
gctgtggtgc accaaggttt aattagaata tgttctaaac cagtggtcct tcaaaaggat 1500
gttgagtcta ggtctgataa ccgttcggcc tccgaggaag tcagaactgg caggtggaaa 1560
gtacctacgt acaaagacta tgtggatctt tttcagcatc ttttaggctg tgaccagatg 1620
gaggatttta ttttaggaga tgaaacattt ctctttgtga actcctccct taaaagtctg 1680
aatcatttac tctatgatga atttataaga tcagttttga agattgtgga aaaattggat 1740
cttacactgg aaaaacagac tgttggagag caggaggatg gaagcactgc tgatgtctgg 1800
gtgatcccaa cttcagatcc agcagctaat ttgcaccctg ctaaaccaag tgatttttca 1860
gctctcatta acctggtgga gttttgcagg gagattcttc ctaggaaaca tgtaggcttt 1920
tttgagccat gggtgtactc atttgcatat gagttaattt tgcagtctac acgattgcca 1980
ctcatcagtg gtttctacaa attgctttct attgcagtga aaaatgccag gaaaataaaa 2040
tattttgagg gaatcagccc aaagagtctg aaacattctc ctgaagatac agaaaagtat 2100

tcttgctttg ctttatttgc aaagtttggc aaagaggtat cagttaaaat gaagcaatac 2160
aaggatgaac tgttggcctc ttgtttaacc tttgttctgt ccttgccaca tgacatcatt 2220
gaacttgatg ttagagccta cgttcctgca ttgcagatgg ctttcaagct gggcctgagc 2280
catatgccac tggcagaaat aggtctacat gcccttaaag agtggtcagt tcacattgat 2340
aagtctatac tgcagcctta ctacaaagac attctcccct gccttgatgg atatctgaat 2400
acttcaacct tatcagatga aaccaagagc cactggggat tgtctgcact ttctcgggct 2460
gcccagaagg gatttaatag acatgtagtg aagcatctaa aaaggactag aaatagttcg 2520
cctgatgaag cactgtcctt agaagagata agaattaaag tagtacaaat acttggctct 2580
ctgggaggac aaataaacaa aagccttgta acagctacat ccggagaaag gatgaagaag 2640
tacgtggcgt gggatgcgga gagaagactc agctttgcag tgcccttcag agagatgaag 2700
cctgtcattt atctggatgt ctttctgcct cgggtcactg aattggctct ttctgctagc 2760
gaccgacaaa ctaaagtagc agcttgtgaa ctgttacaca gcatggttat gtttatgttg 2820
ggaagagcga ctcagatgcc tgaaggtcag gggttgccgc ccatgtacca gctttataag 2880
cacacgtttc ctgtgctgct tcaacttgcg tgtgatgtgg atcaagtgac aaggcagctc 2940
tatgaaccgc ttgttatgca gctgatccac tggttgacta acaataaaaa atttgaaagt 3000
caagatactg ttgccttatt agaagccata ctggatggta ttgtggaccc tgttgacagt 3060
actttaagag atttttgtgg acggtgcgtt caagaattcc tcaaatggtc cattaagcaa 3120
acaacacctc agcagcaaga gaagagtcca gtaaacagca agtcactttt caagcggctc 3180
tacagccttg cacttcatcc taatgctttc aagaggctag gtgcagcact tgcttttaat 3240
cacatctaca aggaattcag ggaagaagga tccctggtag aacagtttgt gtttgaagcc 3300
ttagtgacat atatggaaag tctggccttg gcacatgaag atgagaaatc cttaggcaca 3360
gttcagcagt gctgtgatgc catcgatcac ctaagacgca tcattgaaaa gaagcatgtc 3420
tctttaaaca aagcaaaaaa gcgacgtttg ccacagggat ttccaccttt gacatcatta 3480
tgtttattgg atctggtcga gtggcttttg gctcactgtg gaaggcccca gacagaatgt 3540
cgacacaagt ccatggaact cttttataaa tttgttcctt tactgccagg caacaaatcc 3600
ccttctttat ggttgaaaga ccttataaaa aagaaaggta tttcttttct cataaacaca 3660
tttgaaggag gggcaagcag cagtgatcag ccagcaggca ttcttgctca gccaaccctc 3720
gtctacttac aagggccaat cagcctgcga ggagtgctac aatggctgga cctcctcctt 3780
gcagcactgg agtgctacaa cacatttatt gagaaggaga ctgtgcaagg acaagaggtc 3840
ctgggtgctg aagtacagtc ttcacttttg aagtcagtgg ctttcttttt agaaagcata 3900
gcaacccata gcgctagagc agtagaacaa cgctttggct ccggggcgcc gggccctccc 3960
agcctccacg aggaagagaa gtacaattac agcaagtgca cagtcttagt ccggatcatg 4020
gaattcacca caaccctgct catcgcctct ccagaggact gcaagctcct ggagaaggac 4080
ttgtgtaata caaatcttat gcaggtctta gtgaaaatga tctgtgagcc catgagctta 4140
ggtttcaata ttggcgatgt ccaggttatg aaccatcttc ccagcatttg tgtgaacctg 4200
ctgaaagctc ttaggaagtc cccgtacaga gacatgctgg agactcacct gaaggagaaa 4260
gtgacagtac agagtgttga agagctctgt tccattaact tgtgtagctc tggggctcgc 4320
caagaaaggt ctaagttact ttctatctta tcagcctgta aacagcttca caaagctggt 4380
ttctctcatg ttatatcacc atctcagtct acagcattga accattccgt tggcatgaga 4440
ctgctgtcct tggtttataa aggcattgtg cctgcagagg aaaggcagtg cctgcagtct 4500
ttggacccca gctgtaagag cctagccaat ggactcctgg aattagcctt tggttttgga 4560
ggactgtgtg accatcttgt gagtttgctc ctgaattcgg caatgctgtc tacacaatat 4620
ctgggcagtt cacagagaaa tattagcttc tcccatgggg aatatttcta cagtttgttc 4680
tcagaagtga tcaacagtga gctgttgaag aatcttgata ttgctgtatc aaggctcatg 4740
gagtcatctt cggataatcc caaaatggtg agcactgttt taaatggtat gctagacaca 4800
agcttcaggg atcgagctgt tcagaaacac caaggactga agcttgcaac tgcaattctg 4860
cagaactgga ggaagtgtga ttcatggtgg gccccagatt ctgccccaga gagcaaaaca 4920
actgtgctgt ctttgctggc aaagatgttg cagattgatt cagctttatc ttttgataca 4980
aatcacagct cattttctga aatcttcaca acatacgcta gtctacttgc tgatacaaag 5040
ttgggtctac acttaaaggg ccaagctatc attcttcttc cattcttcac cagtcttaga 5100
gaaggcagtc tagaaaacct aaagcatatt ctggaaaaac tcattgtttg caatttcccc 5160
atgaagtctg atgaatttcc tcctgattcc ctaaagtaca ataattatgt ggactgcatg 5220
aaaaagtttc tagatgcact ggagttatct cagagtccta tgttgtttca gttgatgaca 5280
gatatacttt gtcgggaaca gcgacatatt atggaagaat tgttccaaac cactttcaaa 5340
aggattgcta ggcagagtcc atgtgtcaca cagttaaatc ttctggaaag tgtgtacaca 5400
atgttccgga aggctgacct cccttcaaat gtcactcgcc aggcatttgt agatcgctct 5460
ctcctcacct tgttgtggca ctgtgacttg gacacactga aagagttctt tagtagaatt 5520
gtggtagatg ccattgatgt gttgaagtcc agatttacaa agctaaatga atttactttt 5580
gacactcaaa tcaccaagaa gatgtgctat tacaagatgc tagctgtgat gtattctcgt 5640
cttttaaaag atgatgttca ctctaaagaa gctaaaatta atcaagcttt tcatggctcc 5700
cgtgttgcag aaggaaatga actcacaaag acacttctta agttgtgcca tgatgcattt 5760

acagagaaca tggtggggga gagccagttg ctggagaaga gaagacttta tcattgtgct 5820
gcatacaact gtgccatttc tctgataagc tgtgtcttca atgagttgaa attttaccaa 5880
ggttttttat ttaatgaaaa accagaaaag aacttgttta tttttgaaaa tttgatagac 5940
ctgaaacgtt gctatacatt tcctatagaa gttgaggttc ctatggaaag aaagaaaaag 6000
tacattgaaa ttaggaaaga ggccagggat gcagcaaatg gggcttcagg cagtcctcat 6060
tatatgtctt cattgtcata tttgacagac agtagcctaa gtgaagaaat gagtcaattt 6120
gatttctcaa ctggagttca gagctattca tacagttctc aagatcgtaa acctaccact 6180
ggccattttc agagacggga gcatcaagat tccatgaccc aggatgacat catggagtta 6240
gagatggatg agctcaatca acatgaatgt atggctccca tgatagccct gattaagcac 6300
atgcagagaa atgtgattgc acctaaggga gaagagggtt caataccaaa agatcttcca 6360
ccgtggatga aatttcttca tgacaaacta ggaaatgcat cagtatcttt aaatatctgt 6420
ctcttcttag ccaagcttgt tattaataca gaagaggtct ttcgccctta tgcaaagcac 6480
tggctcagcc ccctgctaca gctagctgtt tgcgagaaca acagagaagg aattcactac 6540
atgatggtag aaatagtagc taccattctc tcttggactg gcttggctac acctacagga 6600
gtccctaaag atgaagtgtt agcaaatcga ttgcttcgtt ttctaatgaa gcatgtcttc 6660
catcccaaaa gagctgtgtt taggcacaac cttgaaatta ttaaaaccct tgttgaatgc 6720
tggaaggagt gtctttccat cccttacagg ttaatatttg aaaaattttc ccataaggac 6780
cctaattcta aagacaattc tgtgggaatt cagttattag gcattgtgat agctaataac 6840
ttgcctccct atgacccaaa ttgtgacata accagtgcca tgtattttga agctttagtc 6900
aataacatgt cctttgtgaa gtataaagaa gtatatgcag ctgcagcaga agttttagga 6960
cttattcttc aatacattac tgagagaaaa cacgtgatag cagagttggt atgtgaactg 7020
gttataaaac aactgaagca acatcaaaat actatggaag acaaatttat tgtttgctta 7080
aacaaaatag cgaagggctt ccctcctctt gctgacaggt tcttgaatgc tttgttcttt 7140
ctgctgccaa aatttcatgg agtaatgaag acactttgtc tggaagttgt actttgtcga 7200
gcagaggaga tcacaggcct atacttacag ttaaagagta aagactttct tcaagtcatg 7260
agacatagag atgatgaaag acaaaaggtg tgtttggata tagtttataa gatggtggca 7320
aaattaaaac caatagaact ccgagaactt ctgaatcctg ttgtggaatt tgtttcccat 7380
ccttctccaa catgtagaga acaaatgtat aatattctta tgtggattca tgacaattac 7440
agagatcaag agagtcaaaa tgatgaagat tcccaggaaa tatttaaact ggcaaaagat 7500
gtactgattc aaggactgat cgacgagaac gttggactcc aactaattat tcggaatttc 7560
tggagccatg aaaccagatt gccttccaat acattggacc gattgctggc attaaattct 7620
ttatattccc ccaagataga agtccacttt ttaagtttag caacaaactt tctgcttgaa 7680
atgaccagga tgagcccaga ttatctaaac cccatttttg agcatcctct gtcagaatgt 7740
gaatttcagg aatatactat agatcctgac tggcgttttc gtagtactgt tcttactccg 7800
atgtttattg agacccaggc ctctccaagt attctccata cccaaaccca agaagggccc 7860
ctctcagacc aaaggcagaa gcctggacaa gtgcgggcca cccaacaaca atatgatttc 7920
actccaacac aagcttcagt tgaaaggagc tcatttgatt ggctgactgg gagcagcatt 7980
gacctactgg cggatcacac cgtcttctct tcagagacct tgtcttcctc cttgctgttt 8040
tcccacaaaa ggactgaaaa gtcacagaga atgtcttgta agtcagtagg accggacttt 8100
ggaacaaaaa agctgggcct tcctgatgat gaggtggata accaagtgaa aagtggcact 8160
ccgagccagg cagatattct gagattgcgc agaagatttt taaaggaccg agaaaagttg 8220
agtttgctgt atgccaaaag gggcctcatg gaacaaaaac tagagaagga tatcaagagt 8280
gagtttaaaa tgaagcagga tgcgcaggtt gttctgtaca gaagttatcg tcatggcgat 8340
cttcctgata tccagatcca gcacagtggt ctgatcaccc ccttgcaggc tgtggcccag 8400
aaagacccaa taattgcaaa acaactcttc agcagcttgt tttctgggat tttgaaagaa 8460
atgaataaat ttaagacaac atctgaaaag aacatcatca ctcaaaattt gctccaagat 8520
ttcaaccgct tccttaatac aactttcttg ttctttccac cctttgtctc ttgcatccag 8580
gaaataagtt gccaacaccc agacttcctg acacttgacc cagcctcagt tcgagtggga 8640
tgcctggcta gtctgcagca gccgggaggt atccgtcttc tggaagaggc cctactccgg 8700
ctgatgccca aggaaccacc caccaaaaga gttcggggca agacttgctt gcctcccgat 8760
gttctccggt ggatggaact tgctaaactc tatcgttcga ttggggagta tgatgttctc 8820
cgtggcattt ttagcagtga gttaggaaca acgcaggaca ctcagaacgc actattagca 8880
gaagctagaa gtgattattg tcaagctgct aagttgtatg atgaggcttt aaataaactg 8940
gagtgggtgg atggtgaacc cacagaagct gagaaggagt tttgggagct ggcatcgctt 9000
gactgttaca acaacctctc taaatggaaa gaacttgagt actgttctac agtcaatata 9060
gtcagtgaga actctcttga cctaagtaaa atgtggagtg aaccatttta tcaggagacg 9120
tatctgccat atgtgatccg tagcaaacta aagctcctgc ttcaaggaga aggcaaccaa 9180
tctctgctaa catttgtgga tgaagccatg aacaaggagc tgcagaagac ggtcctggag 9240
cttcagtata gtcaagagct gagtcttctt tacatcttgc aggatgatat tgacagagcc 9300
acatactaca ttaaaaatgg cattcagatt ttcatgcaaa attattctag tattgatgtt 9360
ctgttatata gaagtagact cgccaagtta cagtctgtac agactttagc agaaattgag 9420

gagttcctaa gttttatatg taaacatggt gacttatcat ctctgggtcc cctaaggaga 9480
cttctaaaaa cctggaccag cagataccca gatgttgtga cagacccaat gcacatctgg 9540
gatgacatca tcacaaatcg ctgcttcttt ctcagtaaaa tagaagagag actgactgct 9600
ccttcaggag atcacagtat gagtgtggat gaggatgaag agtccattga cagggaagtg 9660
tacgagccaa aggaagatgt tcgttgcatg cttcagagct gcagattcac catgaaaatg 9720
aagatgatag aaagcgcctg gaagcagagt aatttctcac tctccatgaa actcctgaaa 9780
gagatgcata aagaatcaaa aacaagagaa atttggcgag tgcagtggtt gcatagttac 9840
tcccagctga accactgccg gagccacact cagagccctc gggaacaagt cctcaatacg 9900
ctcaaaacca tcacgctgtt ggatgaaagt gatatttcaa actacttaaa taaaaacatt 9960
caggcctctt gtgaccagag cattctcttg ggcacaactt gcagaatcat ggctgatgct 10020
ctcagcagag agccagcctg cctgtctgac ctggaggaga acaaggtgaa ctcaatcttg 10080
acactttccg gatctaatgc agagaacaca gagacggtaa tcacgggtct gtaccagaga 10140
gcattccatc acctctccaa ggccgtgcag tcagctgaag aggaaaccca gctttcctgc 10200
tggggccatg aggctgctgc tgagcgggcc catgcttata tgacactggt aggcttctgt 10260
gaccaacaac tccgaaaggt agaagagagt gcctcacaga agacaagtgc agaaatggaa 10320
gcatatccag cacttgtggt ggaaaaaatg ctgagagcat taaagttaaa ctcttctgaa 10380
gccaggctga aatttcctag attacttcag attatagagc agtattcaga ggagacctta 10440
aacataatga caaaagagat ttcatctatt ccttgctggc agttcattgg ctggatcagc 10500
cacatgatgg ccttactgga caaagaggaa gctattgcag tacaacatac tgtggaagaa 10560
attgctgata actacccaca ggccattatc taccctttta taataagcag cgaaagctac 10620
tcctttaaaa acacttcttc tggtcataac aataaagcat ttgttgagag gataaaaagt 10680
aaacttgatc acggagaagt gattcacagt tttattaacg ccttagacca gctctccaat 10740
cctgacctgc tcttcaagga ctgggtttct gatacaaaag atgaactagg aaagaatcct 10800
gtgaataaga aaaatattga aaagttgtat gaaaggatgt atgctgcctt gggtgacctt 10860
cgtgctccag gcctggggcc ctttcggagg aggttcatac aggcgtttgg gaaagaattt 10920
gtcaagagct ttgggaatgg tggttctaag ctgcttacaa tgaaggtcga tgacttttgt 10980
aagatcactg gctcgctact tgtaaggatg aaaaaagact caaagctacc tgggaacctg 11040
aaagagtatt ccccctggat gagtgagttc aaagcgcagt tcctgaaaaa tgaactggag 11100
attcctggac aatatgatgg aaaaagcaaa ccactgcctg aatatcacgt gcggatctct 11160
ggatttgatg agcgggtaaa agtgatgctc tccttgagga agccaaagcg cattgttatt 11220
cgaggccatg atgaaaagga atacccgttc ctggtaaaag gtggtgagga cctccgtcaa 11280
gaccagcgca ttgagcagat ctttgaggtc atgaatgcca ttctctctca ggatgctgcc 11340
tgcagtcaga gaaacatgca gttaaggact taccgtgtcg tgccaatgac ctctagatta 11400
ggattaattg aatggattga aaatactatg accttgaagg atcttctttt gagtaatatg 11460
tcacaagagg agaaagtggc taataacagt gaccccaaag cgccgatccg tgactataaa 11520
gactggctga tgaaagtgtc ggggaaaagc gatgctggag cctatgtgct aatgtacagc 11580
cgagctaacc gtacagaaac agttgtagct ttcagaagaa gggaaagtca agtgccgcct 11640
gacctcttaa agcgggcctt tgtaaagatg agcaccagcc ctgaggcctt cctggcactg 11700
cgttcccact ttgccagctc ccatgcactg ctgtgcatca gccactggct cctcgggatt 11760
ggagacagac acctgaacaa tttcatggtg gccatggaga caggcagtgt gattggaatt 11820
gactttggac atgcgtttgg atcagctact cagtttcttc cagtccctga gttgatgcct 11880
tttcgtctaa cccgccaatt tgtcagtctc atgttgccaa tgaaagaaac gggtctcatg 11940
tgcacggtca tggtgcatgc actgagggct ttccgctctt gtgcaggtct gctcactgac 12000
accatggaaa tttttgtgaa ggaaccctcc tttgattgga agagttttga gcagacaatg 12060
ctgagaaaag gaggatcatg gattcaagaa ataaatgtaa cggaaaagaa ttggtatcca 12120
caacataaaa tacgctatgc taagagaaag ttagcagggg ccaacccagc tgttataact 12180
tgtgatgagc tatatctagg ccatgaagct tcatccgcct ttaggagtta tacagctgta 12240
gctcgaggca acagagacta caacattcgt gcacaagagc cagagagtgg gctttcagaa 12300
gagactcaag tgaagtgctt ggtggaccag gccacagacc ccaatatcct tggcaggact 12360
tgggaaggat gggagccctg gatgtaaagt ctgtggtgtc accaatcata aagcattctg 12420
tctccgagag gacctcctag accttcgctg acagcagtat atttctgaca gacagaggaa 12480
atctacttta tggtgctggc gagatggtca gataaaggca tttgttacca atgctaatgg 12540
cctaactaac ttcaatccca gaaacccata ttggaggcag tgaaccaact ccaggaagtt 12600
gtcctttgac ctccacacat gtaaaaaata aagaatatga caaatataaa aaa 12653

<210> 3
<211> 124
<212> PRT
<213> Rattus norvegicus

<220>
<223> cAMP-dependent protein kinase regulatory subunit RIalpha

<400> 3
Ser Leu Asp Lys Trp Glu Arg Leu Thr Val Ala Asp Ala Leu Glu Pro
1               5                   10                  15

Val Gln Phe Glu Asp Gly Gln Lys Ile Val Val Gln Gly Glu Pro Gly
            20                  25                  30

Asp Glu Phe Phe Ile Ile Leu Glu Gly Thr Ala Ala Val Leu Gln Arg
        35                  40                  45

Arg Ser Glu Asn Glu Glu Phe Val Glu Val Gly Arg Leu Gly Pro Ser
    50                  55                  60

Asp Tyr Phe Gly Glu Ile Ala Leu Leu Met Asn Arg Pro Arg Ala Ala
65                  70                  75                  80

Thr Val Val Ala Arg Gly Pro Leu Lys Cys Val Lys Leu Asp Arg Pro
                85                  90                  95

Arg Phe Glu Arg Val Leu Gly Pro Cys Ser Asp Ile Leu Lys Arg Asn
            100                 105                 110

Ile Gln Gln Tyr Asn Ser Phe Val Ser Leu Ser Val
        115                 120


<210> 4
<211> 5699
<212> DNA
<213> Rattus norvegicus

<220>
<223> cAMP-dependent protein kinase regulatory subunit RIalpha

<400> 4
aattcttttg attcttctat ttcagagtct ctggacaagt gggagcgtct cacagtagcc 60
gatgcattgg agcctgtcca gtttgaagat ggacagaaga tcgtggtgca aggagagccg 120
ggggatgagt tcttcatcat tttagaggta gagagctctg cgagtgactc cttaccatgc 180
gtcacctaaa ggaggggcag ccagttctga tttgtagctt tagttttatt tagtcgttat 240
cttttacttc agaatacagc ttgctgtgta tagtctgcta cagttttaag gaattagagt 300
cagaatcctg caggggccct agacatgatt ggtgagatgg ctctctacta cactaaaaag 360
cccctcagcc ataacagata cggtgattct gagccaaagt gcttaggcta gttagtaatg 420
attgacaatt ctaaaagtca ctgttactga aacatctccc tatcatcctg cttgccaagc 480
aggtccagat ggcagcttca gagtctgaac tcttgtcgca ctgcagatgc agccatggag 540
atgtgagccc tgagtgtgat agtctttaaa atcttatttc caaagatgca gcaggcttga 600
gcttgaccct ttacgtaaac tttactcacc ggagtctgaa ctcctttgtt agtgcctgag 660
cttgatacga gccttgatag cggggtggat gctggttcag tggaagtcag cgttgaaata 720
actgctcagt tcactcagga accggcgatt gcacagtatg tgttaggacc gtgtgtttag 780
cttcttgcgt tggtgtgatt acagggcaca gctgctgtgc tgcagcgtcg gtcagaaaac 840
gaagaatttg ttgaagtggg acgactgggg ccttctgatt attttggtat gttaaatttc 900
ttcataaata catggtttaa ggatctgtgg gacagtgtag tttcccataa ttagttttgt 960
tttctttcct ttacttagtt ctggggcaga gtgtggcctg gaactcactc tgtagaccag 1020
gctgcccaca aactcagaga cccacctgtc tccatttcta gagtgatggg attgaatgta 1080
ggcaccccca caccctgcct ttattgtttt tgagacaggg tctttggtaa cagtgcagac 1140
cggccttctt agatctgcct tagttagcct cctaagtgtt ggggttatag tcatgatacc 1200
tggtagattt cttaacctat actctttctt gtttctcaga gaagtactgt aacattaact 1260
ttattcagtt aatagatgag cacagaccaa gcatgcatgc gcacacgcac ttgcgcttat 1320
gtcctacata gcctcgactg cctggacttc attgtacagt gaagcttgtt ctaactggtc 1380

accttcctgc ctctggctca tgagtgttgg tttgtaggca tgactgtagg ggctgtgtta 1440
tctatttgct agtaatactt tttaatgtac aaatgttgtg tggcctctac taagttagta 1500
gggaagttgc atctttgttc acttgctcag gagtggaccc tttaaggaaa tagcttccat 1560
aggtgatagt cgttactgtc tcttttccag gtgaaattgc cctgctgatg aatcgtcctc 1620
gggctgccac tgtggttgcc cggggccctt tgaagtgcgt taagttggac cggcctcggt 1680
ttgaacgcgt ccttggcccg tgctcagaca tcctcaagcg gaacatccag cagtacaaca 1740
gcttcgtgtc cctgtccgtc tgacccgcgc ctcctgtgcc tcctccccag tccatgcttc 1800
actcacgcag actgctttct ctccctagcc gcagcgccca gtggccactg cttcacagct 1860
tcttgtctct ttatactaaa agttgcttta ttgcaccatt tttcattggg agcgttatta 1920
aatgctcatt cacaaataaa tagagagttg tatggacttg gctgtcaccg cttctctgtg 1980
cagtgctcgt gttcaacctg ggcagcaagt gccgcgcttt ttgctgaggg cggtccctgc 2040
accactgaat taccgtaatg atgtaatagc tgtgcagggc ttttaagtga cacaggtttc 2100
catttccata tgacacaagt gacatactta gactgtggcc atgtttgctg tatttcttta 2160
tagagtaaat gattttaacc tttaaggatt agaagaatga atatagaaaa ctcctagcac 2220
agtagaaaga catctgcctg taattaagct agtttaagtg tgggagtgcc catgttttgc 2280
taatcaccag gcactgcagc tagttcagcc ctctcagcct ggtttggtgt gtctgttgct 2340
agtgtttctt ctccaaccct gaaatgtttt ggtgtggctg gccgtctatc cctgccttta 2400
agtttgaaat catttgtaga tggcaagtac tggttgtgac ttcactgcac ctgtctgagc 2460
tcacagacgc tgataacttt atttatccag ctggtgccaa atcctgagga tgagacacag 2520
aaatgtgaga atttgaggtc ttcactctgg ggagttaacc gtggttaact cgtgtgatct 2580
gccctcagtt acagactagg gcgttggaat tactgagaat agacaaatag agcaagctcc 2640
agagagaagg ggatggaggc agtgctgctg aaggtggatg gcggtcaggt gtccagaagc 2700
aggaagctgg gtctgtcccc tgggtttctg ttaggaacag tggtggggtg tgaccgtgtt 2760
gtggtctgaa aggccacccc gctgagtcct gccttttcct gtctaacctt gaatttctct 2820
ggtaaacttt ctttattgtt ttttgtttgt gtgtgtgtgt ttaaagtaaa cttgtgttct 2880
tgactgtatt tcagtatttt ccagccttat gtgttacatt attccagtga taacccaaca 2940
gtttattttt attactgtta ttttttaaac aaaattcaca gttcgtaacg aggcacactt 3000
atcttgtgat ttctgtataa tgtcgggttc tatttgggag tgactgcgtt ttcatgtgtg 3060
tgcaagtggt tctgattaat aattccctcc ctccctttcc cagttaactt aaattgttgg 3120
ttatggtaga tttttttctt ggtagattga aaagcttcta gattgttact aatttgggaa 3180
aaagtttcat ttaggtttta gcatgggata gaaagctacc ttgttgccat atctaaaact 3240
tccataatct atattaaatg agggttttct gtacttactt tgtggttggc tatctttttt 3300
tatatttaaa aaaataaaaa ttatgttact agcttaaagc ttaatttgat atttgtttaa 3360
atgccaaaaa tgtacgtatg tgaatgatgt agaacgccat aaaatgcatt tcatgtgtat 3420
ataccccctt ggtctgtacg gagataaaca cctgctttct aagagttcca tgctagctta 3480
caagttacgc ccttttgtcg ttttactctt agacttttca agataagtct aaatttaaaa 3540
attaccacat ttaaagcttt atctttgtgc aatttaaata tcagtcagaa gtcgtggttg 3600
gcatactgtg tctttgtgga ctcttggcac cattgtctct ggatgggtgg gcgaatttaa 3660
gcaatctgct gagaaagcag agctggccaa agggacagtt gctgtttcct ggacactagt 3720
ctgatatact ctgtgctaat ctaatcgctg ttcttttgtt acagactgtc tcaatggtag 3780
gtcacagaat aaaaaagatg aaataattta aattcttaaa cgaatcagtt tttcttcctt 3840
tcttcctttt cttcctctgt ttttccccaa aatttcagtg ggaaagaatg aaagtgaatt 3900
ttgggattag aattttgaaa aagtaatgta tttatttttt tttaaaatac attccaattc 3960
agtttgtcaa agggtggatt tcaaaacctt gcttttatgg gtgtgggaca gactcactaa 4020
caagctctgg aaggggaagg ttctgtccaa agccatggcg ttgttttcac aggacagacc 4080
tgtctgtgtg ccttagggct tcacagactg gattggactc ctggttttac aagatccacc 4140
tgtagctgct tgaggatctt acacatcaag atttgtttct gtttcttcct ggagtttgaa 4200
gagtgagcac atgagttact ctggtgataa ctgggatctt cacacgcagg gaagctggag 4260
ttagtgctgg agagtggctg agttctgctg cttaagctct gcctggccag gttcagaagt 4320
cccttcctct caccagctgc atgtccttct tttaaaattt ggcccaggat gcagtttact 4380
gttgtgttga cattctgaaa ttgaattagc acaatggcag tggcgtcact ttgtctccaa 4440
cctcaaagat ttggaccctt tgtgaacttt ggcaaagccc tgccagtgca cttccgggtg 4500
gaccaaaacc atgctaacca tttaatttgc cctccatcac atttgccaag gggaaacaca 4560
aatctctccc ttcttacatt tggggtattt ctgtagaaac catcattttt gtgtacatac 4620
acagctaaaa tttgtttctt ggcataactt tagaactgtg acatcatcat gagtccctgt 4680
aagaacaaga agtagctttg tgattttaaa aaagtcacca ctagatggag agctttttct 4740
ttatgctcag ctttgccaac ctcagatgag tcgcagctgt ttctcagtct gcagatcctg 4800
catttgcgtg tctccctact tggtacaatg taagtgtgac cccgagactg ccacaccaga 4860
agccctctgg gacctgagct gcactcgcag agaaaaacgt gattatccct tgccttccca 4920
cccgaggctg agtgaggtct gcctcacctc atctgtggcc atgtcacctg ggggcctcct 4980
gcaccacgct tgtgtggggg ccctcgtgcc tgctgttgat gaggtcactc ggtatttaga 5040

aggcctccca ggatagtgct catgggctga ctcgcgttcc tgagtttgag aaagctgtct 5100
cttactgtga acgtgcagca gtagacagat gcagtgttaa agaaccagca atgtaaagac 5160
atgatcttta ccacacaaga tgagattctg tagtaatggg tcagtgaagt tagactagag 5220
aatcctaaga acctgacctc atacttctcc taggggcagt aataatttac cagttaagag 5280
gccaggagtt taataggaca gaattgctcc ctataagaga attgacttat ccttagatag 5340
gtccagcatt tggagaatgc tgtggcatat ccctccaaac tgatccctac cagttcacac 5400
agtcttggtt tttttatttg cttgccagtg gtaacatgga aagttttcac atagactcag 5460
aatatttttt ttccccatgg gaagttgagc aatgattcag agtgcgtaag ctgtaagaac 5520
tccacactct gagaatggaa tgtttttgtc ttctggtgct tttgaaaaat gctgctctta 5580
gctttatgtt gttgttgtta gaaaaacaga tagttaaaag tagcctatat agttatccat 5640
catagaatca agccgagtta ctttggtttt gaaatgtaaa gcagatttca gagtctcga 5699

<210> 5
<211> 388
<212> PRT
<213> Homo sapiens

<220>
<223> Protein tyrosine kinase t-Ror1

<400> 5
Met Leu Phe Glu Tyr Ile Asn Gln Gly Asp Leu His Glu Phe Leu Ile
1               5                   10                  15

Met Arg Ser Pro His Ser Asp Val Gly Cys Ser Ser Asp Glu Asp Gly
            20                  25                  30

Thr Val Lys Ser Ser Leu Asp His Gly Asp Phe Leu His Ile Ala Ile
        35                  40                  45

Gln Ile Ala Ala Gly Met Glu Tyr Leu Ser Ser His Phe Phe Val His
    50                  55                  60

Lys Asp Leu Ala Ala Arg Asn Ile Leu Ile Gly Glu Gln Leu His Val
65                  70                  75                  80

Lys Ile Ser Asp Leu Gly Leu Ser Arg Glu Ile Tyr Ser Ala Asp Tyr
                85                  90                  95

Tyr Arg Val Gln Ser Lys Ser Leu Leu Pro Ile Arg Trp Met Pro Pro
            100                 105                 110

Glu Ala Ile Met Tyr Gly Lys Phe Ser Ser Asp Ser Asp Ile Trp Ser
        115                 120                 125

Phe Gly Val Val Leu Trp Glu Ile Phe Ser Phe Gly Leu Gln Pro Tyr
    130                 135                 140

Tyr Gly Phe Ser Asn Gln Glu Val Ile Glu Met Val Arg Lys Arg Gln
145                 150                 155                 160

Leu Leu Pro Cys Ser Glu Asp Cys Pro Pro Arg Met Tyr Ser Leu Met
                165                 170                 175

Thr Glu Cys Trp Asn Glu Ile Pro Ser Arg Arg Pro Arg Phe Lys Asp
            180                 185                 190

Ile His Val Arg Leu Arg Ser Trp Glu Gly Leu Ser Ser His Thr Ser
        195                 200                 205

Ser Thr Thr Pro Ser Gly Gly Asn Ala Thr Thr Gln Thr Thr Ser Leu
    210                 215                 220

Ser Ala Ser Pro Val Ser Asn Leu Ser Asn Pro Arg Tyr Pro Asn Tyr
225                 230                 235                 240

Met Phe Pro Ser Gln Gly Ile Thr Pro Gln Gly Gln Ile Ala Gly Phe
                245                 250                 255

Ile Gly Pro Pro Ile Pro Gln Asn Gln Arg Phe Ile Pro Ile Asn Gly
            260                 265                 270

Tyr Pro Ile Pro Pro Gly Tyr Ala Ala Phe Pro Ala Ala His Tyr Gln
        275                 280                 285

Pro Thr Gly Pro Pro Arg Val Ile Gln His Cys Pro Pro Pro Lys Ser
    290                 295                 300

Arg Ser Pro Ser Ser Ala Ser Gly Ser Thr Ser Thr Gly His Val Thr
305                 310                 315                 320

Ser Leu Pro Ser Ser Gly Ser Asn Gln Glu Ala Asn Ile Pro Leu Leu
                325                 330                 335

Pro His Met Ser Ile Pro Asn His Pro Gly Gly Met Gly Ile Thr Val
            340                 345                 350

Phe Gly Asn Lys Ser Gln Lys Pro Tyr Lys Ile Asp Ser Lys Gln Ala
        355                 360                 365

Ser Leu Leu Gly Asp Ala Asn Ile His Gly His Thr Glu Ser Met Ile
    370                 375                 380

Ser Ala Glu Leu
385


<210> 6
<211> 2373
<212> DNA
<213> Homo sapiens

<220>
<223> Protein tyrosine kinase t-Ror1

<400> 6
gcacgagcgg ttctgagcat tagtttgaga actcgttccc gaatgtgctt tcctccctct 60
cccctgccca cctcaagttt aataaataag gttgtacttt tcttactata aaataaatgt 120
ctgtaactgc tgtgcactgc tgtaaacttg ttagagaaaa aaataacctg catgtgggct 180
cctcagttat tgagtttttg tgatcctatc tcagtctggg ggggaacatt ctcaagaggt 240
gaaatacaga aagccttttt ttcttgatct tttcccgaga ttcaaatctc cgattcccat 300
ttgggggcaa gtttttttct tcaccttcaa tatgagaatt cagcgaactt gaaagaaaaa 360
tcatctgtga gttccttcag gttctcactc atagtcatga tccttcagag ggaatatgca 420
ctggcgagtt taaagtaagg gctatgatat ttgatggtcc caaagtacgg cagctgcaaa 480
aagtagtgga aggaaattgt ctacgtgtct tggaaaaatt agttaggaat ttggatgggt 540
aaaaggtacc cttgccttac tccatcttat tttcttagcc ccctttgagt gttttaactg 600
gtttcatgtc ctagtaggaa gtgcattctc catcctcatc ctctgccctc ccaggaagtc 660
agtgattgtc tttttgggct tcccctccaa aggaccttct gcagtggaag tgccacatcc 720
agttcttttc ttttgttgct gctgtgttta gataattgaa gagatctttg tgccacacag 780
gatttttttt ttttttaaga aaaacctata gatgaaaaat tactaatgaa actgtgtgta 840
cgtgtctgtg cgtgcaacat aaaaatacag tagcacctaa ggagcttgaa tcttggttcc 900

tgtaaaattt caaattgatg tggtattaat aaaaaaaaaa aaaaaaaact cgctcgtgcc 960
gaattcggca cgaggaacaa cctgtgtgca tgctttttga gtatattaat cagggggatc 1020
tccatgagtt cctcatcatg agatccccac actctgatgt tggctgcagc agtgatgaag 1080
atgggactgt gaaatccagc ctggaccacg gagattttct gcacattgca attcagattg 1140
cagctggcat ggaatacctg tctagtcact tctttgtcca caaggacctt gcagctcgca 1200
atattttaat cggagagcaa cttcatgtaa agatttcaga cttggggctt tccagagaaa 1260
tttactccgc tgattactac agggtccaga gtaagtcctt gctgcccatt cgctggatgc 1320
cccctgaagc catcatgtat ggcaaattct cttctgattc agatatctgg tcctttgggg 1380
ttgtcttgtg ggagattttc agttttggac tccagccata ttatggattc agtaaccagg 1440
aagtgattga gatggtgaga aaacggcagc tcttaccatg ctctgaagac tgcccaccca 1500
gaatgtacag cctcatgaca gagtgctgga atgagattcc ttctaggaga ccaagattta 1560
aagatattca cgtccggctt cggtcctggg agggactctc aagtcacaca agctctacta 1620
ctccttcagg gggaaatgcc accacacaga caacctccct cagtgccagc ccagtgagta 1680
atctcagtaa ccccagatat cctaattaca tgttcccgag ccagggtatt acaccacagg 1740
gccagattgc tggtttcatt ggcccgccaa tacctcagaa ccagcgattc attcccatca 1800
atggataccc aatacctcct ggatatgcag cgtttccagc tgcccactac cagccaacag 1860
gtcctcccag agtgattcag cactgcccac ctcccaagag tcggtcccca agcagtgcca 1920
gtgggtcgac tagcactggc catgtgacta gcttgccctc atcaggatcc aatcaggaag 1980
caaatattcc tttactacca cacatgtcaa ttccaaatca tcctggtgga atgggtatca 2040
ccgtttttgg caacaaatct caaaaaccct acaaaattga ctcaaagcaa gcatctttac 2100
taggagacgc caatattcat ggacacaccg aatctatgat ttctgcagaa ctgtaaaatg 2160
cacaactttt gtaaatgtgg tatacaggac aaactagacg gccgtagaaa agatttatat 2220
tcaaatgttt ttattaaagt aaggttctca tttagcagac atcgcaacaa gtaccttctg 2280
tgaagtttca ctgtgtctta ccaagcagga cagacactcg gccagaaaaa aaaaaaaaaa 2340
aaaaaactcg agggggggcc cgtacccgat cgc 2373

<210> 7
<211> 929
<212> PRT
<213> Rattus norvegicus

<220>
<223> Diacylglycerol kinase

<400> 7
Met Glu Pro Arg Asp Pro Ser Pro Glu Ala Arg Ser Ser Asp Ser Glu
1               5                   10                  15

Ser Ala Ser Ala Ser Ser Ser Gly Ser Glu Arg Asp Ala Asp Pro Glu
            20                  25                  30

Pro Asp Lys Ala Pro Arg Arg Leu Thr Lys Arg Arg Phe Pro Gly Leu
        35                  40                  45

Arg Leu Phe Gly His Arg Lys Ala Ile Thr Lys Ser Gly Leu Gln His
    50                  55                  60

Leu Ala Pro Pro Pro Pro Thr Pro Gly Ala Pro Cys Gly Glu Ser Glu
65                  70                  75                  80

Arg Gln Ile Arg Ser Thr Val Asp Trp Ser Glu Ser Ala Ala Tyr Gly
                85                  90                  95

Glu His Ile Trp Phe Glu Thr Asn Val Ser Gly Asp Phe Cys Tyr Val
            100                 105                 110

Gly Glu Gln Tyr Cys Val Ala Lys Met Leu Pro Lys Ser Ala Pro Arg
        115                 120                 125

Arg Lys Cys Ala Ala Cys Lys Ile Val Val His Thr Pro Cys Ile Gly

130                 135                 140

Gln Leu Glu Lys Ile Asn Phe Arg Cys Lys Pro Ser Phe Arg Glu Ser
145                 150                 155                 160

Gly Ser Arg Asn Val Arg Glu Pro Thr Phe Val Arg His His Trp Val
                165                 170                 175

His Arg Arg Arg Gln Asp Gly Lys Cys Arg His Cys Gly Lys Gly Phe
            180                 185                 190

Gln Gln Lys Phe Thr Phe His Ser Lys Glu Ile Val Ala Ile Ser Cys
        195                 200                 205

Ser Trp Cys Lys Gln Ala Tyr His Ser Lys Val Ser Cys Phe Met Leu
    210                 215                 220

Gln Gln Ile Glu Glu Pro Cys Ser Leu Gly Val His Ala Ala Val Val
225                 230                 235                 240

Ile Pro Pro Thr Trp Ile Leu Arg Ala Arg Arg Pro Gln Asn Thr Leu
                245                 250                 255

Lys Ala Ser Lys Lys Lys Lys Arg Ala Ser Phe Lys Arg Arg Ser Ser
            260                 265                 270

Lys Lys Gly Pro Glu Glu Gly Arg Trp Arg Pro Phe Ile Ile Arg Pro
        275                 280                 285

Thr Pro Ser Pro Leu Met Lys Pro Leu Leu Val Phe Val Asn Pro Lys
    290                 295                 300

Ser Gly Gly Asn Gln Gly Ala Lys Ile Ile Gln Ser Phe Leu Trp Tyr
305                 310                 315                 320

Leu Asn Pro Arg Gln Val Phe Asp Leu Ser Gln Gly Gly Pro Arg Glu
                325                 330                 335

Ala Leu Glu Met Tyr Arg Lys Val His Asn Leu Arg Ile Leu Ala Cys
            340                 345                 350

Gly Gly Asp Gly Thr Val Gly Trp Ile Leu Ser Thr Leu Asp Gln Leu
        355                 360                 365

Arg Leu Lys Pro Pro Pro Pro Val Ala Ile Leu Pro Leu Gly Thr Gly
    370                 375                 380

Asn Asp Leu Ala Arg Thr Leu Asn Trp Gly Gly Gly Tyr Thr Asp Glu
385                 390                 395                 400

Pro Val Ser Lys Ile Leu Ser His Val Glu Glu Gly Asn Val Val Gln
                405                 410                 415

Leu Asp Arg Trp Asp Leu Arg Ala Glu Pro Asn Pro Glu Ala Gly Pro
            420                 425                 430

Glu Glu Arg Asp Asp Gly Ala Thr Asp Arg Leu Pro Leu Asp Val Phe
        435                 440                 445

Asn Asn Tyr Phe Ser Leu Gly Phe Asp Ala His Val Thr Leu Glu Phe
    450                 455                 460

His Glu Ser Arg Glu Ala Asn Pro Glu Lys Phe Asn Ser Arg Phe Arg
465                 470                 475                 480

Asn Lys Met Phe Tyr Ala Gly Thr Ala Phe Ser Asp Phe Leu Met Gly
                485                 490                 495

Ser Ser Lys Asp Leu Ala Lys His Ile Arg Val Val Cys Asp Gly Met
            500                 505                 510

Asp Leu Thr Pro Lys Ile Gln Asp Leu Lys Pro Gln Cys Ile Val Phe
        515                 520                 525

Leu Asn Ile Pro Arg Tyr Cys Ala Gly Thr Met Pro Trp Gly His Pro
    530                 535                 540

Gly Glu His His Asp Phe Glu Pro Gln Arg His Asp Asp Gly Tyr Leu
545                 550                 555                 560

Glu Val Ile Gly Phe Thr Met Thr Ser Leu Ala Ala Leu Gln Val Gly
                565                 570                 575

Gly His Gly Glu Arg Leu Thr Gln Cys Arg Glu Val Leu Leu Thr Thr
            580                 585                 590

Ala Lys Ala Ile Pro Val Gln Val Asp Gly Glu Pro Cys Lys Leu Ala
        595                 600                 605

Ala Ser Arg Ile Arg Ile Ala Leu Arg Asn Gln Ala Thr Met Val Gln
    610                 615                 620

Lys Ala Lys Arg Arg Ser Thr Ala Pro Leu His Ser Asp Gln Gln Pro
625                 630                 635                 640

Val Pro Glu Gln Leu Arg Ile Gln Val Ser Arg Val Ser Met His Asp
                645                 650                 655

Tyr Glu Ala Leu His Tyr Asp Lys Glu Gln Leu Lys Glu Ala Ser Val
            660                 665                 670

Pro Leu Gly Thr Val Val Val Pro Gly Asp Ser Asp Leu Glu Leu Cys
        675                 680                 685

Arg Ala His Ile Glu Arg Leu Gln Gln Glu Pro Asp Gly Ala Gly Ala
    690                 695                 700

Lys Ser Pro Met Cys His Pro Leu Ser Ser Lys Trp Cys Phe Leu Asp
705                 710                 715                 720

Ala Thr Thr Ala Ser Arg Phe Tyr Arg Ile Asp Arg Ala Gln Glu His
                725                 730                 735

Leu Asn Tyr Val Thr Glu Ile Ala Gln Asp Glu Ile Tyr Ile Leu Asp
            740                 745                 750

Pro Glu Leu Leu Gly Ala Ser Ala Arg Pro Asp Leu Pro Thr Pro Thr
        755                 760                 765

Ser Pro Leu Pro Ala Ser Pro Cys Ser Pro Thr Pro Gly Ser Leu Gln
    770                 775                 780

Gly Asp Ala Ala Leu Pro Gln Gly Glu Glu Leu Ile Glu Ala Ala Lys
785                 790                 795                 800

Arg Asn Asp Phe Cys Lys Leu Gln Glu Leu His Arg Ala Gly Gly Asp
                805                 810                 815

Leu Met His Arg Asp His Gln Ser Arg Thr Leu Leu His His Ala Val
            820                 825                 830

Ser Thr Gly Ser Lys Glu Val Val Arg Tyr Leu Leu Asp His Ala Pro
        835                 840                 845

Pro Glu Ile Leu Asp Ala Val Glu Glu Asn Gly Glu Thr Cys Leu His
    850                 855                 860

Gln Ala Ala Ala Leu Gly Gln Arg Thr Ile Cys His Tyr Ile Val Glu
865                 870                 875                 880

Ala Gly Ala Ser Leu Met Lys Thr Asp Gln Gln Gly Asp Thr Pro Arg
                885                 890                 895

Gln Arg Ala Glu Lys Ala Gln Asp Thr Glu Leu Ala Ala Tyr Leu Glu
            900                 905                 910

Asn Arg Gln His Tyr Gln Met Ile Gln Arg Glu Asp Gln Glu Thr Ala
        915                 920                 925

Val

<210> 8
<211> 3560
<212> DNA
<213> Rattus norvegicus

<220>
<223> Diacylglycerol kinase

<400> 8
ggagcggagg ctgcttcccc ggcacctggg agtgggtcgc ggggacgcga aggcacaggg 60
cgggcggacg cgccttcgcc atgagcttgg cggcggtgcg gaccgggcgt gctgagctcc 120
ggccgccggc ccggcatggg cgtctcccgc gagcccgctg ccggccgggg ctagggccgg 180
atggagccgc gggaccccag ccccgaggcc cggagcagcg actcggagtc ggcctccgcc 240
tcgtccagcg gctccgagcg cgacgcggat ccggagccgg acaaggctcc gcggcgctta 300
accaagaggc gcttcccggg gctgcggctc tttgggcaca ggaaagccat caccaagtcg 360
ggcctccagc acctggcacc ccctcctccc acgcctgggg ccccgtgcgg tgaatctgag 420
cggcagatcc ggagtactgt ggactggagt gagtcagcag cgtatgggga gcacatctgg 480
tttgagacca acgtgtccgg agatttctgc tatgtcgggg agcagtactg tgtagctaag 540
atgctgccga agtcagcgcc cagaagaaag tgtgcagcct gtaagatcgt ggtgcatacc 600
ccttgcatcg ggcagctgga aaagatcaat ttccgctgta agccatcctt ccgtgaatca 660
ggctccagga atgtccgtga gccaaccttc gtaagacacc actgggtcca caggcgacgc 720
caggatggca agtgtcggca ctgtgggaag ggcttccagc agaagttcac cttccacagc 780
aaagagatcg tagctatcag ctgttcctgg tgcaaacagg cgtaccacag caaggtgtcc 840
tgcttcatgc tgcagcagat tgaggagccc tgttccctag gggtgcatgc agccgtggtc 900
atcccaccca cctggatcct acgggcccgg aggccccaga acaccctcaa ggccagcaag 960
aagaaaaaga gagcatcctt caaaaggaga tccagtaaga aaggacctga ggaaggccgc 1020
tggagaccct tcatcatcag gcccaccccg tcccccctca tgaagcccct gctagtgttt 1080
gtaaacccca agagtggggg taaccagggc gctaagatca tccagtcttt tctgtggtat 1140
ctgaaccccc ggcaagtctt cgatctgagc cagggaggac ccagggaagc gctggaaatg 1200
taccgcaaag tccataattt gaggattctg gcttgcgggg gtgatggcac ggttggctgg 1260

attctttcca ccctagacca gctgcgctta aaaccaccac ctcctgtggc catcctgccc 1320
ctgggcactg gcaatgacct ggcccgaact ctcaactggg gtgggggtta cacagatgag 1380
ccagtgtcaa agatcctttc ccatgtagag gaggggaacg tggtacagct ggaccgttgg 1440
gacctccgag cagagcccaa ccctgaggca gggcctgagg agcgagatga cggagccact 1500
gaccggctgc ccctggatgt cttcaacaac tacttcagtc tgggctttga tgcccacgtc 1560
accctggagt ttcatgagtc tcgagaagcc aacccagaga agttcaacag ccgctttcgg 1620
aataagatgt tctatgctgg gacagccttc tctgacttcc tgatgggcag ctccaaggac 1680
ttagccaagc acatccgagt agtgtgtgac ggaatggacc taacccccaa gattcaggac 1740
ctgaaaccgc agtgcatagt ctttctgaat atccccaggt actgtgcagg caccatgccc 1800
tggggccacc ctggggagca ccatgacttc gagccccaga ggcacgatga tggctacctc 1860
gaggtcatcg gcttcaccat gacatccttg gccgcattgc aggtgggtgg acacggcgag 1920
cggctgaccc agtgccgaga agtgctgctc accactgcca aggccatccc tgtgcaggtg 1980
gatggtgaac cctgcaagct tgcagcatcg cggattcgaa ttgccctgcg caaccaggcc 2040
actatggtgc agaaggccaa gcgccggagt actgccccac tgcacagcga tcagcagcca 2100
gttcctgagc agctgaggat ccaagtgagc agggtcagca tgcacgacta cgaggctctc 2160
cattatgaca aggagcagct caaggaggcc tctgtgcctc tgggtactgt ggtggtcccc 2220
ggagacagtg acctagagct ctgccgagcc cacattgaga gactccagca ggagcccgat 2280
ggggctggag ccaagtcccc catgtgccac ccactgtcct ccaagtggtg tttcctggac 2340
gccaccactg ccagccgctt ctacaggatc gacagggccc aggagcacct caactatgtg 2400
acggagattg cccaggatga gatttatatc ctagacccag agctgctggg tgcatcagcc 2460
cgacctgacc tccctacccc tacctccccg ctccctgcct ccccctgctc ccctacaccc 2520
gggtcactgc agggggatgc cgcactgccc caaggtgaag agctgattga agctgccaag 2580
aggaatgact tctgcaagct ccaggaacta caccgagcag gaggtgacct catgcaccgc 2640
gaccatcaga gccgcacact cctgcaccac gcagtcagca ctggcagtaa ggaagtggtc 2700
cgctatctgc tcgatcatgc accaccagag atcctcgatg ctgtggagga gaacggggag 2760
acctgtctac accaggcagc cgccctaggt cagcgcacca tctgccacta cattgtggaa 2820
gccggggcct ccctcatgaa gacagatcag cagggcgaca ctcccaggca gcgagctgag 2880
aaggctcaag acacagagct agctgcatac ctggagaaca gacaacatta ccagatgatc 2940
caacgtgagg accaggagac agctgtgtag ttaaggggac catggatcaa cgagggacac 3000
taccagagga ctagagctct ccttgcccat ctcactgcca cattcctgtc agatggctat 3060
ggggggaccc tgtcacaggg aaggagcccc gtgccacccc ctgagaagcc gttcagatct 3120
agggctggac tctaaggagc tggactctca cctgtccctg gtttcatggg gaacaggaaa 3180
caagctgggc tgactgggtc cctcccttca ggcggcctcc ccctcaccac agctgatgga 3240
atggctggac agctcagtca gggaggcctg ctctcagcag gactttctaa agccacctga 3300
tcccttgggc tctttggaag gttctgggtg cctagccctc ctctctgacc cacacacctt 3360
gggcatccca gaaactcaag agcctgctgt atttgcctgc ccgctgccct gcttggcacc 3420
taccctggtg atcctcctca tgcacccagt catttcattt ccgactgtat ggcctggggt 3480
ggggggtggg gctcccacgg tgacttgttt acagctgggt gtgactcagt aaagtgaagt 3540
tttttccctt aaaaaaagcg 3560

<210> 9
<211> 380
<212> PRT
<213> Rattus norvegicus

<220>
<223> Elk protein tyrosine kinase

<400> 9
Glu Ala Val Arg Glu Phe Ala Lys Glu Ile Asp Val Ser Phe Val Lys
1               5                   10                  15

Ile Glu Glu Val Ile Gly Ala Gly Glu Phe Gly Glu Val Tyr Lys Gly
            20                  25                  30

Arg Leu Lys Leu Pro Gly Lys Arg Glu Ile Tyr Val Ala Ile Lys Thr
        35                  40                  45

Leu Lys Ala Gly Tyr Ser Glu Lys Gln Arg Arg Asp Phe Leu Ser Glu
    50                  55                  60

Ala Ser Ile Met Gly Gln Phe Asp His Pro Asn Ile Ile Arg Leu Glu
65                  70                  75                  80

Gly Val Val Thr Lys Ser Arg Pro Val Met Ile Ile Thr Glu Phe Met
                85                  90                  95

Glu Asn Gly Ala Leu Asp Ser Phe Leu Arg Gln Asn Asp Gly Gln Phe
            100                 105                 110

Thr Val Ile Gln Leu Val Gly Met Leu Arg Gly Ile Ala Ala Gly Met
        115                 120                 125

Lys Tyr Leu Ser Glu Met Asn Tyr Val His Arg Asp Leu Ala Ala Arg
    130                 135                 140

Asn Ile Leu Val Asn Ser Asn Leu Val Cys Lys Val Ser Asp Phe Gly
145                 150                 155                 160

Leu Ser Arg Tyr Leu Gln Asp Asp Thr Ser Asp Pro Thr Tyr Thr Ser
                165                 170                 175

Ser Leu Gly Gly Lys Ile Pro Val Arg Trp Thr Ala Pro Glu Ala Ile
            180                 185                 190

Ala Tyr Arg Lys Phe Thr Ser Ala Ser Asp Val Trp Ser Tyr Gly Ile
        195                 200                 205

Val Met Trp Glu Val Met Ser Phe Gly Glu Arg Pro Tyr Trp Asp Met
    210                 215                 220

Ser Asn Gln Asp Val Ile Asn Ala Ile Glu Gln Asp Tyr Arg Leu Pro
225                 230                 235                 240

Pro Pro Met Asp Cys Pro Ala Ala Leu His Gln Leu Met Leu Asp Cys
                245                 250                 255

Trp Gln Lys Asp Arg Asn Ser Arg Pro Arg Phe Ala Glu Ile Val Asn
            260                 265                 270

Thr Leu Asp Lys Met Ile Arg Asn Pro Ala Ser Leu Lys Thr Val Ala
        275                 280                 285

Thr Ile Thr Ala Val Pro Ser Gln Pro Leu Leu Asp Arg Ser Ile Pro
    290                 295                 300

Asp Phe Thr Ala Phe Thr Thr Val Asp Asp Trp Leu Ser Ala Ile Lys
305                 310                 315                 320

Met Val Gln Tyr Arg Asp Ser Phe Leu Thr Ala Gly Phe Thr Ser Leu
                325                 330                 335

Gln Leu Val Thr Gln Met Thr Ser Glu Asp Leu Leu Arg Ile Gly Val
            340                 345                 350

Thr Leu Ala Gly His Gln Lys Lys Ile Leu Ser Ser Ile His Ser Met
        355                 360                 365

Arg Val Gln Met Asn Gln Ser Pro Ser Val Met Ala
    370                 375                 380

<210> 10
<211> 1509
<212> DNA
<213> Rattus norvegicus

<220>
<223> Elk protein tyrosine kinase

<400> 10
gaagctgtcc gggagtttgc caaggagatt gatgtgtctt ttgtgaaaat tgaagaggtc 60
atcggagcag gggagtttgg agaagtgtac aagggccgat tgaagctgcc aggcaagagg 120
gaaatctatg tggccatcaa gaccctgaaa gctgggtact cagagaaaca gcgtcgggat 180
tttctgagcg aggcgagcat catgggccag tttgaccatc ccaacatcat tcgcctagag 240
ggtgttgtca ccaagagccg acctgtcatg atcattacgg agttcatgga gaatggcgct 300
ttagactctt tcctccggca aaatgacgga cagttcaccg tgatccagct tgtggggatg 360
ctgaggggca tcgctgctgg catgaagtac ctatctgaga tgaattatgt gcaccgggac 420
ctggctgcta ggaacattct ggtgaacagc aacctggtgt gcaaagtgtc tgactttggc 480
ctctctcgct acctccagga tgacacctca gaccccacct acaccagctc cttggggggg 540
aagatacctg ttagatggac agctccagag gccatcgcct accgcaagtt tacgtcagcc 600
agcgatgtct ggagctacgg gattgtcatg tgggaagtaa tgtcatttgg agagagacct 660
tactgggata tgtccaatca agatgtcatc aatgccattg agcaggacta ccggctgccc 720
cctcctatgg actgcccagc tgccctgcac cagctcatgc tggactgttg gcagaaggat 780
cgaaatagcc ggccccgttt tgcagagatc gtcaacaccc tggacaagat gatccggaac 840
ccagctagtc tcaagactgt ggcaaccatc accgctgtgc cttcccaacc cctgcttgac 900
cgctctatcc cagacttcac ggcctttacc accgtggatg actggctaag tgccatcaaa 960
atggtccagt acagggacag cttcctcacc gctggcttca cctccctcca gctggtcacc 1020
cagatgacat cagaagacct cctgagaata ggggtcacct tggcaggcca tcagaagaag 1080
attcttagca gcatccactc gatgagggtc cagatgaacc agtcaccgtc ggtaatggca 1140
tgagaactcg atgttcctag gggaggagag gaatgaaaag gaccagggtc aaaggggaaa 1200
cagaggttga ccacggtgga atgtgccaga aagatcagct tcttggctga gaaacaactt 1260
cccacctgtg aagaatgaac tggacctgct gcccatgggc aaccgccatt tctcggccat 1320
ggaagtgtgt agaagccgtg ggaaaccaaa cgcgtaatga ttgacgataa agacagagaa 1380
aggtgatgct aaacagaagc gaagctgaaa ccgtatgcgt tgggaaccag ggtgaactca 1440
gttcccatct gagagggact ggagttcccc gtctgcagga agaaaggaag gaggtggagg 1500
gaagagagg 1509

<210> 11
<211> 330
<212> PRT
<213> Rattus norvegicus

<220>
<223> PP-1 alpha

<400> 11
Met Ser Asp Ser Glu Lys Leu Asn Leu Asp Ser Ile Ile Gly Arg Leu
1               5                   10                  15

Leu Glu Val Gln Gly Ser Arg Pro Gly Lys Asn Val Gln Leu Thr Glu
            20                  25                  30

Asn Glu Ile Arg Gly Leu Cys Leu Lys Ser Arg Glu Ile Phe Leu Ser
        35                  40                  45

Gln Pro Ile Leu Leu Glu Leu Glu Ala Pro Leu Lys Ile Cys Gly Asp
    50                  55                  60

Ile His Gly Gln Tyr Tyr Asp Leu Leu Arg Leu Phe Glu Tyr Gly Gly
65                  70                  75                  80

Phe Pro Pro Glu Ser Asn Tyr Leu Phe Leu Gly Asp Tyr Val Asp Arg
                85                  90                  95

Gly Lys Gln Ser Leu Glu Thr Ile Cys Leu Leu Leu Ala Tyr Lys Ile
            100                 105                 110

Lys Tyr Pro Glu Asn Phe Phe Leu Leu Arg Gly Asn His Glu Cys Ala
        115                 120                 125

Ser Ile Asn Arg Ile Tyr Gly Phe Tyr Asp Glu Cys Lys Arg Arg Tyr
    130                 135                 140

Asn Ile Lys Leu Trp Lys Thr Phe Thr Asp Cys Phe Asn Cys Leu Pro
145                 150                 155                 160

Ile Ala Ala Ile Val Asp Glu Lys Ile Phe Cys Cys His Gly Gly Leu
                165                 170                 175

Ser Pro Asp Leu Gln Ser Met Glu Gln Ile Arg Arg Ile Met Arg Pro
            180                 185                 190

Thr Asp Val Pro Asp Gln Gly Leu Leu Cys Asp Leu Leu Trp Ser Asp
        195                 200                 205

Pro Asp Lys Asp Val Gln Gly Trp Gly Glu Asn Asp Arg Gly Val Ser
    210                 215                 220

Phe Thr Phe Gly Ala Glu Val Val Ala Lys Phe Leu His Lys His Asp
225                 230                 235                 240

Leu Asp Leu Ile Cys Arg Ala His Gln Val Val Glu Asp Gly Tyr Glu
                245                 250                 255

Phe Phe Ala Lys Arg Gln Leu Val Thr Leu Phe Ser Ala Pro Asn Tyr
            260                 265                 270

Cys Gly Glu Phe Asp Asn Ala Gly Ala Met Met Ser Val Asp Glu Thr
        275                 280                 285

Leu Met Cys Ser Phe Gln Ile Leu Lys Pro Ala Asp Lys Asn Lys Gly
    290                 295                 300

Lys Tyr Gly Gln Phe Ser Gly Leu Asn Pro Gly Gly Arg Pro Ile Thr
305                 310                 315                 320

Pro Pro Arg Asn Ser Ala Lys Ala Lys Lys
                325                 330


<210> 12
<211> 1392
<212> DNA
<213> Rattus norvegicus

<220>
<223> PP-1 alpha

<400> 12
aggaggctgc aagagggcgg gaggcaggag agggcccgga gctggtgggc cggagcggcg 60
gcgccgccat gtccgacagc gagaagctca acctggattc catcatcggg cgcctgctgg 120

aagtgcaggg ctcacggcct ggaaagaatg tgcagctgac agagaacgag atccgtggtc 180
tttgcctcaa atcccgggag attttcctga gccagcctat tcttctggag cttgaggcgc 240
ctctcaagat ctgtggtgac atccatggcc agtactatga ccttctacgg ctgttcgagt 300
atggtggctt ccctccagag agcaactacc tcttcttggg ggactatgtg gatcggggaa 360
agcagtcttt ggagaccatc tgcttgttgc tggcctataa gatcaaatac ccggagaatt 420
tctttctact acgtgggaac catgagtgtg ccagcatcaa ccgcatctat ggcttctatg 480
atgaatgcaa gagacgatac aacatcaaac tgtggaagac tttcaccgac tgcttcaact 540
gcctgcccat tgcagccatt gtagatgaga agatcttctg ctgccacgga ggcctgtctc 600
cagacttgca atccatggag cagattagac gtattatgcg gcccacggac gtgcctgacc 660
agggcctgtt gtgtgatctg ctgtggtctg accctgacaa ggatgttcaa ggctggggcg 720
agaatgaccg tggagtctcc tttacctttg gggccgaggt tgtagccaag ttcctgcaca 780
agcatgattt ggacctcatc tgcagagcac atcaggttgt agaagatggc tatgagttct 840
ttgccaagag gcagctggtg acactcttct cagctcccaa ctactgtggc gagtttgaca 900
acgctggcgc catgatgagt gtggacgaga cactcatgtg ttccttccag atcctcaagc 960
ccgctgataa gaataagggg aagtatgggc agttcagtgg cctgaacccc ggaggccgtc 1020
ccatcactcc accccgcaat tctgccaaag ccaagaaata gcctccatgt gctgccctct 1080
gccccagatg acggattatt tgtacagaaa tcatgctgcc atgggtcaca ctggcctctc 1140
aggcccaccc atcatgggga acacagcgtt aagtgtcttt cctttatttt ttaaagaatc 1200
aatagcagca tctaattccc cagggctccc tcccaccagc acctgtggtg gctgcaagtg 1260
gaatcctggg gccaaggctg cagctcaggg caatggcaga ccagattgtg ggtctccagc 1320
cttgcatggc tggcagccag atcctggggc aacccatctg gtctcttgaa taaaggtcaa 1380
agctggattc tc 1392

<210> 13
<211> 369
<212> PRT
<213> Rattus norvegicus

<220>
<223> Striatum enriched protein-tyrosine-phosphatase

<400> 13
Met Glu Glu Lys Val Glu Asp Asp Phe Leu Asp Leu Asp Ala Val Pro
1               5                   10                  15

Glu Thr Pro Val Phe Asp Cys Val Met Asp Ile Lys Pro Glu Ala Asp
            20                  25                  30

Pro Thr Ser Leu Thr Val Lys Ser Met Gly Leu Gln Glu Arg Arg Gly
        35                  40                  45

Ser Asn Val Ser Leu Thr Leu Asp Met Cys Thr Pro Gly Cys Asn Glu
    50                  55                  60

Glu Gly Phe Gly Tyr Leu Val Ser Pro Arg Glu Glu Ser Ala His Glu
65                  70                  75                  80

Tyr Leu Leu Ser Ala Ser Arg Val Leu Arg Ala Glu Glu Leu His Glu
                85                  90                  95

Lys Ala Leu Asp Pro Phe Leu Leu Gln Ala Glu Phe Phe Glu Ile Pro
            100                 105                 110

Met Asn Phe Val Asp Pro Lys Glu Tyr Asp Ile Pro Gly Leu Val Arg
        115                 120                 125

Lys Asn Arg Tyr Lys Thr Ile Leu Pro Asn Pro His Ser Arg Val Arg
    130                 135                 140

Leu Thr Ser Pro Asp Pro Glu Asp Pro Leu Ser Ser Tyr Ile Asn Ala

145                 150                 155                 160

Asn Tyr Ile Arg Gly Tyr Asn Gly Glu Glu Lys Val Tyr Ile Ala Thr
                165                 170                 175

Gln Gly Pro Ile Val Ser Thr Val Val Asp Phe Trp Arg Met Val Trp
            180                 185                 190

Gln Glu Arg Thr Pro Ile Ile Val Met Ile Thr Asn Ile Glu Glu Met
        195                 200                 205

Asn Glu Lys Cys Thr Glu Tyr Trp Pro Glu Glu Gln Val Val His Asp
    210                 215                 220

Gly Val Glu Ile Thr Val Gln Lys Val Ile His Thr Glu Asp Tyr Arg
225                 230                 235                 240

Leu Arg Leu Ile Ser Leu Arg Arg Gly Thr Glu Glu Arg Gly Leu Lys
                245                 250                 255

His Tyr Trp Phe Thr Ser Trp Pro Asp Gln Lys Thr Pro Asp Arg Ala
            260                 265                 270

Pro Pro Leu Leu His Leu Val Arg Glu Val Glu Glu Ala Ala Gln Gln
        275                 280                 285

Glu Gly Pro His Cys Ser Pro Ile Ile Val His Cys Ser Ala Gly Ile
    290                 295                 300

Gly Arg Thr Gly Cys Phe Ile Ala Thr Ser Ile Cys Cys Gln Gln Leu
305                 310                 315                 320

Arg Arg Glu Gly Val Val Asp Ile Leu Lys Thr Thr Cys Gln Leu Arg
                325                 330                 335

Gln Asp Arg Gly Gly Met Ile Gln Thr Cys Glu Gln Tyr Gln Phe Val
            340                 345                 350

His His Ala Met Ser Leu Tyr Glu Lys Gln Leu Ser Leu Gln Ser Ser
        355                 360                 365

Glu

<210> 14
<211> 2815
<212> DNA
<213> Rattus norvegicus

<220>
<223> Striatum enriched protein-tyrosine-phosphatase

<400> 14
ggggggatgt tgagggatga gggcctttct ggtggcctcc gttacttcaa gtcttcatga 60
ctggaacgaa gggaagactg gagttctgaa ctacagtctc aggaattaat ttcctcgtat 120
cctgcctacc ctaacccata aataaaaaga caggtatcct gggtctctct gaagacctcc 180
tgtctaagga ggcctttctg gaagacttcc tttctatgtg tacctgtggg gtcttttgtg 240
gggagggatg tctcagtcgc ctgtgattag cttttctcct gaagactgct gaggcctcct 300
ctgggtacac cacctctccc tccactgccc agtctgagag gctcaggagc ttcctggggg 360
ctgaaggtga gctctgcagg gcaatatagg ggcaaagggg tgcagctgtc cactcagtct 420

gcgcccccga ctgccccagc ttgcctgtgg ggtgctctgg ctcagtggcc atggccactc 480
ctggctgcag accaccacag acctcatctt ctcctcgctc accgtgttga accacctggg 540
acctgcagcc tggctaggtt ctgggacctg ggggacacca agtctgctgt tagtctctct 600
gactgtgagc ctgggcatca tcaccaccct ggtgtggcac cttctcaaga cacccccaga 660
gccacctgtc ccactgcccc cagaggacag acgacaatca gtgagccggc agccctcctt 720
cacttactca gagtggatgg aggagaaggt agaggatgac tttctggacc tggacgcagt 780
gccggagaca cccgtgtttg actgtgtgat ggacatcaag cctgaggctg atcccacctc 840
gttgactgtc aagtccatgg gtctgcagga gaggaggggc tccaatgtct ccttgaccct 900
ggacatgtgt actcctggct gcaatgagga gggctttggc tacctggtgt ccccacgaga 960
ggagtcagcc catgaatacc tgctcagtgc ctcccgtgtc ctccgggcag aggagctgca 1020
cgaaaaggcc ctggaccctt tcttgctgca ggcggaattc tttgaaatcc ccatgaactt 1080
tgtggatcca aaagaatatg acatcccagg gctggtacgg aagaatcggt acaaaaccat 1140
ccttcccaat cctcacagca gggtacgtct gacgtcacca gaccctgaag atcctctgag 1200
ttcctacatc aatgccaact atatccgggg ctacaatggg gaggagaagg tgtacatcgc 1260
cacccaggga cccatcgtca gcactgtggt cgacttctgg cgcatggtgt ggcaggagcg 1320
cacacccatc atcgtcatga tcaccaacat cgaggagatg aacgagaagt gcacggaata 1380
ttggccagaa gagcaggtcg tccacgatgg cgtagagatc actgtgcaga aagtcatcca 1440
cacagaggac taccggctgc gacttatctc cctcaggaga gggactgaag agagaggctt 1500
gaagcattac tggttcacat cctggcctga ccagaagacc cccgaccggg cacccccact 1560
cctgcacctg gtacgggagg tggaggaggc agcccagcaa gagggacccc actgttcccc 1620
gatcattgtt cactgcagtg cagggattgg gagaaccggg tgcttcatcg ccaccagcat 1680
ctgctgccag cagctgcgac gtgagggcgt ggtagacatc ctaaagacca cgtgccagct 1740
ccgtcaggac aggggcggca tgatccaaac atgcgaacag tatcagtttg tgcaccacgc 1800
catgagcctc tatgagaagc agctatccct ccagtcctca gagtgaccac actcttcctc 1860
tgagatccac tgggcactgc cgagtctgag gctggggctt caccccaact acccaaggtc 1920
ttaccttggg tcctgggcct gttccctggc tctccccttc agtctgcccc catctgttgt 1980
cctggcctgg tccctacccc aagcctagct cttctactgt acatactgca gagttcggga 2040
gggcagggga ggaatgtgcc aggccaggcc tggggccctg ggcctgaccc acactatata 2100
gacctggggc ctcagtttta accatgattc cacagctact tgatccaaaa tgttttcatg 2160
ccacacccca agtgtcctac cacaacatgt tctgtctgtc catccatcac agctatctgt 2220
accaaacact gtgtcccctc agccaggaaa ggagtactga tctgagctct cacccctcag 2280
ccctcaccca catcgccatt cccagacacc caccctgatc cccaggcaga gatgtctacc 2340
aatctctggc ctcacaggat gtggatggtg gagaccaggc agaggagaat gtggtggttg 2400
accgagatcc cagctgagaa ggttcccagg caagggtggg catctctggc ctatgagaca 2460
ctggagagaa agaggacaga tatagtggga aaccctaagg atctgagccc tgagggggcc 2520
ctccaagagt agggtgggga cagctccaga cctgcaaggc atctttctgt gtcactatgg 2580
gaaaccccaa atgtctcctc gctacatgtt gctccgtgtg ttcccttgtc catgtttgtg 2640
ttgagagccc atgagcaggg catgcatgac tctttggcaa catgtgttat cttggagcca 2700
cgtgttttta tcgctgactt taaatattta ttccacggca tacagagaca tttggtgtct 2760
ttttataatc tgctcgtggt cattgaatag agcaataaac agagcatttt gagca 2815

<210> 15
<211> 1013
<212> PRT
<213> Rattus norvegicus

<220>
<223> Na+,K+ - ATPase alpha subunit

<400> 15
Met Gly Asp Lys Lys Asp Asp Lys Ser Ser Pro Lys Lys Ser Lys Ala
1               5                   10                  15

Lys Glu Arg Arg Asp Leu Asp Asp Leu Lys Lys Glu Val Ala Met Thr
            20                  25                  30

Glu His Lys Met Ser Val Glu Glu Val Cys Arg Lys Tyr Asn Thr Asp
        35                  40                  45

Cys Val Gln Gly Leu Thr His Ser Lys Ala Gln Glu Ile Leu Ala Arg

50                  55                  60

Asp Gly Pro Asn Ala Leu Thr Pro Pro Pro Thr Thr Pro Glu Trp Val
65                  70                  75                  80

Lys Phe Cys Arg Gln Leu Phe Gly Gly Phe Ser Ile Leu Leu Trp Ile
                85                  90                  95

Gly Ala Ile Leu Cys Phe Leu Ala Tyr Gly Ile Gln Ala Gly Thr Glu
            100                 105                 110

Asp Asp Pro Ser Gly Asp Asn Leu Tyr Leu Gly Ile Val Leu Ala Ala
        115                 120                 125

Val Val Ile Ile Thr Gly Cys Phe Ser Tyr Tyr Gln Glu Ala Lys Ser
    130                 135                 140

Ser Lys Ile Met Glu Ser Phe Lys Asn Met Val Pro Gln Gln Ala Leu
145                 150                 155                 160

Val Ile Arg Glu Gly Glu Lys Met Gln Val Asn Ala Glu Glu Val Val
                165                 170                 175

Val Gly Asp Leu Val Glu Ile Lys Gly Gly Asp Arg Val Pro Ala Asp
            180                 185                 190

Leu Arg Ile Ile Ser Ala His Gly Cys Lys Val Asp Asn Ser Ser Leu
        195                 200                 205

Thr Gly Glu Ser Glu Pro Gln Thr Arg Ser Pro Asp Cys Thr His Asp
    210                 215                 220

Asn Pro Leu Glu Thr Arg Asn Ile Thr Phe Phe Ser Thr Asn Cys Val
225                 230                 235                 240

Glu Gly Thr Ala Arg Gly Val Val Val Ala Thr Gly Asp Arg Thr Val
                245                 250                 255

Met Gly Arg Ile Ala Thr Leu Ala Ser Gly Leu Glu Val Gly Lys Thr
            260                 265                 270

Pro Ile Ala Ile Glu Ile Glu His Phe Ile Gln Leu Ile Thr Gly Val
        275                 280                 285

Ala Val Phe Leu Gly Val Ser Phe Phe Ile Leu Ser Leu Ile Leu Gly
    290                 295                 300

Tyr Thr Trp Leu Glu Ala Val Ile Phe Leu Ile Gly Ile Ile Val Ala
305                 310                 315                 320

Asn Val Pro Glu Gly Leu Leu Ala Thr Val Thr Val Cys Leu Thr Leu
                325                 330                 335

Thr Ala Lys Arg Met Ala Arg Lys Asn Cys Leu Val Lys Asn Leu Glu
            340                 345                 350

Ala Val Glu Thr Leu Gly Ser Thr Ser Thr Ile Cys Ser Asp Lys Thr
        355                 360                 365

Gly Thr Leu Thr Gln Asn Arg Met Thr Val Ala His Met Trp Phe Asp
    370                 375                 380

Asn Gln Ile His Glu Ala Asp Thr Thr Glu Asp Gln Ser Gly Thr Ser
385                 390                 395                 400

Phe Asp Lys Ser Ser His Thr Trp Val Ala Leu Ser His Ile Ala Gly
                405                 410                 415

Leu Cys Asn Arg Ala Val Phe Lys Gly Gly Gln Asp Asn Ile Pro Val
            420                 425                 430

Leu Lys Arg Asp Val Ala Gly Asp Ala Ser Glu Ser Ala Leu Leu Lys
        435                 440                 445

Cys Ile Glu Leu Ser Ser Gly Ser Val Lys Leu Met Arg Glu Arg Asn
    450                 455                 460

Lys Lys Val Ala Glu Ile Pro Phe Asn Ser Thr Asn Lys Tyr Gln Leu
465                 470                 475                 480

Ser Ile His Glu Thr Glu Asp Pro Asn Asp Asn Arg Tyr Leu Leu Val
                485                 490                 495

Met Lys Gly Ala Pro Glu Arg Ile Leu Asp Arg Cys Ala Thr Ile Leu
            500                 505                 510

Leu Gln Gly Lys Glu Gln Pro Leu Asp Glu Glu Met Lys Glu Ala Phe
        515                 520                 525

Gln Asn Ala Tyr Leu Glu Leu Gly Gly Leu Gly Glu Arg Val Leu Gly
    530                 535                 540

Phe Cys His Tyr Tyr Leu Pro Glu Glu Gln Phe Pro Lys Gly Phe Ala
545                 550                 555                 560

Phe Asp Cys Asp Asp Val Asn Phe Thr Thr Asp Asn Leu Cys Phe Val
                565                 570                 575

Gly Leu Met Ser Met Ile Asp Pro Pro Arg Ala Ala Val Pro Asp Ala
            580                 585                 590

Val Gly Lys Cys Arg Ser Ala Gly Ile Lys Val Ile Met Val Thr Gly
        595                 600                 605

Asp His Pro Ile Thr Ala Lys Ala Ile Ala Lys Gly Val Gly Ile Ile
    610                 615                 620

Ser Glu Gly Asn Glu Thr Val Glu Asp Ile Ala Ala Arg Leu Asn Ile
625                 630                 635                 640

Pro Val Ser Gln Val Asn Pro Arg Asp Ala Lys Ala Cys Val Ile His
                645                 650                 655

Gly Thr Asp Leu Lys Asp Phe Thr Ser Glu Gln Ile Asp Glu Ile Leu
            660                 665                 670

Gln Asn His Thr Glu Ile Val Phe Ala Arg Thr Ser Pro Gln Gln Lys
        675                 680                 685

Leu Ile Ile Val Glu Gly Cys Gln Arg Gln Gly Ala Ile Val Ala Val
    690                 695                 700

Thr Gly Asp Gly Val Asn Asp Ser Pro Ala Leu Lys Lys Ala Asp Ile
705                 710                 715                 720

Gly Val Ala Met Gly Ile Ala Gly Ser Asp Val Ser Lys Gln Ala Ala
                725                 730                 735

Asp Met Ile Leu Leu Asp Asp Asn Phe Ala Ser Ile Val Thr Gly Val
            740                 745                 750

Glu Glu Gly Arg Leu Ile Phe Asp Asn Leu Lys Lys Ser Ile Ala Tyr
        755                 760                 765

Thr Leu Thr Ser Asn Ile Pro Glu Ile Thr Pro Phe Leu Leu Phe Ile
    770                 775                 780

Met Ala Asn Ile Pro Leu Pro Leu Gly Thr Ile Thr Ile Leu Cys Ile
785                 790                 795                 800

Asp Leu Gly Thr Asp Met Val Pro Ala Ile Ser Leu Ala Tyr Glu Ala
                805                 810                 815

Ala Glu Ser Asp Ile Met Lys Arg Gln Pro Arg Asn Pro Arg Thr Asp
            820                 825                 830

Lys Leu Val Asn Glu Arg Leu Ile Ser Met Ala Tyr Gly Gln Ile Gly
        835                 840                 845

Met Ile Gln Ala Leu Gly Gly Phe Phe Ser Tyr Phe Val Ile Leu Ala
    850                 855                 860

Glu Asn Gly Phe Leu Pro Gly Asn Leu Val Gly Ile Arg Leu Asn Trp
865                 870                 875                 880

Asp Asp Arg Thr Val Asn Asp Leu Glu Asp Ser Tyr Gly Gln Gln Trp
                885                 890                 895

Thr Tyr Glu Gln Arg Lys Val Val Glu Phe Thr Cys His Thr Ala Phe
            900                 905                 910

Phe Val Ser Ile Val Val Val Gln Trp Ala Asp Leu Ile Ile Cys Lys
        915                 920                 925

Thr Arg Arg Asn Ser Val Phe Gln Gln Gly Met Lys Asn Lys Ile Leu
    930                 935                 940

Ile Phe Gly Leu Phe Glu Glu Thr Ala Leu Ala Ala Phe Leu Ser Tyr
945                 950                 955                 960

Cys Pro Gly Met Asp Val Ala Leu Arg Met Tyr Pro Leu Lys Pro Ser
                965                 970                 975

Trp Trp Phe Cys Ala Phe Pro Tyr Ser Phe Leu Ile Phe Val Tyr Asp
            980                 985                 990

Glu Ile Arg Lys Leu Ile Leu Arg Arg Asn Pro Gly Gly Trp Val Glu
        995                 1000                1005

Lys Glu Thr Tyr Tyr
    1010

<210> 16
<211> 3557
<212> DNA
<213> Rattus norvegicus

<220>
<223> Na+,K+ - ATPase alpha subunit

<400> 16
tctgtgccgt gggacccacg accgacagac gcacgctccc aacgcggcgg gcgtgcagag 60
gcccccagcc cgagcccgcg ctgagcccgt cctgcgccac gctcacagcc tgtccgccgt 120
cttcccgcgg agccgccaag atgggggaca aaaaagatga caagagctcg cccaagaaga 180
gcaaggccaa agagcgccgg gacctggatg acctcaagaa ggaagtggct atgacagagc 240
acaagatgtc agtagaagag gtctgccgga aatacaatac cgactgcgtg cagggtctga 300
cacacagtaa agcccaggag atcctagccc gggatgggcc taacgccctc acaccaccgc 360
ccaccacccc agaatgggtc aagttctgcc gacagctatt tggtggcttc tccatcctgc 420
tgtggatcgg ggcgatcctt tgcttcctgg cctatggcat ccaggcaggg actgaggatg 480
acccttccgg tgacaatctg tacctgggca tagtgctggc cgctgtggtg atcatcaccg 540
gctgcttctc ctactaccaa gaagccaaga gctctaagat tatggagtcc ttcaagaaca 600
tggttcccca gcaagccctt gtgatccggg aaggtgaaaa gatgcaggtg aatgcggagg 660
aggtggtggt cggggacctg gtggagatca agggtggcga ccgggtgccc gctgacctgc 720
gcatcatctc ggcccatggc tgcaaggtgg acaactcttc cctgactggc gaatctgagc 780
ctcagacccg ctccccggac tgcacacacg acaacccact ggagacccgg aacatcacct 840
tcttttccac caactgtgtg gaaggcaccg ctcgtggtgt ggtggtagcc acaggtgacc 900
gcaccgtcat gggccgcatt gccaccctgg cctcaggcct ggaggtgggc aagacgccca 960
tcgccattga gatcgagcac ttcatccagc tcatcactgg cgtggccgtg ttcctgggcg 1020
tctccttctt catcctctcc ctcattctgg gctacacctg gctggaggct gtcatcttcc 1080
tcatcggtat cattgtggcc aatgtcccag agggcctgct ggctactgtc acggtgtgtc 1140
tgacgctgac cgccaagcgc atggctcgga agaactgtct ggtaaagaac ctggaggcgg 1200
tggagacgct aggctccaca tccaccatct gctccgacaa gaccggcacc ctcacccaga 1260
accgcatgac cgtcgcccac atgtggtttg acaaccagat ccacgaggcc gacactactg 1320
aggatcagtc agggacctct ttcgacaaga gctcacacac ctgggtggcc ctgtcccaca 1380
tcgccggtct ctgcaaccgg gctgtcttca agggcgggca ggataacatc cctgtactca 1440
agagggacgt ggcgggtgat gcctcagagt ccgccctgct taagtgcatc gagctgtcct 1500
cgggttccgt aaagctgatg cgcgaacgaa acaagaaagt ggccgagatt cccttcaact 1560
ccactaacaa ataccagcta tccatccatg agactgagga ccccaatgac aaccgatacc 1620
tgttagtgat gaagggcgcc cctgaacgca ttctggaccg ctgtgcgacc atcctcctgc 1680
agggcaagga gcagcctctg gatgaggaga tgaaggaggc cttccagaac gcctacctgg 1740
agcttggtgg cctgggcgag cgtgtgctgg gtttctgcca ttactacctg ccggaggaac 1800
agttccccaa gggctttgcc tttgactgtg atgacgtgaa cttcaccaca gacaaccttt 1860
gcttcgtggg tctcatgtcc atgatcgacc ctccccgggc agctgtccct gatgctgtgg 1920
gcaaatgccg cagtgcaggc atcaaggtca tcatggtcac cggcgatcac cccatcactg 1980
cgaaggccat cgccaaaggt gtaggcatca tctccgaggg taacgagact gtggaggaca 2040
tcgctgcccg gctcaacatc cctgtcagcc aggtcaaccc cagggatgcc aaagcctgtg 2100
tgattcatgg caccgacctc aaggacttca cctctgagca gattgacgag atcctacaga 2160
accacactga gatcgtcttt gcccgaacct cccctcagca gaagctcatc atcgtggagg 2220
gctgtcagag acagggagca attgtggctg tgactggcga tggtgtgaat gactcccctg 2280
ctctgaagaa ggctgatatt ggggtggcca tgggcattgc tggctctgat gtctctaagc 2340
aggctgccga catgattctg ctggatgaca attttgcttc cattgtcact ggtgtggagg 2400
aaggccgcct gatctttgac aacctgaaga aatccatcgc ctacactctg accagcaaca 2460
tccctgagat cacacccttc cttctcttca tcatggccaa catcccactg ccccttggca 2520
ccatcaccat cctctgcatt gacctgggca ccgacatggt ccctgcaatc tccttggcct 2580
acgaggctgc agagagtgac atcatgaaga ggcagcccag gaacccacgc acagacaaac 2640
tggtcaacga aaggctcatc agcatggcct atgggcagat cgggatgatc caggccctcg 2700
gtggtttctt ctcctacttt gtcatcctgg cagaaaatgg tttcttgccc ggtaacctgg 2760
tgggcatccg gctcaactgg gatgaccgca ctgtcaacga cctggaagac agctacgggc 2820
agcagtggac ttatgagcag aggaaggtgg ttgagttcac gtgccacaca gccttcttcg 2880
tgagcatagt ggtggtccag tgggctgacc tgatcatttg caagaccagg aggaattccg 2940
tcttccagca gggcatgaag aataagatct tgatcttcgg actgtttgag gaaacggccc 3000
tcgctgcctt cctgtcctac tgcccaggca tggatgtggc ccttcgcatg tacccgctca 3060
agcccagctg gtggttctgt gccttcccct acagtttcct catcttcgtc tatgatgaga 3120

ttcgcaaact catcctgcgc aggaaccccg ggggttgggt ggagaaagag acctactatt 3180
gacctcagcc accacgtcgc ccatgtcttc cctgcccccc agcccaggac agccctctcc 3240
ggtcccccat tttgtactct gggggagggg gccttctctc cccgatggcc cccctctctc 3300
cttgtggccc agtctcctct cccacacctc ggttacctct cactttccct catttctccc 3360
tctggctggc ttctctcccc ccacagcccc tgcctctctt ctccttccct tttctgtgtc 3420
tctccccctc tctcccttgc agccctgccc ctcattttct ccctgggctc ctttttacac 3480
ccctgggccc tgtctcctgg ctaatgccat ctccggttct aaatctgaac aattatcaaa 3540
tatatcagtg gggagag                                                3557

<210> 17
<211> 295
<212> PRT
<213> Rattus norvegicus

<220>
<223> Voltage-dependent anion channel 2

<400> 17
Met Ala Glu Cys Cys Val Pro Val Cys Gln Arg Pro Ile Cys Ile Pro
1               5                   10                  15

Pro Pro Tyr Ala Asp Leu Gly Lys Ala Ala Arg Asp Ile Phe Asn Lys
            20                  25                  30

Gly Phe Gly Phe Gly Leu Val Lys Leu Asp Val Lys Thr Lys Ser Cys
        35                  40                  45

Ser Gly Val Glu Phe Ser Thr Ser Gly Ser Ser Asn Thr Asp Thr Gly
    50                  55                  60

Lys Val Ser Gly Thr Leu Glu Thr Lys Tyr Lys Trp Cys Glu Tyr Gly
65                  70                  75                  80

Leu Thr Phe Thr Glu Lys Trp Asn Thr Asp Asn Thr Leu Gly Thr Glu
                85                  90                  95

Ile Ala Ile Glu Asp Gln Ile Cys Gln Gly Leu Lys Leu Thr Phe Asp
            100                 105                 110

Thr Thr Phe Ser Pro Asn Thr Gly Lys Lys Ser Gly Lys Ile Lys Ser
        115                 120                 125

Ala Tyr Lys Arg Glu Cys Ile Asn Leu Gly Cys Asp Val Asp Phe Asp
    130                 135                 140

Phe Ala Gly Pro Ala Ile His Gly Ser Ala Val Phe Gly Tyr Glu Gly
145                 150                 155                 160

Trp Leu Ala Gly Tyr Gln Met Thr Phe Asp Ser Ala Lys Ser Lys Leu
                165                 170                 175

Thr Arg Ser Asn Phe Ala Val Gly Tyr Arg Thr Gly Asp Phe Gln Leu
            180                 185                 190

His Thr Asn Val Asn Asn Gly Thr Glu Phe Gly Gly Ser Ile Tyr Gln
        195                 200                 205

Lys Val Cys Glu Asp Phe Asp Thr Ser Val Asn Leu Ala Trp Thr Ser
    210                 215                 220

Gly Thr Asn Cys Thr Arg Phe Gly Ile Ala Ala Lys Tyr Gln Leu Asp
225                 230                 235                 240

Pro Thr Ala Ser Ile Ser Ala Lys Val Asn Asn Ser Ser Leu Ile Gly
                245                 250                 255

Val Gly Tyr Thr Gln Thr Leu Arg Pro Gly Val Lys Leu Thr Leu Ser
            260                 265                 270

Ala Leu Val Asp Gly Lys Ser Phe Asn Ala Gly Gly His Lys Leu Gly
        275                 280                 285

Leu Ala Leu Glu Leu Glu Ala
    290                 295


<210> 18
<211> 870
<212> DNA
<213> Rattus norvegicus

<220>
<223> Voltage-dependent anion channel 2

<400> 18
cttcgccatg gctgaatgtt gtgtaccggt atgccaacgg ccaatttgta tccctccacc 60
ctatgctgac cttggcaaag ctgccagaga tattttcaac aaaggatttg gttttgggtt 120
ggtaaagctg gatgtgaaaa cgaagtcatg cagtggtgtg gaattttcaa catctggctc 180
atctaataca gacactggta aagtcagtgg gaccttggag accaagtaca aatggtgtga 240
gtatggtctg actttcacag agaaatggaa cactgacaac actctgggga cggagattgc 300
aattgaagac cagatttgtc aaggtttgaa actgaccttt gacaccacgt tttcaccaaa 360
cacaggaaag aaaagtggta aaatcaagtc tgcttacaag agggaatgta taaaccttgg 420
ctgtgatgtt gattttgatt ttgctggacc tgccatccat gggtcagccg tctttggtta 480
cgagggctgg cttgctgggt accagatgac ctttgacagt gccaagtcaa agctgacaag 540
gagtaacttc gcagttggct acaggactgg ggacttccag ctacacacaa atgtaaataa 600
tgggacagaa tttggaggat caatttatca gaaagtatgt gaagattttg acacttcagt 660
aaaccttgct tggacatcag gtaccaactg cactcgtttt ggcattgcag ctaaatacca 720
gttggacccc actgcttcta tttctgcaaa ggtcaacaac tctagtttaa ttggagtggg 780
ctatactcag actctgaggc ctggtgtgaa gcttacactg tctgctctgg tagacgggaa 840
gagctttaat gctggaggcc acaaacttgg                                  870


<210> 19
<211> 390
<212> PRT
<213> Rattus norvegicus

<220>
<223> Neonatal glycine receptor

<400> 19
Met Asn Arg Gln Leu Val Asn Ile Leu Thr Ala Leu Phe Ala Phe Phe
1               5                   10                  15

Leu Gly Thr Asn His Phe Arg Glu Ala Phe Cys Lys Asp His Asp Ser
            20                  25                  30

Arg Ser Gly Lys His Pro Ser Gln Thr Leu Ser Pro Ser Asp Phe Leu
        35                  40                  45

Asp Lys Leu Met Gly Arg Thr Ser Gly Tyr Asp Ala Arg Ile Arg Pro

50                  55                  60

Asn Phe Lys Gly Pro Pro Val Asn Val Thr Cys Asn Ile Phe Ile Asn
65                  70                  75                  80

Ser Phe Gly Ser Val Thr Glu Thr Thr Met Asp Tyr Arg Val Asn Ile
                85                  90                  95

Phe Leu Arg Gln Gln Trp Asn Asp Ser Arg Leu Ala Tyr Ser Glu Tyr
            100                 105                 110

Pro Asp Asp Ser Leu Asp Leu Asp Pro Ser Met Leu Asp Ser Ile Trp
        115                 120                 125

Lys Pro Asp Leu Phe Phe Ala Asn Glu Lys Gly Ala Asn Phe His Asp
    130                 135                 140

Val Thr Thr Asp Asn Lys Leu Leu Arg Ile Ser Lys Asn Gly Lys Val
145                 150                 155                 160

Leu Tyr Ser Ile Arg Leu Thr Leu Thr Leu Ser Cys Pro Met Asp Leu
                165                 170                 175

Lys Asn Phe Pro Met Asp Val Gln Thr Cys Thr Met Gln Leu Glu Ser
            180                 185                 190

Phe Gly Tyr Thr Met Asn Asp Leu Ile Phe Glu Trp Leu Ser Asp Gly
        195                 200                 205

Pro Val Gln Val Ala Glu Gly Leu Thr Leu Pro Gln Phe Ile Leu Lys
    210                 215                 220

Glu Glu Lys Glu Leu Gly Tyr Cys Thr Lys His Tyr Asn Thr Gly Lys
225                 230                 235                 240

Phe Thr Cys Ile Glu Val Lys Phe His Leu Glu Arg Gln Met Gly Tyr
                245                 250                 255

Tyr Leu Ile Gln Met Tyr Ile Pro Ser Leu Leu Ile Val Ile Leu Ser
            260                 265                 270

Trp Val Ser Phe Trp Ile Asn Met Asp Ala Ala Pro Ala Arg Val Ala
        275                 280                 285

Leu Gly Ile Thr Thr Val Leu Thr Met Thr Thr Gln Ser Ser Gly Ser
    290                 295                 300

Arg Ala Ser Leu Pro Lys Val Ser Tyr Val Lys Ala Ile Asp Ile Trp
305                 310                 315                 320

Met Ala Val Cys Leu Leu Phe Val Phe Ala Ala Leu Leu Glu Tyr Ala
                325                 330                 335

Ala Val Asn Phe Val Ser Arg Gln His Lys Glu Phe Leu Arg Leu Arg
            340                 345                 350

Arg Arg Gln Lys Arg Gln Asn Lys Glu Glu Asp Val Thr Arg Glu Ser
        355                 360                 365

Arg Phe Asn Phe Ser Gly Tyr Gly Met Gly His Cys Leu Gln Val Lys
    370                 375                 380

Asp Gly Thr Ala Val Lys
385                 390

<210> 20
<211> 3865
<212> DNA
<213> Rattus norvegicus

<220>
<223> Neonatal glycine receptor

<400> 20
ttttttttca caccaagcag agtttattga tgccgtgggc ggggcccacg tacacagcag 60
gtggcgcacg cagggcagat actcactggc tggactgcag gggtgttagc agcggcaaaa 120
gcagatgacc tgtggggagc accctttggg gggtgccctc ctcctagcag cattgggggc 180
tttgggtgag catcaagaac cacctagtca ccctaaggtt ctcttggaag cccctccccc 240
tccttcccga gctccttgtg agttgggggc taggatccct tcctgtgatc aaaggtggga 300
tgatcccttg agattcacta taaaaattaa aattcattta taaatcacaa gagaagggca 360
ggagggaaca ccggggaaac ctgacttcat cggttacctt gagcctactc ccctccctca 420
ggttctaact ccaaaagaga gagcaaagag ctaggtctac aggcacagaa aggggcgaga 480
gacagaggaa aggcagtcgg aagaacggga cgagagagga aaaagatgca tcggaagaaa 540
agtatggctg ctgaggctcc tctccccaac agctcccgaa agccatgacc tggggataaa 600
ggagaaaagt tcccagccca gagactccta caaggtaagg ttccaaatgt ccccgatccg 660
gtccttgctc aagccatggc gggccttgag atactcagag ctgtgtggat gggagctgag 720
ggaggcgagt gctactccag gtagatatct tctgtggggc aggcgaatgc caggtgctcc 780
tggtagtact ccaggaaggc ccggcccacg ctccttgcca ctggcctcat agactcctgg 840
gacacaaaga cgtggcaggc aaagcggctt agcagcgggt gtttggtaat gaagccaaaa 900
tagcagctgt ttcggggatg acagccacag aaggagatgt tcttcatctg gaaaaagtgg 960
ctgcaacgcc ctggtttgac ctgaccatga tgcccaagac tggcactttt tgtttttctt 1020
cagcaaactg tacaacccca aatctctttt tgattttcaa ggaaactaga ttcctgccag 1080
aatttgagtc tggacaataa atagacactt tgtcctagtc ttctttctgg aatcatttcg 1140
ggatattttc cacaagcatc acggaaacag gaatgaaccg gcagctagtg aacattttga 1200
cagccttgtt tgcatttttc ttagggacaa accacttcag ggaagcattc tgcaaagacc 1260
atgactccag gtctggaaaa catccctcgc agaccctgtc tccttcagat ttcttggata 1320
aactaatggg aaggacatca ggatatgatg caagaatcag gccaaatttt aaaggtcctc 1380
cagtaaacgt tacttgcaat atttttatca acagttttgg atcggtcaca gaaaccacca 1440
tggactaccg agtgaacatt tttctgagac agcagtggaa cgattcacgg ctggcataca 1500
gtgagtaccc agatgattcc ctggatttgg atccatcgat gctggattcg atttggaaac 1560
cagatttgtt ctttgccaat gagaaaggag ccaatttcca tgatgtcacc actgataaca 1620
agttgctgcg gatttccaaa aatggcaaag tgctctacag tattagactc accttgactt 1680
tatcctgtcc catggacctg aagaactttc caatggatgt ccagacctgt acaatgcagc 1740
tggagagttt tgggtacacc atgaatgacc tgatatttga gtggttaagt gatggtccag 1800
tacaagttgc tgaaggactc accctgcctc agtttatttt gaaagaagag aaggaacttg 1860
gctattgcac aaagcattac aacactggca agtttacctg cattgaggtc aagtttcacc 1920
tggaacgcca gatgggctat tatttgatcc agatgtatat ccccagcctg ctgatagtca 1980
ttttgtcctg ggtctccttt tggataaaca tggatgcagc tcctgccagg gttgcccttg 2040
gcatcacaac cgtcctgaca atgactaccc agagttcagg ttccagggca tctctgccaa 2100
aggtctcata tgtgaaagca attgacatct ggatggcagt gtgccttctg tttgtgtttg 2160
ctgccttact ggaatatgca gcagtgaact ttgtctccag gcaacacaag gagttccttc 2220
gtctccggag acgacagaag aggcagaata aggaagaaga tgttactcgt gaaagccgtt 2280
ttaacttcag cggttatggg atgggtcact gcctccaagt gaaagatggt acagctgtca 2340
aggctacacc tgccaaccca cttccgcaac cccaaaaaga tgcagatgct atcaagaaga 2400
agtttgtgga tcgggcaaaa agaatcgaca ccatatctcg agctgccttc ccactggcct 2460
tcctcatttt caacatcttt tactggatca catacaagat cattcggcat gaagatgtcc 2520
acaagaaata gatgtatcct attgaccctg ggaccttctt tgcctaagtg ttgtgcttgt 2580
aaatacacag taatagtgtc tttatattgc tttgacagag aacattgagg gtggggggag 2640
gaaaatcatg agggtggggt ttctggcacc tacatgacaa aggacaggtt ctatgggcaa 2700
ggaagaaaac tgcacaaaat taaggtgttg cagaatcaca tgagcataac tcccatccat 2760
agtctatagc attgttcttt cagaggacac atcactcaga catgatatgc aaggtcaagt 2820

tcttgagggc tggttatctt atggtttgat ttgacaagtt ctggttctga aaggttagtt 2880
taatgcacac acacacacac acacacacac acacacacac acacacacac acagaaaatg 2940
cttaccatct gacaatagtg actaccatat agtaaattga ggaccaaact ttttcaggaa 3000
aatgctgcct catttttaaa acaagcctcc tgagctatga tatttacaat gtctgtaatt 3060
agtgtttcac ccaagaaatc cttttgtggg ttgtaaattc tttctacaaa tccaggaaac 3120
aacaacaaga taaacacaaa tgactaagag cagatttcta ctctgctgtc tgtgtaggtg 3180
tgcactttga tattattttt ctttcctaga tataatgtgg gggcttcctt gtgtattgcg 3240
taattgattt actggtatat cctcttaaaa gaaatgctca ttttatttta gatcaagtta 3300
gcatattatg tatatttttt cactttggct atacttatgt gtgactttaa gtgcccaagt 3360
gtaatttagc tattgcagct gctatgccac agtatttatg gagacggtgt gttgtgattg 3420
gtgtatctca gagtagctca aacttcccat ttctgccctc attgtagctg taactactag 3480
tcctaatgtt gactgacctg taatttgtac tttcaatcca agtgaatatc gttttaaata 3540
tccttgatta actgaaaaaa attcaaactg tactgtgatt ttcataaccc gttgcctttt 3600
cggtatcaga gctacgtggt ttgaatcctg gctacatgtt ttaagtaaga aaaaaaatat 3660
gtatttttgc ttactcaaaa gacaacctgt aaagttatat ggaaataatt acattttaca 3720
tgtgctgtaa aagggattat tttaaaaaag catttgttca gtttcaataa aggtaagtgt 3780
gccaccaaaa aataattccc atgttgctgg tggtggtagt tgttgaatag ctgttggcat 3840
tggtgaaaaa ggagctggaa ctggc 3865

<210> 21
<211> 267
<212> PRT
<213> Rattus norvegicus

<220>
<223> Syntaxin 13

<400> 21
Met Tyr Arg Asn Pro Gly Pro Ser Gly Leu Arg Pro Arg Asp Phe Asn
1               5                   10                  15

Ser Ile Ile Gln Thr Cys Ser Gly Asn Ile Gln Arg Ile Ser Gln Ala
            20                  25                  30

Thr Ala Gln Ile Lys Asn Leu Met Ser Gln Leu Gly Thr Lys Gln Asp
        35                  40                  45

Ser Ser Lys Leu Gln Glu Asn Leu Gln Gln Phe Gln His Ser Thr Asn
    50                  55                  60

Gln Leu Ala Lys Glu Thr Asn Glu Leu Leu Lys Glu Leu Gly Ser Leu
65                  70                  75                  80

Pro Leu Pro Leu Ser Ala Ser Glu Gln Arg Gln Gln Lys Leu Gln Lys
                85                  90                  95

Glu Arg Leu Met Asn Asp Phe Ser Ser Ala Leu Asn Asn Phe Gln Val
            100                 105                 110

Val Gln Arg Lys Val Ser Glu Lys Glu Lys Glu Ser Ile Ala Arg Ala
        115                 120                 125

Arg Ala Gly Ser Arg Leu Ser Ala Glu Asp Arg Gln Arg Glu Glu Gln
    130                 135                 140

Leu Val Ser Phe Asp Ser His Glu Glu Trp Asn Gln Met Gln Ser Gln
145                 150                 155                 160

Glu Glu Glu Ala Ala Ile Thr Glu Gln Asp Leu Glu Leu Ile Lys Glu
                165                 170                 175

Arg Glu Thr Ala Ile Gln Gln Leu Glu Ala Asp Ile Leu Asp Val Asn
        180                 185                 190

Gln Ile Phe Lys Asp Leu Ala Met Met Ile His Asp Gln Gly Asp Leu
    195                 200                 205

Ile Asp Ser Ile Glu Ala Asn Val Glu Ser Ser Glu Val His Val Glu
    210                 215                 220

Arg Ala Ser Asp Gln Leu Gln Arg Ala Ala Tyr Tyr Gln Lys Lys Ser
225                 230                 235                 240

Arg Lys Lys Met Cys Ile Leu Val Leu Val Leu Ser Val Ile Val Thr
                245                 250                 255

Val Leu Val Val Val Ile Trp Val Ala Ser Lys
            260                 265

<210> 22
<211> 2963
<212> DNA
<213> Rattus norvegicus

<220>
<223> Syntaxin 13

<400> 22
ctggagcttt gagccacctc tcaaaggaat aatcggtagc agcattcaga ctgtcacgtg 60
gttcccaaaa ggagggctta ggtccaaaac acccaccccc attggatact ctttgggtcc 120
caaaattcca ttgcaaatca tccctaagtt acaaccatcc atccatacaa agcagcacag 180
agcaaagcaa agggtttgtg cggttcccct ccattatatc tgccttatag cccaccctcc 240
aaaaacgttt agatagattc caggccggga aaccacctct tcaggacccc gagggtagga 300
tcagaaacga gacacagttt caagaaactc tgctcagacg gaagggacag gagaggcctt 360
tccgtgtgcc caggatctca gcagaaaaac aaaagagaag attaggcaat gtagtattat 420
ttgagaccag gcttgaatgc ccctgagtaa gagagggacc aggaatacag gcatcccagc 480
gggacggtaa cccagagcag accctgaccc ctctcgttca cagttgctaa ccttgctccc 540
ttccaccacc tcctggggga tcagccaaga atcccctttt accttggcta ggccaatccc 600
caacccactc ccctaggcaa acactggctc atagactcaa tcaagaacag ttctctcagc 660
tactgggccc ggtcagtggc agcttccggt agacgccgtg aagcgcgacc ccggtctctg 720
ctccgtgtca tgtctacggt cccttagaca tgtatcggaa tcccgggccg tcggggctca 780
ggccccgtga cttcaacagc atcatccaga catgcagtgg caacatccag cggatcagcc 840
aagccactgc tcagataaag aatttgatga gccaacttgg aactaagcag gactcaagca 900
aactccagga aaatttgcaa cagttccagc actccacaaa tcagcttgcc aaggagacaa 960
atgagttgct gaaggagtta gggtccttgc ccctaccctt gtctgcatca gagcagcgcc 1020
aacagaagct tcagaaggag cggctcatga atgacttctc gtcggccttg aacaatttcc 1080
aggttgtgca gagaaaggta tctgagaagg agaaagaaag catcgcgaga gcgagggctg 1140
gatcccgtct ttctgcagaa gacaggcaaa gagaagagca gctcgtctca tttgacagcc 1200
atgaggagtg gaaccagatg cagagccagg aggaagaggc ggccatcact gagcaagacc 1260
tggaacttat taaagagagg gaaacggcga ttcagcagct ggaggctgac attttggatg 1320
tcaatcagat ctttaaagac ttagctatga tgatccacga ccaaggtgat ctgattgata 1380
gcatagaagc caatgtggaa agctcagagg tgcatgtgga aagagcctct gatcagctac 1440
agagagctgc ttactatcag aaaaaatctc gcaagaagat gtgtatcctc gtgcttgtcc 1500
tctcagtgat tgttacagtc ttggtagttg ttatctgggt tgcttctaag tgaagtgtgg 1560
ttgcctcaga gccttctcct gctgagttgt tttcgagggc aagtgtttga tggagtcttg 1620
ccagaacaaa ctgaccacag aagagagtgt gtaccacagt gtcctgtgat aatttagcta 1680
gaaactaact actaactagt ccttggaatt agtgaccatg gaggcagtat ttatcaacta 1740
caactagtcc ttggaattag tgaccatgga ggcagtattt atcaatttat gcatatatgc 1800
tcttcatttc tcaaagaacg aattaactta tgtgtatttt cctttctctt gtattttgat 1860
tgccctttat tgcaagtgtt tattgacagt cccactgtgg atattctttg tgacaggtga 1920

cactacatca tagtactgtc tttttgtata tacacaaact ataccttttt atgcatatct 1980
gagataaatt tccttctgga gccagcctat cagagtctga gaagctgtac aatatgccag 2040
caatttttat tatttaacgt tttgctttga gtttctgagg ggtctgttct ctgttttgca 2100
aggttttggc actataaata actggggagt aagaacactg cacccatgac ttcagaggaa 2160
atgagaaaaa cttgtcagag tgggcccgct gccagggttt gtgggaaagt ctgggtttgg 2220
gttaactgac cttattgtct gcgggtgttg tttacgcagt ccatcctcgc acactaagcc 2280
atcccgctcc tggtttagag aggatcagtt cattaccatt gttctaatca atcaggtctc 2340
tctcccacct gtctgcatag aagggggatt cgctcagccc agcatgactt tggagagcag 2400
atgaggactg tttggaatga ttgagtccct ctcgtcctcg tgtggctttc attctgatga 2460
atgataaacg ctgctgtcaa aggctgatcc aggcccttgg aagggttgcc tggctttgat 2520
ggcaagattt tgaaatttag actgaagtgc ggtttaaaac tcggtctgtg aacctattga 2580
taaagcaaag caaaggttca ctttgtaaat aacactggtt tagtgatgac agaattagcc 2640
taatttatga acaagagaca aactctgtgt agttttagat aaaaacagtt ttaataaaag 2700
gtttctgtct tgtatataaa tgttgtctac tgcccttttt cacagaccta gaacagtcag 2760
agagcgtgta attggtttgg gctctcagat aaatgtggat tgaggccgga aatgtcagca 2820
gcacatcctt ggctgacgta gaggatctgg ctgctgtggc aatatctgtg tttgttcctt 2880
ctatccctca tgctgtcact tactgctgtc ccacactaat gtgaccatgg agaagcaagc 2940
aaatgcatag gtttcctcga gcc 2963

<210> 23
<211> 1176
<212> PRT
<213> Rattus norvegicus

<220>
<223> ATPase, calcium, plasma membrane, isoform 1

<400> 23
Met Gly Asp Met Ala Asn Asn Ser Val Ala Tyr Ser Gly Val Lys Asn
1               5                   10                  15

Ser Leu Lys Glu Ala Asn His Asp Gly Asp Phe Gly Ile Thr Leu Ala
            20                  25                  30

Glu Leu Arg Ala Leu Met Glu Leu Arg Ser Thr Asp Ala Leu Arg Lys
        35                  40                  45

Ile Gln Glu Ser Tyr Gly Asp Val Tyr Gly Ile Cys Thr Lys Leu Lys
    50                  55                  60

Thr Ser Pro Asn Glu Gly Leu Ser Gly Asn Pro Ala Asp Leu Glu Arg
65                  70                  75                  80

Arg Glu Ala Val Phe Gly Lys Asn Phe Ile Pro Pro Lys Lys Pro Lys
                85                  90                  95

Thr Phe Leu Gln Leu Val Trp Glu Ala Leu Gln Asp Val Thr Leu Ile
            100                 105                 110

Ile Leu Glu Ile Ala Ala Ile Val Ser Leu Gly Leu Ser Phe Tyr Gln
        115                 120                 125

Pro Pro Glu Gly Asp Asn Ala Leu Cys Gly Glu Val Ser Val Gly Glu
    130                 135                 140

Glu Glu Gly Glu Gly Glu Thr Gly Trp Ile Glu Gly Ala Ala Ile Leu
145                 150                 155                 160

Leu Ser Val Val Cys Val Val Leu Val Thr Ala Phe Asn Asp Trp Ser
                165                 170                 175

Lys Glu Lys Gln Phe Arg Gly Leu Gln Ser Arg Ile Glu Gln Glu Gln
            180                 185                 190

Lys Phe Thr Val Ile Arg Gly Gly Gln Val Ile Gln Ile Pro Val Ala
        195                 200                 205

Asp Ile Thr Val Gly Asp Ile Ala Gln Val Lys Tyr Gly Asp Leu Leu
    210                 215                 220

Pro Ala Asp Gly Ile Leu Ile Gln Gly Asn Asp Leu Lys Ile Asp Glu
225                 230                 235                 240

Ser Ser Leu Thr Gly Glu Ser Asp His Val Lys Lys Ser Leu Asp Lys
                245                 250                 255

Asp Pro Leu Leu Leu Ser Gly Thr His Val Met Glu Gly Ser Gly Arg
            260                 265                 270

Met Val Val Thr Ala Val Gly Val Asn Ser Gln Thr Gly Ile Ile Phe
        275                 280                 285

Thr Leu Leu Gly Ala Gly Gly Glu Glu Glu Glu Lys Lys Asp Glu Lys
    290                 295                 300

Lys Lys Glu Lys Lys Asn Lys Lys Gln Asp Gly Ala Ile Glu Asn Arg
305                 310                 315                 320

Asn Lys Ala Lys Ala Gln Asp Gly Ala Ala Met Glu Met Gln Pro Leu
                325                 330                 335

Lys Ser Glu Glu Gly Gly Asp Gly Asp Glu Lys Asp Lys Lys Lys Ala
            340                 345                 350

Asn Leu Pro Lys Lys Glu Lys Ser Val Leu Gln Gly Lys Leu Thr Lys
        355                 360                 365

Leu Ala Val Gln Ile Gly Lys Ala Gly Leu Leu Met Ser Ala Ile Thr
    370                 375                 380

Val Ile Ile Leu Val Leu Tyr Phe Val Ile Asp Thr Phe Trp Val Gln
385                 390                 395                 400

Lys Arg Pro Trp Leu Ala Glu Cys Thr Pro Ile Tyr Ile Gln Tyr Phe
                405                 410                 415

Val Lys Phe Phe Ile Ile Gly Val Thr Val Leu Val Val Ala Val Pro
            420                 425                 430

Glu Gly Leu Pro Leu Ala Val Thr Ile Ser Leu Ala Tyr Ser Val Lys
        435                 440                 445

Lys Met Met Lys Asp Asn Asn Leu Val Arg His Leu Asp Ala Cys Glu
    450                 455                 460

Thr Met Gly Asn Ala Thr Ala Ile Cys Ser Asp Lys Thr Gly Thr Leu
465                 470                 475                 480

Thr Met Asn Arg Met Thr Val Val Gln Ala Tyr Ile Asn Glu Lys His
                485                 490                 495

Tyr Lys Lys Val Pro Glu Pro Glu Ala Ile Pro Pro Asn Ile Leu Ser
            500                 505                 510

Tyr Leu Val Thr Gly Ile Ser Val Asn Cys Ala Tyr Thr Ser Lys Ile
        515                 520                 525

Leu Pro Pro Glu Lys Glu Gly Gly Leu Pro Arg His Val Gly Asn Lys
    530                 535                 540

Thr Glu Cys Ala Leu Leu Gly Phe Leu Leu Asp Leu Lys Arg Asp Tyr
545                 550                 555                 560

Gln Asp Val Arg Asn Glu Ile Pro Glu Glu Ala Leu Tyr Lys Val Tyr
                565                 570                 575

Thr Phe Asn Ser Val Arg Lys Ser Met Ser Thr Val Leu Lys Asn Ser
            580                 585                 590

Asp Gly Ser Phe Arg Ile Phe Ser Lys Gly Ala Ser Glu Ile Ile Leu
        595                 600                 605

Lys Lys Cys Phe Lys Ile Leu Ser Ala Asn Gly Glu Ala Lys Val Phe
    610                 615                 620

Arg Pro Arg Asp Arg Asp Asp Ile Val Lys Thr Val Ile Glu Pro Met
625                 630                 635                 640

Ala Ser Glu Gly Leu Arg Thr Ile Cys Leu Ala Phe Arg Asp Phe Pro
                645                 650                 655

Ala Gly Glu Pro Glu Pro Glu Trp Asp Asn Glu Asn Asp Val Val Thr
            660                 665                 670

Gly Leu Thr Cys Ile Ala Val Val Gly Ile Glu Asp Pro Val Arg Pro
        675                 680                 685

Glu Val Pro Glu Ala Ile Lys Lys Cys Gln Arg Ala Gly Ile Thr Val
    690                 695                 700

Arg Met Val Thr Gly Asp Asn Ile Asn Thr Ala Arg Ala Ile Ala Thr
705                 710                 715                 720

Lys Cys Gly Ile Leu His Pro Gly Glu Asp Phe Leu Cys Leu Glu Gly
                725                 730                 735

Lys Asp Phe Asn Arg Arg Ile Arg Asn Glu Lys Gly Glu Ile Glu Gln
            740                 745                 750

Glu Arg Ile Asp Lys Ile Trp Pro Lys Leu Arg Val Leu Ala Arg Ser
        755                 760                 765

Ser Pro Thr Asp Lys His Thr Leu Val Lys Gly Ile Ile Asp Ser Thr
    770                 775                 780

Val Ser Glu Gln Arg Gln Val Val Ala Val Thr Gly Asp Gly Thr Asn
785                 790                 795                 800

Asp Gly Pro Ala Leu Lys Lys Ala Asp Val Gly Phe Ala Met Gly Ile
                805                 810                 815

Ala Gly Thr Asp Val Ala Lys Glu Ala Ser Asp Ile Ile Leu Thr Asp

820                 825                 830

Asp Asn Phe Thr Ser Ile Val Lys Ala Val Met Trp Gly Arg Asn Val
        835                 840                 845

Tyr Asp Ser Ile Ser Lys Phe Leu Gln Phe Gln Leu Thr Val Asn Val
    850                 855                 860

Val Ala Val Ile Val Ala Phe Thr Gly Ala Cys Ile Thr Gln Asp Ser
865                 870                 875                 880

Pro Leu Lys Ala Val Gln Met Leu Trp Val Asn Leu Ile Met Asp Thr
                885                 890                 895

Leu Ala Ser Leu Ala Leu Ala Thr Glu Pro Pro Thr Glu Ser Leu Leu
            900                 905                 910

Leu Arg Lys Pro Tyr Gly Arg Asn Lys Pro Leu Ile Ser Arg Thr Met
        915                 920                 925

Met Lys Asn Ile Leu Gly His Ala Phe Tyr Gln Leu Val Val Val Phe
    930                 935                 940

Thr Leu Leu Phe Ala Gly Glu Lys Phe Phe Asp Ile Asp Ser Gly Arg
945                 950                 955                 960

Asn Ala Pro Leu His Ala Pro Pro Ser Glu His Tyr Thr Ile Val Phe
                965                 970                 975

Asn Thr Phe Val Leu Met Gln Leu Phe Asn Glu Ile Asn Ala Arg Lys
            980                 985                 990

Ile His Gly Glu Arg Asn Val Phe Glu Gly Ile Phe Asn Asn Ala Ile
        995                 1000                1005

Phe Cys Thr Ile Val Leu Gly Thr Phe Val Val Gln Ile Ile Ile Val
    1010                1015                1020

Gln Phe Gly Gly Lys Pro Phe Ser Cys Ser Glu Leu Ser Ile Glu Gln
1025                1030                1035                1040

Trp Leu Trp Ser Ile Phe Leu Gly Met Gly Thr Leu Leu Trp Gly Gln
                1045                1050                1055

Leu Ile Ser Thr Ile Pro Thr Ser Arg Leu Lys Phe Leu Lys Glu Ala
            1060                1065                1070

Gly His Gly Thr Gln Lys Glu Glu Ile Pro Glu Glu Glu Leu Ala Glu
        1075                1080                1085

Asp Val Glu Glu Ile Asp His Ala Glu Arg Glu Leu Arg Arg Gly Gln
    1090                1095                1100

Ile Leu Trp Phe Arg Gly Leu Asn Arg Ile Gln Thr Gln Met Asp Val
1105                1110                1115                1120

Val Asn Ala Phe Gln Ser Gly Gly Ser Ile Gln Gly Ala Leu Arg Arg
                1125                1130                1135

Gln Pro Ser Ile Ala Ser Gln His His Asp Val Thr Asn Val Ser Thr
            1140                1145                1150

Pro Thr His Val Val Phe Ser Ser Ser Thr Ala Ser Thr Pro Val Gly
        1155                1160                1165

Tyr Pro Ser Gly Glu Cys Ile Ser
    1170                1175

<210> 24
<211> 4228
<212> DNA
<213> Rattus norvegicus

<220>
<223> ATPase, calcium, plasma membrane, isoform 1

<400> 24
tgtggagaag aagggagcgt cggaccggaa cggatgtata catctcaaga ttgagaggaa 60
gaaacaagtc atgggccagt ggtcaagata cttctctggg aattgttgct gctgataact 120
gctttaccaa gtcatacaat gagtgtttgg tttaaggaag attttaatat ttaaaagatt 180
ttcatcttgg aaaattgatc aagtgaagat ttcatttctt tttttttttg ggaaacattt 240
tcctcctcat aaattaccct tgtaatgggc gacatggcaa acaactcagt tgcatatagt 300
ggtgtgaaaa actctttgaa agaagctaat catgatggcg actttggcat cacacttgcc 360
gagctgcggg ccctcatgga gctcaggtcc acagatgcat tacgaaaaat acaggaaagc 420
tatggggatg tctatggaat ttgcaccaag ttgaaaacat ctcccaatga aggtttaagt 480
ggaaatcctg cagatttaga aagaagagaa gcagtgtttg gaaagaactt tatacctcct 540
aagaagccaa aaacctttct tcagttagta tgggaagcat tacaagatgt cactttaatt 600
atactagaaa tcgcagccat agtatcattg ggcctttctt tttatcaacc tccggaaggg 660
gataatgcac tttgtggaga agtttctgtt ggggaagaag aaggtgaagg agaaacgggg 720
tggattgaag gagctgccat cctcttgtcg gtggtgtgtg tggtgttagt gacggctttc 780
aatgactgga gtaaggagaa acagttccga ggcttgcaga gtcgaattga acaggaacag 840
aagttcaccg tcatcagggg tgggcaggtc atccagatac ctgtagctga cattactgtt 900
ggagacattg cccaagtgaa gtacggtgat cttcttccag ctgatggtat attgattcaa 960
ggcaatgacc ttaaaattga cgagagttcg ttgactggcg agtctgacca tgttaagaag 1020
tctctagata aagacccctt gcttctttca ggtactcatg taatggaagg ttctggaagg 1080
atggtcgtta ctgctgtagg tgtgaactct caaactggga tcatctttac cttacttgga 1140
gctggaggcg aagaggagga aaagaaagat gagaagaaga aagaaaagaa aaataagaaa 1200
caagatggag ctattgaaaa tcgcaacaaa gcaaaagccc aagacggcgc ggccatggag 1260
atgcagcctc tgaagagtga agaaggggga gatggtgatg agaaagacaa gaagaaagca 1320
aatctgccaa agaaggaaaa gtctgtgtta caagggaaac ttacgaagct ggctgttcag 1380
attggcaaag caggtctgct gatgtctgcc atcacggtta tcatcctagt gctatatttt 1440
gtgatcgata ccttctgggt tcagaagagg ccgtggcttg ctgagtgcac gccaatctac 1500
atccagtact ttgtgaagtt cttcattatc ggggtcaccg ttctagtggt cgcggtgccg 1560
gaaggcctcc cgctggccgt cacgatctca ctggcttact ccgtcaagaa aatgatgaag 1620
gataacaact tggtgaggca cctggacgct tgtgagacca tggggaacgc tacagctatc 1680
tgttccgata agacggggac cctgacgatg aacaggatga cggtcgttca ggcctacatc 1740
aacgaaaagc attataaaaa ggttccggaa ccagaagcca tcccgccaaa tatcctgtcc 1800
tatctggtaa caggaatttc tgtgaattgt gcttatacat caaaaatatt gccaccggag 1860
aaagagggtg gattacctcg tcatgtgggg aataaaacag aatgtgcctt gttgggattt 1920
ctcttggatt taaagcggga ttatcaagat gttagaaatg aaataccaga agaagcattg 1980
tacaaagtct ataccttcaa ttctgttagg aaatccatga gtacagtcct aaagaattca 2040
gatggaagtt tccggatctt cagcaagggt gcatctgaga taattctgaa aaagtgtttc 2100
aaaatcttga gtgctaatgg tgaggccaaa gtgttccgac ccagagaccg agatgacatt 2160
gtgaagacgg tgattgagcc catggcatct gaaggcctga gaaccatatg cctagcgttc 2220
agagacttcc ccgcaggaga gccagaacca gagtgggata acgagaacga tgtcgtcacc 2280
ggccttactt gcatcgctgt tgtggggatt gaagatcctg tgaggcctga ggtaccagag 2340
gcaataaaga aatgtcagag ggctggaatt accgtgcgca tggtcactgg tgataatatt 2400
aacacagctc gggccattgc taccaaatgc ggtattttac acccagggga agatttcctg 2460
tgcttagaag gtaaagattt taaccgaaga atacgaaatg aaaaaggaga gattgaacaa 2520
gagaggatag acaagatttg gccaaagctt cgagtgcttg caagatcatc tcccactgac 2580
aaacatacac tggttaaagg tataattgac agcacagtct cagagcaacg ccaggttgta 2640

gctgtgacag gggatggtac aaatgacggg cctgcgctga agaaagcaga cgttggcttt 2700
gcaatgggca ttgctggaac tgatgtggct aaagaagcat ctgatatcat tctcacagat 2760
gacaacttca caagcattgt taaagcggtt atgtggggac ggaatgtcta tgacagcatc 2820
tcaaaattcc ttcagttcca gcttactgtt aatgtagtgg ccgtgattgt cgctttcacg 2880
ggcgcttgta ttactcaaga ctcgccactg aaggcggtac agatgctctg ggtcaacctc 2940
atcatggaca cgctggcctc cctggctctg gcgacagaac cacccaccga gtcactcctg 3000
ctccggaagc cttacgggag aaacaagcct ctcatctcac gcacaatgat gaagaatatc 3060
ttgggccatg cgttctatca gctcgtagtg gtcttcacgc tcttgttcgc tggagagaag 3120
tttttcgata tcgacagtgg gaggaacgcc cctctgcatg ctccaccgtc agagcactac 3180
accattgtgt tcaacacctt tgtgctgatg cagctcttca atgaaataaa tgcccggaaa 3240
attcatgggg aaagaaatgt gtttgaaggg atcttcaaca acgccatctt ctgcacaatt 3300
gtcttaggca cctttgtggt gcagataata atcgtgcagt ttggcgggaa gcctttcagc 3360
tgctcagaac tttcaataga acaatggctg tggtccatat tcctgggaat ggggacctta 3420
ctctggggcc agcttatttc aaccattcca accagccgct taaagtttct aaaagaagct 3480
ggccatggga cacagaagga agagatccct gaggaggaat tggcggagga tgttgaagag 3540
attgaccatg ccgaacggga gttgcggcgt ggccagatct tgtggtttag aggcctgaac 3600
agaatccaaa cacagatgga tgtagtgaat gctttccaga gtggaggttc cattcagggg 3660
gctctaaggc gacaaccctc catcgccagc cagcaccatg atgtaacaaa tgtttctacc 3720
cctacacatg tagtgttttc ctcttctact gcttctactc ctgtggggta tccgagtggt 3780
gaatgcattt cgtagctctt tatacgaagg gttagagaag ccagaatcaa gaagttcaat 3840
ccacaacttt atgacacacc ccgagtttag gatagaagat tcagagccgc acatccccct 3900
tatcgatgac actgatgctg aagatgacgc tcctactaaa cgcaactcca gccctccgcc 3960
ctctcctaac aaaaataaca atgccgttga cagcgggatc caccttacaa tagaaatgaa 4020
caagtctgct acctcttcgt ccccagggag cccactgcat agtttggaaa catcactctg 4080
attgtaagct gaacgttaac acactagccg cactgtaaag aaacaaattg aaactgagtc 4140
ttttcacaca ttgtgacgga caagatggcg ttcttgtctc cggacttcaa cagaagacac 4200
acttgtacga atgtagattt attttttt 4228

<210> 25
<211> 723
<212> PRT
<213> Rattus norvegicus

<220>
<223> Calcium transporter CaT2

<400> 25
Met Gly Val Lys Lys Pro Trp Ile Gln Leu Gln Lys Arg Leu Asn Trp
1               5                   10                  15

Trp Val Arg Glu Gln Asp Trp Asn Gln His Val Asp Gln Leu His Met
            20                  25                  30

Leu Gln Gln Lys Ser Ile Trp Glu Ser Pro Leu Leu Arg Ala Ala Lys
        35                  40                  45

Glu Asn Asp Met Cys Thr Leu Lys Arg Leu Gln His Asp Gln Asn Cys
    50                  55                  60

Asp Phe Arg Gln Arg Gly Ala Leu Gly Glu Thr Ala Leu His Val Ala
65                  70                  75                  80

Ala Leu Tyr Asp Asn Leu Asp Ala Ala Ile Met Leu Met Glu Thr Ala
                85                  90                  95

Pro Tyr Leu Val Thr Glu Ser Thr Leu Cys Glu Pro Phe Val Gly Gln
            100                 105                 110

Thr Ala Leu His Ile Ala Ile Met Asn Gln Asn Val Asn Leu Val Arg
        115                 120                 125

Ala Leu Leu Ala Arg Gly Ala Ser Ala Ser Ala Arg Ala Thr Gly Ser
    130                 135                 140

Ala Phe His Arg Ser Ser His Asn Leu Ile Tyr Tyr Gly Glu His Pro
145                 150                 155                 160

Leu Ser Phe Ala Ala Cys Val Gly Ser Glu Glu Ile Val Arg Leu Leu
                165                 170                 175

Ile Glu His Gly Ala Asp Ile Arg Ala Gln Asp Ser Leu Gly Asn Thr
            180                 185                 190

Val Leu His Ile Leu Val Leu Gln Pro Asn Lys Thr Phe Ala Cys Gln
        195                 200                 205

Met Tyr Asn Leu Leu Leu Ser His Asp Gly Gly Asp His Leu Lys Ser
    210                 215                 220

Leu Glu Leu Val Pro Asn Asn Gln Gly Leu Thr Pro Phe Lys Leu Ala
225                 230                 235                 240

Gly Val Glu Gly Asn Thr Val Met Phe Gln His Leu Met Gln Lys Arg
                245                 250                 255

Lys His Ile Gln Trp Ser Leu Gly Pro Leu Thr Ser Ser Ile Tyr Asp
            260                 265                 270

Leu Thr Glu Ile Asp Ser Trp Gly Glu Asp Leu Ser Phe Leu Glu Leu
        275                 280                 285

Val Val Ser Ser Lys Lys Lys Glu Ala Arg Gln Ile Leu Glu Gln Thr
    290                 295                 300

Pro Val Lys Glu Leu Val Ser Leu Lys Trp Lys Lys Tyr Gly Gln Pro
305                 310                 315                 320

Tyr Phe Cys Leu Leu Gly Met Leu Tyr Ile Phe Tyr Met Ile Cys Phe
                325                 330                 335

Thr Thr Cys Cys Val Tyr Arg Pro Leu Lys Phe Arg Asp Ala Asn Arg
            340                 345                 350

Thr His Val Arg Asp Asn Thr Val Leu Glu Gln Lys Pro Leu Gln Glu
        355                 360                 365

Ala Tyr Val Thr Tyr Gln Asp Lys Val Arg Leu Val Gly Glu Leu Val
    370                 375                 380

Thr Val Ile Gly Ala Val Val Ile Leu Leu Ile Glu Ile Pro Asp Ile
385                 390                 395                 400

Phe Arg Val Gly Ala Ser Arg Tyr Phe Gly His Thr Val Leu Gly Gly
                405                 410                 415

Pro Phe His Val Ile Ile Ile Thr Tyr Ala Ser Leu Val Leu Leu Ile
            420                 425                 430

Met Val Met Arg Leu Thr Ser Met Asn Gly Glu Val Val Pro Ile Ser
        435                 440                 445

Met Ala Leu Val Leu Gly Trp Cys Ser Val Met Tyr Phe Ser Arg Gly
    450                 455                 460

Phe Gln Met Leu Gly Pro Phe Thr Ile Met Ile Gln Lys Met Ile Phe
465                 470                 475                 480

Gly Asp Leu Leu Arg Phe Cys Trp Leu Met Ala Met Val Ile Leu Gly
                485                 490                 495

Phe Ala Ser Ala Phe Tyr Ile Ile Phe Gln Thr Glu Asp Pro Glu Ser
            500                 505                 510

Leu Gly Glu Phe Ser Asp Tyr Pro Thr Ala Met Phe Ser Thr Phe Glu
        515                 520                 525

Leu Phe Leu Thr Ile Ile Asp Gly Pro Ala Asn Tyr Ser Val Asp Leu
    530                 535                 540

Pro Phe Met Tyr His Leu Thr Tyr Phe Ala Phe Ala Ile Ile Ala Thr
545                 550                 555                 560

Leu Leu Met Leu Asn Leu Phe Ile Ala Met Met Gly Asp Thr His Trp
                565                 570                 575

Arg Val Ala Gln Glu Arg Asp Glu Leu Trp Arg Ala Gln Val Val Ala
            580                 585                 590

Thr Thr Val Met Leu Glu Arg Lys Met Pro Arg Phe Leu Trp Pro Arg
        595                 600                 605

Ser Gly Ile Cys Gly Cys Glu Tyr Gly Leu Gly Asp Arg Trp Phe Leu
    610                 615                 620

Arg Val Glu His His Gln Glu Gln Asn Pro Tyr Arg Val Leu Arg Tyr
625                 630                 635                 640

Val Glu Ala Phe Lys Ser Ser Asp Lys Glu Glu Val Gln Glu Gln Leu
                645                 650                 655

Ser Glu Lys Gln Pro Ser Gly Thr Glu Thr Gly Thr Leu Ala Arg Gly
            660                 665                 670

Ser Val Val Leu Gln Thr Pro Pro Leu Ser Arg Thr Thr Ser Leu Ser
        675                 680                 685

Ser Asn Ser His Arg Gly Trp Glu Ile Leu Arg Arg Asn Thr Leu Gly
    690                 695                 700

His Leu Asn Leu Gly Gln Asp Leu Gly Glu Gly Asp Gly Glu Glu Ile
705                 710                 715                 720

Tyr His Phe


<210> 26
<211> 4182
<212> DNA
<213> Rattus norvegicus

<220>

<223> Calcium transporter CaT2

<400> 26
taccagggga tatatacctt tcttcaccct agcagaccta gactcctcag cttctgtcac 60
cctagactga ctccttccat gagattcgaa taagtggctg gtatttgcat gaagacatgg 120
gggtgaaaaa accttggatc caacttcaga aacgtctcaa ctggtgggtc agagaacaag 180
actggaacca gcatgtggat cagcttcata tgctacagca gaaaagtatt tgggagtcac 240
cactgcttag agcagccaag gaaaatgata tgtgcacact taagagactt caacatgacc 300
agaactgtga cttccgacaa agaggagctc tgggggagac agcactgcat gtagcagccc 360
tctatgacaa cctggatgct gctataatgc tgatggagac tgctccatac ttggtcacgg 420
agtctacact gtgtgagcca tttgtaggtc agactgcact gcacatcgca attatgaacc 480
agaatgtgaa cctggtccgt gccctgctag cccgaggagc cagtgcctcc gccagggcta 540
caggttctgc cttccatcga agttcccaca acctcatcta ctatggagaa catcctttgt 600
cctttgctgc ctgtgtgggt agtgaggaga ttgttagact gctcattgag catggagctg 660
acattcgggc ccaggactcc ttgggaaata cagtactaca catacttgtc ctacagccca 720
acaaaacctt tgcctgccag atgtacaacc tgctactctc ccatgatggg ggagaccacc 780
tgaagtccct ggaacttgtg cccaacaacc aaggactcac ccccttcaag ctggctgggg 840
tggaaggcaa cactgtgatg tttcaacacc tgatgcaaaa acggaagcac atccagtggt 900
cattaggacc cctaacatct tccatttatg atctcacaga aattgactcc tggggtgagg 960
acttgtcctt tctggagctt gtggtttcct ccaagaagaa agaggctcgt cagattctag 1020
aacaaacccc tgtgaaggag ctggtgagcc tgaagtggaa gaaatatgga caaccctatt 1080
tctgcctcct gggtatgctg tacatcttct acatgatctg cttcaccaca tgctgtgttt 1140
accgccccct caagttccgt gatgccaacc gtacacatgt tagagataac accgtcctgg 1200
aacagaaacc actacaggaa gcgtatgtga cttaccagga taaggtccgg ctggtgggag 1260
aactcgtgac agtcattggg gctgtggtta ttctactcat agagatccca gacatcttca 1320
gagttggtgc ctctcgctac tttggacaca cagttcttgg gggtccattc catgtcatta 1380
tcatcacata tgcctccctt gtgctactga ttatggtgat gaggcttacc agtatgaatg 1440
gggaggtggt ccccatatcc atggccttgg tactgggctg gtgtagtgtc atgtacttct 1500
cccgaggatt ccagatgctg ggccccttca caatcatgat tcagaagatg atttttggag 1560
acctactgcg cttctgctgg ctaatggcta tggtcatcct cggatttgcc tctgccttct 1620
atatcatctt ccagacagag gatccagaga gtctgggaga attctccgac taccctacag 1680
caatgttcag cacctttgag ctcttcctta ccatcattga cggtccggcc aattacagtg 1740
tggatctacc cttcatgtac cacctaacct attttgcttt tgccatcatc gccacactac 1800
tcatgctcaa tttgttcatt gccatgatgg gtgacactca ctggcgagtg gcccaggaga 1860
gggacgaact ctggagagca caggttgtag ccaccacagt gatgctggag aggaaaatgc 1920
cccgtttcct gtggcctcgc tctggtatct gtggttgcga atatggcctg ggagaccgct 1980
ggttcttacg ggttgaacac caccaggagc agaatccgta tcgagtactt cgctatgtgg 2040
aagctttcaa atcttcagac aaggaggaag tacaagaaca actatctgag aagcagcctt 2100
ccgggactga gactgggact ttagctagag gctctgtggt tctgcaaacg cctcctttgt 2160
ccaggactac atccctaagc agcaatagcc accgtggatg ggaaattctc cgtcgaaaca 2220
ccctgggaca tttgaatctt ggacaagacc ttggtgaggg agacggagag gagatctacc 2280
atttctgaag agaattctgt ttctctagac ctagccccag gtgggccagg gtgtagaaga 2340
gaaaggggta gttaggggtg ggcagggaga aggagagagc tgatttgcaa gaatctgaat 2400
tctatggtta tgaatagttg gagggtatgg gcagtggcca tcttgtgttg tatgtacctt 2460
ataagttcct ctaaagctca tttcctgaga accaggcatc tgagcatatg aaatggaggc 2520
taattgtcat cttgggttga catctagacc atctattttg tgccttgaat agaaccagaa 2580
agcagtaagt caggaaactt gcccgattct ataacgtata atgtggaaag aaccatcata 2640
ccatgaacat ccttcccatc tctttagact tcctaggact ctcttcccag ctagcaaatc 2700
ccaatgtaga aaactacagg aaaaaaaata tttccttatc ctgttttaat ttttctctta 2760
tgtcaaaaaa atatacatta cataaaactt atcattttta ctattttcag tataaagtac 2820
attcagatta ttgtataaat atcactagta ttcatctaca aaaggttttc atctcagaca 2880
ccaaaactcc tgtccactga acaataactc tctactattc cttctcgtta gtcctcaata 2940
accattattt tacttcctga ctatgaattg attgtaggtg ctttatttaa aaaccgactg 3000
gaaaagacat gatccgttgc acttgccttg ttttgtttaa tatagtagtt tcaaggtttc 3060
ttctattctc tagaggtatc agaatttcat ttctctttaa gtctgaatat ctacacctta 3120
cttcattcat ccatctatca atgagttttc ttatcttctg tttactgtga acattgctgc 3180
tatgaaaata aagtgtgcat gtctatagag atttaaggtt ttgggtaatt tattcacagt 3240
tccggtgtag tcactggatt gaacaggaca caaggatgaa ataaccatct ggctcagaaa 3300
actaaacatt catttaaaag aatacaaaat caaaacagag caatgatatt ctaaccccca 3360
gtcccacaga agtataattt aaacaaatat caaaacttta ttaatggaaa acacattcac 3420
cagagaacat tcaaaagtta gcaagaccat ttccacaccc tctggaaacc agtacagaca 3480

aaataaggtg agcatttcct aagatgtccc catcagctct tacagcatca ctaccaagcg 3540
agtacggact gacagttaac acaacaaaac ttagccgtct tgctcataac tatggactta 3600
actattcaaa gccaatactg acagttttgt tatctgtcac catcccagac tgaggatata 3660
tttaaattca agctataaag atactgaaat actaattaac aatctttaaa tacagtgaga 3720
aagaccaact tatgttaaaa cttttgaatg tgaaactaaa taaaagccat ctgaaatctg 3780
tttccaagct taaaaccctg cctagtatta ctgtacacac acctcactaa acgtgtggat 3840
gtcataaacc aaaatgtctt tttcccctcc atgtctttgg cctttccaaa ttgtgaatta 3900
tgaattcttt ttttaacaaa cattttccaa tgacattgtg ctttattatt tttttagaaa 3960
aaagttaaag tatataataa actggtaacc ttagtctctt ttattgctct agaatatcct 4020
aattataaaa aaaggtaaga gtaaccccaa agacagaggt gtgttgtgaa agtgtattag 4080
tttccctctt ctagggacca aatgtaggca cctttcctcc catcttcaga accactgagg 4140
caagaggcct ggactgaagg aatatgaccc tgtcacctca ga 4182

<210> 27
<211> 301
<212> PRT
<213> Rattus norvegicus

<220>
<223> Carnitine/acylcarnitine carrier protein

<400> 27
Met Ala Glu Glu Pro Lys Pro Ile Ser Pro Leu Lys Asn Leu Leu Ala
1               5                   10                  15

Gly Gly Phe Gly Gly Val Cys Leu Val Phe Val Gly His Pro Leu Asp
            20                  25                  30

Thr Val Lys Val Arg Leu Gln Thr Gln Pro Pro Ser Leu Pro Gly Gln
        35                  40                  45

Pro Pro Met Tyr Ser Gly Thr Ile Asp Cys Phe Arg Lys Thr Leu Phe
    50                  55                  60

Arg Glu Gly Ile Thr Gly Leu Tyr Arg Gly Met Ala Ala Pro Ile Ile
65                  70                  75                  80

Gly Val Thr Pro Met Phe Ala Val Cys Phe Phe Gly Phe Gly Leu Gly
                85                  90                  95

Lys Arg Leu Gln Gln Lys Ser Pro Glu Asp Glu Leu Thr Tyr Pro Gln
            100                 105                 110

Leu Phe Thr Ala Gly Met Leu Ser Gly Val Phe Thr Thr Gly Ile Met
        115                 120                 125

Thr Pro Gly Glu Arg Ile Lys Cys Leu Leu Gln Ile Gln Ala Ser Ser
    130                 135                 140

Gly Lys Asn Lys Tyr Ser Gly Thr Leu Asp Cys Ala Lys Lys Leu Tyr
145                 150                 155                 160

Gln Glu Phe Gly Ile Arg Gly Phe Tyr Lys Gly Thr Ala Leu Thr Leu
                165                 170                 175

Met Arg Asp Val Pro Ala Ser Gly Met Tyr Phe Met Thr Tyr Glu Trp
            180                 185                 190

Leu Lys Asn Leu Phe Thr Pro Gln Gly Lys Ser Val His Asp Leu Ser
        195                 200                 205

Val Pro Arg Val Leu Val Ala Gly Gly Phe Arg Gly Ile Phe Asn Trp
    210                 215                 220

Val Val Ala Ile Pro Pro Asp Val Leu Lys Ser Arg Phe Gln Thr Ala
225                 230                 235                 240

Pro Pro Gly Lys Tyr Pro Asn Gly Phe Arg Asp Val Leu Arg Glu Leu
                245                 250                 255

Ile Arg Glu Glu Gly Val Thr Ser Leu Tyr Lys Gly Phe Asn Ala Val
            260                 265                 270

Met Ile Arg Ala Phe Pro Ala Asn Ala Ala Cys Phe Leu Gly Phe Glu
        275                 280                 285

Ile Pro Met Lys Ile Leu Asn Trp Ile Ala Pro Asn Leu
    290                 295                 300


<210> 28
<211> 1231
<212> DNA
<213> Rattus norvegicus

<220>
<223> Carnitine/acylcarnitine carrier protein

<400> 28
acagcgacga agatcaagac tgccaggagc tgcaagtaga ctgacagaag actgaccatg 60
gcagaggagc cgaaacccat cagtccgctt aagaacctcc ttgccggcgg ctttggcggc 120
gtgtgcctgg tgtttgtggg gcaccccttg gacacggtca aggtccgact gcagacacaa 180
ccaccaagtt tgcctgggca gccacctatg tactctggga ccatcgactg tttccggaag 240
actcttttta gagagggcat cacagggctg tatcggggca tggctgcacc catcattgga 300
gtcaccccta tgtttgcggt gtgcttcttt gggtttggtt tgggaaagag actacagcag 360
aaatctccag aggatgaact tacctacccg cagctgttta cagctgggat gttatctggt 420
gtgttcacca caggaatcat gacccctgga gaacggatca aatgcttact gcagattcag 480
gcttcttcag ggaaaaacaa atacagtggt accttggact gtgcaaagaa gctgtatcag 540
gagtttggga tccgtggctt ctacaaaggg actgcgctca ctctcatgcg agatgttcct 600
gccagtggga tgtatttcat gacatacgaa tggcttaaaa atctcttcac tccacaggga 660
aagagtgtcc atgacctcag tgtgcctcgg gtcctggtgg ctggcggctt tcgagggatc 720
ttcaattggg ttgtggcaat ccccccagac gtgctcaagt ctcgattcca gaccgcacct 780
cctggaaaat atcctaatgg tttcagagat gtgttgagag agctgatccg agaagaagga 840
gtcacctcct tgtacaaagg gttcaatgca gtcatgatcc gagccttccc agccaatgct 900
gcctgtttcc ttggctttga aattccgatg aagatcctca attggattgc ccccaacttg 960
tgaggctgaa gacatgttga agacctttgg atgctggaag actgttgccg agaagcaggg 1020
gcaggactgg gaagaccctg agggtgaggg gagggaagtg gtgtgatcag agccccaggc 1080
atgactgtga tggtgaaact gttgccttaa tgacatcctc taccttatgt aacttggtgc 1140
cattttgaaa cttgaatata gaaggtttcc agagaattgg agatggaata aatagtttat 1200
agactcgtca aaaaaaaaaa aaaaaaaaaa a                                1231


<210> 29
<211> 516
<212> PRT
<213> Homo sapiens

<220>
<223> Potent brain type organic ion transporter

<400> 29

Met Ala Ser Asp Pro Ile Phe Thr Leu Ala Pro Pro Leu His Cys His
1               5                   10                  15

Tyr Gly Ala Leu Pro Pro Asn Ala Ser Gly Trp Glu Gln Pro Pro Asn
            20                  25                  30

Ser Ser Gly Val Ser Val Ala Gly Ala Ala Leu Ala Ala Ser Ala Ala
        35                  40                  45

Ser Arg Val Val Thr Ser Thr Asp Pro Ser Cys Ser Gly Phe Ala Pro
    50                  55                  60

Pro Asp Phe Asn His Cys Leu Lys Asp Trp Asp Tyr Asn Gly Leu Pro
65                  70                  75                  80

Val Leu Thr Thr Asn Ala Ile Gly Gln Trp Asp Leu Val Cys Asp Leu
                85                  90                  95

Gly Trp Gln Val Ile Leu Glu Gln Ile Leu Phe Ile Leu Gly Phe Ala
            100                 105                 110

Ser Gly Tyr Leu Phe Leu Gly Tyr Pro Ala Asp Arg Phe Gly Arg Arg
        115                 120                 125

Gly Ile Val Leu Leu Thr Leu Gly Leu Val Gly Pro Cys Gly Val Gly
    130                 135                 140

Gly Ala Ala Ala Gly Ser Ser Thr Gly Ile Met Thr Leu Arg Phe Leu
145                 150                 155                 160

Leu Gly Phe Leu Leu Ala Gly Val Asp Leu Gly Val Tyr Leu Met Arg
                165                 170                 175

Leu Glu Leu Cys Asp Pro Thr Gln Arg Leu Arg Val Ala Leu Ala Gly
            180                 185                 190

Glu Leu Val Gly Val Gly Gly His Phe Leu Phe Leu Gly Leu Ala Leu
        195                 200                 205

Val Ser Lys Asp Trp Arg Phe Leu Gln Arg Met Ile Thr Ala Pro Cys
    210                 215                 220

Ile Leu Phe Leu Phe Tyr Gly Trp Pro Gly Leu Phe Leu Glu Ser Ala
225                 230                 235                 240

Arg Trp Leu Ile Val Lys Arg Gln Ile Glu Glu Ala Gln Ser Val Leu
                245                 250                 255

Arg Ile Leu Ala Glu Arg Asn Arg Pro His Gly Gln Met Leu Gly Glu
            260                 265                 270

Glu Ala Gln Glu Ala Leu Gln Glu Leu Glu Asn Thr Cys Pro Leu Pro
        275                 280                 285

Thr Thr Ser Thr Phe Ser Phe Ala Ser Leu Leu Asn Tyr Arg Asn Ile
    290                 295                 300

Trp Lys Asn Leu Leu Ile Leu Gly Phe Thr Asn Phe Ile Ala His Ala
305                 310                 315                 320

Ile Arg His Cys Tyr Gln Pro Val Gly Gly Gly Gly Ser Pro Ser Asp

325                 330                 335

Phe Tyr Leu Cys Ser Leu Leu Ala Ser Gly Thr Ala Ala Leu Ala Cys
            340                 345                 350

Val Phe Leu Gly Val Thr Val Asp Arg Phe Gly Arg Arg Gly Ile Leu
        355                 360                 365

Leu Leu Ser Met Thr Leu Thr Gly Ile Ala Ser Leu Val Leu Leu Gly
    370                 375                 380

Leu Trp Asp Tyr Leu Asn Asp Ala Ala Ile Thr Thr Phe Ser Val Leu
385                 390                 395                 400

Gly Leu Phe Ser Ser Gln Ala Ser Ala Ile Leu Ser Thr Leu Leu Ala
                405                 410                 415

Ala Glu Val Ile Pro Thr Thr Val Arg Gly Arg Gly Leu Gly Leu Ile
            420                 425                 430

Met Ala Leu Gly Ala Leu Gly Gly Leu Ser Cys Pro Ala Gln Arg Leu
        435                 440                 445

His Met Gly His Gly Ala Phe Leu Gln His Val Val Leu Ala Ala Cys
    450                 455                 460

Ala Leu Leu Cys Ile Leu Ser Ile Met Leu Leu Pro Glu Thr Lys Arg
465                 470                 475                 480

Lys Leu Leu Pro Glu Val Leu Arg Asp Gly Glu Leu Cys Arg Arg Pro
                485                 490                 495

Ser Leu Leu Arg Gln Pro Pro Pro Asn Arg Cys Asp His Val Pro Leu
            500                 505                 510

Leu Ala Thr Ser
        515


<210> 30
<211> 2375
<212> DNA
<213> Homo sapiens

<220>
<223> Potent brain type organic ion transporter

<400> 30
ccacgcgtcc gggaggcgcc cgcggctgca gagctgcaga gcggggtctc ccggccctcg 60
gcgtcccagc cctttgcaca tagctgggcc agacaacaac tcattctctc ggcgcggccg 120
tcagggcccc agcggtcggc aagctggctc cccgagtagc caccgggacc cccgagccca 180
atggcggggg cggcaacaaa atcgacagca ccgtggagat cgcccccggc gccaatggac 240
aggtcgggag cctcggagat gcggtccccc ccgagcagct gcagggtgag cgggagcgcc 300
ggcgggaggg ggagggcgac gcgctgggca gcagcctgtc gctggccgcg cccccgggcc 360
ccctcagctt cgaggctcta ctggcccagg tgggggccct gggtggcggc cagcagctgc 420
agctcggcct gtgctgcctg cccgtgctct tcgtggcgct gggcatggcc tcagacccca 480
tcttcacgct ggcgcccccg ctgcactgcc actacggcgc cttgcccccc aacgcttcgg 540
gctgggagca gccccccaac tccagcggcg tcagcgtcgc cggcgcagcc ctagcagcca 600
gcgccgccag cagagtcgtc accagtacgg acccctcgtg cagtggcttc gccccaccgg 660
atttcaacca ctgcctgaag gactgggact acaacggact gcccgtgctc accaccaatg 720
ccatcggcca gtgggatctg gtgtgtgacc tgggctggca ggtgatcctg gagcagatcc 780

tcttcatctt gggctttgcc tccggctacc tgttcctcgg ttaccccgcg gacaggtttg 840
gccgtcgtgg gattgtactg ctgaccttgg ggctcgtggg cccctgtgga gtgggaggag 900
ctgctgcggg ctcctccacg ggcataatga ctcttcgatt cctcctgggc ttcctgctgg 960
ctggcgttga ccttggtgtc tacctgatgc gcctggagct gtgcgaccca acccagaggc 1020
ttcgggtggc cctggcaggg gagttggtag ggtgggaggg gcacttcctg ttcctgggcc 1080
tggcccttgt ctctaaggac tggcggttcc tgcagcgaat gatcaccgct ccttgcatcc 1140
tcttcctgtt ttatggctgg cccggtctgt ttctggagtc cgcacggtgg ctgatagtga 1200
aacggcagat tgaggaagcc cagtctgtgc tgaggatcct ggctgagcga aaccggcccc 1260
atggccagat gctgggagaa gaggcccagg aagccctgca ggagctggag aatacctgtc 1320
ctctccccac aacgtccacc ttttccttcg cctccctcct caactaccga aacatctgga 1380
aaaatctgct tatcctgggc ttcaccaact ttatcgccca tgccattcgc cactgctacc 1440
agcctgtggg aggaggaggg agcccatcag acttctactt gtgctctctt ctggccagcg 1500
gcacagcagc cctggcctgc gtcttcctgg gggtgaccgt ggaccgtttt ggccgtcggg 1560
gcatcctgct tctctcaatg actctcacgg ggattgcatc cctggtcttg ctgggcctgt 1620
gggattatct gaacgatgct gccatcacaa ccttctcggt cctcggactc ttctcctccc 1680
aagcttctgc tatcctcagt accctccttg ctgctgaagt catccccacc actgtccggg 1740
gccgtggcct gggccttatc atggcacttg gggcgcttgg agggctgagc tgtccagctc 1800
agcgcctcca catgggccat ggagctttcc tgcagcatgt ggtactggcg gcctgtgccc 1860
tcctctgcat ccttagcatc atgctgctgc cagagaccaa gcgcaagctt ctgccagagg 1920
tactccggga tggggaactg tgccgtcggc cttccctgct gaggcagcca cctcctaacc 1980
gctgtgacca tgtccccctg ctagccactt cctaatcctg ccctctaagc agcctctgag 2040
cctggtggga ggctggccat ttagaaaggt gacggagggc tggctagcaa gatagacgga 2100
gaggcaaggc caccctgtac atacaaaagg ctccaaggcg cctcacgcca tctaggagag 2160
ctcagagtgc cattcccaac cccctctcct ccccgctgct ttctgttcac ttcatcagca 2220
agagtcagga cggggatagc atctcgctat aaccgttagg tctgggatcc atcctatgcc 2280
caaagacatt ttcccagacc ttgctctttc tcgctcttca ttctgtttca ataaaagaca 2340
ttttgaataa atgagcaaaa aaaaaaaaaa aaaaa 2375

<210> 31
<211> 322
<212> PRT
<213> Rattus norvegicus

<220>
<223> UDP galactose transporter

<400> 31
Met Ala Ala Ser Arg Ser Leu Val Pro Asp Arg Leu Arg Leu Pro Leu
1               5                   10                  15

Cys Phe Leu Gly Val Phe Val Cys Tyr Phe Tyr Tyr Gly Ile Leu Gln
            20                  25                  30

Glu Lys Ile Thr Arg Gly Lys Tyr Gly Glu Gly Pro Lys Gln Glu Thr
        35                  40                  45

Phe Thr Phe Ala Leu Thr Leu Val Phe Ile Gln Cys Val Ile Asn Ala
    50                  55                  60

Met Phe Ala Lys Ile Leu Ile Gln Phe Phe Asp Thr Ala Arg Val Asp
65                  70                  75                  80

Arg Thr Arg Thr Trp Leu Tyr Ala Ala Cys Ser Val Ser Tyr Val Gly
                85                  90                  95

Ala Met Val Ser Ser Asn Ser Ala Leu Gln Phe Val Asn Tyr Pro Thr
            100                 105                 110

Gln Val Leu Gly Lys Ser Cys Lys Pro Ile Pro Val Met Leu Leu Gly
        115                 120                 125

Val Thr Leu Leu Lys Lys Lys Tyr Pro Leu Ala Lys Tyr Leu Cys Val
    130                 135                 140

Leu Leu Ile Val Ala Gly Val Ala Leu Phe Met Tyr Lys Pro Lys Lys
145                 150                 155                 160

Val Val Gly Ile Glu Glu His Thr Val Gly Phe Gly Glu Leu Leu Leu
                165                 170                 175

Leu Leu Ser Leu Thr Leu Asp Gly Leu Thr Gly Val Ser Gln Asp His
            180                 185                 190

Met Arg Ala His Tyr Gln Thr Gly Ser Asn His Met Met Leu Asn Ile
        195                 200                 205

Asn Leu Trp Ser Thr Val Leu Leu Gly Ala Gly Ile Leu Phe Thr Gly
    210                 215                 220

Glu Leu Trp Glu Phe Leu Ser Phe Ala Glu Arg Tyr Pro Thr Ile Ile
225                 230                 235                 240

Tyr Asn Ile Leu Leu Phe Gly Leu Thr Ser Ala Leu Gly Gln Ser Phe
                245                 250                 255

Ile Phe Met Thr Val Val Tyr Phe Gly Pro Leu Thr Cys Ser Ile Ile
            260                 265                 270

Thr Thr Thr Arg Lys Phe Phe Thr Ile Leu Ala Ser Val Ile Leu Phe
        275                 280                 285

Ala Asn Pro Ile Ser Ser Met Gln Trp Val Gly Thr Val Leu Val Phe
    290                 295                 300

Leu Gly Leu Gly Leu Asp Ala Lys Phe Gly Lys Gly Thr Lys Lys Thr
305                 310                 315                 320

Ser His

<210> 32
<211> 1156
<212> DNA
<213> Rattus norvegicus

<220>
<223> UDP galactose transporter

<400> 32
cctggagctt tcgccttcgc ctccggtacc gctacctgtt ctgaacggat ctccggccga 60
ctcgtccctg cgtctcatgg ccgctagcag atccctggtg cccgaccggc tgcgcctacc 120
actctgcttc ttgggtgtct ttgtctgcta cttctactat gggatcctgc aggagaagat 180
aacaagagga aagtatggag aaggacccaa acaggagaca ttcacctttg ccttaacttt 240
ggttttcatc cagtgtgtga tcaatgctat gtttgccaag atcttgatcc agttttttga 300
cactgccagg gtggatcgca ctcggacctg gctctatgct gcctgctctg tctcctatgt 360
gggcgccatg gtctccagca actcagcact acagtttgtc aactatccaa ctcaggtcct 420
tggtaaatcc tgcaagccaa tcccagttat gctcctcgga gtgaccctct tgaagaagaa 480
gtacccattg gccaagtacc tgtgtgtgtt gctaattgtg gctggcgtgg ctcttttcat 540
gtataagccc aagaaggtgg ttgggataga agagcacacg gtcggctttg gagagctcct 600
tctgctcttg tctctgaccc tggatggact gacaggtgtt tcccaggacc atatgcgggc 660

tcattaccaa acaggttcca atcacatgat gttgaacatc aacctttggt ccacggtctt 720
gctcggtgct gggatcctgt ttactgggga gctctgggag ttcttgagtt tcgccgagag 780
gtacccgacc atcatctata acatcctgct ctttggcttg accagtgcct tgggtcagag 840
ctttatcttc atgacagtcg tgtacttcgg ccccctgacc tgctccatca tcaccacgac 900
tcggaagttc ttcaccatct tggcttctgt gatcctcttt gccaatccca tcagctccat 960
gcagtgggtg ggcaccgtgc tggttttcct gggtctgggt cttgatgcca agtttgggaa 1020
aggaacaaag aagacctccc actaggaaaa gagaggcttc ctccactcca gaaacactta 1080
aattattatc tccaacagtg acatcttggg aaaatggact cagtcacgat aagggactgg 1140
gttccaatct ttttat 1156

<210> 33
<211> 635
<212> PRT
<213> Rattus norvegicus

<220>
<223> CHOT1

<400> 33
Met Ala Lys Lys Ser Ala Glu Asn Gly Ile Tyr Ser Val Ser Gly Asp
1               5                   10                  15

Glu Lys Lys Gly Pro Leu Ile Val Ser Gly Pro Asp Gly Ala Pro Ser
            20                  25                  30

Lys Gly Asp Gly Pro Ala Gly Leu Gly Ala Pro Ser Ser Arg Leu Ala
        35                  40                  45

Val Pro Pro Arg Glu Thr Trp Thr Arg Gln Met Asp Phe Ile Met Ser
    50                  55                  60

Cys Val Gly Phe Ala Val Gly Leu Gly Asn Val Trp Arg Phe Pro Tyr
65                  70                  75                  80

Leu Cys Tyr Lys Asn Gly Gly Gly Val Phe Leu Ile Pro Tyr Val Leu
                85                  90                  95

Ile Ala Leu Val Gly Gly Ile Pro Ile Phe Phe Leu Glu Ile Ser Leu
            100                 105                 110

Gly Gln Phe Met Lys Ala Gly Ser Ile Asn Val Trp Asn Ile Cys Pro
        115                 120                 125

Leu Phe Lys Gly Leu Gly Tyr Ala Ser Met Val Ile Val Phe Tyr Cys
    130                 135                 140

Asn Thr Tyr Tyr Ile Met Val Leu Ala Trp Gly Phe Tyr Tyr Leu Val
145                 150                 155                 160

Lys Ser Phe Thr Thr Thr Leu Pro Trp Ala Thr Cys Gly His Thr Trp
                165                 170                 175

Asn Thr Pro Asp Cys Val Glu Ile Phe Arg His Glu Asp Cys Ala Asn
            180                 185                 190

Ala Ser Leu Ala Asn Leu Thr Cys Asp Gln Leu Ala Asp Arg Arg Ser
        195                 200                 205

Pro Val Ile Glu Phe Trp Glu Asn Lys Val Leu Arg Leu Ser Thr Gly
    210                 215                 220

Leu Glu Val Pro Gly Ala Leu Asn Trp Glu Val Thr Leu Cys Leu Leu
225                 230                 235                 240

Ala Cys Trp Val Leu Val Tyr Phe Cys Val Trp Lys Gly Val Lys Ser
                245                 250                 255

Thr Gly Lys Ile Val Tyr Phe Thr Ala Thr Phe Pro Tyr Val Val Leu
            260                 265                 270

Val Val Leu Leu Val Arg Gly Val Leu Leu Pro Gly Ala Leu Asp Gly
        275                 280                 285

Ile Ile Tyr Tyr Leu Lys Pro Asp Trp Ser Lys Leu Gly Ser Pro Gln
    290                 295                 300

Val Trp Ile Asp Ala Gly Thr Gln Ile Phe Phe Ser Tyr Ala Ile Gly
305                 310                 315                 320

Leu Gly Ala Leu Thr Ala Leu Gly Ser Tyr Asn Arg Phe Asn Asn Asn
                325                 330                 335

Cys Tyr Lys Asp Ala Ile Ile Leu Ala Leu Ile Asn Ser Gly Thr Ser
            340                 345                 350

Phe Phe Ala Gly Phe Val Val Phe Ser Ile Leu Gly Phe Met Ala Thr
        355                 360                 365

Glu Gln Gly Val His Ile Ser Lys Val Ala Glu Ser Gly Pro Gly Leu
    370                 375                 380

Ala Phe Ile Ala Tyr Pro Arg Ala Val Thr Leu Met Pro Val Ala Pro
385                 390                 395                 400

Leu Trp Ala Ala Leu Phe Phe Phe Met Leu Leu Leu Leu Gly Leu Asp
                405                 410                 415

Ser Gln Phe Val Gly Val Glu Gly Phe Ile Thr Gly Leu Leu Asp Leu
            420                 425                 430

Leu Pro Ala Ser Tyr Tyr Phe Arg Phe Gln Arg Glu Ile Ser Val Ala
        435                 440                 445

Leu Cys Cys Ala Leu Cys Phe Val Ile Asp Leu Ser Met Val Thr Asp
    450                 455                 460

Gly Gly Met Tyr Val Phe Gln Leu Phe Asp Tyr Tyr Ser Ala Ser Gly
465                 470                 475                 480

Thr Thr Leu Leu Trp Gln Ala Phe Trp Glu Cys Val Val Val Ala Trp
                485                 490                 495

Val Tyr Gly Ala Asp Arg Phe Met Asp Asp Ile Ala Cys Met Ile Gly
            500                 505                 510

Tyr Arg Pro Cys Pro Trp Met Lys Trp Cys Trp Ser Phe Phe Thr Pro
        515                 520                 525

Leu Val Cys Met Gly Ile Phe Ile Phe Asn Val Val Tyr Tyr Lys Pro
    530                 535                 540

Leu Val Tyr Asn Asn Thr Tyr Val Tyr Pro Trp Trp Gly Glu Ala Met
545                 550                 555                 560

Gly Trp Ala Phe Ala Leu Ser Ser Met Leu Cys Val Pro Leu His Leu
                565                 570                 575

Leu Gly Cys Leu Leu Arg Ala Lys Gly Thr Met Ala Glu Arg Trp Gln
            580                 585                 590

His Leu Thr Gln Pro Ile Trp Gly Leu His His Leu Glu Tyr Arg Ala
        595                 600                 605

Gln Asp Ala Asp Val Arg Gly Leu Thr Thr Leu Thr Pro Val Ser Glu
    610                 615                 620

Ser Ser Lys Val Val Val Val Glu Ser Val Met
625                 630                 635


<210> 34
<211> 3972
<212> DNA
<213> Rattus norvegicus

<220>
<223> CHOT1

<400> 34
gggagttacc acacggtctt ccaagggcac tgccaaggat ggagtttcta ggacggtcag 60
ggtagcattt gcggaaacag agccagctga gttctgggca gtgcagctgt agacccccat 120
gtcgtcgact ttcacatcag tgatgaagaa cacatcgtca tctggcataa cgtgcatgcg 180
tcgctctcga gctgcaggga aatcggtgcc tccatccttc tgccaggcaa tctgagggtt 240
agggtggccc gtggcagcac actcgaggcg tgctgtggtg cccgtccgaa tggcaatgtc 300
atggggtatt ttggtgaatg aaggcaacac attcacagtg agcctggctt tgtgtgagta 360
tgtggagccg aagtggtttg tgatgacaca ctggtagcgg ccctcgtgtc caaacgtgac 420
gtgacggagg tgcaggatgg tggtgtactc catcacttcg ccgtcccgca cccctcccgc 480
ccgcccgtcg ccccgtcccc cggcccggcc gcccccgagg cccccgggcg ggcccgcgct 540
ttcggggccc tttctccagg tgccaccgga gccccttgcc cgctcgccac cctcgagacg 600
ccgcgacccc tggcctggcc tcgcggccca ggggccatgg cgaagaagag cgctgaaaac 660
ggtatctata gcgtgtctgg agacgagaag aagggtcctc tcatcgtgtc cgggcccgat 720
ggtgccccgt ccaagggcga tggccctgcg ggcctggggg cgcccagcag ccgccttgct 780
gtgccgccgc gagagacttg gacacgccag atggacttca tcatgtcgtg cgtgggcttc 840
gccgtgggcc tcggtaacgt gtggcgcttc ccctacctgt gctacaagaa cggcggaggt 900
gtgttcctta ttccctatgt cctgattgcc ctggttggag gaatccccat tttcttcctg 960
gaaatctcac tgggccagtt catgaaggcc ggcagcatca atgtctggaa catctgtccc 1020
ctattcaaag gtctgggcta tgcctccatg gtgattgtct tctactgcaa cacttactac 1080
atcatggtgc tggcctgggg cttctattac ctggtcaagt cctttactac cactttgcca 1140
tgggctacgt gtggccacac ctggaacact cctgactgtg tagagatctt tcgccatgaa 1200
gactgtgcca atgccagctt ggccaacctc acatgtgacc agcttgctga ccgacggtcc 1260
cctgtcatcg agttctggga gaacaaagtc ttgaggctct ccacagggct ggaggttcca 1320
ggagccctca actgggaagt gaccctgtgc ctgctggcct gctgggtgct ggtctacttc 1380
tgtgtgtgga agggggtcaa gtcaacagga aagatcgtgt acttcactgc tacattcccc 1440
tacgtggtcc tcgttgtgct gctggtgcga ggggtgctgc tgcctggagc cctggatggt 1500
atcatctact atctcaagcc tgactggtca aagctagggt cccctcaggt atggatagat 1560
gctgggaccc agattttctt ctcttatgcc atcggcctgg gggccctcac agccctaggc 1620
agctacaatc gcttcaacaa caactgctac aaggatgcca tcatcctggc actcatcaac 1680
agcgggacca gcttctttgc tggctttgtg gtcttctcca tcctgggctt catggccaca 1740
gagcagggtg tgcatatctc caaggtggca gaatcagggc ctggtctagc cttcattgcc 1800
tacccacggg ctgtcacact gatgcctgtg gccccactct gggctgcctt gttcttcttc 1860
atgctgctgc tgctcggtct ggacagccag tttgtaggtg tggaggcttt catcactggg 1920
ctcctggatc tcctcccggc ctcctactac ttccgttttc aaagggagat ctccgtggcc 1980

ctctgttgtg ccctctgctt tgtcatcgat ctctccatgg tgactgatgg cgggatgtac 2040
gtcttccagc tgtttgacta ctactcagct agtggcacta ccctgctttg gcaagccttt 2100
tgggagtgtg tggtggtggc ttgggtttat ggagctgacc gcttcatgga cgatattgcc 2160
tgtatgattg ggtaccgacc ttgcccttgg atgaaatggt gctggtcctt cttcacccca 2220
ctggtctgca tgggcatctt catcttcaat gttgtgtact acaagccact ggtctacaac 2280
aatacctacg tgtacccatg gtggggtgaa gctatgggct gggcctttgc actctcttct 2340
atgctctgtg tgcccctcca cctcctgggc tgtctcctca gggcaaaagg aaccatggca 2400
gagcgatggc agcacctgac ccagcctatc tggggcctcc atcacttgga atacagagct 2460
caggatgcag atgtcagggg cctgaccacc ctgactcccg tgtccgagag cagcaaggtc 2520
gtcgtggtgg agagtgtcat gtgacaggca cccccagctc acatcaccag ctcatccttt 2580
ggtagccata gcagccccag ctttagcccc cacccatccc tcgggggctt gcctttcctt 2640
gacagtttgg ggatctgcct ggggaaaggg aggaagcacc atgagtgcta acaaaaacaa 2700
ctttttccat ttttaataaa acgccaaaaa catcacaacc caccaaaaat agatgcctct 2760
cctccccacc ccacaactac gctggtattt tcgttgcttc cagtccccac ctcctttcct 2820
tcttcccagt gcccaggacc catctcctag ccttgccaag cccatctgcc tgcctcccaa 2880
gctctgctct gtaacacacc tttgccctag aaccaacagt ggcagcttgg ggaacaggag 2940
aggagggaaa ataacagtag gacaggaggg gaggcaagag agggacagtg gaaccaaggt 3000
gaatatttca gctcaattag aactcttccc gcccctattc tagaagctag agagccagtc 3060
agcaatggaa ccttgtgttc ctgcgccaat tgccaccaat attaattgtg tgagcttggg 3120
tctgagtgca cgtctgtctc tgtgtgatgt gtgtgtgtgt gtgtgtgtgt gtgtgtgtgt 3180
gtgtgtgtgt gtgtgtgtgt agatctctta tctcttagtg aaggtgaata ccagaagtaa 3240
acagagcctc tgggcaaagg aggcttatat tttgcacatt ttataagaaa aaaaaacttg 3300
agagtgagac ttctgcttgt atatttctaa aaagccccca ccatccccca tttccactcc 3360
tttccttgac atccccacct tctctgaccc tagccaagga gtgtgaattt atagacttaa 3420
ttttcatagg caaaacaaaa gcttcaggct gttgaatatg tgagtctgtt tgtgtggatg 3480
ttctgtatgg tctccagcca gagactggag taggaaggtg ggagcctcaa tggtgtcccc 3540
acactgttcc tttgcctcta agtctgtggg gtagaaggct gtaacattat cccagggaat 3600
ctgggtctgc tcacctgaca tgctcagact tgcccacacc cagctccctg ggctgggcat 3660
gattgcaggg atttcccatg gacatagccc agggaaggga tcctgggtat ggcttccact 3720
tccaagctaa aggctgatgt gctttcgaca tctcaagtct tccgtgtagc agcttaaacc 3780
cacatctgtg tcccatcaag cctgaaacag ccaagtagcc attagaaagg ctttcttcac 3840
acagagctgg actaagccag ggaggtgggg ctgggtaagg gtgcaagcct ttggggacat 3900
tcttactgtg ctaaaaaagc cactgcaaac atagcaataa aaacacttcc attttccaaa 3960
aaaaaaaaaa aa 3972

<210> 35
<211> 447
<212> PRT
<213> Rattus norvegicus

<220>
<223> Rab GDI alpha

<400> 35
Met Asp Glu Glu Tyr Asp Val Ile Val Leu Gly Thr Gly Leu Thr Glu
1               5                   10                  15

Cys Ile Leu Ser Gly Ile Met Ser Val Asn Gly Lys Lys Val Leu His
            20                  25                  30

Met Asp Arg Asn Pro Tyr Tyr Gly Gly Glu Ser Ser Ser Ile Thr Pro
        35                  40                  45

Leu Glu Glu Leu Tyr Lys Arg Phe Gln Leu Leu Glu Gly Pro Pro Glu
    50                  55                  60

Ser Met Gly Arg Gly Arg Asp Trp Asn Val Asp Leu Ile Pro Lys Phe
65                  70                  75                  80

Leu Met Ala Asn Gly Gln Leu Val Lys Met Leu Leu Tyr Thr Glu Val

                85                  90                  95

Thr Arg Tyr Leu Asp Phe Lys Val Val Glu Gly Ser Phe Val Tyr Lys
            100                 105                 110

Gly Gly Lys Ile Tyr Lys Val Pro Ser Thr Glu Thr Glu Ala Leu Ala
        115                 120                 125

Ser Asn Leu Met Gly Met Phe Glu Lys Arg Arg Phe Arg Lys Phe Leu
    130                 135                 140

Val Phe Val Ala Asn Phe Asp Glu Asn Asp Pro Lys Thr Phe Glu Gly
145                 150                 155                 160

Val Asp Pro Gln Thr Thr Ser Met Arg Asp Val Tyr Arg Lys Phe Asp
                165                 170                 175

Leu Gly Gln Asp Val Ile Asp Phe Thr Gly His Ala Leu Ala Leu Tyr
            180                 185                 190

Arg Thr Asp Asp Tyr Leu Val Gln Pro Cys Leu Glu Thr Ile Asn Arg
        195                 200                 205

Ile Lys Leu Tyr Ser Glu Ser Leu Ala Arg Tyr Gly Lys Ser Pro Tyr
    210                 215                 220

Leu Tyr Pro Leu Tyr Gly Leu Gly Glu Leu Pro Gln Gly Phe Ala Arg
225                 230                 235                 240

Leu Ser Ala Ile Tyr Gly Gly Thr Tyr Met Leu Asn Lys Pro Val Asp
                245                 250                 255

Asp Ile Ile Met Glu Asn Gly Lys Val Val Gly Val Lys Ser Glu Gly
            260                 265                 270

Glu Val Ala Arg Cys Lys Gln Leu Ile Cys Asp Pro Ser Tyr Ile Pro
        275                 280                 285

Asp Arg Val Arg Lys Ala Gly Gln Val Ile Arg Ile Ile Cys Ile Leu
    290                 295                 300

Ser His Pro Ile Lys Asn Thr Asn Asp Ala Asn Ser Cys Gln Ile Ile
305                 310                 315                 320

Ile Pro Gln Asn Gln Val Asn Arg Lys Ser Asp Ile Tyr Val Cys Met
                325                 330                 335

Ile Ser Tyr Ala His Asn Val Ala Ala Gln Gly Lys Tyr Ile Ala Ile
            340                 345                 350

Ala Ser Thr Thr Val Glu Thr Ala Glu Pro Glu Lys Glu Val Glu Pro
        355                 360                 365

Ala Leu Glu Leu Leu Glu Pro Ile Asp Gln Lys Phe Val Ala Ile Ser
    370                 375                 380

Asp Leu Tyr Glu Pro Ile Asp Asp Gly Ser Glu Ser Gln Val Phe Cys
385                 390                 395                 400

Ser Cys Ser Tyr Asp Ala Thr Thr His Phe Glu Thr Thr Cys Asn Asp
                405                 410                 415

Ile Lys Asp Ile Tyr Lys Arg Met Ala Gly Ser Ala Phe Asp Phe Glu
            420                 425                 430

Asn Met Lys Arg Lys Gln Asn Asp Val Phe Gly Glu Ala Asp Gln
        435                 440                 445


<210> 36
<211> 1401
<212> DNA
<213> Rattus norvegicus

<220>
<223> Rab GDI alpha

<400> 36
aagagcgggc gctggtacca aggcctgacc atggatgagg aatacgatgt gattgtgctg 60
gggacaggtc ttaccgaatg catcctgtct ggcatcatgt ctgtgaatgg aaagaaggtg 120
ttgcacatgg accgaaaccc ctactatggg ggtgagagct cttctatcac accccttgag 180
gagctatata agcgctttca gttgctggaa gggccccctg aatcaatggg ccggggccga 240
gactggaatg ttgacttgat ccccaaattc ctcatggcca atggtcagct ggtaaagatg 300
ctactgtata cagaagtgac tcgttatctg gacttcaagg tggtagaggg cagctttgta 360
tacaaagggg gcaagatcta caaagtgcca tctactgaga ctgaggcctt ggcttctaat 420
ctgatgggca tgtttgaaaa acgacgcttc cgaaaatttt tggtgtttgt ggcaaacttt 480
gatgagaatg accccaaaac ctttgagggt gttgacccgc agaccaccag catgcgtgat 540
gtctaccgga agtttgacct gggccaagat gtcattgatt tcactggcca tgccctggcg 600
ctctaccgca ctgatgatta cctggttcag ccatgtcttg agactattaa ccgcatcaag 660
ttgtacagtg agtccctggc ccggtatggc aagagcccat atttgtaccc actctatggc 720
ctgggtgagc tgcctcaggg ctttgccaga ttgagtgcca tctatggggg aacatacatg 780
ctgaacaaac cagtggatga catcatcatg gaaaatggca aggtggtggg tgtaaaatct 840
gagggagagg tggcccgctg caagcagctg atctgtgatc ccagctacat cccagaccgt 900
gtgcgaaagg ctggccaggt tattcgcatc atctgcatcc tcagccaccc catcaagaat 960
accaatgatg ccaattcctg ccaaattatc atccctcaga atcaggtcaa caggaagtca 1020
gacatctatg tgtgcatgat ctcctatgca cacaacgtgg ccgcacaagg caaatacatc 1080
gccattgcca gcaccaccgt agagactgca gaaccagaaa aggaggttga gcctgcattg 1140
gagctgttgg aacctattga ccaaaagttt gtggctatca gtgatttata tgagcccatt 1200
gatgacggtt cagagagtca ggtgttctgt tcctgctcct atgatgctac cacacacttt 1260
gagacaacct gcaatgacat caaagatatc tacaaacgca tggctggttc tgcctttgat 1320
tttgagaaca tgaagcgcaa acagaatgat gtctttggag aagccgatca gtgattgctg 1380
actcccctcc tccagcccct g                                           1401


<210> 37
<211> 368
<212> PRT
<213> Rattus norvegicus

<220>
<223> Core histone

<400> 37
Met Ser Ser Arg Gly Gly Lys Lys Lys Ser Thr Lys Thr Ser Arg Ser
  1               5                  10                  15

Ala Lys Ala Gly Val Ile Phe Pro Val Gly Arg Met Leu Arg Tyr Ile
             20                  25                  30

Lys Lys Gly His Pro Lys Tyr Arg Ile Gly Val Gly Ala Pro Val Tyr
         35                  40                  45

Met Ala Ala Val Leu Glu Tyr Leu Thr Ala Glu Ile Leu Glu Leu Ala
    50                  55                  60

Gly Asn Ala Ala Arg Asp Asn Lys Lys Gly Arg Val Thr Pro Arg His
 65                  70                  75                  80

Ile Leu Leu Ala Val Ala Asn Asp Glu Glu Leu Asn Gln Leu Leu Lys
                 85                  90                  95

Gly Val Thr Ile Ala Ser Gly Gly Val Leu Pro Asn Ile His Pro Glu
            100                 105                 110

Leu Leu Ala Lys Lys Arg Gly Ser Lys Gly Lys Leu Glu Ala Ile Ile
        115                 120                 125

Thr Pro Pro Pro Ala Lys Lys Ala Lys Ser Pro Ser Gln Lys Lys Pro
    130                 135                 140

Val Ala Lys Lys Thr Gly Gly Lys Lys Gly Ala Arg Lys Ser Lys Lys
145                 150                 155                 160

Gln Gly Glu Val Ser Lys Ala Ala Ser Ala Asp Ser Thr Thr Glu Gly
                165                 170                 175

Ala Pro Thr Asp Gly Phe Thr Val Leu Ser Thr Lys Ser Leu Phe Leu
            180                 185                 190

Gly Gln Lys Leu Gln Val Val Gln Ala Asp Ile Ala Ser Ile Asp Ser
        195                 200                 205

Asp Ala Val Val His Pro Thr Asn Thr Asp Phe Tyr Ile Gly Gly Glu
    210                 215                 220

Val Gly Ser Thr Leu Glu Lys Lys Gly Gly Lys Glu Phe Val Glu Ala
225                 230                 235                 240

Val Leu Glu Leu Arg Lys Lys Asn Gly Pro Leu Glu Val Ala Gly Ala
                245                 250                 255

Ala Val Ser Ala Gly His Gly Leu Pro Ala Lys Phe Val Ile His Cys
            260                 265                 270

Asn Ser Pro Val Trp Gly Ser Asp Lys Cys Glu Glu Leu Leu Glu Lys
        275                 280                 285

Thr Val Lys Asn Cys Leu Ala Leu Ala Asp Asp Arg Lys Leu Lys Ser
    290                 295                 300

Ile Ala Phe Pro Ser Ile Gly Ser Gly Arg Asn Gly Phe Pro Lys Gln
305                 310                 315                 320

Thr Ala Ala Gln Leu Ile Leu Lys Ala Ile Ser Ser Tyr Phe Val Ser
                325                 330                 335

Thr Met Ser Ser Ser Ile Lys Thr Val Tyr Phe Val Leu Phe Asp Ser
            340                 345                 350

Glu Ser Ile Gly Ile Tyr Val Gln Glu Met Ala Lys Leu Asp Ala Asn
        355                 360                 365

<210> 38
<211> 1172
<212> DNA
<213> Rattus norvegicus

<220>
<223> Core histone

<400> 38
cgtgagcttg ctgatctgtt tctggggccg tcgccatgtc gagccgtggt gggaagaaga 60
aatccaccaa gacctcccgg tcagccaagg ccggggtcat cttccccgtg ggacggatgc 120
tgcggtacat caagaaaggc caccctaagt atcggatcgg agtgggggcg cctgtgtaca 180
tggctgccgt actggaatac ctgaccgctg agatcctgga gctggccggc aatgcagcga 240
gagacaacaa gaaggggcgg gtcacgccgc ggcacatcct gttagcagtg gccaatgatg 300
aggagctaaa ccagctgcta aaaggagtca ccatagccag tgggggcgtg ttgccgaaca 360
tccatcctga gttgctagcg aagaaacgag gatccaaggg aaaattggaa gccatcatca 420
cgcctccacc agccaaaaag gccaagtctc catcccagaa gaagccagtg gctaagaaaa 480
caggaggcaa gaaaggggcc cggaagtcta agaagcaggg agaagtcagc aaggcagcca 540
gcgcagacag tacaacggag ggcgcgccta cagacggctt tactgtcctc tctaccaaga 600
gcctcttcct cggccagaag ttgcaagttg ttcaggctga cattgcctcg atcgacagtg 660
atgctgtcgt tcacccgaca aacactgact tctacatcgg tggtgaagta ggaagcacac 720
tggagaagaa aggtggcaag gagtttgtag aagctgttct ggaacttcgg aaaaagaacg 780
ggcccttgga agtagctgga gctgctgtta gtgcaggcca cggcctgcct gccaagtttg 840
tgatccactg taacagtcct gtctggggct cagacaaatg tgaagaactt ctagaaaaga 900
cggtgaaaaa ctgcctggct ctagctgatg acagaaagct gaagtccatc gccttcccgt 960
ctattggcag tggcaggaac gggttcccga agcagacagc cgcccaactc attctgaagg 1020
ccatctccag ctactttgtg tccactatgt cgtcctccat caaaactgtg tactttgtgc 1080
tttttgacag tgagagcata ggcatctacg tgcaggaaat ggccaaactg gatgccaact 1140
aggccagcgt tccctagagc cagcacatgt cg 1172

<210> 39
<211> 749
<212> PRT
<213> Mus musculus

<220>
<223> Stat1

<400> 39
Met Ser Gln Trp Phe Glu Leu Gln Gln Leu Asp Ser Lys Phe Leu Glu
1               5                   10                  15

Gln Val His Gln Leu Tyr Asp Asp Ser Phe Pro Met Glu Ile Arg Gln
            20                  25                  30

Tyr Leu Ala Gln Trp Leu Glu Lys Gln Asp Trp Glu His Ala Ala Tyr
        35                  40                  45

Asp Val Ser Phe Ala Thr Ile Arg Phe His Asp Leu Leu Ser Gln Leu
    50                  55                  60

Asp Asp Gln Tyr Ser Arg Phe Ser Leu Glu Asn Asn Phe Leu Leu Gln
65                  70                  75                  80

His Asn Ile Arg Lys Ser Lys Arg Asn Leu Gln Asp Asn Phe Gln Glu
                85                  90                  95

Asp Pro Val Gln Met Ser Met Ile Ile Tyr Asn Cys Leu Lys Glu Glu
            100                 105                 110

Arg Lys Ile Leu Glu Asn Ala Gln Arg Phe Asn Gln Ala Gln Glu Gly
        115                 120                 125

Asn Ile Gln Asn Thr Val Met Leu Asp Lys Gln Lys Glu Leu Asp Ser
    130                 135                 140

Lys Val Arg Asn Val Lys Asp Gln Val Met Cys Ile Glu Gln Glu Ile
145                 150                 155                 160

Lys Thr Leu Glu Glu Leu Gln Asp Glu Tyr Asp Phe Lys Cys Lys Thr
                165                 170                 175

Ser Gln Asn Arg Glu Gly Glu Ala Asn Gly Val Ala Lys Ser Asp Gln
            180                 185                 190

Lys Gln Glu Gln Leu Leu Leu His Lys Met Phe Leu Met Leu Asp Asn
        195                 200                 205

Lys Arg Lys Glu Ile Ile His Lys Ile Arg Glu Leu Leu Asn Ser Ile
    210                 215                 220

Glu Leu Thr Gln Asn Thr Leu Ile Asn Asp Glu Leu Val Glu Trp Lys
225                 230                 235                 240

Arg Arg Gln Gln Ser Ala Cys Ile Gly Gly Pro Pro Asn Ala Cys Leu
                245                 250                 255

Asp Gln Leu Gln Thr Trp Phe Thr Ile Val Ala Glu Thr Leu Gln Gln
            260                 265                 270

Ile Arg Gln Gln Leu Lys Lys Leu Glu Glu Leu Glu Gln Lys Phe Thr
        275                 280                 285

Tyr Glu Pro Asp Pro Ile Thr Lys Asn Lys Gln Val Leu Ser Asp Arg
    290                 295                 300

Thr Phe Leu Leu Phe Gln Gln Leu Ile Gln Ser Ser Phe Val Val Glu
305                 310                 315                 320

Arg Gln Pro Cys Met Pro Thr His Pro Gln Arg Pro Leu Val Leu Lys
                325                 330                 335

Thr Gly Val Gln Phe Thr Val Lys Ser Arg Leu Leu Val Lys Leu Gln
            340                 345                 350

Glu Ser Asn Leu Leu Thr Lys Val Lys Cys His Phe Asp Lys Asp Val
        355                 360                 365

Asn Glu Lys Asn Thr Val Lys Gly Phe Arg Lys Phe Asn Ile Leu Gly
    370                 375                 380

Thr His Thr Lys Val Met Asn Met Glu Glu Ser Thr Asn Gly Ser Leu
385                 390                 395                 400

Ala Ala Glu Leu Arg His Leu Gln Leu Lys Glu Gln Lys Asn Ala Gly
                405                 410                 415

Asn Arg Thr Asn Glu Gly Pro Leu Ile Val Thr Glu Glu Leu His Ser

420                 425                 430

Leu Ser Phe Glu Thr Gln Leu Cys Gln Pro Gly Leu Val Ile Asp Leu
        435                 440                 445

Glu Thr Thr Ser Leu Pro Val Val Val Ile Ser Asn Val Ser Gln Leu
    450                 455                 460

Pro Ser Gly Trp Ala Ser Ile Leu Trp Tyr Asn Met Leu Val Thr Glu
465                 470                 475                 480

Pro Arg Asn Leu Ser Phe Phe Leu Asn Pro Pro Cys Ala Trp Trp Ser
                485                 490                 495

Gln Leu Ser Glu Val Leu Ser Trp Gln Phe Ser Ser Val Thr Lys Arg
            500                 505                 510

Gly Leu Asn Ala Asp Gln Leu Ser Met Leu Gly Glu Lys Leu Leu Gly
        515                 520                 525

Pro Asn Ala Gly Pro Asp Gly Leu Ile Pro Trp Thr Arg Phe Cys Lys
    530                 535                 540

Glu Asn Ile Asn Asp Lys Asn Phe Ser Phe Trp Pro Trp Ile Asp Thr
545                 550                 555                 560

Ile Leu Glu Leu Ile Lys Asn Asp Leu Leu Cys Leu Trp Asn Asp Gly
                565                 570                 575

Cys Ile Met Gly Phe Ile Ser Lys Glu Arg Glu Arg Ala Leu Leu Lys
            580                 585                 590

Asp Gln Gln Pro Gly Thr Phe Leu Leu Arg Phe Ser Glu Ser Ser Arg
        595                 600                 605

Glu Gly Ala Ile Thr Phe Thr Trp Val Glu Arg Ser Gln Asn Gly Gly
    610                 615                 620

Glu Pro Asp Phe His Ala Val Glu Pro Tyr Thr Lys Lys Glu Leu Ser
625                 630                 635                 640

Ala Val Thr Phe Pro Asp Ile Ile Arg Asn Tyr Lys Val Met Ala Ala
                645                 650                 655

Glu Asn Ile Pro Glu Asn Pro Leu Lys Tyr Leu Tyr Pro Asn Ile Asp
            660                 665                 670

Lys Asp His Ala Phe Gly Lys Tyr Tyr Ser Arg Pro Lys Glu Ala Pro
        675                 680                 685

Glu Pro Met Glu Leu Asp Asp Pro Lys Arg Thr Gly Tyr Ile Lys Thr
    690                 695                 700

Glu Leu Ile Ser Val Ser Glu Val His Pro Ser Arg Leu Gln Thr Thr
705                 710                 715                 720

Asp Asn Leu Leu Pro Met Ser Pro Glu Glu Phe Asp Glu Met Ser Arg
                725                 730                 735

Ile Val Gly Pro Glu Phe Asp Ser Met Met Ser Thr Val
            740                 745

<210> 40
<211> 2277
<212> DNA
<213> Mus musculus

<220>
<223> Stat1

<400> 40
caggatgtca cagtggttcg agcttcagca gctggactcc aagttcctgg agcaggtcca 60
ccagctgtac gatgacagtt tccccatgga aatcagacag tacctggccc agtggctgga 120
aaagcaagac tgggagcacg ctgcctatga tgtctcgttt gcgaccatcc gcttccatga 180
cctcctctca cagctggacg accagtacag ccgcttttct ctggagaata atttcttgtt 240
gcagcacaac atacggaaaa gcaagcgtaa tctccaggat aacttccaag aagatcccgt 300
acagatgtcc atgatcatct acaactgtct gaaggaagaa aggaagattt tggaaaatgc 360
ccaaagattt aatcaggccc aggagggaaa tattcagaac actgtgatgt tagataaaca 420
gaaggagctg gacagtaaag tcagaaatgt gaaggatcaa gtcatgtgca tagagcagga 480
aatcaagacc ctagaagaat tacaagatga atatgacttt aaatgcaaaa cctctcagaa 540
cagagaaggt gaagccaatg gtgtggcgaa gagcgaccaa aaacaggaac agctgctgct 600
ccacaagatg tttttaatgc ttgacaataa gagaaaggag ataattcaca aaatcagaga 660
gttgctgaat tccatcgagc tcactcagaa cactctgatt aatgacgagc tcgtggagtg 720
gaagcgaagg cagcagagcg cctgcatcgg gggaccgccc aacgcctgcc tggatcagct 780
gcaaacgtgg ttcaccattg ttgcagagac cctgcagcag atccgtcagc agcttaaaaa 840
gctggaggag ttggaacaga aattcaccta tgagcccgac cctattacaa aaaacaagca 900
ggtgttgtca gatcgaacct tcctcctctt ccagcagctc attcagagct ccttcgtggt 960
agaacgacag ccgtgcatgc ccactcaccc gcagaggccc ctggtcttga agactggggt 1020
acagttcact gtcaagtcga gactgttggt gaaattgcaa gagtcgaatc tattaacgaa 1080
agtgaaatgt cactttgaca aagatgtgaa cgagaaaaac acagttaaag gatttcggaa 1140
gttcaacatc ttgggtacgc acacaaaagt gatgaacatg gaagaatcca ccaacggaag 1200
tctggcagct gagctccgac acctgcaact gaaggaacag aaaaacgctg ggaacagaac 1260
taatgagggg cctctcattg tcaccgaaga acttcactct cttagctttg aaacccagtt 1320
gtgccagcca ggcttggtga ttgacctgga gaccacctct cttcctgtcg tggtgatctc 1380
caacgtcagc cagctcccca gtggctgggc gtctatcctg tggtacaaca tgctggtgac 1440
agagcccagg aatctctcct tcttcctgaa ccccccgtgc gcgtggtggt cccagctctc 1500
agaggtgttg agttggcagt tttcatcagt caccaagaga ggtctgaacg cagaccagct 1560
gagcatgctg ggagagaagc tgctgggccc taatgctggc cctgatggtc ttattccatg 1620
gacaaggttt tgtaaggaaa atattaatga taaaaatttc tccttctggc cttggattga 1680
caccatccta gagctcatta agaacgacct gctgtgcctc tggaatgatg ggtgcattat 1740
gggcttcatc agcaaggagc gagaacgcgc tctgctcaag gaccagcagc cagggacgtt 1800
cctgcttaga ttcagtgaga gctcccggga aggggccatc acattcacat gggtggaacg 1860
gtcccagaac ggaggtgaac ctgacttcca tgccgtggag ccctacacga aaaaagaact 1920
ttcagctgtt actttcccag atattattcg caactacaaa gtcatggctg ccgagaacat 1980
accagagaat cccctgaagt atctgtaccc caatattgac aaagaccacg cctttgggaa 2040
gtattattcc agaccaaagg aagcaccaga accgatggag cttgacgacc ctaagcgaac 2100
tggatacatc aagactgagt tgatttctgt gtctgaagtc cacccttcta gacttcagac 2160
cacagacaac ctgcttccca tgtctccaga ggagtttgat gagatgtccc ggatagtggg 2220
ccccgaattt gacagtatga tgagcacagt ataaacacga atttctctct ggcgaca 2277

<210> 41
<211> 2148
<212> PRT
<213> Rattus norvegicus

<220>
<223> Transcription factor IIIC, alpha subunit

<400> 41
Met Asp Ala Leu Glu Ser Leu Leu Asp Glu Val Ala Leu Glu Gly Leu

1                 5                   10                  15

Asp Gly Leu Cys Leu Pro Ala Leu Trp Ser Arg Leu Glu Ser Arg Ser
            20                  25                  30

Pro Ala Phe Pro Leu Pro Leu Glu Pro Tyr Thr Gln Glu Phe Leu Trp
        35                  40                  45

Arg Ala Leu Val Thr His Pro Gly Ile Ser Phe Tyr Glu Glu Pro Arg
    50                  55                  60

Glu Arg Pro Asp Leu Gln Leu Gln Asp Arg Tyr Glu Glu Ile Asp Leu
65                  70                  75                  80

Glu Thr Gly Ile Leu Glu Ser Arg Arg Asp Pro Val Thr Leu Glu Asp
                85                  90                  95

Val Tyr Pro Ile His Met Ile Leu Glu Asn Lys Asp Gly Ile Gln Gly
            100                 105                 110

Ser Cys Arg Tyr Phe Lys Glu Arg Lys Asp Ile Thr Ser Ser Ile Arg
        115                 120                 125

Ser Lys Cys Leu Gln Pro Arg Cys Thr Met Val Glu Ala Phe Ser Arg
    130                 135                 140

Trp Gly Lys Lys Leu Ile Ile Val Ala Ser Gln Asp Met Arg Tyr Arg
145                 150                 155                 160

Ala Leu Ile Gly Leu Glu Gly Asp Pro Asp Leu Lys Leu Pro Asp Phe
                165                 170                 175

Ser Tyr Cys Ile Leu Glu Arg Leu Gly Arg Ser Arg Trp Gln Gly Glu
            180                 185                 190

Leu Gln Arg Asp Leu His Thr Thr Ala Phe Lys Val Asp Ala Gly Lys
        195                 200                 205

Leu His Tyr His Arg Lys Ile Leu Asn Lys Asn Gly Leu Ile Thr Met
    210                 215                 220

Gln Ser His Val Ile Arg Leu Pro Thr Gly Ala Gln Gln His Ser Ile
225                 230                 235                 240

Leu Leu Leu Leu Asn Arg Phe His Val Asp Arg Arg Ser Lys Tyr Asp
                245                 250                 255

Ile Leu Met Glu Lys Leu Ser Met Met Leu Ser Thr Arg Ser Asn Gln
            260                 265                 270

Ile Glu Thr Leu Gly Lys Leu Arg Glu Glu Leu Gly Leu Cys Glu Arg
        275                 280                 285

Thr Phe Lys Arg Leu Tyr Gln Tyr Met Leu Asn Ala Gly Leu Ala Lys
    290                 295                 300

Val Val Ser Leu Pro Leu Gln Glu Ile His Pro Glu Cys Gly Pro Cys
305                 310                 315                 320

Lys Thr Lys Lys Gly Thr Asp Val Met Val Arg Cys Leu Lys Leu Leu
                325                 330                 335

Lys Glu Phe Arg Arg Lys Met Glu Asp Asp His Asp Asp Asp Asp Asp
            340                 345                 350

Glu Glu Ala Ile Ser Lys Ala Val Pro Pro Val Asp Ile Val Phe Glu
        355                 360                 365

Arg Asp Met Leu Thr Gln Thr Tyr Glu Leu Ile Glu Arg Arg Gly Thr
    370                 375                 380

Lys Gly Ile Ser Gln Ala Glu Ile Arg Val Ala Met Asn Val Gly Lys
385                 390                 395                 400

Leu Glu Ala Arg Met Leu Cys Arg Leu Leu Gln Arg Phe Lys Val Val
                405                 410                 415

Lys Gly Phe Met Glu Asp Glu Gly Arg Gln Arg Thr Thr Lys Tyr Ile
            420                 425                 430

Ser Cys Val Phe Ala Glu Glu Ser Asp Leu Ser Arg Gln Tyr Ala Arg
        435                 440                 445

Glu Lys Ala Arg Gly Glu Leu Leu Thr Thr Val Ser Leu Ala Ser Val
    450                 455                 460

Gln Asp Glu Ser Leu Met Pro Glu Gly Glu Glu Ala Phe Leu Ser Asp
465                 470                 475                 480

Ser Glu Ser Glu Glu Glu Ser Ser Cys Ser Gly Lys Arg Arg Gly Arg
                485                 490                 495

Gly Ser Arg Gly His Ser Arg Ala Ser Gly Asp Ala Gly Pro Gly Ser
            500                 505                 510

Arg Pro His His Ser Thr Pro Ala Lys Gly Gly Trp Lys Val Leu Asn
        515                 520                 525

Leu His Pro Leu Lys Lys Pro Lys Ser Ala Ala Val Glu Arg Ser Arg
    530                 535                 540

Arg Ser Ser Ala Cys Arg Asp Gly Leu Asp Thr Ser Ser Ser Ser Glu
545                 550                 555                 560

Leu Asn Ile Pro Phe Asp Pro His Ser Met Asp Ser His Ser Gly Asp
                565                 570                 575

Ile Ala Val Ile Glu Glu Val Arg Leu Asp Asn Pro Lys Glu Gly Gly
            580                 585                 590

Gly Ser Gln Lys Gly Gly Arg His Gly Ser Gly Gln Asp Lys Pro His
        595                 600                 605

Lys Thr Tyr Arg Leu Leu Lys Arg Arg Asn Leu Ile Ile Glu Ala Val
    610                 615                 620

Thr Asn Leu Arg Leu Ile Glu Ser Leu Phe Thr Ile Gln Lys Met Ile
625                 630                 635                 640

Met Asp Gln Glu Lys Gln Glu Gly Val Ser Thr Lys Cys Cys Lys Lys
                645                 650                 655

Ser Ile Ile Arg Leu Val Arg Asn Leu Ser Glu Glu Gly Leu Leu Arg
            660                 665                 670

Leu Tyr Arg Thr Thr Val Ile Gln Asp Gly Ile Lys Lys Lys Val Asp
        675                 680                 685

Leu Val Val His Pro Ser Met Asp Gln Asn Asp Pro Leu Val Arg Ser
    690                 695                 700

Ala Ile Glu Gln Val Arg Phe Arg Ile Ser Asn Ser Ser Thr Ala Asn
705                 710                 715                 720

Arg Val Lys Val Pro Pro Ala Pro Ala Pro Gln Glu Glu Ala Glu Glu
                725                 730                 735

Gly Asn Gln Glu Pro Glu Val Pro Ser Arg Ser Ala Asp Ser Glu Ala
            740                 745                 750

Asn Thr Ser Ser Lys Pro Glu Ser Thr Arg Val Lys Lys Thr Asp Glu
        755                 760                 765

Lys Met Gly Ile Thr Pro Leu Lys Asn Tyr Lys Pro Val Ile Val Pro
    770                 775                 780

Gly Leu Gly Arg Ser Ile Gly Phe Leu Pro Lys Met Pro Arg Leu Arg
785                 790                 795                 800

Val Met His Leu Phe Leu Trp Tyr Leu Val Tyr Gly His Pro Ala Ser
                805                 810                 815

His Thr Gly Glu Gln Pro Thr Phe His Ser Glu Arg Lys Thr Gly Lys
            820                 825                 830

Gln Glu Pro Ser Arg Pro Gly Val Gln Pro Ser Ser Gly Asp Asp Trp
        835                 840                 845

Asp Ser Ser Glu Ala Lys Asn Ser Thr Glu Ser Ser Ser Trp Glu Ala
    850                 855                 860

Glu Met Glu Leu Ser Thr Glu Arg Val Tyr Val Asp Glu Ile Ser Trp
865                 870                 875                 880

Met Arg Tyr Val Pro Pro Ile Pro Ile His Arg Asp Phe Gly Phe Gly
                885                 890                 895

Trp Ala Leu Val Ser Asp Ile Leu Leu Cys Leu Pro Leu Ser Ile Phe
            900                 905                 910

Val Gln Val Val Gln Val Ser Tyr Lys Val Asp Asn Leu Glu Asp Phe
        915                 920                 925

Leu Asn Asp Pro Leu Lys Lys His Thr Leu Ile Arg Phe Leu Pro Arg
    930                 935                 940

His Ile Arg Gln Gln Leu Leu Tyr Lys Arg Arg Tyr Ile Phe Ser Val
945                 950                 955                 960

Val Glu Asn Leu Gln Arg Leu Cys Tyr Met Gly Leu Leu Gln Phe Gly
                965                 970                 975

Pro Thr Glu Lys Phe Gln Asp Lys Asp Gln Val Phe Val Phe Leu Lys

980                 985                 990

Lys Asn Ala Val Ile Val Asp Thr Thr Ile Cys Asp Pro His Tyr Asn
        995                 1000                1005

Leu Ala His Ser Ser Arg Pro Phe Glu Arg Arg Leu Tyr Val Leu Asp
    1010                1015                1020

Ser Met Gln Asp Val Glu Ser Tyr Trp Phe Asp Leu Gln Cys Ile Cys
1025                1030                1035                1040

Leu Asn Thr Pro Leu Gly Val Val Arg Cys Pro Cys Ala Gln Lys Ile
                1045                1050                1055

Cys Pro Asp Pro Gly Ser Asp Pro Glu Gly Ser Leu Arg Lys Glu Gln
            1060                1065                1070

Glu Ser Ala Met Asp Lys His Asn Leu Glu Arg Lys Cys Ala Met Leu
        1075                1080                1085

Glu Tyr Thr Thr Gly Ser Arg Glu Val Val Asp Glu Gly Leu Val Pro
    1090                1095                1100

Gly Asp Gly Leu Gly Ala Ala Gly Leu Asp Ser Ser Phe Tyr Ala His
1105                1110                1115                1120

Leu Lys Arg Asn Trp Val Trp Thr Ser Tyr Ile Ile Asn Lys Ala Arg
                1125                1130                1135

Lys Asn Asn Thr Ser Glu Asn Gly Leu Thr Gly Arg Leu Gln Thr Phe
            1140                1145                1150

Leu Ser Lys Arg Pro Met Pro Leu Gly Ser Gly Gly Ser Gly Arg Leu
        1155                1160                1165

Pro Leu Trp Ser Glu Gly Lys Ala Asp Ala Glu Leu Cys Ala Asp Lys
    1170                1175                1180

Glu Glu His Phe Glu Leu Asp Arg Glu Pro Thr Pro Gly Arg Asn Arg
1185                1190                1195                1200

Lys Val Arg Gly Gly Lys Ser Gln Lys Arg Lys Arg Leu Lys Lys Glu
                1205                1210                1215

Pro Ile Arg Lys Thr Lys Arg Arg Arg Arg Gly Glu His Pro Glu Ala
            1220                1225                1230

Lys Ser Lys Lys Leu Arg Tyr Gln Asp Glu Ala Asp Gln Asn Ala Leu
        1235                1240                1245

Arg Met Met Thr Arg Leu Arg Val Ser Trp Ser Met Gln Glu Asp Gly
    1250                1255                1260

Leu Leu Met Leu Cys Arg Ile Ala Ser Asn Val Leu Asn Thr Lys Val
1265                1270                1275                1280

Lys Gly Pro Phe Val Thr Trp Gln Val Val Arg Asp Ile Leu His Ala
                1285                1290                1295

Thr Phe Glu Glu Ser Leu Asp Lys Thr Ser His Ser Val Gly Arg Arg
            1300                1305                1310

Ala Arg Tyr Ile Val Lys Asn Pro Gln Ala Phe Met Asn Tyr Lys Val
      1315                1320                1325

Cys Leu Ala Glu Val Tyr Gln Asp Lys Ala Leu Val Gly Asp Phe Met
   1330                1335                1340

Ser Arg Lys Asp Asn Tyr Glu Asp Pro Lys Val Cys Ala Lys Glu Phe
1345                1350                1355                1360

Lys Glu Phe Val Glu Lys Leu Lys Glu Lys Phe Ser Ser Gly Leu Arg
               1365                1370                1375

Asn Pro Asn Leu Glu Ile Pro Asp Thr Leu Gln Glu Leu Phe Ala Lys
           1380                1385                1390

Tyr Arg Val Leu Ala Ile Gly Asp Glu Lys Asp Arg Val Arg Lys Glu
      1395                1400                1405

Asp Glu Leu Asn Ser Val Glu Asp Ile His Phe Leu Val Leu Gln Asn
   1410                1415                1420

Leu Ile Gln Ser Thr Leu Ser Leu Ser Asn Ser Gln Ser Asn Ser Cys
1425                1430                1435                1440

Gln Ser Phe Gln Ile Phe Arg Leu Tyr Arg Glu Phe Arg Glu Pro Val
               1445                1450                1455

Leu Val Arg Ala Phe Met Glu Cys Gln Lys Arg Ser Leu Val Asn Arg
           1460                1465                1470

Arg Arg Val Ser His Ser Gln Gly Pro Lys Lys Asn Arg Ala Val Pro
      1475                1480                1485

Phe Val Pro Met Ser Tyr Gln Leu Ser Gln Ser Tyr Tyr Lys Leu Phe
   1490                1495                1500

Thr Trp Arg Phe Pro Thr Thr Val Cys Thr Glu Ser Phe Gln Phe Tyr
1505                1510                1515                1520

Asp Arg Leu Arg Ala Asn Gly Ile Leu Asp Gln Pro Asp His Phe Ser
               1525                1530                1535

Phe Lys Asp Met Asp Ser Asn Asp Pro Ser Ser Asp Leu Val Ala Phe
           1540                1545                1550

Ser Leu Asp Ser Pro Gly Gly His Cys Val Thr Ala Leu Ala Leu Phe
      1555                1560                1565

Ser Leu Gly Leu Leu Ser Val Asp Val Arg Ile Pro Glu Gln Ile Val
   1570                1575                1580

Val Val Asp Ser Ser Met Val Glu Ser Glu Val Met Lys Ser Leu Gly
1585                1590                1595                1600

Lys Asp Gly Gly Leu Asp Asp Asp Asp Glu Glu Glu Asp Leu Asp Glu
               1605                1610                1615

Gly Ser Gly Thr Lys Arg Gln Ser Val Glu Val Lys Ala His Gln Ala
           1620                1625                1630

Ser His Thr Lys Tyr Leu Leu Met Arg Gly Tyr Tyr Thr Val Pro Gly
        1635                1640                1645

Met Val Ser Thr Arg Asn Leu Asn Pro Asn Asp Ser Ile Val Val Asn
    1650                1655                1660

Ser Cys Gln Val Lys Phe Arg Leu Arg Asn Thr Pro Ala Pro Thr His
1665                1670                1675                1680

Leu Gly Pro Thr Gly Pro Thr Ala Thr Pro Leu Glu Glu Leu Gln Ala
                1685                1690                1695

Gly Pro Ser Cys Leu Pro Ala Ser Phe Thr Ser Leu Val Asp Pro Gln
            1700                1705                1710

Leu His Thr Arg Cys Pro Glu Glu Phe Ala His Gln Met Ala Gln Ser
        1715                1720                1725

Gly Tyr Ser Pro Glu Asp Val Ala Ala Ser Leu Glu Ile Leu Gln Ala
    1730                1735                1740

Val Ala Ala Ala Asp Cys Phe Gly Val Asp Arg Glu Lys Leu Ser Arg
1745                1750                1755                1760

Gln Phe Ser Ala Leu Glu Lys Ile Ala Asp Lys Arg Thr Arg Thr Phe
                1765                1770                1775

Leu Asp Tyr Ile Gln Asp Leu Leu Glu Gln Gln Gln Val Met Glu Val
            1780                1785                1790

Gly Gly Asn Thr Val Arg Leu Val Ala Met Ala Ser Ala Gln Pro Trp
        1795                1800                1805

Leu Leu Pro Ser Val Arg Leu Lys Asp Val Glu Ile Asp Thr Lys Ala
    1810                1815                1820

Ser Gly Asp Asp Ser Gln Ser Arg Leu Pro Ala Gly Ser Ser Ile Glu
1825                1830                1835                1840

Asp His Thr Ser Glu Gly Ala Pro Ile Pro Pro Val Ser Ser Asn Gly
                1845                1850                1855

Thr Lys Lys Arg Pro Tyr Cys Ser Ile Gln Ser Pro Glu Thr Asp Ala
            1860                1865                1870

Glu Glu Ala Thr Arg Leu Pro Ala Lys Lys Pro Thr Leu Gln Asp Val
        1875                1880                1885

Cys Val Ala Ala Ser Pro Arg Pro Gly Thr Glu Glu Gln Thr Glu Ala
    1890                1895                1900

Gln Ala Gln Phe Ala Ala Pro Glu Asp Ala Gly Ala Glu Gly Pro Arg
1905                1910                1915                1920

Gln Glu Ser Gln Glu Ser Val Gly Val Ser Gly Leu Glu Gln Leu Gly
                1925                1930                1935

Cys Glu Phe Gln Leu Pro Glu Asn Ser Glu Asp Pro Arg Gly Leu Thr
            1940                1945                1950

Glu Ser Asn Met Ala Gln Val Ala Trp Glu Ser Gly Cys Glu Arg Val

1955                1960                1965

Cys Phe Val Gly Arg Pro Trp Arg Gly Val Asp Gly Arg Leu Asn Met
    1970                1975                1980

Pro Val Cys Lys Gly Met Met Glu Ala Val Leu Tyr His Ile Met Ser
1985                1990                1995                2000

Arg Pro Gly Val Pro Glu Ser Cys Leu Leu Gln Tyr Tyr Gln Gly Val
                2005                2010                2015

Leu Gln Pro Val Ala Val Leu Glu Leu Leu Arg Gly Leu Glu Ser Leu
            2020                2025                2030

Gly Cys Ile Gln Lys Arg Met Leu Lys Lys Pro Ala Ser Val Ser Leu
        2035                2040                2045

Phe Ser Arg Pro Val Val Glu Gly Leu Gly Gln Ala Ser Glu Ala Glu
    2050                2055                2060

Ala Leu Ser Cys Gln Gly Ser Thr Val Thr Phe Tyr Glu Pro Thr Leu
2065                2070                2075                2080

Asp Cys Thr Ile Arg Leu Gly Arg Val Phe Pro His Asp Ile Asn Trp
                2085                2090                2095

Lys Gln Ser Gly Ser Ile Tyr Arg Cys Val Pro Gly Gln Gln Arg Ser
            2100                2105                2110

Leu Cys Pro Cys Leu Ile Val Pro Pro Gly Leu Ser Gln Glu Pro Arg
        2115                2120                2125

Pro Ser His Ser Cys Tyr Gln Ser Ser Ala Gln Pro Ser Thr Gly Val
    2130                2135                2140

Ala Thr Ser Arg
2145


<210> 42
<211> 6878
<212> DNA
<213> Rattus norvegicus

<220>
<223> Transcription factor IIIC, alpha subunit

<400> 42
gccggggctg ctaactgaag aggccatgga cgcgctcgag tcgttgttgg acgaggtggc 60
cctggagggg ctcgacggcc tgtgcctgcc cgcgctgtgg agccgcctgg aatcccgctc 120
gcctgccttc cctttgccgc tggaacccta cacgcaggag tttctgtggc gggccttagt 180
cacgcacccg ggcatcagtt tctacgagga gccgcgagag cgacctgacc tgcagctcca 240
agaccgatat gaagaaatcg acttggaaac tggaattttg gagtccagga gggacccagt 300
cactctggaa gatgtctacc ccattcatat gatcttagag aataaggatg gcatccaggg 360
ctcctgccga tactttaagg agagaaagga catcaccagt agcatcagga gcaagtgttt 420
gcagcctcgt tgtacaatgg tagaagcctt cagcaggtgg ggtaagaagc tcatcattgt 480
ggcttcccag gacatgcgat accgggcctt gataggccta gagggggatc ctgatctgaa 540
gcttcctgat ttctcctact gcattttgga gcggttaggt cgatcgagat ggcaagggga 600
gctgcaacga gaccttcaca ccaccgcttt caaggtcgac gctgggaagc ttcattatca 660
caggaaaatt ttgaacaaga atgggctcat tacaatgcag tcacacgtga tccgactgcc 720
tactggagcc cagcaacact ccatcctcct cctccttaac cggtttcatg tagacaggag 780

gagcaagtac gacatcctca tggagaagct gtctatgatg ctgagcacac ggagcaacca 840
gatcgagact ctgggcaagc tgagggagga gctgggactg tgtgaaagga cgttcaagcg 900
tctgtaccag tacatgctga atgctgggct ggccaaagtg gtgtcccttc ccttgcagga 960
gattcaccct gagtgtggtc cctgtaagac caagaaaggg actgatgtca tggttcggtg 1020
ccttaagctc ctgaaggagt tcaggcgaaa gatggaagac gaccatgacg acgatgacga 1080
tgaagaggcc atctccaagg ctgtacctcc cgtggacatc gtgtttgagc gggacatgct 1140
cacgcagacc tacgagctca ttgagcgcag aggaacgaaa ggaatttccc aagctgagat 1200
ccgagttgct atgaatgtgg gaaaactgga agcaagaatg ctgtgtcggc tccttcagag 1260
gttcaaggtt gtcaagggat tcatggaaga tgaaggccgg cagaggacca ccaagtacat 1320
ttcctgtgta tttgcggagg agagtgactt gagccgccaa tatgcgcgtg aaaaggcccg 1380
aggagagctg ctaaccactg tgagcctcgc ctcagtgcaa gatgaatccc tcatgcctga 1440
gggtgaagag gccttcctct ctgactccga aagtgaggaa gagagcagct gcagtggcaa 1500
gcgtaggggc cgagggtccc gggggcattc aagagcctct ggggatgctg ggcctggctc 1560
tcggcctcat cactccactc cagccaaggg cgggtggaaa gtcctaaatc tgcacccatt 1620
aaagaagccc aaaagtgccg ctgtggaaag atcccgccga agctctgctt gtagggatgg 1680
cctggacacc agcagctcat ccgagctcaa tatacccttt gaccctcaca gcatggacag 1740
tcacagtggt gacatcgctg tgattgagga ggtccggctt gataacccca aggagggtgg 1800
tggctcccag aagggcggac ggcatggctc aggccaggac aagcctcaca aaacataccg 1860
actgctaaag cgcagaaatc tgattataga agctgtcacc aacctccgcc tgattgagag 1920
cctgttcacg attcagaaga tgatcatgga ccaggagaag caagagggtg tctccaccaa 1980
atgctgtaag aagtccatta tccgcttggt gcggaacctg tctgaggagg gtctcttgag 2040
attgtatagg actacagtca ttcaagatgg catcaagaag aaggtagacc tggtcgtgca 2100
cccttccatg gaccagaatg accctcttgt gagaagtgct attgagcagg ttcgcttccg 2160
gatctccaac tcaagcacag caaacagggt taaagttcca ccggccccag caccccaaga 2220
ggaagcagaa gagggaaacc aagaaccaga ggtcccaagt agatcagcag actctgaagc 2280
gaacacatcc tcgaagccag aaagcacacg ggtgaaaaag acagatgaga aaatgggcat 2340
aaccccactc aagaattaca aacctgtcat agttcctgga cttgggcgct ccatagggtt 2400
ccttcccaag atgccacgcc ttcgggtgat gcacctgttc ctgtggtacc tggtgtatgg 2460
gcatcctgcc agccacactg gggaacagcc tactttccac agtgagagga aaacaggcaa 2520
gcaggagccc agcagaccag gggtccagcc ctcctcagga gatgactggg actcctctga 2580
agccaaaaac agcacagaaa gcagctcctg ggaggcagaa atggaactct ccacagagag 2640
agtgtatgtg gatgagatct catggatgcg ctacgtccct cccatcccca tccacagaga 2700
cttcggcttt ggctgggctc ttgtcagtga cattcttctc tgccttcccc tctccatctt 2760
tgtccaagtt gttcaagtca gctacaaggt ggataacctg gaggattttc tgaatgaccc 2820
tctgaagaag cacacactga tccgcttcct ccctaggcac atccggcagc agctactgta 2880
taagaggcgg tatattttct cggtggtgga gaacctgcaa cggctgtgct acatggggct 2940
gctacagttt ggacccacag agaagtttca ggataaagac caggtctttg ttttcctgaa 3000
gaagaatgca gtcattgtgg acaccaccat ctgtgaccca cattacaacc tggcccacag 3060
cagccggcct tttgagagac gtctctacgt cttggattcc atgcaggatg tggaaagcta 3120
ctggtttgac ctgcagtgta tctgcctaaa caccccacta ggtgtggtgc gttgtccatg 3180
tgcacagaag atctgccctg atccgggcag tgacccagaa ggcagcttgc ggaaggagca 3240
agagagcgcc atggacaagc acaacctaga gcgcaagtgt gccatgctgg agtataccac 3300
ggggagccga gaagttgtgg atgaaggctt agtccctggg gatggactgg gagctgctgg 3360
gctggactct agcttctatg cacacctgaa gcgcaactgg gtctggacca gctatatcat 3420
caacaaagcc agaaagaaca acacctccga aaatgggctc acaggaagac tccagacttt 3480
cctgtctaag cgcccaatgc cacttgggtc tggaggcagt ggccgtctgc ctctctggtc 3540
agaggggaaa gctgatgctg agctctgtgc tgacaaagag gaacactttg aactggaccg 3600
agaacctacc cctggcagga accgaaaagt gcgtgggggc aagagtcaga agcggaagcg 3660
gctgaagaag gagcccatca ggaagaccaa gcgaaggaga agaggagagc atccagaagc 3720
caaaagcaaa aaactacgct accaagatga ggctgaccag aacgccctcc ggatgatgac 3780
ccggcttcgg gtttcttggt ccatgcagga ggatgggtta ctaatgctct gccgaattgc 3840
cagcaatgtc ctcaacacca aggtgaaagg accatttgtc acctggcagg tcgtgcgtga 3900
cattttgcat gctacctttg aagagtcttt ggataaaaca tctcactcgg ttggacgaag 3960
agctcgctac atagtcaaaa atccccaggc ctttatgaac tacaaagtct gcctggcaga 4020
agtgtaccaa gataaggcac tcgttggaga tttcatgagt cggaaggata actacgaaga 4080
cccaaaggtc tgtgccaagg agtttaaaga atttgtggag aagctgaaag agaagttcag 4140
ctctggcctg aggaacccca accttgagat cccagacaca ctgcaggaac tctttgccaa 4200
ataccgggtg ttggcaatcg gagatgaaaa ggaccgtgtc aggaaggagg atgaactgaa 4260
tagtgtggag gacatccact tcctggtgct acagaacctg atccagagca cactgtccct 4320
ctctaacagc cagtccaact cttgccagtc cttccagatc ttccgcctct accgggagtt 4380
ccgggagcct gtgctggtgc gggccttcat ggagtgccaa aagaggagcc tggtcaaccg 4440

acgccgtgtc agccactccc agggtcccaa gaagaaccgt gctgtgccct ttgtgcccat 4500
gtcctaccag ctctcccagt catactacaa gcttttcacc tggcgatttc ccaccaccgt 4560
ctgcaccgaa tccttccagt tctatgacag actacgggcc aacggcatac tagaccagcc 4620
tgatcatttt tctttcaagg acatggatag caatgaccct tcaagtgacc tggtggcgtt 4680
ttctttggac agccctggag gacactgtgt gacagccctg gccctcttct ccttgggcct 4740
cctctctgtg gatgtccgaa tcccagagca gattgtggtg gtagacagct ccatggtgga 4800
gagtgaggtc atgaagagct tggggaaaga tggcggcttg gatgatgatg atgaggagga 4860
agacctggat gaaggttcag ggaccaagcg tcagagtgtg gaagtaaagg cccatcaggc 4920
ctcacacacc aagtacctgc tgatgagagg ttactacaca gtgcctggca tggtcagcac 4980
gcgcaacctg aaccccaatg acagcattgt ggtcaattcc tgccaggtta aattccgact 5040
gcgcaatacc cctgcaccca cccatcttgg gcccactggg cccacagcca caccgctaga 5100
agagctccag gctggccctt cctgcctccc tgcctccttt accagcctgg tggatccaca 5160
gctgcacacc cgctgcccag aagagtttgc ccaccagatg gcacagtctg ggtacagccc 5220
tgaggacgtg gctgcgtccc tagagatcct gcaggctgtg gcagctgctg actgcttcgg 5280
ggttgacagg gagaagctga gcagacagtt ctctgccttg gagaagatag cggacaagcg 5340
caccaggacg ttcctggact atatccagga cctcttggaa cagcaacaag tcatggaagt 5400
tggaggcaac actgtgcgcc tagtggccat ggcctctgcc cagccctggc tcctgccttc 5460
agtgcgtctg aaagatgtgg aaatagacac caaggcctca ggggatgaca gccaatctag 5520
actccctgcg ggtcttccca tcgaagacca cacctctgag ggtgcaccaa ttcctccagt 5580
cagctccaat ggcactaaaa agcgccccta ctgctctatt cagagccctg aaactgatgc 5640
tgaggaagct acaaggctcc ctgccaagaa gcccacatta caagatgtct gtgtggctgc 5700
cagtcctagg cctggaacag aagagcagac agaggcccag gctcagtttg cagctcctga 5760
agatgcaggt gcagagggcc ccaggcagga gagccaagag agtgttggtg tctcaggcct 5820
agagcagctg ggctgtgagt tccagcttcc agaaaattct gaagacccca gaggacttac 5880
agagagcaac atggcccagg tagcatggga gagcggctgt gaaagagtat gttttgtcgg 5940
tcgcccatgg cgtggtgtgg acgggcgcct gaacatgccc gtgtgcaaag gcatgatgga 6000
agccgtgctg taccacatca tgtcaaggcc cggtgttcct gagagctgcc tgctacagta 6060
ctaccagggc gtcctacagc ctgttgctgt gcttgagctg ctccggggct tagagtctct 6120
cggctgtatc cagaagcgga tgctgaagaa gccagcgagt gtctcactct tctccaggcc 6180
tgtggtggag ggcctaggtc aggccagtga ggccgaggca ctctcctgtc aggggagcac 6240
tgtcaccttt tatgaaccta ctctggactg taccatccgt ctgggccgtg tcttccccca 6300
tgacatcaac tggaaacaaa gtggatccat ttatagatgc gtcccaggcc agcagaggag 6360
cctctgcccg tgcttaattg ttccgcctgg actgtcccaa gagccccgtc cttcacacag 6420
ctgttaccag agctcggccc agcctagcac aggtgtagca actagcaggt gacttcagtc 6480
ccctctgctc tcggctgctc ttggtcccag gtgcccagcc ttcctgcagc cctgtgtcct 6540
gccctggact caggctctag catcagcagt atctgcaagg gatgttgttt agggagcagg 6600
ctgatgacca agccctgagt cacctcacac gcaccacacc attcagactc agaggaagac 6660
acatcacagg tgtacctggc agagtctgct cctcttaagg tagggcatcc caacttggcc 6720
tgaactgagc tcaggtctcc agctcccaag ggcccctccc gccagaagca aggcctgtag 6780
gttttgcttt tgtttttgtt ttattttgcg ctcttttcac tagatacaag cccctggcat 6840
gtatttattt attttttaat taaagtaaga tgaaatgc 6878

<210> 43
<211> 937
<212> PRT
<213> Homo sapiens

<220>
<223> Lon protease-like protein

<400> 43
Met Leu Ala Ala Ala Gly Gly Arg Val Pro Thr Ala Ala Gly Ala Trp
1               5                   10                  15

Leu Leu Arg Gly Gln Arg Thr Cys Asp Ala Ser Pro Pro Trp Ala Leu
            20                  25                  30

Trp Gly Arg Gly Pro Ala Ile Gly Gly Gln Trp Arg Gly Phe Trp Glu
        35                  40                  45

Ala Ser Ser Arg Gly Gly Gly Ala Phe Ser Gly Gly Glu Asp Ala Ser
    50                  55                  60

Glu Gly Gly Ala Glu Glu Gly Ala Gly Gly Ala Gly Gly Ser Ala Gly
65                  70                  75                  80

Ala Gly Glu Gly Pro Val Ile Thr Ala Leu Thr Pro Met Thr Ile Pro
                85                  90                  95

Asp Val Phe Pro His Leu Pro Leu Ile Ala Ile Thr Arg Asn Pro Val
            100                 105                 110

Phe Pro Arg Phe Ile Lys Ile Ile Glu Val Lys Asn Lys Lys Leu Val
        115                 120                 125

Glu Leu Leu Arg Arg Lys Val Arg Leu Ala Gln Pro Tyr Val Gly Val
    130                 135                 140

Phe Leu Lys Arg Asp Asp Ser Asn Glu Ser Asp Val Val Glu Ser Leu
145                 150                 155                 160

Asp Glu Ile Tyr His Thr Gly Thr Phe Ala Gln Ile His Glu Met Gln
                165                 170                 175

Asp Leu Gly Asp Lys Leu Arg Met Ile Val Met Gly His Arg Arg Val
            180                 185                 190

His Ile Ser Arg Gln Leu Glu Val Glu Pro Glu Glu Pro Glu Ala Glu
        195                 200                 205

Asn Lys His Lys Pro Arg Arg Lys Ser Lys Arg Gly Lys Lys Glu Ala
    210                 215                 220

Glu Asp Glu Leu Ser Ala Arg His Pro Ala Glu Leu Ala Met Glu Pro
225                 230                 235                 240

Thr Pro Glu Leu Pro Ala Glu Val Leu Met Val Glu Val Glu Asn Val
                245                 250                 255

Val His Glu Asp Phe Gln Val Thr Glu Glu Val Lys Ala Leu Thr Ala
            260                 265                 270

Glu Ile Val Lys Thr Ile Arg Asp Ile Ile Ala Leu Asn Pro Leu Tyr
        275                 280                 285

Arg Glu Ser Val Leu Gln Met Met Gln Ala Gly Gln Arg Val Val Asp
    290                 295                 300

Asn Pro Ile Tyr Leu Ser Asp Met Gly Ala Ala Leu Thr Gly Ala Glu
305                 310                 315                 320

Ser His Glu Leu Gln Asp Val Leu Glu Glu Thr Asn Ile Pro Lys Arg
                325                 330                 335

Leu Tyr Lys Ala Leu Ser Leu Leu Lys Lys Glu Phe Glu Leu Ser Lys
            340                 345                 350

Leu Gln Gln Arg Leu Gly Arg Glu Val Glu Glu Lys Ile Lys Gln Thr
        355                 360                 365

His Arg Lys Tyr Leu Leu Gln Glu Gln Leu Lys Ile Ile Lys Lys Glu

370                 375                 380

Leu Gly Leu Glu Lys Asp Asp Lys Asp Ala Ile Glu Glu Lys Phe Arg
385                 390                 395                 400

Glu Arg Leu Lys Glu Leu Val Val Pro Lys His Val Met Asp Val Val
                405                 410                 415

Asp Glu Glu Leu Ser Lys Leu Gly Leu Leu Asp Asn His Ser Ser Glu
            420                 425                 430

Phe Asn Val Thr Arg Asn Tyr Leu Asp Trp Leu Thr Ser Ile Pro Trp
        435                 440                 445

Gly Lys Tyr Ser Asn Glu Asn Leu Asp Leu Ala Arg Ala Gln Ala Val
    450                 455                 460

Leu Glu Glu Asp His Tyr Gly Met Glu Asp Val Lys Lys Arg Ile Leu
465                 470                 475                 480

Glu Phe Ile Ala Val Ser Gln Leu Arg Gly Ser Thr Gln Gly Lys Ile
                485                 490                 495

Leu Cys Phe Tyr Gly Pro Pro Gly Val Gly Lys Thr Ser Ile Ala Arg
            500                 505                 510

Ser Ile Ala Arg Ala Leu Asn Arg Glu Tyr Phe Arg Phe Ser Val Gly
        515                 520                 525

Gly Met Thr Asp Val Ala Glu Ile Lys Gly His Arg Arg Thr Tyr Val
    530                 535                 540

Gly Ala Met Pro Gly Lys Ile Ile Gln Cys Leu Lys Lys Thr Lys Thr
545                 550                 555                 560

Glu Asn Pro Leu Ile Leu Ile Asp Glu Val Asp Lys Ile Gly Arg Gly
                565                 570                 575

Tyr Gln Gly Asp Pro Ser Ser Ala Leu Leu Glu Leu Leu Asp Pro Glu
            580                 585                 590

Gln Asn Ala Asn Phe Leu Asp His Tyr Leu Asp Val Pro Val Asp Leu
        595                 600                 605

Ser Lys Val Leu Phe Ile Cys Thr Ala Asn Val Thr Asp Thr Ile Pro
    610                 615                 620

Glu Pro Leu Arg Asp Arg Met Glu Met Ile Asn Val Ser Gly Tyr Val
625                 630                 635                 640

Ala Gln Glu Lys Leu Ala Ile Ala Glu Arg Tyr Leu Val Pro Gln Ala
                645                 650                 655

Arg Ala Leu Cys Gly Leu Asp Glu Ser Lys Ala Lys Leu Ser Ser Asp
            660                 665                 670

Val Leu Thr Leu Leu Ile Lys Gln Tyr Cys Arg Glu Ser Gly Val Arg
        675                 680                 685

Asn Leu Gln Lys Gln Val Glu Lys Val Leu Arg Lys Ser Ala Tyr Lys
    690                 695                 700

Ile Val Ser Gly Glu Ala Glu Ser Val Glu Val Thr Pro Glu Asn Leu
705                 710                 715                 720

Gln Asp Phe Val Gly Lys Pro Val Phe Thr Val Glu Arg Met Tyr Asp
                725                 730                 735

Val Thr Pro Pro Gly Val Val Met Gly Leu Ala Trp Thr Ala Met Gly
            740                 745                 750

Gly Ser Thr Leu Phe Val Glu Thr Ser Leu Arg Arg Pro Gln Asp Lys
        755                 760                 765

Asp Ala Lys Gly Asp Lys Asp Gly Ser Leu Glu Val Thr Gly Gln Leu
    770                 775                 780

Gly Glu Val Met Lys Glu Ser Ala Arg Ile Ala Tyr Thr Phe Ala Arg
785                 790                 795                 800

Ala Phe Leu Met Gln His Ala Pro Ala Asn Asp Tyr Leu Val Thr Ser
                805                 810                 815

His Ile His Leu His Val Pro Glu Gly Ala Thr Pro Lys Asp Gly Pro
            820                 825                 830

Ser Ala Gly Cys Thr Ile Val Thr Ala Leu Leu Ser Leu Ala Met Gly
        835                 840                 845

Arg Pro Val Arg Gln Asn Leu Ala Met Thr Gly Glu Val Ser Leu Thr
    850                 855                 860

Gly Lys Ile Leu Pro Val Gly Gly Ile Lys Glu Lys Thr Ile Ala Ala
865                 870                 875                 880

Lys Arg Ala Gly Val Thr Cys Ile Ile Leu Pro Ala Glu Asn Lys Lys
                885                 890                 895

Asp Phe Tyr Asp Leu Ala Ala Phe Ile Thr Glu Gly Leu Glu Val His
            900                 905                 910

Phe Val Glu His Tyr Arg Glu Ile Phe Asp Ile Ala Phe Pro Asp Glu
        915                 920                 925

Gln Ala Glu Ala Leu Ala Val Glu Arg
    930                 935


<210> 44
<211> 3407
<212> DNA
<213> Homo sapiens

<220>
<223> Lon protease-like protein

<400> 44
ggcgagcact ggctacgtgc gactgtgggg agcggcgcgg tgctgggtgc tgcggcggcc 60
gatgctggcc gccgccgggg ggcgggttcc cactgcagca ggagcgtggt tgctccgagg 120
ccagcggacc tgcgacgcct ctcctccttg ggcactgtgg ggccgaggcc cggcaattgg 180
gggccaatgg cgggggtttt gggaagcgag cagccgcggc ggaggcgcat tctcgggggg 240
cgaggacgcc tccgagggcg gcgcggagga aggagccggc ggcgcggggg gcagcgcggg 300

cgccggggaa ggcccggtca taacggcgct cacgcccatg acgatccccg atgtgtttcc 360
gcacctgccg ctcatcgcca tcacccgcaa cccggtgttc ccgcgcttta tcaagattat 420
cgaggttaaa aataagaagt tggttgagct gctgagaagg aaagttcgtc tcgcccagcc 480
ttatgtcggc gtctttctaa agagagatga cagcaatgag tcggatgtgg tcgagagcct 540
ggatgaaatc taccacacgg ggacgtttgc ccagatccat gagatgcagg accttgggga 600
caagctgcgc atgatcgtca tgggacacag aagagtccat atcagcagac agctggaggt 660
ggagcccgag gagccggagg cggagaacaa gcacaagccc cgcaggaagt caaagcgggg 720
caagaaggag gcggaggacg agctgagcgc caggcacccg gcggagctgg cgatggagcc 780
cacccctgag ctcccggctg aggtgctcat ggtggaggta gagaacgttg tccacgagga 840
cttccaggtc acggaggagg tgaaagccct gactgcagag atcgtgaaga ccatccggga 900
catcattgcc ttgaaccctc tctacaggga gtcagtgctg cagatgatgc aggctggcca 960
gcgggtggtg gacaacccca tctacctgag cgacatgggc gccgcgctca ccggggccga 1020
gtcccatgag ctgcaggacg tcctggaaga gaccaatatt cctaagcggc tgtacaaggc 1080
cctctccctg ctgaagaagg aatttgaact gagcaagctg cagcagcgcc tgggcgggga 1140
ggtggaggag aagatcaagc agacccaccg taagtacctg ctgcaggagc agctaaagat 1200
catcaagaag gagctgggcc tggagaagga cgacaaggat gccatcgagg agaagttccg 1260
ggagcgcctg aaggagctcg tggtccccaa gcacgtcatg gatgttgtgg acgaggagct 1320
gagcaagctg ggcctgctgg acaaccactc ctcggagttc aatgtcaccc gcaactacct 1380
agactggctc acgtccatcc cttggggcaa gtacagcaac gagaacctgg acctggcgcg 1440
ggcacaggca gtgctggagg aagaccacta cggcatggag gacgtcaaga aacgcatcct 1500
ggagttcatt gccgttagcc agctccgcgg ctccacccag ggcaagatcc tctgcttcta 1560
tggcccccct ggcgtgggta agaccagcat tgctcgctcc atcgcccgcg ccctgaaccg 1620
agagtacttc cgcttcagcg tcgggggcat gactgacgtg gctgagatca agggccacag 1680
gcggacctac gtgggcgcca tgcccgggaa gatcatccag tgtttgaaga agaccaagac 1740
ggagaacccc ctgatcctca tcgacgaggt ggacaagatc ggccgaggct accaggggga 1800
cccgtcgtcg gcactgctgg agctgctgga cccagagcag aatgccaact tcctggacca 1860
ctacctggac gtgcccgtgg acttgtccaa ggtgctgttc atctgcacgg ccaacgtcac 1920
ggacaccatc cccgagccgc tgcgagaccg tatggagatg atcaacgtgt caggctacgt 1980
ggcccaggag aagctggcca ttgcggagcg ctacctggtg ccccaggctc gcgccctgtg 2040
tggcttggat gagagcaagg ccaagctgtc atcggacgtg ctgacgctgc tcatcaagca 2100
gtactgccgc gagagcggtg tccgcaacct gcagaagcaa gtggagaagg tgttacggaa 2160
atcggcctac aagattgtca gcggcgaggc cgagtccgtg gaggtgacgc ccgagaacct 2220
gcaggacttc gtggggaagc ccgtgttcac cgtggagcgc atgtatgacg tgacaccgcc 2280
cggcgtggtc atggggctgg cctggaccgc aatgggaggc tccacgctgt ttgtggagac 2340
atccctgaga cggccacagg acaaggatgc caagggtgac aaggatggca gcctggaggt 2400
gacaggccag ctgggggagg tgatgaagga gagcgcccgc atagcctaca ccttcgccag 2460
agccttcctc atgcagcacg cccccgccaa tgactacctg gtgacctcac acatccacct 2520
gcatgtgccc gagggcgcca cccccaagga cggcccaagc gcaggctgca ccatcgtcac 2580
ggccctgctg tccctggcca tgggcaggcc tgtccggcag aatctggcca tgactggcga 2640
agtctccctc acgggcaaga tcctgcctgt tggtggcatc aaggagaaga ccattgcggc 2700
caagcgcgca ggggtgacgt gcatcatcct gccagccgag aacaagaagg acttctacga 2760
cctggcagcc ttcatcaccg agggcctgga ggtgcacttc gtggaacact accgggagat 2820
cttcgacatc gccttcccgg acgagcaggc agaggcgctg gccgtggaac ggtgacggcc 2880
accccgggac tgcaggcggc ggatgtcagg ccctgtctgg gccagaactg agcgctgtgg 2940
ggagcgcgcc cggacctggc agtggagcca ccgagcgagc agctcggtcc agtgacccag 3000
atcccaggga cctcagtcgg cttaatcaga gtgtggcata gaagctattt aatgattaaa 3060
gtcatttgca gtgggagtta gcatcactaa cctgacagtt gttgccagga atttgctttg 3120
tttactgcta gtatattaga aatcctagat ctcagaatca caatagtaat aaacaacagg 3180
ggtcattttt tcctaactta ctctgtgttc aggtgtggaa tttctgtctc ccaagaggaa 3240
atgtgacttc actttggtgc caatggacag aaaattctac ctgtgctaca taggagaagt 3300
ttggaatgca cttaatagct ggtttttaca ccttgatttc gaggtggaaa gaaattgatc 3360
atgaatctct aataaattta aatctcttaa accaaaaaaa aaaaaaa 3407

<210> 45
<211> 359
<212> PRT
<213> Rattus norvegicus

<220>
<223> GM3 synthase

<400> 45
Met Arg Arg Pro Ser Leu Leu Leu Lys Asp Ile Leu Lys Cys Met Leu
1               5                   10                  15

Val Val Phe Gly Val Trp Leu Leu Tyr Ile Leu Lys Leu Asn Tyr Thr
            20                  25                  30

Ala Glu Glu Cys Asp Met Lys Lys Leu Asn Tyr Val Asp Pro Ala Arg
        35                  40                  45

Ile Lys Arg Ala His Arg Asn Thr Gln Glu Val Phe Gln Lys Glu Cys
    50                  55                  60

Arg Pro Gly His Ala Lys Lys Thr Met Asp Leu Leu Phe Lys Gly Lys
65                  70                  75                  80

Tyr Ser Met Asp Leu Glu Pro Phe Val Gln Lys Ile Pro Thr Ala Ser
                85                  90                  95

Glu Ala Glu Leu Lys Tyr Asp Pro Pro Phe Gly Phe Arg Lys Phe Ser
            100                 105                 110

Ser Lys Val Gln Ser Leu Leu Asp Met Leu Pro Glu His Asp Phe Pro
        115                 120                 125

Glu His Leu Arg Ala Lys His Cys Lys Arg Cys Val Val Ile Gly Asn
    130                 135                 140

Gly Gly Ile Leu His Gly Leu Glu Leu Gly His Ala Leu Asn Gln Phe
145                 150                 155                 160

Asp Val Val Ile Arg Leu Asn Ser Ala Pro Ile Glu Gly Tyr Ser Glu
                165                 170                 175

His Val Gly Asn Lys Thr Thr Ile Arg Met Thr Tyr Pro Glu Gly Ala
            180                 185                 190

Pro Leu Ser Asp Ala Glu Tyr Tyr Ala Asn Asp Leu Phe Val Ala Val
        195                 200                 205

Leu Phe Lys Ser Val Asp Phe Lys Trp Leu Gln Ala Met Val Lys Asn
    210                 215                 220

Glu Ser Leu Pro Phe Trp Ile Arg Leu Phe Phe Trp Lys Gln Val Ala
225                 230                 235                 240

Glu Lys Ile Pro Leu Gln Pro Lys His Phe Arg Ile Leu Asn Pro Val
                245                 250                 255

Ile Ile Lys Glu Thr Ala Phe Asp Ile Leu Gln Tyr Ser Glu Pro Gln
            260                 265                 270

Ser Arg Phe Trp Gly His Asp Lys Asn Ile Pro Thr Ile Gly Ile Ile
        275                 280                 285

Ala Ile Val Leu Ala Thr His Leu Cys Asp Glu Val Ser Leu Ala Gly
    290                 295                 300

Phe Gly Tyr Asp Leu Ser Gln Pro Arg Thr Pro Leu His Tyr Phe Asp
305                 310                 315                 320

Ser Gln Cys Met Gly Ala Met Asn Trp Gln Val Met His Asn Val Thr
                325                 330                 335

Thr Glu Thr Gln Phe Leu Gln Lys Leu Ile Lys Glu Gly Val Val Gln
            340                 345                 350

Asp Leu Ser Gly Gly Ile His
        355

<210> 46
<211> 2364
<212> DNA
<213> Rattus norvegicus

<220>
<223> GM3 synthase

<400> 46
gcggcacctg cctgccgagg tacgtggcgc agcccccagt cagcagagcg gacctcgggg 60
aacccaggca gcagcgatcc gcctttgccc actcataccc caggcccagc agctcctccc 120
cagccctgag acgccggacg cgcctgcgag gggacgccgg cggagggtcg cgaccccggg 180
ccgcctatat gggcgccccc ggcgcagcct gtcgaggtgt gaacgcggtg ctgtggcaat 240
gccaaatgag ttcacctctg caaagctgag aagtgattgc tcaaggacct ccctgcaatg 300
gtacacccag acccagcaca agatgagaag acccagcttg ttattaaaag acatcctcaa 360
gtgcatgttg gttgtgtttg gagtgtggct cctgtacatc ctcaagttaa attacactgc 420
tgaagaatgt gacatgaaaa aactgaatta tgtagaccct gcccgaataa agagagctca 480
tagaaacact caggaagtct ttcagaagga atgtcgtcct gggcacgcga agaaaactat 540
ggatctgtta tttaagggca agtacagcat ggacttggag cctttcgtgc agaagatccc 600
cacagccagt gaagctgagc tcaagtatga cccgcctttt ggattccgga agttctccag 660
taaagtccag agcctcttgg atatgctgcc tgagcacgac tttcctgaac acttgagagc 720
caagcactgc aagcgctgtg tggttattgg gaacgggggc atccttcacg gactagagct 780
gggccacgca ctcaaccagt tcgatgtggt aataaggtta aacagcgcac caattgaggg 840
ttattctgaa catgttggaa ataaaactac tataaggatg acttacccag agggtgctcc 900
actgtcggac gctgaatact acgctaatga cttgttcgtt gctgttttat ttaagagtgt 960
tgatttcaaa tggcttcaag caatggtgaa aaatgaaagc ctgccatttt ggattcgtct 1020
cttcttttgg aagcaggtgg cagaaaaaat cccactccag ccaaagcatt tcaggatttt 1080
gaacccagtt atcatcaaag aaactgcctt cgacatcctt cagtactcag agcctcagtc 1140
aagattctgg ggccatgata agaatatccc cacgatcggc atcattgcca ttgtcttggc 1200
cacgcatctg tgtgatgaag ttagcctggc aggctttgga tatgacctca gtcaaccaag 1260
gacccctctg cactactttg acagtcagtg catgggcgcc atgaactggc aagtcatgca 1320
caacgtgacc acagagaccc agttcctcca gaagctcatc aaggagggcg tggtgcagga 1380
cctcagcggg ggcatccact gagaactttg aacaccgcag acctcactta gcaatgcagc 1440
tgagagctgt ggtgagcagc ctccccaggg agtttaccct gcagctgctt tggcctacag 1500
ctagtgtttt taaactcagt tttaaaagaa aaggaaagaa caacagcaac aacaaaagtt 1560
cagcccttta cacctctttg tcctatttat ttgaagtcag tgttgggttt tgcacaattt 1620
tgtaagttaa ttttaagaat ggggatggaa agacttttca aagagaatcg tatggtttat 1680
tgtattttaa agaagtaatt taatttgcaa aaactgtaca catgtactct ccccaggtgt 1740
tgaggtggga ggagaggggc ttctgcacct tcgatgatgg cctcgatacc tgactgcggg 1800
ttctgcagct ctgtttggta gcactgctgg cagggaaact tcctgcctcc tggatgaagg 1860
aagggtttac tcatcatcac tggcagctgc aggaagtcgg gggcctgata aacaagttca 1920
taaccccttt ctgctagtcc gtctgccaga agactcagag gacccaagca gggcagctgt 1980
tccacaggcg agccactgtc agctgagtac cgtgatgctg ccccaggatg ggagaagcca 2040
tgtcgtccta cactgtcagg gcacttttca gcttctggag aagtgtctgt tctctctgcc 2100
ctcccaaagc acttggtgtt atttaaggcg tgaggctgtc tgtctgagat ggttcatcag 2160
aattgtgaca gccatgggga gcaagacctc tgcagatgac cacattgcta aagcactgga 2220
gtgaaccccc cctgttgtaa cttaagaccc atcctgaagt gccctgaagt gcccaccaac 2280
caatgtgaaa ggagaatgag gaaggggagg gaaaagaaca gaaagaaaga aagaaaaata 2340
ataaaagctt ttgaggagcc cctg                                        2364

<210> 47
<211> 434
<212> PRT
<213> Rattus norvegicus

<220>
<223> Neuron-specific enolase

<400> 47
Met Ser Ile Gln Lys Ile Trp Ala Arg Glu Ile Leu Asp Ser Arg Gly
1               5                   10                  15

Asn Pro Thr Val Glu Val Asp Leu His Thr Ala Lys Gly Leu Phe Arg
            20                  25                  30

Ala Ala Val Pro Ser Gly Ala Ser Thr Gly Ile Tyr Glu Ala Leu Glu
        35                  40                  45

Leu Arg Asp Gly Asp Lys Gln Arg Tyr Leu Gly Lys Gly Val Leu Lys
    50                  55                  60

Ala Val Asp His Ile Asn Ser Thr Ile Ala Pro Ala Leu Ile Ser Ser
65                  70                  75                  80

Gly Leu Ser Val Val Glu Gln Glu Lys Leu Asp Asn Leu Met Leu Glu
                85                  90                  95

Leu Asp Gly Thr Glu Asn Lys Ser Lys Phe Gly Ala Asn Ala Ile Leu
            100                 105                 110

Gly Val Ser Leu Ala Val Cys Lys Ala Gly Ala Ala Glu Lys Asp Leu
        115                 120                 125

Pro Leu Tyr Arg His Ile Ala Gln Leu Ala Gly Asn Ser Asp Leu Ile
    130                 135                 140

Leu Pro Val Pro Ala Phe Asn Val Ile Asn Gly Gly Ser His Ala Gly
145                 150                 155                 160

Asn Lys Leu Ala Met Gln Glu Phe Met Ile Leu Pro Val Gly Ala Glu
                165                 170                 175

Ser Phe Arg Asp Ala Met Arg Leu Gly Ala Glu Val Tyr His Thr Leu
            180                 185                 190

Lys Gly Val Ile Lys Asp Lys Tyr Gly Lys Asp Ala Thr Asn Val Gly
        195                 200                 205

Asp Glu Gly Gly Phe Ala Pro Asn Ile Leu Glu Asn Ser Glu Ala Leu
    210                 215                 220

Glu Leu Val Lys Glu Ala Ile Asp Lys Ala Gly Tyr Thr Glu Lys Met
225                 230                 235                 240

Val Ile Gly Met Asp Val Ala Ala Ser Glu Phe Tyr Arg Asp Gly Lys
                245                 250                 255

Tyr Asp Leu Asp Phe Lys Ser Pro Ala Asp Pro Ser Arg Cys Ile Thr
            260                 265                 270

Gly Asp Gln Leu Gly Ala Leu Tyr Gln Asp Phe Val Arg Asn Tyr Pro
        275                 280                 285

Val Val Ser Ile Glu Asp Pro Phe Asp Gln Asp Asp Trp Ala Ala Trp
    290                 295                 300

Ser Lys Phe Thr Ala Asn Val Gly Ile Gln Ile Val Gly Asp Asp Leu
305                 310                 315                 320

Thr Val Thr Asn Pro Lys Arg Ile Glu Arg Ala Val Glu Glu Lys Ala
                325                 330                 335

Cys Asn Cys Leu Leu Leu Lys Val Asn Gln Ile Gly Ser Val Thr Glu
            340                 345                 350

Ala Ile Gln Ala Cys Lys Leu Ala Gln Glu Asn Gly Trp Gly Val Met
        355                 360                 365

Val Ser His Arg Ser Gly Glu Thr Glu Asp Thr Phe Ile Ala Asp Leu
    370                 375                 380

Val Val Gly Leu Cys Thr Gly Gln Ile Lys Thr Gly Ala Pro Cys Arg
385                 390                 395                 400

Ser Glu Arg Leu Ala Lys Tyr Asn Gln Leu Met Arg Ile Glu Glu Glu
                405                 410                 415

Leu Gly Glu Glu Ala Arg Phe Ala Gly His Asn Phe Arg Asn Pro Ser
            420                 425                 430

Val Leu

<210> 48
<211> 2218
<212> DNA
<213> Rattus norvegicus

<220>
<223> Neuron-specific enolase

<400> 48
cgccgccgcc gtcaccaccg ccaccgccac cggctgagtc tgcagtcctc gaggagatcc 60
cagccatcat gtctatacag aagatctggg cccgagagat cttggactcc cgtgggaatc 120
ccaccgtgga ggtggatctc catactgcca aaggtctttt ccgggctgca gtccccagtg 180
gggcctccac tggcatctat gaggccctgg agctaaggga tggggacaaa cagcgttact 240
taggcaaagg tgtcctgaag gctgtggacc acatcaacag caccatcgca ccggccctca 300
tcagctcagg cctctctgtg gtggagcagg agaagctgga caacctgatg ctggagttgg 360
atgggactga gaacaaatcc aagtttgggg ccaatgccat cctgggtgtg tccctggccg 420
tgtgcaaggc tggggcagcc gagaaggact tgcccctcta tcgccacatt gctcaactgg 480
ctgggaactc cgacctcatc ctgcccgtgc cggcctttaa tgtgatcaac ggtggctctc 540
atgctgggaa caagttggcc atgcaggagt tcatgatcct cccagtgggt gctgagagct 600
ttcgggatgc catgcgactt ggggccgagg tgtaccacac actcaagggg gtcatcaagg 660
acaagtacgg caaggatgcc actaatgtgg gggatgaagg cggctttgcc cccaatatcc 720
tggagaacag cgaagctttg gagctggtga aggaagccat tgacaaggct ggctacacgg 780
aaaagatggt gattggtatg gatgtggctg cctctgagtt ttaccgcgat ggcaaatacg 840
acttggattt caagtctcct gctgaccctt cccgatgcat cactggggac cagcttgggg 900
cactctacca ggactttgtc cggaactatc ctgtggtctc cattgaagac ccattcgacc 960
aggatgactg ggcagcttgg tccaagttca cagccaatgt cggcatccag atagtgggtg 1020
atgacctgac ggtgaccaac cccaagcgca tcgagcgggc agtggaggag aaggcctgca 1080

actgtttgct gctcaaggtc aaccagatcg gctcagtcac agaagccatc caagcgtgca 1140
agctggccca ggagaacggc tgggggttta tggtgagtca tcgctctgga gaaaccgagg 1200
acacgttcat tgcagacctc gtagtgggac tgtgtacagg tcagatcaag actggtgccc 1260
catgcagatc tgaacgtctg gcgaagtaca accagctcat gaggattgaa gaggagttgg 1320
gggaggaggc tcgcttcgcg ggacacaact tccggaatcc cagtgtgctg tgaccccctg 1380
cttgcctgaa caccggaaca tcatctcatt ctcctggagc ctctttcttg ctgtcccgac 1440
ccgccatagt taccttgata ccttgagccc caagtcaccc agaacacctc gactcacctg 1500
ctctggctgt tcttggcttc cacaacccct tgctgtctcc tactcttcct cctctctggg 1560
ccccattttt ggggggattc cagtctgccc actttccctt ctattctctc taatcttaaa 1620
aaaaaaaaaa aaatgacgac tagaagaagc ggtccacaga agaaccgcca gcgtccgaga 1680
ggagcttcag gattggtgtg ttggggcgtt taaagtgggg ccacgtggca cgtgtgcttc 1740
cctgccatcc atggtgtgtt aagccttgaa ctatgcacag agctggtgtt tggggagtgc 1800
tggatgtgtg tgtgttcaca tttgtttgtt tgtttattta tttattcact tatttatttc 1860
tcagcctgtc agtcgtctgc cattactctt acagtctgaa agcatcagta ttttcacgtg 1920
gttccatttc aagatgacct aggatgggag gttttgttag catgggaaag gtcacagaaa 1980
ggttagcaat ggtttttcat ttggtgcact aactgaagct cggtacttta cagaatgggg 2040
ctgtgtaccc ggggactttt ctcctataac tctctccccc agccctaggt tcctcagtct 2100
ttttctccgg ctgcaccaga gcgctgcctc attcccccgt gccatgtccc acagttgcca 2160
ctgtctctgt ggctttgaaa tgaccaccac tattaaagtc tgaaccacag tgcacacc 2218

<210> 49
<211> 337
<212> PRT
<213> Mus musculus

<220>
<223> CA XIV

<400> 49
Met Leu Phe Phe Ala Leu Leu Leu Lys Val Thr Trp Ile Leu Ala Ala
1               5                   10                  15

Asp Gly Gly His His Trp Thr Tyr Glu Gly Pro His Gly Gln Asp His
            20                  25                  30

Trp Pro Thr Ser Tyr Pro Glu Cys Gly Gly Asp Ala Gln Ser Pro Ile
        35                  40                  45

Asn Ile Gln Thr Asp Ser Val Ile Phe Asp Pro Asp Leu Pro Ala Val
    50                  55                  60

Gln Pro His Gly Tyr Asp Gln Leu Gly Thr Glu Pro Leu Asp Leu His
65                  70                  75                  80

Asn Asn Gly His Thr Val Gln Leu Ser Leu Pro Pro Thr Leu His Leu
                85                  90                  95

Gly Gly Leu Pro Arg Lys Tyr Thr Ala Ala Gln Leu His Leu His Trp
            100                 105                 110

Gly Gln Arg Gly Ser Leu Glu Gly Ser Glu His Gln Ile Asn Ser Glu
        115                 120                 125

Ala Thr Ala Ala Glu Leu His Val Val His Tyr Asp Ser Gln Ser Tyr
    130                 135                 140

Ser Ser Leu Ser Glu Ala Ala Gln Lys Pro Gln Gly Leu Ala Val Leu
145                 150                 155                 160

Gly Ile Leu Ile Glu Val Gly Glu Thr Glu Asn Pro Ala Tyr Asp His

                165                 170                 175

Ile Leu Ser Arg Leu His Glu Ile Arg Tyr Lys Asp Gln Lys Thr Ser
            180                 185                 190

Val Pro Pro Phe Ser Val Arg Glu Leu Phe Pro Gln Gln Leu Glu Gln
        195                 200                 205

Phe Phe Arg Tyr Asn Gly Ser Leu Thr Thr Pro Pro Cys Tyr Gln Ser
    210                 215                 220

Val Leu Trp Thr Val Phe Asn Arg Arg Ala Gln Ile Ser Met Gly Gln
225                 230                 235                 240

Leu Glu Lys Leu Gln Glu Thr Leu Ser Ser Thr Glu Glu Asp Pro Ser
                245                 250                 255

Glu Pro Leu Val Gln Asn Tyr Arg Val Pro Gln Pro Leu Asn Gln Arg
            260                 265                 270

Thr Ile Phe Ala Ser Phe Ile Gln Ala Gly Pro Leu Tyr Thr Thr Gly
        275                 280                 285

Glu Met Leu Gly Leu Gly Val Gly Ile Leu Ala Gly Cys Leu Cys Leu
    290                 295                 300

Leu Leu Ala Val Tyr Phe Ile Ala Gln Lys Ile Arg Lys Lys Arg Leu
305                 310                 315                 320

Gly Asn Arg Lys Ser Val Val Phe Thr Ser Ala Arg Ala Thr Thr Glu
                325                 330                 335

Ala

<210> 50
<211> 1624
<212> DNA
<213> Mus musculus

<220>
<223> CA XIV

<400> 50
tggccagtaa gtggtgtgga gattacgaag ggacatacgg agagggcaga gagaggaaga 60
gagagaaagt gagagaggaa gaaatttatc ctgggatcca gaagttcgtg ttaacctgtg 120
gaaaatcaag tccctggaag tctgcagaga cagagacaag aagaaaagag atagaacgcc 180
agaaactcct ctctctccct ccctctccac ctctctcttc taaaccccaa attcctggtc 240
ccttgtaccc cattgtgggg ataatatgtt gttcttcgct ctcctgttaa aggtgacttg 300
gatcctggct gcagatgggg gtcaccactg gacatatgaa ggcccacacg gtcaggacca 360
ttggccaacc tcttatcctg agtgtggagg cgatgcccag tcccccatca atatccagac 420
agacagtgtg atatttgacc ccgatctgcc tgctgtacag ccccatggat atgaccagct 480
tgggactgag cctttggatc tacacaataa tggccataca gtgcagcttt ccctgccccc 540
aaccctgcac ctgggtggac tgccccgaaa atacacagca gcccagctcc acctgcactg 600
gggtcagaga ggatccctcg agggatcaga gcaccagatc aacagtgaag ccacggctgc 660
ggagctccac gtggttcact atgactccca gtcctacagc agcttgagtg aggcagctca 720
gaagccacag ggcctggctg tcctaggcat cctcattgag gtgggcgaga ctgagaatcc 780
agcttatgat cacattctga gtcgtctaca tgaaataaga tacaaagatc agaagacctc 840
tgtgcctccc ttcagcgtga gagagctgtt cccccaacag ctggagcaat tcttccgcta 900
caacggctca ctcacaactc ccccctgcta ccagagtgtg ctctggacag tcttcaacag 960

aagggcccag atttcaatgg gacagttaga gaagctccag gagacattgt cctctacaga 1020
agaggacccc tctgagcccc ttgtacagaa ctacagagtc ccccagcctc tcaaccagag 1080
gaccatcttt gcttctttca tccaagcagg accactgtat accacaggag agatgctggg 1140
tctaggtgtg ggaatcttgg ctggatgtct ttgccttctg ctggctgttt atttcatcgc 1200
tcaaaaaatt aggaagaagc ggctgggaaa caggaaaagt gtggttttca cctctgctcg 1260
ggctaccaca gaggcgtgaa ctctgtctca agatgctgca gatgcctccc tctcctcctg 1320
ccagggagcc tctctgaggg ctgcaggcac tggacgacca gttgtaggca ggcacgtgtc 1380
ctgctaggta accctctcta tacaccaggg accccagctc ttcaggaagg gctgagcctt 1440
tggccttcaa agctgtggat gaggagattc cctgtcctca tgcttggttg gaagaagctg 1500
aggataaact tccatctttc acctcacacc gccatgttct ctcccttaga aaaacgttgt 1560
agtttttttt ttcctctagg aaaaaagggc tgctgctggg attttaatta ttttttgctc 1620
aata 1624

<210> 51
<211> 334
<212> PRT
<213> Rattus norvegicus

<220>
<223> Cytosolic malate dehydrogenase

<400> 51
Met Ser Glu Pro Ile Arg Val Leu Val Thr Gly Ala Ala Gly Gln Ile
1               5                   10                  15

Ala Tyr Ser Leu Leu Tyr Ser Ile Gly Asn Gly Ser Val Phe Gly Lys
            20                  25                  30

Asp Gln Pro Ile Ile Leu Val Leu Leu Asp Ile Thr Pro Met Met Gly
        35                  40                  45

Val Leu Asp Gly Val Leu Met Glu Leu Gln Asp Cys Ala Leu Pro Leu
    50                  55                  60

Leu Gln Asp Val Ile Ala Thr Asp Lys Glu Glu Val Ala Phe Lys Asp
65                  70                  75                  80

Leu Asp Val Ala Val Leu Val Gly Ser Met Pro Arg Arg Glu Gly Met
                85                  90                  95

Glu Arg Lys Asp Leu Leu Lys Ala Asn Val Lys Ile Phe Lys Ser Gln
            100                 105                 110

Gly Ala Ala Leu Glu Lys Tyr Ala Lys Lys Ser Val Lys Val Ile Val
        115                 120                 125

Val Gly Asn Pro Ala Asn Thr Asn Cys Leu Thr Ala Ser Lys Ser Ala
    130                 135                 140

Pro Ser Ile Pro Lys Glu Asn Phe Ser Cys Leu Thr Arg Leu Asp His
145                 150                 155                 160

Asn Arg Ala Lys Ser Gln Ile Ala Leu Lys Leu Gly Val Thr Ala Asp
                165                 170                 175

Asp Val Lys Asn Val Ile Ile Trp Gly Asn His Ser Ser Thr Gln Tyr
            180                 185                 190

Pro Asp Val Asn His Ala Lys Val Lys Leu Gln Gly Lys Glu Val Gly
        195                 200                 205

Val Tyr Glu Ala Leu Lys Asp Asp Ser Trp Leu Lys Gly Glu Phe Ile
    210                 215                 220

Thr Thr Val Gln Gln Arg Gly Ala Ala Val Ile Lys Ala Arg Lys Leu
225                 230                 235                 240

Ser Ser Ala Met Ser Ala Ala Lys Ala Ile Ser Asp His Ile Arg Asp
                245                 250                 255

Ile Trp Phe Gly Thr Pro Glu Gly Glu Phe Val Ser Met Gly Val Ile
            260                 265                 270

Ser Asp Gly Asn Ser Tyr Gly Val Pro Asp Asp Leu Leu Tyr Ser Phe
        275                 280                 285

Pro Val Val Ile Lys Asn Lys Thr Trp Lys Phe Val Glu Gly Leu Pro
    290                 295                 300

Ile Asn Asp Phe Ser Arg Glu Lys Met Asp Leu Thr Ala Lys Glu Leu
305                 310                 315                 320

Thr Glu Glu Lys Glu Thr Ala Phe Glu Phe Leu Ser Ser Ala
                325                 330

<210> 52
<211> 1266
<212> DNA
<213> Rattus norvegicus

<220>
<223> Cytosolic malate dehydrogenase

<400> 52
ctttgctggt gcagagatcg gccttgcagt ttgcaataat gtctgagcca atcagagtcc 60
tcgtgacagg agcagccggt cagattgcat attcgctgct gtacagcatt ggaaatggat 120
ctgtctttgg gaaagaccag cccatcattc ttgtgctgtt ggacatcacc cccatgatgg 180
gtgttctgga cggtgtcctg atggagctgc aagactgtgc ccttcccctt ctgcaggatg 240
tcattgcaac agacaaagaa gaggttgcct tcaaagacct ggacgtggct gtccttgtgg 300
gctccatgcc aagaagggag ggcatggaga ggaaggacct actgaaagcc aacgtgaaga 360
tcttcaaatc ccagggcgca gccttggaga agtacgccaa gaaatcagtt aaggtcattg 420
ttgtggggaa cccagccaat acaaactgcc tgacggcctc caagtcagca ccatcgatcc 480
ccaaggagaa cttcagttgc ctgactcgat tggaccacaa ccgagcaaaa tctcaaattg 540
ctcttaaact cggtgtaacc gctgatgatg taaaaaatgt cattatctgg ggaaatcatt 600
catcaaccca gtatccagat gtcaatcatg ccaaggtgaa attgcaagga aaagaagttg 660
gtgtgtatga agccctcaaa gacgacagct ggctcaaggg agagttcatc acgactgtgc 720
agcagcgtgg tgctgctgtc atcaaggctc ggaagctgtc cagtgccatg tctgctgcga 780
aggccatctc ggaccacatc agagacatct ggtttggaac cccggagggc gagttcgtgt 840
cgatgggcgt aatctctgat ggcaactcct atggtgtccc tgatgacctg ctctactcgt 900
tccctgtcgt gatcaagaat aagacctgga agtttgttga aggcctcccc attaacgact 960
tctcccgtga gaagatggac ctgacagcaa aggagctgac cgaggaaaag gaaacggctt 1020
ttgagtttct ctcctccgca tgactacaca gtcgtgttga cgtcagcaga catcccaagg 1080
ctgaggaatc aaaatgtcgt ctttgagcct agtaccaaac agtaataatg gtacattcag 1140
attgtgaaca gcaaaatatt ttaaatatta tgtgctttgt gattcgtgaa agtctatcac 1200
gttgttagtg ctgcgatcta aataaaagta tattcaagtg aaaaaaaaaa aaaaaaaaaa 1260
aaaaaa                                                            1266

<210> 53
<211> 199

<212> PRT
<213> Rattus norvegicus

<220>
<223> HBP23

<400> 53
Met Ser Ser Gly Asn Ala Lys Ile Gly His Pro Ala Pro Ser Phe Lys
1               5                   10                  15

Ala Thr Ala Val Met Pro Asp Gly Gln Phe Lys Asp Ile Ser Leu Ser
            20                  25                  30

Asp Tyr Lys Gly Lys Tyr Val Val Phe Phe Phe Tyr Pro Leu Asp Phe
        35                  40                  45

Thr Phe Val Cys Pro Thr Glu Ile Ile Ala Phe Ser Asp Arg Ala Glu
    50                  55                  60

Glu Phe Lys Lys Leu Asn Cys Gln Val Ile Gly Ala Ser Val Asp Ser
65                  70                  75                  80

His Phe Cys His Leu Ala Trp Ile Asn Thr Pro Lys Lys Gln Gly Gly
                85                  90                  95

Leu Gly Pro Met Asn Ile Pro Leu Val Ser Asp Pro Lys Arg Thr Ile
            100                 105                 110

Ala Gln Asp Tyr Gly Val Leu Lys Ala Asp Glu Gly Ile Ser Phe Arg
        115                 120                 125

Gly Leu Phe Ile Ile Asp Asp Lys Gly Ile Leu Arg Gln Ile Thr Ile
    130                 135                 140

Asn Asp Leu Pro Val Gly Arg Ser Val Asp Glu Ile Leu Arg Leu Val
145                 150                 155                 160

Gln Ala Phe Gln Phe Thr Asp Lys His Gly Glu Val Cys Pro Ala Gly
                165                 170                 175

Trp Lys Pro Gly Ser Asp Thr Ile Lys Pro Asp Val Asn Lys Ser Lys
            180                 185                 190

Glu Tyr Phe Ser Lys Gln Lys
        195


<210> 54
<211> 882
<212> DNA
<213> Rattus norvegicus

<220>
<223> HBP23

<400> 54
ggctcacggt tggttctgtt tgtgagacct gtagctcgac tctgctgata gcaagatgtc 60
ttcaggaaat gcaaaaattg ggcatcctgc tcccagcttc aaagccacgg ctgttatgcc 120
ggatggacaa ttcaaagata tcagcctaag tgattacaaa ggaaaatatg ttgtattctt 180
tttttaccct cttgacttta cttttgtgtg tcccacggag atcattgctt tcagtgatag 240
agcagaagaa tttaagaaac tcaactgcca agtgattgga gcttctgtgg attctcactt 300

ctgtcatctg gcatggatta acacacccaa gaaacaagga ggattgggac ccatgaacat 360
tcccttggta tcagatccca agcgcaccat tgctcaggat tatggagtct taaaagctga 420
tgaaggtatc tctttcaggg gcctctttat tattgatgat aaaggtatcc ttcgccagat 480
aacaataaat gatcttcctg ttggccgctc tgtggatgag attctgagac tagtccaggc 540
cttccagttc actgacaaac atggtgaagt gtgcccagct ggctggaaac ctggcagtga 600
taccatcaag cctgatgtca ataagagtaa agagtatttc tctaagcaga agtgagcact 660
ggaccagttt tctggcagac agctttgagc agccagaaga aatttgtact ctacacatga 720
cgtggtgtga ttccagataa gcctttccta caagggctag gggtggttag cctttcttcc 780
actattggta aggggcagac catcttatat cagtcacaga aaccaacctg ttaattctct 840
ctctctcttt ttttttttta agtatctatt aaacgtgaat tc 882

<210> 55
<211> 573
<212> PRT
<213> Rattus norvegicus

<220>
<223> Squalene epoxidase

<400> 55
Met Trp Thr Phe Leu Gly Ile Ala Thr Phe Thr Tyr Phe Tyr Lys Lys
1               5                   10                  15

Cys Gly Asp Val Thr Leu Ala Asn Lys Glu Leu Leu Leu Cys Val Leu
            20                  25                  30

Val Phe Leu Ser Leu Gly Leu Val Leu Ser Tyr Arg Cys Arg His Arg
        35                  40                  45

Asn Gly Gly Leu Leu Gly Arg His Gln Ser Gly Ser Gln Phe Ala Ala
    50                  55                  60

Phe Ser Asp Ile Leu Ser Ala Leu Pro Leu Ile Gly Phe Phe Trp Ala
65                  70                  75                  80

Lys Ser Pro Pro Glu Ser Glu Lys Lys Glu Gln Leu Glu Ser Lys Arg
                85                  90                  95

Arg Arg Lys Glu Val Asn Leu Ser Glu Thr Thr Leu Thr Gly Ala Ala
            100                 105                 110

Thr Ser Val Ser Thr Ser Ser Val Thr Asp Pro Glu Val Ile Ile Ile
        115                 120                 125

Gly Ser Gly Val Leu Gly Ser Ala Leu Ala Thr Val Leu Ser Arg Asp
    130                 135                 140

Gly Arg Thr Val Thr Val Ile Glu Arg Asp Leu Lys Glu Pro Asp Arg
145                 150                 155                 160

Ile Leu Gly Glu Cys Leu Gln Pro Gly Gly Tyr Arg Val Leu Arg Glu
                165                 170                 175

Leu Gly Leu Gly Asp Thr Val Glu Ser Leu Asn Ala His His Ile His
            180                 185                 190

Gly Tyr Val Ile His Asp Cys Glu Ser Arg Ser Glu Val Gln Ile Pro
        195                 200                 205

Tyr Pro Val Ser Glu Asn Asn Gln Val Gln Ser Gly Val Ala Phe His

210                 215                 220

His Gly Lys Phe Ile Met Ser Leu Arg Lys Ala Ala Met Ala Glu Pro
225                 230                 235                 240

Asn Val Lys Phe Ile Glu Gly Val Val Leu Arg Leu Leu Glu Glu Asp
                245                 250                 255

Asp Ala Val Ile Gly Val Gln Tyr Lys Asp Lys Glu Thr Gly Asp Thr
            260                 265                 270

Lys Glu Leu His Ala Pro Leu Thr Val Val Ala Asp Gly Leu Phe Ser
        275                 280                 285

Lys Phe Arg Lys Asn Leu Ile Ser Asn Lys Val Ser Val Ser Ser His
    290                 295                 300

Phe Val Gly Phe Ile Met Lys Asp Ala Pro Gln Phe Lys Ala Asn Phe
305                 310                 315                 320

Ala Glu Leu Val Leu Val Asp Pro Ser Pro Val Leu Ile Tyr Gln Ile
                325                 330                 335

Ser Pro Ser Glu Thr Arg Val Leu Val Asp Ile Arg Gly Glu Leu Pro
            340                 345                 350

Arg Asn Leu Arg Glu Tyr Met Thr Glu Gln Ile Tyr Pro Gln Ile Pro
        355                 360                 365

Asp His Leu Lys Glu Ser Phe Leu Glu Ala Cys Gln Asn Ala Arg Leu
    370                 375                 380

Arg Thr Met Pro Ala Ser Phe Leu Pro Pro Ser Ser Val Asn Lys Arg
385                 390                 395                 400

Gly Val Leu Leu Leu Gly Asp Ala Tyr Asn Leu Arg His Pro Leu Thr
                405                 410                 415

Gly Gly Gly Met Thr Val Ala Leu Lys Asp Ile Lys Ile Trp Arg Gln
            420                 425                 430

Leu Leu Lys Asp Ile Pro Asp Leu Tyr Asp Asp Ala Ala Ile Phe Gln
        435                 440                 445

Ala Lys Lys Ser Phe Phe Trp Ser Arg Lys Arg Ser His Ser Phe Val
    450                 455                 460

Val Asn Val Leu Ala Gln Ala Leu Tyr Glu Leu Phe Ser Ala Thr Asp
465                 470                 475                 480

Asp Ser Leu Arg Gln Leu Arg Lys Ala Cys Phe Leu Tyr Phe Lys Leu
                485                 490                 495

Gly Gly Glu Cys Leu Thr Gly Pro Val Gly Leu Leu Ser Ile Leu Ser
            500                 505                 510

Pro Asp Pro Leu Leu Leu Ile Arg His Phe Phe Ser Val Ala Val Tyr
        515                 520                 525

Ala Thr Tyr Phe Cys Phe Lys Ser Glu Pro Trp Ala Thr Lys Pro Arg
    530                 535                 540

Ala Leu Phe Ser Ser Gly Ala Ile Leu Tyr Lys Ala Cys Ser Ile Ile
545                 550                 555                 560

Phe Pro Leu Ile Tyr Ser Glu Met Lys Tyr Leu Val His
                565                 570

<210> 56
<211> 2199
<212> DNA
<213> Rattus norvegicus

<220>
<223> Squalene epoxidase

<400> 56
ccggaaggag ttcggcaggt ctcccggacc ccctttgtga actacatttt gggtagaaca 60
ttttaatcac cgaaaccctt ctgaggaaaa aaagactccc actgccccct gcaaaaaaac 120
aaaagcaaaa acaaaaaaac caaaccccaa acccccaaat ctcagtctta gctggtgcca 180
ccaaagaagc ttttgagcca tgtggacttt tcttggaatt gctactttca cctattttta 240
taagaaatgc ggggatgtca ccctggccaa caaggagctc ctgctgtgcg tactggtgtt 300
cctgtcgctg ggcctggtgc tctcctaccg ctgtcgccat cgaaacgggg gcctccttgg 360
ccgccatcag agcggctccc agttcgctgc cttctcagat attctctctg ctttgcctct 420
tattggcttc ttctgggcca aatcaccccc tgaatcagaa aagaaagaac agctggagtc 480
caagaggcgc agaaaagaag tcaatctgtc agagacgacg ttaacgggag cagctacctc 540
agtatcgaca tcatctgtga cggacccaga agtgatcatc atagggtctg gtgtacttgg 600
ctctgctttg gcaacagtac tctctagaga tggaagaacg gtgacagtaa tcgagagaga 660
tttaaaagag cctgacagaa tacttggaga gtgtctgcag cccggtggct atcgagttct 720
tcgagagctg ggccttggag atacagtaga aagtcttaat gcccatcata tacacggcta 780
cgtaattcat gactgtgaaa gcaggtctga agttcaaatt ccatacccgg tgtcagaaaa 840
caaccaagta cagagtgggg ttgctttcca ccatggcaag ttcatcatga gtctccggaa 900
agcagctatg gcagagccca atgtaaagtt tatagaaggt gttgtgcttc ggttactaga 960
ggaagatgat gctgtgattg gtgtgcaata caaggacaag gagactgggg acaccaagga 1020
gctccatgcc ccgctcactg ttgttgccga cgggctcttc tccaagttca ggaagaacct 1080
catctccaat aaagtctctg tttcctccca cttcgttggc ttcattatga aggatgcacc 1140
acagtttaaa gccaatttcg cggagcttgt tctggtcgat cccagtccag ttctcatcta 1200
ccagatttca cccagcgaaa ctagggtgct tgtcgatatt cgaggagaat tgccaagaaa 1260
cttaagagaa tacatgacag aacaaattta cccacagata cctgatcact tgaaggaatc 1320
atttctggag gcctgtcaga atgctcgtct gcggaccatg ccagcaagct tccttcctcc 1380
ttcctcagtg aacaaacgag gcgtcctact tctgggagat gcgtataacc tgaggcatcc 1440
tcttactggt ggaggaatga cagtcgcttt aaaagatata aaaatatgga gacaactgtt 1500
aaaagatatt cctgaccttt atgatgatgc cgctattttc caggccaaaa agtcattctt 1560
ttggtcaaga aaaaggagcc attcctttgt tgtgaacgtg ctggctcagg cgttgtatga 1620
actattttct gctacagatg attccttgcg tcagctccga aaagcttgct ttctttattt 1680
taaacttggt ggagaatgtt tgactggtcc tgttgggttg ctttctatat tgtctcctga 1740
ccctctccta ttgattcgac acttcttctc cgttgcagtc tatgccacgt atttctgctt 1800
caagtcagag ccatgggcta caaaacctcg ggctcttttc agtagtggtg ctatactgta 1860
caaagcgtgc tctataatat ttcctctcat ctattcagaa atgaagtatc tggttcactg 1920
aaagggaaac cacatggaag tcattgggac ttgaggagga tgtacatagc ctaggactgt 1980
gtgtgtgact tctgcagtgg tgactcaagg accaagcttg tgtataagca tgtaaatata 2040
tgccttaatt cacaattgag aatgaagggt caataagcta gatgtttaaa agaatgattg 2100
tttccacaaa ttaatgttaa cactcaagtg tttggatttc agtattgggg tgattcaatt 2160
ggggacaatg ccaaataaaa tgaaggagtg gaactcctt 2199

<210> 57
<211> 152
<212> PRT
<213> Rattus norvegicus

<220>
<223> Copper-zinc containing superoxide dismutase

<400> 57
Met Lys Ala Val Cys Val Leu Lys Gly Asp Gly Pro Val Gln Gly Val
1               5                   10                  15

Ile His Phe Glu Gln Lys Ala Ser Gly Glu Pro Val Val Val Ser Gly
            20                  25                  30

Gln Ile Thr Gly Leu Thr Glu Gly Glu His Gly Phe His Val His Gln
        35                  40                  45

Tyr Gly Asp Asn Thr Gln Gly Cys Thr Thr Ala Gly Pro His Phe Asn
    50                  55                  60

Pro His Ser Lys Lys His Gly Gly Pro Ala Asp Glu Glu Arg His Val
65                  70                  75                  80

Gly Asp Leu Gly Asn Val Ala Ala Gly Lys Asp Gly Val Ala Asn Val
                85                  90                  95

Ser Ile Glu Asp Arg Val Ile Ser Leu Ser Gly Glu His Ser Ile Ile
            100                 105                 110

Gly Arg Thr Met Val Val His Glu Lys Gln Asp Asp Leu Gly Lys Gly
        115                 120                 125

Gly Asn Glu Glu Ser Thr Lys Thr Gly Asn Ala Gly Ser Arg Leu Ala
    130                 135                 140

Cys Gly Val Ile Gly Ile Ala Gln
145                 150


<210> 58
<211> 523
<212> DNA
<213> Rattus norvegicus

<220>
<223> Copper-zinc containing superoxide dismutase

<400> 58
cgatgaaggc cgtgtgcgtg ctgaagggcg acggtccggt gcagggcgtc attcacttcg 60
agcagaaggc aagcggtgaa ccagttgtgg tgtcaggaca gattacagga ttaactgaag 120
gcgagcatgg gttccatgtc catcaatatg gggacaatac acaaggctgt accactgcag 180
gacctcattt taatcctcac tctaagaaac atggcggtcc agcggatgaa gagaggcatg 240
ttggagacct gggcaatgtg gctgctggaa aggacggtgt ggccaatgtg tccattgaag 300
atcgtgtgat ctcactctca ggagagcatt ccatcattgg ccgtactatg gtggtccacg 360
agaaacaaga tgacttgggc aaaggtggaa atgaagaaag tacaaagact ggaaatgctg 420
gaagccgctt ggcttgtggt gtgattggga ttgcccaata aacgttccct atgtggtctg 480
agtctcagac ggcatctgct gcctgctaaa ctgtaaaaaa aaa                   523


<210> 59
<211> 413
<212> PRT
<213> Rattus norvegicus

<220>
<223> Aspartate aminotransferase

<400> 59
Met Ala Pro Pro Ser Phe Phe Ala Gln Val Pro Gln Ala Pro Pro Val
1               5                   10                  15

Leu Val Phe Lys Leu Ile Ala Asp Phe Arg Asp Asp Pro Asp Pro Arg
            20                  25                  30

Lys Val Asn Leu Gly Val Gly Ala Tyr Arg Thr Asp Asp Ser Gln Pro
        35                  40                  45

Trp Val Leu Pro Val Val Thr Lys Val Glu Gln Lys Ile Ala Asn Asp
    50                  55                  60

His Ser Leu Asn His Glu Tyr Leu Pro Ile Leu Gly Leu Ala Glu Phe
65                  70                  75                  80

Arg Ser Cys Ala Ser Gln Leu Val Leu Gly Asp Asn Ser Pro Ala Leu
                85                  90                  95

Arg Glu Asn Gly Val Gly Gly Val Gln Ser Leu Gly Ala Thr Gly Ala
            100                 105                 110

Leu Arg Ile Gly Ala Asp Phe Leu Ala Arg Trp Tyr Asn Gly Thr Asp
        115                 120                 125

Asn Lys Asn Thr Pro Val Tyr Val Ser Ser Pro Thr Trp Glu Asn His
    130                 135                 140

Asn Gly Val Phe Ser Ala Ala Gly Phe Lys Asp Ile Arg Ser Tyr Arg
145                 150                 155                 160

Tyr Trp Asp Ala Glu Lys Arg Gly Leu Asp Leu Gln Gly Phe Leu Asn
                165                 170                 175

Asp Leu Glu Asn Ala Pro Glu Phe Ser Ile Phe Val Leu His Ala Cys
            180                 185                 190

Ala His Asn Pro Thr Gly Thr Asp Pro Thr Glu Glu Glu Trp Lys Gln
        195                 200                 205

Ile Ala Ala Val Met Lys Arg Arg Phe Leu Phe Pro Phe Phe Asp Ser
    210                 215                 220

Ala Tyr Gln Gly Phe Ala Ser Gly Asp Leu Glu Lys Asp Ala Trp Ala
225                 230                 235                 240

Ile Arg Tyr Phe Val Ser Glu Gly Phe Glu Leu Phe Cys Pro Gln Ser
                245                 250                 255

Phe Ser Lys Asn Phe Gly Leu Tyr Asn Glu Arg Val Gly Asn Leu Thr
            260                 265                 270

Val Val Gly Lys Glu His Asp Ser Val Leu Arg Val Leu Ser Gln Met
        275                 280                 285

Glu Lys Ile Val Arg Ile Thr Trp Ser Asn Pro Pro Ala Gln Gly Ala
    290                 295                 300

Arg Ile Val Ala Thr Thr Leu Ser Asn Pro Glu Leu Phe Lys Glu Trp
305                 310                 315                 320

Lys Gly Asn Val Lys Thr Met Ala Asp Arg Ile Leu Thr Met Arg Ser
                325                 330                 335

Glu Leu Arg Ala Arg Leu Glu Ala Leu Lys Thr Pro Gly Thr Trp Ser
            340                 345                 350

His Ile Thr Glu Gln Ile Gly Met Phe Ser Phe Thr Gly Leu Asn Pro
        355                 360                 365

Lys Gln Val Glu Tyr Leu Val Asn Glu Lys His Ile Tyr Leu Met Pro
    370                 375                 380

Ser Gly Arg Ile Asn Met Cys Gly Leu Thr Thr Lys Asn Leu Asp Tyr
385                 390                 395                 400

Val Ala Thr Ser Ile Asn Glu Ala Val Thr Lys Phe Gln
                405                 410

<210> 60
<211> 1937
<212> DNA
<213> Rattus norvegicus

<220>
<223> Aspartate aminotransferase

<400> 60
ccgacgtccc ctcagattcc atcgcgatgg cccctccatc attctttgcc caggttccac 60
aggccccgcc ggttctggtc tttaagctca ttgcggactt ccgggatgat cccgatcccc 120
gcaaggttaa cctcggcgtg ggagcgtacc gcacagatga ctctcagccc tgggttttgc 180
cagtagtgac gaaggtcgaa cagaagattg ctaacgacca cagtctcaac cacgagtact 240
tgcccatcct gggcctggcg gagttccgga gctgtgcttc tcagctagta cttggggaca 300
acagcccagc tctcagggag aatggggttg ggggtgtgca gtctttggga gcgaccggtg 360
cacttcgaat tggagctgac ttcttagcgc gatggtacaa tggcacagac aacaagaaca 420
cgcccgtcta cgtatcatcg ccgacctggg agaaccataa tggcgtgttt tctgccgctg 480
gttttaaaga cattcggtcc tatcgctact gggatgcaga gaagagagga cttgatctcc 540
agggtttcct gaatgatctg gagaatgctc ctgagttctc catctttgtc ctccacgcct 600
gtgcacacaa cccaacgggg accgacccaa ctgaagagga gtggaagcag atcgccgccg 660
tcatgaagcg ccgttttctg ttccccttct ttgactcagc ctatcagggc tttgcatctg 720
gagacctaga gaaagatgcc tgggctattc gctattttgt gtctgaaggc ttcgagctct 780
tctgtcccca gtccttctcc aagaacttcg ggctctacaa tgagagagtg gggaatctga 840
ccgtggtcgg aaaagagcat gacagcgtcc tgcgggtcct ttcccagatg gagaagattg 900
tacgaatcac ctggtccaat ccccctgccc agggagctcg gatcgtggcc accaccctct 960
ccaaccctga gctctttaag gagtggaaag gaaacgtgaa gacaatggct gaccggattc 1020
tgaccatgag atccgaactc agggcgcgac tagaagctct caagactccc gggacttggt 1080
ctcacatcac tgagcagatt ggaatgttca gctttactgg gttgaacccc aagcaggtcg 1140
agtatttggt caacgagaag cacatctatc tgatgccgag cggtcggatc aacatgtgcg 1200
gcttgaccac caagaaccta gattatgtgg ctacctccat caatgaagct gtcaccaaat 1260
tccagtgaag aaacaccgag tagttcatac cccaaagcag ttcctgtcac agctttcctg 1320
cctgcgcaaa cctagccgta catgttgttt attagagatg accaccatgg ggaggcagcc 1380
gctgtttagc tggccccaca agagaagaca tttcttgaaa tgaacctggg tcgggtgggg 1440
ggatgactgg ggttagggcc ttttggaaac cagagcagat taaagttatt taagaataaa 1500
aaaacccttt gatatgagat gtaatcatct tgccttcctc tgtagtattc tgcaggagtg 1560
ttgcccacga agccgtgggc ttctgcacgt tgcttgagtc tgtacagagt cctgtcccca 1620
aaatcaagtt gtctgaggag ccggctgtga ctgtggatgt tggcattaaa actcaccatt 1680
tccatcgtct ctgtctctcg gccccctgat ctttccgcat ggttgtgacc ctggtcttgg 1740
aacattagtt ttttaaggcc actgtggcca gtatttatat catgacacac aagtggattt 1800

acatatttaa ctgagatgaa agttccgcta aacggtattt gctcttgtga tacgtggcac 1860
attgtgacat tttcttagtc tcttctgtcg tgttctgttt catttaaaaa aataaaaatg 1920
ctgatcaaga caaacgg 1937

<210> 61
<211> 362
<212> PRT
<213> Rattus norvegicus

<220>
<223> Cytosolic branch chain aminotransferase

<400> 61
Phe Arg Ala Lys Asp Leu Ile Ile Thr Lys Ala Asp Val Leu Lys Lys
1               5                   10                  15

Lys Pro Asp Pro Ser Ser Leu Val Phe Gly Ala Ser Phe Thr Asp His
            20                  25                  30

Met Leu Met Val Glu Trp Thr Ser Lys Tyr Gly Trp Asp Lys Pro His
        35                  40                  45

Thr Lys Pro Phe Glu Asn Leu Ser Ile His Pro Ala Ala Ser Val Leu
    50                  55                  60

His Tyr Ala Val Glu Leu Phe Glu Gly Leu Lys Ala Phe Arg Glu Val
65                  70                  75                  80

Asp Asn Lys Ile Arg Leu Phe Arg Pro Asp Leu Asn Met Lys Arg Met
                85                  90                  95

Cys Arg Ser Ala Val Arg Thr Thr Leu Pro Glu Phe Asp Lys Glu Glu
            100                 105                 110

Leu Leu Gln Cys Val Leu Gln Leu Ile Gln Leu Asp Arg Glu Trp Val
        115                 120                 125

Pro Tyr Ser Thr Ser Ala Ser Leu Tyr Ile Arg Pro Thr Phe Ile Gly
    130                 135                 140

Ile Glu Pro Ser Leu Gly Val Lys Lys Pro Ser Lys Ala Leu Leu Phe
145                 150                 155                 160

Val Ile Leu Ser Pro Val Gly Ser Tyr Phe Ser Asn Gly Thr Phe Ser
                165                 170                 175

Pro Val Ser Leu Trp Ala Asn Pro Lys Phe Val Lys Ser Trp Lys Gly
            180                 185                 190

Gly Thr Gly Asp Phe Lys Met Gly Cys Asn Tyr Gly Ser Ser Leu Leu
        195                 200                 205

Ala Gln Cys Glu Ala Ala Glu Asn Gly Cys His Gln Val Leu Trp Leu
    210                 215                 220

Tyr Gly Lys Glu Asn Arg Ile Thr Glu Val Gly Thr Met Asn Leu Phe
225                 230                 235                 240

Leu Tyr Trp Ile Asn Lys Asp Gly Glu Glu Glu Leu Ala Thr Pro Pro
                245                 250                 255

Leu Asp Gly Val Ile Leu Pro Gly Val Thr Arg Gln Ser Ile Leu Glu
            260                 265                 270

Leu Gly Glu Glu Trp Gly Glu Phe Lys Val Cys Asp Arg His Ile Thr
        275                 280                 285

Met Asp Gly Leu Ser Thr Ala Leu Glu Glu Asn Arg Val Lys Glu Met
    290                 295                 300

Phe Gly Ser Gly Thr Ala Cys Val Val Cys Pro Val Ala Ser Ile Leu
305                 310                 315                 320

Tyr Lys Gly Gln Met Leu His Ile Pro Thr Met Glu Asn Gly His Lys
                325                 330                 335

Leu Ser Ser Arg Ile Met Ala Lys Leu Thr Asp Ile Gln Tyr Gly Arg
            340                 345                 350

Ile Lys Ser Glu Trp Thr Leu Glu Leu Pro
        355                 360


<210> 62
<211> 2228
<212> DNA
<213> Rattus norvegicus

<220>
<223> Cytosolic branch chain aminotransferase

<400> 62
tttagggcca aagatctcat tatcacaaaa gccgatgtct taaaaaagaa gcccgacccc 60
agctcgctgg tctttggagc ttcgttcact gaccacatgc tgatggtgga gtggacctcc 120
aagtatggat gggataagcc tcacaccaag ccttttgaaa acttgtccat acatcccgct 180
gcctctgttt tgcactatgc tgtggaactg tttgaaggct tgaaggcttt tcgggaagtt 240
gataacaaga tccgattgtt ccggccggac ctcaacatga aaagaatgtg ccgatctgcc 300
gtgaggacca ctctgccgga gtttgacaag gaggagcttc tgcaatgcgt tcttcagctc 360
atacagctcg accgagagtg ggtcccctac tcaacctctg ccagtctgta catccgcccc 420
acattcatcg ggattgagcc atcgcttgga gtcaagaagc cttctaaagc tctgctcttt 480
gtgattttga gccccgtggg atcttatttt tctaatggga ccttttctcc ggtgtccctg 540
tgggccaacc caaagttcgt caaatcctgg aaaggtggga ctggagactt caagatgggc 600
tgcaactatg gatcttccct tctggcgcag tgtgaggcgg cagagaatgg ctgtcaccag 660
gtcctgtggc tgtacggcaa ggaaaaccgg ataactgaag tgggcacgat gaatctcttc 720
ctctactgga taaacaaaga tggagaagaa gaactggcga cgcctccgct agatggcgtc 780
attctcccag gagtcactcg gcagagcatc ctggagctgg gagaagagtg gggcgagttt 840
aaggtgtgcg acagacacat caccatggat ggcctgtcca ccgccctgga ggagaacaga 900
gtgaaggaga tgttcggctc ggggacagcc tgcgttgtct gcccagttgc cagtattctg 960
tacaagggcc agatgctgca cattccaacc atggagaatg gccacaagct ttcaagtcgc 1020
atcatggcaa agttgactga tatccagtat ggacggatca agagcgaatg gacactggag 1080
ttgccctaat gggagagctc agggtatcgg tgacggggtg ccacagaaga ccgactcctg 1140
tcctcgagct gggacaacct gtgactactt taactccttg tagttccatt gttcattgtt 1200
accaaacgag tttgtgtccc cccccccccg tgagccaaga aaattgccag tgttaagtgt 1260
cacctgtgaa catgatagta gctgctttac tgtacctttc cttggaaagc tatcaatgaa 1320
taattgcctc ccctcactcc ataccaatcc ttagtaggag aatggttccc ccaccaatga 1380
gatgactgtg tgctggaagg gctactgagg aaatgccata ggcggcctcc ctgtgactca 1440
tgggcaccct cagagcggga gccctttgtt acttttgctg gcccttgtac ttgttctgtt 1500
gtttacgttt ttgtttgttt gttgtctaat tggggaagat ttgggctccc tgtgaagccc 1560
tgctggcctg gtattccgtg atgtctgccc aggctggtct cagactcgga gcgggtcttc 1620
ctttctcaca cagccttctg gatgctgggg cgatggacag gtgtgatacc ttgatttgtt 1680
tctgtctttt aaacaaaagt ttggtcggct ggatttcgtt tctccgcgct tctgtaagtc 1740

cctgagtccg cgctaaggtg gccgtgcacc cttcttggta tcatggcctg cactaatgtc 1800
accgaaggtg tctgtgaggc tcactgccct ttttctgtaa ctatacctgg ctgctcactg 1860
cgctgctatg agactagggg tcctgtgcct tctagatctt tgtatcctca aagcccagca 1920
catagtaagt attcagtact tgtttaccat gagaatgaac atagaagtgt actttctgtc 1980
tctccatgct gaaacccttt cggaaaccac gcctgattcg ggcagcatgt agcagtattt 2040
atcttaggct gtctttcttg tccctggact cctccagaca gcatggagga attctgtttt 2100
aagattatct tggagtttaa gtgattgccc tggaaaaatg aaatgtacca accatgtgga 2160
gatgacagct actgtacatt atgtatgaaa tgccaaataa agggacaagt tgggagataa 2220
aaaaaaaa                                                          2228

<210> 63
<211> 442
<212> PRT
<213> Rattus norvegicus

<220>
<223> Dihydrolipoamide succinyltransferase

<400> 63
Met Arg Ser Leu Ser Ala Phe Gln Lys Gly Asn Cys Pro Leu Gly Arg
1               5                   10                  15

Arg Ser Leu Pro Gly Val Ser Leu Cys Gln Gly Pro Gly Tyr Pro Asp
            20                  25                  30

Ser Arg Lys Met Val Ile Asn Thr Val Ala Val Phe Ser Val Arg Phe
        35                  40                  45

Phe Gln Thr Thr Ala Val Cys Lys Asn Asp Val Ile Thr Val Gln Thr
    50                  55                  60

Pro Ala Phe Ala Glu Ser Val Thr Glu Gly Asp Val Arg Trp Glu Lys
65                  70                  75                  80

Ala Val Gly Asp Ala Val Ala Glu Asp Glu Val Val Cys Glu Ile Glu
                85                  90                  95

Thr Asp Lys Thr Ser Val Gln Val Pro Ser Pro Ala Asn Gly Ile Ile
            100                 105                 110

Glu Ala Leu Leu Val Pro Asp Gly Gly Lys Val Glu Gly Gly Thr Pro
        115                 120                 125

Leu Phe Thr Leu Arg Lys Thr Gly Ala Ala Pro Ala Lys Ala Lys Pro
    130                 135                 140

Ala Glu Ala Pro Ala Thr Ala His Lys Ala Ala Pro Glu Ala Pro Ala
145                 150                 155                 160

Ala Pro Pro Pro Pro Val Ala Pro Val Pro Thr Gln Met Pro Pro Val
                165                 170                 175

Pro Ser Pro Ser Gln Pro Pro Ser Ser Lys Pro Val Ser Ala Ile Lys
            180                 185                 190

Pro Thr Ala Ala Pro Pro Leu Ala Glu Ala Gly Ala Ala Lys Gly Leu
        195                 200                 205

Arg Ser Glu His Arg Glu Lys Met Asn Arg Met Arg Gln Arg Ile Ala
    210                 215                 220

Gln Arg Leu Lys Glu Ala Gln Asn Thr Cys Ala Met Leu Thr Thr Phe
225                 230                 235                 240

Asn Glu Val Asp Met Ser Asn Ile Gln Glu Met Arg Ala Arg His Lys
                245                 250                 255

Asp Ala Phe Leu Lys Lys His Asn Leu Lys Leu Gly Leu Met Ser Ala
            260                 265                 270

Phe Val Lys Ala Ser Ala Phe Ala Leu Gln Glu Gln Pro Val Val Asn
        275                 280                 285

Ala Val Ile Asp Asp Ala Thr Lys Glu Val Val Tyr Arg Asp Tyr Ile
    290                 295                 300

Asp Ile Ser Val Ala Val Ala Thr Pro Arg Gly Leu Val Val Pro Val
305                 310                 315                 320

Ile Arg Asn Val Glu Thr Met Asn Tyr Ala Asp Ile Glu Arg Thr Ile
                325                 330                 335

Asn Glu Leu Gly Glu Lys Ala Arg Lys Asn Glu Leu Ala Ile Glu Asp
            340                 345                 350

Met Asp Gly Gly Thr Phe Thr Ile Ser Asn Gly Gly Val Phe Gly Ser
        355                 360                 365

Leu Phe Gly Thr Pro Ile Ile Asn Pro Pro Gln Ser Ala Ile Leu Gly
    370                 375                 380

Met His Gly Ile Phe Asp Arg Pro Val Ala Val Gly Gly Lys Val Glu
385                 390                 395                 400

Val Arg Pro Met Met Tyr Val Ala Leu Thr Tyr Asp His Arg Leu Ile
                405                 410                 415

Asp Gly Arg Glu Ala Val Thr Phe Leu Arg Lys Ile Lys Ala Ala Val
            420                 425                 430

Glu Asp Pro Ala Val Leu Leu Leu Asp Leu
        435                 440


<210> 64
<211> 2749
<212> DNA
<213> Rattus norvegicus

<220>
<223> Dihydrolipoamide succinyltransferase

<400> 64
tcccggtccc gctgcgtgtc ccgggcgttc atgcgctcgc tttctgcctt ccagaagggg 60
aactgccctc tagggagacg ttccctgcct ggggtctcct tatgtcaggg accaggttac 120
cctgacagca ggaagatggt cattaacaca gtagccgtct tcagtgttcg cttcttccaa 180
accacggcag tgtgcaagaa tgatgtgatt acagtccaga ccccagcgtt tgcagagtct 240
gtcacagagg gagatgtcag gtgggagaaa gctgttggag atgcagttgc agaagatgaa 300
gtggtgtgtg agattgagac agacaagact tctgtgcagg ttccatcacc agcaaatggc 360
atcattgaag ctcttttggt acccgatggg ggcaaagttg aaggaggaac tcctctattc 420
acactcagga aaaccggtgc tgctcctgct aaggccaaac cagctgaagc ccctgctaca 480

gcccacaaag cagcgcctga agcaccggcg gcccctcctc ctcctgtagc accagtgccc 540
actcagatgc cacctgtgcc ctcaccctca caacctcctt ctagcaaacc agtgtctgca 600
ataaaaccca ctgctgcccc tccactggct gaggcgggag ctgctaaagg cctgcgctca 660
gaacatcggg aaaagatgaa caggatgcgg cagcgcatcg cccagcgtct gaaggaagcc 720
cagaacacct gcgcaatgct gacgacgttc aatgaggttg acatgagtaa catacaagag 780
atgagagctc ggcacaaaga tgctttcctg aaaaaacata acctgaaatt aggcctcatg 840
tcggcatttg tgaaggcctc agcattcgcc ttgcaggagc agcctgtagt aaacgcagtg 900
attgatgacg caaccaagga ggtggtgtac agagattata ttgacatcag tgtcgcagtt 960
gctaccccaa ggggtctcgt ggttcctgtc atcaggaatg tggaaactat gaattatgca 1020
gatattgaac ggaccattaa tgaactagga gagaaggccc ggaagaatga acttgccatc 1080
gaagacatgg atgggggcac cttcaccatc agcaatggag gagttttcgg ctcacttttc 1140
ggaacaccca ttatcaaccc gcctcagtct gccattctgg gcatgcatgg catcttcgac 1200
aggcctgtgg ctgtgggggg caaggtggaa gtccgaccta tgatgtatgt agccctgacc 1260
tacgaccacc ggctgattga tggcagagag gctgtgactt tcctccgaaa aatcaaggca 1320
gcagtagaag atccagcagt cctcctccta gacctttagg aggaagccac acatgcctac 1380
ctactgatca tgcaggaatg aaaaaccagt cttctagccc gtgcaacctc ctcagtccag 1440
gttaactgag ggcaggcagg cactaccatt ggcctcgagc aaggactcca gggcactacg 1500
taaccaggcc tagggcatca gtcctttcat gggtcttttt atgctccttc tggctcttcc 1560
tctctcctgt gctcttcctt ctgctctctt ggcctgacag agagagagag agagagagag 1620
agagagagag agagagagag agagagagcc tgtgaatgcc catcaatagt cctgccttta 1680
ttccagtcct ctacaaagac acctttgctt ctctccagta acagtatgct atagggaata 1740
ccaccaggga ccatgtgatt gagtttcttg tctttggaaa gtgttctccc aaagatgcct 1800
agggggaagc tttgctccgg ggctcaaagc agcctgtgtc gcagctgttc ttggtctcac 1860
ttgatgcccc ctgtgtgggt attgaagata ggcaaagcgg ggctacctca tcctaacctg 1920
tcagcggctt caaggtgcct ctctacctct gacaggagca caggctggag gatttaggga 1980
taggcacaat tgtgctgggt ccctgaggct acctgtgtag tgcacagatg ggagtgaaac 2040
ttggcttgcc ctttacaggg ctccattgta tgctggcact gggaagattc atgttgtcat 2100
aactacgtac aactgttgtc catcatacca accctttcca gcttctagga atcattctag 2160
gacggtgttc tcacgtaaag cagattttag catatcatgg cagtgggaca cctggtgaac 2220
ggactgttct caccagatca ggctgatgag accttttaaa ttcaaggtgg tacgagcatg 2280
tgctggggta gggcctgttc tctcccaacc tgggatgtgg cctgtgggga gcaaggcctt 2340
ggttcctctg gtgttggaca cagataaggg gaggatggac tgtggcggga accagcctca 2400
ggagttagga gagtgaagag ataatgacgt catcgctaca ctcacccttc aggggatagg 2460
tggtagttag aatatttttg aagcatggtg aaatgtaatt aactggttat gaaagctcct 2520
agcccttgtg tccttcactg agcctgagac tgctgacagc tgacatgggg ccattagtgc 2580
tgaatgccct tttttcttgt aatccaagac ttcctggggg aagaggggaa gaggtcagta 2640
ttgagtgcct gcaacatctg ctacgtatag ctataccatt cagaacttgt aatgaaaata 2700
tttaaagaga ctgattttga atgaaaatta aagggcaaat gttctcaaa 2749

<210> 65
<211> 127
<212> PRT
<213> Rattus norvegicus

<220>
<223> RNA polymerase II

<400> 65
Met Ser Asp Asn Glu Asp Asn Phe Asp Gly Asp Asp Phe Asp Asp Val
1               5                   10                  15

Glu Glu Asp Glu Gly Leu Asp Asp Leu Glu Asn Ala Glu Glu Glu Gly
            20                  25                  30

Gln Glu Asn Val Glu Ile Leu Pro Ser Gly Glu Arg Pro Gln Ala Asn
        35                  40                  45

Gln Lys Arg Ile Thr Thr Pro Tyr Met Thr Lys Tyr Glu Arg Ala Arg
    50                  55                  60

Val Leu Gly Thr Arg Ala Leu Gln Ile Ala Met Cys Ala Pro Val Met
65                  70                  75                  80

Val Glu Leu Glu Gly Glu Thr Asp Pro Leu Leu Ile Ala Met Lys Glu
                85                  90                  95

Leu Lys Ala Arg Lys Ile Pro Ile Ile Ile Arg Arg Tyr Leu Pro Asp
            100                 105                 110

Gly Ser Tyr Glu Asp Trp Gly Val Asp Glu Leu Ile Ile Ser Asp
        115                 120                 125

<210> 66
<211> 470
<212> DNA
<213> Rattus norvegicus

<220>
<223> RNA polymerase II

<400> 66
tttctctgct ctgcgacccg gaccggccag taaggagaag gtatcatgtc agacaacgaa 60
gacaatttcg atggcgatga ctttgatgac gtggaggagg acgaagggct tgacgacttg 120
gaaaatgctg aggaggaggg tcaggaaaat gttgagattc tcccatctgg tgaacgaccg 180
caggccaacc agaagcgtat caccactcca tacatgacca agtacgagcg agcccgagtg 240
ctgggcaccc gggctctcca gattgcgatg tgtgccccag taatggtgga gctggagggg 300
gagacagacc ctttgctcat cgccatgaag gaactcaagg cccggaagat ccccatcatc 360
attcgccgtt acctgccaga tggaagctat gaggattggg gcgtggatga gctcatcatc 420
agcgactgag cagagctgtc aggctgtgcc aggcaccatc tctatgccca           470

<210> 67
<211> 416
<212> PRT
<213> Rattus norvegicus

<220>
<223> Squalene synthetase

<400> 67
Met Glu Phe Val Lys Cys Leu Gly His Pro Glu Glu Phe Tyr Asn Leu
1               5                   10                  15

Leu Arg Phe Arg Met Gly Gly Arg Arg Asn Phe Ile Pro Lys Met Asp
            20                  25                  30

Arg Asn Ser Leu Ser Asn Ser Leu Lys Thr Cys Tyr Lys Tyr Leu Asp
        35                  40                  45

Gln Thr Ser Arg Ser Phe Ala Ala Val Ile Gln Ala Leu Asp Gly Asp
    50                  55                  60

Ile Arg His Ala Val Cys Val Phe Tyr Leu Ile Leu Arg Ala Met Asp
65                  70                  75                  80

Thr Val Glu Asp Asp Met Ala Ile Ser Val Glu Lys Lys Ile Pro Leu
                85                  90                  95

Leu Arg Asn Phe His Thr Phe Leu Tyr Glu Pro Glu Trp Arg Phe Thr
            100                 105                 110

Glu Ser Lys Glu Lys His Arg Val Val Leu Glu Asp Phe Pro Thr Ile
        115                 120                 125

Ser Leu Glu Phe Arg Asn Leu Ala Glu Lys Tyr Gln Thr Val Ile Ala
    130                 135                 140

Asp Ile Cys His Arg Met Gly Cys Gly Met Ala Glu Phe Leu Asn Lys
145                 150                 155                 160

Asp Val Thr Ser Lys Gln Asp Trp Asp Lys Tyr Cys His Tyr Val Ala
                165                 170                 175

Gly Leu Val Gly Ile Gly Leu Ser Arg Leu Phe Ser Ala Ser Glu Phe
            180                 185                 190

Glu Asp Pro Ile Val Gly Glu Asp Thr Glu Cys Ala Asn Ser Met Gly
        195                 200                 205

Leu Phe Leu Gln Lys Thr Asn Ile Ile Arg Asp Tyr Leu Glu Asp Gln
    210                 215                 220

Gln Glu Gly Arg Gln Phe Trp Pro Gln Glu Val Trp Gly Lys Tyr Val
225                 230                 235                 240

Lys Lys Leu Glu Asp Phe Val Lys Pro Glu Asn Val Asp Val Ala Val
                245                 250                 255

Lys Cys Leu Asn Glu Leu Ile Thr Asn Ala Leu Gln His Ile Pro Asp
            260                 265                 270

Val Ile Thr Tyr Leu Ser Arg Leu Arg Asn Gln Ser Val Phe Asn Phe
        275                 280                 285

Cys Ala Ile Pro Gln Val Met Ala Ile Ala Thr Leu Ala Ala Cys Tyr
    290                 295                 300

Asn Asn His Gln Val Phe Lys Gly Val Val Lys Ile Arg Lys Gly Gln
305                 310                 315                 320

Ala Val Thr Leu Met Met Asp Ala Thr Asn Met Pro Ala Val Lys Ala
                325                 330                 335

Ile Ile Tyr Gln Tyr Ile Glu Glu Ile Tyr His Arg Val Pro Asn Ser
            340                 345                 350

Asp Pro Ser Ala Ser Lys Ala Lys Gln Leu Ile Ser Asn Ile Arg Thr
        355                 360                 365

Gln Ser Leu Pro Asn Cys Gln Leu Ile Ser Arg Ser His Tyr Ser Pro
    370                 375                 380

Ile Tyr Leu Ser Phe Ile Met Leu Leu Ala Ala Leu Ser Trp Gln Tyr
385                 390                 395                 400

Leu Ser Thr Leu Ser Gln Val Thr Glu Asp Tyr Val Gln Arg Glu His
                405                 410                 415

<210> 68
<211> 1662
<212> DNA
<213> Rattus norvegicus

<220>
<223> Squalene synthetase

<400> 68
ggccccgctc ctcgccctgc ctaccgccgg catctaaaca caggtgggag tgggagatcc 60
cgacaggtga gccccgcgcc ccgcagccac aaggatggag ttcgtgaagt gtctaggcca 120
cccggaggag ttctacaacc tgctgcgatt ccgcatggga ggccggcgga atttcatacc 180
caagatggac cggaactcgc tcagcaacag cttgaagact tgctataagt atcttgatca 240
gaccagtcgc agcttcgccg cggttatcca ggcgctggat ggggacatac gtcatgcggt 300
gtgtgtgttt tacctgatcc tccgagccat ggacacagtg gaggatgaca tggccatcag 360
tgtggagaag aagatcccac tgctgcgaaa ctttcacact ttcctctatg agccggagtg 420
gcggttcacc gagagcaagg agaagcaccg agtagtgctg gaggacttcc ccacgatctc 480
cctggagttt agaaatttgg ctgagaaata tcaaacagtg atcgctgaca tctgtcacag 540
gatgggatgt gggatggcag aatttctaaa caaggatgta acctccaaac aggactggga 600
caagtactgt cactatgttg ctggactggt gggaatcggc ctttctcgcc tattctctgc 660
ctcagagttt gaagatccca tagttggtga agacacagag tgtgccaatt ctatgggtct 720
gtttctgcag aaaacaaata tcattcgtga ttatctggaa gaccaacaag aaggaagaca 780
gttttggcct caagaggtat ggggcaaata tgttaagaag ctggaagact ttgttaagcc 840
agagaacgta gatgtggccg tgaagtgctt gaatgaactc ataaccaacg ccctacaaca 900
catccctgac gtcatcacct acctgtcaag gctccggaac caaagtgtgt ttaacttctg 960
tgccattcca caggtaatgg ccattgctac gctggctgcc tgttacaata accatcaggt 1020
attcaaggga gtagtgaaga ttcggaaggg gcaagcagtt accctcatga tggatgccac 1080
caacatgcca gctgtcaaag ctatcatata ccagtacata gaagagattt atcaccgggt 1140
ccccaactca gacccgtcag ctagcaaggc caagcagctc atctccaaca tcaggacgca 1200
gagccttccc aattgccagc tcatctcccg aagccactac tcccccattt acctgtcctt 1260
catcatgctc ttggctgccc tgagctggca gtacttgagc actctgtccc aggtcacaga 1320
agactatgtc cagagagaac actgactttg tttagctgga agcggaagtc cacgtgaagt 1380
gggtttttct tcttccccca gctggatttt gacttccctt ggtttttcct tctactctaa 1440
tctttcggag aactgagtgt gggaccttta ggaactctga agaggaaagg acgccttgcc 1500
ctcagcagcc tggtgcttcc tggatgtggt ccctgcctct tgtagccact ggcatcatgt 1560
tgaccgaagc actggaaagg ccacatgtga tcctagtgaa cctggctaga atgctgattg 1620
aatctattta atttgaaaca gcctttgaat acctatcaca gt 1662

<210> 69
<211> 382
<212> PRT
<213> Rattus norvegicus

<220>
<223> UDP-Gal

<400> 69
Met Ser Ala Leu Lys Arg Met Met Arg Val Ser Asn Arg Ser Leu Ile
1               5                   10                  15

Ala Phe Ile Phe Phe Phe Ser Leu Ser Thr Ser Cys Leu Tyr Phe Ile
            20                  25                  30

Tyr Val Ala Pro Gly Ile Ala Asn Thr Tyr Leu Phe Met Val Gln Ala
        35                  40                  45

Arg Gly Ile Met Leu Arg Glu Asn Val Lys Thr Ile Gly His Met Ile
    50                  55                  60

Arg Leu Tyr Thr Asn Lys Asn Thr Thr Leu Asn Gly Thr Asp Tyr Pro
65                  70                  75                  80

Glu Gly Asn Asn Thr Ser Asp Tyr Leu Val Gln Thr Thr Thr Tyr Leu
                85                  90                  95

Pro Gln Asn Phe Thr Tyr Ser Pro His Leu Pro Cys Pro Glu Lys Leu
            100                 105                 110

Pro Tyr Met Arg Gly Phe Leu Ser Val Asn Val Ser Glu Ile Ser Phe
        115                 120                 125

Asp Glu Val His Gln Leu Phe Ser Lys Asp Ser Glu Ile Glu Pro Gly
    130                 135                 140

Gly His Trp Arg Pro Gln Asp Cys Lys Pro Arg Trp Lys Val Ala Val
145                 150                 155                 160

Leu Ile Pro Phe Arg Asn Arg His Glu His Leu Pro Ile Phe Phe Leu
                165                 170                 175

His Leu Ile Pro Met Leu Gln Lys Gln Arg Leu Glu Phe Ala Phe Tyr
            180                 185                 190

Val Ile Glu Gln Thr Gly Thr Gln Pro Phe Asn Arg Ala Met Leu Phe
        195                 200                 205

Asn Val Gly Phe Lys Glu Ala Met Lys Asp Arg Ala Trp Asp Cys Val
    210                 215                 220

Ile Phe His Asp Val Asp His Leu Pro Glu Asn Asp Arg Asn Tyr Tyr
225                 230                 235                 240

Gly Cys Gly Glu Met Pro Arg His Phe Ala Ala Lys Leu Asp Lys Tyr
                245                 250                 255

Met Tyr Ile Leu Pro Tyr Lys Glu Phe Phe Gly Gly Val Ser Gly Leu
            260                 265                 270

Thr Val Glu Gln Phe Arg Lys Ile Asn Gly Phe Pro Asn Ala Phe Trp
        275                 280                 285

Gly Trp Gly Gly Glu Asp Asp Asp Leu Trp Asn Arg Val His Tyr Ser
    290                 295                 300

Gly Tyr Asn Val Thr Arg Pro Glu Gly Asp Leu Gly Lys Tyr Thr Ser
305                 310                 315                 320

Ile Pro His His His Arg Gly Glu Val Gln Phe Leu Gly Arg Tyr Lys
                325                 330                 335

Leu Leu Arg Tyr Ser Lys Glu Arg Gln Phe Ile Asp Gly Leu Asn Asn
            340                 345                 350

Leu Leu Tyr Thr Pro Lys Ile Leu Val Asp Arg Leu Tyr Thr Asn Ile
        355                 360                 365

Ser Val Asn Leu Met Pro Glu Leu Ala Pro Val Glu Asp Tyr
    370                 375                 380

<210> 70
<211> 5729
<212> DNA
<213> Rattus norvegicus

<220>
<223> UDP-Gal

<400> 70
agcctgcagg ggtctcgggg cgcacagggc tgccgggatt gtcctcgtgg gtgtccgcag 60
tccgagatcg ccgcgtgtct gtcatctgtt tccttcgtcc tccgggcccc ggccgcgcgc 120
atccgccggg tagtccgggg tccgtgcggg gggcggcggc gcctctccct cgggccgggc 180
ggcgggaatg cggttgcctt ggccggctgg gcagcgggcg cggctgcaaa cggaacccgg 240
gtgcactcgc cgctggccgg gacgccgccc tggcctgctc tcggctcccg aggccgccct 300
gcgctctggg gcccggcgcg gaatgaggtg caggccgcgc accgccgacg ccccctccgc 360
ggccccctgg ttcaacgcgg ccgcccgcgc ctcgcgtgcc ccagcgccca cgttagagcc 420
tcgcactggc agcgctcgcg gaggcacagg cctgcagagc gcacgccact cggcccgggt 480
cccgatcgcg gggactcgcc tcttggacac cgccccaggt gcgggacccc gacacgctgc 540
aagcgcgcgg gccgcctccg catcgcagag ctcctctagc ctggcccgtg gctttggcct 600
ccacggagcc ggatctccct gcctccctcc acatttctcc gcatcctttc aacacccttc 660
ccgctgctct accacctccc attcattgca tccatttctc cttcccggct cggttcttct 720
tcccataacc tccccggccg gaccccgcta cccacccggt acattcatgg aagtttaggg 780
cctggaccgt ggctcggagt ctggcctgag agcgcagcgg agctgctggt agggcagagg 840
aagatgtctg cgctcaagcg gatgatgcgg gtctccaatc gctccctgat cgccttcatc 900
ttcttcttct ccctctccac gtcctgtctc tatttcatct atgtggctcc aggcatcgcc 960
aacacatacc tctttatggt acaagctaga ggcatcatgc tgagagaaaa tgtgaaaaca 1020
attggacaca tgatcaggct gtataccaat aaaaacacta cgctcaatgg gacagattat 1080
cccgaaggca ataatacaag tgactatctt gttcaaacaa caacgtatct tccgcagaac 1140
ttcacgtatt caccgcacct cccctgtcca gagaagctgc cttatatgcg gggattcctc 1200
agtgtcaacg tgagtgaaat cagctttgat gaagtccatc agctcttctc caaggactcg 1260
gagattgagc cgggaggcca ctggaggccc caggactgca agcctagatg gaaggtggca 1320
gttctcatcc ccttccgtaa ccgccatgaa cacctcccga tctttttcct gcacctgatc 1380
ccgatgctcc agaaacagag gctggaattt gccttttatg tcatcgaaca gaccggcaca 1440
caaccgttca atcgtgccat gctctttaac gtgggtttca aagaggccat gaaggacaga 1500
gcctgggact gtgtgatctt ccacgacgtg gatcatcttc ctgagaacga tcggaactat 1560
tacggatgcg gggagatgcc gcggcacttt gcagcgaagc tggacaagta catgtacatt 1620
cttccttata aggagttctt tggtggggtg agtggcctga cagtggagca atttaggaag 1680
atcaatggct ttcccaatgc cttctgggga tggggaggag aggatgacga cctttggaac 1740
agagttcact actctggata caatgtaacc cggccagagg gggacttggg aaaatataca 1800
tctattcccc atcaccatcg aggggaagta caatttttag gacggtataa attacttcgc 1860
tattctaagg agcgtcagtt cattgatgga ttgaacaatt tattatacac acccaaaata 1920
ctggttgaca ggctgtatac aaatatatct gtaaacctca tgccggagtt agctccagtt 1980
gaagactatt aaaaagaagt cattcccgtg gtggggtaga ccacagcact gaatcatgaa 2040
cttggcttgg aatgcactca ccgtggaagt tagagattgg tgtgtatctc agtgtcccag 2100
tctgtaaaaa gaaccagcag tccttaatgt ttacagcgtg ggctccatct gtcacactcc 2160
ccactctcct ccctgctggg agcacctccc atggcgagca ctgtcgagat acaaacccat 2220
tgcttgggat tggaagtgaa gaaaactccc tacacagaga agattttttt tatactgaaa 2280
tctataattt aattcaagag aatgctttta tttccgttca aacatatttt gtacatatgt 2340
gatgtaaatt aaatatgttc aaattgttgg aaaataagat gtcataattt tgcatgtaat 2400
tggttatgcc ttcattggac ttttatagac atttttttta tttgcatggg gggaaaaaac 2460
tgtaaattta tgtcgccaga caaaggttaa gccttgtgtg aaatgaaggg actgtcagcg 2520
acggacaaag ataataccgt aactgtttcg ttgaactggt gttcagcttt agccttcagc 2580
ctcatgggaa gaggcacagt cctctgaggg ctttgaagga aggaaggaag gactcgaata 2640
gcttttcttc ctgccaggat tacctgtttt gtttgtttgt ttttttcact tgcttgcagt 2700
ctcatcagat cttgccaagt cagcagccat attgtttatg catactgcat gagtattacc 2760
aagaaaatct tcagagttgt aaagtgacat gctgtacagg acctcttcgt gttaaatgtc 2820
tctatgtacc tctgctgtgt cagggatctt gtgcctcaaa aaaaaaatgt ccccaaggtt 2880
gtgtttatac acggtctata tttttttcaa tctcataatg aacagcatta ttatttccac 2940
tttagaagaa ccaaaagaag gtatcttctt ttaaaaacat tttttttaaa gaatgaattt 3000
atggaaaatc cagtagtgct tatacagatg aattatacta ttagatgaga aattagatgc 3060
tctattttta tgaagataaa tgcttagatt aaaacagact ttaactttaa ttctcaaacg 3120

tacatagttc cctttcacat cagccagtca cttgaactgt tttttttttc tttttttccg 3180
ctttcaagaa atctctggtg ttattcaaat ttttttttca ctaccagtat aatttaatgc 3240
cccccccccc ccccgtcccc agtggagctg ggaattgaat ctagggcctg tagacttacc 3300
ttaaaatcat tattgtatgt gtggatgctg tgacttctga actaggtctc acagcctaac 3360
gccaggtctg ctcatcctcc tcacttgggt gaggcttttg acaaccaaag aacaacaaag 3420
cccaaacagt gttctgaaaa ttatcaattc taaagattct tgaaaaatag cttgtattct 3480
catgtaagat cttatctccc atatttaaac aaattgaaaa catcgcctat ctgtcaagtg 3540
aaccctccca aagagcaaag agtgagagaa ttctgaatgt ggggaagttt gtccccttgt 3600
ctgacgtgag agcttagctt gcagttgtcc ttgtctgtca cagttttaat gctgatggcc 3660
acaagccaaa gtaattagat ccttacaccg gagaagacag acaccctatc tgttgtgaca 3720
gcagtctttc caggttcttc ttagacgtgc ttctaaaata cttacctccc aacttttttg 3780
gttactcaaa gtagtggggg gggggggaat cacaacctgt ggaaatgaag atttttgtat 3840
gctatcagtt attctctgtc tcttgtggct aatgaggtgt ttaattcgac ggaatgacag 3900
taaagtgtaa tgttaatgtt aaggctggtg agacaggact ctgggtgtgt ggtgagccca 3960
gccctggtcc ctcctcctgg ttctgtggaa tgtggtacca cgtggattct tactggggga 4020
aacacacttt ataaggtgac aggctatcca cagtcagcac cttttgcatt gatatatagt 4080
tcacagcttt aggaaaattc agaactggtg aactgcctta ctctcttagg gtaagtctgg 4140
cattaacatg gctgcgggag tcggatgtat ttctgctaaa tcttcatgct gaatctgctg 4200
tgtcagcttg tgagaaccgc cttttctaat tcagacctgg aattattttc tttttctttt 4260
tctttttttt tttttttttt tttggagctg aggactgaac ccagggcctt gagcttgcta 4320
ggcaagagct ctaccactga gctaaatcct caaccccctg gaattatttt cagtagtgaa 4380
tagtgatctc tatatgtcac cacaaatgac agttttgccc tgttaccata accacagtga 4440
atctcctcct tctatattag cccaagcaga cagtctgata gaatcaaatg cacttggtgg 4500
ctttgcactg tctgcccaca tccgctgcct tgccaacggt ggtatttaaa cgtggttttt 4560
gtttgctttt gcctgaacaa gtcaaatcga gacagttgaa acttacctga gattaggaag 4620
aatgtcctct ggtaacttac ctgtcacttg tcttagactc ggagccagtg tggccacaga 4680
tgctgggagg ccctcgtggc actggggaaa tgatcgatgc ctttgaactg aagaaagaaa 4740
gcatgactga caggccgaaa gttcccacgt ccactgtaac attctgtctg attttttaat 4800
gaagatgtca tttaattatt tgccaaaact agggttttaa tgaagtggct tccccagtcc 4860
cctttttata gttattttct ggtgtagacc cttgaggtgg ctcagggtat aaaggtgcct 4920
ctcccaagcc tgacaaccta cgcttgatcc ctggggttca cactgtagaa ggagaggact 4980
accctggtac attgttctca gacttctgta tgtgtgcggc tcgcgctcac acacacacac 5040
acacacacac acacacgcgc gcgcgcaagg caaattaaaa ggtatataaa agttaaattt 5100
ctgttttata aaacggtgtt taataagaaa atatttataa aatttaagta caaaattatc 5160
tattaaaaat tcctattcct gccagtcaat ggtggagttt gcctttaatc caagctctct 5220
ggaagcaagg caggttctct gaattggagg ccagcctggc ctgtggagtg agttccaaga 5280
cagctggggc gacacagaga aactgtctca aaaacaaaac caaatcaaaa caaaaactgc 5340
tattccagtt gatactgata accattgtaa aagagagcag aaatatcatc aatacaatat 5400
cgtccctttg ggactccctg ggggggaagc agattgtatt tgtgtcattg tatctgccct 5460
gtttctttat aaataacact ctagatttct tgccttgggt ttgcagagtt gttgagaagc 5520
aaactgtcgc cgtgtttttt ggaaggtgga agtgtactta agattattta ttatgaaatg 5580
gagtacttct atgtaagtgc gttgatgtaa acgctagtga tttctcgctt tttttttttg 5640
tcttgttttg cttctcatgg aaatgtttgt acttttttta tcattgtctt ttatgaaata 5700
caatgttcta aagaaaaaaa aaaaaaaaa 5729

<210> 71
<211> 142
<212> PRT
<213> Rattus norvegicus

<220>
<223> Light chain 3 subunit of microtubule-associated proteins 1A and 1B

<400> 71

<210> 72
<211> 861
<212> DNA
<213> Rattus norvegicus

<220>
<223> Light chain 3 subunit of microtubule-associated proteins 1A and 1B

<400> 72
ggcacgagcc aggacccccg cgcgccatgc cgtccgagaa gaccttcaaa cagcgccgga 60
gcttcgaaca aagagtggaa gatgtccggc tcatccggga gcagcacccc accaagatcc 120
cagtgattat agagcgatac aagggtgaga agcagctgcc cgtcctggac aagaccaagt 180
tccttgtacc tgatcacgtg aatatgagcg aactcatcaa gataattaga aggcgcctgc 240
agctcaatgc taaccaagcc ttcttcctcc tggtgaatgg gcacagcatg gtgagtgtgt 300
ccacacccat ctctgaagtg tacgagagcg agagagatga agacggcttc ctgtacatgg 360
tctatgcctc ccaggagacg ttcgggacag cactggctgt tacatacatg tcagctctga 420
aggcaacagc aacaggaaga gagccatgct tgtgacagac atacagccac ttccaactaa 480
agcaagcctc tgcttcctgc tacctgcatg gagcccactg tgacactcag accatccccg 540
gtcactcact cgtgtctgag aatctcagtg agagctgcct ctgtcacgga ccggaagcca 600
acacagccac ctctcgacct gctccccaca gcacccaccc tccctgcatg caagctgtcc 660
ctgctaaccc ccaatgttat gttacactgt gtaaatcccc actgctgccg tgtgtgggtt 720
gtgtacgtcg tcatgtccct ggtttataac tatggtgcgg tcgggaagga ttcctgtaat 780
gctgctctaa ggatctggct caggcagcca ttgtaggaca cctgtactct gatgcactaa 840
gtccaataaa ggcacaactg g                                           861

<210> 73
<211> 422
<212> PRT
<213> Mus musculus

<220>
<223> MD6

<400> 73
Met Glu Arg Lys Asp Phe Glu Thr Trp Leu Asp Asn Ile Ser Val Thr
1               5                   10                  15

Phe Leu Ser Leu Met Asp Leu Gln Lys Asn Glu Thr Leu Asp His Leu

20                  25                  30

Ile Ser Leu Ser Gly Ala Val Gln Leu Arg His Leu Ser Asn Asn Leu
        35                  40                  45

Glu Thr Leu Leu Lys Arg Asp Phe Leu Lys Leu Leu Pro Leu Glu Leu
    50                  55                  60

Ser Phe Tyr Leu Leu Lys Trp Leu Asp Pro Gln Thr Leu Leu Thr Cys
65                  70                  75                  80

Cys Leu Val Ser Lys Gln Arg Asn Lys Val Ile Ser Ala Cys Thr Glu
                85                  90                  95

Val Trp Gln Thr Ala Cys Lys Asn Leu Gly Trp Gln Ile Asp Asp Ser
            100                 105                 110

Val Gln Asp Ser Leu His Trp Lys Lys Val Tyr Leu Lys Ala Ile Leu
        115                 120                 125

Arg Met Lys Gln Leu Glu Asp His Glu Ala Phe Glu Thr Ser Ser Leu
    130                 135                 140

Ile Gly His Ser Ala Arg Val Tyr Ala Leu Tyr Tyr Lys Asp Gly Leu
145                 150                 155                 160

Leu Cys Thr Gly Ser Asp Asp Leu Ser Ala Lys Leu Trp Asp Val Ser
                165                 170                 175

Thr Gly Gln Cys Val Tyr Gly Ile Gln Thr His Thr Cys Ala Ala Val
            180                 185                 190

Lys Phe Asp Glu Gln Lys Leu Val Thr Gly Ser Phe Asp Asn Thr Val
        195                 200                 205

Ala Cys Trp Glu Trp Ser Ser Gly Ala Arg Thr Gln His Phe Arg Gly
    210                 215                 220

His Thr Gly Ala Val Phe Ser Val Asp Tyr Ser Asp Glu Leu Asp Ile
225                 230                 235                 240

Leu Val Ser Gly Ser Ala Asp Phe Ala Val Lys Val Trp Ala Leu Ser
                245                 250                 255

Ala Gly Thr Cys Leu Asn Thr Leu Thr Gly His Thr Glu Trp Val Thr
            260                 265                 270

Lys Val Val Leu Gln Lys Cys Lys Val Lys Ser Leu Leu His Ser Pro
        275                 280                 285

Gly Asp Tyr Ile Leu Leu Ser Ala Asp Lys Tyr Glu Ile Lys Ile Trp
    290                 295                 300

Pro Ile Gly Arg Glu Ile Asn Cys Lys Cys Leu Lys Thr Leu Ser Val
305                 310                 315                 320

Ser Glu Asp Arg Ser Ile Cys Leu Gln Pro Arg Leu His Phe Asp Gly
                325                 330                 335

Lys Tyr Ile Val Cys Ser Ser Ala Leu Gly Leu Tyr Gln Trp Asp Phe
            340                 345                 350

Ala Ser Tyr Asp Ile Leu Arg Val Ile Lys Thr Pro Glu Val Ala Asn
        355                 360                 365

Leu Ala Leu Leu Gly Phe Gly Asp Val Phe Ala Leu Leu Phe Asp Asn
    370                 375                 380

His Tyr Leu Tyr Ile Met Asp Leu Arg Thr Glu Ser Leu Ile Ser Arg
385                 390                 395                 400

Trp Pro Leu Pro Glu Tyr Arg Lys Ser Lys Arg Gly Thr Ser Phe Leu
                405                 410                 415

Ala Gly Glu Arg Pro Gly
            420


<210> 74
<211> 1825
<212> DNA
<213> Mus musculus

<220>
<223> MD6

<400> 74
tggctgtgga gcggacccgg ccgctgcgac gctctggcgg cccgagcgcg cctagtcggt 60
gtgagcccgg cgcgaggtcc cgggccccgg ggcgctcgct caggtaaata tttccataac 120
cttatggaga gaaaggactt tgagacatgg cttgataaca tttctgttac atttctttct 180
ctgatggact tgcagaaaaa tgaaactctg gaccacctga ttagtctgag tggggcagtc 240
cagctcaggc atctctccaa taacctggag actctcctca agcgggactt cctcaaactc 300
cttcccctgg agctcagttt ttatttgtta aaatggctcg atcctcagac tttactcaca 360
tgctgcctgg tctctaagca gcggaataag gtgataagtg cctgtacaga ggtgtggcag 420
actgcatgta aaaatttggg ctggcagata gatgattctg ttcaggactc attgcactgg 480
aagaaggttt atttgaaggc tattttgagg atgaagcaac tggaggacca tgaagccttt 540
gagacctctt cgttaattgg acatagtgcc agagtgtatg cactttacta caaagatgga 600
cttctctgta cagggtcaga tgacttgtct gcaaagctgt gggatgtaag cacagggcag 660
tgtgtttacg gcatccagac ccacacttgt gcagctgtga agttcgatga acagaagctt 720
gtgacaggct cctttgacaa cactgtggcc tgctgggagt ggagttccgg agccaggacc 780
cagcacttcc gggggcacac gggggcggtg ttcagtgtgg actacagtga tgaactggat 840
attttggtga gtggctctgc ggacttcgct gtgaaagtat gggctttatc tgctgggaca 900
tgcctgaata cactcactgg gcatactgaa tgggtcacca aggtggtttt gcagaagtgc 960
aaagtcaagt ctctcttgca cagccctgga gactacatcc tcttaagtgc agacaaatat 1020
gagatcaaga tttggccaat tgggagagaa atcaactgta agtgcttgaa gacactgtct 1080
gtctctgagg atagaagtat ctgcctgcag ccaagacttc attttgatgg aaaatacatt 1140
gtctgtagtt cagccctggg tctgtaccag tgggactttg ccagttatga tattctcagg 1200
gtcatcaaga cacctgaggt agcaaacttg gccttgcttg gctttggaga tgtcttcgcc 1260
ctgctgtttg acaaccacta cctatatatc atggacttga ggacagagag cctaattagc 1320
cgctggcctc tgccagagta caggaaatca aagagaggca ccagcttcct ggcaggcgaa 1380
cgtcctggtt gaatggattg gatgggcaca atgacacggg cttagtcttt gccaccagca 1440
tgcctgacca cagtattcac ctggtgttat ggaaggagca ttgctgacac caggagctac 1500
caccgctgac tgactttggg tgccagggct gcgggttttg ggtgcaatgt ctatggcagc 1560
caactgcatg aaccaaagtt ctcacctaaa ggtatcatca cgcagtgcac aatcatttat 1620
ctgtttgcca gggctggggc ggggagggct tgtttactga catacaccgc agcatgctaa 1680
tgggatacac cattgacttc atttgatctt agttatgttg gtcagtgtaa gagaggttgc 1740
atttttggat ttatctttct gagtggaata ttgagtaaag aaagttaaat gattcactaa 1800
tctgcctaat tggttgccca taaaa                                       1825


<210> 75
<211> 403

<212> PRT
<213> Rattus rattus

<220>
<223> Ribosomal protein L3

<400> 75
Met Ser His Arg Lys Phe Ser Ala Pro Arg His Gly Ser Leu Gly Phe
1               5                   10                  15

Leu Pro Arg Lys Arg Ser Ser Arg His Arg Gly Lys Val Lys Ser Phe
            20                  25                  30

Pro Lys Asp Asp Pro Ser Lys Pro Val His Leu Thr Ala Phe Leu Gly
        35                  40                  45

Tyr Lys Ala Gly Met Thr His Ile Val Arg Glu Val Asp Arg Pro Gly
    50                  55                  60

Ser Lys Val Asn Lys Lys Glu Val Val Glu Ala Val Thr Ile Val Glu
65                  70                  75                  80

Thr Pro Pro Met Val Val Val Gly Ile Val Gly Tyr Val Glu Thr Pro
                85                  90                  95

Arg Gly Leu Arg Thr Phe Lys Thr Val Phe Ala Glu His Ile Ser Asp
            100                 105                 110

Glu Cys Lys Arg Arg Phe Tyr Lys Asn Trp His Lys Ser Lys Lys Lys
        115                 120                 125

Ala Phe Thr Lys Tyr Cys Lys Lys Trp Gln Asp Asp Thr Gly Lys Lys
    130                 135                 140

Gln Leu Glu Lys Asp Phe Asn Ser Met Lys Lys Tyr Cys Gln Val Ile
145                 150                 155                 160

Arg Ile Ile Ala His Thr Gln Met Arg Leu Leu Pro Leu Arg Gln Lys
                165                 170                 175

Lys Ala His Leu Met Glu Ile Gln Val Asn Gly Gly Thr Val Ala Glu
            180                 185                 190

Lys Leu Asp Trp Ala Arg Glu Arg Leu Glu Gln Gln Val Pro Val Asn
        195                 200                 205

Gln Val Phe Gly Gln Asp Glu Met Ile Asp Val Ile Gly Val Thr Lys
    210                 215                 220

Gly Lys Gly Tyr Lys Gly Val Thr Ser Arg Trp His Thr Lys Lys Leu
225                 230                 235                 240

Pro Arg Lys Thr His Arg Gly Leu Arg Lys Val Ala Cys Ile Gly Ala
                245                 250                 255

Trp His Pro Ala Arg Val Ala Phe Ser Val Ala Arg Ala Gly Gln Lys
            260                 265                 270

Gly Tyr His His Arg Thr Glu Ile Asn Lys Lys Ile Tyr Lys Ile Gly
        275                 280                 285

Gln Gly Tyr Leu Ile Lys Asp Gly Lys Leu Ile Lys Asn Asn Ala Ser
    290                 295                 300

Thr Asp Tyr Asp Leu Ser Asp Lys Ser Ile Asn Pro Leu Gly Gly Phe
305                 310                 315                 320

Val His Tyr Gly Glu Val Thr Asn Asp Phe Ile Met Leu Lys Gly Cys
                325                 330                 335

Val Val Gly Thr Lys Lys Arg Val Leu Thr Leu Arg Lys Ser Leu Leu
            340                 345                 350

Val Gln Thr Lys Arg Arg Ala Leu Glu Lys Ile Asp Leu Lys Phe Ile
        355                 360                 365

Asp Thr Thr Ser Lys Phe Gly His Gly Arg Phe Gln Thr Met Glu Glu
    370                 375                 380

Lys Lys Ala Phe Met Gly Pro Leu Lys Lys Asp Arg Ile Ala Lys Glu
385                 390                 395                 400

Glu Gly Ala

<210> 76
<211> 1316
<212> DNA
<213> Rattus rattus

<220>
<223> Ribosomal protein L3

<400> 76
cgagacatat cggcggcgtg tggcggcgag atgtctcaca ggaaattctc agctcctagg 60
catgggtcct tgggcttctt gcctcggaag cgcagcagcc ggcatcgtgg aaaagtgaag 120
agcttcccta aggatgaccc ttccaagcct gttcacctca cagccttcct agggtacaag 180
gctggcatga cccacattgt ccgggaagtt gaccggccag gatctaaggt gaataagaaa 240
gaagttgtgg aggctgtgac cattgtggaa accccaccca tggtggttgt gggtattgtg 300
ggatatgtag aaaccccacg aggcctccgg accttcaaga ctgtatttgc tgagcacatc 360
agcgatgagt gtaaaaggcg tttctataag aattggcaca aatctaagaa gaaggctttt 420
accaagtact gtaagaaatg gcaagatgac acaggcaaga agcagctgga gaaggacttc 480
aacagcatga agaagtactg ccaggtcatc cgcataattg ctcacactca gatgcgcctg 540
cttcctctgc gccagaagaa ggcacacttg atggagatcc aggtgaatgg gggcactgta 600
gctgagaagc tagactgggc ccgagagagg ctggagcagc aggtccctgt gaaccaggtg 660
tttgggcaag atgagatgat tgacgtcatc ggcgtgacaa agggcaaagg ctacaaaggg 720
gtgaccagtc gttggcatac aaagaagctg ccccgaaaga cccacagagg tctgcgcaaa 780
gttgcttgta ttggagcttg gcatcctgcc cgtgtagcct tctctgtggc tcgagctggg 840
cagaaaggct accatcaccg aacagagatc aacaagaaga tttacaagat tggtcaaggc 900
tacctcatca aggatggtaa gctgatcaag aacaatgcat ctactgacta cgacctgtct 960
gacaagagca tcaacccact gggtggcttt gtccattatg gtgaggtgac caatgacttc 1020
atcatgctca aaggctgtgt ggtgggaacc aagaagcgag tgcttactct ccggaagtcc 1080
ttgctggtcc agaccaagcg tcgggctctt gagaagattg acctgaagtt cattgacacc 1140
acctccaaat tcggacatgg tcgcttccag accatggagg aaaagaaagc attcatggga 1200
ccgctcaaga aagatcgcat tgccaaggag gaaggtgcct gatgccagga gtactttgtg 1260
cagctggtgg ggtctcatca ataaaatatt ttcaattaaa aaaaaaaaaa aaaaaa 1316

<210> 77
<211> 263
<212> PRT

<213> Rattus rattus

<220>
<223> Ribosomal protein S4

<400> 77


<210> 78
<211> 907
<212> DNA

<213> Rattus rattus

<220>
<223> Ribosomal protein S4

<400> 78
tctttccgtt cccagcgcag catggctcgt ggtcccaaga aacatctgaa gcgcgtagcg 60
gctccaaaac actggatgct ggataagctg actggcgtgt ttgctcctcg tccatccact 120
ggtccacaca aactgaggga atgcctgcct ctgatcattt tcctaaggaa cagacttaag 180
tatgccctga ctggagatga agtaaaaaag atttgcatgc agcgattcat taagattgat 240
gggaaagtca ggaccgatat aacctaccct gctgggttta tggatgtcat cagcattgac 300
aagactggag agaacttccg tctgatctat gacaccaagg gtcgctttgc tgtccatcga 360
attacacctg aggaggccaa gtacaagttg tgcaaagtga gaaagatctt tgtgggcaca 420
aaaggaatcc cacatctggt gacccatgat gcccgtacta ttcgataccc tgatcccctc 480
atcaaggtga atgacaccat tcagattgat ttggaaacag gcaagataac tgatttcatc 540
aagttcgaca ctgggaacct gtgtatggtg actggaggtg ctaacttggg gagaattggt 600
gtaatcacca atagggagag acatcctggc tcttttgatg tggttcatgt gaaagatgcc 660
aacggcaaca gctttgccac tcgtctttcc aacatttttg ttattggcaa gggcaacaaa 720
ccgtggattt ctcttccccg aggaaaagga atccgcctta ccattgctga agagagagac 780
aagaggctag cagccaaaca gagcagtggg tgaaatggtc tctaggagac atgctggagg 840
agtatttgta cctcaacaca acaagccttt ctaagcaaca tgctagatta aacagcatga 900
tgaaatc 907

<210> 79
<211> 727
<212> PRT
<213> Rattus norvegicus

<220>
<223> Synaptic vesicle protein 2C

<400> 79
Met Glu Asp Ser Tyr Lys Asp Arg Thr Ser Leu Met Lys Gly Ala Lys
1               5                   10                  15

Asp Ile Ala Lys Glu Val Lys Lys Gln Thr Val Lys Lys Val Asn Gln
            20                  25                  30

Ala Val Asp Arg Ala Gln Asp Glu Tyr Thr Gln Arg Ser Tyr Ser Arg
        35                  40                  45

Phe Gln Asp Glu Asp Asp Asp Asp Asp Tyr Tyr Pro Pro Gly Glu Thr
    50                  55                  60

Tyr Ser Gly Glu Ala Asn Asp Asp Glu Gly Ser Ser Glu Ala Thr Glu
65                  70                  75                  80

Gly His Asp Glu Glu Asp Glu Ile Tyr Glu Gly Glu Tyr Gln Gly Ile
                85                  90                  95

Pro Ser Thr Asn Gln Gly Lys Asp Ser Ile Val Ser Val Gly Gln Pro
            100                 105                 110

Lys Gly Asp Glu Tyr Lys Asp Arg Arg Glu Leu Glu Ser Glu Arg Arg
        115                 120                 125

Ala Asp Glu Glu Glu Leu Ala Gln Gln Tyr Glu Leu Ile Ile Gln Glu
    130                 135                 140

Cys Gly His Gly Arg Phe Gln Trp Ala Leu Phe Phe Val Leu Gly Met

145                 150                 155                 160

Ala Leu Met Ala Asp Gly Val Glu Val Phe Val Val Gly Phe Val Leu
                165                 170                 175

Pro Ser Ala Glu Thr Asp Leu Cys Ile Pro Asn Ser Gly Ser Gly Trp
            180                 185                 190

Leu Gly Ser Ile Val Tyr Leu Gly Met Met Val Gly Ala Phe Phe Trp
        195                 200                 205

Gly Gly Leu Ala Asp Lys Val Gly Arg Lys Gln Ser Leu Leu Ile Cys
    210                 215                 220

Met Ser Val Asn Gly Phe Phe Ala Phe Leu Ser Ser Phe Val Gln Gly
225                 230                 235                 240

Tyr Gly Phe Phe Leu Leu Cys Arg Leu Leu Ser Gly Phe Gly Ile Gly
                245                 250                 255

Gly Ala Ile Pro Thr Val Phe Ser Tyr Phe Ala Glu Val Leu Ala Arg
            260                 265                 270

Glu Lys Arg Gly Glu His Leu Ser Trp Leu Cys Met Phe Trp Met Ile
        275                 280                 285

Gly Gly Ile Tyr Ala Ser Ala Met Ala Trp Ala Ile Ile Pro His Tyr
    290                 295                 300

Gly Trp Ser Phe Ser Met Gly Ser Ala Tyr Gln Phe His Ser Trp Arg
305                 310                 315                 320

Val Phe Val Ile Val Cys Ala Leu Pro Cys Val Ser Ser Val Val Ala
                325                 330                 335

Leu Thr Phe Met Pro Glu Ser Pro Arg Phe Leu Leu Glu Val Gly Lys
            340                 345                 350

His Asp Glu Ala Trp Met Ile Leu Lys Leu Ile His Asp Thr Asn Met
        355                 360                 365

Arg Ala Arg Gly Gln Pro Glu Lys Val Phe Thr Val Asn Lys Ile Lys
    370                 375                 380

Thr Pro Lys Gln Ile Asp Glu Leu Ile Glu Ile Glu Ser Asp Thr Gly
385                 390                 395                 400

Thr Trp Tyr Arg Arg Cys Phe Val Arg Ile Arg Thr Glu Leu Tyr Gly
                405                 410                 415

Ile Trp Leu Thr Phe Met Arg Cys Phe Asn Tyr Pro Val Arg Glu Asn
            420                 425                 430

Thr Ile Lys Leu Thr Ile Val Trp Phe Thr Leu Ser Phe Gly Tyr Tyr
        435                 440                 445

Gly Leu Ser Val Trp Phe Pro Asp Val Ile Lys His Leu Gln Ser Asp
    450                 455                 460

Glu Tyr Ala Leu Leu Thr Arg Asn Val Gln Lys Asp Lys Tyr Ala Asn
465                 470                 475                 480

Phe Ser Ile Asn Phe Thr Met Glu Asn Gln Val His Thr Gly Met Glu
                485                 490                 495

Tyr Asp Asn Gly Arg Phe Leu Gly Val Lys Phe Lys Ser Val Thr Phe
            500                 505                 510

Lys Asp Ser Val Phe Lys Ser Cys Thr Phe Asp Asp Val Thr Ser Val
        515                 520                 525

Asn Thr Tyr Phe Lys Asn Cys Thr Phe Ile Asp Thr Leu Phe Glu Asn
    530                 535                 540

Thr Asp Phe Glu Pro Tyr Lys Phe Ile Asp Ser Glu Phe Gln Asn Cys
545                 550                 555                 560

Ser Phe Leu His Asn Lys Thr Gly Cys Gln Ile Thr Phe Asp Asp Asp
                565                 570                 575

Tyr Ser Ala Tyr Trp Ile Tyr Phe Val Asn Phe Leu Gly Thr Leu Ala
            580                 585                 590

Val Leu Pro Gly Asn Ile Val Ser Ala Leu Leu Met Asp Arg Ile Gly
        595                 600                 605

Arg Leu Thr Met Leu Gly Gly Ser Met Val Leu Ser Gly Ile Ser Cys
    610                 615                 620

Phe Phe Leu Trp Phe Gly Thr Ser Glu Ser Met Met Ile Gly Met Leu
625                 630                 635                 640

Cys Leu Tyr Asn Gly Leu Thr Ile Ser Ala Trp Asn Ser Leu Asp Val
                645                 650                 655

Val Thr Val Glu Leu Tyr Pro Thr Asp Arg Arg Ala Thr Gly Phe Gly
            660                 665                 670

Phe Leu Asn Ala Leu Cys Lys Ala Ala Ala Val Leu Gly Asn Leu Ile
        675                 680                 685

Phe Gly Ser Leu Val Ser Ile Thr Lys Ala Ile Pro Ile Leu Leu Ala
    690                 695                 700

Ser Thr Val Leu Val Cys Gly Gly Leu Val Gly Leu Arg Leu Pro Asp
705                 710                 715                 720

Thr Arg Thr Gln Val Leu Met
                725


<210> 80
<211> 2622
<212> DNA
<213> Rattus norvegicus

<220>
<223> Synaptic vesicle protein 2C

<400> 80
gcgcgctgca ggaagagtgg cagaccgaag cggcctcggg ctgcaaacgg aggggcgctc 60
gcgcggcgac ggctgcaggg ctgacaccgc tcagggcagg ggggtcccag gcggctggaa 120

cgctctattc tgaactgtga gtggatgatg ctgttgcagc caagctgctg aacacactcc 180
gtggactctt ccctgctgtg ccttgcccat cggccgagat aaaatggaag actcctacaa 240
ggataggact tcactgatga agggcgccaa ggacattgcc aaagaggtga agaagcaaac 300
agtgaagaag gtgaaccagg cagtggaccg ggcccaggat gaatacaccc agcggtccta 360
cagtcgattc caggatgaag atgatgatga tgactactac ccacctggag aaacctacag 420
tggggaggcc aatgatgatg aaggctcaag tgaagccact gagggtcacg atgaagaaga 480
cgagatctat gaaggggaat accagggcat ccccagcacg aaccaaggga aggacagcat 540
agtgtctgta ggacaaccca aaggagatga gtacaaggac cgcagagagc tggagtcaga 600
gaggagggct gatgaggagg agctcgccca gcagtatgag ctgataatcc aagagtgtgg 660
ccatggccgt ttccagtggg ccctttcttt cgtcctgggc atggctctca tggcagacgg 720
cgtggaggtg tttgtggtgg gctttgtgtt acccagtgca gagacagacc tatgcatacc 780
gaattcagga tctggatggc taggcagcat agtgtacctc gggatgatgg tgggggcgtt 840
cttctgggga ggactggcag acaaagtggg aaggaagcag tctcttctga tttgcatgtc 900
cgtcaacgga ttctttgcct tcctttcttc atttgtccaa ggttacggct tctttctcct 960
ctgtcgtttg ctttcaggat tcgggattgg aggcgccatt cccactgtgt tctcctactt 1020
tgctgaagtc ctggcccggg agaagcgcgg tgagcacctc agttggctct gcatgttctg 1080
gatgattggc ggtatctatg cttcagccat ggcctgggcc atcatccccc actatgggtg 1140
gagcttcagc atgggctcag cctaccagtt ccacagctgg cgcgtcttcg tcatcgtctg 1200
tgccctcccg tgcgtctcct cggtggtggc cctcaccttc atgcccgaaa gccctcggtt 1260
cttgctggag gtaggaaaac atgatgaagc ctggatgatt ctgaagctaa ttcatgatac 1320
caacatgaga gcccggggcc agccagagaa ggtcttcacg gtaaataaaa tcaagactcc 1380
caagcaaata gatgagctga ttgagattga gagcgacaca ggaacctggt accggaggtg 1440
ttttgttcgg atccgcacag aactgtacgg aatttggttg acttttatga gatgcttcaa 1500
ctacccggtc agggaaaaca ccataaagct tacgattgtt tggttcaccc tgtcctttgg 1560
gtactatgga ctgtccgttt ggttcccaga tgtcattaaa cacctccagt ctgacgagta 1620
cgccctgctg actcggaatg tgcagaagga taaatatgca aactttagca ttaacttcac 1680
catggaaaac caggtccaca ccggaatgga atatgacaat ggcaggttcc tcggagtcaa 1740
attcaaatcg gtaaccttca aggattcagt gtttaagtcc tgcacctttg acgatgtgac 1800
ctcagtcaac acctacttca agaactgcac gtttattgat accctttttg agaacacaga 1860
ttttgagccc tataaattca tagacagcga gtttcaaaac tgctcgtttc ttcacaataa 1920
gacgggatgt cagattactt ttgacgacga ctacagtgcc tactggattt actttgtcaa 1980
ctttctcggg acattggcag tgttaccagg aaatatcgtg tctgctctcc tgatggacag 2040
gatcgggcgc ttaacgatgc taggtggctc catggtgctc tcggggatca gctgcttctt 2100
cctgtggttt ggcaccagcg aatccatgat gataggcatg ctgtgcttgt acaacggact 2160
gaccatctca gcgtggaact ctcttgatgt agtcacggtg gaactatacc ccacagaccg 2220
gagagcaacg ggctttggct tcttgaacgc actctgtaaa gcagcggccg tcctgggaaa 2280
cttaatattc ggctccttgg tcagcatcac caaagcaatc cctatcctgc tggcttccac 2340
cgtgctcgtg tgtggaggac tcgtggggct gcgcctgccc gacacaagaa cccaggttct 2400
gatgtgacaa aagccattct cttctctcac catgggtcag ccctattgcc tgactcaagg 2460
cttcagagtt tttatgtata gaaaggtggc caagtatcag aactcaaact tttgctgtga 2520
cgtaaatgta gctgtgtatt gtccccgcca gtgtgatttg cagggtcctc cccctccccc 2580
gcgccttgtt atcttttcct aattgtgatg ttcctgcttc cg 2622

<210> 81
<211> 203
<212> PRT
<213> Mus musculus

<220>
<223> ARL-6 interacting protein-1

<400> 81
Met Ala Glu Gly Asp Asn Arg Ser Ser Asn Leu Leu Ala Val Glu Thr
1               5                   10                  15

Ala Ser Leu Glu Glu Gln Leu Gln Gly Trp Gly Glu Val Met Leu Met
            20                  25                  30

Ala Asp Lys Val Leu Arg Trp Glu Arg Ala Trp Phe Pro Pro Ala Ile
        35                  40                  45

Met Gly Val Val Ser Leu Leu Phe Leu Ile Ile Tyr Tyr Leu Asp Pro
    50                  55                  60

Ser Val Leu Ser Gly Val Ser Cys Phe Val Met Phe Leu Cys Leu Ala
65                  70                  75                  80

Asp Tyr Leu Val Pro Ile Leu Ala Pro Arg Ile Phe Gly Ser Asn Lys
                85                  90                  95

Trp Thr Thr Glu Gln Gln Gln Arg Phe His Glu Ile Cys Ser Asn Leu
            100                 105                 110

Val Lys Thr Arg Arg Arg Ala Val Gly Trp Trp Lys Arg Leu Phe Ser
        115                 120                 125

Leu Lys Glu Glu Lys Pro Lys Met Tyr Phe Met Thr Met Ile Ile Ser
    130                 135                 140

Leu Ala Ala Val Ala Trp Val Gly Gln Gln Val His Asn Leu Leu Leu
145                 150                 155                 160

Thr Tyr Leu Ile Val Thr Phe Val Leu Leu Leu Pro Gly Leu Asn Gln
                165                 170                 175

His Gly Ile Ile Leu Lys Tyr Ile Gly Met Ala Lys Arg Glu Ile Asn
            180                 185                 190

Lys Leu Leu Lys Gln Lys Glu Lys Lys Asn Glu
        195                 200

<210> 82
<211> 2080
<212> DNA
<213> Mus musculus

<220>
<223> ARL-6 interacting protein-1

<400> 82
cttgctggtg ttggggggat agtgtgtgtt cgctcgttga taaccgcgag tctggaacga 60
tggcggaggg ggataaccgc agcagcaacc tgctggccgt ggagactgca agtcttgaag 120
agcagctgca aggctgggga gaggtgatgc tgatggctga caaagtcctt cgatgggaaa 180
gagcctggtt tccacctgcc atcatgggtg tggtttccct gctgttcctg attatctatt 240
atctcgatcc atctgtgctg tctggtgttt cctgctttgt tatgtttttg tgcctggctg 300
actaccttgt tcccattcta gcaccaagaa tttttggctc taataaatgg accactgaac 360
aacagcaaag atttcatgaa atctgcagta atctagtaaa aactcgacgc agagctgtgg 420
gctggtggaa acgcctcttt tccctaaagg aagaaaagcc taaaatgtac ttcatgacca 480
tgatcatttc tcttgctgcg gtggcttggg tgggacagca agttcacaac ctgcttctca 540
cctacctgat tgtgacattt gtgctgttgc ttcctggact aaaccaacat gggatcattc 600
tgaagtacat tggaatggcc aaaagggaga taaacaaact tctcaagcaa aaagaaaaga 660
aaaatgagta gtgtggctgc cgcggagtgt gccctggccc tgggcactga agtgttacag 720
tggctcgttg cttccctcac tctgcccttg ttaatagaaa ttctcacagg ccaggctctg 780
tgcgggctct agtgccaggt gtagctgggt acattccccg tcagtgtgac ttcctttcat 840
tttgacaact tccttccttt aagatgctaa gtggtgaact ttactgtttt gtacagtgaa 900
ctgtgacttt gttgatgttt gctttagcta tatcaaggca gtggtctgtg gctacaggaa 960
gctggttcta ctgtctgctt aacaaactgt ctttgtgagt acagagattg ggtttaccac 1020
ttctgctgag gagaccactt aaaattcatt cctgagtttc cactgtggga gaatacccat 1080
ggaataagat aaaaaattaa ttccacaccc tagtgtgtgt aagttcctcg ctcataggaa 1140
atgcaacagt ttgagggagc attgcccctt tgttcctgtg aggactggca ggctttgact 1200

gacctctcat tccctttgca ctgcttcagg ttagttctag tccataatgg cctttgagca 1260
gcaagctttc agaagattat atttgaggat tgtagcagtt tctttgtttc agattgtcag 1320
ctgacttaaa atgtttgtga ctttcctgta agggcagcca gggataaaag tatcctttgt 1380
tcaatgagta gaaaactagt tatctattaa tatttaagac caggaaaaaa aaaaaagagg 1440
cttaagagta agcctggaaa atctcacttg ttttcgtagt attggctgta tttcagtgcc 1500
tccagctttg cacttgagag acaagtgttt actgctgttt gactttcccc ttgacatttc 1560
tcagaaaaat gcagggctgc agctgagagc agactgctga ttggtgactc tggtctgcct 1620
agcttttgca actctttaag cttactgtga tcacgtgccc tctcagtagg gggcttttag 1680
agcagttgaa atctgcagaa ctgaacctct gagggccaaa ataggacatt tgggaacaac 1740
tccttaaccc atgaactagc tgtaacatag tttgtggatc catgcactga tgctgtgaag 1800
cctgcccctg tgtgtatctg ttctctcacc tccctagcac tgtaagcagc ggtgcctctt 1860
gtagcatgtg tgtttgtttt ttaatgtttc atgactccca ctttgatttt acatgttttc 1920
ttgtaaataa gcctgccttc ctacctggaa tttgtgtgtg tgtgactaat acatgttcta 1980
cgagttaact actttggcag ttttaatcat ttcttctttg ttttgtggta aaggggaaaa 2040
attaaaaacc agttggaacc caaaaaaaaa aaaaaaaaaa 2080

<210> 83
<211> 214
<212> PRT
<213> Rattus norvegicus

<220>
<223> Ribosomal protein L14

<400> 83
Met Val Phe Arg Arg Phe Val Glu Val Gly Arg Val Ala Tyr Ile Ser
1               5                   10                  15

Phe Gly Pro His Ala Gly Lys Leu Val Ala Ile Val Asp Val Ile Asp
            20                  25                  30

Gln Asn Arg Ala Leu Val Asp Gly Pro Cys Thr Arg Val Arg Arg Gln
        35                  40                  45

Ala Met Pro Phe Lys Cys Met Gln Leu Thr Asp Phe Ile Leu Lys Phe
    50                  55                  60

Pro His Ser Ala Arg Gln Lys Tyr Val Arg Lys Ala Trp Glu Lys Ala
65                  70                  75                  80

Asp Ile Asn Thr Lys Trp Ala Ala Thr Arg Trp Ala Lys Lys Ile Asp
                85                  90                  95

Ala Arg Glu Arg Lys Ala Lys Met Thr Asp Phe Asp Arg Phe Lys Val
            100                 105                 110

Met Lys Ala Lys Lys Met Arg Asn Arg Ile Ile Lys Thr Glu Val Lys
        115                 120                 125

Lys Leu Gln Arg Ala Ala Leu Leu Lys Ala Ser Pro Lys Lys Ala Ala
    130                 135                 140

Val Ala Lys Ala Ala Ile Ala Ala Ala Ala Ala Ala Lys Ala Lys Val
145                 150                 155                 160

Pro Ala Lys Lys Ala Thr Gly Pro Gly Gln Lys Ala Ala Ala Gln Lys
                165                 170                 175

Ala Ser Ala Gln Lys Ala Ala Gly Gln Lys Ala Ala Pro Pro Ala Lys
            180                 185                 190

Gly Gln Lys Gly Gln Lys Thr Pro Ala Gln Lys Ala Pro Ala Pro Lys
        195                 200                 205

Ala Ala Gly Lys Lys Ala
    210


<210> 84
<211> 715
<212> DNA
<213> Rattus norvegicus

<220>
<223> Ribosomal protein L14

<400> 84
tctcgctgag cccgccaaca tggtgttcag gcgtttcgtg gaggttggcc gagtggccta 60
catctccttt gggccccatg ctggaaagct ggtcgcaatc gtagatgtta ttgatcaaaa 120
cagggcttta gtggatggac cctgcacccg ggtgaggaga caggccatgc ctttcaaatg 180
catgcagctc actgacttca tcctcaagtt cccacacagt gcgcgccaga agtatgtacg 240
gaaagcttgg gagaaggcag acatcaatac aaagtgggcc gccacacgat gggccaagaa 300
aattgatgcc agagaaagga aagccaagat gacagatttt gatcggttca aagtcatgaa 360
ggcaaagaaa atgaggaaca gaataatcaa gactgaagtg aagaaactcc agagagctgc 420
tctcctgaaa gcttctccta aaaaagctgc tgttgctaag gctgccattg cggccgctgc 480
agcagctaaa gccaaggtcc cagccaagaa ggcaacagga ccaggccaga aggccgcagc 540
gcagaaggcc tctgcacaga aggctgcagg ccagaaggca gcgccccctg ctaaaggtca 600
gaagggtcag aagaccccgg cccagaaggc acctgctcca aaggcagctg gcaagaaagc 660
atgaggaggc tacacaaaga ataaaggttc tttttgactg aaaaaaaaaa aaaaa      715


<210> 85
<211> 186
<212> PRT
<213> Mus musculus

<220>
<223> Dynactin light chain

<400> 85
Met Ala Ala Leu Thr Asp Val Gln Arg Leu Gln Ser Arg Val Glu Glu
1               5                   10                  15

Leu Glu Arg Trp Val Tyr Gly Pro Gly Gly Thr Arg Gly Ser Arg Lys
            20                  25                  30

Val Ala Asp Gly Leu Val Lys Val Gln Val Ala Leu Gly Asn Ile Ala
        35                  40                  45

Ser Lys Arg Glu Arg Val Lys Ile Leu Tyr Lys Lys Ile Glu Asp Leu
    50                  55                  60

Ile Lys Tyr Leu Asp Pro Glu Tyr Ile Asp Arg Ile Ala Ile Pro Glu
65                  70                  75                  80

Ala Ser Lys Leu Gln Phe Ile Leu Ala Glu Glu Gln Phe Ile Leu Ser
                85                  90                  95

Gln Val Ala Leu Leu Glu Gln Val Asn Ala Leu Val Pro Val Leu Asp
            100                 105                 110

Ser Ala Ser Ile Lys Ala Val Pro Glu His Ala Ala Arg Leu Gln Arg
        115                 120                 125

Leu Ala Glu Ile His Ile Gln Gln Gln Asp Gln Cys Val Ala Ile Thr
    130                 135                 140

Glu Glu Ser Lys Ala Leu Leu Glu Gly Tyr Asn Lys Thr Thr Met Leu
145                 150                 155                 160

Leu Ser Lys Gln Phe Val Gln Trp Asp Glu Leu Leu Cys Gln Leu Glu
                165                 170                 175

Ala Ala Lys Gln Val Lys Pro Ala Glu Glu
            180                 185

<210> 86
<211> 819
<212> DNA
<213> Mus musculus

<220>
<223> Dynactin light chain

<400> 86
cggcacgagg cgatggcggc tctgaccgat gtgcagcggc tacagtctcg agtggaggag 60
ctggagcgct gggtgtacgg gccgggcggg acccgaggct ccaggaaggt ggctgacggt 120
ctggtcaagg tgcaggtggc tttagggaac attgccagta agagggagag ggtgaagatt 180
ctctacaaaa agattgaaga cctgattaaa tacctggatc ctgagtacat tgatcgcatt 240
gccatacctg aggcctcgaa gttgcagttc atcttagcag aggagcagtt catcctctcc 300
caggtggcac tcctggaaca agtgaacgcc ctggtgcctg tgctggacag cgcttctatc 360
aaagctgttc ctgagcatgc tgcccgcctg cagcgcctgg cggagatcca catccagcag 420
caggaccagt gtgtggcgat cactgaagag tctaaggctc tcctggaagg gtataacaag 480
actacaatgc ttctctccaa gcagtttgtg cagtgggatg agttactctg ccagctagag 540
gctgccaagc aagtgaagcc agcagaggaa tgacagccac tccccacgtc cccaccaggg 600
gaaggcaggc ggctcgaggg cctcgcacca acctgtctct ataaggcaga gacagcttca 660
tctctgaatt cagatactac ctgtctgtgt tcgactggcg gctctgaggt cagggtcagg 720
ttgaccttag agtcatgatc tgtactccct gtcaatgtat ttattccagt gacaataaac 780
ttgaacgacc actaaaaaaa aaaaaaaaaa aaaaaaaaa                        819

<210> 87
<211> 128
<212> PRT
<213> Rattus norvegicus

<220>
<223> Ubiquitin/ribosomal protein L40

<400> 87
Met Gln Ile Phe Val Lys Thr Leu Thr Gly Lys Thr Ile Thr Leu Glu
1               5                   10                  15

Val Glu Pro Ser Asp Thr Ile Glu Asn Val Lys Ala Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Gly Ile Ile Glu Pro
65                  70                  75                  80

Ser Leu Arg Gln Leu Ala Gln Lys Tyr Asn Cys Asp Lys Met Ile Cys
                85                  90                  95

Arg Lys Cys Tyr Ala Arg Leu His Pro Arg Ala Val Asn Cys Arg Lys
            100                 105                 110

Lys Lys Cys Gly His Thr Asn Asn Leu Arg Pro Lys Lys Lys Val Lys
        115                 120                 125

<210> 88
<211> 467
<212> DNA
<213> Rattus norvegicus

<220>
<223> Ubiquitin/ribosomal protein L40

<220>
<221> misc_feature
<222> (467)
<223> n is a or g or c or t

<400> 88
cggcagccaa catgcagatc ttcgtgaaga ccctgacggg caagaccatc actcttgagg 60
tcgagcccag tgacaccatc gagaatgtca aggccaagat ccaagacaag gaaggcatcc 120
cccctgacca gcagagactg atctttgctg gcaaacagct ggaggatggc cgcaccctgt 180
ccgactacaa catccagaaa gagtccacct tgcacctggt gctgcgtctt cgcggtggca 240
tcatcgagcc gtcccttcgt cagcttgccc agaagtacaa ctgtgacaag atgatctgcc 300
gcaagtgcta cgcacgcctg cacccccgtg cagtcaactg ccgcaagaag aagtgcggcc 360
acaccaacaa cctgcgcccc aagaagaagg tcaaatagag ctgggcctat acctggcctg 420
tgaccccggg accaaataaa gtccccttcc atcgactgga gcagtan 467

<210> 89
<211> 694
<212> PRT
<213> Rattus norvegicus

<220>
<223> Short type PB-cadherin

<400> 89
Met Arg Pro Arg Pro Ala Gly Ala Leu Arg Ala Gly Ala Ala Leu Ser
1               5                   10                  15

Pro Val Leu Leu Leu Leu Leu Leu Leu Gln Leu Leu Gly His Leu Trp
            20                  25                  30

Ala Ala Ser Thr Pro Ala Pro Ser Ser Leu Ser Pro Gly Thr Gln Gln
        35                  40                  45

Asp Asn Gln Leu Gly Ala Gly Arg Val Lys Arg Gly Trp Val Trp Asn
    50                  55                  60

Gln Phe Phe Val Val Glu Glu Tyr Thr Gly Thr Glu Pro Leu Tyr Val
65                  70                  75                  80

Gly Lys Ile His Ser Asp Ser Asp Glu Gly Asp Gly Thr Ile Lys Tyr
                85                  90                  95

Thr Ile Ser Gly Glu Gly Ala Gly Thr Ile Phe Leu Ile Asp Glu Leu
            100                 105                 110

Thr Gly Asp Ile His Ala Thr Glu Arg Leu Asp Arg Glu Gln Lys Thr
        115                 120                 125

Phe Tyr Thr Leu Arg Ala Gln Ala Arg Asp Arg Ala Thr Asn Arg Leu
    130                 135                 140

Leu Glu Pro Glu Ser Glu Phe Ile Ile Lys Val Gln Asp Ile Asn Asp
145                 150                 155                 160

Ser Glu Pro Arg Phe Leu His Gly Pro Tyr Ile Gly Ser Val Ala Glu
                165                 170                 175

Leu Ser Pro Thr Gly Thr Ser Val Met Gln Val Met Ala Ser Asp Ala
            180                 185                 190

Asp Asp Pro Thr Tyr Gly Ser Ser Ala Arg Leu Val Tyr Ser Val Leu
        195                 200                 205

Asp Gly Glu His His Phe Thr Val Asp Pro Lys Thr Gly Val Ile Arg
    210                 215                 220

Thr Ala Val Pro Asp Leu Asp Arg Glu Ser Gln Glu Arg Tyr Glu Val
225                 230                 235                 240

Val Ile Gln Ala Thr Asp Met Ala Gly Gln Leu Gly Gly Leu Ser Gly
                245                 250                 255

Ser Thr Thr Val Thr Ile Val Val Thr Asp Val Asn Asp Asn Pro Pro
            260                 265                 270

Arg Phe Pro Gln Lys Met Tyr Gln Phe Ser Ile Gln Glu Ser Ala Pro
        275                 280                 285

Ile Gly Thr Ala Val Gly Arg Val Lys Ala Glu Asp Ser Asp Val Gly
    290                 295                 300

Glu Asn Thr Asp Met Thr Tyr His Leu Arg Glu Glu Ser Gly Ser Gly
305                 310                 315                 320

Gly Asp Ala Phe Lys Val Thr Thr Asp Ser Asp Thr Gln Glu Ala Ile
                325                 330                 335

Ile Val Val Gln Lys His Leu Asp Phe Glu Ser Gln Gln Val His Thr
            340                 345                 350

Val Val Leu Glu Ala Leu Asn Lys Phe Val Asp Pro Arg Phe Ala Asp
        355                 360                 365

Leu Gly Thr Phe Arg Asp Gln Ala Ile Val Arg Val Ala Val Thr Asp
    370                 375                 380

Val Asp Glu Pro Pro Glu Phe Arg Pro Pro Ser Gly Leu Leu Glu Val
385                 390                 395                 400

Gln Glu Asp Ala Gln Val Gly Ser Leu Val Gly Val Val Thr Ala Arg
                405                 410                 415

Asp Pro Asp Ala Ala Asn Arg Pro Val Arg Tyr Ala Ile Asp Arg Asp
            420                 425                 430

Ser Asp Leu Glu Gln Ile Phe Asp Ile Asp Ala Asp Thr Gly Ala Ile
        435                 440                 445

Val Thr Gly Lys Gly Leu Asp Arg Glu Thr Ala Gly Trp His Asn Ile
    450                 455                 460

Thr Val Leu Ala Met Glu Ala Asp Asn His Ala Gln Leu Ser Arg Ala
465                 470                 475                 480

Ser Leu Arg Ile Arg Ile Leu Asp Val Asn Asp Asn Pro Pro Glu Leu
                485                 490                 495

Ala Thr Pro Tyr Glu Ala Ala Val Cys Glu Asp Ala Lys Pro Gly Gln
            500                 505                 510

Leu Ile Gln Thr Ile Ser Val Val Asp Arg Asp Glu Pro Gln Gly Gly
        515                 520                 525

His Arg Phe Tyr Phe Arg Leu Val Pro Glu Glu Pro Ser Asn Pro His
    530                 535                 540

Phe Ser Leu Leu Asp Ile Glu Asp Asn Thr Ala Ala Val His Thr Gln
545                 550                 555                 560

His Val Gly Phe Asn Arg Gln Glu Gln Asp Val Phe Leu Leu Pro Ile
                565                 570                 575

Leu Val Val Asp Ser Gly Pro Pro Thr Leu Ser Ser Thr Gly Thr Leu
            580                 585                 590

Thr Ile Arg Ile Cys Gly Cys Asp Ser Ser Gly Thr Ile Gln Ser Cys
        595                 600                 605

Asn Thr Thr Ala Phe Val Met Ala Ala Ser Leu Ser Pro Gly Ala Leu
    610                 615                 620

Ile Ala Leu Leu Val Cys Val Leu Ile Leu Val Val Leu Ala Leu Leu
625                 630                 635                 640

Ile Leu Thr Leu Arg Arg His His Lys Ser His Leu Ser Ser Asp Val
                645                 650                 655

Asp Glu Asp Met Arg Asp Asn Val Ile Lys Tyr Thr Leu Gly Ser Gly
            660                 665                 670

His Arg Gly Thr Ser Asn Lys Glu Asp His Gln Cys Pro Ala Ser Leu
        675                 680                 685

Ser Leu Gly Phe Lys Pro
    690

<210> 90
<211> 4153
<212> DNA
<213> Rattus norvegicus

<220>
<223> Short type PB-cadherin

<400> 90
ctcatgcttg gcggcgcggg ccaagccccg gagcatggac cagaggcacc gcagcggtac 60
tgcgtcacat ggtgacctca gcctcaggga tgccgggctg gactgggagg gggcgcggcc 120
gcacccgggc atggacaccc ggtcctggcc cgcgcccccc tcgctgaagc cccaggtcca 180
gtcactaggc agcccggccg ctccccgagg ttcccaatgg atctggatga cccctaatcc 240
cagaaagact tcaagatcct tttgctccca ctgaccacac catctgggta cagggaccat 300
ggcacaagcc ctactcagga gcagctactg ggctgcctag tgactggacc tggggcttca 360
gcgagggggg cgcgggccag gaccgggtgt ccatgcccgg gtgcggccgc gccccctccc 420
agtccagccc ggcatccctg aggctgaggt caccatgtga cgcagtaccg ctgcggtgcc 480
tctggtccat gctccggggc ttggcccgcg ccgccaagca tgaggccacg gccagcaggg 540
gcgctccggg ccggggctgc gctgtcgcct gtgctgctgc ttctgctact gctacagctg 600
ctggggcatc tgtgggccgc aagcacacct gccccatcct ccttgtcacc tgggactcag 660
caggacaacc agctgggtgc aggccgagtg aagcgcggtt gggtgtggaa ccaattcttc 720
gtggtggagg agtacacggg gacagaaccc ctgtacgtgg gcaagatcca ctcggattct 780
gacgaaggcg acgggaccat caagtacacc atctcgggcg agggtgcagg gaccatcttc 840
ctgatcgacg agctgacagg tgacatccac gccacggagc gcctggaccg ggagcagaaa 900
accttctaca cactccgggc ccaagccagg gatcgagcca ccaaccgtct gctggagccg 960
gagtcagagt tcatcatcaa agtgcaggac atcaacgaca gtgaaccacg ctttctccac 1020
ggcccctaca tcggcagtgt ggccgagctg tcacctacag gcacgtccgt gatgcaggtg 1080
atggcctctg atgcagatga ccccacttac ggcagcagtg cccggctggt gtacagcgtc 1140
ctggacggcg agcaccactt cacagtggac cccaagacag gtgtgatccg gacagcagtg 1200
cctgacctcg accgtgagag ccaggaacgc tacgaggtgg taatccaagc cacggacatg 1260
gcaggccagc tgggcggcct ctcaggctcc actacagtga ccatcgtggt tactgatgtc 1320
aacgacaacc caccccgctt tccacaaaag atgtaccagt tcagcataca ggagtcagcg 1380
cccatcggca cagccgtagg ccgagtgaag gcggaggact cagatgtggg cgagaacaca 1440
gacatgactt atcacctgag agaagaaagt ggcagtggag gagacgcatt caaggtcacc 1500
acggacagcg acactcagga ggccatcata gtggtgcaga agcacctgga cttcgagtcg 1560
cagcaggtcc acacagtggt actggaagcc ctcaacaagt ttgtggaccc ccgcttcgcc 1620
gacctgggca cattccgaga ccaggcaatc gtgcgcgtgg ccgtgaccga tgtggatgag 1680
ccccccgagt tccggccacc ctccggccta ctggaggtgc aggaggacgc gcaggtgggc 1740
tccctggtcg gcgtggtgac ggcgcgggac cccgacgccg ccaaccggcc cgtccggtac 1800
gccattgacc gagactcgga tttggagcag atcttcgaca tcgatgcgga cactggcgcc 1860
atcgtgactg gcaaggggct ggaccgtgaa accgccggct ggcacaacat tactgtgttg 1920
gccatggaag cagacaatca tgctcagctg tctcgggcat ccctacggat ccgaatcctg 1980
gatgtgaatg acaatccccc agagctggcc acaccctacg aggccgctgt gtgtgaagac 2040
gccaagccag gccagctcat tcaaaccatc agtgtggtgg acagagacga accacagggc 2100
ggccaccgct tctatttccg cctggtacca gaggaaccca gcaaccctca cttctctctg 2160
ctggacatcg aagataacac ggctgcggtg cacacccagc atgtgggctt caaccggcaa 2220
gagcaggatg tgttcctcct gcccatcctt gttgtggaca gcgggccacc caccctgagc 2280
agcacaggca ccctcaccat ccgtatctgc ggctgtgaca gttccggtac catccagtcc 2340
tgcaacacca ctgcctttgt catggctgcc tccctcagcc cgggagcact catcgctctc 2400
ttggtctgtg tcctcatcct ggtcgtgctg gctttgctga ttctcaccct caggcggcac 2460
cacaagagcc acctgagctc agatgtggat gaagacatgc gggacaacgt gatcaaatac 2520
accctgggat caggacatcg agggacaagc aacaaagagg atcatcagtg tccagctagt 2580
ctctctctgg ggttcaaacc ttgaatcaat gccctagtga gggggcactg gccatgaccc 2640
ctcaaccttt gctctgcttg tatcttgagt ctgagtcctt ccctgtacat ctgtgctcgt 2700
gttcatcagc tagtgaactg aagtgcttcc ctccccgagg ggggtcgtcc cttgtgactg 2760
accatgttgt cctaacaatg tcctgagcag aagggtcccc aatgggaacc tctcaggagg 2820
ggggtttggg gcggcggggg ggggaccagc atcttgctgg caactgtggg tggggtgggg 2880
tgcttccttc tggaatgagc atgtggctga cccccaaagg catgccccct ccccccccac 2940
ccaccttctt ggagatgtcc cttccttttg gggtagtggg gacattagga gcaacctcct 3000
agggttgttg tgagaattaa atgaactgta gcagcctgag gcagggctgg gcagaaacct 3060
cagcacatgt ttgttgaaag gtttgcaggt ggatcaagtc acccaactca cagtctcatg 3120

tgtctcgtct attctcctgc agactcctgt agcccctatg cccagggctc ttcttgctcc 3180
agctaggggc tcctaaagac aacaggccgt ccaggagact ttgagtaagg acatgagtaa 3240
tgtgtgtggc catttccaga gcactttcta tgtaatatga ccgtgttatt cctatgtaat 3300
tcgtgtgtgt caagggggta gcctcgtgtc tagagagggg tgaattggca ttgatttttt 3360
tttattagtt ttcatgactt ttaaagaggt aggtgggaaa gagaactggg agagccccac 3420
ctcactcgtg gggttgagga caatggactc ttttgctaca gtggctaaga cgggggtggg 3480
gacaaagtcg gacatggttg gcattgtgat gacctctcat ggtccggttt cccccgtccc 3540
tgcctaacgt tttttattat caaacatttc acaacgggga aatttcttgc ttcccaaggg 3600
tggtcttggg gagctgctgg gaatcagggg agggggtggt ggtggcccaa gtctggtagc 3660
atgtgctgac ctcacctctt agcaacggtg tgaccttaaa caagtcattt agcttccaag 3720
tttcagttcc tttcacacct agatagacat ttaaaaagaa aaaaaaaaag gaaagagaaa 3780
gtggggggtg gggggaggtg gggtggtggg gggtgggggg aaaacagctg gtctacccca 3840
cagagtgctt ctgaagacca aacgatgtaa ctcacaagaa agtgccttgt aaatcgccca 3900
gaaattgcca aatacttact aaccaaggag gctgtcccta ccgcccactg gccagcccga 3960
ggctcactgt tgtgagcaca cagtaggggc tgggtatttg tgctcgataa ataatttcca 4020
gagttagaaa gaaaaaaaaa gttcattcaa caaacatttt ctgagacgct gaccatgtgc 4080
cccatgcacc aggctatggc tgaggtacaa agataatgac gaacagtctc tttcggaggc 4140
taattaggac ccg 4153

<210> 91
<211> 58
<212> PRT
<213> Rattus norvegicus

<220>
<223> Guanine nucleotide binding protein gamma 3

<400> 91
Ser Thr Met Ser Ile Gly Gln Ala Arg Lys Met Val Glu Gln Leu Lys
1               5                   10                  15

Ile Glu Ala Ser Leu Cys Arg Ile Lys Val Ser Lys Ala Ala Ala Asp
            20                  25                  30

Leu Met Thr Tyr Cys Asp Ala His Ala Cys Glu Asp Pro Leu Ile Thr
        35                  40                  45

Pro Val Pro Thr Ser Glu Asn Pro Phe Arg
    50                  55

<210> 92
<211> 175
<212> DNA
<213> Rattus norvegicus

<220>
<223> Guanine nucleotide binding protein gamma 3

<400> 92
cagcactatg agtattggcc aagcacgcaa gatggtggaa cagcttaaga ttgaggccag 60
cttgtgccgg ataaaggtgt ccaaggcagc agcagacctg atgacttact gtgatgccca 120
cgcttgtgag gatcccctca tcactcctgt gcccacttcg gagaacccct tccgg 175

<210> 93
<211> 375
<212> PRT
<213> Rattus rattus

<220>
<223> Cytoplasmic gamma isoform of actin

<400> 93


<210> 94
<211> 1880
<212> DNA
<213> Rattus rattus

<220>
<223> Cytoplasmic gamma isoform of actin

<400> 94
gggggggggg gctgccgatc gcaatggaag aagaaatcgc cgccctcgtc attgacaatg 60
gctccggcat gtgcaaagct ggctttgctg gggacgacgc ccccagggcc gtgtttcctt 120
ccatcgtcgg gcgcccccga caccagggtg tcatggtggg catgggccag aaagactcgt 180
acgtgggtga tgaggcccag agcaagaggg gtattctgac cctgaagtac cctattgagc 240
acggcattgt caccaactgg gacgacatgg agaagatctg gcaccacacc ttctacaacg 300
agctgcgtgt ggcccctgag gagcacccgg tgcttctgac cgaggccccc ctgaacccca 360
aagctaacag agagaagatg acgcagataa tgtttgaaac cttcaatacc ccagccatgt 420
acgtggccat tcaggcggtg ctctccttgt atgcatctgg gcgtaccact ggcattgtca 480
tggactctgg tgacggggtc acacacacag tgcccatcta tgagggctac gcccttcccc 540
acgccatctt gcgtctggac ctggctggcc gggacctgac agactacctc atgaagatcc 600
tgactgaaag gggctacagc tttaccacca ctgctgagag ggaaattgtt cgtgacataa 660
aggagaagct gtgctatgtt gccctggatt ttgagcaaga aatggctact gctgcatcat 720
cttcctcttt ggagaagagt tatgagctgc ctgatgggca ggtgatcacc attggcaatg 780
agcgcttccg gtgtccagag gctctcttcc agccttcctt cctgggcatg gagtcctgtg 840
gcatccacga gaccaccttc aactccatca tgaagtgtga tgtggacatc cgcaaagacc 900
tgtatgccaa cacagtgctg tctggtggta ccaccatgta tccaggcatt gctgacagga 960
tgcagaagga gatcacagcc ctggctccca gcacaatgaa gattaagatc attgctcctc 1020
ctgaacgcaa gtactcagtc tggattggcg gctccatcct ggcctcactg tccaccttcc 1080
agcagatgtg gatcagcaag caggagtatg acgagtcagg cccctccatt gtccaccgca 1140
aatgcttcta gatggactga gcaggtgcca ggcatctgct gcatgagctg attctgaagt 1200
atcgatttgc cctggcaaat gtacacacct catgctagcc tcatgaaact ggaataagcc 1260
tttgaaaaga aatttgtcct tgaagcttgt atctgatatc agcactggat cgtagaactt 1320
gttgctgatt tttgaccttg tattcaagtt aactgttccc ttggtatatg tttaataccc 1380
tgtgcatatc ttgatttagt ccttagttca tgtggctcgg tcacttggtg gctggggaga 1440
tctgtggaaa agtcagtcag tccccagcct agtggatctc tgtgagcacc atgtagtgat 1500
ctgtgcaggg tattaaccaa cagcagactt ccaggatttc ccgaggctgg caagggttcc 1560
tgaactagtt accacttctt ttcttgccag tctaacaggg tgggaaagtc cgagccttag 1620
gacccagttt ctgttctggt tttttccctc ctgacctcca tgggttgtta cttgccttga 1680
gttgggaacg tttgcatcga cacctgtaaa tgtattcatc cttttaattt atgtaaggtt 1740
tttgtactca attctttaag aaatgacaaa ttttggtttt ctactgttca gtgagaacat 1800
taggccccag caacacgtca ttgtgtaaag agaaataaaa gtgctgcagt aaaaaaaaaa 1860
aaaaaccccc cccccccccc                                              1880


<210> 95
<211> 295

<212> PRT
<213> Rattus norvegicus

<220>
<223> Beta chimaerin

<400> 95
Met Leu Cys Thr Ser Pro Val Asn Cys Leu Asp Ser Val Cys Cys Thr
1               5                   10                  15

Ser Ile Asn Ile Ser Ser Leu Val Arg Arg Ala Ala Leu Thr His Asn
            20                  25                  30

Asp Asn His Phe Asn Tyr Glu Lys Thr His Asn Phe Lys Val His Thr
        35                  40                  45

Phe Arg Gly Pro His Trp Cys Glu Tyr Cys Ala Asn Phe Met Trp Gly
    50                  55                  60

Leu Ile Ala Gln Gly Val Arg Cys Ser Asp Cys Gly Leu Asn Val His
65                  70                  75                  80

Lys Gln Cys Ser Lys His Val Pro Asn Asp Cys Gln Pro Asp Leu Lys
                85                  90                  95

Arg Ile Lys Lys Val Tyr Cys Cys Asp Leu Thr Thr Leu Val Lys Ala
            100                 105                 110

His Asn Thr Gln Arg Pro Met Val Val Asp Ile Cys Ile Arg Glu Ile
        115                 120                 125

Glu Ala Arg Gly Leu Lys Ser Glu Gly Leu Tyr Arg Val Ser Gly Phe
    130                 135                 140

Thr Glu His Ile Glu Asp Val Lys Met Ala Phe Asp Arg Asp Gly Glu
145                 150                 155                 160

Lys Ala Asp Ile Ser Ala Asn Ile Tyr Pro Asp Ile Asn Ile Ile Thr
                165                 170                 175

Gly Ala Leu Lys Leu Tyr Phe Arg Asp Leu Pro Ile Pro Ile Ile Thr
            180                 185                 190

Tyr Asp Thr Tyr Thr Lys Phe Ile Glu Ala Ala Lys Ile Ser Asn Ala
        195                 200                 205

Asp Glu Arg Leu Glu Ala Val His Glu Val Leu Met Leu Leu Pro Pro
    210                 215                 220

Ala His Tyr Glu Thr Leu Arg Tyr Leu Met Ile His Leu Lys Lys Val
225                 230                 235                 240

Thr Met Asn Glu Lys Asp Asn Leu Met Asn Ala Glu Asn Leu Gly Ile
                245                 250                 255

Val Phe Gly Pro Thr Leu Met Arg Pro Pro Glu Asp Ser Thr Leu Thr
            260                 265                 270

Thr Leu His Asp Met Arg Tyr Gln Lys Leu Ile Val Gln Ile Leu Ile
        275                 280                 285

Glu Asn Glu Asp Val Leu Phe
    290                 295


<210> 96
<211> 1118
<212> DNA
<213> Rattus norvegicus

<220>
<223> Beta chimaerin

<400> 96
acgactggta tcagattaat gtcttgtata tctgaattcc gcggaattac gcagcattca 60
acagagagaa gagacctggg actaaaactg ggattccaaa aagaatcctg cagtgtggca 120
gcctgcttct caccttctca cctcggagtt ggctgaaccc agaaccaaat gctttgcacg 180
tctcccgtca actgcttgga ctcagtttgt tgcacttcga taaatatctc ttcactggtc 240
cgaagagctg ccctcacaca caatgacaac cacttcaact atgagaagac acacaacttc 300
aaggtgcaca catttcgggg cccacactgg tgtgaatact gtgccaattt tatgtggggg 360
ctcatcgccc agggagtccg gtgttcagat tgtgggttaa acgtacacaa acaatgttcc 420
aagcacgtcc ccaacgactg ccagcccgat ctcaagagga tcaagaaagt atactgttgt 480
gatctcacca cacttgtaaa agctcacaac actcagagac ccatggtggt ggacatatgt 540
attcgggaaa ttgaagcaag aggattaaaa tcagaagggc tttacagagt ctctgggttc 600
acagaacaca ttgaagatgt caagatggcg ttcgacagag atggtgaaaa ggcggatatc 660
tctgctaaca tctacccaga cataaacatc atcaccggag ccctgaaact gtacttcaga 720
gacttaccca tccccatcat cacatatgac acctatacca agtttataga ggcagcaaaa 780
atctccaatg cagatgagag gcttgaagct gtccatgagg tcctgatgct gctgcctcct 840
gcccactatg agaccctgcg ctacctgatg atccatctca aaaaggttac catgaatgaa 900
aaggacaatt taatgaacgc agaaaatctg gggatcgtgt ttgggcccac cctgatgaga 960
ccccctgaag acagcaccct caccaccctc catgacatgc ggtaccaaaa gctgatcgtg 1020
cagattttaa tagaaaacga agatgttctg ttttagtcca tcagggaagt gcagctgata 1080
gagctagtgg aataaaaacg tttctcaaaa aaaaaaaa                         1118


<210> 97
<211> 110
<212> PRT
<213> Rattus norvegicus

<220>
<223> Parvalbumin

<400> 97
Met Ser Met Thr Asp Leu Leu Ser Ala Glu Asp Ile Lys Lys Ala Ile
1               5                   10                  15

Gly Ala Phe Thr Ala Ala Asp Ser Phe Asp His Lys Lys Phe Phe Gln
            20                  25                  30

Met Val Gly Leu Lys Lys Lys Ser Ala Asp Asp Val Lys Lys Val Phe
        35                  40                  45

His Ile Leu Asp Lys Asp Lys Ser Gly Phe Ile Glu Glu Asp Glu Leu
    50                  55                  60

Gly Ser Ile Leu Lys Gly Phe Ser Ser Asp Ala Arg Asp Leu Ser Ala
65                  70                  75                  80

Lys Glu Thr Lys Thr Leu Met Ala Ala Gly Asp Lys Asp Gly Asp Gly
                85                  90                  95

Lys Ile Gly Val Glu Glu Phe Ser Thr Leu Val Ala Glu Ser
            100                 105                 110

<210> 98
<211> 961
<212> DNA
<213> Rattus norvegicus

<220>
<223> Parvalbumin

<400> 98
tttcccaaca ggatctccca ccagcccagc ttttctatat aggctctgac ctctggtcat 60
ccaagttgca ggatgtcgat gacagacttg ctcagcgctg aggacatcaa gaaggcgata 120
ggagccttta ctgctgcaga ctccttcgac cacaaaaagt tcttccagat ggtgggcctg 180
aagaaaaaga gtgcggatga tgtgaagaag gtgttccaca ttctggacaa agacaaaagt 240
ggcttcattg aggaggatga gctggggtcc attctgaagg gcttctcctc agatgccaga 300
gacttgtctg ctaaggaaac aaagacgctg atggctgctg gagacaagga cggggacggc 360
aagattgggg ttgaagaatt ctccactctg gtggccgaaa gctaagtggc gctgactgct 420
tgggtctccc acctctccac cccccatgcc ccatctcagc ccttctcgcg gccctcctgg 480
gtttctgttc agtttgttta tgttattttt tactccccca tcctttatgg ccctcgaatg 540
acaccactct tctggaaaat gctggagaaa caataaaggc tgtacccatc ggacaccacc 600
tgtagggagg acccaggcct ggtaggtgtt ggtttggcaa gtttttccgg acagcagtgg 660
gggtatagta gaaaaagtga gagagagcga aggaccacgc cctgatattt cctgcctgct 720
tggtaccgag tggtcacgtg ggccaccttg ttcagtcttt gtgcctttcc tacaagggga 780
tgggatggcg caggggattt taaagatgca gaaactgcct tttaaagagc agaacggaag 840
gggctgagtc cacaggtgat tactttatgt ccctgaggaa taactaggtc gaaggactca 900
aatgacactc tatcaattgc ttttgacttt gctgtgataa aattcctgat aagagaaact 960
t 961

<210> 99
<211> 245
<212> PRT
<213> Mus musculus

<220>
<223> BAP31

<400> 99
Met Ser Leu Gln Trp Thr Thr Val Ala Thr Phe Leu Tyr Ala Glu Val
1               5                   10                  15

Phe Ala Val Leu Leu Leu Cys Ile Pro Phe Ile Ser Pro Lys Arg Trp
            20                  25                  30

Gln Lys Val Phe Lys Ser Arg Leu Val Glu Leu Val Val Thr Tyr Gly
        35                  40                  45

Asn Thr Phe Phe Val Val Leu Ile Val Ile Leu Val Leu Leu Val Ile
    50                  55                  60

Asp Ala Val Arg Glu Ile Leu Lys Tyr Asp Asp Val Thr Glu Lys Val
65                  70                  75                  80

Asn Leu Gln Asn Asn Pro Gly Ala Met Glu His Phe His Met Lys Leu
                85                  90                  95

Phe Arg Ala Gln Arg Asn Leu Tyr Ile Ala Gly Leu Ser Leu Leu Leu
            100                 105                 110

Ser Phe Leu Leu Arg Arg Leu Val Thr Leu Ile Ser Gln Gln Ala Thr
        115                 120                 125

Leu Leu Ala Ser Asn Glu Ala Phe Lys Lys Gln Ala Glu Ser Ala Ser
    130                 135                 140

Glu Ala Ala Lys Lys Tyr Met Glu Glu Asn Asp Gln Leu Lys Lys Gly
145                 150                 155                 160

Ala Ala Glu Asp Gly Asp Lys Leu Asp Ile Gly Asn Thr Glu Met Lys
                165                 170                 175

Leu Glu Glu Asn Lys Ser Leu Lys Asn Asp Leu Arg Lys Leu Lys Asp
            180                 185                 190

Glu Leu Ala Ser Thr Lys Lys Lys Leu Glu Lys Ala Glu Asn Glu Ala
        195                 200                 205

Leu Ala Met Gln Lys Gln Ser Glu Gly Leu Thr Lys Glu Tyr Asp Arg
    210                 215                 220

Leu Leu Glu Glu His Ala Lys Leu Gln Ala Ser Val Arg Gly Pro Ser
225                 230                 235                 240

Val Lys Lys Glu Glu
                245

<210> 100
<211> 1132
<212> DNA
<213> Mus musculus

<220>
<223> BAP31

<400> 100
ggatgagttt gcagtggact acagttgcca ccttcctcta cgcagaggtc tttgctgtgt 60
tgcttctctg cattcccttc atttctccaa aaagatggca gaaggttttt aaatcccggc 120
tggtggagtt ggtagtgacc tatggcaaca ctttctttgt ggttctcatc gtcatccttg 180
tactgttggt tattgatgct gtacgagaga tcctgaaata cgatgatgtg acagaaaagg 240
tgaacctcca gaacaatcca ggtgccatgg agcacttcca catgaagctt ttccgtgctc 300
agaggaatct ctatattgct ggcctttcct tgctgctgtc cttcctgctt agacgcctgg 360
tgactctcat ctcccagcag gccacactgc tggcctccaa tgaagccttt aaaaagcagg 420
cagaaagtgc cagtgaggcg gccaagaaat acatggagga gaatgatcag ctaaagaagg 480
gagctgccga ggatggagac aagttggata ttgggaatac tgaaatgaag ttagaggaga 540
acaagagcct gaagaatgac ctgaggaagc taaaagatga gctggccagc accaagaaaa 600
aacttgagaa agctgaaaac gaggctctgg ctatgcagaa gcagtctgag ggccttacca 660
aagaatatga ccgcctgcta gaagaacatg ccaaactgca ggcatcagta cgtggtccct 720
cagtcaagaa ggaggagtaa aggcttggtg tttccctgcc tgccgctggc ttctacctga 780
cccatgctta ctgcttcctt ggagcccaga ctatccctct ggtacttggg tttattccct 840
acttccccaa ttttcttcca tggcttatag atcattattt tggcaccatt acacatactg 900
ctcttatacc aaaagggacc tgattgttgt ttattcagag tacttttgcc actgttctgc 960
ctggctaggg cactttccac tcctggaagt gtagaaaagc actggtgacc tggcctgcag 1020
tttgaacccc tttttatttt gcaatgtacc ctaaaggagg ctgctgtgaa gcaggtcaac 1080
tgttttatcc tgaggggaat aaatgttgtt atgttataaa aaaaaaaaaa aa         1132

<210> 101
<211> 928

<212> PRT
<213> Rattus norvegicus

<220>
<223> Protocadherin 2C

<400> 101
Met Gln Arg Arg Glu Asp Cys Lys Gly Leu Val Phe Leu Gly Val Leu
1               5                   10                  15

Leu Gly Met Leu Trp Lys Ala Gly Ser Thr Gln Ile Arg Tyr Ser Val
            20                  25                  30

Pro Glu Glu Leu Glu Lys Gly Ser Arg Val Gly Asn Ile Ser Lys Asp
        35                  40                  45

Leu Gly Leu Lys Ser Cys Glu Leu Ala Glu Arg Gly Val Arg Ile Val
    50                  55                  60

Ser Arg Gly Arg Ala Gln Leu Phe Ala Leu Asn Pro Arg Ser Gly Ser
65                  70                  75                  80

Leu Val Thr Ala Gly Arg Ile Asp Arg Glu Glu Leu Cys Met Gly Ser
                85                  90                  95

Ile Lys Cys Gln Leu Asn Leu Glu Ile Leu Met Glu Asp Ile Gly Lys
            100                 105                 110

Ile Tyr Gly Val Glu Ile Glu Val Arg Asp Ile Asn Asp Asn Ala Pro
        115                 120                 125

Tyr Phe Arg Glu Ser Glu Leu Glu Ile Lys Met Ser Glu Asn Ala Ala
    130                 135                 140

Ala Gly Met Arg Phe Pro Leu Pro His Ala Trp Asp Pro Asp Ile Gly
145                 150                 155                 160

Lys Asn Ser Leu Gln Ser Tyr Gln Leu Ser Pro Asn Ala His Phe Ser
                165                 170                 175

Leu Glu Val Gln Asn Gly Ala Asp Gly Asn Lys Tyr Pro Glu Leu Val
            180                 185                 190

Leu Glu Ser Ser Leu Asp Arg Glu Glu Lys Ala Ala His Leu Leu Val
        195                 200                 205

Leu Thr Ala Ser Asp Ala Gly Asp Pro Val Arg Thr Gly Thr Ala Arg
    210                 215                 220

Ile Arg Val Met Val Val Asp Ala Asn Asp Asn Ala Pro Ala Phe Ala
225                 230                 235                 240

Arg Ser Glu Tyr Arg Val Ser Trp Glu Asn Val Ala Val Gly Thr Gln
                245                 250                 255

Leu Leu Leu Val Asn Ala Thr Asp Pro Asp Glu Gly Ala Asn Ala Glu
            260                 265                 270

Val Ile Tyr Ser Phe Arg Tyr Val Asp Asp Lys Ala Ala Lys Val Phe
        275                 280                 285

Lys Leu Asp Ser Asn Leu Gly Thr Ile Ser Thr Ile Gly Glu Leu Asn
    290                 295                 300

His Glu Glu Ser Gly Phe Tyr Glu Met Glu Val Gln Ala Thr Asp Asn
305                 310                 315                 320

Ala Gly Tyr Ser Ala Arg Ala Lys Val Leu Val Thr Val Leu Asp Val
                325                 330                 335

Asn Asp Asn Ala Pro Glu Val Ala Ile Thr Ser Leu Thr Asn Ser Val
            340                 345                 350

Pro Glu Asn Ser Pro Gln Gly Thr Leu Ile Ala Leu Leu Asn Val Asn
        355                 360                 365

Asp Gln Asp Ser Gly Glu Asn Gly Gln Val Ile Cys Ser Ile Gln Glu
    370                 375                 380

Asn Leu Pro Phe Lys Leu Glu Lys Ser Tyr Gly Asn Tyr Tyr Arg Leu
385                 390                 395                 400

Val Thr Asp Ala Val Leu Asp Arg Glu Glu Val Pro Ser Tyr Asn Ile
                405                 410                 415

Thr Met Thr Ala Thr Asp Arg Gly Ser Pro Pro Leu Thr Thr Glu Thr
            420                 425                 430

His Leu Ala Leu Asp Ile Ala Asp Thr Asn Asp Asn Ser Pro Val Phe
        435                 440                 445

Leu Gln Ala Ser Tyr Trp Ala Tyr Ile Pro Glu Asn Asn Pro Arg Gly
    450                 455                 460

Ala Ser Ile Ala Ser Val Thr Ala His Asp Pro Asp Ser Asp Lys Asn
465                 470                 475                 480

Ala Gln Val Thr Tyr Ser Leu Ala Glu Asp Thr His Gln Gly Val Pro
                485                 490                 495

Leu Ser Ser Tyr Val Ser Ile Asn Ser Asp Thr Gly Val Leu Tyr Ala
            500                 505                 510

Leu His Ser Phe Asp Tyr Glu Gln Phe Pro Asp Leu Gln Leu Gln Val
        515                 520                 525

Ile Ala Arg Asp Ser Gly Asp Pro Pro Leu Ser Ser Asn Val Ser Leu
    530                 535                 540

Ser Leu Phe Val Leu Asp Gln Asn Asp Asn Val Pro Glu Ile Leu Tyr
545                 550                 555                 560

Pro Thr Leu Pro Thr Asp Gly Ser Thr Gly Val Glu Leu Ala Pro Arg
                565                 570                 575

Ser Ala Glu Pro Gly Tyr Leu Val Thr Lys Val Val Ala Val Asp Arg
            580                 585                 590

Asp Ser Gly Gln Asn Ala Trp Leu Ser Tyr Arg Leu Leu Lys Ala Ser
        595                 600                 605

Glu Pro Gly Leu Phe Ser Val Gly Leu His Thr Gly Glu Ile Ser Thr

610                 615                 620

Ala Arg Ala Leu Met Asp Arg Asp Ala Leu Lys Gln Asn Leu Val Val
625                 630                 635                 640

Ser Val Gln Asp His Gly Gln Pro Pro Leu Ser Ala Thr Val Thr Leu
                645                 650                 655

Thr Val Ala Ile Ala Asp Ser Ile Pro Asp Val Leu Ala Asp Leu Asp
            660                 665                 670

Asn Leu Glu Ser Pro Ser Thr Ser Glu Thr Ser Gly Leu Thr Leu Tyr
        675                 680                 685

Leu Val Val Ala Val Ala Ala Val Ser Thr Val Phe Leu Val Phe Val
    690                 695                 700

Leu Val Leu Leu Ala Leu Arg Leu Arg Arg Trp His Ser Leu Arg Leu
705                 710                 715                 720

Leu Arg Ala Gly Gly Pro Gly Leu Ala Asp Val Ala Ala Ser His Phe
                725                 730                 735

Val Gly Val Asp Gly Val Gln Ala Phe Leu Gln Thr Tyr Ser His Glu
            740                 745                 750

Val Ser Leu Thr Ala Gly Ser Arg Lys Ser His Leu Ile Phe Pro Gln
        755                 760                 765

Pro Asn Tyr Ala Asp Thr Leu Ile Ser Gln Glu Ser Cys Glu Lys Thr
    770                 775                 780

Glu Pro Leu Leu Pro Ser Gly Asp Ser Val Phe Ser Lys Asp Asn His
785                 790                 795                 800

Ala Leu Asn Gln Gln Ala Pro Pro Asn Thr Asp Trp Arg Phe Ser Gln
                805                 810                 815

Ala Gln Arg Pro Gly Thr Ser Gly Ser Gln Asn Gly Asp Glu Thr Gly
            820                 825                 830

Thr Trp Pro Asn Asn Gln Phe Asp Thr Glu Met Leu Gln Ala Met Ile
        835                 840                 845

Leu Ala Ser Ala Ser Glu Ala Ala Asp Gly Ser Ser Thr Leu Gly Gly
    850                 855                 860

Gly Ala Gly Thr Met Gly Leu Ser Ala Arg Tyr Gly Pro Gln Phe Thr
865                 870                 875                 880

Leu Gln His Val Pro Asp Tyr Arg Gln Asn Val Tyr Ile Pro Gly Ser
                885                 890                 895

Asn Ala Thr Leu Thr Asn Ala Ala Gly Lys Arg Asp Gly Lys Ala Pro
            900                 905                 910

Ala Gly Gly Asn Gly Asn Lys Lys Lys Ser Gly Lys Lys Glu Lys Lys
        915                 920                 925

<210> 102
<211> 4643
<212> DNA
<213> Rattus norvegicus

<220>
<223> Protocadherin 2C

<400> 102
ctcgtcccac ctcctaactg tcgtgctcca aggcttcaaa agcagattct ggttcaggtg 60
atcagaggca gaattggaaa taatcctcag ttggtgatga tccccatgca acggcgggag 120
gactgcaaag ggctggtctt cctgggagtc ctcctcggga tgctgtggaa agctggatcc 180
acccagatac gctattcggt tcccgaggag ctggagaaag gctctagggt gggcaacatc 240
tccaaggatc tggggctgaa gtcctgcgag ctggcggagc gaggggtccg catcgtctcc 300
agaggtaggg ctcaactttt cgctctaaac cctcgaagcg gcagcctggt caccgcgggc 360
aggatagacc gggaggagct gtgtatgggg tccatcaagt gtcagttaaa tctagagatt 420
ctgatggaag atatagggaa aatatacgga gtagaaatag aagtgaggga cattaacgac 480
aacgctccct acttccgtga aagtgaatta gaaataaaaa tgagcgaaaa cgccgccgct 540
gggatgcggt tccctcttcc ccacgcttgg gatccagata tcgggaagaa ctcgcttcag 600
agctaccagc tcagcccgaa tgctcacttc tcactggagg tgcagaacgg agctgatggt 660
aacaagtacc cagaattggt gctggagagc tcgctggatc gggaggaaaa ggccgctcat 720
ctcctagtcc tcaccgcttc cgacgcggga gacccggttc gcactggcac agctcgcatc 780
cgcgtgatgg ttgtggatgc gaacgacaac gcacctgcgt ttgctcggtc cgagtaccgc 840
gtgagctggg agaacgtggc tgtgggcact cagctgctgc tggtcaacgc caccgaccca 900
gacgaaggcg ccaatgcgga agtgatctat tccttccggt acgtggacga caaggcagcg 960
aaagttttca agctggacag taacttgggg acaatatcaa caatagggga gctgaatcat 1020
gaagaatcgg ggttctacga gatggaggtg caagctacag ataacgcggg atattctgca 1080
cgggccaagg tcctggtcac agttctggac gtgaacgaca atgcccctga agtagccatc 1140
acgtccctca ccaactctgt cccagaaaac tctccccaag gtacattaat agcactttta 1200
aacgtaaatg atcaagattc tggggaaaat ggacaggtaa tctgttccat ccaagagaat 1260
ctgcccttta agttagaaaa gtcttacgga aactattata gattagtcac agatgcagtc 1320
ctggaccgag aagaggttcc tagttacaac atcacaatga ccgccactga caggggaagt 1380
ccgcccctga caacagaaac tcacctcgca ctggacatag cagacacgaa cgataactcg 1440
cccgttttcc ttcaggcctc atactgggcc tacatcccag agaataaccc tagaggggcc 1500
tctatcgcat ccgtgaccgc ccacgacccc gacagtgaca aaaatgccca agtcacttac 1560
tccctagctg aggacaccca ccagggcgtg cccctttcct cttacgtttc catcaactcg 1620
gacactggtg tcctgtacgc actgcattcc tttgactacg agcagttccc agacctacaa 1680
ctgcaagtga tagcgcgtga cagcggggac ccgccactca gcagcaacgt gtcactgagc 1740
ttgttcgtgc tggatcagaa tgacaacgtc cccgaaatct tgtaccccac tctccctacc 1800
gacggttcta ctggagtgga gctagcaccc cgctcagcag agcctggcta tctggtgaca 1860
aaggtggtgg cagtggacag agactcagga cagaacgcct ggctgtccta ccgtctgcta 1920
aaggccagcg agccagggct cttctccgtg gggctgcaca cgggcgagat aagcacggct 1980
cgggccctga tggacagaga tgctctcaaa cagaacctcg tagtgtctgt gcaggaccac 2040
ggccagcctc ctctctctgc cactgtcacg ctcactgtgg cgatcgccga cagcatcccc 2100
gacgtcctgg cagatctaga caatctagaa tctccttcca cctccgaaac ttcagggctc 2160
actctctatc tggtggtggc cgtggcggcg gtctccaccg tcttcctggt ctttgttctg 2220
gtgctgctgg cgctcaggct gcggcgctgg cactcactgc gcctgctccg ggctggggga 2280
cccggattgg ctgacgttgc agcctctcac tttgtgggcg tagacggagt gcaagctttc 2340
ttgcagactt attcgcacga ggtctcgctc actgctggct cccgaaagag tcacttgatt 2400
ttcccgcagc ccaactatgc agacacgctc atcagccagg agagctgcga gaaaaccgag 2460
cctcttttac catcgggtga ttcggttttt tctaaagaca atcatgcatt aaatcagcaa 2520
gccccgccca acactgactg gcgtttctct caagcccaga gacccggcac gagcggatcc 2580
caaaatggtg atgaaactgg cacctggccc aacaaccagt ttgatacaga gatgctgcaa 2640
gccatgatct tggcctctgc cagcgaagct gctgatggga gctctaccct gggagggggc 2700
gctggcacca tgggtctgag cgctcgatac ggaccccagt ttaccctgca gcacgtgcct 2760
gactaccgcc agaacgtgta catccctggc agcaatgcca cgctgaccaa tgccgctggc 2820
aaacgagatg gcaaggctcc agcaggtggt aatggcaaca agaagaaatc gggcaagaaa 2880
gagaagaagt aatatggagg ccaggccttg aaccacaagg cagcctccct ccccagccag 2940
tccagctcgt ccttacttgt acccaggcct cagaatttca gggctcaccc caggatactg 3000

gtaggagcca gggccatgct ccccgttggg aaacagaaac acgtgcccaa gccaacaccc 3060
cctctctata ccctagggag ttgaatatgc aaagagagtt ctgctgggtc ccccctattc 3120
aatcagtgat tgtacccaca tgggtagcag ggtttgtgtg tgtggacata cacacacaca 3180
cacacacaca cacacacaaa ccatgccaca ctcccttagt tacagctgaa ctcctccatc 3240
ttccaaccca ggcaggctta tccatctcct gccgccctct ccccacccca cccactcctt 3300
cagtccctct ctttgagcaa ggtagctggg ctgttggggt gaccggtgag ctaagccggc 3360
tcctagttct gaacggtagg gagagtatta caatcttttg gcctctcctt cagttcagct 3420
ttcccccaaa gcatggtttg gtaccagtct tctcttttcc ttccagagcc tgagaccaat 3480
gcttaagttt tgggggaaca ggcaccctcc ctctggtact gatgatgctt gctggatttt 3540
gggaaggcat tttgctacta agcctctttt caatgcctgg ggacaactag tcttttgttt 3600
ttcattgttt gatgttccca ctgcatgctg tgacttccct acacctcaaa gaagagactc 3660
ctttgcatgt ctggagacag tagggggtgg taaactaaag ggaagttgag agtgtatact 3720
gctgggagtg gggcagtgga caaaacagcc tgttaatagg gtcttgcaag gggctctgta 3780
tgtacccagg ggactggctc aatcctcaca ttctagccat agacaaacac caggccagac 3840
ccctccattc tggttcagcc tgggcagctt gggctgagcc accaggacca atggatttaa 3900
gctgacattt cagtccaaga acacgacttc taagtgagtt taagaccaga gaggaaagag 3960
gggcctctgt gggtgctggg tactccagag gtgcccttgg tgggaggacc agtggtctta 4020
gcaggaaggg gggcccagca aggtcattct tggaccctgg gtctagtcca gtagctagaa 4080
aaagggacca agtggccata aagtcccagc caatgatggg gcttttccag tgggcccctg 4140
tagacctcaa gcccctggcc tccaccttac caggtgccat ttcttctcag aaggccactg 4200
cccaggcccc caggccgccc ccttgtggcc agagaacggt taaagccccc cagtgcctcc 4260
ttgtgcatag aacttcctct gccctagccc ttacagtagt agtagaagat ccccctcccc 4320
cgctggtgta gaatagccaa tagtatagtg tggtgtgctt ttacgtgatg gcgagtgggc 4380
agcgggcggt gggctgtaca cagccgtctg tcctttgaat ctcaatctgc ctgcgcggcc 4440
cgtgctgtgt tttgtgctgt gtccacacgc tgcggcgacc cctccctgta ctgacttctc 4500
tataaaaagc ttctcttcgc atagtcacgt agctcctgcc catccccctt ccgatatctc 4560
tcgcaagttt tatactctaa tatttatatg gcttttttct tcgaaaatta aaaataaaaa 4620
aatggtttct tctgaaaaaa aaa 4643

<210> 103
<211> 165
<212> PRT
<213> Rattus rattus

<220>
<223> Ribosomal protein L12

<400> 103
Met Pro Pro Lys Phe Asp Pro Asn Glu Ile Lys Val Val Tyr Leu Arg
1               5                   10                  15

Cys Thr Gly Gly Glu Val Gly Ala Thr Ser Ala Leu Ala Pro Lys Ile
            20                  25                  30

Gly Pro Leu Gly Leu Ser Pro Lys Lys Val Gly Asp Asp Ile Ala Lys
        35                  40                  45

Ala Thr Gly Asp Trp Lys Gly Leu Arg Ile Thr Val Lys Leu Thr Ile
    50                  55                  60

Gln Asn Arg Gln Ala Gln Ile Glu Val Val Pro Ser Ala Ser Ala Leu
65                  70                  75                  80

Ile Ile Lys Ala Leu Lys Glu Pro Pro Arg Asp Arg Lys Lys Gln Lys
                85                  90                  95

Asn Ile Lys His Asn Gly Asn Ile Thr Phe Asp Glu Ile Val Asn Ile
            100                 105                 110

Ala Arg Gln Met Arg His Arg Ser Leu Ala Arg Glu Leu Ser Gly Thr

115                 120                 125

Ile Lys Glu Ile Leu Gly Thr Ala Gln Ser Val Gly Cys Asn Val Asp
    130                 135                 140

Gly Arg His Pro His Asp Ile Ile Asp Asp Ile Asn Ser Gly Ala Val
145                 150                 155                 160

Glu Cys Pro Ala Ser
                165


<210> 104
<211> 664
<212> DNA
<213> Rattus rattus

<220>
<223> Ribosomal protein L12

<400> 104
ctttcggttc ggaggaggca acggtgcaac tttcttcggt cgtcccgaat ccgggttcat 60
ccgacaccag ccacctccac catgccgccc aagttcgacc ccaacgagat caaagtcgtg 120
tacttgaggt gcaccggagg cgaggtcggc gccacatccg ccttggcccc taagatcggt 180
cctctgggtc tgtctcccaa aaaagttggt gatgacatcg ccaaggctac cggtgactgg 240
aaaggcctca ggattacagt gaaactgacc atccagaaca gacaggccca gattgaggtg 300
gtgccctctg cctctgccct gatcatcaaa gccctcaagg agccaccaag agacaggaag 360
aagcagaaaa acattaaaca caatggaaac atcacttttg atgagattgt caacattgcc 420
cggcagatga gacaccggtc tttggccaga gaactttctg gaactatcaa ggagatcctg 480
ggtactgcac agtctgtggg ctgcaatgtg gacggccgcc accctcatga catcatagat 540
gacatcaaca gtggtgcggt ggagtgccca gctagttaag aagcaacgag aaggggttgg 600
gaatttagct cagtggtaga gcgcttgcca agcccaaggc cctgggttca gtccccagct 660
ccgg                                                              664


<210> 105
<211> 244
<212> PRT
<213> Mus musculus

<220>
<223> Cell surface glycoprotein A15

<400> 105
Met Glu Thr Lys Pro Val Ile Thr Cys Leu Lys Thr Leu Leu Ile Ile
1               5                   10                  15

Tyr Ser Phe Val Phe Trp Ile Thr Gly Val Ile Leu Leu Ala Val Gly
            20                  25                  30

Val Trp Gly Lys Leu Thr Leu Gly Thr Tyr Ile Ser Leu Ile Ala Glu
        35                  40                  45

Asn Ser Thr Asn Ala Pro Tyr Val Leu Ile Gly Thr Gly Thr Thr Ile
    50                  55                  60

Val Val Phe Gly Leu Phe Gly Cys Phe Ala Thr Cys Arg Gly Ser Pro
65                  70                  75                  80

Trp Met Leu Lys Leu Tyr Ala Met Phe Leu Ser Leu Val Phe Leu Ala
                85                  90                  95

Glu Leu Val Ala Gly Ile Ser Gly Phe Val Phe Arg His Glu Ile Lys
            100                 105                 110

Asp Thr Phe Leu Arg Thr Tyr Thr Asp Ala Met Gln Asn Tyr Asn Gly
        115                 120                 125

Asn Asp Glu Arg Ser Arg Ala Val Asp His Val Gln Arg Ser Leu Ser
    130                 135                 140

Cys Cys Gly Val Gln Asn Tyr Thr Asn Trp Ser Ser Ser Pro Tyr Phe
145                 150                 155                 160

Leu Asp His Gly Ile Pro Pro Ser Cys Cys Met Asn Glu Thr Asp Cys
                165                 170                 175

Asn Pro Leu Asp Leu His Asn Leu Thr Val Ala Ala Thr Lys Val Asn
            180                 185                 190

Gln Lys Gly Cys Tyr Asp Leu Val Thr Ser Phe Met Glu Thr Asn Met
        195                 200                 205

Gly Ile Ile Ala Gly Val Ala Phe Gly Ile Ala Phe Ser Gln Leu Ile
    210                 215                 220

Gly Met Leu Leu Ala Cys Cys Leu Ser Arg Phe Ile Thr Ala Asn Gln
225                 230                 235                 240

Tyr Glu Met Val

<210> 106
<211> 1720
<212> DNA
<213> Mus musculus

<220>
<223> Cell surface glycoprotein A15

<400> 106
ggagaatgga gaccaaacct gtgataacct gtctcaaaac cctcctcatc atctactcct 60
tcgtcttctg gatcactggg gtgatcctgt tggccgttgg agtctgggga aagctgactt 120
tgggaaccta tatctccctg attgctgaga actccacaaa tgctccctat gtgctcattg 180
gaaccggcac caccatcgtg gtttttggcc tctttggatg ctttgctaca tgccgtggta 240
gtccatggat gctgaaactg tatgccatgt tcctgtccct ggtgttcctg gctgagcttg 300
ttgctggcat ttctggattt gtgtttcgtc atgagatcaa ggacaccttc ctgaggactt 360
acacggatgc catgcagaac tacaatggca atgatgagag gagccgggct gtggaccatg 420
tgcagcgcag cctgagctgc tgtggcgtgc agaactacac caactggagc agcagcccct 480
acttcctgga ccatggcatc ccccccagtt gctgcatgaa cgaaactgac tgcaatcccc 540
tggatctgca caatctgact gtggccgcca ccaaagttaa tcagaagggc tgttatgatc 600
tggtgaccag ttttatggag actaacatgg ggatcattgc tggagtggca tttggaattg 660
cgttctccca gttgattggc atgctgctgg cttgctgtct gtcccggttc atcactgcca 720
atcagtatga gatggtgtaa agagaagtct ttcaagaact ttggaataag aggcctgttt 780
ttaaaaagga actgccgcaa tctctgaaag acttccaaag aacgttagag cacagtacat 840
actatccctg ccctgctccc acccccagct ccctgcagcc cctcccccta cgtgcaactg 900
acactcctac ccagtcaaag ctctgccttt cagcccacgt catacgtagt gtccatcttg 960
tgaagccctg ttgtgcccca cagtgtagcc aggccccctg cagctagtca tagtgaagcc 1020
caccctaagg ccctgtgagc gccagctccc tcacatgtac ttggtccaac tgtaggtgac 1080
tggttatcac atcttcactg gcccctgtgg ccctgcatgt agtaggggag gcttcagtca 1140
gcccctcact gtggtccaag aatgccacac acctttccgt agatatgccg atggtaatta 1200

tcttttcttt tggcttaatc cgatttggtt tggtttttcc ctttccctgg attctttcct 1260
accttcttca ggctgtcaag cacgggagag gagacacagt gggagggatt cataaggaga 1320
aaacagcgtc tggcaggcat ggcagaaagt gctgttaagc attagctggc ttccttgttc 1380
attgttccaa gagatgggtg tggagagacg gcttagtgta ggatcctctc gaatgaagcc 1440
tgtgtaatcc tgttacaggt tgtgttgtta cttccctgat ttgccccaac tacttactat 1500
tatttcgctt tcgttttcct gtattttttt cttgcattga cattattgac gttgaggacc 1560
tcatcagatc aatttaaaag tgagtgtgga agggaaacga aagtcaacat atttttaaaa 1620
gatcttaaaa ataatgcctc tgtctagcat gccaacaaga atgcattgat attgtgaaca 1680
tttgtgatac atgtattaat aaatagagca attaaaaatg 1720

<210> 107
<211> 320
<212> PRT
<213> Mus musculus

<220>
<223> Translation initiation factor eIF3-p44

<400> 107
Met Pro Thr Gly Asp Phe Asp Ser Lys Pro Ser Trp Ala Asp Gln Val
1               5                   10                  15

Ala Ala Glu Gly Gln Asp Asp Thr Cys Val Thr Ser Glu Leu Leu Thr
            20                  25                  30

Gly Ile Pro Leu Pro Thr Gly Asp Thr Ser Pro Glu Pro Glu Leu Leu
        35                  40                  45

Pro Gly Asp Pro Leu Pro Pro Pro Lys Glu Val Pro Asn Gly Asn Ile
    50                  55                  60

Lys Thr Val Thr Glu Tyr Lys Ile Glu Glu Asp Gly Lys Lys Phe Lys
65                  70                  75                  80

Ile Val Arg Thr Phe Arg Ile Ser Thr Arg Lys Ala Ser Lys Ala Val
                85                  90                  95

Ala Arg Arg Lys Asn Trp Lys Lys Phe Gly Asn Ser Glu Phe Asp Pro
            100                 105                 110

Pro Gly Pro Asn Val Ala Thr Thr Thr Val Ser Asp Asp Val Ser Met
        115                 120                 125

Thr Phe Ile Thr Ser Lys Glu Asp Leu Asn Cys Gln Glu Glu Glu Asp
    130                 135                 140

Pro Met Asn Lys Leu Lys Gly Gln Lys Ile Val Ser Cys Arg Ile Cys
145                 150                 155                 160

Lys Gly Asp His Trp Thr Thr Arg Cys Pro Tyr Lys Asp Thr Leu Gly
                165                 170                 175

Pro Met Gln Lys Glu Leu Ala Glu Gln Leu Gly Leu Ser Thr Gly Glu
            180                 185                 190

Lys Glu Lys Leu Pro Gly Glu Leu Glu Pro Val Gln Ala Ala Gln Ser
        195                 200                 205

Lys Thr Gly Lys Tyr Val Pro Pro Ser Leu Arg Asp Gly Pro Ala Arg
    210                 215                 220

Arg Gly Glu Ser Met Gln Pro Asn Arg Arg Ala Asp Asp Asn Ala Thr
225                 230                 235                 240

Ile Arg Val Thr Asn Leu Ser Glu Asp Thr Arg Glu Thr Asp Leu Gln
                245                 250                 255

Glu Leu Phe Arg Pro Phe Gly Ser Ile Ser Arg Ile Tyr Leu Ala Lys
            260                 265                 270

Asp Lys Thr Thr Gly Gln Ser Lys Gly Phe Ala Phe Ile Ser Phe His
        275                 280                 285

Arg Arg Glu Asp Ala Ala Arg Ala Ile Ala Gly Val Ser Gly Phe Gly
    290                 295                 300

Tyr Asp His Leu Ile Leu Asn Val Glu Trp Ala Lys Pro Ser Thr Asn
305                 310                 315                 320

<210> 108
<211> 1025
<212> DNA
<213> Mus musculus

<220>
<223> Translation initiation factor eIF3-p44

<400> 108
ggagttaaga cttcgagatg ccgactggcg actttgactc gaagcccagc tgggccgacc 60
aggtggcggc cgagggacag gacgacacat gtgtcaccag cgagctcctg acaggaatcc 120
ctctgcccac cggggacacc agtccagagc ccgagttact gcccggagac ccgctgccac 180
ctcccaagga agtacccaat gggaacataa agacagtgac tgagtacaag atagaggaag 240
atgggaagaa attcaagatt gtcagaacct tcaggatatc gacccggaag gcatcaaagg 300
ctgtggcaag gaggaagaat tggaagaagt ttggcaactc ggagtttgac ccaccggggc 360
ccaatgtggc cactaccaca gtcagtgatg acgtctctat gacattcatc actagcaaag 420
aggacctaaa ctgtcaggag gaggaggacc caatgaacaa gcttaagggc cagaagattg 480
tgtcctgccg catttgcaag ggcgaccatt ggaccacccg ttgcccctac aaggacacac 540
taggacccat gcagaaggag ctggctgagc agctcggcct gtctactggg gagaaggaga 600
aactgccagg agagctggag cccgtgcagg ccgcccagag caagacaggg aagtatgtgc 660
cccccagcct ccgggacggg ccagcccgcc gtggggagtc catgcagcca aaccgcagag 720
ctgacgacaa tgccaccatc cgtgtcacta acctgtccga ggacactcgt gagactgact 780
tgcaggaact cttccggcct ttcggctcca tctcccgaat ctacttggcc aaggacaaga 840
ctactgggca gtccaagggt tttgctttta tcagctttca ccgccgggag gatgctgcac 900
gtgccattgc aggggtgtct ggttttggct acgaccatct tatcctcaat gtcgagtggg 960
ccaagccatc aacgaattaa taagacatct gctacctgct gctgttgtcc agcccctcag 1020
gacag                                                             1025

<210> 109
<211> 391
<212> PRT
<213> Mus musculus

<220>
<223> Tumor susceptibility protein TSG101

<400> 109

Met Ala Val Ser Glu Ser Gln Leu Lys Lys Met Met Ser Lys Tyr Lys
1               5                   10                  15

Tyr Arg Asp Leu Thr Val Arg Gln Thr Val Asn Val Ile Ala Met Tyr
            20                  25                  30

Lys Asp Leu Lys Pro Val Leu Asp Ser Tyr Val Phe Asn Asp Gly Ser
        35                  40                  45

Ser Arg Glu Leu Val Asn Leu Thr Gly Thr Ile Pro Val Arg Tyr Arg
    50                  55                  60

Gly Asn Ile Tyr Asn Ile Pro Ile Cys Leu Trp Leu Leu Asp Thr Tyr
65                  70                  75                  80

Pro Tyr Asn Pro Pro Ile Cys Phe Val Lys Pro Thr Ser Ser Met Thr
                85                  90                  95

Ile Lys Thr Gly Lys His Val Asp Ala Asn Gly Lys Ile Tyr Leu Pro
            100                 105                 110

Tyr Leu His Asp Trp Lys His Pro Arg Ser Glu Leu Leu Glu Leu Ile
        115                 120                 125

Gln Ile Met Ile Val Ile Phe Gly Glu Glu Pro Pro Val Phe Ser Arg
    130                 135                 140

Pro Thr Val Ser Ala Ser Tyr Pro Pro Tyr Thr Ala Thr Gly Pro Pro
145                 150                 155                 160

Asn Thr Ser Tyr Met Pro Gly Met Pro Ser Gly Ile Ser Ala Tyr Pro
                165                 170                 175

Ser Gly Tyr Pro Pro Asn Pro Ser Gly Tyr Pro Gly Cys Pro Tyr Pro
            180                 185                 190

Pro Ala Gly Pro Tyr Pro Ala Thr Thr Ser Ser Gln Tyr Pro Ser Gln
        195                 200                 205

Pro Pro Val Thr Thr Val Gly Pro Ser Arg Asp Gly Thr Ile Ser Glu
    210                 215                 220

Asp Thr Ile Arg Ala Ser Leu Ile Ser Ala Val Ser Asp Lys Leu Arg
225                 230                 235                 240

Trp Arg Met Lys Glu Glu Met Asp Gly Ala Gln Ala Glu Leu Asn Ala
                245                 250                 255

Leu Lys Arg Thr Glu Glu Asp Leu Lys Lys Gly His Gln Lys Leu Glu
            260                 265                 270

Glu Met Val Thr Arg Leu Asp Gln Glu Val Ala Glu Val Asp Lys Asn
        275                 280                 285

Ile Glu Leu Leu Lys Lys Lys Asp Glu Glu Leu Ser Ser Ala Leu Glu
    290                 295                 300

Lys Met Glu Asn Gln Ser Glu Asn Asn Asp Ile Asp Glu Val Ile Ile
305                 310                 315                 320

Pro Thr Ala Pro Leu Tyr Lys Gln Ile Leu Asn Leu Tyr Ala Glu Glu

325                 330                 335

Asn Ala Ile Glu Asp Thr Ile Phe Tyr Leu Gly Glu Ala Leu Arg Arg
            340                 345                 350

Gly Val Ile Asp Leu Asp Val Phe Leu Lys His Val Arg Leu Leu Ser
        355                 360                 365

Arg Lys Gln Phe Gln Leu Arg Ala Leu Met Gln Lys Ala Arg Lys Thr
    370                 375                 380

Ala Gly Leu Ser Asp Leu Tyr
385                 390


<210> 110
<211> 1450
<212> DNA
<213> Mus musculus

<220>
<223> Tumor susceptibility protein TSG101

<400> 110
cccctctgcc tgtggggacg gaggagcgcg ccatggcggt gtccgagagt cagctgaaga 60
agatgatgtc caagtacaaa tatagagatc taaccgtccg tcaaactgtc aatgtcatcg 120
ctatgtacaa agatctcaaa cctgtattgg attcatatgt ttttaatgat ggcagttcca 180
gggagctggt gaacctcact ggtacaatcc cagtgcgtta tcgaggtaat atatataata 240
ttccaatatg cctgtggctg ctggacacat acccatataa cccccctatc tgttttgtta 300
agcctactag ttcaatgact attaaaacag gaaagcatgt ggatgcaaat gggaaaatct 360
acctacctta tctacatgac tggaaacatc cacggtcaga gttgctggag cttattcaaa 420
tcatgattgt gatatttgga gaggagcctc cagtgttctc ccggcctact gtttctgcat 480
cctacccacc atacacagca acagggccac caaatacctc ctacatgcca ggcatgccaa 540
gtggaatctc tgcatatcca tctggatacc ctcccaaccc cagtggttat cctggctgtc 600
cttacccacc tgctggccca taccctgcca caacaagctc acagtaccct tcccagcctc 660
ctgtgaccac tgttggtccc agcagagatg gcacaatcag tgaggacact atccgtgcat 720
ctctcatctc agcagtcagt gacaaactga gatggcggat gaaggaggaa atggatggtg 780
cccaggcaga gcttaatgcc ttgaaacgaa cagaggaaga tctgaaaaaa ggccaccaga 840
aactggaaga gatggtcacc cgcttagatc aagaagtagc tgaagttgat aaaaacatag 900
aacttttgaa aaagaaggat gaagaactaa gttctgctct ggagaaaatg gaaaatcaat 960
ctgaaaataa tgatattgat gaagttatca ttcccacagc cccactgtat aaacagattc 1020
taaatctgta tgcagaggaa aatgctattg aagacactat cttttacctt ggagaagctt 1080
tgcggcgggg agtcatagac ctggatgtgt tcctgaaaca cgtccgcctc ctgtcccgta 1140
aacagttcca gctaagggca ctaatgcaaa aggcaaggaa gactgcgggc cttagtgacc 1200
tctactgaca tgtgctgtca gctggagacc gacctctccg taaagcattc ttttcttctt 1260
ctttttctca tcagtagaac ccacaataag ttattgcagt ttatcattca agtgttaaat 1320
attttgaatc aataatatat tttctgtttc ctttgggtaa aaactggctt ttattaatgc 1380
actttctacc ctctgtaagc gtctgtgctg tgctgggact gactgggcta aataaaattt 1440
gttgcataaa                                                        1450


<210> 111
<211> 44
<212> PRT
<213> Rattus norvegicus

<220>
<223> Thymosin beta-4

<400> 111
Met Ser Asp Lys Pro Asp Met Ala Glu Ile Glu Lys Phe Asp Lys Ser

1               5                   10                  15

Lys Leu Lys Lys Thr Glu Thr Gln Glu Lys Asn Pro Leu Pro Ser Lys
            20                  25                  30

Glu Thr Ile Glu Gln Glu Lys Gln Ala Gly Glu Ser
        35                  40


<210> 112
<211> 686
<212> DNA
<213> Rattus norvegicus

<220>
<223> Thymosin beta-4

<400> 112
gcggcctctg agcagatcgt ctctccttgt tcgcccagct cgctcagctc cttccagcaa 60
ccatgtctga caaacccgat atggctgaga tcgagaaatt cgataagtcg aagttgaaga 120
agacagaaac acaagagaaa aatcctctgc cttcaaaaga aacaattgaa caagagaagc 180
aagctggcga atcgtaatga ggcgagcgcc gccaatatgc actgtacatt ccacgagcat 240
tgccttctta ttttacttct tttagctgtt taacttcgta agatgcaaag aggttggatc 300
aagtttaaat gactgtgctg cccctttcac atcaaagaat cagaactact gaccaggaag 360
gcctcccccc gcctctcccg cccacctgct ggcctggctg gcagagaggg aaaagaactt 420
gcatgttggt gaaggaaaaa gctgggtggg agatggtgga attgagagta aaataaagat 480
gtcttgcagg atataaaatg cagtttaatc agagtgccat ttttttttgt tcaaacgatt 540
ttaattattg gaatgcacaa tttttttaat atgcaaataa agttttaaaa cctgaaaaaa 600
aaaaaaaaaa agaaacaaca gtcgttatgg ccataaaaca catcatctgt gattttggag 660
agaaccactg gaaagaaagt ttgagg                                       686


<210> 113
<211> 188
<212> PRT
<213> Rattus norvegicus

<220>
<223> Ribosomal protein L18

<400> 113
Met Gly Val Asp Ile Arg His Asn Lys Asp Arg Lys Val Arg Arg Lys
1               5                   10                  15

Glu Pro Lys Ser Gln Asp Ile Tyr Leu Arg Leu Leu Val Lys Leu Tyr
            20                  25                  30

Arg Phe Leu Ala Arg Arg Thr Asn Ser Thr Phe Asn Gln Val Val Leu
        35                  40                  45

Lys Arg Leu Phe Met Ser Arg Thr Asn Arg Pro Pro Leu Ser Leu Ser
    50                  55                  60

Arg Met Ile Arg Lys Met Lys Leu Pro Gly Arg Glu Asn Lys Thr Ala
65                  70                  75                  80

Val Val Val Gly Thr Ile Thr Asp Asp Val Arg Ile Leu Glu Val Pro
                85                  90                  95

Lys Leu Lys Val Cys Ala Leu Arg Val Ser Ser Arg Ala Arg Ser Arg
            100                 105                 110

Ile Leu Lys Ala Gly Gly Lys Ile Leu Thr Phe Asp Gln Leu Ala Leu
        115                 120                 125

Glu Ser Pro Lys Gly Arg Gly Thr Val Leu Leu Ser Gly Pro Arg Lys
    130                 135                 140

Gly Arg Glu Val Tyr Arg His Phe Gly Lys Ala Pro Gly Thr Pro His
145                 150                 155                 160

Ser His Thr Lys Pro Tyr Val Arg Ser Lys Gly Arg Lys Phe Glu Arg
                165                 170                 175

Ala Arg Gly Arg Arg Ala Ser Arg Gly Tyr Lys Asn
            180                 185


<210> 114
<211> 607
<212> DNA
<213> Rattus norvegicus

<220>
<223> Ribosomal protein L18

<400> 114
catgggtgtt gacattcgcc acaacaagga ccgaaaggtt cggcgcaagg agcccaagag 60
ccaggatatc tacctgcggc tgctagtcaa gctgtacagg tttctggcca gacggaccaa 120
ctccaccttc aaccaggttg tgctgaaaag gttatttatg agccgaacta accggccacc 180
tctgtccctg tcccgaatga tccggaagat gaagcttcct ggtcgggaga acaaaactgc 240
tgtggttgtg gggacgatca cagatgatgt gcggattctg gaagtgccca agctgaaggt 300
gtgtgcactg agggtgagca gccgggcccg aagtcggatc ctcaaggctg ggggtaagat 360
cctgaccttc gaccagctgg ccctggagtc tcccaagggc aggggcactg tgctcttgtc 420
tggtcctcgg aagggccgag aggtgtaccg acactttggc aaggccccag gaactccaca 480
cagccacacc aaaccctatg tccgttccaa gggccggaag ttcgagcgtg ccagaggccg 540
aagggccagc cgaggctaca aaaactaacc ctggctctta ctgttaataa aaaagctttg 600
gatgttg                                                           607


<210> 115
<211> 350
<212> PRT
<213> Mus musculus

<220>
<223> AOS1

<400> 115
Met Val Glu Lys Glu Glu Ala Gly Gly Gly Gly Gly Gly Gly Ile Ser
  1               5                  10                  15

Glu Glu Glu Ala Ala Gln Tyr Asp Arg Gln Ile Arg Leu Trp Gly Leu
             20                  25                  30

Glu Ala Gln Lys Arg Leu Arg Ala Ser Arg Val Leu Ile Val Gly Met
         35                  40                  45

Lys Gly Leu Gly Ala Glu Ile Ala Lys Asn Leu Ile Leu Ala Gly Val
     50                  55                  60

Lys Gly Leu Thr Met Leu Asp His Glu Gln Val Ser Pro Glu Asp Pro

65                  70                  75                  80

Gly Ala Gln Phe Leu Ile Gln Thr Gly Ser Val Gly Arg Asn Arg Ala
                85                  90                  95

Glu Ala Ser Leu Glu Arg Ala Gln Asn Leu Asn Pro Met Val Asp Val
            100                 105                 110

Lys Val Asp Thr Glu Asp Val Glu Lys Lys Pro Glu Ser Phe Phe Thr
        115                 120                 125

Lys Phe Asp Ala Val Cys Leu Thr Cys Cys Ser Arg Asp Val Ile Ile
    130                 135                 140

Lys Val Asp Gln Ile Cys His Arg Asn Ser Ile Lys Phe Phe Thr Gly
145                 150                 155                 160

Asp Val Phe Gly Tyr His Gly Tyr Thr Phe Ala Asn Leu Gly Glu His
                165                 170                 175

Glu Phe Val Glu Glu Lys Thr Lys Val Ala Lys Val Ser Gln Gly Val
            180                 185                 190

Glu Asp Gly Pro Glu Ala Lys Arg Ala Lys Leu Asp Ser Ser Glu Thr
        195                 200                 205

Thr Met Val Lys Lys Lys Val Leu Phe Cys Pro Val Lys Glu Ala Leu
    210                 215                 220

Glu Val Asp Trp Ser Gly Glu Lys Ala Lys Ala Ala Leu Lys Arg Thr
225                 230                 235                 240

Ala Pro Asp Tyr Phe Leu Leu Gln Val Leu Leu Lys Phe Arg Thr Asp
                245                 250                 255

Lys Gly Arg Asp Pro Thr Ser Glu Ser Tyr Lys Glu Asp Ala Glu Leu
            260                 265                 270

Leu Leu Gln Ile Arg Asn Asp Val Phe Asp Ser Leu Gly Ile Ser Pro
        275                 280                 285

Asp Leu Leu Pro Asp Asp Phe Val Arg Tyr Cys Phe Ser Glu Met Ala
    290                 295                 300

Pro Val Cys Ala Val Val Gly Gly Ile Leu Ala Gln Glu Ile Val Lys
305                 310                 315                 320

Ala Leu Ser Gln Arg Asp Pro Pro His Asn Asn Phe Phe Phe Phe Asp
                325                 330                 335

Gly Met Lys Gly Ser Gly Ile Val Glu Cys Leu Gly Pro Gln
            340                 345                 350

<210> 116
<211> 1870
<212> DNA
<213> Mus musculus

<220>
<223> AOS1

<400> 116
cgagagcggg agcgggtgag ctgaaaccgg cgccatggta gagaaggagg aggctggcgg 60
cggcggcggc ggtggcatca gcgaggagga ggcagcgcag tatgaccgac agatccgcct 120
gtggggactg gaggcccaga agcggcttcg agcttcccgg gtgctgattg ttggcatgaa 180
aggacttgga gctgaaattg ccaagaatct tatcctggca ggagtcaaag ggctcaccat 240
gctggaccac gaacaggtat ctccagaaga ccctggagct cagttcttga ttcagactgg 300
gtctgtgggc cgcaataggg ccgaggcctc tttggaacga gcccagaatc tcaatcccat 360
ggtggatgta aaggtggaca ctgaggatgt agagaagaag ccagagtcct tcttcacgaa 420
gtttgatgcg gtgtgcctga cttgctgctc cagagatgtc atcattaaag tcgaccagat 480
ctgtcacaga aacagcatta agttcttcac aggagatgtc tttgggtacc atgggtacac 540
ctttgcgaat cttggagagc atgaatttgt agaagagaaa accaaagttg ccaaagtaag 600
ccaaggagtg gaagatggac ctgaggccaa gagagcaaag cttgattcat cagagacaac 660
catggtcaaa aagaaagtgc ttttttgccc tgtaaaagag gcgctagaag tggactggag 720
tggagagaaa gccaaggctg ccctgaagcg tactgccccc gactactttc tgctgcaagt 780
gctgctgaaa ttccgcacag ataaagggag agatccgact tctgagagct acaaggagga 840
tgcggagctg ctgctgcaga tacggaatga cgtgtttgac tccctgggca tcagtcctga 900
cctgcttccc gatgactttg tcaggtactg cttctctgag atggccccag tatgtgctgt 960
ggttggaggg atcttggcac aggaaattgt gaaggccctg tctcaaaggg accctcctca 1020
caacaacttc ttcttcttcg acggcatgaa ggggagtgga attgtggagt gccttggtcc 1080
ccagtgagct gaggtgtgta caaggccacc gagtcacctg ccctctctcc ggatcatttt 1140
tctagagcaa gcaggccaga cttcatcctg cctttgtctt cgaagctatc agggaactgc 1200
ggggtcccaa atttgataat cacgtggcac ctctggcatt gctcccttgg gctgtcactg 1260
gggactgtca gttcctagtg agagagagca tcatacttca tgatgtcaga gtgtcttgtc 1320
accgtttgga cttaacttcc ctctatagtc agcagagggg atctgaggcc cagtgctgcc 1380
tggagaactg agaatagacc tctgttgttg acccttcctc aggctggagc ctgggtgttg 1440
gaagggagag actgtcaccc agttgtcatg tcttttggtt gtgagcctat gcaccttacc 1500
tttaatggtt gaaccatctt ttcagcccaa acagtttatt tctaaaaagt agatttaatg 1560
ctaggggcca ttatgcaggc ttgtaatcct agatgttggg atgccggggc aggatttact 1620
gtaaatttca gggccaagtt ggtctacata atgtaccaag aacctttctc aaaatataat 1680
aaagggcata ttccagagtt agggtggggc actgttaccc agctccttat actgggcacc 1740
cgggcccctg acaggagtgg gttggggggc agttggtttg gtcttccttc taggagtatg 1800
agggcagcct gtctagtttg tagatcccct tggttgctgg ataaaggatg ataataaagt 1860
gtgtttctct 1870

<210> 117
<211> 1762
<212> PRT
<213> Rattus norvegicus

<220>
<223> Ankyrin isoform

<400> 117
Met Ala Ser Ser Gly Thr Ser Ser Pro Val Gly Ala Gly Ser Pro Asp
1               5                   10                  15

Leu Glu Gln Gly Asp Phe Gly Ser Cys Tyr Ser Arg Ser Ser Gln Lys
            20                  25                  30

Ser Asp Ala Asn Ala Ser Tyr Leu Arg Ala Ala Arg Ala Gly His Leu
        35                  40                  45

Glu Lys Ala Leu Asp Tyr Ile Lys Asn Gly Val Asp Val Asn Ile Cys
    50                  55                  60

Asn Gln Asn Gly Leu Asn Ala Leu His Leu Ala Ser Lys Glu Gly His
65                  70                  75                  80

Val Glu Val Val Ser Glu Leu Leu Gln Arg Glu Ala Asn Val Asp Gln

                85                  90                  95

Pro Thr Lys Lys Gly Asn Thr Ala Leu His Ile Ala Ser Leu Ala Gly
            100                 105                 110

Gln Ala Glu Val Val Lys Val Leu Val Thr Asn Gly Ala Asn Val Asn
        115                 120                 125

Ala Gln Ser Gln Asn Gly Phe Thr Pro Leu Tyr Met Ala Ala Gln Glu
    130                 135                 140

Asn His Leu Glu Val Val Arg Phe Leu Leu Asp Asn Gly Ala Ser Gln
145                 150                 155                 160

Ser Leu Ala Thr Glu Asp Gly Phe Thr Pro Leu Ala Val Ala Leu Gln
                165                 170                 175

Gln Gly His Asp Gln Val Val Ser Leu Leu Leu Glu Asn Asp Thr Lys
            180                 185                 190

Gly Lys Val Arg Leu Pro Ala Leu His Ile Ala Ala Arg Lys Asp Asp
        195                 200                 205

Thr Lys Ala Ala Ala Leu Leu Leu Gln Asn Asp Thr Asn Ala Asp Ile
    210                 215                 220

Glu Ser Lys Ser Gly Phe Thr Pro Leu His Ile Ala Ala His Tyr Gly
225                 230                 235                 240

Asn Ile Asn Val Ala Thr Leu Leu Leu Asn Arg Ala Ala Ala Val Asp
                245                 250                 255

Phe Thr Ala Arg Asn Asp Ile Thr Pro Leu His Val Ala Ser Lys Arg
            260                 265                 270

Gly Asn Ala Asn Met Val Lys Leu Leu Leu Asp Arg Gly Ala Lys Ile
        275                 280                 285

Asp Ala Lys Thr Arg Asp Gly Leu Thr Pro Leu His Cys Gly Ala Arg
    290                 295                 300

Ser Gly His Glu Gln Val Val Glu Met Leu Leu Asp Arg Ala Ala Pro
305                 310                 315                 320

Ile Leu Ser Lys Thr Lys Asn Gly Leu Ser Pro Leu His Met Ala Thr
                325                 330                 335

Gln Gly Asp His Leu Asn Cys Val Gln Leu Leu Leu Gln His Asn Val
            340                 345                 350

Pro Val Asp Asp Val Thr Asn Asp Tyr Leu Thr Ala Leu His Val Ala
        355                 360                 365

Ala His Cys Gly His Tyr Lys Val Ala Lys Val Leu Leu Asp Lys Lys
    370                 375                 380

Ala Asn Pro Asn Ala Lys Ala Leu Asn Gly Phe Thr Pro Leu His Ile
385                 390                 395                 400

Ala Cys Lys Lys Asn Arg Ile Arg Val Met Glu Leu Leu Leu Lys His
                405                 410                 415

Gly Ala Ser Ile Gln Ala Val Thr Glu Ser Gly Leu Thr Pro Ile His
            420                 425                 430

Val Ala Ala Phe Met Gly His Val Asn Ile Val Ser Gln Leu Met His
        435                 440                 445

His Gly Ala Ser Pro Asn Thr Thr Asn Val Arg Gly Glu Thr Ala Leu
    450                 455                 460

His Met Ala Ala Arg Ser Gly Gln Ala Glu Val Val Arg Tyr Leu Val
465                 470                 475                 480

Gln Asp Gly Ala Gln Val Glu Ala Lys Ala Lys Asp Glu Gln His Pro
                485                 490                 495

Thr Pro His Phe Ser Arg Leu Gly Lys Ala Glu Ile Val Gln Gln Val
            500                 505                 510

Leu Gln Gln Gly Ala Ser Pro Asn Ala Ala Thr Thr Ser Gly Tyr Thr
        515                 520                 525

Pro Leu His Leu Ser Ala Arg Glu Gly His Glu Asp Val Ala Ala Phe
    530                 535                 540

Leu Leu Asp His Gly Ala Phe Leu Ser Ile Thr Thr Lys Lys Gly Phe
545                 550                 555                 560

Thr Pro Leu His Val Ala Ala Lys Tyr Gly Lys Leu Glu Val Ala Ser
                565                 570                 575

Leu Leu Leu Gln Lys Ser Ala Ser Pro Asp Ala Ala Gly Lys Ser Gly
            580                 585                 590

Leu Thr Pro Leu His Val Ala Ala His Tyr Asp Asn Gln Lys Val Ala
        595                 600                 605

Leu Leu Leu Leu Asp Gln Gly Ala Ser Pro His Ala Ala Ala Lys Asn
    610                 615                 620

Gly Tyr Thr Pro Leu His Ile Ala Ala Lys Lys Asn Gln Met Asp Ile
625                 630                 635                 640

Ala Thr Ser Leu Leu Glu Tyr Gly Ala Asp Ala Asn Ala Val Thr Arg
                645                 650                 655

Gln Gly Ile Ala Ser Val His Leu Ala Ala Gln Glu Gly His Val Asp
            660                 665                 670

Met Val Ser Leu Leu Leu Ser Arg Asn Ala Asn Val Asn Leu Ser Asn
        675                 680                 685

Lys Arg Gly Leu Asn Pro Leu His Leu Gly Gly Gln Glu Asp Arg Val
    690                 695                 700

Asn Val Ala Glu Val Leu Val Asn Gln Gly Ala His Val Asp Ala Gln
705                 710                 715                 720

Thr Lys Met Gly Tyr Thr Pro Leu His Val Gly Cys His Tyr Gly Asn
                725                 730                 735

Ile Lys Ile Val Asn Phe Leu Leu Gln His Ser Ala Lys Val Asn Ala
            740                 745                 750

Lys Thr Lys Asn Gly Tyr Thr Pro Leu His Gln Ala Ala Gln Gln Gly
        755                 760                 765

His Thr His Ile Ile Asn Val Leu Leu Gln Asn Asn Ala Ser Pro Asn
    770                 775                 780

Glu Leu Thr Val Asn Gly Asn Thr Ala Leu Ala Ile Ala Arg Arg Leu
785                 790                 795                 800

Gly Tyr Ile Ser Val Val Asp Thr Leu Lys Val Val Thr Glu Glu Ile
                805                 810                 815

Met Thr Thr Thr Thr Ile Thr Glu Lys His Lys Met Asn Val Pro Glu
            820                 825                 830

Thr Met Asn Glu Val Leu Asp Met Ser Asp Asp Glu Val Gly Lys Gly
        835                 840                 845

Ser Ala Pro Glu Lys Leu Ser His Gly Glu Tyr Ile Ser Asp Gly Glu
    850                 855                 860

Glu Gly Glu Asp Ala Ile Thr Gly Asp Thr Asp Lys Ser Leu Gly Pro
865                 870                 875                 880

Gln Asp Leu Lys Glu Leu Gly Asp Asp Ser Leu Pro Ala Glu Gly Tyr
                885                 890                 895

Val Gly Phe Ser Leu Gly Ala Arg Ser Gly Ser Leu Arg Ser Phe Ser
            900                 905                 910

Ser Asp Arg Ser Tyr Thr Leu Asn Arg Ser Ser Tyr Ala Arg Asp Ser
        915                 920                 925

Met Met Ile Glu Glu Leu Leu Val Pro Ser Lys Glu Gln His Leu Pro
    930                 935                 940

Phe Thr Arg Glu Phe Asp Ser Asp Ser Leu Arg His Tyr Ser Trp Pro
945                 950                 955                 960

Ala Asp Thr Leu Asp Asn Val Asn Leu Val Ser Ser Pro Val His Ser
                965                 970                 975

Gly Phe Leu Val Ser Phe Met Val Asp Ala Arg Gly Gly Ser Met Arg
            980                 985                 990

Gly Ser Arg His His Gly Met Arg Ile Ile Ile Pro Pro Arg Lys Cys
        995                 1000                1005

Thr Ala Pro Thr Arg Ile Thr Cys Arg Leu Val Lys Arg His Lys Leu
    1010                1015                1020

Ala Asn Pro Pro Pro His Gly Gly Arg Arg Gly Ile Ser Ser Arg Leu
1025                1030                1035                1040

Val Glu Met Gly Pro Ala Gly Ala Gln Phe Leu Gly Pro Val Ile Val
                1045                1050                1055

Glu Ile Pro His Phe Gly Ser Met Arg Gly Lys Glu Arg Glu Leu Ile

1060                1065                1070

Val Leu Arg Ser Glu Asn Gly Glu Thr Trp Lys Glu His Gln Phe Asp
        1075                1080                1085

Ser Lys Asn Glu Asp Leu Ser Glu Leu Leu Asn Gly Met Asp Glu Glu
    1090                1095                1100

Leu Asp Ser Pro Glu Glu Leu Gly Thr Lys Arg Ile Cys Arg Ile Ile
1105                1110                1115                1120

Thr Lys Asp Phe Pro Gln Tyr Phe Ala Val Val Ser Arg Ile Lys Gln
                1125                1130                1135

Glu Ser Asn Gln Ile Gly Pro Glu Gly Gly Ile Leu Ser Ser Thr Thr
            1140                1145                1150

Val Pro Leu Val Gln Ala Ser Phe Pro Glu Gly Ala Leu Thr Lys Arg
        1155                1160                1165

Ile Arg Val Gly Leu Gln Ala Gln Pro Val Pro Glu Glu Thr Val Lys
    1170                1175                1180

Lys Ile Leu Gly Asn Lys Ala Thr Phe Ser Pro Ile Val Thr Val Glu
1185                1190                1195                1200

Pro Arg Arg Arg Lys His Lys Pro Ile Thr Met Thr Ile Pro Ile Thr
                1205                1210                1215

Met Thr Ile Pro Val Pro Pro Pro Ser Gly Glu Gly Val Ser Asn Gly
            1220                1225                1230

Tyr Lys Gly Gly Asn Thr Pro Ser Leu Arg Phe Leu Cys Arg Ile Thr
        1235                1240                1245

Gly Gly Thr Ser Pro Ala Gln Trp Glu Asp Ile Thr Gly Thr Thr Pro
    1250                1255                1260

Leu Thr Phe Ile Lys Asp Cys Val Ser Phe Thr Thr Asn Val Ser Ala
1265                1270                1275                1280

Arg Phe Trp Leu Ala Asp Cys His Gln Val Leu Glu Thr Val Gly Leu
                1285                1290                1295

Ala Ser Gln Leu Tyr Arg Glu Leu Ile Cys Val Pro Tyr Met Ala Lys
            1300                1305                1310

Phe Val Val Phe Ala Lys Thr Asn Asp Pro Val Glu Ala Ala Leu Arg
        1315                1320                1325

Cys Phe Cys Met Thr Asp Asp Arg Val Asp Lys Thr Leu Glu Gln Gln
    1330                1335                1340

Glu Asn Phe Glu Glu Val Ala Arg Ser Lys Asp Ile Glu Val Leu Glu
1345                1350                1355                1360

Gly Lys Pro Ile Tyr Val Asp Cys Tyr Gly Asn Leu Ala Pro Leu Thr
                1365                1370                1375

Lys Gly Gly Gln Gln Leu Val Phe Asn Phe Tyr Ser Phe Lys Glu Asn
            1380                1385                1390

Arg Leu Pro Phe Ser Ile Lys Val Arg Asp Thr Ser Gln Glu Pro Cys
        1395                1400                1405

Ala Val Cys Leu Phe Leu Lys Glu Pro Lys Thr Thr Lys Gly Leu Pro
    1410                1415                1420

Gln Thr Ala Val Cys Asn Leu Asn Ile Thr Leu Pro Ala His Lys Lys
1425                1430                1435                1440

Ala Glu Lys Ala Asp Arg Arg Gln Ser Phe Thr Ser Leu Ala Leu Arg
                1445                1450                1455

Lys Arg Tyr Ser Tyr Leu Thr Glu Pro Ser Met Ser Pro Gln Ser Pro
            1460                1465                1470

Cys Glu Arg Thr Asp Ile Arg Met Ala Ile Val Ala Asp His Leu Gly
        1475                1480                1485

Leu Ser Trp Thr Glu Leu Ala Arg Glu Leu Asn Phe Ser Val Asp Glu
    1490                1495                1500

Ile Asn Gln Ile Arg Val Glu Asn Pro Asn Ser Leu Ile Ser Gln Ser
1505                1510                1515                1520

Phe Met Leu Leu Lys Lys Trp Val Thr Arg Asp Gly Lys Asn Ala Thr
                1525                1530                1535

Thr Asp Ala Leu Thr Ser Val Leu Thr Lys Ile Asn Arg Ile Asp Ile
            1540                1545                1550

Val Thr Leu Leu Glu Gly Pro Ile Phe Asp Tyr Gly Asn Ile Ser Gly
        1555                1560                1565

Thr Arg Ser Phe Ala Asp Glu Asn Asn Val Phe His Asp Pro Val Asp
    1570                1575                1580

Gly Trp Gln Asn Glu Thr Pro Ser Gly Ser Leu Glu Ser Pro Ala Gln
1585                1590                1595                1600

Ala Arg Arg Ile Thr Gly Gly Leu Leu Asp Arg Leu Asp Asp Arg Ser
                1605                1610                1615

Asp Gln Val Arg Asp Pro Ile Thr Ser Tyr Leu Thr Gly Glu Ala Gly
            1620                1625                1630

Lys Phe Glu Ala Asn Gly Asn His Ala Glu Val Ile Pro Glu Ala Lys
        1635                1640                1645

Ala Lys Ala Tyr Phe Pro Glu Ser Gln Asn Asp Ile Gly Lys Gln Ser
    1650                1655                1660

Ile Lys Glu Asn Leu Lys Pro Lys Thr His Gly Cys Gly Arg Ala Glu
1665                1670                1675                1680

Glu Pro Val Ser Pro Leu Thr Gly Tyr Gln Lys Ser Leu Glu Glu Thr
                1685                1690                1695

Ser Lys Leu Val Ile Glu Asp Ala Pro Lys Pro Cys Val Pro Val Gly
            1700                1705                1710

Met Lys Lys Met Thr Arg Thr Pro Ala Asp Gly Lys Ala Arg Leu Asn
      1715                1720                1725

Leu Gln Glu Glu Glu Gly Ser Ala Arg Ser Glu Pro Lys Gln Gly Glu
   1730                1735                1740

Gly Tyr Lys Val Lys Thr Lys Lys Glu Ile Arg Asn Val Glu Lys Lys
1745                1750                1755                1760

Ala His

<210> 118
<211> 6207
<212> DNA
<213> Rattus norvegicus

<220>
<223> Ankyrin isoform

<400> 118
cacgagcgga tcccgggttg aggagacgcc gacccgggcg cagagccgag ttcctggaag 60
gaggagcgcg cgggacccca ccgtatggcc agctccggca cttcgtcccc agtgggcgcg 120
gggagccccg atcttgagca gggtgacttt ggatcctgtt attcacgaag ctctcaaaag 180
tcggatgcca atgcaagtta cttgagagca gctcgggcgg ggcacctgga aaaggccctt 240
gactacatca aaaatggagt ggacgtcaac atctgtaacc agaatgggtt gaatgcgctc 300
catcttgctt ccaaagaagg gcatgtggaa gtggtctccg agctgctgca gagagaagcc 360
aacgtcgatc agccaacaaa gaaaggaaac acagccttac acatcgcatc cttggctgga 420
caagcggaag tggtcaaggt cttggttacg aacggagcga acgtcaacgc acagtctcag 480
aatggcttca cgccgctgta tatggcggcc caggagaacc acctggaagt cgtcaggttt 540
cttctggaca atggtgccag ccaaagcctg gctacagagg atggcttcac gccattggcc 600
gtggctcttc aacaaggtca cgaccaagtc gtatccctcc tgctcgagaa cgacacgaag 660
ggaaaagtgc gcctcccagc cctccacatc gcggcccgga aagatgacac caaagcggca 720
gctctgctcc tacagaacga caccaatgca gacatagagt caaagagtgg cttcacccca 780
ctccacatag ctgcccacta cgggaatatc aatgtggcca cgttgctgtt aaaccgagcg 840
gctgccgtgg acttcaccgc acggaatgac atcactcctt tgcatgttgc ctcgaaacgg 900
ggaaacgcaa atatggtaaa gctgttgctc gaccggggtg cgaagatcga cgccaagacc 960
agggacggtc tgaccccgct gcactgcggg gcgagaagtg gccacgaaca ggtggtagag 1020
atgttgctgg acagagccgc ccccatcctt tcgaaaacca agaatggact gtccccactg 1080
cacatggcca cacaaggaga ccatttaaac tgcgtccaac tcctcctcca gcacaacgtg 1140
cccgtggatg acgtcaccaa cgactacctg accgccctcc acgtggcggc ccactgcggg 1200
cattacaaag ttgccaaggt tcttttggat aagaaagcta accccaatgc caaagctctg 1260
aatggcttca cccccctcca tatcgcctgc aaaaagaacc gaatccgagt aatggaactc 1320
cttttgaagc acggtgcatc tattcaagcc gtaaccgagt cgggccttac cccaatccat 1380
gtcgctgcct tcatgggaca tgtaaatatc gtgtcacagc taatgcatca tggagcctcc 1440
ccaaacacca ccaacgtgag aggagaaaca gcattgcata tggcggctcg gtcggggcaa 1500
gcagaagtgg tgcggtattt ggtacaagat ggagctcagg tagaagcaaa agctaaggat 1560
gagcaacacc ccactccaca tttcagccga ctcgggaagg ctgagatagt gcaacaagtc 1620
ttacagcaag gagcgtcccc gaatgcagca acaacttccg ggtacacccc ccttcacctt 1680
tcggccagag aggggcatga ggatgtagct gccttccttc tggaccatgg agcattctta 1740
tccataacaa caaagaaggg attcacgcct ctgcatgtgg ctgcaaagta tgggaagctt 1800
gaagtggcaa gtctcctgct gcagaagagt gcgtctcctg atgccgctgg gaagagcggg 1860
ctaactccac tgcatgtagc agcacattac gataatcaga aagtggccct tctgcttttg 1920
gaccagggag cctcacccca cgcagctgca aagaacggtt atacgccact gcacatcgcc 1980
gccaagaaga accagatgga catagccacg tccctgctgg agtacggtgc tgatgcaaac 2040
gccgttaccc ggcaagggat tgcgtccgtc caccttgcag cgcaggaagg gcacgtggac 2100
atggtgtcgc tgctcctcag tagaaatgcg aacgtcaact tgagcaataa gaggggcctc 2160
aacccactcc acctgggggg tcaagaagac cgagtgaacg tggctgaggt ccttgtcaac 2220
caaggggccc acgtggatgc acagacaaag atgggctaca cgccgctcca tgtgggctgt 2280
cactatggaa acatcaaaat agtcaatttt ctgctgcagc attctgcgaa agttaatgcc 2340

aagacaaaga atggatacac gccactgcac caggcagccc agcaggggca cacacacatc 2400
atcaacgtcc tgctccagaa caacgcttcc cccaatgaac tcactgtgaa tgggaacaca 2460
gccctggcca tcgcccgacg ccttggctac atctcggtgg ttgacaccct aaaggtcgtg 2520
acagaggaaa ttatgaccac cactaccatc acggagaagc acaaaatgaa tgtcccagaa 2580
acaatgaatg aagtcctcga catgtcagac gatgaagttg gtaaaggcag cgcccccgaa 2640
aagctcagtc atggggaata tatctcagat ggtgaagaag gtgaagatgc catcacaggg 2700
gacactgaca aatctctcgg cccacaggac ctcaaggagc tgggcgatga ctccctgcct 2760
gccgagggct atgtgggctt cagtctcgga gcccgttctg gcagtcttcg ctccttcagt 2820
tcggataggt cctacaccct gaacagaagc tcctatgcgc gggacagcat gatgatcgag 2880
gagcttctgg tgccatccaa agagcagcac ctcccattca cgagggagtt tgactccgac 2940
tccctcagac actacagttg gccagcagac acactggaca atgtgaacct ggtctccagc 3000
cctgtgcatt ctgggtttct ggttagcttt atggtggacg cgagaggggg ctccatgcga 3060
ggaagccgcc accacgggat gcggatcatc atccctcccc gcaagtgtac ggcccccacc 3120
cgcatcactt gccgcctggt aaagagacat aaactggcca acccaccccc ccatggtgga 3180
aggagaggga ttagcagtag gctggtagaa atgggtcctg cgggggcaca atttttaggc 3240
ccggtcatag tggaaatccc tcattttggg tccatgagag gaaaggagag ggaactcatt 3300
gtcctccgga gcgagaacgg agagacctgg aaggaacacc agtttgacag caaaaacgaa 3360
gacctctcgg agctcctcaa tggcatggac gaagaacttg acagcccaga agagttgggc 3420
acaaagcgca tctgcagaat catcacgaag gatttccccc agtatttcgc tgtggtttcc 3480
cggattaagc aggaaagcaa ccagattggt cccgaaggtg ggattctgag cagcaccacg 3540
gtccccctcg tccaggcgtc tttcccagag ggtgccttaa ccaaaaggat ccgtgtgggt 3600
ctgcaggctc agcccgtacc agaggagact gtgaaaaaga tccttggaaa caaagcaacg 3660
tttagcccaa ttgtcacggt ggagccgagg aggaggaaac ataagccgat caccatgacc 3720
atcccgatca ccatgaccat cccggtgccc ccaccctcgg gagaaggggt gtccaatggg 3780
tacaaagggg gaaacacgcc cagcctgcga ttcctctgca ggattacagg agggacttcc 3840
ccggctcagt gggaagacat cacaggcaca acccctctga ccttcataaa ggactgtgtg 3900
tctttcacaa ccaatgtttc agccaggttc tggctggcgg actgccatca ggtgttagaa 3960
accgtaggct tagcctcgca gctgtacaga gagttgatat gtgtccctta catggccaag 4020
tttgttgttt ttgccaaaac aaacgacccc gtggaagccg ccctaaggtg cttctgtatg 4080
acagacgaca gagtggacaa aaccctggaa cagcaggaga atttcgagga ggttgccaga 4140
agcaaagaca tcgaggttct ggaaggaaag cccatctatg ttgattgcta tggaaacctg 4200
gctcctctga ccaaaggagg gcagcagctt gtttttaact tctattcttt caaagaaaac 4260
agactgccat tttccatcaa ggttagagac accagccaag agccctgcgc cgtctgtctt 4320
ttcctgaagg agccgaaaac aacaaaagga ttaccccaaa cggctgtttg caacttaaat 4380
attactctac cggcgcataa aaaggctgag aaggcagaca gacgccagag cttcacatcc 4440
ctagccttac gtaagcgcta cagctacttg actgagccca gcatgagtcc gcagagtcct 4500
tgtgagcgga cggatatcag gatggcaata gtagccgatc acctgggact tagttggaca 4560
gagctggcaa gggaactgaa tttttcagtg gatgaaatca accaaatacg agtggaaaat 4620
cccaattctt taatttctca gagcttcatg ttattaaaaa agtgggtgac gagagacggg 4680
aagaatgcca caactgatgc cttaacttcg gtcttaacaa aaattaaccg aatagacata 4740
gtaactctgc tggaaggacc aatatttgat tatgggaata tttcaggcac cagaagcttt 4800
gcagatgaaa acaatgtttt ccatgaccca gttgatggtt ggcagaacga gacgccaagt 4860
ggaagcctag agtccccggc gcaagcgcga agaatcaccg gtgggttact ggaccgtctg 4920
gacgacaggt ctgaccaggt tcgggatccc attacctcat acctcacagg agaagctggg 4980
aagttcgaag caaacggaaa ccacgcagaa gtcattccag aagcaaaggc caaggcctac 5040
tttcctgaat cccaaaacga tatagggaaa cagagcatca aggagaactt gaaaccaaaa 5100
acacacggat gtggtcgcgc tgaggaacca gtgtcacccc tcacaggcta ccagaaatct 5160
ctggaagaaa ccagcaagct tgtcatagaa gacgcaccta aaccctgtgt gcctgtcggc 5220
atgaaaaaga tgaccaggac tccggctgac ggcaaagcca ggctcaacct ccaggaagag 5280
gaggggtccg ccaggtcaga gcctaagcag ggagaaggct ataaggtgaa aacaaagaag 5340
gaaatccgga atgtggagaa gaaagcccac tagtgacggt gacgtcagtc caggaccacg 5400
cgttcgtact gccagtattg agaaactcac cagagatcag cagggaggag aatctctagc 5460
aagagcggag tgtggatctg ccaccccaca catcagtgac atgtttgggg ttttgggttt 5520
ttttggtttt tttttttaat gcagaaggat aaagaaaatc accttttttt tatttagcat 5580
gagccaattt acagagcatg gagactcact ttcattcccg gcgctgacac gcgtgcacta 5640
agcaagtgca atgtaagata caagaagcca tgttgttcag actcagcctc aagtaatttg 5700
gtgtcacttt cggagaagga cagacaacca acgacacctg tgccaccgga aggagggggg 5760
ccagcaccaa gtggagcggt cccaatccag gaagacaccc ttgaaattac taaaacccac 5820
cacaagctcg ccttattttt cttggaccca aactaactgt cagggtataa acactgtttg 5880
tttctttttt ttttttaaaa gtttaaataa taataagaga aatcaatagt tttgctgtaa 5940
ataataacac tgtaaattct aacagaagaa actacaataa agaattataa ggcacctgcc 6000

tcacggaaca caaagatacc acaaacgcgt ccaatgacag gcgtaccaag tggcctcacc 6060
ctggacaagg acgggaatct ggcgcgtgcg ggacgctctc tcactgactc ctgtatatta 6120
gcaattatga tgtttgtaca cgccaaactg ctggcttggt tttaaaaaga aaatgcaaat 6180
ctttaaaaaa aaaaaaaaaa aaaaaaa 6207

<210> 119
<211> 393
<212> PRT
<213> Rattus norvegicus

<220>
<223> Zygin 1

<400> 119
Met Glu Ala Pro Leu Val Ser Leu Asp Glu Glu Phe Glu Asp Ile Arg
1               5                   10                  15

Pro Cys Cys Thr Glu Asp Pro Glu Glu Lys Pro Gln Ser Leu Tyr Gly
            20                  25                  30

Thr Ser Pro His His Leu Glu Asp Pro Ser Leu Ser Glu Leu Glu Asn
        35                  40                  45

Phe Ser Ser Glu Ile Ile Ser Phe Lys Ser Met Glu Asp Leu Val Asn
    50                  55                  60

Glu Phe Asp Glu Lys Leu Asn Val Cys Phe Arg Asn Tyr Asn Ala Lys
65                  70                  75                  80

Thr Glu Asn Leu Ala Pro Val Lys Asn Gln Leu Gln Ile Gln Glu Glu
                85                  90                  95

Glu Glu Thr Leu Arg Asp Glu Glu Val Trp Asp Ala Leu Thr Asp Asn
            100                 105                 110

Tyr Ile Pro Ser Leu Ser Glu Asp Trp Arg Asp Pro Asn Ile Glu Ala
        115                 120                 125

Leu Asn Gly Asn Ser Ser Asp Thr Glu Ile His Glu Lys Glu Glu Glu
    130                 135                 140

Asp Glu Phe Ile Glu Lys Ser Glu Asn Asp Ser Gly Ile Asn Glu Glu
145                 150                 155                 160

Pro Leu Leu Thr Ala Asp Gln Val Ile Glu Glu Ile Glu Glu Met Met
                165                 170                 175

Gln Asn Ser Pro Asp Pro Glu Glu Glu Val Glu Val Leu Glu Glu Glu
            180                 185                 190

Asp Gly Gly Glu Ile Ser Ser Gln Ala Asp Ser Val Leu Leu Gln Glu
        195                 200                 205

Met Gln Ala Leu Thr Gln Thr Phe Asn Asn Asn Trp Ser Tyr Glu Gly
    210                 215                 220

Leu Arg His Met Ser Gly Ser Glu Leu Thr Glu Leu Leu Asp Gln Val
225                 230                 235                 240

Glu Gly Ala Ile Arg Asp Phe Ser Glu Glu Leu Val His Gln Leu Ala

                245                 250                 255

Arg Arg Asp Glu Leu Glu Phe Glu Lys Glu Val Lys Asn Ser Phe Ile
            260                 265                 270

Thr Val Leu Ile Glu Val Gln Asn Lys Gln Lys Glu Gln Arg Glu Leu
        275                 280                 285

Met Lys Lys Arg Arg Lys Glu Lys Gly Leu Ser Leu Gln Ser Ser Arg
    290                 295                 300

Ile Glu Lys Gly Asn Gln Met Pro Leu Lys Arg Phe Ser Met Glu Gly
305                 310                 315                 320

Ile Ser Asn Ile Leu Gln Ser Gly Ile Arg Gln Thr Phe Gly Ser Ser
                325                 330                 335

Gly Ala Asp Arg Gln Tyr Leu Asn Thr Val Ile Pro Tyr Glu Lys Lys
            340                 345                 350

Ser Ser Pro Pro Ser Val Glu Asp Leu Gln Met Leu Thr Asn Ile Leu
        355                 360                 365

Phe Ala Met Lys Glu Asp Asn Glu Lys Val Pro Thr Leu Leu Thr Asp
    370                 375                 380

Tyr Ile Leu Lys Val Leu Cys Pro Thr
385                 390

<210> 120
<211> 1697
<212> DNA
<213> Rattus norvegicus

<220>
<223> Zygin 1

<400> 120
tagctggctt gcccgctgcc gttgcactgc accgggccac ctctagccgg tggatgggga 60
tgtctggctg aaccccggga tccgattccc tcctccagga cctgctcagg gaaacggatc 120
ttgacagttg ccatcattgt actacggtgc caggatggag gctccactgg tgagtctgga 180
tgaagagttt gaagatatca ggccctgctg cactgaggac ccagaggaaa agccacaaag 240
tctctatggc acatctcctc accacctgga ggacccttcc ctctccgagc ttgagaattt 300
ttcttccgaa ataatcagct tcaagtccat ggaggacctc gtgaatgaat ttgatgagaa 360
gctcaatgtc tgctttcgga actacaatgc caagaccgag aacctggctc cagtgaagaa 420
ccagttgcag atccaggagg aggaggagac tcttcgggat gaggaggttt gggatgctct 480
gacagacaat tacatccctt cactctcaga agactggagg gacccaaaca ttgaagctct 540
gaatggcaac agctctgaca ctgagatcca cgagaaggaa gaagaagatg agttcattga 600
gaagagtgaa aacgactctg gtatcaacga ggagcctctc ctcacagctg accaggtgat 660
tgaggagatc gaagaaatga tgcagaactc cccagatcct gaggaggaag tggaggtgct 720
ggaagaggag gatggaggag aaatctcgtc tcaggcagat tcagtcctgc tacaagagat 780
gcaggccctg acgcagacct tcaacaacaa ctggtcctat gaagggctga gacacatgtc 840
tgggtctgag ctgacagagc tgctggacca ggtggagggt gccatccgtg acttctcgga 900
ggagctggtc caccagctgg cccgcagaga tgagctggag tttgagaagg aagtgaagaa 960
ctctttcatc acggtgctca ttgaggttca gaataagcag aaggagcagc gggaactgat 1020
gaagaagagg cggaaagaga aggggttaag cctgcagagc agtcggatag agaaggggaa 1080
ccagatgcct ctcaagcgtt tcagcatgga gggcatctcc aacatcctgc agagtggcat 1140
ccgccagacc tttggctcct caggagctga cagacagtac ctgaacacag tcatccctta 1200
tgagaagaaa tcttctcctc cctctgtgga agacctgcag atgctgacaa acattctctt 1260
cgccatgaag gaggataacg agaaggtgcc tactttgcta acggactaca ttttaaaagt 1320

gctctgtcca acctaacctc acctttggag cagcctcact gcaggaggtc actgagcaag 1380
aaagtcattc catcactggg attgcatgac accacgtaac ctccggcgtg tatttaaacg 1440
tgtatagctt aacctggatt aaacatgagc aaccgcgcac gcgcggggtc ctttgctgtt 1500
ggcttctagt gctagtaatc attggatgca tgatcgggcg tagggccagt gactctgctc 1560
cctctaacct gtgttgggga agacaggtgc cctcaccact cattatgtag tctggctcca 1620
gccctcaacc cagcttatac tcttcagact aattttgaaa taaaccttca tttaattaat 1680
atcaaaaaaa aaaaaaa 1697

<210> 121
<211> 226
<212> PRT
<213> Rattus norvegicus

<220>
<223> PrP

<400> 121
Gly Gly Trp Asn Thr Gly Gly Ser Arg Tyr Pro Gly Gln Gly Ser Pro
1               5                   10                  15

Gly Gly Asn Arg Tyr Pro Pro Gln Ser Gly Gly Thr Trp Gly Gln Pro
            20                  25                  30

His Gly Gly Gly Trp Gly Gln Pro His Gly Gly Gly Trp Gly Gln Pro
        35                  40                  45

His Gly Gly Gly Trp Gly Gln Pro His Gly Gly Gly Trp Ser Gln Gly
    50                  55                  60

Gly Gly Thr His Asn Gln Trp Asn Lys Pro Ser Lys Pro Lys Thr Asn
65                  70                  75                  80

Leu Lys His Val Ala Gly Ala Ala Ala Ala Gly Ala Val Val Gly Gly
                85                  90                  95

Leu Gly Gly Tyr Met Leu Gly Ser Ala Met Ser Arg Pro Met Leu His
            100                 105                 110

Phe Gly Asn Asp Trp Glu Asp Arg Tyr Tyr Arg Glu Asn Met Tyr Arg
        115                 120                 125

Tyr Pro Asn Gln Val Tyr Tyr Arg Pro Val Asp Gln Tyr Ser Asn Gln
    130                 135                 140

Asn Asn Phe Val His Asp Cys Val Asn Ile Thr Ile Lys Gln His Thr
145                 150                 155                 160

Val Thr Thr Thr Thr Lys Gly Glu Asn Phe Thr Glu Thr Asp Val Lys
                165                 170                 175

Met Met Glu Arg Val Val Glu Gln Met Cys Val Thr Gln Tyr Gln Lys
            180                 185                 190

Glu Ser Gln Ala Tyr Tyr Asp Gly Arg Arg Ser Ser Ala Val Leu Phe
        195                 200                 205

Ser Ser Pro Pro Val Ile Leu Leu Ile Ser Phe Leu Ile Phe Leu Ile
    210                 215                 220

Val Gly

225

<210> 122
<211> 1733
<212> DNA
<213> Rattus norvegicus

<220>
<223> PrP

<400> 122
ggagggtgga acactggtgg aagccggtac cctgggcagg gaagccctgg aggcaaccgt 60
tacccacctc agagtggtgg tacctggggg cagccccatg gtggtggctg gggacaacct 120
catggtggtg gctggggaca acctcatggt ggtggctggg gtcagcccca tggcgggggc 180
tggagtcaag gagggggtac ccataatcag tggaacaagc ccagcaagcc aaaaaccaac 240
ctcaagcatg tggcaggggc tgccgcagct ggggcagtag tggggggcct tggtggctac 300
atgttgggga gtgccatgag caggcccatg ctccattttg gcaacgactg ggaggaccgc 360
tactaccgag aaaacatgta ccgttaccct aaccaagtgt actacaggcc ggtggatcag 420
tacagcaacc agaacaactt cgtgcacgac tgtgtcaata tcaccatcaa gcagcataca 480
gtcaccacca ccaccaaggg ggagaacttc acggagaccg acgtgaagat gatggagcgt 540
gtggtggagc agatgtgcgt cacccagtat cagaaggagt cccaggccta ttacgacggg 600
agaagatcta gcgccgtgct tttctcctcc cctcctgtga tcctcctcat ctccttcctc 660
atcttcctga tcgtgggatg aggaggcctt cctgcttgtt ccttctcatt ctcgtggtct 720
aggctggggg aggggttacc cacctgtagc tctttcaatt gaggtggtgt ctcattcttg 780
cttctctttg tccactattc taataccctt ggcagtgatg ggtctgggga aatgtacagt 840
agaccagatg ctattcgctt cagcgtcctt tgattgagtc catcatgggc caggttaaca 900
ccaggccagt aagaatataa caccaaataa ctgctggcta gtcagggctt tgttttggtc 960
tactgagtaa atactgtgta acccctgaat ttgtacccag aggacatggt gacagagaca 1020
cacataactt agtataggca aaggggtcta tagccaaaga agcactgtgt gggcatggca 1080
ccctggataa cagcctcccg cctgggatat ctagagcatc acatgtgaat tctttctttt 1140
ctaacataaa ccatagctga ttgaaggcaa caagaaaaag aatcaaatta tcctactgac 1200
attgaaagca aactatgttc attccctagg cgctggaatg atttttagcc ttggattaaa 1260
ccaggagatt ttgactctga ggagaaccag cagtacaaaa gcatggtctc ctgtgatggg 1320
agagatggtg aggggacaaa ggcaagaccc ctgcgtttct tcatttctgt ctcataatta 1380
tcaagagcta gaattaggtc gtgtctcata attatcaaga gctagaatta ggtcgtgtgc 1440
cctaagtttc tgtactcgta tttgaactgg acaacaaaga gacaatctac aaattctctt 1500
gggctgcaga ggagagaaat aggctccatt ccaaagtgga aagagaaatt ctgctagcat 1560
tgtctaagta aggctaactt ttccttaaat cgctttgtat ttcccccagc agacatcaca 1620
accctgtgat cggttcagcc tgcaccgcgg gtgttctgtg tagaatatat aaatataact 1680
tcaagcttag gccttctatt ttaaaacatc tgaagtgtgg aacgcactgg ccc 1733

<210> 123
<211> 266
<212> PRT
<213> Rattus rattus

<220>
<223> Ribosomal protein L7a

<400> 123

<210> 124
<211> 851
<212> DNA
<213> Rattus rattus

<220>
<223> Ribosomal protein L7a

<400> 124
cgagcggatg cccaaaggaa agaaggccaa ggggaagaag gtggccccgg cccccgccgt 60
ggtcaagaaa caagaggcca aaaaggtggt caatcctttg tttgagaaaa ggcctaagaa 120
cttcggcatt gggcaggaca tccagcccaa aagagatctc acgcgcttcg tcaaatggcc 180
ccgctacatc aggctgcagc ggcaaagagc catcctctat aagcggctca aagtacctcc 240
tgccatcaac cagttcaccc aggccctgga caggcaaaca gcgactcagc tgcttaagct 300
tgcccacaag tacaggccag agacaaagca ggagaagaag cagaggctgc tggcccgcgc 360
tgagaagaaa gctgctggca aaggggacgt cccaactaag agaccacctg tcctccgagc 420
aggggtcaat acagtcacca ctttggtgga gaacaagaag gctcagctgg tggtgattgc 480
ccatgatgta gaccccattg agctggtggt tttcctgcct gccctgtgtc gaaagatggg 540

ggtgccctac tgcatcatca agggaaaggc caggctgggg cgcctggtcc acaggaagac 600
atgcaccact gttgccttca cacaggtgaa ctcggaagac aagggtgctc tggctaagct 660
ggtggaagct attaggacca attataatga cagatatgac gagatccgcc gccactgggg 720
aggcaacgtc ctgggtccta agtctgtggc tcgcattgcc aagctggaaa aggcaaaggc 780
taaagaactc gccactaagc tgggttaaat gtacacttaa gttttctgta cataaatata 840
attacaaagt t 851

<210> 125
<211> 472
<212> PRT
<213> Rattus norvegicus

<220>
<223> Type 1 astrocyte and olefactory-limbic associated protein

<400> 125
Met Asp Tyr Gly Thr Ser Leu Ser Ala Asp Leu Ser Leu Asn Lys Glu
1               5                   10                  15

Thr Gly Ser Leu Ser Leu Lys Gly Ser Lys Leu His Asn Lys Thr Leu
            20                  25                  30

Lys Arg Thr Arg Arg Phe Val Val Asp Gly Val Glu Val Ser Ile Thr
        35                  40                  45

Thr Ser Lys Ile Ile Ser Glu Asp Glu Lys Lys Asp Glu Glu Met Arg
    50                  55                  60

Phe Leu Arg Arg Gln Glu Leu Arg Glu Leu Arg Leu Leu Gln Lys Glu
65                  70                  75                  80

Glu His Arg Asn Gln Thr Gln Leu Ser Thr Lys His Glu Leu Gln Leu
                85                  90                  95

Glu Gln Met His Arg Arg Phe Glu Gln Glu Ile Asn Ala Lys Lys Lys
            100                 105                 110

Phe Tyr Asp Val Glu Leu Glu Asn Leu Glu Arg Gln Gln Lys Gln Gln
        115                 120                 125

Val Glu Lys Met Glu Gln Asp His Ser Val Arg Arg Arg Glu Glu Ala
    130                 135                 140

Lys Arg Ile Arg Leu Glu Gln Asp Arg Asp Tyr Ala Arg Phe Gln Glu
145                 150                 155                 160

Gln Leu Lys Gln Met Lys Lys Glu Val Lys Asn Glu Val Glu Lys Leu
                165                 170                 175

Pro Arg Gln Gln Arg Lys Glu Ser Met Lys Gln Lys Met Glu Glu His
            180                 185                 190

Ala Gln Lys Lys Gln Leu Leu Asp Arg Asp Phe Val Ala Lys Gln Lys
        195                 200                 205

Glu Asp Leu Glu Leu Ala Met Lys Lys Leu Thr Ala Glu Asn Arg Arg
    210                 215                 220

Glu Ile Cys Asp Lys Glu Arg Asp Cys Leu Asn Lys Lys Gln Glu Leu
225                 230                 235                 240

Leu Arg Asp Arg Glu Ala Ala Leu Trp Glu Met Glu Glu His Gln Leu
                245                 250                 255

Gln Glu Arg His Gln Leu Val Lys Gln Gln Leu Lys Asp Gln Tyr Phe
            260                 265                 270

Leu Gln Arg His Asp Leu Leu Arg Lys His Glu Lys Glu Arg Glu Gln
        275                 280                 285

Met Gln Arg Tyr Asn Gln Arg Met Met Glu Gln Leu Lys Val Arg Gln
    290                 295                 300

Gln Gln Glu Lys Ala Arg Leu Pro Lys Ile Gln Arg Ser Asp Gly Lys
305                 310                 315                 320

Thr Arg Met Ala Met Tyr Lys Lys Ser Leu His Ile Asn Gly Ala Gly
                325                 330                 335

Ser Ala Ser Glu Gln Arg Glu Lys Val Lys Gln Phe Ser Gln Gln Glu
            340                 345                 350

Glu Lys Arg Gln Lys Ala Glu Arg Leu Gln Gln Gln Gln Lys His Glu
        355                 360                 365

Asn Gln Met Arg Asp Met Val Ala Gln Cys Glu Ser Asn Met Asn Glu
    370                 375                 380

Leu Gln Gln Leu Gln Asn Glu Lys Cys His Leu Leu Val Glu His Glu
385                 390                 395                 400

Thr Gln Lys Leu Lys Ala Leu Asp Glu Ser His Asn Gln Ser Leu Lys
                405                 410                 415

Glu Trp Arg Asp Lys Leu Arg Pro Arg Lys Lys Ala Leu Glu Glu Asp
            420                 425                 430

Leu Asn Gln Lys Lys Arg Glu Gln Glu Met Phe Phe Arg Leu Ser Glu
        435                 440                 445

Glu Ala Glu Thr Arg Pro Thr Thr Pro Asn Arg Ala Ser Lys Phe Phe
    450                 455                 460

Pro Tyr Ser Ser Gly Asp Ala Ser
465                 470

<210> 126
<211> 4301
<212> DNA
<213> Rattus norvegicus

<220>
<223> Type 1 astrocyte and olefactory-limbic associated protein

<400> 126
ggcacgagaa gtcggaccct cccaccctgc tcacaccctc caagtggtcc acggagttcc 60
gagactttct gaagatagcc ttggataaga acccagaaac ccggcccagt gctgcgcagc 120
tgctgcagca tcccttcgtc agtacagtca ccagtaataa ggcccttcgg gagctggtgg 180
ctgaggcgaa ggctgaggtg atggaggaga tcgaggacgg caaggaggac ggcagggagg 240
acggcaggga ggatgggaaa gaggaggatt gagacgagaa ggatgctgtg agtgctgttc 300

cgcccccagt caaccacact caggactcct ctgccaatgg aactcagcca agcctcaact 360
ctgacaagct tctccaggat tcttctaccc ccctgcctcc cagccagcct caggagcctg 420
tgaacgggcc ctgtaaccaa ccctcagggg atggatcccc ccagaacacc agccctgcag 480
atgaggtctc caagaatgac aatggcttaa aggtacctgt tcccctccgg aagtcccggc 540
cattgtccgt ggatgccaga attcaggtga ccgaagagaa acaaatcact gaccaggctg 600
agaaccccag ttctgcagcc agcaaacccc cgaaggtcaa ccagagccga cctaacagca 660
gcgccctgga gactttgggt gtcgagactc tggccaatgg aggcctggag ctccctggct 720
ctgtaactcc aaaccattct aagagggcgt cggactgtag caacctgtct acctcagaga 780
gcatggacta cggcacctcc ttgtctgctg acctgtcatt gaacaaagag acgggctcat 840
tgtctctcaa gggctcaaaa ctgcacaaca agaccctgaa gaggacccgc cggtttgtgg 900
tggacggtgt ggaggtgagc atcaccacct ccaagatcat cagcgaagac gagaagaaag 960
acgaggagat gaggtttctc aggcgccagg aactccgaga gcttcggctc ctgcagaaag 1020
aagagcatcg gaaccagacg cagctgagca ccaagcacga gctgcagctg gagcagatgc 1080
acagacgatt tgaacaagaa atcaacgcca agaagaaatt ctatgacgtg gagctagaga 1140
acctggagcg gcagcagaag cagcaggtgg agaagatgga gcaggaccac agcgtgcgtc 1200
gcagagagga ggccaagcgg atccgcctgg agcaggatcg agactacgcc aggttccaag 1260
agcagctcaa gcagatgaag aaggaggtga agaatgaggt tgagaaactg ccccggcaac 1320
agcggaagga gagcatgaag cagaagatgg aggagcacgc acagaagaaa caactgctgg 1380
accgagactt tgtagccaag cagaaggaag acctggagct ggccatgaag aagctcacgg 1440
cagaaaacag gcgtgagatc tgtgacaagg aacgtgattg ccttaacaag aagcaggagc 1500
tcctccgaga ccgagaggca gccctgtggg agatggagga gcaccagtta caggagagac 1560
atcagctggt gaagcagcag cttaaggacc agtacttcct gcagcggcat gacctgctgc 1620
gcaagcacga gaaggagcgg gagcagatgc agcgctacaa ccagcgtatg atggagcagc 1680
tgaaggtcag acagcagcag gagaaggcgc ggctacccaa gatccagagg agtgacggca 1740
agacccgcat ggccatgtac aagaagagcc tgcacatcaa tggtgcgggc agtgcctccg 1800
agcagcggga gaaggtcaag cagttctccc agcaggaaga gaagaggcag aaggcggaga 1860
ggctgcagca gcagcagaaa cacgagaacc agatgcgaga catggtggca cagtgcgaga 1920
gcaacatgaa cgagctgcag cagctgcaga atgaaaagtg tcatctgtta gtggagcatg 1980
aaacccagaa gctgaaggcc ctggacgaga gccataacca gagcctgaag gaatggcgag 2040
acaagcttcg gccacgcaaa aaggccctgg aagaggattt gaaccagaag aagcgggaac 2100
aggaaatgtt cttcagacta agtgaggagg cagagaccag acccaccaca cccaacagag 2160
ccagcaagtt cttcccctac agctctgggg atgcttccta acacacacat gcctgggctg 2220
cggtgcggca gtacagccac cagggccacc aaccctctac aaacaagtga ctcaggacct 2280
cttcctcttg cttctgtgcc agctccaact acccagcacc ccagttgccc acagcaccac 2340
cccagtgctt ctgatggatg acctcatccc aactcagatt cccatcacct ggaagtgacc 2400
tgggctgttg ggtccgggga ccgagcggga tgggcgtacc cctcctgttt gccaaaacac 2460
cagctctact gtctgtgggc acaagcgcta ctgatgacat caccacgaac ccatccttat 2520
tgtgatcctt gtggtttttt cttctccttc agtaattcct cacagtgttg gaaaacatcc 2580
ctcagagcca ttttgcttct cagcagccag ctctcagggg tgtccccatt accctgcttc 2640
gcacagctga ctttgtgctc gatgagacgc tgtgtatgtg ggggtaggga gtggggaaag 2700
ggaggccaga aatgttcatt ctgctggttt ctgacatttt atgccatctc attttgcctc 2760
tccctgtcac acacacacac acacacacac acacacacac acacacacac acacacacaa 2820
tgcaaacaca caacttggcc ctcctgaacc tgatcgtagg acacggagta cagagcatgt 2880
caggtggagc agctgctggg gcatgctgag tgctggcccc aaagcccaga gaaggcacag 2940
gctgtactgc agcctgcctg ccactcgttt ggctgcacac aggatcctgt gttcaggggt 3000
aaactcccct ccacacttgt cttctgctgc ctagcgcatg ccaatctcgc ccttgcccag 3060
ttgttggcaa gtactgggga aggctcctga cctttgacct ttgccccagt cctgcactgg 3120
agtcccactg tacatttcca ctaagccgga cagtcctttg gacttctctg tttaggaaga 3180
gatgcttccc accccctgga acagccgaag ctcaggaaaa tgccaagcct cgtgcctggg 3240
cctttgggtt gctcaggtag cctcccaaga tgctgcgccc cataggctac catgcccaga 3300
aaagcagctg gtcggcccag ccggcgttcg ctgatagcgc cttagggctc agttaaagca 3360
caggtaaatg gctggctgct ttgtataccc tccttttaga cagcatcacc ccaggattta 3420
ggatgggatg ggtgggggcg gggcacccag gcagtggagt ctgggagtgg ctgagacctc 3480
agcagtattt ccccatcact gccccatgct gagacaacct tctaggacgt ttcctcagat 3540
gctgactggg tgcttgggag gggagtgggc tagtaaaaca aaataggaaa acaggtcttg 3600
ggactcccag atcttgtgtg cagtaaggaa gttcacagag ccccaggaag gcgatagttc 3660
tcagggtagc gagcgtcagc ttgctttcag gccgcacacc gaggagtctt gaggaacagt 3720
tgacttcttt cttactggtg catgggggct gggaaacaca agttgtcaga gtgcagctgt 3780
gggactcaga gatgggaagt gggcaaggcc acgccctgca gggctctacc attgtttaca 3840
atgtacttgg ctgcattcgg gggtgggggg aacttgacag tggctattag gcaaaatgcc 3900
ggttttgtgg ttcaggtaac agtctttgac cactccctga cgtcattcgt actgtcctcc 3960

tccttgttgc ttccacactt agtcccacct gagctctggt acctctgctg tgcctttttt 4020
gagtggggtc tagccttgtc ttccagcctc ataatttaac ctaagtgcaa tgcctgccac 4080
cgacaaaggc ccgtgaagta ttcctcatgt cctgtgctaa cgttttctgt ataggaacag 4140
gcagaaatgt ctttagcacc gcggatataa ctaacttata tttcccttca cgaaggatag 4200
aagtaacggg tgtgtcattt ccaacggtca tgtataattt ttgtaaactg ttctctgcaa 4260
acaaaaaaaa tgtaaatatg cttctaataa aataataagg t 4301

<210> 127
<211> 267
<212> PRT
<213> Rattus norvegicus

<220>
<223> C1-13 protein

<400> 127
Met Asp Cys Val Trp Ser Asn Trp Lys Ser Gln Ala Ile Asp Leu Leu
1               5                   10                  15

Tyr Trp Arg Asp Ile Lys Gln Thr Gly Ile Val Phe Gly Ser Phe Leu
            20                  25                  30

Leu Leu Leu Phe Ser Leu Thr Gln Phe Ser Val Val Ser Val Val Ala
        35                  40                  45

Tyr Leu Ala Leu Ala Ala Leu Ser Ala Thr Ile Ser Phe Arg Ile Tyr
    50                  55                  60

Lys Ser Val Leu Gln Ala Val Gln Lys Thr Asp Glu Gly His Pro Phe
65                  70                  75                  80

Lys Ala Tyr Leu Glu Leu Glu Ile Thr Leu Ser Gln Glu Gln Ile Gln
                85                  90                  95

Lys Tyr Thr Asp Cys Leu Gln Leu Tyr Val Asn Ser Thr Leu Lys Glu
            100                 105                 110

Leu Arg Arg Leu Phe Leu Val Gln Asp Leu Val Asp Ser Leu Lys Phe
        115                 120                 125

Ala Val Leu Met Trp Leu Leu Thr Tyr Val Gly Ala Leu Phe Asn Gly
    130                 135                 140

Leu Thr Leu Leu Leu Met Ala Val Val Ser Met Phe Thr Leu Pro Val
145                 150                 155                 160

Val Tyr Val Lys His Gln Ala Gln Val Asp Gln Tyr Leu Gly Leu Val
                165                 170                 175

Arg Thr His Ile Asn Thr Val Val Ala Lys Ile Gln Ala Lys Ile Pro
            180                 185                 190

Gly Ala Arg Gly Met Leu Ser Arg Trp Leu Pro Gln Glu Lys Pro Asp
        195                 200                 205

Met Asn Gly Gly Val Trp Ser Gly Asn Ser Ser Leu Leu Pro Arg Tyr
    210                 215                 220

Cys Glu Leu Ile Val Ser Leu Pro Gln Tyr His Asn Leu Arg Gly Lys
225                 230                 235                 240

Leu Arg Asp Arg Cys Phe Gln Ser Phe Pro Val Leu Leu Gly Tyr Leu
                245                 250                 255

Ser Pro Pro Arg Pro Leu Ser Ser Thr Lys Val
            260                 265


<210> 128
<211> 1473
<212> DNA
<213> Rattus norvegicus

<220>
<223> C1-13 protein

<400> 128
cccaccgccg ccgcagccgg gcagcgacgc agagtgcaag ctcagcgact ggaggagagc 60
cacgagcccg ccaagcccgc accatgcagc cactgccgat tccaccaaga tggactgtgt 120
gtggagcaac tggaaaagtc aggctattga ccttctgtac tggcgggaca tcaagcagac 180
ggggattgtg ttcgggagct tcctgctgct gctcttctcc ctgacccagt tcagcgttgt 240
gagcgtcgtc gcctacctgg ccctggctgc cctctctgcc accatcagct tccgcatcta 300
caagtccgtt ctacaagctg tgcagaaaac agatgagggt caccctttca aggcctacct 360
ggagctggag atcaccctgt cccaggagca gatccagaag tacacagact gcctgcagct 420
atacgtgaac agcactctga aggagctacg gaggctcttc cttgtccaag acctagtgga 480
ttccttaaaa tttgcagtcc tcatgtggct cctgacctac gttggcgcac tcttcaatgg 540
cctgaccctg ctgcttatgg ctgtggtttc gatgtttact ctacctgtgg tatatgttaa 600
gcaccaggca caggttgacc aatatctggg acttgtgagg actcacataa acaccgttgt 660
ggcaaagatc caggctaaaa tccccggcgc aagaggcatg ctgagtagat ggttgcccca 720
ggagaagccg gacatgaatg gggtgtctgg gagtggtaac agctctctcc ttcctcgtta 780
ctgcgaattg attgtttccc ttccccagta ccataatctt agaggcaaac ttcgagacag 840
gtgttttcag tcttttcctg tactcttagg atatttgagt cccccccgcc ccttgtcaag 900
cactaaagta tagagtgtgt tagatgtggt ttttaatttt gtgttgctaa aaaaaaaaaa 960
agtgcatgat ggtgagagct cacgttctcc ttcgtccctt gctgtcctcc ccctgcaatg 1020
cttctgtggc ttctactgtc cctgtggcta ggcccttctt gccaagtgcg ctgacacata 1080
gtgggtcgct tatcatgtcc ttgggttgct ggttggattc atctctaata acactatata 1140
gaatcatagg ccaatgtttt agcattttac ccccccacac acagaacata aaaattaata 1200
taaaacagtt gaccgtgtat atggtgatcc gttttgtata actaagaatg atcgaattaa 1260
agaataaatc caaaaatgaa cgcacagtac ttttattgta tgggatgcct gggctgattt 1320
acatgtatgg taactaaagt gccagcatgt tgactttatt acgatttgta tttcttcctc 1380
tgtagttcct aacggactca atcacagact ctgggtattt gcacttatgt acttgatact 1440
agaatgcacc aataaatgtg actcaaatgt aaa                              1473


<210> 129
<211> 591
<212> PRT
<213> Rattus rattus

<220>
<223> Calnexin

<400> 129
Met Glu Gly Lys Trp Leu Leu Cys Leu Leu Leu Val Leu Gly Thr Ala
1               5                   10                  15

Ala Ile Gln Ala His Asp Gly His Asp Asp Asp Met Ile Asp Ile Glu
            20                  25                  30

Asp Asp Leu Asp Asp Val Ile Glu Glu Val Glu Asp Ser Lys Ser Lys
        35                  40                  45

Ser Asp Thr Ser Thr Pro Pro Ser Pro Lys Val Thr Tyr Lys Ala Pro
    50                  55                  60

Val Pro Thr Gly Glu Val Tyr Phe Ala Asp Ser Phe Asp Arg Gly Ser
65                  70                  75                  80

Leu Ser Gly Trp Ile Leu Ser Lys Ala Lys Lys Asp Asp Thr Asp Asp
                85                  90                  95

Glu Ile Ala Lys Tyr Asp Gly Lys Trp Glu Val Asp Glu Met Lys Glu
            100                 105                 110

Thr Lys Leu Pro Gly Asp Lys Gly Leu Val Leu Met Ser Arg Ala Lys
        115                 120                 125

His His Ala Ile Ser Ala Lys Leu Asn Lys Pro Phe Leu Phe Asp Thr
    130                 135                 140

Lys Pro Leu Ile Val Gln Tyr Glu Val Asn Phe Gln Asn Gly Ile Glu
145                 150                 155                 160

Cys Gly Gly Ala Tyr Val Lys Leu Leu Ser Lys Thr Ser Glu Leu Asn
                165                 170                 175

Leu Asp Gln Phe His Asp Lys Thr Pro Tyr Thr Ile Met Phe Gly Pro
            180                 185                 190

Asp Lys Cys Gly Glu Asp Tyr Lys Leu His Phe Ile Phe Arg His Lys
        195                 200                 205

Asn Pro Lys Thr Gly Val Tyr Glu Glu Lys His Ala Lys Arg Pro Asp
    210                 215                 220

Ala Asp Leu Lys Thr Tyr Phe Thr Asp Lys Lys Thr His Leu Tyr Thr
225                 230                 235                 240

Leu Ile Leu Asn Pro Asp Asn Ser Phe Glu Ile Leu Val Asp Gln Ser
                245                 250                 255

Val Val Asn Ser Gly Asn Leu Leu Asn Asp Met Thr Pro Pro Val Asn
            260                 265                 270

Pro Ser Arg Glu Ile Glu Asp Pro Glu Asp Arg Lys Pro Glu Asp Trp
        275                 280                 285

Asp Glu Arg Pro Lys Ile Ala Asp Pro Asp Ala Val Lys Pro Asp Asp
    290                 295                 300

Trp Asp Glu Asp Ala Pro Ser Lys Ile Pro Asp Glu Glu Ala Thr Lys
305                 310                 315                 320

Pro Glu Gly Trp Leu Asp Asp Glu Pro Glu Tyr Ile Pro Asp Pro Asp
                325                 330                 335

Ala Glu Lys Pro Glu Asp Trp Asp Glu Asp Met Asp Gly Glu Trp Glu
            340                 345                 350

Ala Pro Gln Ile Ala Asn Pro Lys Cys Glu Ser Ala Pro Gly Cys Gly
        355                 360                 365

Val Trp Gln Arg Pro Met Ile Asp Asn Pro Asn Tyr Lys Gly Lys Trp
    370                 375                 380

Lys Pro Pro Met Ile Asp Asn Pro Asn Tyr Gln Gly Ile Trp Lys Pro
385                 390                 395                 400

Arg Lys Ile Pro Asn Pro Asp Phe Phe Glu Asp Leu Glu Pro Phe Arg
                405                 410                 415

Met Thr Pro Phe Ser Ala Ile Gly Leu Glu Leu Trp Ser Met Thr Ser
            420                 425                 430

Asp Ile Phe Phe Asp Asn Phe Ile Ile Ser Gly Asp Arg Arg Val Val
        435                 440                 445

Asp Asp Trp Ala Asn Asp Gly Trp Gly Leu Lys Lys Ala Ala Asp Gly
    450                 455                 460

Ala Ala Glu Pro Gly Val Val Gly Gln Met Leu Glu Ala Ala Glu Glu
465                 470                 475                 480

Arg Pro Trp Leu Trp Val Val Tyr Ile Leu Thr Val Ala Leu Pro Val
                485                 490                 495

Phe Leu Val Ile Leu Phe Cys Cys Ser Gly Lys Lys Gln Ser Asn Ala
            500                 505                 510

Met Glu Tyr Lys Lys Thr Asp Ala Pro Gln Pro Asp Val Lys Asp Glu
        515                 520                 525

Glu Gly Lys Glu Glu Glu Lys Asn Lys Gly Asp Glu Glu Glu Glu Glu
    530                 535                 540

Glu Lys Leu Glu Glu Lys Gln Lys Ser Asp Ala Glu Glu Asp Gly Gly
545                 550                 555                 560

Thr Gly Ser Gln Asp Glu Glu Asp Ser Lys Pro Lys Ala Glu Glu Asp
                565                 570                 575

Glu Ile Leu Asn Arg Ser Pro Arg Asn Arg Lys Pro Arg Arg Glu
            580                 585                 590


<210> 130
<211> 1776
<212> DNA
<213> Rattus rattus

<220>
<223> Calnexin

<400> 130
atggaaggga agtggttact gtgtttgcta ctggtccttg gaactgcagc tattcaggct 60
catgatggac atgatgatga catgattgat attgaagatg atcttgatga tgttatcgaa 120
gaggtagaag attcaaaatc taaatcagat accagcactc ctccatctcc aaaggtcacc 180
tacaaagctc cagttccaac aggggaggtt tattttgctg actcctttga cagagggtct 240
ctgtcagggt ggattttatc taaagccaaa aaagacgaca ctgatgatga aattgccaaa 300
tatgacggaa agtgggaggt agatgaaatg aaggaaacaa agcttccagg tgataaagga 360
cttgtattga tgtctcgggc caagcatcat gccatctctg ctaaactgaa caagcccttc 420
ctgtttgata ccaagcctct cattgttcag tatgaggtta actttcagaa tggaatagaa 480
tgcggtggtg cctacgtgaa gctgctatcc aagacctcag aactcaacct ggatcaattc 540

cacgacaaga ctccgtatac tattatgttt ggtccagata aatgtggaga ggactacaaa 600
ctgcatttca tctttcgcca caaaaacccc aagacaggcg tatatgaaga aaaacatgct 660
aagaggccag atgcagatct gaagacctat ttcactgaca agaaaacaca tctttacaca 720
ttaatcttga atccagacaa tagttttgaa atattagttg accagtctgt tgtgaacagt 780
ggaaacctgc taaatgacat gacccctccg gtaaaccctt cacgtgaaat tgaggaccca 840
gaagaccgga agcctgaaga ttgggatgaa aggcccaaaa tagcagatcc agatgctgtc 900
aagccagatg actgggatga agatgcccct tctaagattc cagatgaaga ggccacaaag 960
cctgaaggct ggttagatga tgaacccgag tatattccag accctgatgc agagaaacca 1020
gaagattggg atgaggatat ggacggagaa tgggaggctc ctcagattgc caaccctaag 1080
tgtgagtccg cccctgggtg tggtgtctgg cagcgaccta tgattgacaa ccccaattat 1140
aagggcaaat ggaagcctcc tatgattgac aatcctaact accagggaat ctggaaacca 1200
aggaaaatac caaatccaga tttctttgaa gacctggaac cttttaggat gactcctttc 1260
agcgctattg gtttggagct ctggtccatg acatccgaca tcttttttga caactttatc 1320
attagtggtg accgaagagt agttgatgac tgggccaatg atgggtgggg cctgaagaaa 1380
gctgctgatg gggctgcaga gccaggtgta gtggggcaga tgctggaggc agctgaagag 1440
cgtccatggc tttgggtggt ctacattctg actgtagcgt tgccagtgtt ccttgtgatc 1500
ctcttctgct gttctggaaa gaaacagtcc aatgctatgg agtacaagaa gacagatgct 1560
ccccagccag atgtgaagga cgaagaaggg aaggaagaag agaagaacaa gggagatgaa 1620
gaggaagaag aggagaagct tgaagagaaa cagaagagtg atgctgaaga agatggtggc 1680
actggcagtc aagatgagga agatagcaag cctaaagcag aggaggatga aattttgaac 1740
agatcaccaa gaaacagaaa gccacgaaga gagtga 1776

<210> 131
<211> 269
<212> PRT
<213> Rattus norvegicus

<220>
<223> Preproenkephalin

<400> 131
Met Ala Gln Phe Leu Arg Leu Cys Ile Trp Leu Val Ala Leu Gly Ser
1               5                   10                  15

Cys Leu Leu Ala Thr Val Gln Ala Asp Cys Ser Gln Asp Cys Ala Lys
            20                  25                  30

Cys Ser Tyr Arg Leu Val Arg Pro Gly Asp Ile Asn Phe Leu Ala Cys
        35                  40                  45

Thr Leu Glu Cys Glu Gly Gln Leu Pro Ser Phe Lys Ile Trp Glu Thr
    50                  55                  60

Cys Lys Asp Leu Leu Gln Val Ser Lys Pro Glu Phe Pro Trp Asp Asn
65                  70                  75                  80

Ile Asp Met Tyr Lys Asp Ser Ser Lys Gln Asp Glu Ser His Leu Leu
                85                  90                  95

Ala Lys Lys Tyr Gly Gly Phe Met Lys Arg Tyr Gly Gly Phe Met Lys
            100                 105                 110

Lys Met Asp Glu Leu Tyr Pro Val Glu Pro Glu Glu Glu Ala Asn Gly
        115                 120                 125

Gly Glu Ile Leu Ala Lys Arg Tyr Gly Gly Phe Met Lys Lys Asp Ala
    130                 135                 140

Asp Glu Gly Asp Thr Leu Ala Asn Ser Ser Asp Leu Leu Lys Glu Leu
145                 150                 155                 160

Leu Gly Thr Gly Asp Asn Arg Ala Lys Asp Ser His Gln Gln Glu Ser
                165                 170                 175

Thr Asn Asn Asp Glu Asp Ser Thr Ser Lys Arg Tyr Gly Gly Phe Met
            180                 185                 190

Arg Gly Leu Lys Arg Ser Pro Gln Leu Glu Asp Glu Ala Lys Glu Leu
        195                 200                 205

Gln Lys Arg Tyr Gly Gly Phe Met Arg Arg Val Gly Arg Pro Glu Trp
    210                 215                 220

Trp Met Asp Tyr Gln Lys Arg Tyr Gly Gly Phe Leu Lys Arg Phe Ala
225                 230                 235                 240

Glu Ser Leu Pro Ser Asp Glu Glu Gly Glu Ser Tyr Ser Lys Glu Val
                245                 250                 255

Pro Glu Met Glu Lys Arg Tyr Gly Gly Phe Met Arg Phe
            260                 265

<210> 132
<211> 1446
<212> DNA
<213> Rattus norvegicus

<220>
<223> Preproenkephalin

<400> 132
ctcggaagga caggatgcca tgccatcggg aagacaggac tccccaagga gaacaggatg 60
ccatcaggga gacaggactc ccccgtggaa gataggatac ctccaggaag acagaatgcc 120
ccccaggcag ccctgggaca gcgggaaaca ctagggacca agctatccct ggttcactcg 180
ggattttgtt gggttgtggg cggggctcag gaaagattgt ccctgctggt cctgctccac 240
gaccacccac ccggcaaggt tccctcctag agaaccttgt cagagacaga acgggtcccc 300
acaggcgcat tcttctttcc aacacccatg gcgcagttcc tgagactttg catctggctc 360
gtagcgcttg ggtcctgcct cctggctaca gtgcaggcag actgcagcca ggactgcgct 420
aaatgcagct accgcctggt acgtcccggc gacatcaact tcctggcatg cacactcgaa 480
tgtgaagggc agctgccttc tttcaaaatc tgggagacct gcaaggatct cctgcaggtg 540
tccaagcccg agttcccttg ggataacatc gacatgtaca aagacagcag caaacaggat 600
gagagccact tgctagccaa gaagtatgga gggttcatga aacggtatgg aggcttcatg 660
aagaagatgg atgagcttta ccccgtggag ccagaagaag aggccaatgg aggcgagatc 720
cttgccaaga ggtatggcgg tttcatgaag aaggatgcag atgagggaga caccttggcc 780
aactcctccg acctgctgaa agagctactg ggaacaggag acaaccgtgc gaaagatagc 840
caccaacagg aaagcaccaa caatgatgaa gacagcacga gcaagaggta tgggggcttc 900
atgagaggcc tcaaaagaag cccccagctg gaagacgaag caaaggagct gcagaagcgc 960
tatgggggct tcatgagaag ggtcgggcgc cccgagtggt ggatggacta tcagaagaga 1020
tacggaggct tcctgaagcg ctttgctgag tctctaccct cggatgaaga aggcgaaagt 1080
tactctaaag aagttcccga gatggaaaaa agatacggag gctttatgcg gttttgaagc 1140
ccttcccagc agtgatcccg ggcccccact agcctgctcc atcccccgtg agcaactgcc 1200
ccatcagtga tgtttcttgt catgtgctgc ttgtgctgta cagttgccct cgtggtctgg 1260
ataactgctg cctgaaagct gtgattttta gggggttgtc tgtattcttt tgagtcttga 1320
agctcagtat tggtctcttg tggctatgtt atcatgctga aacagtctgt tacctcatcc 1380
cttctgacaa aacgtcaata aatgcttatt tgtatataaa aataataaac ccgtgaaccc 1440
aactgc                                                            1446

<210> 133
<211> 807

<212> PRT
<213> Rattus norvegicus

<220>
<223> F-spondin

<400> 133
Met Arg Leu Ser Pro Ala Pro Leu Arg Leu Ser Arg Gly Pro Ala Leu
1               5                   10                  15

Leu Ala Leu Ala Leu Pro Leu Ala Ala Ala Leu Ala Phe Ser Asp Glu
            20                  25                  30

Thr Leu Asp Lys Val Ala Lys Ser Glu Gly Tyr Cys Ser Arg Ile Leu
        35                  40                  45

Arg Ala Gln Gly Thr Arg Arg Glu Gly Tyr Thr Glu Phe Ser Leu Arg
    50                  55                  60

Val Glu Gly Asp Pro Asp Phe Tyr Lys Pro Gly Ser Ser Tyr Arg Val
65                  70                  75                  80

Thr Leu Ser Ala Ala Pro Pro Ser Tyr Phe Arg Gly Phe Thr Leu Ile
                85                  90                  95

Ala Leu Lys Glu Asn Arg Glu Gly Asp Lys Glu Glu Asp His Ala Gly
            100                 105                 110

Thr Phe Gln Ile Ile Asp Glu Glu Glu Thr Gln Phe Met Ser Asn Cys
        115                 120                 125

Pro Val Ala Val Thr Glu Ser Thr Pro Arg Arg Arg Thr Arg Ile Gln
    130                 135                 140

Val Phe Trp Ile Ala Pro Pro Thr Gly Thr Gly Cys Val Ile Leu Lys
145                 150                 155                 160

Ala Ser Ile Val Gln Lys Arg Ile Ile Tyr Phe Gln Asp Glu Gly Ser
                165                 170                 175

Leu Thr Lys Lys Leu Cys Glu Gln Asp Pro Thr Leu Asp Gly Val Thr
            180                 185                 190

Asp Arg Pro Ile Leu Asp Cys Cys Ala Cys Gly Thr Ala Lys Tyr Arg
        195                 200                 205

Leu Thr Phe Tyr Gly Asn Trp Ser Glu Lys Thr His Pro Lys Asp Tyr
    210                 215                 220

Pro Arg Arg Ala Asn His Trp Ser Ala Ile Ile Gly Gly Ser His Ser
225                 230                 235                 240

Lys Asn Tyr Val Leu Trp Glu Tyr Gly Gly Tyr Ala Ser Glu Gly Val
                245                 250                 255

Lys Gln Val Ala Glu Leu Gly Ser Pro Val Lys Met Glu Glu Glu Ile
            260                 265                 270

Arg Gln Gln Ser Asp Glu Val Leu Thr Val Ile Lys Ala Lys Ala Gln
        275                 280                 285

Trp Pro Ser Trp Gln Pro Val Asn Val Arg Ala Ala Pro Ser Ala Glu
    290                 295                 300

Phe Ser Val Asp Arg Thr Arg His Leu Met Ser Phe Leu Thr Met Met
305                 310                 315                 320

Gly Pro Ser Pro Asp Trp Asn Val Gly Leu Ser Ala Glu Asp Leu Cys
                325                 330                 335

Thr Lys Glu Cys Gly Trp Val Gln Lys Val Val Gln Asp Leu Ile Pro
            340                 345                 350

Trp Asp Ala Gly Thr Asp Ser Gly Val Thr Tyr Glu Ser Pro Asn Lys
    355                 360                 365

Pro Thr Ile Pro Gln Glu Lys Ile Arg Pro Leu Thr Ser Leu Asp His
    370                 375                 380

Pro Gln Ser Pro Phe Tyr Asp Pro Glu Gly Gly Ser Ile Thr Gln Val
385                 390                 395                 400

Ala Arg Val Val Ile Glu Arg Ile Ala Arg Lys Gly Glu Gln Cys Asn
                405                 410                 415

Ile Val Pro Asp Asn Val Asp Asp Ile Val Ala Asp Leu Ala Pro Glu
            420                 425                 430

Glu Lys Asp Glu Asp Asp Thr Pro Glu Thr Cys Ile Tyr Ser Asn Trp
    435                 440                 445

Ser Pro Trp Ser Ala Cys Ser Ser Ser Thr Cys Glu Lys Gly Lys Arg
    450                 455                 460

Met Arg Gln Arg Met Leu Lys Ala Gln Leu Asp Leu Ser Val Pro Cys
465                 470                 475                 480

Pro Asp Thr Gln Asp Phe Gln Pro Cys Met Gly Pro Gly Cys Ser Asp
                485                 490                 495

Glu Asp Gly Ser Thr Cys Thr Met Ser Glu Trp Ile Thr Trp Ser Pro
            500                 505                 510

Cys Ser Val Ser Cys Gly Met Gly Met Arg Ser Arg Glu Arg Tyr Val
    515                 520                 525

Lys Gln Phe Pro Glu Asp Gly Ser Val Cys Met Leu Pro Thr Glu Glu
    530                 535                 540

Thr Glu Lys Cys Thr Val Asn Glu Glu Cys Ser Pro Ser Ser Cys Leu
545                 550                 555                 560

Val Thr Glu Trp Gly Glu Trp Asp Asp Cys Ser Ala Thr Cys Gly Met
                565                 570                 575

Gly Met Lys Lys Arg His Arg Met Val Lys Met Ser Pro Ala Asp Gly
            580                 585                 590

Ser Met Cys Lys Ala Glu Thr Ser Gln Ala Glu Lys Cys Met Met Pro
    595                 600                 605

Glu Cys His Thr Ile Pro Cys Leu Leu Ser Pro Trp Ser Glu Trp Ser

610                 615                 620

Asp Cys Ser Val Thr Cys Gly Lys Gly Met Arg Thr Arg Gln Arg Met
625                 630                 635                 640

Leu Lys Ser Leu Ala Glu Leu Gly Asp Cys Asn Glu Asp Leu Glu Gln
                645                 650                 655

Ala Glu Lys Cys Met Leu Pro Glu Cys Pro Ile Asp Cys Glu Leu Ser
            660                 665                 670

Glu Trp Ser Gln Trp Ser Glu Cys Asn Lys Ser Cys Gly Lys Gly His
        675                 680                 685

Met Ile Arg Thr Arg Thr Ile Gln Met Glu Pro Gln Phe Gly Gly Ala
    690                 695                 700

Pro Cys Pro Glu Thr Val Gln Arg Lys Lys Cys Arg Ala Arg Lys Cys
705                 710                 715                 720

Leu Arg Ser Pro Ser Ile Gln Lys Leu Arg Trp Arg Glu Ala Arg Glu
                725                 730                 735

Ser Arg Arg Ser Glu Gln Leu Arg Glu Glu Ser Asp Gly Glu Gln Phe
            740                 745                 750

Pro Gly Cys Arg Met Arg Pro Trp Thr Ala Trp Ser Glu Cys Thr Lys
        755                 760                 765

Leu Cys Gly Gly Gly Ile Gln Glu Arg Tyr Met Thr Val Lys Lys Arg
    770                 775                 780

Phe Lys Ser Ser Gln Phe Thr Ser Cys Lys Asp Lys Lys Glu Ile Arg
785                 790                 795                 800

Ala Cys Asn Val His Pro Cys
                805


<210> 134
<211> 4029
<212> DNA
<213> Rattus norvegicus

<220>
<223> F-spondin

<400> 134
ccctccctct tcgcgctcct tcgccaccgc ccgcccctca gctccgctgc tcggctccgc 60
tcagagcagc gcagctccgc agccaaagcg aggcgggctc gggctcccca ccgccagtgc 120
cacccgggct cctccagctt tcgcctctgc agctcccgtc acttggagta aaagtgtcct 180
gacaggggtc tgcaacatca gcagaaagtt gggaggtcct cgagaatgag gctatctccc 240
gcgcccctga ggcttagccg gggtccggcg ctgctggccc tggcgctgcc cctggccgca 300
gcgctcgctt tctcggatga gaccctggac aaagtggcca agtcggaggg ctactgcagc 360
cgcatcttgc gcgcccaggg cacacggcgt gagggataca cagagttcag cctccgcgtg 420
gaaggcgacc ctgacttcta taagccagga agcagctacc gagtgacact ctcggctgcc 480
cctccctcct acttcagagg cttcacgtta attgctctca aagagaaccg cgaaggcgat 540
aaggaagaag accacgcggg caccttccag atcatagatg aagaagaaac ccagtttatg 600
agtaactgtc ctgtggcagt cactgaaagc acccctcgga ggaggacacg gatccaggtg 660
ttttggatag cgccacccac agggacaggc tgtgtgattc tgaaggccag cattgtacag 720
aaacgcatta tctattttca agacgagggc tccctgacca agaagctgtg tgaacaggat 780

cccacacttg atggagtgac ggacagaccg atcttagact gctgcgcctg cggaactgcc 840
aagtacagac tcacgtttta tgggaactgg tcggagaaga ctcatccaaa ggattaccct 900
cgtcgggcta atcactggtc tgccatcatt ggcggatccc actccaagaa ctacgtgctg 960
tgggagtacg gagggtatgc cagtgaaggg gtcaagcaag ttgctgaact tggctcacca 1020
gtaaaaatgg aggaagaaat tcgacaacag agtgatgaag tcctcactgt catcaaagcc 1080
aaagcccagt ggccatcctg gcagcctgtc aatgtgagag cagcaccctc agccgaattc 1140
tcagtggaca ggacacgcca cttgatgtcc ttcctaacca tgatgggccc cagtcctgac 1200
tggaacgtgg gcctatctgc agaggatctg tgcaccaagg agtgtggctg ggtccagaaa 1260
gtggtgcagg acctaattcc ctgggatgct ggcacggaca gcggggtgac ctacgagtca 1320
ccaaacaagc ccacaattcc tcaggaaaaa atccgacccc tgactagtct ggaccatcct 1380
cagagtcctt tctatgaccc ggaaggtggg tccatcacac aagtggccag agtcgtcatc 1440
gagagaattg cccggaaggg agaacaatgc aacattgtac ctgacaatgt ggatgatatt 1500
gtagccgacc tggctccaga agagaaagat gaagatgaca cccctgaaac ctgcatctac 1560
tccaactggt ccccatggtc ggcctgcagc tcttccactt gtgaaaaggg taagaggatg 1620
cggcaacgca tgctgaaggc acagctggac ctcagtgtcc cctgtcctga cacccaggac 1680
ttccagccct gcatgggccc cggctgcagc gatgaagatg gctccacctg taccatgtcg 1740
gagtggatca cctggtcacc ctgcagtgtc tcgtgtggca tgggtatgag gtcccgggag 1800
aggtacgtga agcagttccc ggaagacggc tcggtgtgca tgctgcccac ggaagagaca 1860
gagaagtgca cggtcaacga ggagtgctct cctagcagct gcctggtgac tgagtggggt 1920
gagtgggatg actgcagcgc cacctgtgga atgggcatga agaagcggca ccgcatggtc 1980
aagatgagcc ccgcggacgg ctccatgtgc aaggcggaga cttcgcaggc ggagaaatgc 2040
atgatgcctg agtgccatac catcccgtgc ttgctgtctc cttggtccga gtggagcgac 2100
tgtagcgtga cctgtgggaa gggcatgcgg acgcgccagc ggatgctcaa gtctctggca 2160
gagctggggg actgtaatga ggatctggag caggcggaga agtgtatgct gccagagtgc 2220
cccattgact gcgaactcag tgagtggtcc cagtggtctg aatgtaacaa gtcctgtggg 2280
aaaggtcaca tgattcgaac ccggacaatc caaatggaac ctcagtttgg aggtgcaccc 2340
tgcccagaga ctgtgcaacg caagaagtgc cgtgcccgga aatgccttcg cagcccatcg 2400
atccagaagc tgcgctggag ggaggcccga gagagcagga ggagtgagca gctgagggaa 2460
gagtcagatg gagagcagtt cccaggctgt cggatgcgcc cgtggacagc ctggtcagag 2520
tgcaccaaac tgtgcggagg tgggatccaa gaacgctaca tgactgtgaa gaagaggttc 2580
aaaagctccc agtttaccag ctgcaaagac aagaaggaga tcagagcgtg caacgtgcac 2640
ccttgttagt aggggttcaa ctccccaggg ctgcattcca gattctagtc accaatggtt 2700
gggtggtgta tttgcttgtt taagatgatt taaattgtgt ccacatgttt tcatttttac 2760
cggtgtggtt tgcccaatag tcttatggag gccgagggac atcttgtctg aatacttctt 2820
ggtgagtaca ggccaagcgg ggcatcttgt ccccaggcgc catcttcctg cactgagttg 2880
agtagtgttg gttcaccttg gtactaaact gaatcgtgtc cctctggagc atcccctggt 2940
caagcagggt ggagactttg gccatccaca aggagaagca accaggatgc agcatgcggg 3000
agacacagcc attaattgca aaggacagat cctcctctct cacctttggc ctgctcactc 3060
ttacagaaac ctgtttgtcc gcctcctttt ttatttagca caactccagg catcttggta 3120
agtctccagg gtcatgggtt cttcggtgcc ctgaaggaga agccctgagg tgaggtggca 3180
tttgttacaa acctcccaat actgctttac tggcatcaca aggtcagcag gtgatgatgg 3240
ctacttcatt tcattgtgag ccgtgatttc cgttgagttt tgattgttgg tgccataaat 3300
gtcctaggat gctggacgga cacatcagcc ttgtcagcag atccttcttt gagccaatgt 3360
agacagtaag ctgggcactg gttccaaagc caacttaaaa tcttcctaca catatccaga 3420
ccttttttta ggttgcccaa acttccttag aataaagcat tttagctctg agaactactt 3480
gataagtctg ccaggaagcc cccaagtcaa ttcttcaaca aaaatactat cttccctact 3540
taattttttt taagtcatga tattttatag ttagaggaga gagagacaat ctattcccat 3600
gactaagaca caaacctaca agaaagggtt actcagtcaa gcctgtgcct gacttctgga 3660
ccaggcccct gattttcatg gatagtccaa aggaaggcca ggggttccca ctgactccaa 3720
gccatcagca gcacccaaac ccaggagcaa caaatattca gagaaagagg atgtttatct 3780
cagctatgag ctcattggca ggttgtactc atgcatctgt taaaagcacc accacatcct 3840
tttgcaagtc tgtttattac cgcttcatcc aaatacattt tgtggtcaag atcgacacag 3900
tgctatgaat acagtacttt aaggtctgca ttaaacacat cagaatattt cctgccacat 3960
ctatgtacaa cccctgaata tgtatttttc cttaacacaa gagagcctgt tcaattaaaa 4020
aaaaaaaaa 4029

<210> 135
<211> 11
<212> PRT
<213> Rattus norvegicus

<220>
<223> NF 68

<400> 135
Met Ser Ser Phe Ser Tyr Glu Pro Tyr Phe Ser
1               5                   10


<210> 136
<211> 2322
<212> DNA
<213> Rattus norvegicus

<220>
<223> NF 68

<400> 136
ccccctgcac cactcccttg tgggtagtct gctctgcagt caaaagtatt atcgaggagc 60
cctccctgcc ccaaaggaat cacacacaca cacacacaca cacacacaca cacacacaca 120
cacacacaca cacgagagag agagagagag gagagagaga gagagagaga gagaggctta 180
agatctctga gaatgacctg taacttccga ggaaacgtgc tggcagaatg gaagcgaaca 240
atgtccaata gaggtgtgtg cagaccctct acaaaagatt gtgaaataat tcaggttaca 300
tggagcgagc ttgggggaaa caattgcaac tcttcgtcta tatgagtaat tggaaggaag 360
aaaagctatg acgtatctga gaccttctct ctgttcctcc cccttgagtc ctcttgatca 420
ctgatgaatg agaaaaagat gcaggttaca gagagacatc ttaataaatg cttggctctg 480
tatgccagcc acacactcag gcaacctggg tgattaggtt tgaaagctta gattgtataa 540
acatgggtgg gcctctaata tggcttagac ttaacagctt taagccatca gcaacctcac 600
agaagtcctc tgcttcagcc cattcctctc ccgtgaaggg acaagttaat cagaatcaca 660
gtgtgtgtaa cacgagaaag gagtatgcag aataaagctt ccttcacact ctttttggat 720
acagctccaa aaatttgcat tactttttaa tttgaaagcc gttacctctc actgtgccat 780
ttaaaggaac cagtcagcca gtgttatgtt gaacaaatta aacaaaacgc caactgcttg 840
gttaaaggtt ttatttattt atttatttat ttatttattt atttatttat ttatacaaat 900
ctctgcaata tctcttcgaa accgcaggca accacaacgg cagtatgaaa acgttccacc 960
catgtgcgtt ttcgtaccca ctctgctacc tgggtatcct gaaggaagtt ggtagcttcc 1020
taagggcacc cgttacctgt gaatttcagc aggctgtttc agcaggctcc acgctgagtt 1080
ctcttccgga agaccacacc cagagctggt agcactttgc agcattctct ctctctctct 1140
ctctctctct ctctctctct ctctctctct ccctccctca ctccctcact cccttcctcc 1200
ctctctcctt ccctccctct ctctccatcc aacctacatg gcttgtcaaa accatcagat 1260
taggctacca cacaggtgtt aaaaagggga agaggctttc gatgcggtgg aggtagcata 1320
caggcaccct gaagttctcc cgcagcagag cgcatctttt gccttgcaaa tctttgcctg 1380
gttagtttca acccgtccta agcattccag acttcatttc ccataaagaa aggggaaatt 1440
ctatggaagg cgccagacaa attagctgct gtaaaggttt taaaatgtca aaatgaacac 1500
ccgctatcct gcggttccaa gggtgtgtgt agcggtaccc gtcccctatg tattgagaag 1560
cagattatac ggaaagaaat aaaaaagaaa aaataaaaga aggcaggaga aaggggggtg 1620
aattcaagaa aatgtcagaa aatagctaca aaggcatgca aaacttttcc tgcaggaatg 1680
tcgcaagggc gaaaccttaa actattgtca gtaacctaac caccctggcc ttacaaccag 1740
gctccttaaa taagaatcac ggtgcttgag agggtccctg aggcagaaag ggcgagccag 1800
gggcggactg gggtggggtg agggtgtctt tgtgacatta gatgttaaaa gttgatgtgt 1860
cagtagggac ctattatttg gaaattttaa gggtcctagt gaggcgggtt ttaggtattg 1920
acaggcagga tgggtcgggg gagtaggaaa ggaatgagca gggaagttat gggggtttca 1980
gagaaagtag ggggcctcag agaaaagcgg ccaggcggga gccgcagtcc gctagcctcc 2040
tgtcgctcgc ccccgccttt gctcttgcgc agaatcctcc ccttggctgc agcagcacgc 2100
tgcccccact ggcctgcgca acgcgatcga tcacagtctg cgtcagagtc ccggcgtata 2160
aatagaggtg gcaggacagc ccagagccgc acacagccat ccatccttcc ctttccctct 2220
tctccccggt tcttctctct aggtccccta tctccgcccc gggcctgagg ggcgcaccga 2280
ccgccaccat gagttcgttc agctacgagc cgtacttttc ga                    2322


<210> 137
<211> 824

<212> DNA
<213> Mus musculus

<220>
<223> E800

<400> 137
gaattccggg agcccttgat tgacaaggaa tattaccgca agccggtggc gggaactcac 60
cgaagaggag aagtatgacc aggagctcaa gaaaacccag tttatcaaag ctgcggccgc 120
gactgaaacc agctcggtgt tcgcagaccc ggtcatcagc aaattcacca acatgacgat 180
gaagggaggc aacaaagttc tggcccggtc tctcatggct cagactctgg aagctgtgaa 240
aaggaagcag tttgaaaagt accgtgctgc ctctgcggag ggaacaggcc accatttgaa 300
cggaacccct accaggatct tccacgaggg cactcaaaaa ctgtgtagcc tgtggattgg 360
gttggtgccg atcctcaaag ggggccattt ctaccaggtc cccgtgcctc tcggtgtacc 420
gacggtcggc gcttcttggg catgaagtgg gatgatcaca gagtgccgag agaataaacg 480
gcggcggaca attatgccag agggaacttt tcaaatgagc tgctggaggc ttttcataac 540
cgaggtcccg tgatcaagag gaagcacaat atgcataaga tggcagaggc caaccgtgcc 600
ctggcccact accgctggtg gtagcaggag aggagaggtg ggaaaggaca cactccctcg 660
agaaaccacc cgacggctgg agaggtggct cagtggttaa agccactcac tgctctttcc 720
acaggacctg gtttgattcc cagcactcac acggaggctc gctgaccacc tgtaactcct 780
gttccaaagg tttcgatacc ttctgacctc tgggcggaat tcgc 824


<210> 138
<211> 286
<212> PRT
<213> Homo sapiens

<220>
<223> Human capping protein alpha subunit isoform 1

<400> 138
Met Ala Asp Phe Asp Asp Arg Val Ser Asp Glu Glu Lys Val Arg Ile
1               5                   10                  15

Ala Ala Lys Phe Ile Thr His Ala Pro Pro Gly Glu Phe Asn Glu Val
            20                  25                  30

Phe Asn Asp Val Arg Leu Leu Leu Asn Asn Asp Asn Leu Leu Arg Glu
        35                  40                  45

Gly Ala Ala His Ala Phe Ala Gln Tyr Asn Met Asp Gln Phe Thr Pro
    50                  55                  60

Val Lys Ile Glu Gly Tyr Glu Asp Gln Val Leu Ile Thr Glu His Gly
65                  70                  75                  80

Asp Leu Gly Asn Ser Arg Phe Leu Asp Pro Arg Asn Lys Ile Ser Phe
                85                  90                  95

Lys Phe Asp His Leu Arg Lys Glu Ala Ser Asp Pro Gln Pro Glu Glu
            100                 105                 110

Ala Asp Gly Gly Leu Lys Ser Trp Arg Glu Ser Cys Asp Ser Ala Leu
        115                 120                 125

Arg Ala Tyr Val Lys Asp His Tyr Ser Asn Gly Phe Cys Thr Val Tyr
    130                 135                 140

Ala Lys Thr Ile Asp Gly Gln Gln Thr Ile Ile Ala Cys Ile Glu Ser
145                 150                 155                 160

His Gln Phe Gln Pro Lys Asn Phe Trp Asn Gly Arg Trp Arg Ser Glu
                165                 170                 175

Trp Lys Phe Thr Ile Thr Pro Pro Thr Ala Gln Val Val Gly Val Leu
            180                 185                 190

Lys Ile Gln Val His Tyr Tyr Glu Asp Gly Asn Val Gln Leu Val Ser
        195                 200                 205

His Lys Asp Val Gln Asp Ser Leu Thr Val Ser Asn Glu Ala Gln Thr
    210                 215                 220

Ala Lys Glu Phe Ile Lys Ile Ile Glu Asn Ala Glu Asn Glu Tyr Gln
225                 230                 235                 240

Thr Ala Ile Ser Glu Asn Tyr Gln Thr Met Ser Asp Thr Thr Phe Lys
                245                 250                 255

Ala Leu Arg Arg Gln Leu Pro Val Thr Arg Thr Lys Ile Asp Trp Asn
            260                 265                 270

Lys Ile Leu Ser Tyr Lys Ile Gly Lys Glu Met Gln Asn Ala
        275                 280                 285

<210> 139
<211> 2385
<212> DNA
<213> Homo sapiens

<220>
<223> Human capping protein alpha subunit isoform 1

<400> 139
atggccgact tcgatgatcg tgtgtcggat gaggagaagg tacgcatagc tgctaaattc 60
atcactcatg cacccccagg ggaatttaat gaagtattca atgacgttcg gctactactt 120
aataatgaca atctcctcag ggaaggggca gcacatgcat ttgcccagta taacatggat 180
cagttcacgc ctgtgaagat agaaggatat gaagatcagg tcttaattac agagcacggt 240
gacctgggta atagcagatt tttagatcca agaaacaaaa tttcctttaa atttgaccac 300
ttacggaaag aagcaagtga cccccagcca gaagaagcag atggaggtct gaagtcttgg 360
agagaatcct gtgacagtgc tttaagagcc tatgtgaaag accattattc caacggcttc 420
tgtactgttt atgctaaaac tatcgatggg caacagacta ttattgcatg tattgaaagc 480
caccagtttc agcctaaaaa cttctggaat ggtcgttgga gatcagagtg gaagttcacc 540
atcacaccac ctacagccca ggtggttggc gtgcttaaga ttcaggttca ctattatgaa 600
gatggcaatg ttcagttggt tagtcataaa gatgtacagg attcactaac tgtttcgaat 660
gaagcccaaa ctgccaagga gtttattaaa atcatagaga atgcagaaaa tgagtatcag 720
acagcaatta gtgaaaacta tcaaacaatg tcagatacca cattcaaggc cttgcgccgc 780
cagcttccag ttacccgcac caaaatcgac tggaacaaga tactcagcta caagattggc 840
aaagaaatgc agaatgctta aaggctgaat gtaggattct tcagtatgtg gaaagacaag 900
gattcaacgt gtggtcatat gataaataag tgatttataa acaagagtga tattttgcta 960
gggctttcaa agttaaccgg ttttctagcc tcatggaata ctgttgaacc tatagcgttg 1020
tcttgattct tttgtgttct ctgccttgta attttctgtt actgctatat ctacgtgtaa 1080
atcttttttt cttttttttt tttttttttt ttcttttttg gttaattctg ccacatttaa 1140
tgttggtgag agagtgatct atcctaatga catttactgt ttaaaaaagt ttcctagcca 1200
tgaagccctg ctactgattt agacaaggta ttatggtcat tactttgtac ccctatcctt 1260
ccaagcactt ctggtacttc agtcgttttt actgatccac caacacctaa agaggctatg 1320
ctacagtctc tagctaaatg gaagacacat tcatccttct ccctctgact gctttgatca 1380
tcatttattg catcgtcata tcatatttat cgcatctcat aactaacttt ctaaagtttg 1440
gattgggact tttcaggtcc tttttggagg gcaaaggaag ttccagcttc tctggggaac 1500
ttgtttttaa atccaaagac ttgaaccaca ttccctgcac atgaacatgt ttgcttttat 1560

cccttctctc attggctcct tcccatctta gtaccattgt agttatacat ctgcattttt 1620
tagaagcatt ttacccattt atttttttaa acattcaaga actgctgacg tactgtggat 1680
gtagagtata aaacttgaaa aatgcagatg ttgaaggaat aataggtatc ttgtgcttta 1740
atactttatg gcaggattgt actataagca aatgaattaa acagctatgt aaatcataaa 1800
gaaaaactaa aaatgaacca aagtgaaagg ataacttcca ggcagtatct ttctattgta 1860
acctgttatt taaggaaata ctagtgattt cttctaaata ggatgtaaac ttctttcaaa 1920
ttactcttcc tcagtctgcc tgccaagaac tcaagtgtaa ctgtgataaa ataacctttc 1980
ccaggtatat tcggcaggta tgtgtgtaat ctcagaatac acaggtgaca tagatatgat 2040
atgacaactg gtaatggtgg attcatttac attgtttaca cttctatgac caggccttaa 2100
gggaaggtca gttttttaaa aaaccaagta gtgtcttcct acctatctcc agatacatgt 2160
caaaaagaaa aggtgtttgt gctccgtttt gtttctgctc agtaatatag tcaagcaagt 2220
ttgttccagg tgacccattg agctgtgtat gcatttttgt ttatttcaat aaaatatatt 2280
tgtattattt gtccttcata ctatccatcc ataccacact atcttctgta tcaggtagtc 2340
taatagaaat atacctgttt tgttctaaaa aaaaaaaaaa aaaaa 2385

<210> 140
<211> 1095
<212> PRT
<213> Homo sapiens

<220>
<223> KIAA0456 Protein

<400> 140
Arg Thr Arg Gly Leu Asp Phe Phe Arg Gly Ser Ile Asn Ser Gln Phe
1               5                   10                  15

Glu Phe Gly Arg Lys Lys Glu Asn Met Thr Ser Pro Ala Lys Phe Lys
            20                  25                  30

Lys Asp Lys Glu Ile Ile Ala Glu Tyr Asp Thr Gln Val Lys Glu Ile
        35                  40                  45

Arg Ala Gln Leu Thr Glu Gln Met Lys Cys Leu Asp Gln Gln Cys Glu
    50                  55                  60

Leu Arg Val Gln Leu Leu Gln Asp Leu Gln Asp Phe Phe Arg Lys Lys
65                  70                  75                  80

Ala Glu Ile Glu Met Asp Tyr Ser Arg Asn Leu Glu Lys Leu Ala Glu
                85                  90                  95

Arg Phe Leu Ala Lys Thr Arg Ser Thr Lys Asp Gln Gln Phe Lys Lys
            100                 105                 110

Asp Gln Asn Val Leu Ser Pro Val Asn Cys Trp Asn Leu Leu Leu Asn
        115                 120                 125

Gln Val Lys Arg Glu Ser Arg Asp His Thr Thr Leu Ser Asp Ile Tyr
    130                 135                 140

Leu Asn Asn Ile Ile Pro Arg Phe Val Gln Val Ser Glu Asp Ser Gly
145                 150                 155                 160

Arg Leu Phe Lys Lys Ser Lys Glu Val Gly Gln Gln Leu Gln Asp Asp
                165                 170                 175

Leu Met Lys Val Leu Asn Glu Leu Tyr Ser Val Met Lys Thr Tyr His
            180                 185                 190

Met Tyr Asn Ala Asp Ser Ile Ser Ala Gln Ser Lys Leu Lys Glu Ala
        195                 200                 205

Glu Lys Gln Glu Glu Lys Gln Ile Gly Lys Ser Val Lys Gln Glu Asp
    210                 215                 220

Arg Gln Thr Pro Arg Ser Pro Asp Ser Thr Ala Asn Val Arg Ile Glu
225                 230                 235                 240

Glu Lys His Val Arg Arg Ser Ser Val Lys Lys Ile Glu Lys Met Lys
                245                 250                 255

Glu Lys Arg Gln Ala Lys Tyr Thr Glu Asn Lys Leu Lys Ala Ile Lys
            260                 265                 270

Ala Arg Asn Glu Tyr Leu Leu Ala Leu Glu Ala Thr Asn Ala Ser Val
        275                 280                 285

Phe Lys Tyr Tyr Ile His Asp Leu Ser Asp Leu Ile Asp Gln Cys Cys
    290                 295                 300

Asp Leu Gly Tyr His Ala Ser Leu Asn Arg Ala Leu Arg Thr Phe Leu
305                 310                 315                 320

Ser Ala Glu Leu Asn Leu Glu Gln Ser Lys His Glu Gly Leu Asp Ala
                325                 330                 335

Ile Glu Asn Ala Val Glu Asn Leu Asp Ala Thr Ser Asp Lys Gln Arg
            340                 345                 350

Leu Met Glu Met Tyr Asn Asn Val Phe Cys Pro Pro Met Lys Phe Glu
        355                 360                 365

Phe Gln Pro His Met Gly Asp Met Ala Ser Gln Leu Cys Ala Gln Gln
    370                 375                 380

Pro Val Gln Ser Glu Leu Val Gln Arg Cys Gln Gln Leu Gln Ser Arg
385                 390                 395                 400

Leu Ser Thr Leu Lys Ile Glu Asn Glu Glu Val Lys Lys Thr Met Glu
                405                 410                 415

Ala Thr Leu Gln Thr Ile Gln Asp Ile Val Thr Val Glu Asp Phe Asp
            420                 425                 430

Val Ser Asp Cys Phe Gln Tyr Ser Asn Ser Met Glu Ser Val Lys Ser
        435                 440                 445

Thr Val Ser Glu Thr Phe Met Ser Lys Pro Ser Ile Ala Lys Arg Arg
    450                 455                 460

Ala Asn Gln Gln Glu Thr Glu Gln Phe Tyr Phe Thr Lys Met Lys Glu
465                 470                 475                 480

Tyr Leu Glu Gly Arg Asn Leu Ile Thr Lys Leu Gln Ala Lys His Asp
                485                 490                 495

Leu Leu Gln Lys Thr Leu Gly Glu Ser Gln Arg Thr Asp Cys Ser Leu
            500                 505                 510

Ala Arg Arg Ser Ser Thr Val Arg Lys Gln Asp Ser Ser Gln Ala Ile

515                 520                 525

Pro Leu Val Val Glu Ser Cys Ile Arg Phe Ile Ser Arg His Gly Leu
    530                 535                 540

Gln His Glu Gly Ile Phe Arg Val Ser Gly Ser Gln Val Glu Val Asn
545                 550                 555                 560

Asp Ile Lys Asn Ala Phe Glu Arg Gly Glu Asp Pro Leu Ala Gly Asp
                565                 570                 575

Gln Asn Asp His Asp Met Asp Ser Ile Ala Gly Val Leu Lys Leu Tyr
            580                 585                 590

Phe Arg Gly Leu Glu His Pro Leu Phe Pro Lys Asp Ile Phe His Asp
        595                 600                 605

Leu Met Ala Cys Val Thr Met Asp Asn Leu Gln Glu Arg Ala Leu His
    610                 615                 620

Ile Arg Lys Val Leu Leu Val Leu Pro Lys Thr Thr Leu Ile Ile Met
625                 630                 635                 640

Arg Tyr Leu Phe Ala Phe Leu Asn His Leu Ser Gln Phe Ser Glu Glu
                645                 650                 655

Asn Met Met Asp Pro Tyr Asn Leu Ala Ile Cys Phe Gly Pro Ser Leu
            660                 665                 670

Met Ser Val Pro Glu Gly His Asp Gln Val Ser Cys Gln Ala His Val
        675                 680                 685

Asn Glu Leu Ile Lys Thr Ile Ile Ile Gln His Glu Asn Ile Phe Pro
    690                 695                 700

Ser Pro Arg Glu Leu Glu Gly Pro Val Tyr Ser Arg Gly Gly Ser Met
705                 710                 715                 720

Glu Asp Tyr Cys Asp Ser Pro His Gly Glu Thr Thr Pro Val Glu Asp
                725                 730                 735

Ser Thr Gln Asp Val Thr Ala Glu His His Thr Ser Asp Asp Glu Cys
            740                 745                 750

Glu Pro Ile Glu Ala Ile Ala Lys Phe Asp Tyr Val Gly Arg Thr Ala
        755                 760                 765

Arg Glu Leu Ser Phe Lys Lys Gly Ala Ser Leu Leu Leu Tyr Gln Arg
    770                 775                 780

Ala Ser Asp Asp Trp Trp Glu Gly Arg His Asn Gly Ile Asp Gly Leu
785                 790                 795                 800

Ile Pro His Gln Tyr Ile Val Val Gln Asp Thr Glu Asp Gly Val Val
                805                 810                 815

Glu Arg Ser Ser Pro Lys Ser Glu Ile Glu Val Ile Ser Glu Pro Pro
            820                 825                 830

Glu Glu Lys Val Thr Ala Arg Ala Gly Ala Ser Cys Pro Ser Gly Gly
        835                 840                 845

His Val Ala Asp Ile Tyr Leu Ala Asn Ile Asn Lys Gln Arg Lys Arg
    850                 855                 860

Pro Glu Ser Gly Ser Ile Arg Lys Thr Phe Arg Ser Asp Ser His Gly
865                 870                 875                 880

Leu Ser Ser Ser Leu Thr Asp Ser Ser Ser Pro Gly Val Gly Ala Ser
                885                 890                 895

Cys Arg Pro Ser Ser Gln Pro Ile Met Ser Gln Ser Leu Pro Lys Glu
            900                 905                 910

Gly Pro Asp Lys Cys Ser Ile Ser Gly His Gly Ser Leu Asn Ser Ile
        915                 920                 925

Ser Arg His Ser Ser Leu Lys Asn Arg Leu Asp Ser Pro Gln Ile Arg
    930                 935                 940

Lys Thr Ala Thr Ala Gly Arg Ser Lys Ser Phe Asn Asn His Arg Pro
945                 950                 955                 960

Met Asp Pro Glu Val Ile Ala Gln Asp Ile Glu Ala Thr Met Asn Ser
                965                 970                 975

Ala Leu Asn Glu Leu Arg Glu Leu Glu Arg Gln Ser Ser Val Lys His
            980                 985                 990

Thr Pro Asp Val Val Leu Asp Thr Leu Glu Pro Leu Lys Thr Ser Pro
        995                 1000                1005

Val Val Ala Pro Thr Ser Glu Pro Ser Ser Pro Leu His Thr Gln Leu
   1010                1015                1020

Leu Lys Asp Pro Glu Pro Ala Phe Gln Arg Ser Ala Ser Thr Ala Gly
1025                1030                1035                1040

Asp Ile Ala Cys Ala Phe Arg Pro Val Lys Ser Val Lys Met Ala Ala
                1045                1050                1055

Pro Val Lys Pro Pro Ala Thr Arg Pro Lys Pro Thr Val Phe Pro Lys
            1060                1065                1070

Thr Asn Ala Thr Ser Pro Gly Val Asn Ser Ser Thr Ser Pro Gln Ser
        1075                1080                1085

Thr Asp Lys Ser Cys Thr Val
   1090                1095


<210> 141
<211> 6305
<212> DNA
<213> Homo sapiens

<220>
<223> KIAA0456 Protein

<400> 141
cggacgcgtg ggttggattt tttccgtgga tctatcaatt cacaattcga atttggaaga 60
aagaaggaaa acatgacgtc tccagccaaa ttcaaaaagg ataaggagat catagcagag 120

tacgatactc aggtcaaaga gatccgtgct cagctcacag agcagatgaa atgcctggac 180
cagcagtgtg agcttcgggt gcaactgttg caggacctcc aggacttctt ccgaaagaag 240
gcagagattg agatggacta ctcccgcaac ctggagaagc tggcagaacg cttcctggcc 300
aagacacgca gcaccaagga ccagcaattc aagaaggatc agaatgttct ctctccagtc 360
aactgctgga atctcctctt aaaccaggtg aagcgggaaa gcagggacca taccaccctg 420
agtgacatct acctgaataa tatcattcct cgatttgtac aagtcagcga ggactcagga 480
agactcttta aaaagagtaa agaagtcggc cagcagctcc aagatgattt gatgaaggtc 540
ctgaacgagc tctactcggt gatgaagaca tatcacatgt acaatgccga cagcatcagt 600
gctcagagca aactaaagga ggcggagaag caggaggaga agcaaattgg taaatcggta 660
aagcaggagg accggcagac cccacgctcc cctgactcca cggccaacgt tcgcattgag 720
gagaaacatg tccggaggag ctcagtgaag aagattgaga agatgaagga gaagcgtcaa 780
gccaagtaca cggagaataa gctgaaggcc atcaaagccc ggaatgagta cttgctggct 840
ttggaggcaa ccaatgcatc tgtcttcaag tactacatcc atgacctatc tgaccttatt 900
gatcagtgtt gtgacttagg ctaccatgca agtctgaacc gggctctacg caccttcctc 960
tctgctgagt taaacctgga acagtcgaag catgagggtc tggatgccat cgagaatgca 1020
gtagaaaacc tggatgccac cagtgacaag cagcgcctca tggagatgta caacaacgtc 1080
ttctgccccc ctatgaagtt tgagtttcag ccccacatgg gggatatggc ttcccagctc 1140
tgtgcccagc agcctgtcca gagtgagctg gtacagagat gccaacaact gcagtctcgc 1200
ttatccactc taaagattga aaacgaagag gtaaagaaga caatggaggc caccctgcaa 1260
accatccagg acattgtgac tgtcgaggac tttgatgtgt ctgactgctt ccagtacagc 1320
aactccatgg agtccgtcaa gtccacggtc tctgaaacct tcatgagcaa gcccagcatt 1380
gctaagagga gagccaacca gcaagagaca gagcagtttt atttcacaaa aatgaaagag 1440
tacctggagg gcaggaacct catcaccaag ttacaagcca agcatgacct tctgcagaaa 1500
accctgggag aaagtcagcg gacagattgc agtctagcca ggcgcagctc aactgtgagg 1560
aaacaggact ccagccaggc aattcctctg gtggtggaaa gctgtatccg gtttatcagc 1620
agacacggac tacagcatga aggaattttc cgggtgtcag gatcccaggt ggaagtgaat 1680
gacatcaaaa atgcctttga gagaggagag gaccccctgg ctggggacca gaacgaccat 1740
gacatggatt ccatagctgg tgtcctgaag ctttacttcc gggggctgga acaccctctc 1800
ttccccaagg acatctttca tgacctgatg gcctgcgtca caatggacaa cctgcaggag 1860
agagctctgc acatccggaa agtcctccta gtcctgccca aaaccactct gattatcatg 1920
agatacctct ttgccttcct caatcattta tcacagttca gtgaagagaa catgatggac 1980
ccctacaacc tcgccatctg cttcgggccc tcgctaatgt cagtgccaga gggccacgac 2040
caggtgtcct gccaagccca cgtgaatgag ctgatcaaaa ccatcatcat ccagcatgag 2100
aacatcttcc caagccccag ggagctggag ggccctgtct acagcagagg aggaagcatg 2160
gaggattact gtgatagccc tcatggagag actaccccgg ttgaagactc aacccaggat 2220
gtgaccgcag agcaccacac gagcgatgac gaatgtgagc ccatcgaggc cattgccaag 2280
tttgactacg tgggccggac agcccgagag ctgtccttta agaagggagc atccctgctg 2340
ctttaccagc gggcttccga cgactggtgg gaaggccggc acaatggcat cgacggactc 2400
atcccccatc agtacatcgt ggtccaagac accgaggacg gtgtcgtgga gaggtccagc 2460
cccaagtctg agattgaggt catttctgag ccacctgaag aaaaggtgac agccagagcg 2520
ggggccagct gtcccagtgg gggtcatgta gccgatattt atcttgcaaa catcaacaag 2580
caaaggaagc gtccagaatc tgggagcatc cggaaaactt ttcggagtga cagccatggg 2640
ctgagcagtt ccctgactga ctcctcctcc ccaggggtgg gggctagctg ccgcccatcc 2700
tcccagccca tcatgagcca gagcctcccc aaagaagggc cagataagtg ttccatcagt 2760
gggcacggga gcctcaactc catcagccgc cactcatccc tgaagaatcg gctggatagt 2820
ccacagatcc ggaagactgc cacagcggga aggtcaaaaa gcttcaataa ccatcggccc 2880
atggaccctg aggtcattgc tcaggatatt gaggcaacaa tgaactcggc cctgaatgag 2940
ctacgggaac tagaacggca gagcagtgtc aaacacaccc ctgacgtggt tctggacacc 3000
ttggagcccc tcaaaacctc cccagtggtg gcccccacgt cagagccctc cagccctctg 3060
cacacccagc tcctcaagga ccccgagccc gccttccagc gcagcgccag tactgctggg 3120
gacatcgcct gcgccttccg gcctgtcaag tctgtcaaga tggctgcccc ggtcaaacca 3180
ccagccacac ggcccaagcc cactgtcttc cccaaaacaa atgccactag ccctggtgtc 3240
aactcatcaa cttccccaca gtctactgac aagtcttgta ctgtctgagg gataataatt 3300
taattgttct agacaagggg actataggga ctgactgtta ttaaaatctt cctatttaac 3360
tagcttgggg acttcagttg aaaattaggt tctaagttgt tcttgcagga attagcctcc 3420
ccgtctccca aaaccttgag aatgaagccc ttggtatcgc ctctcccttc ccactgccct 3480
ctgcttcccc cagtcgtcgt aattcagcca gctgcagtcc gtaccgttct taggttagcc 3540
agagacaggt tttcattatc aggtcactgt gaaatctggt aaggcagtcc tgaggacatg 3600
ggctcaagtc tcagtcccct cagaccacgg tgatgccttg accagatggt tggctactgc 3660
catccagctt tcagtggcat cttgttttgg gaactgatta tagagaatca tatatagtcc 3720
agtcttcatt ttacatacac acacatattt tacacacaca cacattttac acacacacac 3780

acacacacac acacacacat atgtttttct agtctctggc atgtgtagcc tctcctggtc 3840
atagaccagt ctctttgtaa gttattgtgg cagttcacac agtagccacc aggggtctct 3900
gtttccatta caaactttgt tctgtctggg ccagagaacc tagcctttga aatctctcca 3960
tcatgtgaaa cataacggga tgggacaatc ccgtaacctg tttggggttg ggggctttct 4020
ctctgtgttc tttccattga tgtgaattgg tcattggtgt ttgctcttgc ctctcctcca 4080
tcccctagaa gtacaccccc gtcttattaa ggagctttta aagtttttct gaatgtatag 4140
acatttctcg ggttcctacc tttgtctctg atggaccatt ttccatttaa gacattttcc 4200
tgtataagac agttttatag ctggttcctt ttagggtaaa gagtcttaag agagttttat 4260
tgtgtctatg gcaggtttgg gaaaggtaag aaatgggtcc tttttcctcc taatgttttt 4320
ggcacttaaa acataaaatt cattatccta ttaaaaaatt aaattcagct ttgctaatcc 4380
agaaattgtt cccaaatgaa aacttgtttt aagtccaccc cttagtttcc ttattttaca 4440
aggtctctct tcagggacca acaggggctt agagagcctt agttagatta aagggagacc 4500
ctacctctta aaaccagttt tcatttatgc aaacaaggac aattaaggga accctgaccc 4560
cacaggctct caagtcttcc caaggccaga atcgaaagaa aattaaaatt tgaatgctga 4620
atattctggc tctactctgg cctttttctt ggttcccttc caaaatgcac aaatcatacc 4680
cttgtctgct ccaattcagt ctccaaacct ggtgcctgtg ctcctggccc ccctagcatc 4740
atgctatccc aggagtatca ggaccagaca catccacagc caggctcatg ggtctcagac 4800
agcaacttga gttaaagctg aaactcatcc ttcttctctg tgttttctgg tttaaaagct 4860
gcacttatat tttagcctta ttattttctg tagttccgga gagatggtgg gttgccattc 4920
tggtaggaaa atctgagttt tcttatcttt gacctagaag agattctttt tggaccaacc 4980
tgcgaaattg gtagttaggt ctatggaaag tggtaggatt tttttttttt taatcctgtg 5040
caaaggaaaa gaggtgcttt gtgggaaatc actaatgaga aggctaacct gcagcaccag 5100
agaaaccttt ccaagtgcta ggcaggagaa ctgaagaact ctttcagtga agtgagtcag 5160
cctagaagag gcaacccaca gtcttgattt ttgttgctgt ttcctgacct gttcttgcct 5220
gtcacctggg cctacacagg tcccgagcca acggggcttt catacccaag gatctgtttc 5280
cttgctgaaa atgaaaccct atctttcact ttacattcct ttcaatccaa ctgatcaaaa 5340
ctggtaccca cacttgccct ttctccctct ctccagcaca ctcccctcta agaaagtaaa 5400
agcaaagctt tcttaatggc aacactttgg gcctgttctg ttgctcctgc cttatttctc 5460
tttcaaccct gtacatgact cgtgttcacc atccccttga tcagtgtcca tccgcgctaa 5520
tttgcatcat gaactgaaca gtgtgtgagt ggtcacctac taaacccagc tctgggggca 5580
gagctgtctt ccccatctct ggtgctccta acacctgtga gatggtcctg tcgagaggac 5640
taggaaccga taggaggaga gtccttctcg gcagagctca ctgcaaacaa ctggaattga 5700
ggttgcacac tgtgattttt acaccgaaaa gccaaaagga gctggccatc cagggcctag 5760
ggagaccagc cttcctcagc tatgcttgcc gaaaccagca tgatgcttca aagagccctg 5820
ctccacccct catggcatgg atccttttcc tggtgtggac tctgaagggt cagtcttcgg 5880
ggaagagagg tggggtgggg ctactagcat cccaatttag aaaatagagg agtttgtagc 5940
cagcagcctg taaactggaa acactggtct cagccaacct cctcagggcg ccctggcttc 6000
tccccaagga gatgaggagc ggtgatgcca gcaccgggat gcgcagagca ctggaagggc 6060
tggtgcagat ctacttccca tgcagaagag aagtcacatc ttccagggaa tcgcaatgtt 6120
gtggcgtctg acttgtatgt cacatttgtg taaaatggta tattctttaa aatagtgttg 6180
ataactggaa tattgtatgt atgcttggag atgctttgtg tgaacctaag actgtcactc 6240
aacagatgtt ggattgggga aaatccaaag cacaacttca aaataaaata catttttagg 6300
tttcg 6305

<210> 142
<211> 330
<212> PRT
<213> Homo sapiens

<220>
<223> Human potassium channel modulatory factor

<400> 142
Met Ser Arg His Glu Gly Val Ser Cys Asp Ala Cys Leu Lys Gly Asn
1               5                   10                  15

Phe Arg Gly Arg Arg Tyr Lys Cys Leu Ile Cys Tyr Asp Tyr Asp Leu
            20                  25                  30

Cys Ala Ser Cys Tyr Glu Ser Gly Ala Thr Thr Thr Arg His Thr Thr

35                  40                  45

Asp His Pro Met Gln Cys Ile Leu Thr Arg Val Asp Phe Asp Leu Tyr
    50                  55                  60

Tyr Gly Gly Glu Ala Phe Ser Val Glu Gln Pro Gln Ser Phe Thr Cys
65                  70                  75                  80

Pro Tyr Cys Gly Lys Met Gly Tyr Thr Glu Thr Ser Leu Gln Glu His
                85                  90                  95

Val Thr Ser Glu His Ala Glu Thr Ser Thr Glu Val Ile Cys Pro Ile
            100                 105                 110

Cys Ala Ala Leu Pro Gly Gly Asp Pro Asn His Val Thr Asp Asp Phe
        115                 120                 125

Ala Ala His Leu Thr Leu Glu His Arg Ala Pro Arg Asp Leu Asp Glu
    130                 135                 140

Ser Ser Gly Val Arg His Val Arg Arg Met Phe His Pro Gly Arg Gly
145                 150                 155                 160

Leu Gly Gly Pro Arg Ala Arg Arg Ser Asn Met His Phe Thr Ser Ser
                165                 170                 175

Ser Thr Gly Gly Leu Ser Ser Ser Gln Ser Ser Tyr Ser Pro Ser Asn
            180                 185                 190

Arg Glu Ala Met Asp Pro Ile Ala Glu Leu Leu Ser Gln Leu Ser Gly
        195                 200                 205

Val Arg Arg Ser Ala Gly Gly Gln Leu Asn Ser Ser Gly Pro Ser Ala
    210                 215                 220

Ser Gln Leu Gln Gln Leu Gln Met Gln Leu Gln Leu Glu Arg Gln His
225                 230                 235                 240

Ala Arg Ala Ala Arg Gln Gln Leu Glu Thr Ala Arg Asn Ala Thr Arg
                245                 250                 255

Arg Thr Asn Thr Ser Ser Val Thr Thr Thr Ile Thr Gln Ser Thr Ala
            260                 265                 270

Thr Thr Asn Ile Ala Asn Thr Glu Ser Ser Gln Gln Thr Leu Gln Asn
        275                 280                 285

Ser Gln Phe Leu Leu Thr Arg Leu Asn Asp Pro Lys Met Ser Glu Thr
    290                 295                 300

Glu Arg Gln Ser Met Glu Ser Glu Arg Ala Asp Arg Ser Leu Phe Val
305                 310                 315                 320

Gln Glu Leu Leu Leu Ser Thr Leu Val Arg
                325                 330

<210> 143
<211> 1595
<212> DNA
<213> Homo sapiens

<220>
<223> Human potassium channel modulatory factor

<400> 143
ggacactgca gccggagccc gggaggggcc gcgccgccac cgtctgaact aggatgtccc 60
gacatgaagg tgtcagctgt gatgcatgtt taaaaggaaa ttttcgaggt cgcagatata 120
agtgtttaat ttgctacgat tacgatcttt gtgcatcttg ttatgaaagt ggtgcaacaa 180
caacaaggca tacaactgac cacccaatgc agtgcatatt aacaagggta gattttgatt 240
tatactatgg tggggaagct ttctctgtag agcagccaca gtcttttact tgtccctatt 300
gtggaaaaat gggctatacg gagacatctc ttcaagaaca tgttacttct gaacatgcag 360
aaacatcaac agaagtgatt tgtccaatat gtgcagcgtt acctggaggc gatcctaatc 420
atgtcacgga tgactttgca gctcatctta cacttgaaca cagagcccct agagatttag 480
atgaatcgag tggtgttcga catgtacgta gaatgtttca ccctggccgg ggattaggag 540
gtcctcgtgc tcgtagatca aacatgcact ttactagcag ttctactggt ggactttctt 600
cttctcagag ttcatattct ccaagcaata gggaagccat ggatcctata gctgagcttt 660
tatctcagtt atcaggagtg agacgttctg caggaggaca gcttaattcc tctggccctt 720
ccgcttctca gttacaacaa ctgcagatgc agctgcagct agaacggcag catgccaggg 780
cagcacggca acaactggag accgcacgca acgcaacccg gcgtactaac acaagcagtg 840
tcaccactac aatcacacaa tccacagcaa caaccaacat agctaataca gaaagcagtc 900
agcagactct acagaattcc cagtttcttt taacaaggtt gaatgatcct aaaatgtctg 960
aaacggagcg ccagtccatg gaaagcgagc gtgcagaccg cagcctgttt gtccaagagc 1020
tccttctgtc cactttagtg cgtgaagaga gctcatcctc agatgaggat gatcgggggg 1080
agatggcaga ttttggtgct atgggctgtg tagatattat gcctttagat gttgctttag 1140
aaaacctaaa tttaaaagag agtaataaag gaaatgagcc tccaccacct cctctttgat 1200
gacatcccaa ttcgcagaca atgtcctctg tgctgtattt gccaatgaaa gtggacaaca 1260
actatcttgg gtttgtttgg tgattgtaat ttcaggtctg tcactcttgt tacattgtgt 1320
acattcaaaa ggaagagaga aaatatatat gataatcatt tccacttaac taatttttac 1380
ttctagcagg taaatgtagg tagcagtgca ggggtgatct ctgcttcctg taccttgaca 1440
tgcaaaaggc tctcctaata ctccacattc aaactgaaga ggaaaattga aatctctaat 1500
gaagctgctg tgtgtattta tgaatattaa tgaataaaaa ctgcttggat ggtttacctt 1560
aaaaaaaaaa aaaaaaaaaa acaacaaaaa aaaaa 1595

<210> 144
<211> 589
<212> PRT
<213> Mus musculus

<220>
<223> RNA1 homolog

<400> 144
Met Ala Ser Glu Asp Ile Ala Lys Leu Ala Glu Thr Leu Ala Lys Thr
1               5                   10                  15

Gln Val Ala Gly Gly Gln Leu Ser Phe Lys Gly Lys Gly Leu Lys Leu
            20                  25                  30

Asn Thr Ala Glu Asp Ala Lys Asp Val Ile Lys Glu Ile Glu Glu Phe
        35                  40                  45

Asp Gly Leu Glu Ala Leu Arg Leu Glu Gly Asn Thr Val Gly Val Glu
    50                  55                  60

Ala Ala Arg Val Ile Ala Lys Ala Leu Glu Lys Lys Ser Glu Leu Lys
65                  70                  75                  80

Arg Cys His Trp Ser Asp Met Phe Thr Gly Arg Leu Arg Ser Glu Ile
                85                  90                  95

Pro Pro Ala Leu Ile Ser Leu Gly Glu Gly Leu Ile Thr Ala Gly Ala
            100                 105                 110

Gln Leu Val Glu Leu Asp Leu Ser Asp Asn Ala Phe Gly Pro Asp Gly
        115                 120                 125

Val Arg Gly Phe Glu Ala Leu Leu Lys Ser Pro Ala Cys Phe Thr Leu
    130                 135                 140

Gln Glu Leu Lys Leu Asn Asn Cys Gly Met Gly Ile Gly Gly Gly Lys
145                 150                 155                 160

Ile Leu Ala Ala Ala Leu Thr Glu Cys His Arg Lys Ser Ser Ala Gln
                165                 170                 175

Gly Lys Pro Leu Arg Leu Lys Val Phe Val Ala Gly Arg Asn Arg Leu
            180                 185                 190

Glu Asn Asp Gly Ala Thr Ala Leu Ala Glu Ala Phe Gly Ile Ile Gly
        195                 200                 205

Thr Leu Glu Glu Val His Met Pro Gln Asn Gly Ile Asn His Pro Gly
    210                 215                 220

Val Thr Ala Leu Ala Gln Ala Phe Ala Ile Asn Pro Leu Leu Arg Val
225                 230                 235                 240

Ile Asn Leu Asn Asp Asn Thr Phe Thr Glu Lys Gly Gly Val Ala Met
                245                 250                 255

Ala Glu Thr Leu Lys Thr Leu Arg Gln Val Glu Val Ile Asn Phe Gly
            260                 265                 270

Asp Cys Leu Val Arg Ser Lys Gly Ala Val Ala Ile Ala Asp Ala Val
        275                 280                 285

Arg Gly Gly Leu Pro Lys Leu Lys Glu Leu Asn Leu Ser Phe Cys Glu
    290                 295                 300

Ile Lys Arg Asp Ala Ala Leu Val Val Ala Glu Ala Val Ala Asp Lys
305                 310                 315                 320

Ala Glu Leu Glu Lys Leu Asp Leu Asn Gly Asn Ala Leu Gly Glu Glu
                325                 330                 335

Gly Cys Glu Gln Leu Gln Glu Val Met Asp Ser Phe Asn Met Ala Lys
            340                 345                 350

Val Leu Ala Ser Leu Ser Asp Asp Glu Gly Glu Asp Glu Asp Glu Glu
        355                 360                 365

Glu Glu Gly Glu Glu Asp Asp Glu Glu Glu Glu Asp Glu Glu Asp Glu
    370                 375                 380

Glu Asp Asp Asp Glu Glu Glu Glu Glu Gln Glu Glu Glu Glu Glu Pro
385                 390                 395                 400

Pro Gln Arg Gly Ser Gly Glu Glu Pro Ala Thr Pro Ser Arg Lys Ile
                405                 410                 415

Leu Asp Pro Asn Ser Gly Glu Pro Ala Pro Val Leu Ser Ser Pro Thr

420                 425                 430

Pro Thr Asp Leu Ser Thr Phe Leu Ser Phe Pro Ser Pro Glu Lys Leu
        435                 440                 445

Leu Arg Leu Gly Pro Lys Val Ser Val Leu Ile Val Gln Gln Thr Asp
    450                 455                 460

Thr Ser Asp Pro Glu Lys Val Val Ser Ala Phe Leu Lys Val Ala Ser
465                 470                 475                 480

Val Phe Arg Asp Asp Ala Ser Val Lys Thr Ala Val Leu Asp Ala Ile
                485                 490                 495

Asp Ala Leu Met Lys Lys Ala Phe Ser Cys Ser Ser Phe Asn Ser Asn
            500                 505                 510

Thr Phe Leu Thr Arg Leu Leu Ile His Met Gly Leu Leu Lys Ser Glu
        515                 520                 525

Asp Lys Ile Lys Ala Ile Pro Ser Leu His Gly Pro Leu Met Val Leu
    530                 535                 540

Asn His Val Val Arg Gln Asp Tyr Phe Pro Lys Ala Leu Ala Pro Leu
545                 550                 555                 560

Leu Leu Ala Phe Val Thr Lys Pro Asn Gly Ala Leu Glu Thr Cys Ser
                565                 570                 575

Phe Ala Arg His Asn Leu Leu Gln Thr Leu Tyr Asn Ile
            580                 585

<210> 145
<211> 2981
<212> DNA
<213> Mus musculus

<220>
<223> RNA1 homolog

<400> 145
caaaaatcct cctccgccgc cataatcggc tggggatcgt ggagcaggtg gtgtgcacga 60
gctaaacgag gagccgcggc agcaaggaga ggcaagcaga ctggtcactg ggcctggccc 120
ccacgcggac tctcaagcag tgaccgacgc gcgcgttccg tccccccacg ccgcggtgcc 180
gctaatttgt ggccgtctgc tgaccttgga tagctcaata ccatccctgt tttgctttgt 240
ttacagacct gcaggggagc ccgccactgc agccatggcc tctgaagaca ttgccaaact 300
ggcagagacc ctggctaaaa ctcaggtagc cggagggcag ctgagtttta agggcaaggg 360
tctcaaacta aatactgcag aagatgctaa agatgtgatt aaggagattg aagagtttga 420
cggcctcgaa gctctacgat tggagggcaa cacggtgggc gtggaagcgg ccagagtcat 480
cgccaaggcc ttagagaaga agtctgagct gaagcgatgc cactggagtg acatgttcac 540
aggaaggctt cggtctgaga tcccaccagc tctgatctca ctaggggagg gactcatcac 600
tgcaggagca cagctggtgg aactggacct cagtgacaac gcatttggac ccgatggagt 660
gcgaggcttc gaggccctcc tcaagagccc agcctgcttc accctacagg aactcaagct 720
taacaactgt ggcatgggca tcgggggtgg caagattctg gcagcagctc tgactgagtg 780
tcaccgcaag tccagtgccc aaggcaagcc cctgcgcctg aaggtgtttg tggctggcag 840
aaatcgcctg gagaatgacg gagccactgc actggcagaa gcttttggga tcatcgggac 900
tctggaagag gtgcatatgc cacagaacgg aattaaccat cctggtgtca cagccctggc 960
ccaggccttt gccatcaacc ccctgttgcg agtcatcaac ctaaatgaca acaccttcac 1020
tgagaagggt ggggtggcca tggctgagac tctgaagact ctgcgacaag tggaagtgat 1080
caatttggga gactgcctgg tgcgctctaa gggtgccgtt gccatcgcag atgctgtccg 1140

tggtggcctg cccaagctaa aggagctgaa tctgtcgttc tgcgaaatca agagagatgc 1200
tgccctggtt gttgctgagg ctgtggccga caaggctgag ctggagaagc tggacctcaa 1260
tggcaatgcc ctcggagaag agggctgtga gcagcttcag gaggtgatgg acagcttcaa 1320
tatggccaag gtgctggcat ccctcagtga tgatgaggga gaggatgaag atgaggagga 1380
agaaggagaa gaggatgatg aggaggagga agatgaagag gatgaggaag atgatgatga 1440
ggaggaggag gagcaggagg aagaggaaga gcctccgcag agagggtcag gggaggagcc 1500
ggctacaccc tcgaggaaga tcctggaccc taacagtggg gagccagctc ccgtgctgtc 1560
ctccccgact cccacagacc tctccacctt cctgtcattc ccctccccag agaagctgct 1620
gcgtctgggt cccaaggtct cggtgctgat agtccagcag actgatacct ccgacccaga 1680
gaaggttgtt tcagccttcc tgaaggtggc atctgtgttc agggatgacg cctcagtaaa 1740
gaccgcagtg ctggatgcca tcgatgccct catgaagaag gccttcagct gctcctcttt 1800
caattccaac accttcctta ccaggctgct catccacatg ggtctgctca agagtgaaga 1860
caagatcaag gccataccca gcctgcatgg ccccctgatg gtgttgaacc acgtggtgag 1920
gcaggactat ttccccaagg cccttgcacc cctgctcttg gcatttgtga caaagcccaa 1980
tggcgcgctg gaaacctgct cctttgcccg ccacaatctg ctgcagacgc tatacaacat 2040
ctagatttaa aggcagctgc ttcttgtccc tcgccctgcc ccagtgactg agggtcttgg 2100
atggactgag ctcggccagc atctccattg gatattttga ccagaggaag ggagagggtc 2160
ttgtctcctg tgtcatattg gtcctttgat actgtgttgg tttggtgtgt gtgtgtgggt 2220
atgtacttcc tgtctggatt gcagatcttc atctccctgc tggactaacc caagtccact 2280
gtggggaatt gatgcctagg ggaagggtag aaagctgctt tgttattaaa cttaggccaa 2340
cttagccata ggcccccgtt ggctgtcctg tctgccctgc cttcctgtcc caccaaagcc 2400
tgggaattct gatccgtgct gtgtccaccg tggagtccta aataacagag gctatcagac 2460
gatgggctat cattagaaag gtaaaaactc aggtggtaca tgccttaatc ccagactcag 2520
gaacagaggc aagaattctg agtttgaggc cagcctggtc tacaaagtga gttccaggac 2580
agccaggtct atacagagga accctgtctc aaaaaaacaa aaacaaaaaa aaaaaaacct 2640
ctgcccttaa ccatgtgact tggaggagca gccaggggag tgggagctgt ctggggtaac 2700
ctggctctag gaccaggtgg aaaaagcacc ttgctgccca gccctgttct tctcctgggg 2760
aggggaaggg caggtcccct aatgcctgaa ccacatgttg ggcctactct ctgaggtttt 2820
tctaagacag gagtgtgtaa tagggatgga gccgggtaag agatgctctg tgacttcccg 2880
aatgggctgc ttggccacct cctacttccc gttgtgttgt gtgtctgctg tgcccgggag 2940
cttcacaaat aaacgtcatc tgtgtagttg caaaaaaaaa a 2981

<210> 146
<211> 399
<212> PRT
<213> Homo sapiens

<220>
<223> Sec62

<400> 146
Met Ala Glu Arg Arg Arg His Lys Lys Arg Ile Gln Glu Val Gly Glu
1               5                   10                  15

Pro Ser Lys Glu Glu Lys Ala Val Ala Lys Tyr Leu Arg Phe Asn Cys
            20                  25                  30

Pro Thr Lys Ser Thr Asn Met Met Gly His Arg Val Asp Tyr Phe Ile
        35                  40                  45

Ala Ser Lys Ala Val Asp Cys Leu Leu Asp Ser Lys Trp Ala Lys Ala
    50                  55                  60

Lys Lys Gly Glu Glu Ala Leu Phe Thr Thr Arg Glu Ser Val Val Asp
65                  70                  75                  80

Tyr Cys Asn Arg Leu Leu Lys Lys Gln Phe Phe His Arg Ala Leu Lys
                85                  90                  95

Val Met Lys Met Lys Tyr Asp Lys Asp Ile Lys Lys Glu Lys Asp Lys

                100                 105                 110

Gly Lys Ala Glu Ser Gly Lys Glu Glu Asp Lys Lys Ser Lys Lys Glu
        115                 120                 125

Asn Ile Lys Asp Glu Lys Thr Lys Lys Glu Lys Glu Lys Lys Lys Asp
    130                 135                 140

Gly Glu Lys Glu Glu Ser Lys Lys Glu Glu Thr Pro Gly Thr Pro Lys
145                 150                 155                 160

Lys Lys Glu Thr Lys Lys Lys Phe Lys Leu Glu Pro His Asp Asp Gln
                165                 170                 175

Val Phe Leu Asp Gly Asn Glu Val Tyr Val Trp Ile Tyr Asp Pro Val
            180                 185                 190

His Phe Lys Thr Phe Val Met Gly Leu Ile Leu Val Ile Ala Val Ile
        195                 200                 205

Ala Ala Thr Leu Phe Pro Leu Trp Pro Ala Glu Met Arg Val Gly Val
    210                 215                 220

Tyr Tyr Leu Ser Val Gly Ala Gly Cys Phe Val Ala Ser Ile Leu Leu
225                 230                 235                 240

Leu Ala Val Ala Arg Cys Ile Leu Phe Leu Ile Ile Trp Leu Ile Thr
                245                 250                 255

Gly Gly Arg His His Phe Trp Phe Leu Pro Asn Leu Thr Ala Asp Val
            260                 265                 270

Gly Phe Ile Asp Ser Phe Arg Pro Leu Tyr Thr His Glu Tyr Lys Gly
        275                 280                 285

Pro Lys Ala Asp Leu Lys Lys Asp Glu Lys Ser Glu Thr Lys Lys Gln
    290                 295                 300

Gln Lys Ser Asp Ser Glu Glu Lys Ser Asp Ser Glu Lys Lys Glu Asp
305                 310                 315                 320

Glu Glu Gly Lys Val Gly Pro Gly Asn His Gly Thr Glu Gly Ser Gly
                325                 330                 335

Gly Glu Arg His Ser Asp Thr Asp Ser Asp Arg Arg Glu Asp Asp Arg
            340                 345                 350

Ser Gln His Ser Ser Gly Asn Gly Asn Asp Phe Glu Met Ile Thr Lys
        355                 360                 365

Glu Glu Leu Glu Gln Gln Thr Asp Gly Asp Cys Glu Glu Asp Glu Glu
    370                 375                 380

Glu Glu Asn Asp Gly Glu Thr Pro Lys Ser Ser His Glu Lys Ser
385                 390                 395

<210> 147
<211> 2337
<212> DNA
<213> Homo sapiens

<220>
<223> Sec62

<400> 147
ggagcggcca acatggcgga acgcaggaga cacaagaagc ggatccagga agttggtgaa 60
ccatctaaag aagagaaggc tgtggccaag tatcttcgat tcaactgtcc aacaaagtcc 120
accaatatga tgggtcaccg ggttgattat tttattgctt caaaagcagt ggactgtctt 180
ttggattcaa agtgggcaaa ggccaagaaa ggagaggaag ctttatttac aaccagggag 240
tctgtggttg actactgcaa caggctttta aagaagcagt tttttcaccg agccctaaaa 300
gtaatgaaaa tgaaatatga taaagacata aagaaagaaa aagataaagg aaaagctgaa 360
agtggaaaag aagaagataa aaagagcaag aaagaaaata taaaggatga gaagacaaaa 420
aaagaaaaag agaaaaaaaa agatggtgaa aaggaagaat ccaaaaagga ggaaactcca 480
ggaactccta aaaagaagga aactaagaaa aaattcaaac ttgagccaca tgatgatcag 540
gtttttctgg atggaaatga ggtgtatgta tggatctatg acccagttca ctttaaaaca 600
tttgtcatgg gattaattct tgtgattgca gtaatagcgg ccaccctctt ccccctttgg 660
ccagcagaaa tgagagtagg tgtttattac ctcagtgtgg gtgcaggctg ttttgtagcc 720
agtattcttc tccttgctgt tgctcgatgc attctatttc tcatcatttg gctcataact 780
ggaggaaggc accacttttg gttcttgcca aatctgactg ctgatgtggg cttcattgac 840
tccttcaggc ctctgtacac acatgaatac aaaggaccaa aagcagactt aaagaaagat 900
gagaagtctg aaaccaaaaa gcaacagaag tccgacagtg aggaaaagtc agacagtgag 960
aaaaaggaag atgaggaggg gaaagtagga ccaggaaatc atggaacaga aggctcgggg 1020
ggagaacggc attcagacac ggacagtgac aggagggaag atgatcgatc ccagcacagt 1080
agtggaaatg gaaatgattt tgaaatgata acaaaagagg aactggaaca gcaaacagat 1140
ggggattgtg aagaggatga ggaagaggaa aatgatggag aaacacctaa atcttcacat 1200
gaaaaatcat aatctgacta attttgggac tgaatgaata agtacaagag gttggatttt 1260
ctatgttggc tgattaccat attgaacaca tggcatttgt agcattcttt aaatctatct 1320
actgaaatgt atttgacatt caggcagtta tattcggtcc ttcattttat agaatattgg 1380
cactattatt ggtacagttt aaagccatta atatgtttta tccatttgat aattttacag 1440
taagtaggtc tcattcattt tgacagttat caaagatgta ctttccacag ttaaatttac 1500
attaatggca atttttgata gttttatggc tttttactgt tagactaatc aaaaataact 1560
ttaaaaggaa caaagaaact ccaacatttc acattatgca tagttatgta gccatttcac 1620
agtttcttta agatgtgtaa actcattgtc cttgatagtt tttatttttc attataaaat 1680
tataccagga gatttctttt aagattctga gttagcagag ttcaaaacta ttttgtggaa 1740
acaagccaac tagtaacaat gcagcaacac ttctggttta gctaaattat ttttccaatg 1800
taggaaatcc acactgattt gtacgtctga ctgagagaaa gatggtcgtc tccagcagag 1860
aaagtgaaca gcatttgttg gaaggtgatg gctctccctc ctccctcccc atttcattgg 1920
cgtaacgtaa agtgtattct gtacataatt tacaaataaa acattttatt ttaattgtta 1980
cttattattt agatatttct caacacttaa attcataaaa ttaagaccat gtaagggtat 2040
gtttttagag aaatggaagt ttgagtaacc cacagaacat ctgtgatctt tctacagcag 2100
cttcagtttt gtgccaacat tccatgtatt ttgaatatga gcaaaaactg atcttaagag 2160
cagacttaaa gtagctttgt acgccttaat gttcattttg atttatttta aatctttaca 2220
ttcagaaatg agatactgta ttatcagacc aggaggcatt gctgtgaaag ataatttcct 2280
attctaaaat atcaaattta aaataaagat aatgaaagaa aaaaaaaaaa aaaaaaa 2337

<210> 148
<211> 108
<212> PRT
<213> Mus musculus

<220>
<223> Co-chaperonin cofactor A

<400> 148
Met Ala Asp Pro Arg Val Arg Gln Ile Lys Ile Lys Thr Gly Val Val
1               5                   10                  15

Arg Arg Leu Val Lys Glu Arg Val Met Tyr Glu Lys Glu Ala Lys Gln
            20                  25                  30

Gln Glu Glu Lys Ile Glu Lys Met Lys Ala Glu Asp Gly Glu Asn Tyr
        35                  40                  45

Ala Ile Lys Lys Gln Ala Glu Ile Leu Gln Glu Ser Arg Met Met Ile
    50                  55                  60

Pro Asp Cys Gln Arg Arg Leu Glu Ala Ala Tyr Thr Asp Leu Gln Gln
65                  70                  75                  80

Ile Leu Glu Ser Glu Lys Asp Leu Glu Glu Ala Glu Glu Tyr Lys Glu
                85                  90                  95

Ala Arg Val Val Leu Asp Ser Val Lys Leu Glu Ala
            100                 105


<210> 149
<211> 508
<212> DNA
<213> Mus musculus

<220>
<223> Co-chaperonin cofactor A

<400> 149
agggaccatg gccgatcctc gcgtgaggca gatcaagatc aagaccggag tagtgaggcg 60
attggtcaaa gaaagagtga tgtacgaaaa agaagcaaag cagcaagaag aaaagattga 120
aaaaatgaag gctgaagatg gagagaacta cgccattaag aagcaggcag agatcctgca 180
agagtcccgg atgatgatcc cagattgcca gcgtcggtta gaggctgcat atactgatct 240
tcagcagata ttagaaagtg aaaaagacct ggaagaagcc gaggagtata aagaagcccg 300
tgtagtgctg gattccgtaa agctagaagc ctgacatttt ctgtatggga tgtttttttg 360
cattaaatcc tggggtccat tctacaattc attgttttca gccactgctg tgtttaataa 420
tatgaaaacg tggttctttt tatgcagttg catttatttt tctttgcata acttaatgtg 480
tcaaataaat gagttcatct aaaaaaaa                                    508


<210> 150
<211> 139
<212> PRT
<213> Homo sapiens

<220>
<223> LSm4 protein

<400> 150
Met Leu Pro Leu Ser Leu Leu Lys Thr Ala Gln Asn His Pro Met Leu
1               5                   10                  15

Val Glu Leu Lys Asn Gly Glu Thr Tyr Asn Gly His Leu Val Ser Cys
            20                  25                  30

Asp Asn Trp Met Asn Ile Asn Leu Arg Glu Val Ile Cys Thr Ser Arg
        35                  40                  45

Asp Gly Asp Lys Phe Trp Arg Met Pro Glu Cys Tyr Ile Arg Gly Ser
    50                  55                  60

Thr Ile Lys Tyr Leu Arg Ile Pro Asp Glu Ile Ile Asp Met Val Lys
65                  70                  75                  80

Glu Glu Val Val Ala Lys Gly Arg Gly Arg Gly Gly Leu Gln Gln Gln

85                  90                  95

Lys Gln Gln Lys Gly Arg Gly Met Gly Gly Ala Gly Arg Gly Val Phe
            100                 105                 110

Gly Gly Arg Gly Arg Gly Gly Ile Pro Gly Thr Gly Arg Gly Gln Pro
        115                 120                 125

Glu Lys Lys Pro Gly Arg Gln Ala Gly Lys Gln
    130                 135


<210> 151
<211> 1033
<212> DNA
<213> Homo sapiens

<220>
<223> LSm4 protein

<400> 151
cagcggcggc ggcggaagtg gccggcgagc ccggtccccg ccggcaccat gcttcccttg 60
tcactgctga agacggctca gaatcacccc atgttggtgg agctgaaaaa tggggagacg 120
tacaatggac acctggtgag ctgcgacaac tggatgaaca ttaacctgcg agaagtcatc 180
tgcacgtcca gggacgggga caagttctgg cggatgcccg agtgctacat ccgcggcagc 240
accatcaagt acctgcgcat ccccgacgag atcatcgaca tggtcaagga ggaggtggtg 300
gccaagggcc gcggccgcgg aggcctgcag cagcagaagc agcagaaagg ccgcggcatg 360
ggcggcgctg gccgaggtgt gtttggtggc cggggccgag gtgggatccc gggcacaggc 420
agaggccagc cagagaagaa gcctggcaga caggcgggca aacagtgagc gcccacccag 480
accggctgct gcgccccctc ctgccagggt ggcgattccg ctccacagtc tcggacggat 540
ctgctcagaa aggaagaggc aggcgccagg gggaaccccc ttcgtgtttt gtgaccctcc 600
cttttaggtg aagccccttt ttcttgctaa aaccggcaat tctccggtta gaaatgttac 660
ttggtgtttt ttggttttgt gaaacggccg tcccaaagct ggctggattc ctagaagagt 720
ctgtgttgaa ggcatctttc aacccctcgc tctggttctc agggcagcat tttccaggcg 780
ggtttgtttt gcatttcttg gagcctctcc gagcagcaac cagacgggag atttttattt 840
taagctgttc atgctgggac tgacagcctg cagggtttcc ttgggcgcgg ccccaaaatt 900
gccttcaaaa caaacccggg acggttgaaa gccttcgaac cgtgcagggg atgcctcggg 960
ccctggccct tcgcttcctc tcttgtgtta tggaaataaa aacaaataaa actacaaaaa 1020
aaaaaaaaaa aaa                                                    1033


<210> 152
<211> 1358
<212> PRT
<213> Mus musculus

<220>
<223> Mismatch repair protein

<400> 152
Met Ser Arg Gln Ser Thr Leu Tyr Ser Phe Phe Pro Lys Ser Pro Ala
1               5                   10                  15

Leu Gly Asp Thr Lys Lys Ala Ala Ala Glu Ala Ser Arg Gln Gly Ala
            20                  25                  30

Ala Ala Ser Gly Ala Ser Ala Ser Arg Gly Gly Asp Ala Ala Trp Ser
        35                  40                  45

Glu Ala Glu Pro Gly Ser Arg Ser Ala Ala Val Ser Ala Ser Ser Pro
    50                  55                  60

Glu Ala Lys Asp Leu Asn Gly Gly Leu Arg Arg Ala Ser Ser Ser Ala
65                  70                  75                  80

Gln Ala Val Pro Pro Ser Ser Cys Asp Phe Ser Pro Gly Asp Leu Val
                85                  90                  95

Trp Ala Lys Met Glu Gly Tyr Pro Trp Trp Pro Cys Leu Val Tyr Asn
            100                 105                 110

His Pro Phe Asp Gly Thr Phe Ile Arg Lys Lys Gly Lys Ser Val Arg
        115                 120                 125

Val His Val Gln Phe Phe Asp Asp Ser Pro Thr Arg Gly Trp Val Ser
    130                 135                 140

Lys Arg Met Leu Lys Pro Tyr Thr Gly Ser Lys Ser Lys Glu Ala Gln
145                 150                 155                 160

Lys Gly Gly His Phe Tyr Ser Ser Lys Ser Glu Ile Leu Arg Ala Met
                165                 170                 175

Gln Arg Ala Asp Glu Ala Leu Ser Lys Asp Thr Ala Glu Arg Leu Gln
            180                 185                 190

Leu Ala Val Cys Asp Glu Pro Ser Glu Pro Glu Glu Glu Glu Glu Thr
        195                 200                 205

Glu Val His Glu Ala Tyr Leu Ser Asp Lys Ser Glu Glu Asp Asn Tyr
    210                 215                 220

Asn Glu Ser Glu Glu Glu Ala Gln Pro Ser Val Gln Gly Pro Arg Arg
225                 230                 235                 240

Ser Ser Arg Gln Val Lys Lys Arg Arg Val Ile Ser Asp Ser Glu Ser
                245                 250                 255

Asp Ile Gly Gly Ser Asp Val Glu Phe Lys Pro Asp Thr Lys Gln Glu
            260                 265                 270

Gly Ser Ser Asp Asp Ala Ser Ser Gly Val Gly Asp Ser Asp Ser Glu
        275                 280                 285

Asp Leu Gly Thr Phe Gly Lys Gly Ala Pro Lys Arg Lys Arg Ala Met
    290                 295                 300

Val Ala Gln Gly Gly Leu Arg Arg Lys Ser Leu Lys Lys Glu Thr Gly
305                 310                 315                 320

Ser Ala Lys Arg Ala Thr Pro Ile Leu Ser Glu Thr Lys Ser Thr Leu
                325                 330                 335

Ser Ala Phe Ser Ala Pro Gln Asn Ser Glu Ser Gln Thr His Val Ser
            340                 345                 350

Gly Gly Gly Asn Asp Ser Ser Gly Pro Thr Val Trp Tyr His Glu Thr
        355                 360                 365

Leu Glu Trp Leu Lys Pro Glu Lys Arg Arg Asp Glu His Arg Arg Arg
    370                 375                 380

Pro Asp His Pro Glu Phe Asn Pro Thr Thr Leu Tyr Val Pro Glu Glu
385                 390                 395                 400

Phe Leu Asn Ser Cys Thr Pro Gly Met Arg Lys Trp Trp Gln Leu Lys
                405                 410                 415

Ser Gln Asn Phe Asp Leu Val Ile Phe Tyr Lys Val Gly Lys Phe Tyr
            420                 425                 430

Glu Leu Tyr His Met Asp Ala Val Ile Gly Val Ser Glu Leu Gly Leu
        435                 440                 445

Ile Phe Met Lys Gly Asn Trp Ala His Ser Gly Phe Pro Glu Ile Ala
    450                 455                 460

Phe Gly Arg Phe Ser Asp Ser Leu Val Gln Lys Gly Tyr Lys Val Ala
465                 470                 475                 480

Arg Val Glu Gln Thr Glu Thr Pro Glu Met Met Glu Ala Arg Cys Arg
                485                 490                 495

Lys Met Ala His Val Ser Lys Phe Asp Arg Val Val Arg Arg Glu Ile
            500                 505                 510

Cys Arg Ile Ile Thr Lys Gly Thr Gln Thr Tyr Ser Val Leu Asp Gly
        515                 520                 525

Asp Pro Ser Glu Asn Tyr Ser Arg Tyr Leu Leu Ser Leu Lys Glu Lys
    530                 535                 540

Glu Glu Glu Thr Ser Gly His Thr Arg Val Tyr Gly Val Cys Phe Val
545                 550                 555                 560

Asp Thr Ser Leu Gly Lys Phe Phe Ile Gly Gln Phe Ser Asp Asp Arg
                565                 570                 575

His Cys Ser Arg Phe Arg Thr Leu Val Ala His Tyr Pro Pro Val Gln
            580                 585                 590

Ile Leu Phe Glu Lys Gly Asn Leu Ser Thr Glu Thr Lys Thr Val Leu
        595                 600                 605

Lys Gly Ser Leu Ser Ser Cys Leu Gln Glu Gly Leu Ile Pro Gly Ser
    610                 615                 620

Gln Phe Trp Asp Ala Thr Lys Thr Leu Arg Thr Leu Leu Glu Gly Gly
625                 630                 635                 640

Tyr Phe Thr Gly Asn Gly Asp Ser Ser Thr Val Leu Pro Leu Val Leu
                645                 650                 655

Lys Gly Met Thr Ser Glu Ser Asp Ser Val Gly Leu Thr Pro Gly Glu
            660                 665                 670

Glu Ser Glu Leu Ala Leu Ser Ala Leu Gly Gly Ile Val Phe Tyr Leu
        675                 680                 685

Lys Lys Cys Leu Ile Asp Gln Glu Leu Leu Ser Met Ala Asn Phe Glu
    690                 695                 700

Glu Tyr Phe Pro Leu Asp Ser Asp Thr Val Ser Thr Val Lys Pro Gly

705                 710                 715                 720

Ala Val Phe Thr Lys Ala Ser Gln Arg Met Val Leu Asp Ala Val Thr
                725                 730                 735

Leu Asn Asn Leu Glu Ile Phe Leu Asn Gly Thr Asn Gly Ser Thr Glu
            740                 745                 750

Gly Thr Leu Leu Glu Arg Leu Asp Thr Cys His Thr Pro Phe Gly Lys
        755                 760                 765

Arg Leu Leu Lys Gln Trp Leu Cys Ala Pro Leu Cys Ser Pro Ser Ala
    770                 775                 780

Ile Ser Asp Arg Leu Asp Ala Val Glu Asp Leu Met Ala Val Pro Asp
785                 790                 795                 800

Lys Val Thr Glu Val Ala Asp Leu Leu Lys Lys Leu Pro Asp Leu Glu
                805                 810                 815

Arg Leu Leu Ser Lys Ile His Asn Val Gly Ser Pro Leu Lys Ser Gln
            820                 825                 830

Asn His Pro Asp Ser Arg Ala Ile Met Tyr Glu Glu Thr Thr Tyr Ser
        835                 840                 845

Lys Lys Lys Ile Ile Asp Phe Leu Ser Ala Leu Glu Gly Phe Lys Val
    850                 855                 860

Met Cys Lys Val Ser Gly Leu Leu Glu Glu Val Ala Gly Gly Phe Thr
865                 870                 875                 880

Ser Lys Thr Leu Lys Gln Val Val Thr Leu Gln Ser Lys Ser Pro Lys
                885                 890                 895

Gly Arg Phe Pro Asp Leu Thr Ala Glu Leu Gln Arg Trp Asp Thr Ala
            900                 905                 910

Phe Asp His Glu Lys Ala Arg Lys Thr Gly Leu Ile Thr Pro Lys Ala
        915                 920                 925

Gly Phe Asp Ser Asp Tyr Asp Gln Ala Leu Ala Asp Ile Arg Glu Asn
    930                 935                 940

Glu Gln Ser Leu Leu Glu Tyr Leu Asp Lys Gln Arg Ser Arg Leu Gly
945                 950                 955                 960

Cys Lys Ser Ile Val Tyr Trp Gly Ile Gly Arg Asn Arg Tyr Gln Leu
                965                 970                 975

Glu Ile Pro Glu Asn Phe Ala Thr Arg Asn Leu Pro Glu Glu Tyr Glu
            980                 985                 990

Leu Lys Ser Thr Lys Lys Gly Cys Lys Arg Tyr Trp Thr Lys Thr Ile
        995                 1000                1005

Glu Lys Lys Leu Ala Asn Leu Ile Asn Ala Glu Glu Arg Arg Asp Thr
    1010                1015                1020

Ser Leu Lys Asp Cys Met Arg Arg Leu Phe Cys Asn Phe Asp Lys Asn
1025                1030                1035                1040

His Lys Asp Trp Gln Ser Ala Val Glu Cys Ile Ala Val Leu Asp Val
                1045                1050                1055

Leu Leu Cys Leu Ala Asn Tyr Ser Gln Gly Gly Asp Gly Pro Met Cys
            1060                1065                1070

Arg Pro Glu Ile Val Leu Pro Gly Glu Asp Thr His Pro Phe Leu Glu
        1075                1080                1085

Phe Lys Gly Ser Arg His Pro Cys Ile Thr Lys Thr Phe Phe Gly Asp
    1090                1095                1100

Asp Phe Ile Pro Asn Asp Ile Leu Ile Gly Cys Glu Glu Glu Ala Glu
1105                1110                1115                1120

Glu His Gly Lys Ala Tyr Cys Val Leu Val Thr Gly Pro Asn Met Gly
                1125                1130                1135

Gly Lys Ser Thr Leu Ile Arg Gln Ala Gly Leu Leu Ala Val Met Ala
            1140                1145                1150

Gln Leu Gly Cys Tyr Val Pro Ala Glu Lys Cys Arg Leu Thr Pro Val
        1155                1160                1165

Asp Arg Val Phe Thr Arg Leu Gly Ala Ser Asp Arg Ile Met Ser Gly
    1170                1175                1180

Glu Ser Thr Phe Phe Val Glu Leu Ser Glu Thr Ala Ser Ile Leu Arg
1185                1190                1195                1200

His Ala Thr Ala His Ser Leu Val Leu Val Asp Glu Leu Gly Arg Gly
                1205                1210                1215

Thr Ala Thr Phe Asp Gly Thr Ala Ile Ala Asn Ala Val Val Lys Glu
            1220                1225                1230

Leu Ala Glu Thr Ile Lys Cys Arg Thr Leu Phe Ser Thr His Tyr His
        1235                1240                1245

Ser Leu Val Glu Asp Tyr Ser Lys Ser Val Cys Val Arg Leu Gly His
    1250                1255                1260

Met Ala Cys Met Val Glu Asn Glu Cys Glu Asp Pro Ser Gln Glu Thr
1265                1270                1275                1280

Ile Thr Phe Leu Tyr Lys Phe Ile Lys Gly Ala Cys Pro Lys Ser Tyr
                1285                1290                1295

Gly Phe Asn Ala Ala Arg Leu Ala Asn Leu Pro Glu Glu Val Ile Gln
            1300                1305                1310

Lys Gly His Arg Lys Ala Arg Glu Phe Glu Arg Met Asn Gln Ser Leu
        1315                1320                1325

Gln Leu Phe Arg Glu Val Cys Leu Ala Thr Glu Lys Pro Thr Ile Asn
    1330                1335                1340

Gly Glu Ala Ile His Arg Leu Leu Ala Leu Ile Asn Gly Leu
1345                1350                1355

<210> 153
<211> 8038
<212> DNA
<213> Mus musculus

<220>
<223> Mismatch repair protein

<400> 153
gctagagatt atatttagta ttaccagtcc tgttcagcat agtgctgggg ctagagccca 60
aggcctgtgc atgcttcaca ggcaccctgt caactgagtg accccctagc ctggttattc 120
agttgggaca attttagaaa gagttcctaa acaaactaca cttgtatctg ctttgaggtt 180
tttcatacct tgacccaggg ttctgacatt agaagaaaga ctcaagcagc cttggatgac 240
tatacagcga ttcaaacaag taaaactggg gattctagtg tcattgctac cttcccccgt 300
tagcttttga tctgtgcata tgaaaaatgg ggcattaaaa agtataactg attgtaactt 360
cctgtttgtg ttataaattg gccaactctt cagccacctg gttcttattt atatttctag 420
gttcaaagtc aaaggaagcc caaaagggag gtcatttcta cagttcaaag tctgagatac 480
tcagagcaat gcaacgtgca gatgaggcct taagtaaaga cacggctgag aggctgcagc 540
tggcagtgtg tgatgagcct tcagagccgg aggaggagga ggagacagag gtcagtcact 600
ccattctctt aggggggaag gaggatatga tgtgtatgtg atgaagtgag aaataataaa 660
aaatatagta attgtatgtg aatgcaccat ttcatttgta catgcagaca tctcccctcc 720
ctggatttag cttatttaga atttcaacag tctgtctgtc tgtctctgtc tctctctctg 780
ttttgtactt gaagctggcc tcaagctcag aatcttctta ctttctgctt ttcaaatggt 840
ctcccagtcc agggattgaa tttagggtct caaatgtgtt aggcaaatgt tcttccactt 900
ggcccaccca ctcccttagg gaaaaagctg gctttgaatt caacaagtgg ggaaaataac 960
tagccatgct tctgcttctg cttcctaagt gctggagtta gaggtgcatg tcaacatacc 1020
tggggttttt ttttttttgt tttgttttgt ttttttggag acagggtttc tctgtgtagc 1080
cctggctgtc ctggaactca ctctgtagac caggctgccc tcgaactcag aaatccgcct 1140
gcttctgctt cccgagtgct gggattaaag gcgtacacct ctacacccgg caacatacct 1200
ggtttatgaa ttgctgagga tcaaacgtag gatcaactga gcttcatctc tgccccatgc 1260
tggccttaat actacagtgg tctttaagtg ctggggttat aggcctgaat cacaacatct 1320
gggttccaca gatgtactta tcaaatttga ttgattgatg gcaccagaga ttgaactaag 1380
gacccgtttt atatttacaa gcccctttct ttgtttggca ggtgcatgag gcttatttat 1440
ctgataagag tgaagaggat aattacaacg agagtgaaga agaagcacag cccagtgttc 1500
aggggcctag gcgaagcagc cggcaagtca agaagcgacg ggtcatctca gactcggaga 1560
gtgacattgg tggctctgat gtagaattca agccagacac taagcaggaa ggaagcagtg 1620
atgacgcgag cagtggagtt ggggacagcg atagtgagga cctaggcacc tttggcaaag 1680
gcgctccaaa gcggaagaga gccatggttg ctcagggtgg acttagaagg aaaagtctga 1740
agaaggaaac aggctcagcc aaacgagcaa ctcccattct ctcagaaacc aagagtacct 1800
tgagcgcttt ttctgcccct cagaattctg aatcccagac ccacgtcagt ggaggaggta 1860
acgacagtag tggacccact gtttggtatc acgaaacttt agaatggctt aagccagaaa 1920
agagaagaga tgagcacagg agacgccccg atcaccctga atttaacccc accacactgt 1980
atgtgcctga agagttcctc aattcttgta ccccagggat gaggaagtgg tggcagctca 2040
agtctcagaa ctttgacctc gtcatctttt ataaggtggg gaagttttat gagttgtatc 2100
acatggatgc tgttattgga gtcagtgagc tggggctgat attcatgaag ggcaactggg 2160
cccattctgg ttttccagag attgcgtttg gccggttctc ggattccttg gtgcagaagg 2220
gctataaggt agcacgagtg gagcagaccg agactccaga aatgatggag gcgcgatgtc 2280
ggaaaatggc acacgtgtcc aagtttgata gagtggtgag aagggagatt tgcaggatca 2340
ttaccaaggg cacacagacc tacagtgtgc tggatggtga tccctctgag aactacagta 2400
ggtatcttct tagcctcaaa gagaaggagg aagagacttc tggtcacact cgtgtgtatg 2460
gtgtttgctt tgtggacact tcactgggca agtttttcat cggccagttt tcggatgacc 2520
gccactgttc cagatttagg acgctagtgg ctcactatcc tccagtacaa attttgttcg 2580
agaaaggaaa tctctcaaca gagaccaaaa ctgtcctaaa gggttcactg tcatcttgtc 2640
ttcaggaagg tctcatacca ggttcccaat tttgggatgc cactaagacc ttgcgaacac 2700
tccttgaagg agggtacttt actggaaatg gagacagtag tacagtgctg cctctggtgc 2760
ttaaaggaat gacctcggag tcagattctg ttgggttgac accaggcgag gagagtgaat 2820
tggctctctc tgctctaggt ggtattgttt tctacctcaa aaaatgcctg attgatcagg 2880
agcttctgtc aatggctaat tttgaagagt atttcccttt ggattctgac acggtcagca 2940
cagtaaaacc tggggctgtc tttactaaag ccagtcagcg gatggtgcta gacgcagtga 3000
cattaaacaa cctggagatt ttcctgaatg ggacaaatgg ctctactgaa gggaccctgc 3060

tagagagatt ggatacttgc catacgccct ttggcaagcg gctcttaaaa cagtggctct 3120
gtgcccccct ctgcagccct tctgccatca gtgaccgtct agatgcggtg gaagatctga 3180
tggccgtgcc tgacaaggtc actgaagttg cagacctctt aaagaagctg ccagaccttg 3240
agaggctact gagtaagatt cataatgttg ggtctcccct gaagagccag aaccatccag 3300
acagcagggc tataatgtac gaagaaacga catacagcaa aaagaagatc attgattttc 3360
tctctgctct agaaggattc aaagtaatgt gtaaagtctc agggcttctg gaggaggtcg 3420
cgggtggttt tacgtccaaa accctcaagc aggttgttac tctgcagtca aaaagtccta 3480
aaggccgctt tcctgacctg actgcagaac tgcagcgatg ggacacagcc tttgaccatg 3540
agaaggctcg gaagaccgga ctcatcactc caaaagcagg gtttgactct gattatgacc 3600
aagcacttgc tgacatcaga gagaatgaac agagcctcct ggagtactta gacaaacagc 3660
gcagtcggct tggctgtaag agcatagtct actggggaat cggtaggaac cgttaccagt 3720
tagagattcc agagaatttc gctacccgta atctacctga agaatatgag ctgaaatcta 3780
ctaaaaaagg ctgtaaacga tactggacca aaacgattga gaagaaatta gctaatctta 3840
ttaatgctga agaacgcagg gacacatcct taaaggactg catgcggcgc ttgttctgta 3900
acttcgacaa gaaccacaag gactggcagt ctgctgtaga gtgcattgca gtgttgggta 3960
aggctttggt taacccttgt gtcccaacta tccatctcta aacctagcta tgtttaatat 4020
gggtgtgtgc atggatgttt aaataactac aacttaggtt tcactataat agccttatga 4080
caatttttcc tagtcttgga atagtttcga attttatcat tgagggaaga tttattttca 4140
agtctctttt taaaaacact tactggtact aggtaatttc ctgatttgtt taaggatctg 4200
tagcccaagc tgatgttgaa ctcctggcaa tcctcctgcc tcacatcccc aagtgctagg 4260
attagaggtc tgggccacta tttcccagct tttacaattt tattttgtca tgctggcatc 4320
agatctagac ctttgtgcat gcagggctat ttaaacattt tggccacgga gccaaaatct 4380
tgagcccaaa atgcttttat aaatgcttat tgaaaggttt ttcttttggc tttattcttt 4440
gtggttcttc cttttgtatc gagtcagatt tgcatggaaa taagactgga gctggatgta 4500
ggctttctgt gcgccctttg gttatcttta aacatcagca cttcgcatct ggagctgtct 4560
gggcatcagc tttgttagca gtgtgggggg ttaaacgtag tgctgcaagc acaggctgta 4620
ctatcacacg gagggaggtc tgtaacagag cttctctcat ctaagcagtg agcagatgca 4680
ctacagtagt ggaatcctcc ctcagcacag gtgctctata tcggcccact cacctggctc 4740
cctagctcct tgtaatgtca ttgtcaactg cccccccccc cccccccaag cactagctgt 4800
aaagtctagc acgctgtgtc ctcctctctc atctccacct cccaactttg ctattctggc 4860
ctaagctggt ctggagctca cacagatgcc tgccgtggcc aaaacagaaa atgttgcttt 4920
tactgagaag tacaatactt tgttttgtgt gttgggagtt tgggcacgca gtaagtagtt 4980
cttacggagg tctctttcca cgggggattg aacttgggtg ggtctctggg ctttgatctg 5040
ctgaatcatc ttgctggtaa aagacacaat agaagcaagc acaggtacta tctagtgggt 5100
ataagtttac ccagcaagaa attaacaaaa tgatgaagtc ttacttgtat cctcttgaca 5160
gatgtcttac tgtgcctggc taactacagt caaggaggag atggtcctat gtgtcgccca 5220
gaaattgtgt taccaggaga agacacacat cccttcttag agtttaaagg gtcacgacat 5280
ccctgcatta caaagacttt ttttggagac gattttattc ctaacgacat tctaataggc 5340
tgcgaggaag aagcagagga acatggcaaa gcctattgtg tgcttgtgac tggaccaaat 5400
atggggggca agtctacact cataagacag gtaatttgtt cttcatgttc tattaagtag 5460
aaagcaattg taagatgaag ggcagatgct atagaatcta gtctactata taatctaatc 5520
cactgtccag agagcaaagt cccataagag aagcaaaggg aagctcctcc tataaagttg 5580
tttcatcagt cttctggctc ttagctaatg ttaaagaaaa cacatgatta tcaggacttt 5640
agagagtaca ttcatggtga ataaaattac aggggtgata taataaaaat aatggcaccc 5700
aattattttg atcctagggg agcccagggc attttttttt ttttaaattt gaaacatgaa 5760
catattctta ctgctaagct gctatcacca ctactgtcct ctgcctggta cttgtgttaa 5820
agatttagac ttgtttgtgc tccttgatgt ccttttagag gaaactagtc atatctttgt 5880
aatgtaattt gctaacaaaa ttggctgtat tgtatgttat ctttcaactt taaaacaagt 5940
agaggaggtt attgtctgag ataaacttgc tggatccagg tattcctggt agttaaagcc 6000
cagaggtctt tgttagctag gtatggaata ccaggttcct acttaaggat tcttgaatcc 6060
aatggtattg tgtacagata gattttcaaa taccaattcc tttcttccca tcttcccagg 6120
ctggtctgtt ggctgtgatg gcccagctgg gttgttacgt acctgctgag aagtgcaggc 6180
tcacaccagt tgacagagtg tttactaggc ttggtgcctc agatagaata atgtcaggtg 6240
agttgctttt cctccacaca attttacaat tcagttggca aggataacct gccttttgtt 6300
ttaaacttga tgtaacaaca tatatgcttt tagtttaaat tcttgctcat cattttcaaa 6360
caagatataa tctgctctta actcagttgt ttcttttttt aggagaaagc acattttttg 6420
ttgagttgag tgaaactgct agcatactca ggcatgcaac agcacattct ttggtacttg 6480
tggatgaatt aggtaagaca gattttgttt gaatgccatt agggtggaaa acccatgtcc 6540
ccaatagcta ctgtttcccc ctctctggcg gtcctgggat ttcctgtctt gtctggtagg 6600
caagagctgt actgctgaga tgcagtctcc ataatcattt ttatttaaga ctgctcatca 6660
gtcaagagca tgcatgcatc tctgtaccat gtacatgcag tgcctggggg ggggggggaa 6720

atggaagtcc cgccctttcc cttttttttt tctccctttt ggttgtgaga catggtttct 6780
ctgcgtagcc ctggctgctc tagagctcac tttaatgtag agtgggctgg ctatgaactc 6840
cgagatccac ctttctctgt gcctcaggca gtgcctggct gggttcattg cttttaactg 6900
ctgagcattt acacatttct ggtgtggaag catatggggg tcagcagatg ctcagcatat 6960
tgtatgtcgt atccaggcca ctgggctcct gctcatttct gacaataagt agacaaggtt 7020
ggcctttccc catgtactgg ggacctgttg catgatgcca ctgagtgact ggaagatcgg 7080
tgttcccatt ctctgtacct gaattgtaga tgtttattat tgtcacaagg tgtacatctc 7140
ttaatgtaac agtaattcca cttccacagg aagaggcacc gcaacttttg atgggacagc 7200
gatagccaat gcggttgtta aagaacttgc tgaaaccata aagtgtcgaa cactgttttc 7260
tacacactat cattcattag tagaagatta ttctaaaagt gtttgtgtgc gcctaggaca 7320
tatggtatgt ggaatcaacc agacattttt ttcttttggc ataaagtaaa cagaactgtg 7380
ataaaatgaa cttttctttc ttgaaggcat gcatggtaga aaatgaatgt gaggacccca 7440
gccaggagac tattactttc ctctataaat tcattaaggg agcttgtcct aagagctatg 7500
gctttaatgc agcaaggctt gctaatctcc cggaggaggt tattcagaag ggacacagaa 7560
aggcaagaga atttgagagg atgaatcagt cattacagct atttcggtaa ttatatcata 7620
atcttccttt aaagctgact ttaaagactt ggaaggctgg tacagagaca aaatgaaata 7680
tttttttcct ttttttaagg gaagtttgtc tggctactga aaagccgact ataaacggtg 7740
aagctatcca taggctgctg gccttgatta acggattgta gactacattg gaagctgagc 7800
tgacttttga aaaaggtggt aaattcagac aacattatga tctaataaac tttattttta 7860
aaaaatgacc atttttccat tttctttcta ggaaattaaa cccttttaat tcttatctac 7920
cttctacata atggttattg aatactccac aatatattaa gtctagatgt tatggtacat 7980
gcatacactt tcaggctgtt atacccactg tcaccaatac acataaatgg ggggaggg 8038

<210> 154
<211> 558
<212> PRT
<213> Rattus norvegicus

<220>
<223> Glypican

<400> 154
Met Glu Leu Arg Ala Arg Gly Trp Trp Leu Leu Cys Ala Ala Ala Ala
1               5                   10                  15

Leu Val Ala Cys Thr Arg Gly Asp Pro Ala Ser Lys Ser Arg Ser Cys
            20                  25                  30

Ser Glu Val Arg Gln Ile Tyr Gly Ala Lys Gly Phe Ser Leu Ser Asp
        35                  40                  45

Val Pro Gln Ala Glu Ile Ser Gly Glu His Leu Arg Ile Cys Pro Gln
    50                  55                  60

Gly Tyr Thr Cys Cys Thr Ser Glu Met Glu Glu Asn Leu Ala Asn His
65                  70                  75                  80

Ser Arg Met Glu Leu Glu Thr Ala Leu His Asp Ser Ser Arg Ala Leu
                85                  90                  95

Gln Ala Thr Leu Ala Thr Gln Leu His Gly Ile Asp Asp His Phe Gln
            100                 105                 110

Arg Leu Leu Asn Asp Ser Glu Arg Thr Leu Gln Asp Ala Phe Pro Gly
        115                 120                 125

Ala Phe Gly Asp Leu Tyr Thr Gln Asn Thr Arg Ala Phe Arg Asp Leu
    130                 135                 140

Tyr Ala Glu Leu Arg Leu Tyr Tyr Arg Gly Ala Asn Leu His Leu Glu

145                 150                 155                 160

Glu Thr Leu Ala Glu Phe Trp Ala Arg Leu Leu Glu Arg Leu Phe Lys
                165                 170                 175

Gln Leu His Pro Gln Leu Leu Leu Pro Asp Asp Tyr Leu Asp Cys Leu
            180                 185                 190

Gly Lys Gln Ala Glu Ala Leu Arg Pro Phe Gly Asp Ala Pro Arg Glu
        195                 200                 205

Leu Arg Leu Arg Ala Thr Arg Ala Phe Val Ala Ala Arg Ser Phe Val
    210                 215                 220

Gln Gly Leu Gly Val Ala Ser Asp Val Val Arg Lys Val Ala Gln Val
225                 230                 235                 240

Pro Leu Ala Pro Glu Cys Ser Arg Ala Val Met Lys Leu Val Tyr Cys
                245                 250                 255

Ala His Cys Arg Gly Val Pro Gly Ala Arg Pro Cys Pro Asp Tyr Cys
            260                 265                 270

Arg Asn Val Leu Lys Gly Cys Leu Ala Asn Gln Ala Asp Leu Asp Ala
        275                 280                 285

Glu Trp Arg Asn Leu Leu Asp Ser Met Val Leu Ile Thr Asp Lys Phe
    290                 295                 300

Trp Gly Pro Ser Gly Ala Glu Tyr Val Ile Gly Ser Val His Met Trp
305                 310                 315                 320

Leu Ala Glu Ala Ile Asn Ala Leu Gln Asp Asn Lys Asp Thr Leu Thr
                325                 330                 335

Ala Lys Val Ile Gln Gly Cys Gly Asn Pro Lys Val Asn Pro His Gly
            340                 345                 350

Ser Gly Pro Glu Glu Lys Arg Arg Arg Ala Lys Leu Ala Leu Gln Glu
        355                 360                 365

Lys Ser Ser Thr Gly Thr Leu Glu Lys Leu Val Ser Glu Ala Lys Ala
    370                 375                 380

Gln Leu Arg Asp Ile Gln Asp Tyr Trp Ile Ser Leu Pro Gly Thr Leu
385                 390                 395                 400

Cys Ser Glu Lys Met Ala Met Ser Pro Ala Ser Asp Asp Arg Cys Trp
                405                 410                 415

Asn Gly Ile Ser Lys Gly Arg Tyr Leu Pro Glu Val Met Gly Asp Gly
            420                 425                 430

Leu Ala Asn Gln Ile Asn Asn Pro Glu Val Glu Val Asp Ile Thr Lys
        435                 440                 445

Pro Asp Met Thr Ile Arg Gln Gln Ile Met Gln Leu Lys Ile Met Thr
    450                 455                 460

Asn Arg Leu Arg Gly Ala Tyr Gly Gly Asn Asp Val Asp Phe Gln Asp
465                 470                 475                 480

Ala Ser Asp Asp Gly Ser Gly Ser Gly Ser Gly Gly Gly Cys Pro Asp
                485                 490                 495

Asp Ala Cys Gly Arg Arg Val Ser Lys Lys Ser Ser Ser Ser Arg Thr
            500                 505                 510

Pro Leu Ile His Ala Leu Pro Gly Leu Ser Glu Gln Glu Gly Gln Lys
        515                 520                 525

Thr Ser Ala Ala Thr Arg Pro Glu Pro His Tyr Phe Phe Leu Leu Phe
    530                 535                 540

Leu Phe Thr Leu Val Leu Ala Ala Ala Arg Pro Arg Trp Arg
545                 550                 555


<210> 155
<211> 3509
<212> DNA
<213> Rattus norvegicus

<220>
<223> Glypican

<400> 155
cgcccgcctg agtggccgga cctcgcgcct cgcgcctcgc gtcccgcgct gcagccgcag 60
tcggcttttg ttgtctccgc ctcctcgtcg gtccccgact ctggaccacg agcggcgcgc 120
gctgggacct tggctctgcc cttcgcgcag actgggctga gcgggccggg cggccatcca 180
gggaggcgcg cacgggcggt cggggagccg cgggccccgc catggagctc cgggcccgag 240
gctggtggct gctgtgcgcg gccgccgcgc tagtcgcctg cacccgcggg gaccccgcca 300
gcaagagccg gagctgcagc gaagtccgcc agatctacgg ggctaagggc tttagcctga 360
gcgacgtgcc ccaggcagag atctcgggag agcacctgcg gatctgcccc cagggctaca 420
cctgctgcac cagtgagatg gaggagaacc tggccaacca cagccggatg gagctggaga 480
ccgcactcca cgacagcagc cgtgccctgc aggctacact ggccacccag ctgcatggta 540
tcgatgacca cttccagcgc ctgctgaatg actcggagcg tacactgcag gatgcttttc 600
ccggggcctt tggggacctg tacacgcaga acactcgggc cttccgggac ctgtatgctg 660
agctgcgtct ctactaccga ggggccaacc tacaccttga ggagacactg gccgagttct 720
gggcacggct gctggagcgt ctcttcaagc agctgcaccc ccagctgctg ctgcccgatg 780
actatctgga ctgcctgggc aagcaggcgg aggcactgcg gccgtttggg gatgcccctc 840
gagaactgcg cctgagggcc acccgtgctt ttgtggcggc acgatccttt gtgcagggcc 900
tgggtgtggc cagtgacgta gtccgaaagg tggcccaggt tcctctggcc ccagaatgtt 960
ctcgggctgt catgaagttg gtctactgtg cccattgccg gggagtccct ggtgcccggc 1020
cctgtcccga ctattgccga aatgtgctca aaggctgcct tgccaaccag gccgacctgg 1080
atgccgagtg gaggaacctc ctggactcca tggtgctcat cactgacaag ttctggggcc 1140
cgtcgggtgc ggagtatgtc attggcagtg tgcatatgtg gctggcggag gccatcaacg 1200
ccctccagga caacaaggac acactcacag ctaaggtcat ccagggctgc ggaaacccca 1260
aggtcaatcc ccatggctct gggcctgagg agaagcgtcg ccgtgccaaa ctggcactgc 1320
aggagaagtc ctccacaggt actctggaaa agctggtctc tgaggccaag gcccagctcc 1380
gagacattca ggactactgg atcagcctcc cagggacact gtgtagtgag aagatggcca 1440
tgagtcctgc cagcgatgac cgctgctgga atgggatttc caagggccgg tacctacctg 1500
aggtgatggg tgatgggctg gccaaccaga tcaacaaccc tgaagtggag gtggacatca 1560
ccaagccgga tatgaccatc cggcagcaga tcatgcagct caagatcatg accaaccgtt 1620
tacgtggcgc ctacggtgga aatgatgtgg acttccagga tgccagtgat gacggtagtg 1680
gttccggcag cggtggcgga tgcccagatg acgcctgtgg tcggagggtc agcaagaaga 1740
gctccagctc ccggaccccc ttgatccatg ccctccccgg cttgtcagaa caggagggac 1800
agaagacctc ggccgccact cgcccagagc ctcactactt ctttctgctc ttcctgttca 1860
ccttggtcct tgctgcagcc aggcccaggt ggcggtaact gccccctagc ccaaaggact 1920
gtcttggcca aaacatgcaa cagaccatat ttacttccct tggccttcga ggcccagggc 1980
aggataagga gacagtagct ctgagtgctg gggcagggcg catggggtcc tggccttcct 2040
gggtctggcc acgcctgtca ccctagcttc tagttgttgt atcaggtcag ctgcgagcca 2100

gtgtccccaa aagccatgtt tttcagggac ctcaggggca cctctggctg cacactcctc 2160
ccctaccctc ctgtaccacc ccagaagctc acgaggtcac ccagaagggc ggttattagc 2220
tacaacccat cggagacctc aagtgagtct gtgtcttcct ctcctgcctc ttcccggtgg 2280
ggactcccca ccagacccca tgggacacag atgtcagaaa ttgaggccca tcccgcagct 2340
ccccaggaag cctggaaggg atgccagtat gtcgctgacc aggctctggc agggcctaca 2400
agtttatgca tgataccttc tcctcagaag aagctctgca ggcaggatcc caacacacac 2460
cagaccagga ccctgtgcca ctgtggagct cagtgaatct ggttctcaaa gaagacccac 2520
tgtggggttc ctctagtgtg acataggtga ggtggcagct ggcaaggccg cactaggctg 2580
ccacactgtt tgccccctca gatgggctca taaagactgg gcttgagggt ccacatgaag 2640
agcctcactt caggggaata gccggccacg tctagcccct atcccactta gaaaggtcac 2700
ctggccacgg tgatgctggg tggctgatga aacttaagct cagggtcagt gggacctggc 2760
actgcaggtc aagaggacgc ctgggcccct tcctgaccca ggcagctctg caggagctgg 2820
gggagcattg acagtcaagg gcttttatag acatacacat tcagaccctc ggtgtccttg 2880
tccactgagt gctgtatctc atcgtatctc aatcttcatt gacagcactg gagaggctcg 2940
gggcaccact tggagccttg tatcccgcag gcttgagacc tgggggcacc tggcttacca 3000
aggttggcaa ggctccatct tcccttccag gggcttgggg atgctaagtt gctgtatcca 3060
ggaagacagt agtcctcaca tgcagaaggc ctgggaaaag gctgcttgtc gctttttttt 3120
tttcctttct ttttgtcact gggttagaga ggctcccgcc agcacagcac caaggcctgc 3180
ccagtagtca ggtctcctgg tccaggatgg gtgtgctctg tagttggtct gtgggtttct 3240
taggcccatg ccctgagcac attcacccag ccacatccct gctagtgaca ctcaggcagg 3300
ggcactggga accagacctg ggctctggca tcaacgagtg cctaggtgtc aggacagcgg 3360
cacccatcct gtacagggca gccaggatag tggccaagct actgtgtcct ttctccatga 3420
ggctccctgt cactcggtgc cagtgggtaa tgtgtgttct ttgagtcctt atatgaataa 3480
aaggctggag acctaccaaa aaaaaaaaa 3509

<210> 156
<211> 394
<212> PRT
<213> Mus musculus

<220>
<223> Cytoplasmic protein Ndr1

<400> 156
Met Ser Arg Glu Leu His Asp Val Asp Leu Ala Glu Val Lys Pro Leu
1               5                   10                  15

Val Glu Lys Gly Glu Ser Ile Thr Gly Leu Leu Gln Glu Phe Asp Val
            20                  25                  30

Gln Glu Gln Asp Ile Glu Thr Leu His Gly Ser Leu His Val Thr Leu
        35                  40                  45

Cys Gly Thr Pro Lys Gly Asn Arg Pro Val Ile Leu Thr Tyr His Asp
    50                  55                  60

Ile Gly Met Asn His Lys Thr Cys Tyr Asn Pro Leu Phe Asn Ser Glu
65                  70                  75                  80

Asp Met Gln Glu Ile Thr Gln His Phe Ala Val Cys His Val Asp Ala
                85                  90                  95

Pro Gly Gln Gln Asp Gly Ala Pro Ser Phe Pro Val Gly Tyr Met Tyr
            100                 105                 110

Pro Ser Met Asp Gln Leu Ala Glu Met Leu Pro Gly Val Leu His Gln
        115                 120                 125

Phe Gly Leu Lys Ser Val Ile Gly Met Gly Thr Gly Ala Gly Ala Tyr
    130                 135                 140

Ile Leu Thr Arg Phe Ala Leu Asn Asn Pro Glu Met Val Glu Gly Leu
145                 150                 155                 160

Val Leu Met Asn Val Asn Pro Cys Ala Glu Gly Trp Met Asp Trp Ala
                165                 170                 175

Ala Ser Lys Ile Ser Gly Trp Thr Gln Ala Leu Pro Asp Met Val Val
            180                 185                 190

Ser His Leu Phe Gly Lys Glu Glu Ile His Asn Asn Val Glu Val Val
        195                 200                 205

His Thr Tyr Arg Gln His Ile Leu Asn Asp Met Asn Pro Ser Asn Leu
    210                 215                 220

His Leu Phe Ile Ser Ala Tyr Asn Ser Arg Arg Asp Leu Glu Ile Glu
225                 230                 235                 240

Arg Pro Met Pro Gly Thr His Thr Val Thr Leu Gln Cys Pro Ala Leu
                245                 250                 255

Leu Val Val Gly Asp Asn Ser Pro Ala Val Asp Ala Val Val Glu Cys
            260                 265                 270

Asn Ser Lys Leu Asp Pro Thr Lys Thr Thr Leu Leu Lys Met Ala Asp
        275                 280                 285

Cys Gly Gly Leu Pro Gln Ile Ser Gln Pro Ala Lys Leu Ala Glu Ala
    290                 295                 300

Phe Lys Tyr Phe Val Gln Gly Met Gly Tyr Met Pro Ser Ala Ser Met
305                 310                 315                 320

Thr Arg Leu Met Arg Ser Arg Thr Ala Ser Gly Ser Ser Val Thr Ser
                325                 330                 335

Leu Glu Gly Thr Arg Ser Arg Ser His Thr Ser Glu Gly Pro Arg Ser
            340                 345                 350

Arg Ser His Thr Ser Glu Gly Ser Arg Ser Arg Ser His Thr Ser Glu
        355                 360                 365

Asp Ala Arg Leu Asn Ile Thr Pro Asn Ser Gly Ala Thr Gly Asn Asn
    370                 375                 380

Ala Gly Pro Lys Ser Met Glu Val Ser Cys
385                 390

<210> 157
<211> 2905
<212> DNA
<213> Mus musculus

<220>
<223> Cytoplasmic protein Ndr1

<400> 157
aaacaaacct tgccaggcta ccctctcgcg ttgcaggcag attgctcacc catcttcgtc 60
tctctcttca cctcccgcgg ggcgctgcac ctccaacctt gtggttggct cctttccgtg 120

cttgcttgct cattaggtgt gtgatagcac ggaaatgtcc cgagagctac atgacgtgga 180
cctcgctgag gtgaagcctc tggtggaaaa aggggagagc atcactggtc tcctgcaaga 240
gttcgatgtt caggagcagg atattgagac cctacatggc tccctgcatg tcaccctgtg 300
tgggaccccc aagggcaacc gtcctgtcat cctcacgtat cacgacatcg gcatgaacca 360
caagacctgc tacaaccccc tcttcaactc tgaggacatg caggagatca cacaacattt 420
tgctgtctgc catgtggatg cccctggcca acaggatggt gccccttcct tcccagtggg 480
gtacatgtac ccctcaatgg atcagttggc tgaaatgctt cctggagtcc ttcaccagtt 540
tgggctcaag agtgtcattg gcatggggac aggagctggc gcctacatcc tgacccgctt 600
cgcactcaac aaccctgaga tggtagaggg tctcgtgctc atgaatgtga acccctgtgc 660
tgaaggctgg atggactggg ctgcctctaa gatctcagga tggacccaag ccctgcctga 720
catggtggtg tcccacctct tcggcaagga ggagatacac aacaacgtgg aggtagtgca 780
cacgtaccgc cagcacatcc tcaacgacat gaacccgagc aacctacact tattcatcag 840
cgcctacaac agccggagag acctggagat tgagcggcca atgcctggaa cccacactgt 900
cactttgcag tgccctgctc tgctggtagt tggagacaac tctcctgccg tggatgctgt 960
ggtggaatgc aattctaaat tggacccaac gaagaccacc ctgctcaaga tggcagactg 1020
tggaggcctt cctcagatct ctcagcccgc caagcttgct gaggccttca agtactttgt 1080
gcagggcatg ggatacatgc cttctgccag catgactcgc ctgatgcggt cccgcacagc 1140
ctctggctcc agtgtcacat ccctggaggg cacccgtagc cgctcccata ccagcgaggg 1200
cccccgaagc cgctcgcaca ccagtgaggg gtcccgtagc cgctcccata ccagtgagga 1260
tgcccgtctc aacatcaccc ccaactcggg tgccaccgga aacaatgccg ggcccaagtc 1320
catggaggtg tcctgctaag caatctgtgc atcagtcacc ctggactggt ggcctctccc 1380
ttagccccca acccttgcct gccaccctac gctaatgcgg tattaatcta aagctgcttt 1440
tgtaagaatg aactctggcg gtggcagacc caggctgttc tgactgcctc ctgggaccag 1500
ggtgggtggt ggtggaccaa aggcaagaag cagttcagcg tactgttctg ctaacaagtg 1560
gcctggtgga ccctctctct accctcagac accaaactgc caaaactaag aaatagatag 1620
ctgtaacact gcctggctcc aagcccaggc tgatccagca ttctggttca taggcgtttt 1680
gccaacctgc ctacttcctg tttcctcccc actaaactca ttcctggaaa caaacttctg 1740
ttttgaaagg agagagagag gcaggaaagt tgggcgcctt attttaagta gagaaaccaa 1800
gaaaggaaga gaaaaaaagg cagggtctag ggcaggtaga ggaaaaacac aacaagtcta 1860
ctaggctcac ttgaaggaat tgattttaaa gttctggacg ctgaaaccca caacctgttt 1920
ttctggcaac cttttaacgt gcaagaaaaa ctggtaacaa accccaggct ttatagagaa 1980
agtgatccag caaatgtggg gcatcagtga tttaagactc ttgctatgca gtctactgta 2040
ccccgctccc attccacagt gaatgagtgg atgacacctc tgtccctcgg gaagatggtt 2100
ttgatttgtt gttgcattct ttgaggcaga gggagaaccc agcaggtgac aggggtcctt 2160
gtctgggatg agtctttagg atgggaaggg tggggataga atggccctgg gattttgagg 2220
actcatggta gggccaagtg ctagaacctg gtgggtgtga tttcattgat ctggaatctt 2280
gtgtgactga gtcacacctt gtctcccata attgatgtct ggcattgatt tgaaacttga 2340
tagctgaatg tctcttaact ggccatcgct ggttcttcag agagaaccgt atgttctagt 2400
gggggaagga ctggttttgg gttctgtaca ttccacggga acttgcttcc ctctcctgcc 2460
cctcctgctg actgggtttg tagcattggc ctcttaagca atgccagcct aatgcccatg 2520
ctccatacta ctgccttttg attcttactg tggctaacct tgaacctttc ctataggaaa 2580
tgtgatcaga tcagagacac ttgatcgttg ggagtcagcc ctcaaggcct ggcgacttcc 2640
aggatcttgg agttgctaga ggaaaagtag ttatgcccta taggcggcat gagagacaag 2700
ggatcagttt acctgccaac tcctaccgtg gaggaggagt tccactgacc ttgtgtagaa 2760
agatcaggaa tccaggccag ggtgattgaa tgctgacaag aaatcactgt atatgtctct 2820
ccagggcagt ttcttaataa gatgtttgta tttatttcca gaccaataaa tttgtaactt 2880
tgcccaaaaa aaaaaaaaaa aaaaa 2905

<210> 158
<211> 143
<212> PRT
<213> Rattus norvegicus

<220>
<223> Ribosomal protein S23

<400> 158


<210> 159
<211> 506
<212> DNA
<213> Rattus norvegicus

<220>
<223> Ribosomal protein S23

<400> 159
ctttctctcg ggcccgtgcg ccggcaagat gggtaagtgt cgtggtctcc gtactgcccg 60
gaagctccgc agtcaccgac gggaccagaa gtggcatgat aagcagtaca agaaagccca 120
cttgggcaca gccctgaagg ccaatccgtt tgggggtgcc tctcacgcaa agggaattgt 180
gctggaaaaa gtaggggttg aagccaaaca gccaaattct gccatccgga agtgtgtcag 240
ggtgcagctc attaagaacg gcaagaagat cacagcgttc gtgcccaatg acggttgctt 300
gaacttcatt gaggaaaatg atgaagttct ggttgctgga tttggtcgca aaggccatgc 360
cgtgggtgat attcctggag tccgctttaa ggtggttaaa gtagccaatg tgtctctttt 420
ggctctgtac aaaggcaaga aggaaagacc aagatcataa aattttgaca atggaaacag 480
aataataaat tttatatcca gaaaaa                                       506


<210> 160
<211> 145
<212> PRT
<213> Rattus norvegicus

<220>
<223> Insulinoma rig

<400> 160


<210> 161
<211> 487
<212> DNA
<213> Rattus norvegicus

<220>
<223> Insulinoma rig

<400> 161
tttttcccag cagccgccaa gatggccgaa gtggagcaga agaaaaagcg aaccttccgc 60
aagttcacct accgtggcgt ggacctcgac cagctgctgg acatgtccta tgagcagctg 120
atgcagttgt acagcgcccg gcagagacgg cgcctgaacc gaggcctgcg gaggaagcag 180
cactcactgc tcaagcgcct gaggaaggcc aagaaggagg cgccacccat ggagaagccg 240
gaggtcgtga agacccacct tagggacatg atcattctgc ccgagatggt cggcagcatg 300
gtgggtgtgt acaacggcaa gaccttcaac caggtggaga tcaaacccga gatgatcggc 360
cactacctgg gcgagttctc catcacctac aagcctgtga agcacggccg gcccggcatt 420
ggtgccaccc actcctcccg atttatcccc ctcaagtagt ggggacaata aagactcgtt 480
ttcagcc                                                           487


<210> 162
<211> 226
<212> PRT
<213> Rattus norvegicus

<220>
<223> CD9; cell surface protein

<400> 162
Met Pro Val Lys Gly Gly Ser Lys Cys Ile Lys Tyr Leu Leu Phe Gly
  1               5                  10                  15

Phe Asn Phe Ile Phe Trp Leu Ala Gly Ile Ala Val Leu Ala Ile Gly
             20                  25                  30

Leu Trp Leu Arg Phe Asp Ser Gln Thr Lys Ser Ile Phe Glu Gln Glu
         35                  40                  45

Thr Asn His Ser Ser Phe Tyr Thr Gly Val Tyr Ile Leu Ile Gly Ala
    50                  55                  60

Gly Ala Leu Met Met Leu Val Gly Phe Leu Gly Cys Cys Gly Ala Val
65                  70                  75                  80

Gln Glu Ser Gln Cys Met Leu Gly Leu Phe Phe Gly Phe Leu Leu Val
                85                  90                  95

Ile Phe Ala Ile Glu Ile Ala Ala Ala Val Trp Gly Tyr Thr His Lys
            100                 105                 110

Asp Glu Val Ile Lys Glu Leu Gln Glu Phe Tyr Lys Asp Thr Tyr Gln
        115                 120                 125

Lys Leu Arg Asn Lys Asp Glu Pro Gln Arg Glu Thr Leu Lys Ala Ile
    130                 135                 140

His Met Ala Leu Asn Cys Cys Gly Ile Ala Gly Gly Val Glu Gln Phe
145                 150                 155                 160

Ile Ser Asp Ile Cys Pro Lys Lys Gln Val Leu Glu Ser Phe Gln Val
                165                 170                 175

Lys Ser Cys Pro Asp Ala Ile Asp Glu Val Phe His Ser Lys Phe His
            180                 185                 190

Ile Ile Gly Ala Val Gly Ile Gly Ile Ala Val Val Met Ile Phe Gly
        195                 200                 205

Met Ile Phe Ser Met Ile Leu Cys Cys Ala Ile Arg Arg Ser Arg Glu
    210                 215                 220

Met Val
225


<210> 163
<211> 678
<212> DNA
<213> Rattus norvegicus

<220>
<223> CD9; cell surface protein

<400> 163
atgccggtca aaggaggtag caagtgcatc aaatacctgc tctttggatt taacttcatc 60
ttctggctcg ccggcatcgc agtgcttgcc attggactat ggctgcggtt cgactctcag 120
accaagagca tcttcgagca agaaactaat cattccagct tctacacagg agtgtacatt 180
ctgattggag ctggggccct catgatgcta gttggtttcc tgggctgctg tggagctgta 240
caagagtcac agtgcatgct gggattgttc ttcggattcc tcttggtgat attcgccatt 300
gagatcgccg cagccgtctg gggctatacc cacaaggacg aggtgattaa agaactccag 360
gagttttaca aggacaccta ccaaaagtta aggaacaagg atgagcccca gcgggaaaca 420
ctcaaagcca tccatatggc gttgaactgc tgtggcatag ctggtggtgt ggagcagttt 480
atctcggaca tctgccccaa aaagcaggtt ctggaatcct tccaggttaa gtcctgcccg 540
gatgccatcg atgaggtctt ccacagcaag ttccacatca ttggagcagt gggcatcggt 600
atcgccgtgg tgatgatctt cggtatgatt ttcagcatga ttctgtgctg cgccatccgc 660
aggagccgag aaatggtc                                                678

<210> 164
<211> 379
<212> PRT
<213> Rattus norvegicus

<220>
<223> Foocen-m2 reticulon

<400> 164
Met Glu Asp Ile Asp Gln Ser Ser Leu Val Ser Ser Ser Thr Asp Ser
1               5                   10                  15

Pro Pro Arg Pro Pro Pro Ala Phe Lys Tyr Gln Phe Val Thr Glu Pro
            20                  25                  30

Glu Asp Glu Glu Asp Glu Glu Glu Glu Glu Asp Glu Glu Glu Asp Asp
        35                  40                  45

Glu Asp Leu Glu Glu Leu Glu Val Leu Glu Arg Lys Pro Ala Ala Gly
    50                  55                  60

Leu Ser Ala Ala Ala Val Pro Pro Ala Ala Ala Ala Pro Leu Leu Asp
65                  70                  75                  80

Phe Ser Ser Asp Ser Val Pro Pro Ala Pro Arg Gly Pro Leu Pro Ala
                85                  90                  95

Ala Pro Pro Ala Ala Pro Glu Arg Gln Pro Ser Trp Glu Arg Ser Pro
            100                 105                 110

Ala Ala Pro Ala Pro Ser Leu Pro Pro Ala Ala Ala Val Leu Pro Ser
        115                 120                 125

Lys Leu Pro Glu Asp Asp Glu Pro Pro Ala Arg Pro Pro Pro Pro Pro
    130                 135                 140

Pro Ala Gly Ala Ser Pro Leu Ala Glu Pro Ala Ala Pro Pro Ser Thr
145                 150                 155                 160

Pro Ala Ala Pro Lys Arg Arg Gly Ser Gly Ser Val Asp Glu Thr Leu
                165                 170                 175

Phe Ala Leu Pro Ala Ala Ser Glu Pro Val Ile Pro Ser Ser Ala Val
            180                 185                 190

Val Asp Leu Leu Tyr Trp Arg Asp Ile Lys Lys Thr Gly Val Val Phe
        195                 200                 205

Gly Ala Ser Leu Phe Leu Leu Leu Ser Leu Thr Val Phe Ser Ile Val
    210                 215                 220

Ser Val Thr Ala Tyr Ile Ala Leu Ala Leu Leu Ser Val Thr Ile Ser
225                 230                 235                 240

Phe Arg Ile Tyr Lys Gly Val Ile Gln Ala Ile Gln Lys Ser Asp Glu
                245                 250                 255

Gly His Pro Phe Arg Ala Tyr Leu Glu Ser Glu Val Ala Ile Ser Glu
            260                 265                 270

Glu Leu Val Gln Lys Tyr Ser Asn Ser Ala Leu Gly His Val Asn Ser

275                 280                 285

Thr Ile Lys Glu Leu Arg Arg Leu Phe Leu Val Asp Asp Leu Val Asp
    290                 295                 300

Ser Leu Lys Phe Ala Val Leu Met Trp Val Phe Thr Tyr Val Gly Ala
305                 310                 315                 320

Leu Phe Asn Gly Leu Thr Leu Leu Ile Leu Ala Leu Ile Ser Leu Phe
                325                 330                 335

Ser Ile Pro Val Ile Tyr Glu Arg His Gln Val Gln Ile Asp His Tyr
            340                 345                 350

Leu Gly Leu Ala Asn Lys Ser Val Lys Asp Ala Met Ala Lys Ile Gln
        355                 360                 365

Ala Lys Ile Pro Gly Leu Lys Arg Lys Ala Asp
    370                 375

<210> 165
<211> 2782
<212> DNA
<213> Rattus norvegicus

<220>
<223> Foocen-m2 reticulon

<400> 165
gccatggtaa tcctgctcag tacgagagga accgcaggtt cagacatttg gtgtatgtgc 60
ttggctgagg agccaatggg gcgaagctac catctgtggg attatgactg aacgcctcta 120
agtcagaatc ccgcccaggc ggaacgatac ggcagcgccg aaggagcctc ggttggcccc 180
ggatagccgg ctccccgtcc gtccccgtcc ggcgggtccc cgcctcgtcg cccccccggg 240
tgcggggcgg gtccccccgc cgggcgtcgg gaccggggtc cggtgcggag agccattcgt 300
cccgggaaac ggggtgcggc cggaaagggg gccgccctct cgcccgtcac gcttaacgca 360
cgttcgtgtg gaacctggcg ctaaaccatt cgtagacgac ctgcttctgg gtcggggttt 420
cgtacgtagc agagcagctc cctcgctgcg atctattgaa agcggcggcg gcggctgcag 480
cctgggacag ggcgggtggc acatctcgat cgcgaaggca ggagaagcag tctcattgtt 540
ccgggagccg tcgcctctgc aggttcttcg gctcggctcg gcacgactcg gcctgcctgg 600
cccctgccag tcttgcccaa cccccacaac cgcccgcgac tctgaggaga agcggccctg 660
cggcggctgt agctgcagca tcgtcggcga cccgccagcc atggaagaca tagaccagtc 720
gtcgctggtc tcctcgtcca cggacagccc gccccggcct ccgcccgcct tcaagtacca 780
gttcgtgacg gagcccgagg acgaggagga cgaggaggag gaggaggacg aggaggagga 840
cgacgaggac ctagaggaac tggaggtgct ggagaggaag cccgcagccg ggctgtccgc 900
agctgcggtg ccgcccgccg ccgccgcgcc gctgctggac ttcagcagcg actcggtgcc 960
ccccgcgccc cgcgggccgc tgccggccgc gccccctgcc gctcctgaga ggcagccatc 1020
ctgggaacgc agccccgcgg cgcccgcgcc atccctgccg cccgctgccg cagtcctgcc 1080
ctccaagctc ccagaggacg acgagcctcc ggcgaggccc ccgcctccgc cgccagccgg 1140
cgcgagcccc ctggcggagc ccgccgcgcc cccttccacg ccggccgcgc ccaagcgcag 1200
gggctccggc tcagtggatg agaccctttt tgctcttcct gctgcatctg aacctgtgat 1260
accctcctct gcagttgttg acctcctcta ctggagagac attaagaaga ctggagtggt 1320
gtttggtgcc agcttattcc tgctgctgtc tctgacagtg ttcagcattg tcagtgtaac 1380
ggcctacatt gccttggccc tgctctcggt gactatcagc tttaggatat ataagggcgt 1440
gatccaggct atccagaaat cagatgaagg ccacccattc agggcatatt tagaatctga 1500
agttgctata tcagaggaat tggttcagaa atacagtaat tctgctcttg gtcatgtgaa 1560
cagcacaata aaagaactga ggcggctttt cttagttgat gatttagttg attccctgaa 1620
gtttgcagtg ttgatgtggg tgtttactta tgttggtgcc ttgttcaatg gtctgacact 1680
actgatttta gctctgatct cactcttcag tattcctgtt atttatgaac ggcatcaggt 1740
gcagatagat cattatctag gacttgcaaa caagagtgtt aaggatgcca tggccaaaat 1800
ccaagcaaaa atccctggat tgaagcgcaa agcagattga aaaagcccca aacagaagtt 1860

catctttaaa ggggacactc acttgattac gggggtggga gggtcagggg tgagcccttg 1920
gtggccgtgc ggtttcagct ctttattttt agcagtgcac tgtttgagga aaaattacct 1980
gtcttgactt cctgtgttta tcatcttaag tattgtaagc tgctgtgtat ggatctcatt 2040
gtagtcacac ttgtcttccc caatgaggcg cctggtgaat aaaggactcg gggaaagctg 2100
tgcattgtat ctgctgcagg gtagtctagc tgtatgcaga gagttgtaaa gaaggcaaat 2160
ctgggggcag ggaaaaccct tttcacagtg tactgtgttt ggtcagtgta aaactgatgc 2220
agatttttct gaaatgaaat gtttagatga gagcatacta ctaaagcaga gtggaaaact 2280
ctgtctttat ggtgtgttct aggtgtattg tgaatttact gttatattgc caatataagt 2340
aaatatagac ctaatctata tatagtgttt cacaaagctt agatctttaa ccttgcagct 2400
gccccacagt gcttgacctc tgagtcattg gttatgcagt gtagtcccaa gcacataaac 2460
taggaagaga aatgtatttg taggagtgct acctaccacc tgttttcaag aaaatataga 2520
actccaacaa aaatatagaa tgtcatttca aagacttact gtatgtatag ttaattttgt 2580
cacagactct gaaattctat ggactgaatt tcatgcttcc aaatgtttgc agttatcaaa 2640
cattgttatg caagaaatca taaaatgaag acttatacca ttgtggttta agccgtactg 2700
aattatctgt ggaatgcatt gtgaactgta aaagcaaagt atcaataaag cttatagact 2760
taaaaaaaaa aaaaaaaaaa aa 2782

<210> 166
<211> 447
<212> PRT
<213> Rattus norvegicus

<220>
<223> Clusterin

<400> 166
Met Lys Ile Leu Leu Leu Cys Val Ala Leu Leu Leu Thr Trp Asp Asn
1               5                   10                  15

Gly Met Val Leu Gly Glu Gln Glu Phe Ser Asp Asn Glu Leu Gln Glu
            20                  25                  30

Leu Ser Thr Gln Gly Ser Arg Tyr Val Asn Lys Glu Ile Gln Asn Ala
        35                  40                  45

Val Gln Gly Val Lys His Ile Lys Thr Leu Ile Glu Lys Thr Asn Ala
    50                  55                  60

Glu Arg Lys Ser Leu Leu Asn Ser Leu Glu Glu Ala Lys Lys Lys Lys
65                  70                  75                  80

Glu Gly Ala Leu Asp Asp Thr Arg Asp Ser Glu Met Lys Leu Lys Ala
                85                  90                  95

Phe Pro Glu Val Cys Asn Glu Thr Met Met Ala Leu Trp Glu Glu Cys
            100                 105                 110

Lys Pro Cys Leu Lys His Thr Cys Met Lys Phe Tyr Ala Arg Val Cys
        115                 120                 125

Arg Ser Gly Ser Gly Leu Val Gly Arg Gln Leu Glu Glu Phe Leu Asn
    130                 135                 140

Gln Ser Ser Pro Phe Tyr Phe Trp Met Asn Gly Asp Arg Ile Asp Ser
145                 150                 155                 160

Leu Leu Glu Ser Asp Arg Gln Gln Ser Gln Val Leu Asp Ala Met Gln
                165                 170                 175

Asp Ser Phe Thr Arg Ala Ser Gly Ile Ile His Thr Leu Phe Gln Asp

180                 185                 190

Arg Phe Phe Thr His Glu Pro Gln Asp Ile His His Phe Ser Pro Met
        195                 200                 205

Gly Phe Pro His Lys Arg Pro His Phe Leu Tyr Pro Lys Ser Arg Leu
    210                 215                 220

Val Arg Ser Leu Met Pro Leu Ser His Tyr Gly Pro Leu Ser Phe His
225                 230                 235                 240

Asn Met Phe Gln Pro Phe Phe Asp Met Ile His Gln Ala Gln Gln Ala
                245                 250                 255

Met Asp Val Gln Leu His Ser Pro Ala Leu Gln Phe Pro Asp Val Asp
            260                 265                 270

Phe Leu Lys Glu Gly Glu Asp Asp Pro Thr Val Cys Lys Glu Ile Arg
        275                 280                 285

His Asn Ser Thr Gly Cys Leu Lys Met Lys Gly Gln Cys Glu Lys Cys
    290                 295                 300

Gln Glu Ile Leu Ser Val Asp Cys Ser Thr Asn Asn Pro Ala Gln Ala
305                 310                 315                 320

Asn Leu Arg Gln Glu Leu Asn Asp Ser Leu Gln Val Ala Glu Arg Leu
                325                 330                 335

Thr Gln Gln Tyr Asn Glu Leu Leu His Ser Leu Gln Ser Lys Met Leu
            340                 345                 350

Asn Thr Ser Ser Leu Leu Glu Gln Leu Asn Asp Gln Phe Thr Trp Val
        355                 360                 365

Ser Gln Leu Ala Asn Leu Thr Gln Gly Asp Asp Gln Tyr Leu Arg Val
    370                 375                 380

Ser Thr Val Thr Thr His Ser Ser Asp Ser Glu Val Pro Ser Arg Val
385                 390                 395                 400

Thr Glu Val Val Val Lys Leu Phe Asp Ser Asp Pro Ile Thr Val Val
                405                 410                 415

Leu Pro Glu Glu Val Ser Lys Asp Asn Pro Lys Phe Met Asp Thr Val
            420                 425                 430

Ala Glu Lys Ala Leu Gln Glu Tyr Arg Arg Lys Ser Arg Met Glu
        435                 440                 445

<210> 167
<211> 1857
<212> DNA
<213> Rattus norvegicus

<220>
<223> Clusterin

<400> 167
ctcaagctca cactggctgg acttcctcgc catgacagtc tgtacctcta actgatccca 60

gggatgatac cacctacatt tggggtggtt cttctcgcct cagttaaacc tctctgggag 120
caccatcaca gacacccaca gaagtttgtt ccctagatga ttctaggtcc tgtggagttg 180
acaagattga ccatcacgct ctcagcaatc gggtgaagta aacaccaccg ttgtctccat 240
ggaaatgctt aactacggct tgctagtaag gactccagac tccaaagagg ccacaccatg 300
aagattctcc tgctgtgtgt ggcactgctg ctgacctggg acaatggcat ggtcctggga 360
gagcaggagt tctctgacaa tgagctccaa gaactgtcca ctcaaggaag taggtatgtt 420
aataaggaga ttcagaacgc cgtccagggg gtgaagcaca taaagaccct catagaaaaa 480
accaacgcag agcgcaagtc cctgctcaac agtttagagg aagccaaaaa gaagaaagag 540
ggtgctctag atgacaccag ggattctgaa atgaagctga aggctttccc ggaagtgtgt 600
aacgagacca tgatggccct ctgggaagag tgtaagccct gcctgaagca cacctgcatg 660
aagttctacg cacgcgtctg caggagcggc tcggggctgg ttggtcgcca gctagaggag 720
tttctgaacc agagctcacc cttctacttc tggatgaacg gggaccgcat cgactccctg 780
ctggagagtg accggcagca gagccaagtc ctagatgcta tgcaggacag cttcactcgg 840
gcgtctggca tcatacatac gcttttccag gaccggttct tcacccatga gccccaggac 900
atccaccatt tctcccccat gggcttccca cacaagcggc ctcatttctt gtaccccaag 960
tcccgcttgg tccgcagcct catgcctctc tcccactacg ggcctctgag cttccacaac 1020
atgttccagc ctttctttga tatgatacac caggctcaac aggccatgga cgtccagctc 1080
catagcccag ctttacagtt cccggatgtg gatttcttaa aagaaggtga agatgacccg 1140
acagtgtgca aggagatccg ccataactcc acaggatgcc tgaagatgaa gggccagtgt 1200
gagaagtgcc aagagatctt gtctgtggac tgttcgacca acaatcctgc ccaggctaac 1260
ctgcgccagg agctaaacga ctcgctccag gtggctgaga ggctgaccca gcagtacaac 1320
gagctgcttc attccctcca gtccaagatg ctcaacacct catccctgct ggaacagctg 1380
aacgaccagt tcacgtgggt gtcccagctg gctaacctca cacagggcga tgaccagtac 1440
cttcgggtct ccacagtgac aacccattct tctgactcag aagtcccctc tcgtgtcact 1500
gaggtggtgg tgaagctgtt tgactctgac cccatcacag tggtgttacc agaagaagtc 1560
tccaaggata accctaagtt tatggacaca gtggcagaga aagcgctaca ggaataccgc 1620
aggaaaagcc gcatggaatg agacagaagc atcagttttc tatatgtagg agtctcaagg 1680
agggaatctc ccagctttcc gaggttgctg cagaccccta gagaactcac atgtctccag 1740
cgcctaggcc tccaccccag cagcctctcc ttcctctggg ttctgtactc taatgcctgc 1800
acttgatgct ctgggaagaa ctgcttcccc cacgcaacta atccaataaa gcacctt 1857

<210> 168
<211> 374
<212> PRT
<213> Homo sapiens

<220>
<223> Speckle-type protein

<400> 168

<210> 169
<211> 1642
<212> DNA
<213> Homo sapiens

<220>
<223> Speckle-type protein

<400> 169
gaatcggcgg tcccgcaggt cccggatgtt gcggacagta tgaggcaagc gcagggggac 60
ggggaccagc agctgtcgcc gccgctctca gggtgaagag ggaacagaaa tctttgcccc 120
ctgactttgg aaatctcgtt taaccttcaa actggcgatg tcaagggttc caagtcctcc 180
acctccggca gaaatgtcga gtggccccgt agctgagagt tggtgctaca cacagatcaa 240
ggtagtgaaa ttctcctaca tgtggaccat caataacttt agcttttgcc gggaggaaat 300
gggtgaagtc attaaaagtt ctacattttc atcaggagca aatgataaac tgaaatggtg 360
tttgcgagta aaccccaaag ggttagatga agaaagcaaa gattacctgt cactttacct 420
gttactggtc agctgtccaa agagtgaagt tcgggcaaaa ttcaaattct ccatcctgaa 480
tgccaaggga gaagaaacca aagctatgga gagtcaacgg gcatataggt ttgtgcaagg 540
caaagactgg ggattcaaga aattcatccg tagagatttt cttttggatg aggccaacgg 600
gcttctccct gatgacaagc ttaccctctt ctgcgaggtg agtgttgtgc aagattctgt 660
caacatttct ggccagaata ccatgaacat ggtaaaggtt cctgagtgcc ggctggcaga 720
tgagttagga ggactgtggg agaattcccg gttcacagac tgctgcttgt gtgttgccgg 780
ccaggaattc caggctcaca aggctatctt agcagctcgt tctccggttt ttagtgccat 840
gtttgaacat gaaatggagg agagcaaaaa gaatcgagtt gaaatcaatg atgtggagcc 900
tgaagttttt aaggaaatga tgtgcttcat ttacacgggg aaggctccaa acctcgacaa 960
aatggctgat gatttgctgg cagctgctga caagtatgcc ctggagcgct taaaggtcat 1020
gtgtgaggat gccctctgca gtaacctgtc cgtggagaac gctgcagaaa ttctcatcct 1080
ggccgacctc cacagtgcag atcagttgaa aactcaggca gtggatttca tcaactatca 1140
tgcttcggat gtcttggaga cctctgggtg gaagtcaatg gtggtgtcac atccccactt 1200
ggtggctgag gcataccgct ctctggcttc agcacagtgc ccttttctgg gacccccacg 1260
caaacgcctg aagcaatcct aagatcctgc ttgttgtaag actccgttta atttccagaa 1320
gcagcagcca ctgttgctgc cactgaccac caggtagaca gcgcaatctg tggagctttt 1380
actctgttgt gaggggaaga gactgcattg tggccccaga cttttaaaac agcactaaat 1440
aacttggggg aaacgggggg agggaaaatg aaatgaaaac cctgttgctg cgtcactgtg 1500
ttccctttgg cctgtctgag tttgatactg tggggattca gtttaggcgc tggcccgagg 1560
atatcccagc ggtggtactt cggagacacc tgtctgcatc tgactgagca gaacaaatcg 1620
tcaggtgcct ggagcaaaaa gg 1642

<210> 170
<211> 47
<212> PRT
<213> Homo sapiens

<220>
<223> OCP2

<400> 170
Asn Glu Glu Lys Arg Thr Asp Ala Ile Pro Ala Trp Asp Gln Lys Phe
1               5                   10                  15

Leu Lys Ile Asp Pro Gly Thr Leu Phe Glu Val Ile Leu Ala Ala Asn
            20                  25                  30

Tyr Leu Asp Ile Lys Gly Leu Leu Asp Val Pro Cys Lys Thr Val
        35                  40                  45

<210> 171
<211> 143
<212> DNA
<213> Homo sapiens

<220>
<223> OCP2

<400> 171
agaatgaaga aaagcgaaca gatgctatcc ctgcttggga ccaaaaattc ctgaaaattg 60
acccaggaac actttttgaa gtcattttgg ctgcaaacta cttagacatc aaaggtttgc 120

ttgatgttcc atgcaagact gtt                                          143

<210> 172
<211> 619
<212> PRT
<213> Homo sapiens

<220>
<223> NS1 binding protein

<400> 172
Met Ile Pro Asn Gly Tyr Leu Met Phe Glu Asp Glu Asn Phe Ile Glu
1               5                   10                  15

Ser Ser Val Ala Lys Leu Asn Ala Leu Arg Lys Ser Gly Gln Phe Cys
            20                  25                  30

Asp Val Arg Leu Gln Val Cys Gly His Glu Met Leu Ala His Arg Ala
        35                  40                  45

Val Leu Ala Cys Cys Ser Pro Tyr Leu Phe Glu Ile Phe Asn Ser Asp
    50                  55                  60

Ser Asp Pro His Gly Ile Ser His Val Lys Phe Asp Asp Leu Asn Pro
65                  70                  75                  80

Glu Ala Val Glu Val Leu Leu Asn Tyr Ala Tyr Thr Ala Gln Leu Lys
                85                  90                  95

Ala Asp Lys Glu Leu Val Lys Asp Val Tyr Ser Ala Ala Lys Glu Leu
            100                 105                 110

Lys Met Asp Arg Val Lys Gln Val Cys Gly Asp Tyr Leu Leu Ser Arg
        115                 120                 125

Met Asp Val Thr Ser Cys Ile Ser Tyr Arg Asn Phe Ala Ser Cys Met
    130                 135                 140

Gly Asp Ser His Leu Leu Asn Lys Val Asp Ala Tyr Ile Gln Glu His
145                 150                 155                 160

Leu Leu Gln Ile Ser Glu Glu Glu Glu Phe Leu Lys Leu Pro Arg Leu
                165                 170                 175

Lys Leu Glu Val Met Leu Glu Asp Asn Val Cys Leu Pro Ser Asn Gly
            180                 185                 190

Lys Leu Tyr Thr Lys Val Ile Asn Trp Val Gln Arg Ser Ile Trp Glu
        195                 200                 205

Asn Gly Asp Ser Leu Glu Glu Leu Met Glu Glu Val Gln Thr Leu Tyr
    210                 215                 220

Tyr Ser Ala Asp His Lys Leu Leu Asp Gly Asn Leu Leu Asp Gly Gln
225                 230                 235                 240

Ala Glu Val Phe Gly Ser Asp Asp Asp His Ile Gln Phe Val Gln Lys
                245                 250                 255

Lys Pro Pro Arg Glu Asn Gly His Lys Gln Ile Ser Ser Ser Ser Thr

260                 265                 270

Gly Cys Leu Ser Ser Pro Asn Ala Thr Val Gln Ser Pro Lys His Glu
        275                 280                 285

Trp Lys Ile Val Ala Ser Glu Lys Thr Ser Asn Asn Thr Tyr Leu Cys
    290                 295                 300

Leu Ala Val Leu Asp Gly Ile Phe Cys Val Ile Phe Leu His Gly Arg
305                 310                 315                 320

Asn Ser Pro Gln Ser Ser Pro Thr Ser Thr Pro Lys Leu Ser Lys Ser
                325                 330                 335

Leu Ser Phe Glu Met Gln Gln Asp Glu Leu Ile Glu Lys Pro Met Ser
            340                 345                 350

Pro Met Gln Tyr Ala Arg Ser Gly Leu Gly Thr Ala Glu Met Asn Gly
        355                 360                 365

Lys Leu Ile Ala Ala Gly Gly Tyr Asn Arg Glu Glu Cys Leu Arg Thr
    370                 375                 380

Val Glu Cys Tyr Asn Pro His Thr Asp His Trp Ser Phe Leu Ala Pro
385                 390                 395                 400

Met Arg Thr Pro Arg Ala Arg Phe Gln Met Ala Val Leu Met Gly Gln
                405                 410                 415

Leu Tyr Val Val Gly Gly Ser Asn Gly His Ser Asp Asp Leu Ser Cys
            420                 425                 430

Gly Glu Met Tyr Asp Ser Asn Ile Asp Asp Trp Ile Pro Val Pro Glu
        435                 440                 445

Leu Arg Thr Asn Arg Cys Asn Ala Gly Val Cys Ala Leu Asn Gly Lys
    450                 455                 460

Leu Tyr Ile Val Gly Gly Ser Asp Pro Tyr Gly Gln Lys Gly Leu Lys
465                 470                 475                 480

Asn Cys Asp Val Phe Asp Pro Val Thr Lys Leu Trp Thr Ser Cys Ala
                485                 490                 495

Pro Leu Asn Ile Arg Arg His Gln Ser Ala Val Cys Glu Leu Gly Gly
            500                 505                 510

Tyr Leu Tyr Ile Ile Gly Gly Ala Glu Ser Trp Asn Cys Leu Asn Thr
        515                 520                 525

Val Glu Arg Tyr Asn Pro Glu Asn Asn Thr Trp Thr Leu Ile Ala Pro
    530                 535                 540

Met Asn Val Ala Arg Arg Gly Ala Gly Val Ala Val Leu Asn Gly Lys
545                 550                 555                 560

Leu Phe Val Cys Gly Gly Phe Asp Gly Ser His Ala Ile Ser Cys Val
                565                 570                 575

Glu Met Tyr Asp Pro Thr Arg Asn Glu Trp Lys Met Met Gly His Met
            580                 585                 590

Thr Ser Pro Arg Ser Asn Ala Gly Ile Ala Thr Val Gly Asn Thr Ile
        595                 600                 605

Tyr Ala Val Glu Asp Ser Met Ala Met Asn Phe
    610                 615


<210> 173
<211> 2752
<212> DNA
<213> Homo sapiens

<220>
<223> NS1 binding protein

<400> 173
agtgtctccc ggtcgcgcgt ggaggtcggt cgctcagagc tgctgggcgc agtttctccg 60
cctgctgctt cggcgcggct gtatcggcga gcgagcgagt tcccgcgagt tctcggtggc 120
gctccccctt cctttcagtc tccacggact ggcccctcgt ccttctactt gaccgctccc 180
gtcttctgcc gccttctggc gctttccgtt gggccgattc ccgcccgctt cctcctgctt 240
cccatcgaag ctctagaaat gaatgtttcc atctcttcag agatgaacca gattatgatg 300
catcattatc acagaagaaa ttcgtgtcta tagcttttaa ggacttgatt acatcatttt 360
caagcctgat agttttggaa tcaccattag agcttaagac acacctgcct tcatttcaac 420
cacctgtctt cataccctga cgaagtgcac cttttaacac tcctttgtcc ttggattact 480
taagagttcc cagaaataca tttgccacca acagagtagc caaatttata aggaaaaatg 540
attcccaatg gatatttgat gtttgaggat gaaaatttta ttgagtcttc tgttgccaaa 600
ttaaatgccc tgaggaaaag tggccagttc tgtgatgttc gacttcaggt ctgtggccat 660
gaaatgttag cacacagagc agtgctagct tgctgcagtc cctatttatt tgaaatcttt 720
aatagtgata gtgatcctca tggaatttct cacgttaaat ttgatgatct caatccagaa 780
gctgttgaag tcttgttgaa ttatgcctac actgctcagt tgaaagcaga taaggaattg 840
gtaaaagatg tttattctgc agcaaaagag ctgaagatgg atcgagtaaa gcaggtttgt 900
ggtgattatt tactgtctag aatggatgtt accagctgca tctcttaccg aaattttgca 960
agttgtatgg gagactccca tttgttgaat aaggttgatg cttatattca ggagcatttg 1020
ttacaaattt ctgaagagga ggagtttctt aagcttccaa ggctaaagtt ggaggtaatg 1080
cttgaagata atgtttgctt gcccagcaat ggcaaattat atacaaaggt aatcaactgg 1140
gtgcagcgta gcatctggga gaatggagac agtctggaag agctgatgga agaggttcaa 1200
accttgtact actcagctga tcacaagctg cttgatggga acctactaga tggacaggct 1260
gaggtgtttg gcagtgatga tgaccacatt cagtttgtgc agaaaaagcc accacgtgag 1320
aatggccata agcagataag tagcagttca actggatgtc tctcttctcc aaatgctaca 1380
gtacaaagcc ctaagcatga gtggaaaatc gttgcttcag aaaagacttc aaataacact 1440
tacttgtgcc tggctgtgct ggatggtata ttctgtgtca tttttcttca tgggagaaac 1500
agcccacaga gctcaccaac aagtactcca aaactaagta agagtttaag ctttgagatg 1560
caacaagatg agctaatcga aaagcccatg tctcctatgc agtacgcacg atctggtctg 1620
ggaacagcag agatgaatgg caaactcata gctgcaggtg gctataacag agaggaatgt 1680
cttcgaacag tcgaatgcta taatccacat acagatcact ggtcctttct tgctcccatg 1740
agaacaccaa gagcccgatt tcaaatggct gtactcatgg gccagctcta tgtggtaggt 1800
ggatcaaatg gccactcaga tgacctgagt tgtggagaga tgtatgattc aaacatagat 1860
gactggattc ctgttccaga attgagaact aaccgttgta atgcaggagt gtgtgctctg 1920
aatggaaagt tatacatcgt tggtggctct gatccatatg gtcaaaaagg actgaaaaat 1980
tgtgatgtat ttgatcctgt aacaaagttg tggacaagct gtgcccctct taacattcgg 2040
agacaccagt ctgcagtctg tgagcttggt ggttatttgt acataatcgg aggtgcagaa 2100
tcttggaatt gtctgaacac agtagaacga tacaatcctg aaaataatac ctggacttta 2160
attgcaccca tgaatgtggc taggcgagga gctggagtgg ctgttcttaa tggaaaactg 2220
tttgtatgtg gtggctttga tggttctcat gccatcagtt gtgtggaaat gtatgatcca 2280
actagaaatg aatggaagat gatgggacat atgacttcac caaggagcaa tgctgggatt 2340
gcaactgtag ggaacaccat ttatgcagtg gaggattcga tggcaatgaa tttctgaata 2400
cggcggaagt ctataacctt gagtcaaatg aatggagccc ctatacaaag attttccagt 2460
tttaacgggt ttaagaccct ctcaaactaa caggcttagt gatgtaatta tggttagcag 2520
aggtacactt gtgaataaag agggtgggtg gggatagatg ttgctaacag caacacaaag 2580
cttttgcata ttgcatacta ttaaacatgc tgtacatact ttttgggttt atttgggaag 2640

gaatgcaaag atgaaggtct gtttgtgtac ttttaagact ttggttattt tactttttgg 2700
aaaagataaa ccaagaattg attgggcaca tcaaaaaaaa aaaaaaaaaa aa 2752

<210> 174
<211> 451
<212> PRT
<213> Rattus norvegicus

<220>
<223> Alpha-tubulin

<400> 174


<210> 175
<211> 1617
<212> DNA
<213> Rattus norvegicus

<220>
<223> Alpha-tubulin

<400> 175
gcgctgtaag aagcaacacc tcctcctcgc ctccgccatc cacccggcag ccgcgaagca 60
gcaaccatgc gtgagtgtat ctccatccac gtcggccagg ctggtgtcca gatcggcaat 120
gcctgctggg agctctactg cctggaacat ggcatccagc ctgatggcca gatgccaagc 180
gacaagacca ttgggggagg agatgactcc ttcaacacct tcttcagtga gacaggagct 240
ggcaagcacg tgccccgggc ggtgttcgta gacctggaac ccacagttat tgatgaagtt 300
cgcactggca cctaccgcca gctcttccac ccagagcagc tcatcacagg caaggaagat 360
gctgccaata actatgcccg tggccactac accattggca aggagatcat tgaccttgtc 420
ttggacagaa ttcgcaagct ggctgaccag tgcacgggtc tccagggctt cttggttttc 480
cacagctttg gtgggggaac tggctctggg ttcacctccc tgctgatgga gaggctctct 540
gtcgactacg gaaagaagtc caagctggag ttctccattt acccagcccc ccaggtttcc 600
actgctgtgg ttgagcccta caattccatc ctcaccaccc acaccaccct ggagcactct 660
gattgtgcct tcatggtaga caatgaggcc atctatgaca tctgtcgtag aaacctcgac 720

attgagcgcc caacctacac taacttaaac aggttgatag gtcaaattgt gtcttccatc 780
actgcttccc tcagatttga tggggccctg aatgttgatc tgacagaatt ccagaccaac 840
ctggtgccct accctcgcat ccacttccct ctggccactt atgcccctgt catctctgct 900
gagaaagcct accatgaaca gctttctgta gcagagatca ccaatgcctg ctttgagcca 960
gccaaccaga tggtgaaatg tgaccctcgc catggtaaat acatggcttg ctgcctgctg 1020
taccgtggtg atgtggtccc caaagatgtc aatgctgcca ttgccaccat caagaccaag 1080
cgtaccatcc agtttgtgga ctggtgcccc actggcttca aggttggcat taattaccag 1140
cctcccactg tggtccctgg tggcgacctg gccaaggtcc agagagctgt gtgtatgctg 1200
agcaacacca cagccattgc tgaggcctgg gctcgcctgg atcacaagtt tgatctgatg 1260
tatgccaagc gtgcctttgt gcactggtac gtgggtgagg gcatggagga gggagagttc 1320
tctgaggccc gtgaggacat ggctgcccta gagaaggatt atgaggaggt tggtgtggat 1380
tctgtggagg gtgagggtga ggaagaagga gaggaatact aaattaaatg tcacaaggtg 1440
ctgctttcac agggatgttt attctggtcc aacatagaaa gttgtgggct gatcagttaa 1500
tttgtatgtg gcaatgtgtg ctttcataca gttactgact ttaagtgtga atgatttgtc 1560
agagacccga gccgtccact tcactgatgg gttttaaata aaatactccc tgtctta 1617

<210> 176
<211> 554
<212> PRT
<213> Rattus sp.

<220>
<223> Prosaposin

<400> 176
Met Tyr Ala Leu Ala Leu Leu Ala Ser Leu Leu Val Thr Ala Leu Thr
1               5                   10                  15

Ser Pro Val Gln Asp Pro Lys Ile Cys Ser Gly Gly Ser Ala Val Val
            20                  25                  30

Cys Arg Asp Val Lys Thr Ala Val Asp Cys Arg Ala Val Lys His Cys
        35                  40                  45

Gln Gln Met Val Trp Ser Lys Pro Thr Ala Lys Ser Leu Pro Cys Asp
    50                  55                  60

Ile Cys Lys Thr Val Val Thr Glu Ala Gly Asn Leu Leu Lys Asp Asn
65                  70                  75                  80

Ala Thr Glu Glu Glu Ile Leu His Tyr Leu Glu Lys Thr Cys Ala Trp
                85                  90                  95

Ile His Asp Ser Ser Leu Ser Ala Ser Cys Lys Glu Val Val Asp Ser
            100                 105                 110

Tyr Leu Leu Val Ile Leu Asp Met Ile Lys Gly Glu Met Ser Asn Pro
        115                 120                 125

Gly Glu Val Cys Ser Ala Leu Asn Leu Cys Gln Ser Leu Gln Glu Tyr
    130                 135                 140

Leu Ala Glu Gln Asn Gln Arg Gln Leu Glu Ser Asn Lys Ile Pro Glu
145                 150                 155                 160

Val Asp Leu Ala Arg Val Val Ala Pro Phe Met Ser Asn Ile Pro Leu
                165                 170                 175

Leu Leu Tyr Pro Gln Asp Arg Pro Arg Ser Gln Pro Gln Pro Lys Ala
            180                 185                 190

Asn Glu Asp Val Cys Gln Asp Cys Met Lys Leu Val Thr Asp Ile Gln
        195                 200                 205

Thr Ala Val Arg Thr Asn Ser Ser Phe Val Gln Gly Leu Val Asp His
    210                 215                 220

Val Lys Glu Asp Cys Asp Arg Leu Gly Pro Gly Val Ser Asp Ile Cys
225                 230                 235                 240

Lys Asn Tyr Val Asp Gln Tyr Ser Glu Val Ala Val Gln Met Met Met
                245                 250                 255

His Met Gln Pro Lys Glu Ile Cys Val Met Val Gly Phe Cys Asp Glu
            260                 265                 270

Val Lys Arg Val Pro Met Arg Thr Leu Val Pro Ala Thr Glu Ala Ile
        275                 280                 285

Lys Asn Ile Leu Pro Ala Leu Glu Leu Thr Asp Pro Tyr Glu Gln Asp
    290                 295                 300

Val Ile Gln Ala Gln Asn Val Ile Phe Cys Gln Val Cys Gln Leu Val
305                 310                 315                 320

Met Arg Lys Leu Ser Glu Leu Ile Ile Asn Asn Ala Thr Glu Glu Leu
                325                 330                 335

Leu Ile Lys Gly Leu Ser Lys Ala Cys Ser Leu Leu Pro Ala Pro Ala
            340                 345                 350

Ser Thr Lys Cys Gln Glu Val Leu Val Thr Phe Gly Pro Ser Leu Leu
        355                 360                 365

Asp Val Leu Met His Glu Val Asn Pro Asn Phe Leu Cys Gly Val Ile
    370                 375                 380

Ser Leu Cys Ser Ala Asn Pro Asn Leu Val Gly Thr Leu Glu Gln Pro
385                 390                 395                 400

Ala Ala Ala Ile Val Ser Ala Leu Pro Lys Glu Pro Ala Pro Pro Lys
                405                 410                 415

Gln Pro Glu Glu Pro Lys Gln Ser Ala Leu Arg Ala His Val Pro Pro
            420                 425                 430

Gln Lys Asn Gly Gly Phe Cys Glu Val Cys Lys Lys Leu Val Ile Tyr
        435                 440                 445

Leu Glu His Asn Leu Glu Lys Asn Ser Thr Lys Glu Glu Val Leu Ala
    450                 455                 460

Ala Leu Glu Lys Gly Cys Ser Phe Leu Pro Asp Pro Tyr Gln Lys Gln
465                 470                 475                 480

Cys Asp Glu Phe Val Ala Glu Tyr Glu Pro Leu Leu Leu Glu Ile Leu
                485                 490                 495

Val Glu Val Met Asp Pro Ser Phe Val Cys Ser Lys Ile Gly Val Cys
            500                 505                 510

Pro Ser Ala Tyr Lys Leu Leu Leu Gly Thr Glu Lys Cys Val Arg Gly
        515                 520                 525

Pro Gly Tyr Trp Cys Gln Asn Met Glu Thr Ala Ala Arg Cys Asn Ala
    530                 535                 540

Val Asp His Cys Lys Arg His Val Trp Asn
545                 550


<210> 177
<211> 1737
<212> DNA
<213> Rattus sp.

<220>
<223> Prosaposin

<400> 177
gcagcctgcg gagtgaagcg cgccatgtat gctctcgctc tcctcgccag ccttctggtc 60
accgctctga ccagccctgt ccaggacccg aagatctgct ctgggggctc agcagtagtg 120
tgcagagatg tgaagacggc ggtggactgt agggctgtga agcactgcca gcagatggtc 180
tggagcaagc ccacagcaaa atcccttcct tgtgacatat gcaaaacggt tgtcaccgaa 240
gctgggaact tgctgaaaga taatgctact gaggaggaga tcctccatta cctggagaag 300
acctgtgcgt ggattcatga ctccagcctg tcagcctctt gcaaggaggt ggttgactct 360
tacctgcttg tcatcctgga catgattaag ggggagatga gcaaccccgg ggaagtgtgc 420
tctgcgctca acctctgcca gtcccttcag gagtacttgg ccgagcaaaa ccagagacag 480
ctggagtcca acaagatccc ggaggtggac ctggcccgcg tggttgcccc cttcatgtcc 540
aacatccctc tcctgctgta ccctcaggat cggcctcgca gccagccgca gcccaaggct 600
aacgaggacg tctgccagga ctgtatgaag ttggtgactg acatccagac tgctgtgagg 660
accaactcca gctttgtcca gggcttggtg gaccacgtca aggaggattg tgaccgcctc 720
gggccaggcg tgtctgacat atgcaagaac tatgttgacc agtattctga ggtcgccgtc 780
cagatgatga tgcacatgca acccaaggaa atctgtgtga tggttggctt ctgtgatgag 840
gtcaagagag tgccaatgag gactctggtc cccgccactg aggccatcaa gaatatcctc 900
cctgccctgg agctgacgga cccctatgag caggatgtga tccaggccca aaatgtgatt 960
ttctgccaag tttgtcagct tgtgatgcgc aagttgtctg aactgattat caacaatgcc 1020
actgaggaac ttctaattaa aggtttgagt aaagcctgct ccctgctccc tgctcctgct 1080
tcaaccaagt gccaggaagt gctggtaaca tttggcccct ccctgttgga cgtcctcatg 1140
catgaggtga acccgaactt tctgtgcggt gtgatcagcc tctgttctgc caacccgaat 1200
ttggtgggga cccttgaaca acctgcagca gccattgtat ctgcactgcc caaagagcct 1260
gcaccgccaa aacagcctga agagcccaag cagtctgcat tgcgcgccca tgtgcctcct 1320
cagaagaacg gggggttctg tgaggtgtgc aagaagctgg tcatctattt ggaacataac 1380
ctggagaaaa acagcaccaa ggaggaggtc ctggctgcac ttgagaaggg ctgcagcttc 1440
ctgcccgacc cttaccagaa gcagtgtgat gaatttgtgg ctgagtatga gcccttactg 1500
ctggaaatcc ttgtggaggt gatggatcct tcctttgtgt gctcgaaaat tggagtctgc 1560
ccttctgcct ataagctgct gctgggaacc gagaagtgtg tccgggggcc aggctactgg 1620
tgtcagaaca tggagactgc tgcccgatgc aatgctgtcg atcattgcaa acgccatgtg 1680
tggaactagc tttccagctt gcagaagtcg cctacttgtg ggtctagggt aatgaag    1737

## Description

### FIELD OF THE INVENTION

The present invention relates to nucleic acids, their expression products and pathways involved in pain, and their use in screening for molecules that can alleviate pain. The invention further relates to methods for the assay and diagnosis of pain in patients.

### BACKGROUND TO THE INVENTION

Pain is currently classified into four general types. Post-operative acute pain can be successfully treated with existing pain medications of e.g. the opioid and non-steroidal anti-inflammatory (NSAID) types, and is usually short-term and self-limiting. A second type of pain, present e.g. in cancer and arthritis, is also responsive to medication initially with a NSAID and in its later stages with opioids. Neuropathic pain arises from damage to the central or peripheral nerve systems, and is more effectively treated with antidepressants or anticonvulsants. A fourth type of pain called central sensitization results from changes in the central nervous system as a result of chronic pain, these changes often being irreversible and difficult to treat. Nerve pain from shingles or diabetes falls into this and the neuropathic category. Changes occur where pain is at first poorly controlled and gradually progress to the point where a person is sensitive to stimuli which would not normally cause pain, for example a light touch. People with pain of this kind often describe a widening of the pain area to include areas which had originally not been injured or which were thought not to be involved in pain. This classification is, however based on clinical symptoms rather than on the underlying pain mechanisms.

Opiates such as morphine belong to a traditional class of pain-relieving compounds that are now recognized as binding to opiate receptors. Naturally occurring polypeptides have also been found to have opiate-like effects on the central nervous system, and these include β-endorphin, met-enkephalin and leu-enkephalin.

Salicin was isolated at the beginning of the 19th century, and from that discovery a number of NSAIDs such as aspirin, paracetamol, ibuprofen, flurbiprofen and naproxen were developed. NSAIDs are by far the most widely used pain-relieving compounds, but can exhibit side effects, in particular irritation of the GI tract that can lead to the formation of ulcers, gastrointestinal bleeding and anaemia.

Interest in the neurobiology of pain is developing: see a colloquium sponsored by the US National Academy of Sciences in December 1998 concerning the neurobiology of pain and reviewed in *The Scientist* **13[1]**, 12, 1999. Many pain mechanisms were discussed including the role of the capsaicin receptors in pain, (M.J. Caterina *et al*., *Nature,* **389**, 816-824, 1997). Large dosages of capsaicin were reported to disable that receptor, (W.R. Robbins *et al*., *Anesthesia and Analgesia,* **86**, 579-583, 1998). Additionally, a tetrodotoxin-resistant sodium channel found in small diameter pain-sensing neurons (PN3) was discussed (A.N. Akopian *et al*., *Nature,* **379**, 257-262, 1986) and L. Sangameswaran *et al., Journal of Biological Chemistry,* **271**, 953-956, 1996). Its involvement in transmission and sensitization to pain signals has been reported, (S.D. Novakovic *et al., Journal of Neuroscience,* **18**, 2174-2187, 1998). A further tetrodotoxin-resistant sodium channel has been reported (S. Tate *et al., Nature Neuroscience,* **1**, 653-655 1998).

Second messenger systems have also been shown to be important since knockout-mice lacking protein kinase C (**PKC**) γ were reported to respond to acute pain e.g. from a hot surface, but not to respond to neuropathic pain when their spinal nerves are injured (Malmberg *et al*., Science, **278,** 279-283 (1997).

Present methods for identifying novel compounds that relieve pain of one or more of the types indicated above suffer from the defect that they are dependent either on the relatively limited number of receptors known to be involved in pain or on the empirical identification of new receptors which is an uncertain process. In relation to known receptors, for example the opioid receptor, research directed to improved compounds offers the possibility of searching compounds that have a better therapeutic ratio and fewer side effects. Thist does not lead naturally to compounds for different pain receptors that have new modes of action and new and qualitatively different benefits. Even when newly identified additional receptors are taken into account, known receptors revolve around tens of gene products. However, there are between 30,000 and 40,000 genes in the genome of an animal and more of them are concerned with nervous system function than with peripheral function. We therefore concluded that a large number of receptors and pathways are important to the transduction of pain, but up to now have remained unknown.

### SUMMARY OF THE INVENTION

It is an object of the invention to provide sequences of genetic material for which no role in pain has previously been disclosed, and which are useful, for example, in:
- identifying metabolic pathways for the transduction of pain
- identifying from said metabolic pathways compounds having utility in the diagnosis or treatment of pain
- producing proteins and polypeptides with a role in the transduction of pain;
- producing genetically modified non-human animals that are useful in the screening of compounds for the treatment or diagnosis of pain.
- Identifying ligand molecules for said receptors involved in metabolic pathways and having utility in the treatment of pain.

It is yet a further object of the invention to provide non-human animals and microorganisms that can be used in screening compounds for pharmacological activity, especially pain-reducing activity.

In one aspect, the invention relates to the use in the screening of compounds that are effective in the treatment of pain, or in the diagnosis of pain, of:
(a) an isolated gene sequence that is down regulated in the spinal cord of a mammal in response to streptozocin-induced diabetes;
(b) an isolated gene sequence having at least 80% sequence identity with the nucleic acid sequences of Tables I-X
(c) an isolated nucleic acid sequence comprising a sequence that is hybridizable to any of the gene sequences according to (a) or (b) under stringent hybridisation conditions;
(d) a recombinant vector comprising any of the gene sequences according to (a) to (c);
(e) a host cell containing the vector according to (d);
(f) a non-human animal, for use in the screening of compounds that are effective in the treatment of pain, or in the diagnosis of pain, having in its genome an introduced gene sequence or a removed or down-regulated nucleotide sequence, said sequence becoming down-regulated in the spinal cord of a mammal in response to streptozocin-induced diabetes;
(g) an isolated polypeptide containing an amino acid sequence at least 90% identical to an amino acid sequence encoded by a nucleic acid sequence according to any one of (a) to (d), or a polypeptide variant thereof with sequential amino acid deletions from the C terminus and/or the N-terminus; or
(h) an isolated antibody that binds specifically to the isolated polypeptide according to (g).

The invention also relates to the use of naturally occurring compounds such as peptide ligands of the expression products of the above gene sequences and their associated signal transduction pathways for the treatment of pain.

### DESCRIPTION OF PREFERRED EMBODIMENTS

### DEFINITIONS

Within the context of the present invention:
- "Comprising" means consisting of or including. Thus nucleic acid comprising a defined sequence includes nucleic acid that may contain a full-length gene or full-length cDNA. The gene may include any of the naturally occurring regulatory sequence(s), such as a transcription and translation start site, a promoter, a TATA box in the case of eukaryotes, and transcriptional and translational stop sites. Further, a nucleic acid sequence comprising a cDNA or gene may include any appropriate regulatory sequences for the efficient expression thereof *in vitro*
- "Isolated" requires that the material be removed from its original environment (e.g. the natural environment if it is naturally occurring). For example, a naturally occurring polynucleotide or a peptide present in a living animal is not isolated, but the same polynucleotide or peptide, separated from some or all of the coexisting materials in the natural system, is isolated. Such polynucleotide can be part of a vector and/or such polynucleotide or peptide can be part of a composition, and still be isolated in that the vector or composition is not a part of its natural environment.
- "Purified" does not require absolute purity; instead it is intended as a relative definition. Purification of starting materials or natural materials from their natural environment to at least one order of magnitude, preferably two or three orders, and more preferably four or five orders of magnitude is expressly contemplated.
"Nucleic acid sequence" or "gene sequence" means a sequence of nucleotides or any variant or homologue thereof, or truncated or extended sequence thereof, and is preferably indicated by a Genebank accession number. Also within the scope of the present invention are down-regulated nucleic acid sequences which encode expression products which are components of signaling pathways. This invention also includes any variant or homologue or truncated or extended sequence of the down-regulated nucleotide sequence. Also within the scope of the present invention, the term "nucleic acid(s) product", or "expression product" or "gene product" or a combination of these terms refers without being biased, to any, protein(s), polypeptide(s), peptide(s) or fragment(s) encoded by the up-regulated nucleotide sequence.
- " Operably linked" refers to a linkage of polynucleotide elements in a functional relationship. For instance, a promoter or an enhancer is operably linked to a coding sequence if it regulates the transcription of the coding sequence. In particular, two DNA molecules (such as a polynucleotide containing a promoter region and a polynucleotide encoding a desired polypeptide) are said to be "operably linked" if the nature of the linkage between the two polynucleotides does not (1) result in the introduction of a frame-shift mutation and (2) interfere with the ability of the polynucleotide containing the promoter to direct the transcription of the coding polynucleotide.
- "Pain" includes chronic pain and in particular diabetic pain.
- "Stringent hybridization conditions" is a recognized term in the art and for a given nucleic acid sequence refers to those conditions which permit hybridization of that sequence to its complementary sequence and closely homologous sequences. Conditions of high stringency may be illustrated in relation to filter-bound DNA as for example 2X SSC, 65°C (where SSC = 0.15M sodium chloride, 0.015M sodium citrate, pH 7.2), or as 0.5M NaHPO₄, 7% sodium dodecyl sulfate (SDS), 1mM EDTA, at 65°C, and washing in 0.1xSCC/0.1% SDS at 68°C (Ausubel F.M. *et al*., eds, 1989, *Current Protocols in Molecular Biology,* Vol. I, Green Publishing Associates, Inc., and John Wiley & Sons Inc., New York, at p. 2. 10.3). Hybridization conditions can be rendered highly stringent by raising the temperature and/or by the addition of increasing amounts of formamide, to destabilize the hybrid duplex of non-homologous nucleic acid sequence relative to homologous and closely homologous nucleic acid sequences. Thus, particular hybridisation conditions can be readily manipulated, and will generally be chosen depending on the desired results.
- "Variants or homologues" include (a) sequence variations of naturally existing gene(s) resulting from polymorphism(s), mutation(s), or other alteration(s) as compared to the above identified sequences, and which do not deprive the encoded protein of function (b) recombinant DNA molecules, such as cDNA molecules encoding genes indicated by the relevant Genebank accession numbers and (c) any sequence that hybridizes with the above nucleic acids under stringent conditions and encodes a functional protein or fragment thereof.
"Gene product" refers to polypeptide - which is interchangeable with the term protein - which is encoded by a nucleotide sequence and includes single-chain polypeptide molecules as well as multiple-polypeptide complexes where individual constituent polypeptides are linked by covalent or non-covalent means.
Polypeptides of the present invention may be produced by synthetic means (e.g. as described by Geysen *et al*., 1996) or by recombinant means.
The terms "variant", "homologue", "fragment", "analogue" or "derivative" in relation to the amino acid sequence for the polypeptide of the present invention includes any substitution of, variation of, modification of, replacement of, deletion of or addition of one (or more) amino acid from or to the sequence providing the resultant polypeptide has the native gene product activity. In particular, the term "homologue" covers homology with respect to structure and/or function. With respect to sequence homology, there is at least 90%, more preferably at least 95% homology to an amino acid sequence encoded by the relevant nucleotide sequence shown in Tables I - X, preferably there is at least 98% homology.
Typically, for the variant, homologue or fragment of the present invention, the types of amino acid substitutions that could be made should maintain the hydrophobicity/hydrophilicity of the amino acid sequence. Amino acid substitutions may include the use of non-naturally occurring amino acid analogues.
In addition, or in the alternative, the protein itself could be produced using chemical methods to synthesize a polypeptide, in whole or in part. For example, peptides can be synthesized by solid phase techniques, cleaved from the resin, and purified by preparative high performance liquid chromatography (e.g. Creighton (1983) Proteins Structures and Molecular Principles, WH Freeman and Co., New York, NY, USA).
The composition of the synthetic peptides may be confirmed by amino acid analysis or sequencing (e.g. the Edman degradation procedure).
Direct peptide synthesis can be performed using various solid-phase techniques (Roberge JY *et al* Science Vol 269 1995 202-204) and automated synthesis may be achieved, for example, using the ABI 431 A Peptide Synthesizer (Perkin Elmer) in accordance with the instructions provided by the manufacturer. Additionally, the amino acid sequence of a gene product, or any part thereof, may be altered during direct synthesis and/or combined using chemical methods with a sequence from other subunits, or any part thereof, to produce a variant polypeptide.
In another embodiment of the invention, a gene product natural, modified or recombinant amino acid sequence may be ligated to a heterologous sequence to encode a fusion protein. For example, for screening of libraries for compounds and peptide agonists and antagonists of gene product activity, it may be useful to encode a chimeric gene product expressing a heterologous epitope that is recognised by a commercially available antibody. A fusion protein may also be engineered to contain a cleavage site located between a gene product sequence and the heterologous protein sequence, so that the gene product may be cleaved and purified away from the heterologous moiety.
The gene product may also be expressed as a recombinant protein with one or more additional polypeptide domains added to facilitate protein purification. Such purification facilitating domains include, but are not limited to, metal chelating peptides such as histidine-tryptophan modules that allow purification on immobilised metals (Porath J, Protein Expr Purif Vol 3 1992 p263-281), protein A domains that allow purification on immobilised immunoglobulin, and the domain utilised in the FLAGS extension/affinity purification system (Immunex Corp, Seattle, WA, USA). The inclusion of a cleavable linker sequence such as Factor XA or enterokinase (Invitrogen, San Diego, CA, USA) between the purification domain and the gene product is useful to facilitate purification.

### IDENTIFIED SEQUENCES

The inventors have identified nucleotide acid sequences that give rise to expression products listed in the Tables I - X that are down regulated in the spinal cord in response to streptozocin-induced diabetes and are believed to be involved in the transduction of pain. However, they have not been found to be down-regulated in a chronic constrictive injury (CCI) model of pain. These nucleic acid sequences have not previously been implicated in the transduction of pain. In Tables I - X, * denotes more preferred nucleic acid sequences and ** denotes most preferred nucleic acid sequences.

The validity of the present experimental procedure was confirmed by the fact that nucleotide sequences were obtained as a result of the investigation whose function in the transduction of pain has been previously confirmed and established. These nucleic acid sequences are not part of this invention. Any of the nucleic acid sequences and expression products can be used to develop screening technologies for the identification of novel molecules for the prevention or treatment of pain. These screening technologies could also be used to ascribe new pain therapeutic indications to molecules that have not previously been identified as being useful for the prevention or treatment of pain. Furthermore, the said nucleic acid sequences can be used as diagnostic tools and for the development of diagnostic tools.

**Table I -**

| **Sequences whose expression products are kinases** | | | | | |
|---|---|---|---|---|---|
| **Expression product or name (Ref)** | **Rat Accession Number** | **Mouse accession number** | **Human Accession Number** | **Reaction** | **Assay** |
| Putative DNA dependent protein kinase catalytic subunit (Ref : 1) | | D87521 (SEQ ID No's 1 - 2) | | Recombination and DNA repair | Ser/Thr kinase |
| Putative cAMP dependent protein kinase regulatory subunit RI alpha * (Ref : 2) | AJ278429 (SEQ ID No's 3 - 4) | | | Regulatory subunit | Kinase |
| Putative protein tyrosine kinase t-Ror1 (Ref : 3) | | | U38894 (SEQ ID NO's 5 - 6) | N/A | receptor tyrosine kinase |
| Diacylglycerol kinase (DGK-IV) * (Ref : 4) | D78588 (SEQ ID No's 7 - 8) | | U51477 | Diacyl glycerol activity | kinase |
| Elk protein tyrosine kinase * (Ref:5) | X13411 (SEQ ID No's 9-10) | | | N/A | receptor tyrosine kinase |

**Table II -**

| **Sequences whose expression products are phosphatases** | | | | | |
|---|---|---|---|---|---|
| **Expression product or name (Ref)** | **Rat accession number** | **Mouse accession number** | **Human Accession Number** | **Reaction** | **Assay** |
| PP-1 alpha for catalytic subunit of protein phosphatase type 1 alpha (Ref: 6) | D00859 (SEQ ID NO's 11-12) | U25809 | X70848 | Ser/Thr phosphatase | Phosphatase |
| Protein tyrosine phosphatase, striatum enriched (Ref : 7) | S49400 (SEQ ID No's 13-14) | U28216 | U27831 | Cell signalling | Non-receptor Tyr |

**Table III -**

| **Sequences whose expression products are ion channel proteins** | | | | |
|---|---|---|---|---|
| **EXPRESSION PRODUCT (Refs)** | **RAT ACCESSION NUMBER** | **MOUSE ACCESSION NUMBER** | **HUMAN ACCESSION NUMBER** | **REACTION** |
| Na⁺, K⁺-ATPase alpha-subunit (Ref: 8) | D00189 (SEQ ID No's 15-16) | | | Membrane potential |
| Voltage dependent anion channel 2 * (Ref : N/A) | AF268468 (SEQ ID No's 17-18) | U30838 | L06328 | N/A |

**Table IV -**

| **Sequences whose expression products are receptors** | | | | | |
|---|---|---|---|---|---|
| **Expression Product or Name** | **Rat Accession Number** | **Mouse accession Number** | **Human Accession Number** | **Reaction** | **Assay** |
| Neonatal glycine receptor NglyR * (Ref : 9) | X57281 (SEQ ID No's 19-20) | | | Glycine gated Cl channels in *Xenopus* oocyte | N/A |
| Syntaxin 13 * (Ref:10) | AF044581 (SEQ ID NO's 21 -22) | | | Neurite outgrowth and endosomal trafficking | SNARE |

**Table V -**

| **Sequences whose expression products are transporters** | | | | | |
|---|---|---|---|---|---|
| **Expression Product or Name** | **Rat Accession Number** | **Mouse Accession Number** | **Human Accession Number** | Reaction | **Assay** |
| ATPase, calcium, plasma membrane, isoform 1 (Ref: 11) | J03753 (SEQ ID No's 23-24) | | | Calcium transport across plasma membrane | Calcium |
| Calcium transporter CaT2 (Ref:12) | AF209196 (SEQ ID No's 25-26) | | | Calcium transport across plasma membrane | Calcium |
| Carnitine/acyl carnitine carrier protein (Ref : 13) | X97831 (SEQ ID No's 27-28) | | | Fatty acid metabolism | N/A |
| Putative potent brain type organic ion transporter (Ref : N/A) | | AB012808 | AB040056 (SEQ ID No's 29-30) | Transmembrane transport | Transporter |
| UDP-galactose transporter (Ref: 14) | D87991 (SEQ ID No's 31-32) | | | Nucleotidesugar transporter | transporter |
| Choline transporter CHOT1 ** (Ref:15) | X66494 (SEQ ID No's 33-34) | | | Neurotransmitter release | Choline |

**Table VI -**

| **Sequences whose expression products are G-protein coupled receptor proteins** | | | | | |
|---|---|---|---|---|---|
| **Expression product or Name** | **Rat Accession Number** | **Mouse Accession Number** | **Human Accession Number** | **Reaction** | **Assay** |
| Rab GDI, alpha species, ras related GTPase (Ref : 16) | X74402 (SEQ ID NO's 35-36) | | | G-protein | N/A |

**Table VII -**

| **Sequences whose expression products are DNA-binding proteins** | | | | | |
|---|---|---|---|---|---|
| **Expression Product or Name** | **Rat Accession Number** | **Mouse Accession Number** | **Human Accession Number** | **Reaction** | **Assay** |
| Histone, core, H2A.1 (Ref: 17) | M99065 (SEQ ID No's 37-38) | | | DNA binding | DNA binding |
| Putative signal transducer and regulator transcription 1 (Stat1) (Ref: 18) | | U06924 (SEQ ID No's 39-40) | | Transcription factor | DNA binding |
| Transcription factor IIIC, alpha subunit (Ref : 19) | L28801 (SEQ ID No's 41-42) | | | RNA synthesis | DNA binding |

**Table VIII -**

| **Sequences whose expression products are proteases** | | | | | |
|---|---|---|---|---|---|
| **Expression Product or Name** | **Rat Accession Number** | **Mouse Accession Number** | **Human Accession Number** | **Reaction** | **Assay** |
| Putative Lon proteinase (Ref: 20) | | | X76040 (SEQ ID No's 43-44) | Endopeptidase | peptidase |

**Table IX -**

| **Sequences whose expression products are other enzymes** | | | | | |
|---|---|---|---|---|---|
| **Expression product or Name** | **Rat Accession Number** | **Mouse Accession Number** | **Human Accession Number** | **Reaction** | **Assay** |
| GM3 synthase (Ref : N/A) | AB018049 (SEQ ID No's 45-46) | | | Ganglioside synthesis | Ligase |
| Enolase, neuron specific (Ref: 21) | M11931 (SEQ ID No's 47-48) | | | Glycolysis | Lyase |
| Putative carbonic anhydrase XIV (Ref : 22) | | AB005450 (SEQ ID No's 49-50) | | Acid-base balance | Lyase |
| Cytosolic malate dehydrogenase (Mdh) * (Ref : N/a) | AF093773 (SEQ ID NO's 51-52) | M29462 | D55654 | Carbohydrate metabolism | Oxidoreductase |
| Heme-binding protein (HBP23) (Ref: 23) | D30035 (SEQ ID No's 53-54) | | | N/A | Oxidoreductase |
| Squalene epoxidase * (Ref : 24) | D37920 (SEQ ID No's 55-56) | | | Sterol biosynthesis | Oxidoreductase |
| Superoxide dismutase, copper-zinc (SOD-1) (REF : 25) | M21060 (SEQ ID No's 57-58) | X05634 | K00065 | Destroying radicals | Oxidoreductase |
| Aspartate aminotransferase, cytosolic * (Ref:26) | J04171 (SEQ ID No's 59-60) | | | Amino acid metabolism | Transferase |
| Branched-chain aminotransferase, cytosolic, brain (Ref:27) | AF165887 (SEQ ID No's 61-62) | | | Amino acid metabolism | Transferase |
| Dihydrolipoamide succinyl transferase (Ref:28) | D90401 SEQ ID NO's 63-64) | | | TCA cycle | Transferase |
| RNA polymerase II (Ref 29) | AB017711 (SEQ ID NO's 65-66) | | | Transcription | Transferase |
| Squalene synthetase, hepatic (Ref: 30) | M95591 (SEQ ID No's 67-68) | | | Isoprene and cholesterol biosynthesis | Transferase |
| UDP-Gal: glucosylceramide beta-1,4 galactosyl transferase (Ref: 31) | AF048687 (SEQ ID No's 69-70) | | | Lactosyl ceramide synthesis | Transferase |

**Table X-**

| **Miscellaneous sequences** | | | | |
|---|---|---|---|---|
| **Expression product or Name** | **Rat Accession Number** | **Mouse Accession Number** | **Human Accession Number** | **Reaction** |
| Microtubule associated proteins 1A and !B light chain subunit 3 (Ref:32) | U05784 (SEQ ID No's 71-72) | | | Cytoskeleton protein |
| Putative MD6 (Ref: N/A) | | X54352 (SEQ ID No's 73-74) | | Unknown |
| Ribosomal protein L3 (Ref: 33) | X62166 (SEQ ID No's 75-76) | | | Protein synthesis |
| Ribosomal protein S4 (Ref: 34) | X14210 (SEQ ID No's 77-78) | M73436 | M58458 | Protein synthesis |
| Synaptic vesicle protein 2C (Ref: 35) | AF060174 (SEQ ID No's 79-80) | Synaptic vesicle protein 2C | | Unknown |
| Putative ARL-6 interacting protein-1 (Ref;36) | | AF223953 (SEQ ID No's 81-82) | | Unknown |
| Ribosomal protein L14 (Ref : 37) | X94242 (SEQ ID No's 83-84) | | | Protein synthesis |
| Putative dynactin light ) chain (P24) (Ref: 38) | | AF098508 (SEQ ID No's 85-86) | | Cytoskeleton |
| Ubiquitin/ribosomal protein L40, fusion protein (Ref: 39) | X82636 (SEQ ID No's 87-88) | AF118042 | | |
| Cadherin PB, short type (Ref : 40) | D83349 (SEQ ID No's 89-90) | | | Cell adhesion |
| Guanine nucleotide binding protein, γ-3 subunit (Ref: 41) | AF022088 (SEQ ID No's 91-92) | | | Cell signalling |
| Actin, γ, cytoplasmic (Ref: 42) | X52815 (SEQ ID No's 93-94) | | | Cytoskeleton protein |
| Chimaerin, β (bch) (Ref: 43) | L07494 (SEQ ID No's 95-96) | | | Cell signalling |
| Parvalbumin (Ref: 44) | M12725 (SEQ ID No's 97-98) | | X63070 | Ca²⁺ buffer |
| Putative BAP 31 (Ref: 45) | | X81816 (SEQ ID No's 99-100) | | Unknown |
| Putative protocadherin 2C (Ref:46) | U88550 (SEQ ID No's 101-102) | | | Neural development |
| Ribosomal protein 112 (Ref : 47) | X53504 (SEQ ID No's 103-104) | L04280 | L06505 | Protein synthesis |
| Putative cell surface glycoprotein A15(PE 15) gene (Ref: N/A) | | D26483 (SEQ ID No's 105-106) | | Unknown |
| Putative translation initiation factor elF3-p44(Ref:N/A) | | U70733 (SEQ ID No's 107-108) | | Protein synthesis |
| Putative tumor susceptibility tsg101 gene (Ref : 48) | | U52945 (SEQ ID No's 109-110) | | Unknown |
| Thymosin β 4, spleen (Ref : 49) | M34043 (SEQ ID No's 111-112) | U38967 | D85181 | Unknown |
| Ribosomal protein L18 (Ref: 50) | M20156 (SEQ ID No's 113-114) | | | Protein synthesis |
| Putative activation of Sentrin/SUMO protein AOS1 (Ref N/A) | | AB024303 (SEQ ID No's 115-116) | | |
| 190 kDa ankyrin isoform (Ref: 51) | AF069525 (SEQ ID No's 117-118) | | | Cytoskeleton protein |
| Synaptotagmin interacting protein zygin 1 (Ref: N/A) | U63740 (SEQ ID No's 119-120) | | U69139 | Axonal outgrowth |
| Prion-related protein (PrP) (Ref : 52) | M20313 (SEQ ID No's 121-122) | | | Cell signalling |
| Ribosomal protein L7a (Ref : 53) | X15013 (SEQ ID No's 123-124) | | | Protein synthesis |
| Astrocyte and olefactroy-limbic associated, type 1 (Ref:54) | U33472 (SEQ ID No's 125-126) | | | Unknown |
| C1-13 protein neuronal-specific (Reff:55) | X52817 (SEQ ID NO's 127-128) | | | Unknown |
| Calnexin (Ref: 56) | L18889 (SEQ ID No's 129-130) | | | Protein production |
| Enkephalin (Enk), prepro ** (Ref : 57) | Y07503 (SEQ ID No's 131-132) | | | Neurotransmitter |
| f-spondin (Ref: 58) | M88469 (SEQ ID No's 133-134) | | | Neurite extension, neural cell adhesion |
| Neurofilament 68kDa (Ref: 59) | X53981 (SEQ ID No's 135-136) | | | Cytoskeleton |
| Putative E800 (Ref : 60) | | Y10968 (SEQ ID No 137) | | Unknown |
| Putative F-actin capping protein α-1 subunit (CAPZ) (Ref: 61) | | | U56637 (SEQ ID No's 138-139) | Cytoskeleton protein |
| Putative KIAA0456 (Ref : 62) | | | AB007925 (SEQ ID No's 140-141) | Unknown |
| Putative potassium channel modulatory factor (Ref: N /A) | | | AF155652 (SEQ ID No's 142-143) | K⁺ current modulator |
| Putative RNA1 homolog (Ref : 63) | | U08110 (SEQ ID NO's 144-145) | | Unknown |
| Putative Sec 62 gene (Ref : 64) | | | U93239 (SEQ ID No's 146-147) | Protein translocation |
| Putative TCP-1-chaperonin cofactor A (Ref:65) | | U05333 (SEQ ID No's 148-149) | | protein folding |
| Putative U6 sn-RNA associated Sm-like protein (Ref : N/A) | | | AF182290 (SEQ ID No's 150-151) | Nucleic acid splicing |
| Putative vitiligo associated protein VIT-1 (Ref : 66) | | AF031087 (SEQ ID No's 152-153) | | Unknown |
| Glypican (Ref : 67) | L02896 (SEQ ID No's 154-155) | | | Heparan sulphate glycoprotein |
| Putative Ndr1 gene (Ref: 68) | | U60593 (SEQ ID No's 156-157) | | Unknown |
| Ribosomal protein S23 (Ref : 69) | X77398 (SEQ ID No's 158-159) | | | protein synthesis |
| Putative DNA binding protein insulinoma rig (Ref :70) | M19393 (SEQ ID No's 160-161) | M33330 | J02984 | Protein synthesis |
| G9 surface glycoprotein, platelet (Ref:71) | X76489 (seq id No's 162-163) | | | Unknown |
| Foocen-m2 reticulon gene (Ref: N/A) | AF132045 (SEQ ID No's 164-165) | | | Inhibits neurite outgrowth |
| Clusterin (Ref : 72) | M16975 (SEQ ID No's 166-167) | | | Unknown |
| Prosaposin (Ref 77) | S18373 (SEQ ID 176, 177) | | | Sphingolipid degradation |
| Putative nuclear speckle-type protein (Ref:73) | | | AJ000644 (SEQ ID No's 168-169) | Unknown |
| Putative OCP-2 (Ref : 74) | | | L49173 (SEQ ID No's 170-171) | Unknown |
| Putative NS1-binding protein (NS1-BP) (Ref :75) | | | AJ012449 (SEQ ID No's 172-173) | Inknown |
| Tubulin, α (Ref : 76) | V01227 (SEQ ID No's 174-175) | M13446 | K00558 | Cytoskeleton protein |

### PRODUCTION OF POLYPEPTIDES AND NUCLEIC ACIDS

### Vectors

Recombinant expression vectors can be employed to express any of the nucleic acid sequences of the invention. The expression products derived from such vector constructs can be used to develop screening technologies for the identification of molecules that can be used to prevent or treat pain, and in the development of diagnostic tools for the identification and characterization of pain. The expression vectors may also be used for constructing transgenic non-human animals.

Gene expression requires that appropriate signals be provided in the vectors, said signals including various regulatory elements such as enhancers/promoters from viral and/or mammalian sources that drive expression of the genes or nucleic acid sequences of interest in host cells. The regulatory sequences of the expression vectors used in the invention are operably linked to the nucleic acid encoding the pain-associated protein of interest or a peptide fragment thereof.

Generally, recombinant expression vectors include origins of replication, selectable markers, and a promoter derived from a highly expressed gene to direct transcription of a downstream nucleotide sequence. The heterologous nucleotide sequence is assembled in an appropriate frame with the translation, initiation and termination sequences, and if applicable a leader sequence to direct the expression product to the periplasmic space, the extra-cellular medium or cell membrane.

In a specific embodiment in which the vector is adapted for expressing desired sequences in mammalian host cells, preferred vectors will comprise an origin of replication from the desired host, a suitable promoter and an enhancer, and also any necessary ribosome binding sites, polyadenylation site, transcriptional termination sequences, and optionally 5'-flanking non-transcribed sequences. DNA sequences derived from the SV40 or CMV viral genomes, for example SV40 or CMV origin, early promoters, enhancers, and polyadenylation sites may be used to provide the required non-transcribed genetic elements.

A recombinant expression vector used in the invention advantageously also comprises an untranscribed polynucleotide region located at the 3'end of the coding sequence (ORF), this 3'-UTR (untranslated region) polynucleotide being useful for stabilizing the corresponding mRNA or for increasing the expression rate of the vector insert if this 3'-UTR harbours regulation signal elements such as enhancer sequences.

Suitable promoter regions used in the expression vectors are chosen taking into account the host cell in which the nucleic acid sequence is to be expressed. A suitable promoter may be heterologous with respect to the nucleic acid sequence for which it controls the expression, or alternatively can be endogenous to the native polynucleotide containing the coding sequence to be expressed. Additionally, the promoter is generally heterologous with respect to the recombinant vector sequences within which the construct promoter/coding sequence has been inserted. Preferred promoters are the LacI, LacZ, T3 or T7 bacteriophage RNA polymerase promoters, the lambda PR, PL and Trp promoters (EP-0 036 776), the polyhedrin promoter, or the p10 protein promoter from *baculovirus* (kit Novagen; Smith *et al*., (1983); O'Reilly *et al.* (1992)).

Preferred selectable marker genes contained in the expression recombinant vectors used in the invention for selection of transformed host cells are preferably dehydrofolate reductase or neomycin resistance for eukaryotic cell culture, TRP1 for *S. cerevisiae* or tetracycline, rifampicin or ampicillin resistance in *E. coli,* or Levamsaccharase for *Mycobacteria*, this latter marker being a negative selection marker.

Preferred bacterial vectors are listed hereafter as illustrative but not limitative examples: pQE70, pQE60, pQE-9 (Quiagen), pD10, phagescript, psiX174, p.Bluescript SK, pNH8A, pNH16A, pNH18A, pNH46A (Stratagene); pKK223-3, pKK233-3, pDR540, pRIT5 (Pharmacia); pWLNEO, pSV2CAT, pOG44, pXT1, pSG (Stratagene); pSVK3, pBPV, pMSG, pSVL (Pharmacia); pQE-30 (QIA express).

Preferred bacteriophage recombinant vectors of the invention are P1 bacteriophage vectors such as described by Sternberg N.L. (1992; 1994).

A suitable vector for the expression of any of the pain associated polypeptides used in the invention or fragments thereof, is a baculovirus vector that can be propagated in insect cells and in insect cell-lines. A specific suitable host vector system is the pVL 1392/1393 *baculovirus* transfer vector (Pharmingen) that is used to transfect the SF9 cell line (ATCC N°CRL 1711) that is derived from *Spodoptera frugiperda.*

The recombinant expression vectors of the invention may also be derived from an adenovirus. Suitable adenoviruses are described by Feldman and Steig (1996) or Ohno *et al*. (1994). Another preferred recombinant adenovirus is the human adenovirus type two or five (Ad 2 or Ad 5) or an adenovirus of animal origin (Patent Application WO 94/26914)

Particularly preferred retrovirus for the preparation or construction of retroviral in *vitro* or *in vivo* gene delivery vehicles include retroviruses selected from the group consisting of Mink-Cell Focus Inducing Virus, Murine Sarcoma Virus, and Ross Sarcoma Virus. Other preferred retroviral vectors are those described in Roth *et al*. (1996), in PCT Application WO 93/25234, in PCT Application WO 94/06920, and also in Roux *et al.* (1989), Julan *et al*.(1992) and Nada *et al.* (1991).

Yet, another viral vector system that is contemplated consist in the Adeno Associated Viruses (AAV) such as those described by Flotte *et al*. (1992), Samulski *et al.* (1989) and McLaughlin *et al*. (1996).

### Host cells expressing pain associated polypeptides

Host cells that endogenously express pain associated polypeptides or have been transformed or transfected with one of the nucleic acid sequences described herein, or with one of the recombinant vectors described above, particularly a recombinant expression vector, can be used in the present invention. Also included are host cells that are transformed (prokaryotic cells) or are transfected (eukaryotic cells) with a recombinant vector such as one of those described above.

Preferred host cells used as recipients for the expression vectors used in the invention are the following:
(a) prokaryotic host cells: *Escherichia coli,* strains. (i.e. DH5-α, strain) *Bacillus subtilis, Salmonella typhimurium* and strains from species like *Pseudomonas, Streptomyces* and *Staphylococcus* for the expression of down regulated nucleic acid sequences modulated by pain, characterized by having at least 80% sequence identity with any of the nucleic acid sequences of Tables I - X. Plasmid propagation in these host cells can provide plasmids for transfecting other cells.
(b) eukaryotic host cells: HeLa cells (ATCC N°CCL2; N°CCL2.1; N°CCL2.2), Cv 1 cells (ATCC N°CCL70), COS cells (ATCC N°CRL 1650; N°CRL 1651), Sf-9 cells (ATCC N°CRL 1711), C127 cells (ATCC N°CRL-1804), 3T3 cells (ATCC N°CRL-6361), CHO cells (ATCC N°CCL-61), human kidney 293 cells (ATCC N° 45504; N°CRL-1573), BHK (ECACC N°84100 501; N°84111301), PC12 (ATCC N° CRL-1721), NT2, SHSY5Y (ATCC N° CRL-2266), NG108 (ECACC N°88112302) and F11, SK-N-SH (ATCC N° CRL-HTB-11), SK-N-BE(2) (ATCC N° CRL-2271), IMR-32 (ATCC N° CCL-127). A preferred system to which the nucleic acids of the invention can be expressed are neuronal cell lines such as PC12, NT2, SHSY5Y, NG108 and F11, SK-N-SH, SK-N-BE(2), IMR-32 cell lines, COS cells, 3T3 cells, HeLa cells, 292 cells and CHO cells. The above cell lines could be used for the expression of any of the nucleic acid sequences of Tables I - X.

When a nucleic acid sequence of any of Tables I - X is expressed using a neuronal cell line, the sequence can be expressed under an endogenous promoter or native neuronal promoter, or an exogenous promoter. Suitable exogenous promoters include SV40 and CMV and eukaryotic promoters such as the tetracycline promoter. The preferred promoter when pain associated molecules are endogenously expressed is an endogenous promoter. A preferred promoter in a recombinant cell line is the CMV promoter.

In a specific embodiment of the host cells described above, these host cells have also been transfected or transformed with a polynucleotide or a recombinant vector for the expression of a natural ligand of any of the nucleic acid sequences of Tables I - X or a modulator of these expression products.

### PROTEINS, POLYPEPTIDES AND FRAGMENTS

The expression products of the nucleic acid sequences of Tables I - X or fragment(s) thereof can be prepared using recombinant technology, from cell lines or by chemical synthesis. Recombinant methods, chemical methods or chemical synthetic methods can be used to modify a gene in order to introduce into the gene product, or a fragment of the gene product, features such as recognition tags, cleavage sites or other modifications. For efficient polypeptide production, the endogenous expression system or recombinant expression system should allow the expression products to be expressed in a manner that will allows the production of a functional protein or fragment thereof which can be purified. Preferred cell lines are those that allow high levels of expression of polypeptide or fragments thereof. Such cell lines include cell lines which naturally express any of the nucleic acid sequences of Tables I, II or III or common mammalian cell lines such as CHO cells or COS cells, etc, or more specific neuronal cell lines such as PC12. However, other cell types that are commonly used for recombinant protein production such as insect cells, amphibian cells such as oocytes, yeast and prokaryotic cell lines such as E. *coli* can also be used.

The expression products of Tables I - X or fragments thereof can be utilized in screens to identify potential therapeutic ligands, either as a purified protein, as a protein chimera such as those produced by phage display, as a cell membrane (lipid or detergent) preparation, or in intact cells.

The invention also relates generally to the use of proteins, peptides and peptide fragments for the development of screening technologies for the identification of molecules for the prevention or treatment of pain, and the development of diagnostic tools for the identification and characterization of pain. These peptides include expression products of the nucleic acid sequences of Tables I - X and purified or isolated polypeptides or fragments thereof having at least 90%, preferably 95%, more preferably 98% and most preferably 99% sequence identity with the any of the expression products of nucleic acid sequences of Tables I - X. Expressed peptides and fragments of any of these nucleic acid sequences can be used to develop screening technologies for the identification of novel molecules for the prevention or treatment of pain. These screening technologies could also be used to ascribe new pain therapeutic indications to molecules, which have not previously been ascribed for the prevention or treatment of pain. Furthermore the said expressed peptides and fragments can be used as diagnostic tools and for the development of diagnostic tools.

### SCREENING METHODS

As discussed above, we have identified nucleic acid sequences whose expression is regulated by pain, particularly chronic pain and more particularly diabetic pain. The expression products of these nucleic acids can be used for screening ligand molecules for their ability to prevent or treat pain, and particularly, but not exclusively, chronic pain. The main types of screens that can be used are described below. The test compound can be a peptide, protein or chemical entity, either alone or in combination(s), or in a mixture with any substance. The test compound may even represent a library of compounds.

The expression products of any of the nucleic acid sequences of Tables I - X or fragments thereof can be utilized in a ligand binding screen format, a functional screen format or in vivo format. Examples of screening formats are provided.

### A) Ligand binding screen

In ligand binding screening a test compound is contacted with an expression product of one of the sequences of Tables I to X, and the ability of said test compound to interact with said expression product is determined, e.g. the ability of the test compound(s) to bind to the expression product. The expression product can be a part of an intact cell, lipidic preparation or a purified polypeptide(s), optionally attached to a support, such as beads, a column or a plate etc.

Binding of the test compound is performed in the presence of a ligand to allow an assessment of the binding activity of each test compound. The ligand may be contacted with the expression product either before, simultaneously or after the test compound. The ligand should be detectable and/or quantifiable. To achieve this, the ligand can be labelled in a number of ways, for example with a chromophore, radioactive, fluorescent, phosphorescent, enzymatic or antibody label. Methods of labelling are known to those in the art. If the ligand is not directly detectable it should be amenable to detection and quantification by secondary detection, which may employ the above technologies. Alternatively the expression product or fragment thereof can be detectable or quantifiable using, for example a chromophore, radioactive, fluorescent, phosphorescent, enzymatic or antibody label.

Binding of the test compound modifies the interaction of the ligand with its binding site and changes the affinity or binding of the ligand for/to its binding site. The difference between the observed amounts of ligand bound relative to the theoretical maximum amount of ligand bound (or to the ligand bound in the absence of a test compound under the same conditions) is a reflection of the binding ability (and optionally the amount and/or affinity) of a test compound to bind the expression product.

Alternatively, the amount of test compound bound to the expression product can be determined by a combination of chromatography and spectroscopy. This can be achieved with technologies such as Biacore (Amersham Pharmacia). The amount of test compound bound to the expression product can also be determined by direct measurement of the change in mass upon compound or ligand binding to the expression product. Alternatively, the expression product, compound or ligand can be fluorescently labelled and the association of expression product with the test compound can be followed by changes in Fluorescence Energy Transfer (FRET).

The invention therefore includes a method of screening for pain alleviating compounds, comprising:
a) contacting a test compound or test compounds in the presence of a ligand with an expression product of any of the nucleic acid sequences of Tables I to X or with a cell expressing at least one copy of the expression product or with a lipidic matrix containing at least one copy of the expression product;
b) determining the binding of the test compound to the expression product, and
c) selecting test compounds on the basis of their binding abilities.

In the above method, the ligand may be added prior to, simultaneously with or after contact of the test compound with the expression product. Non limiting examples and methodology can be gained from the teachings of the *Molecular Probes handbook* and references therein (Molecular Probes, Inc., 4849 Pitchford Ave, Eugene, USA), *Methods in neurotransmitter receptor analysis* (Yamamura HI., Enna, SJ., and Kuhar, MJ., Raven Press New York, the *Glaxo Pocket Guide to Pharmacology,* Dr. Michael Sheehan, Glaxo Group Research Ltd, Ware, Herts SG12 0DP), *Bylund DB and Murrin LC* (2000, Life Sciences, 67 (24) 2897-911), *Owicki JC* (*2000, J. biomol Screen (5) 297-306), Alberts et al (1994, Molecular Biology of the Cell, 3*^{*rd*} *Edn, Garland Publication* *Inc), Butler JE, (2000 Methods 22(1):4-23, Sanberg SA* (2000, Curr Opin Biotechnol 11(1)47-53), and *Christopoulos A* (1999, biochem Pharmacol 58(5) 735-48).

### B) Functional screening

### (a) Kinase assays

The expression products of any of the nucleic acid sequences of Tables I to X which encodes a kinase, and in particular the nucleic acid sequences listed in Table I, are amenable to screening using kinase assay technology.

Kinases have the ability to add phosphate molecules to specific residues in ligands such as binding peptides in the presence of a substrate such as adenosine triphosphate (ATP). Formation of a complex between the kinase, the ligand and substrate results in the transfer of a phosphate group from the substrate to the ligand. Compounds that modulate the activity of the kinase can be determined with a kinase functional screen. Functional screening for modulators of kinase activity therefore involves contacting one or more test compounds with an expression product of one of the nucleic acid sequences of Tables I to X which encodes a kinase, and determining the ability of said test compound to modulate the transfer of a phosphate group from the substrate to the ligand.

The expression product can be part of an intact cell or of a lipidic preparation or it can be a purified polypeptide(s), optionally attached to a support, for example beads, a column, or a plate. Binding is preferably performed in the presence of ligand and substrate to allow an assessment of the binding activity of each test compound.

The ligand should contain a specific kinase recognition sequence and it should not be phosphorylated at its phosphoryation site. The ligand and/or substrate may be contacted with the kinase either before, simultaneously or after the test compound. Optionally the substrate may be labelled with a kinase transferable labelled phosphate, the assay being monitored by the phosphorylation state of the substrate and/or the ligand. The ligand should be such that its phosphorylation state can be determined. An alternative method to do this is to label the ligand with a phosphorylation-state-sensitive molecule. To achieve this, the ligand can be labelled in a number of ways, for example with a chromophore, radioactive, fluorescent, phosphorescent, enzymatic or antibody label. If the ligand is not directly detectable it should be amenable to detection and quantification by secondary detection, which may employ the above technologies. Such technologies are known to those in the art.

Binding of the test compound to the kinase modifies its ability to transfer a phosphate group from the substrate to the ligand. The difference between the observed amounts of phosphate transfer relative to the theoretical maximum amount of phosphate transfer is a reflection of the modulatory effect of the test compound. Alternatively, the degree of phosphate transfer can be determined by a combination of chromatography and spectroscopy. The extent of phosphorylation of the ligand peptide or dephosphorylation of the substrate can also be determined by direct measurement. This can be achieved with technologies such as Biacore (Amersham Pharmacia).

The invention also provides a method for screening compounds for the ability to relieve pain, which comprises:
(a) contacting one or more test compounds in the presence of ligand and substrate with an expression product of any of the nucleic acid sequences of Tables I to X which is a kinase or with a cell containing at least 1 copy of the expression product or with a lipidic matrix containing at least 1 copy of an expression product;
(b) determining the amount of phosphate transfer from the substrate to the ligand; and
(c) selecting test compounds on the basis of their capacity to modulate phosphate transfer.

Optionally ligand, substrate and/or other essential molecules may be added prior to contacting the test compound with expression product of step (a) or after step (a). Non limiting examples and methodology can be gained from the teachings of the *Molecular Probes handbook* and references therein (Molecular Probes, Inc., 4849 Pitchford Ave, Eugene, USA), *Methods in Molecular Biology 2000*; 99: 191-201, *Oncogene* 2000 20; 19(49): 5690-701, and *FASAB Journal,* (10, 6, P55, P1458, 1996, Pocius D Amrein K *et al*).

### b) Phosphatase assays

The expression products of any of the nucleic acid sequences of Tables I to X which encodes a phosphatase, and in particular the nucleic acid sequences listed in Table II, are amenable to screening using phosphatase assay technology.

Phosphatase enzymes have the ability to remove phosphate molecules from specific residues in ligands such as peptides. This reaction takes place in the presence of a substrate such as Adenosine Diphosphate (ADP). The complexing of the phosphatase polypeptide, ligand and substrate results in the transfer of a phosphate group from the ligand to substrate. Compounds that modulate the activity of the Phosphatase can be determined with a Phosphatase functional screen. This screen detects the reverse of the Kinase functional screen outlined above.

The invention also provides a method for screening compounds for the ability to relieve pain, which comprises:
(a) contacting one or more test compounds in the presence of ligand and substrate with an expression product of any of the nucleic acid sequences of Tables I to X which is a phosphatase or with a cell containing at least 1 copy of the expression product or with a lipidic matrix containing at least 1 copy of an expression product;
(b) determining the amount of phosphate transfer from the ligand to the substrate; and
(c) selecting test compounds on the basis of their capacity to modulate phosphate transfer.

Optionally ligand, substrate and/or other essential molecules may be added prior to contacting the test compound with expression product of step (a) or after step (a). Non-limiting examples and methodology can be gained from the teachings of the *Molecular Probes handbook* and references therein (Molecular Probes, Inc., 4849 Pitchford Ave, Eugene, USA), and *FASAB Journal,* (10, 6, P55, P1458, 1996, Pocius D Amrein K *et al*).

### c) Ion Channel Protein assays

An expression product of any of the nucleic acid sequences of Tables I to X which encodes an ion channel protein, and in particular the nucleic acid sequence listed in Table III, is amenable to screening using ion channel protein assay technology.

Ion channels are membrane associated proteins. They are divided into three main groups:
(1) ligand gated ion channels;
(2) voltage gated ion channels; and
(3) mechano gated ion channels.

Ion channels allow the passage of ions through cellular membranes upon stimulation by ligand, change in membrane potential or physical changes in environment such as temperature and pH. Compounds that modulate the activity of ion channels can be determined with an ion channel functional screen.

Functional screening for modulators of ion channels involves contacting a test compound with an expression product as aforesaid which is an ion channel protein or a fragment thereof and determining the ability of said test compound to modulate the activity of said expression product or fragment thereof. The expression product can be a part of an intact cell, membrane preparation or lipidic preparation, optionally attached to a support, for example beads, a column, or a plate. The ligand may be contacted with the ion channel peptide before, simultaneously with or after the test compound. Optionally other molecules essential for the function of the ion channel may be present. Ion channel opening is detectable with the addition of Ion channel sensitive dye, such dyes are known to those in the art. Examples are provided in *Molecular Probes handbook* and references therein (Molecular Probes, Inc., 4849 Pitchford Ave, Eugene, USA) and *Glaxo Pocket Guide to Pharmacology* (Dr Michael Sheenal Pharmacology Division Staff, Glaxo Group Research Ltd., Ware, Herts SG12 0DP). Binding of the test compound to the ion channel protein modifies its ability to allow ion molecules across a membrane. The difference between the observed amounts of movement of ions across a membrane relative to the theoretical maximum amount of ions that can move across the membrane is a reflection of the modulatory effect of the test compound.

The invention therefore also relates to a method of screening compounds for their ability to alleviate pain, which method comprises:
(a) contacting at least one test compound in the presence of voltage potential sensing dye with a cell containing at least 1 copy of an expression product as aforesaid which is an ion channel protein or with a lipidic matrix containing at least one copy of the expression product;
a) applying a ligand or stimulus;
b) determining the opening of the ion channel, and
c) selecting a test compound on the basis of its ability to modulate movement of ions across the membrane.

### d) Receptor assays

An expression product of any of the nucleic acid sequences of Tables I to X which encodes a receptor, and in particular any of the nucleic acid sequences listed in Table IV, is amenable to screening using receptor assay technology.

Receptors are membrane associated proteins that initiate intracellular signalling upon ligand binding. Therefore, the identification of molecules for the prevention and treatment of pain can be achieved with the use of a ligand-binding assay, as outlined above. Such an assay would utilize an endogenous or non-endogenous ligand as a component of the ligand-binding assay. The binding of this ligand to the receptor in the presence of one or more test compounds would be measures as described above. Such is the nature of receptors that the assay is usually, but not exclusively performed with a receptor as an intact cell or membranous preparation.

The invention therefore includes a method of screening for pain alleviating compounds, comprising:
a) contacting a test compound or test compounds in the presence of a ligand with cells expressing at least one copy of the expression product of any of the nucleic acid sequences of Tables I to X which is a receptor or with a lipidic matrix containing at least one copy of the expression product;
b) determining the binding of the test compound to the expression product, and
c) selecting test compounds on the basis of their binding abilities.

### e) Transporter protein assays

An expression product of any of the nucleic acid sequences of Tables I to X which encodes a transporter protein, and in particular any of the nucleic acid sequences listed in Table V, is amenable to screening using transporter protein assay technology. Non limiting examples of technologies and methodologies are given by *Carroll FI, et al* (1995, Medical Research Review, Sep15 (5) p419-444), *Veldhuis JD and Johnson Ml* (1994, Neurosci. Biobehav Rep., winter 18(4) 605-12), *Hediger MA and Nussberger S (1995,* Expt Nephrol, July-Aug 3(4) p211-218, *Endou H and Kanai Y,* (1999, Nippon Yakurigaku Zasshi, Oct. 114 Suppl 1:1p-16p), *Olivier B et al* (2000, Prog. Drug Res., 54, 59-119), *Braun A et al* (2000, Eur J Pharm Sci, Oct 11, Suppl 2 S51-60) and *Molecular Probes handbook and references therein* (Molecular Probes, Inc., 4849 Pitchford Ave, Eugene, USA).

The main function of transporter proteins is to facilitate the movement of molecules across a cellular membrane. Compounds that modulate the activity of transporter proteins can be determined with a transporter protein functional screen. Functional screening for modulators of transporter proteins comprises contacting at least one test compound with an expression product as aforesaid which is a transporter protein and determining the ability of said test compound to modulate the activity of said transporter protein. The expression product can be part of an intact cell, or lipidic preparation, optionally attached to a support, for example beads, a column or a plate. Binding is preferably performed in the presence of the molecule to be transported, which should only able to pass through a cell membrane or lipidic matrix with the aid of the transporter protein. The molecule to be transported should be able to be followed when it moves into a cell or through a lipidic matrix. Preferably the molecule to be transported is labelled to aid in characterization, e.g. with a chromophore, radioactive, fluorescent, phosphorescent, enzymatic or antibody label. If the molecule to be transported is not directly detectable it should be amenable to detection and quantification by secondary detection, which may employ the above technologies. The molecule to be transported may be contacted with the transporter protein before, simultaneously with or after the test compound. If binding of the test compound to the transporter protein modifies its ability to transport molecules through a membranous or lipidic matrix then the difference between the observed amount of transported molecule in a cell/or through a lipidic matrix relative to the theoretical maximum amount is a reflection of the modulatory effect of the test compound.

The invention further provides a method for screening compounds for their ability to relieve pain, comprising
a) contacting at least one test compound in the presence of transported molecules with a cell containing at least one copy of an expression product of any of the nucleic acid sequences of Tables I to X which is a transporter protein or with a lipidic matrix containing at least one copy of the expression product;
b) measuring the movement of transported molecules into or from the cell, or across the lipidic matrix; and
c) selecting test compounds on the basis of their ability to modulate the movement of transported molecules.

### f) G-protein coupled receptor protein assays

An expression product of any of the nucleic acid sequences of Tables I to X that encodes a G-protein coupled receptor protein, and in particular the nucleic acid sequences listed in Table VI, is amenable to screening using G-protein coupled receptor protein assay technology.

G-protein coupled receptor proteins (GPCRs) are membranous proteins whose main function is to transduce a signal through a cellular membrane. Upon ligand binding, GPCRs undergo a conformational change that allows complexing of the GPCRs with a G-protein. G-proteins possess a GTP/GDP binding site. The formation of the G-protein/ligand complex allows exchange of GTP for GDP, resulting in a conformational change of the G-protein. This conformational change initiates signal transduction.

Functional screening for modulators of GPCRs comprises contacting at least one test compound with an expression product as aforesaid which is a G-protein coupled receptor protein, and assessing the ability of the test compound(s) to modulate the exchange of GTP for GDP or the modulation of the GPCR signal transduction pathway. The expression product can be part of an intact cell or lipidic preparation, optionally attached to a support, for example beads, a column or a plate. Binding is preferably performed in the presence of a ligand, G-protein and GTP/GDP to allow an assessment of the binding activity of each test compound. Alternatively components of the signalling pathway are also included. In particular, in a preferred embodiment, a labelled GTP is used and the ability of the test compound(s) to modulate the exchange of GTP to GDP is determined. A further optional characteristic of the assay can be the inclusion of a reporter molecule that enables monitoring the regulation of the signalling pathway. The ligand may be contacted with the GPCR before, simultaneously with or after the test compound. Binding of the test compound to the GPCR modifies its ability to modulate the exchange of GTP for GDP and hence the modulation of signal transduction. The difference between the observed amount of GTP exchanged for GDP relative to the theoretical maximum amount of GTP is a reflection of the modulatory effect of the test compound. Likewise the relative activities of signal transduction reporter molecules are also a reflection of the modulatory effect of the test compound. Non limiting examples of technologies and methodologies can be found in *Molecular Probes handbook* and references therein (Molecular Probes, Inc., 4849 Pitchford Ave, Eugene, USA), *Glaxo Pocket Guide to Pharmacology,* (Michael Sheehan, Pharmacology Division staff, Glaxo Group Research Ltd., Ware, Herts SC12 0DP) and *Xing et al* (2000, J. Recept. Signal. Transduct. Res. 20(40 189-210).

The invention provides a method of screening compounds for their ability to relieve pain, comprising:
a) contacting at least one test compound in the presence of a ligand, GTP/GDP, G-protein with a cell containing at least one copy of an expression product of any of the nucleic acid sequences of Tables I to X which is a G-protein coupled receptor protein or with a lipidic matrix containing at least one copy of the expression product;
b) measuring the exchange of GTP for GDP, and
c) selecting test compounds on the basis of their ability to modulate said exchange.

In the above method, the ligand, GTP/GDP, G-protein and other essential molecules can be added before, simultaneously with or after the contacting of the test compound(s) with the cell line or lipidic matrix in step a).

### g) DNA-binding protein assays

An expression product of any of the nucleic acid sequences of Tables I to X that encodes a DNA-binding protein, and in particular any of the nucleic acid sequences listed in Table VII, is amenable to screening using DNA-binding protein assay technology.

DNA binding proteins are proteins that are able to complex with DNA. The complexing of the DNA binding protein with the DNA in some instances requires a specific nucleic acid sequence. Screens can be developed in a similar manner to ligand binding screens as previously indicated and will utilise DNA as the ligand. DNA-binding protein assays can be carried out using similar principles described in ligand binding assays as described above. Non limiting examples of methodology and technology can be found in the teachings of *Haukanes BI and Kvam* C (Biotechnology, 1993 Jan 11 60-63), *Alberts B et al* (Molecular Biology of the Cell, 1994, 3^{rd} Edn., Garland Publications Inc, *Kirigiti P and Machida CA* (2000 Methods Mol Biol, 126, 431-51) and *Molecular Probes handbook* and references therein (Molecular Probes, Inc., 4849 Pitchford Ave., Eugene, USA).

The invention therefore includes a method of screening for pain alleviating compounds, comprising:
a) Contacting a test compound or test compounds in the presence of a plurality of nucleic acid sequences with an expression product of any of the nucleic acid sequences of Tables I to X which is a DNA binding protein or with cell expressing at least one copy of the expression product or with a lipidic matrix containing at least one copy of the expression product;
b) Determining the binding of the test compound to the expression product, and
c) Selecting test compounds on the basis of their binding abilities.

In the above method, the plurality of nucleic acid sequence may be added prior to, simultaneously with or after contact of the test compound with the expression product.

### h) Protease assays

An expression product of any of the above nucleic acid sequences that encodes a protease protein, and in particular any of the nucleic acid sequences listed in Table VIII, is amenable to screening using protease protein assay technology. Non-limiting examples of such technologies are illustrated in *Molecular Probes handbook* and references therein (Molecular Probes, Inc., 4849 Pitchford Ave, Eugene, USA), *Cook et al* (1992, Structure and function of the aspartic proteinases, Ed. Dunn BM, Plenum Press, New York, 525-528) and *Peranteau AG et al* (1995, Anal Biochem, 1, 227(1) 242-5).

Protease proteins have the property of being able to cleave peptide sequences. In brief, one characteristic of the protease-screening assay is the peptide ligand, which may contains at least one copy of a protease protein recognition sequence. The cleavage of this polypeptide by the protease results in the fragmentation of the peptide ligand. The propensity of the protease to fragment the peptide ligand in the presence of a candidate compound(s) is a measure of the inhibitory qualities of the candidate compound(s).

The protease-screening assay may be performed in the presence of at least a ligand binding peptide and a protease. These screening assays can be performed in the presence of isolated expression product, expression product in cells or expression product in a lipidic matrix. The invention therefore includes a method of screening for pain alleviating compounds, comprising:
(a) contacting one or more test compounds in the presence of peptide ligand with an expression product of any of the nucleic acid sequences of Tables I to X which is a protease or with a lipidic matrix containing at least one copy of the expression product;
(b) determining the amount of cleavage of peptide ligand; and
(c) selecting test compounds on the basis of their ability to modulate the amount of ligand cleavage.

### i) Assays using other enzymes

Expression products of any of the nucleic acid sequences of Tables I to X that encode other enzymes, e.g. ligases, lyases, oxidoreductases, transferases and hydrolases, and in particular any of the nucleic acid sequences listed in Table IX, is amenable to screening using appropriate assay technology. Each class of enzyme has a defined function.

Ligases have the property of being able to splice molecules together. This is achieved with the conversion of ATP substrate to AMP. Therefore, the activity of a ligase can be followed by monitoring the conversion of ATP to AMP. Such technologies are known to those in the art. Non-limiting examples and methodologies can are illustrated by Ghee. *T. Tan et al* (1996,Biochem J. 314, 993-1000, *Yang SW et al* (1992, 15: 89(6) 2227-31 and references therein, and in *Molecular Probes handbook* and references therein (Molecular Probes, Inc., 4849 Pitchford Ave, Eugene, USA).

The invention also provides methods of screening for pain alleviating compounds, comprising;
a) contacting one or more test compounds in the presence of ATP with an expression product of any of the nucleic acid sequences of Tables I to X which is a ligase or with a cell expressing at least 1 copy of a expression product or with a lipidic matrix containing at least 1 copy of an expression product;
b) determining the amount of ATP converted to AMP, and
c) selecting test compounds on the basis of their ability to modulate said conversion.

Lyases are enzymes which catalyse the cleavage of by reactions other than hydrolysis. These enzymes can be grouped into seven groups according to type of bond cleaved. These groups are:
carbon-carbon lyases (E.C. No 4.1);
carbon-oxygen lyases (E.C. No 4.2);
carbon-nitrogen lyases (E.C. No 4.3);
carbon-sulphur lyase (E.C. No 4.4);
carbon-halide lyases (E.C. No 4.5);
phosphorous-oxygen lyases (E.C. No 4.6); and
other lyases (E.C. No 4.99) (Analytical Biochemistry 3^{rd} Edn, David J. Holme and Hazel Peck, Longman press).

The enzyme commission number (E.C.) of the International Union of Biochemistry relates to the type of reaction catalysed by the enzyme. Further teachings on how to develop assays and screens for lyases can be obtained from *Methods in Enzymology* (Academic Press).

Oxidoreductases are enzymes that catalyse the transfer of hydrogen or oxygen atoms or electrons. These enzymes can by sub-grouped into twenty categories according to their specific mode of action. These groups are oxidoreductases acting on:
the CH-OH group of donors (E.C. No1.1);
the aldehyde or oxo group of donors (E.C. No 1.2);
the CH-CH group of the donor (E.C. No 1.3);
the CH-NH2 group of donors (E.C. 1.4);
CH-NH group of donors (E.C. 1.5);
NADH or NADPH (E.C. No 1.6);
other nitrogen compounds as donors (E.C. No 1.7);
a sulphur group of donors (E.C. No 1.8);
a haem group of donors (E.C. No1.9);
diphenols and related substances as donors (B.C. 1.10);
hydrogen peroxide as acceptor (E.C. No 1.11);
hydrogen as donor (E.C. No 1.12);
single donors with incorporation of molecular oxygen (E.C. No 1.13);
oxidoreductases acting on paired donors with incorporation of molecular oxygen (E.C. No 1.14);
superoxide radicals as acceptors (E.C. 1.15);
oxidizing metal ions (E.C. No 1.16);
oxidoreductases acting on -CH2 groups (E.C. No 1.17);
reduced ferredoxin as donor (E.C. No 1.18);
reduced flavodoxin as donor (E.C. No 1.19); and
other oxidoreductases (E.C. No 1.97) (Analytical Biochemistry 3^{rd} Edn, David J. Holme and Hazel Peck, Longman Press).

The enzyme commission number (E.C.) of the International Union of Biochemistry relates to the type of reaction catalysed by the enzyme. Further teachings on how to develop assays and screens for oxidoreductases can be obtained from *Methods in Enzymology* (Academic Press) with special reference to volume 249.

Transferases are enzymes that catalyse the transfer of specific groups. They are classified into eight sub groups according to function, transferring one-carbon group (E.C. No 2.1), Transferring aldehyde or ketonic residues (E.C. No 2.2), Acetyltransferases (E.C. 2.3), glycosyltransferases (E.C. No 2.4), transferring alkyl or aryl groups other than methyl groups (E.C. No 2.5), transferring nitrogenous groups (E.C. No 2.6), transferring phosphorous-containing groups (E.C. No 2.7) and transferring sulphur-containing groups (E.C. No 2.8) (Analytical Biochemistry 3^{rd} Edn, David J. Holme and Hazel Peck, Longman Press). The enzyme commission number (E.C.) of the International Union of Biochemistry relates to the type of reaction catalysed by the enzyme. Further teachings on how to develop assays and screens for transferases can be obtained from Methods in Enzymology (Academic Press).

Hydrolases are enzymes that catalyse hydrolytic reactions and are sub-grouped into eleven classes according to the type of reaction they carry out, viz:
ester bonds (E.C. No 3.1);
glycosyl compounds (E.C. No 3.2);
ether bonds (E.C. No 3.3);
peptide bonds (E.C. No 3.4);
hydrolases acting on carbon-nitrogen bonds, other than peptide bonds (E.C. No 3.5);
acid anhydrides (E.C. No 3.6);
carbon-carbon bonds (E.C. No 3.7);
halide bonds (E.C. No 3.8);
phosphorous-nitrogen bonds (E.C. No 3.9);
sulphur-nitrogen bonds (E.C. No 3.10); and
hydrolases acting on carbon-phosphorous bonds. (See Analytical Biochemistry 3^{rd} End, David J. Holmes and Hazel Peck, Longman Press).

The enzyme commission number (E.C.) of the International Union of Biochemistry relates to the type of reaction catalyzed by the enzyme. Further teachings on how to develop assays and screens for hydrolases can be obtained from Methods in Enzymology (Academic Press) with special reference to volume 249.

### C) In vivo functional screen

Any of the nucleic acid sequences described in Tables I to X or homologues thereof may be inserted by means of an appropriate vector into the genome of a lower vertebrate or of an invertebrate animal or may be inactivated or down regulated in the genome of said animal. The resulting genetically modified animal may be used for screening compounds for effectiveness in the regulation of pain. The invertebrate may, for example, be a nematode e.g. *Caenorhabditis elegans*, which is a favourable organism for the study of response to noxious stimuli. Its genome sequence has been determined, see *Science,* **282,** 2012 (1998), it can be bred and handled with the speed of a micro-organism (it is a self-fertilizing hermaphrodite) and can therefore be used in a high throughput screening format (WO 00/63424, WO 00/63425, WO 00/63426 and WO 00/63427), and it offers a full set of organ systems, including a simple nervous system and contains many similarly functioning genes and signaling pathways to mammals. A thermal avoidance model based on a reflexive withdrawal reaction to an acute heat stimulus has been described by Wittenburg *et al, Proc. Natl. Acad. Sci. USA*, **96,** 10477-10482 (1999), and allows the screening of compounds for the treatment of pain with the modulation of pain sensation as an endpoint.

The genome of *C. elegans* can be manipulated using homologous recombination technology which allows direct replacement of nucleic acids encoding *C. elegans* with their identified mammalian counterpart. Replacement of these nucleic acids with those nucleic acids outlined above would allow for the direct screening of test compound(s) with their expression products. Any of the pain-related genes described above may be ligated into a plasmid and introduced into oocytes of the worm by microinjection to produce germline transformants. Successful plasmid injection into *C. elegans* and expression of inserted sequences has been reported by Devgen B.V., Ghent, Belgium. It is also possible to produce by routine methods worms in which the target sequences are down-regulated or not expressed (knock-out worms). Further non limiting examples of methodology and technology can be found in the teachings of *Hazendonk et al* (1997, Nat genet. 17(1) 119-21), *Alberts et al*, (1994, Molecular Biology of the Cell 3^{rd} Ed. Garland Publishing Inc, *Caenorhabditis elegans* is anatomically and genetically simple), *Broverman S et al*, (1993, PNAS USA 15; 90(10) 4359-63) and Mello et al (1991, 10(12) 3959-70).

A further method for screening compounds for ability to modify response to pain, e.g. relieve pain, comprises:
(a) contacting one or more test compounds with at least one *C elegans* containing at least one copy of a sequence as set out above;
(b) subjecting the *C. elegans* to a nociceptive stimulus;
(c) observing the response of the *C.elegans* to said stimulus; and
d) selecting test compounds on the basis of their ability to modify the response of *C. elegans* to said stimulus.

### DIAGNOSTIC TOOLS AND KITS

### Affinity peptides, ligands and substrates

Pain associated polypeptides and fragments thereof can be detected at the tissue and cellular levels with the use of affinity peptides, ligands and substrates, which will enable a skilled person to define more precisely a patient's ailment and help in the prescription of a medicament. Such affinity peptides are characterized in that firstly they are able to bind specifically to a pain associated polypeptide, and secondly that they are capable of being detected. Such peptides can take the form of a peptide or polypeptide for example an antibody domain or fragment, or a peptide/polypeptide ligand or substrate, or a polypeptide complex such as an antibody.

The preparation of such peptides and polypeptides are known to those in the art. Antibodies, these may be polyclonal or monoclonal, and include antibodies derived from immunized animals or from hybridomas, or derivatives thereof such as humanized antibodies, Fab or F(ab')2 antibody fragments or any other antibody fragment retaining the antigen binding specificity.

Antibodies directed against pain-associated gene product molecules may be produced according to conventional techniques, including the immunization of a suitable mammal with the peptides or polypeptides or fragment thereof. Polyclonal antibodies can be obtained directly from the serum of immunized animals. Monoclonal antibodies are usually produced from hybridomas, resulting from a fusion between splenocytes of immunized animals and an immortalized cell line (such as a myeloma). Fragments of said antibodies can be produced by protease cleavage, according to known techniques. Single chain antibodies can be prepared according to the techniques described in US 4,946,778. Detection of these affinity peptides could be achieved by labelling. Technologies that allow detection of peptides, such as enzymatic labelling, fluorescence labelling or radiolabelling are well known to those in the art. Optionally these affinity peptides, ligands and substrates could themselves be detected with the use of a molecule that has specific affinity to the peptides, ligands and substrates and is itself labelled.

The invention further provides a kit, which comprises;
(a) affinity peptide and/or ligand and/or substrate for an expression product of a gene sequence that is down-regulated in the spinal cord of a mammal in response to streptozocin-induced diabetes; and
(b) a defined quantity of an expression product of a gene sequence that is down-regulated in the spinal cord of a mammal both in response to streptozocin-induced diabetes, for simultaneous, separate or sequential use in detecting and/or quantifying an expression product of a gene sequence that is down-regulated in the spinal cord of a mammal in response to streptozocin-induced diabetes

### Complimentary nucleic acids

Pain associated nucleic acid sequences can be characterized at the tissue and cellular levels with the use of complimentary nucleic acid sequences. Detection of the level of expression of pain associated nucleic acid sequences can help in the prognosis of a pain condition and the prescription of a medicament. These complimentary nucleic acid sequences are characterized in that they can hybridize to a pain associated nucleic acid sequence and their presence can be detected through various techniques. Such techniques are known to those in the art and may include detection by polymerase chain reaction or detection by labelling of complimentary nucleic acid sequences by enzymatic labelling, affinity labelling fluorescent labelling or radio-labelling. Complimentary strand nucleic acids of this invention are 10 to 50 bases long, more preferably 15 to 50 bases long and most preferably 15 to 30 bases long, and hybridize to the coding sequence of the nucleic acid.

A further aspect of this invention is a kit that comprises:
(a) nucleic acid sequences capable of hybridization to a nucleic acid sequence that is down-regulated in the spinal cord of a mammal in response streptozocin-induced diabetes and
(b) a defined quantity of one or more nucleic acid sequences capable of hybridization to a nucleic acid sequence that is down-regulated in the spinal cord of a mammal in response to streptozocin-induced diabetes, for simultaneous, separate or sequential use in detecting and/or quantifying a gene sequence that is down-regulated in the spinal cord of a mammal in response to streptozocin-induced diabetes.

### IDENTIFICATION AND VALIDATION

Subtractive hybridization enables the identification of nucleic acid sequences whose expression profiles are modified by a stimulus. Upon stimulation of a system (in the case of this invention a nociceptive stimulus on an animal model) all observed changes in the level of nucleic acid sequence expression are due to the reaction of the system to the stimulus. Characterization of these changes in expression by way of identification of nucleic acid sequence and level of expression is both identification and validation.

The inventors have developed a four step process which allows for the simultaneous identification and validation of nucleic acid sequences whose expression are regulated by a pain stimulus, preferably a chronic pain stimulus, and more preferably a diabetic pain stimulus. This process comprises the following steps:
(a) induction of a nociceptive stimulus in test animals;
(b) extraction of nucleic acid sequences from specific neuronal tissue of test and control animals;
(c) selective amplification of differentially expressed nucleic acid sequences; and
(d) identification and characterization of differentially expressed gene products that are modulated by a nociceptive stimulus.

The above process is described in more detail below.

### (a) Induction of nociceptive stimulus

The effect of the selected nociceptive stimulus on the test animal needs to be confirmable. The test subjects are therefore a species that has a "developed" nervous system, preferably similar to that of humans, most preferably rats or mice. Advantageously, the nociceptive stimulus is analogous to known pain paradigms in humans. One such paradigm of pain is the pain associated with diabetes, which can be induced in rodents with the use of streptozotocin (STZ).

Streptozotocin (STZ) induces hyperglycemia and Type 1 diabetes mellitus in rats. In particular, STZ contains a glucose analogue that allows it to be taken up by the glucose transporter 2 present on the surface of pancreatic β cells, the site of insulin synthesis. Once inside the cell, STZ causes a reduction in the level of nicotinamide adenine dinucleotide (NAD⁺). The decrease in NAD⁺ levels eventually leads to necrosis of the pancreatic β cell, causing a reduction in insulin levels and then diabetes, leading to neuropathy (diabetic) and neuropathic pain (R. B. Weiss, *Cancer Treat. Rep.,* **66,** 427-438 1982, Guy *et al, Diabetologica,* **28,** 131-137 1985; Ziegler *et al, Pain*, **34,** 1-10 1988; Archeret *al, J. Neurol. Neurosurg. Psychiatry*, **46,** 491-499 1983). The diabetic rat model has been shown to be a reliable model of hyperalgesia. We have used an STZ-induced diabetic rat model to create a state of hyperlagesia that can be compared with control animals (Courteix *et al, Pain*, **53,** 81-88 1993).

### (b) Extraction of nucleic acids from neuronal tissue of test and control animals

The inventors have determined that RNA extraction of whole spinal cord nervous tissue provides a way of identifying nucleic acid sequences whose expression is modulated by streptozotocin induced pain. Test (subjected to the nociceptive stimulus) and control animals were sacrificed, and the tissue to be studied e.g. neural tissue separated. Techniques for so doing vary widely from animal to animal and will be familiar to skilled persons.

A cDNA library can be prepared from total RNA extracted from neural tissue of the test and control animals. Where possible, however, it is preferred to isolate the mRNA from the total RNA of the test and control animals, by affinity chromatography on oligo (dT)-cellulose, and then reverse transcribe the mRNA from the test and control animals to give test and control cDNA. Converting mRNA from the test and control animals to corresponding cDNA may be carried out by any suitable reverse transcription method, e.g. a method as described by Gubler & Hoffman, *Gene,* 25, 263-269 (1983). If desired a proprietary kit may be used e.g. the CapFinder PCR cDNA Library Construction Kit (Life Technologies) which is based on long-distance PCR and permits the construction of cDNA libraries from nanograms of total RNA.

### (c) Selective amplification of differentially expressed nucleic acids

The reverse transcribed cDNA of the test and control animals is subjected to subtractive hybridisation and amplification so that differentially expressed sequences become selectively amplified and commonly expressed sequences become suppressed, so as to over-produce DNA associated with said nociceptic stimulus. A wide range of subtractive hybridisation methods can be used, but the preferred method is so-called suppression subtractive hybridisation, see US-A-5565340 and Diatchenko *et al, Proc. Nat. Acad. Sci. USA,* 93, 6025-6030 (1996), the disclosures of which are herein incorporated by reference. Kits for carrying out this method are available from CLONTECH Laboratories, Inc.

### (d) Cloning and Sequencing the differentially expressed cDNA

The differentially expressed cDNA is ligated into a cloning vector, after which cells of E. *coli* are transformed with the vector and cultured. Positive clones are selected and burst to release plasmids containing the cDNA insert. The plasmids are primed using forward and reverse primers to either side of the cloning site and the cDNA insert is sequenced. Vector and adaptor sequences are then removed from the output data from the sequencer, leaving only the nucleotide sequence of the differentially expressed gene. The sequence is then checked against data held in a database for homology to known genes, expressed sequence tags (ESTs) and proteins.

### (e) Validation of the above method

The importance of the sequences that we have identified in pain is confirmed by the fact that genes that represent nucleic acid sequences which have previously been implicated in pain, including Calmodulin (pRCM1, Genebank X13933), Enkephalin (Genebank Y07503) and Neurotensin receptor type 2 (Genebank X97121) have also been identified using this method.

The inventors have identified nucleic acid sequences of the MAP kinase pathway, a previously non pain-associated biological pathway. The inventors have subsequently shown that intra-spinal injection of a MEK inhibitor (MEK is part of the MAP kinase pathway) produces a powerful inhibition of pain (Patent application No US 60/144292). Subsequently, it has been shown that the MAP kinase is also implicated in acute inflammatory pain (Woolf *et al*, Nature Neuroscience 1999).

The invention will now be further described in the following Example.

### EXAMPLE

### Induction of diabetes

Diabetes was induced in adult (150-200g) male Sprague-Dawley rats (n=6) as described by Courteix *et al (supra).* Animals were injected intraperitoneally with streptozotocin (STZ)(50 mg/kg) dissolved in distilled water. Control or sham animals (age-matched animals, n=6) were injected with distilled water only.

### Nociceptive testing

Static allodynia (a form of hyperlagesia) was measured using a method described by Chaplan *let al*, "Quantitative assessment of tactile allodinya in the rat paw", *J. Neurosci. Methods,* **53**, 55-63 (1994). A series of von Frey filaments of different buckling weight (i.e. the load required for the filament to bend) were applied to the plantar surface of the right hand paw. The starting filament had a buckling weight of 20g. Lifting of the paw was taken to be a positive result, in which case a filament with the next lowest buckling weight was used for the next measurement. The test was continued until a filament was found for which there was an absence of response for longer than 5 seconds whereas a re-test with the next heaviest filament gave a positive response. Animals were considered hyperalgesic if their thresholds were found to be <4g of those of comparable untreated rats, see Calcutt & Chaplan, "Spinal pharmacology of tactile allodynia in diabetic rats", *British J. Pharmacol*, **122,** 1478-1482 (1997).

### Tissue Extraction

STZ-treated and control animals were anaesthetized with 4% halothane and perfused with ice-cold 0.9% saline containing 1% citric acid (pH adjusted to 7.4 with NaOH). The animals were decapitated and the lumbar spinal cord exposed. A 2-centimetre length of spinal cord ending at L6 (lumbar-6 forward) was removed from the spinal column. Attached dorsal root ganglia and contaminating spinal connective tissues were removed. The spinal cord tissue was snap frozen in dry ice and isopentane.

### Total RNA Extraction

Total RNA was extracted from the above pooled male rat tissues using the TRIZOL Reagent Kit (Life Technologies). Briefly, tissue samples were homogenised fully using a Polytron homogenizer in 1ml of TRIZOL reagent per 50-100mg of tissue. Homogenized samples were then incubated at room temperature for 5 minutes and phase separated using 0.2ml chloroform per 1ml TRIZOL reagent followed by centrifugation at 3,000g. The aqueous phase was transferred to a fresh tube and the RNA precipitated with an equal volume of isopropyl alcohol and followed by centrifugation at 10,000g. The RNA pellet was washed in 75% ethanol and re-centrifuged. The pellet was then air dried and re-suspended in water.

### mRNA Extraction

In contrast to ribosomal RNA and transfer RNA, the vast majority of mRNAs of mammalian cells carry tracts of poly(A⁺) at their 3' termini. mRNAs can therefore be separated from the bulk of cellular RNA by affinity chromatography on oligo (dT)-cellulose. mRNA was extracted from Total RNA using the MESSAGEMAKER Kit (Life Technologies) in which mRNA (previously heated to 65°C in order to disrupt secondary structures and so expose the poly (A⁺) tails) was bound to oligo(dT) cellulose under high salt concentrations (0.5M NaCI) in a filter syringe. Unbound RNA was then washed away and the poly(A⁺) mRNA eluted in distilled water. A tenth of the volume of 7.5 M Ammonium Acetate, 50µg of glycogen/ml mRNA sample and 2 volumes of absolute alcohol were then added to the samples which are then placed at -20°C overnight. Following precipitation, the mRNA was spun down at 12,000g for 30 minutes at 4°C. RNAase free water was used to re-suspend the pellets, which were then and stored at - 80°C.

### PCR SELECT

The technique used was based on that of the CLONTECH PCR Select Subtraction Kit. The following protocol was performed using STZ-treated lumbar spinal cord Poly A ⁺ RNA as the 'Tester' and Sham lumbar spinal cord poly A⁺ RNA as the 'Driver' (Forward Subtraction). A second subtraction experiment using the Sham lumbar spinal cord mRNA as the 'Tester' and STZ treated lumbar spinal cord mRNA as the 'Driver' (Reverse Subtraction) was performed in parallel using the same reagents and protocol. As a control for both experiments, the subtraction was also carried out using human skeletal muscle mRNA that had been provided by the kit manufacturer.

### First-Strand cDNA Synthesis

2 µg of PolyA⁺ RNA and 1 µl of cDNA synthesis primer (10 □ M) were combined in a 0.5ml Eppendorf tube and sterile H₂O was added where necessary to achieve a final volume of 5 µl. The contents were mixed gently and incubated in a thermal cycler at 70°C for 2 min. The tubes were then cooled on ice for two minutes, after which 2 µl of 5X First-strand buffer, 1 µl of dNTP mix (10 mM each), sterile H₂O and 1 µl of AMV reverse transcriptase (20 units/µl) was also added. The tubes were then placed at 42°C for 1.5 hr in an air incubator. First-strand cDNA synthesis was terminated by placing the tubes on ice. (the human skeletal muscle cDNA produced by this step was used as the 'control driver' in later steps).

### Second-Strand cDNA Synthesis

48.4 µl of Sterile H₂O, 16.0 µl of 5X Second-strand buffer, 1.6 µl of dNTP mix (10 mM) and 4.0 µl of 20X Second-strand enzyme cocktail were added to each of the first-strand synthesis reaction tubes. The contents were then mixed and incubated at 16°C in a thermal cycler for 2 hr. 6 units (2 µl) of T4 DNA polymerase was then introduced and the tubes were incubated for a further 30 min at 16°C. In order to terminate second-strand synthesis, 4 µl of 20X EDTA/glycogen mix was added. A phenol:chloroform extraction was then carried out using the following protocol:-

100 µl of phenol:chloroform:isoamyl alcohol (25:24:1) was added to the tubes which were then vortexed thoroughly and centrifuged at 14,000 rpm for 10 min at room temperature. The top aqueous layer was removed and placed in a fresh tube. 100 µl of chloroform:isoamyl alcohol (24:1) was then added to the aqueous layer and the tubes were again vortexed and centrifuged at 14,000 rpm for 10 min. 40 µl of 4 M NH₄OAc and 300 µl of 95% ethanol were then added and the tubes centrifuged at 14,000 rpm for 20 min. The supernatant was removed carefully, then 500 µl of 80% ethanol was added to the pellet. The tubes were centrifuged at 14,000 rpm for 10 min and the supernatant was removed so that the pellet could be air-dried. The precipitate was then dissolved in 50 µl of H₂O. 6 µl was transferred to a fresh microcentrifuge tube. The remainder of the sample was stored at -20°C until needed.

### Rsa I Digestion

All products of the above procedures were subjected to a restriction digest, using the restriction endonuclease *Rsa* I, in order to generate ds cDNA fragments that are short and thus are optimal for subtraction hybridisation due to the standard kinetics of the hybridisation. Also, as *Rsa* I makes a double stranded cut in the middle of a recognition sequence, 'blunt ends' of a known nucleotide sequence are produced allowing ligation of adaptors onto these ends in a later step. The following reagents were added to the 6 µl product of the second hybridisation (see above): 43.5 µl of ds cDNA, 5.0 µl 10X *Rsa* I restriction buffer and 1.5 µl of *Rsa* I (10 units/ µl). The reaction mixture was incubated at 37°C for 1.5 hr. 2.5 µl of 20X EDTA/glycogen mix was used to terminate the reaction. A phenol:chloroform extraction was then performed as above (second-strand cDNA synthesis section). The pellet produced was then dissolved in 5.5 µl of H₂O and stored at -20°C until needed. The preparation of the experimental 'Driver' cDNAs and the control skeletal muscle cDNA was thus completed.

### Adaptor Ligation

The adaptors were not ligated to the driver cDNA.

1 µl of each *Rsa* I-digested experimental cDNA (from the *Rsa* I Digestion above) was diluted with 5 µl of sterile water. Preparation of the control skeletal muscle tester cDNA was then undertaken by briefly centrifuging the tube containing control DNA (*Hae* III-digest of φX174 DNA [3 ng/ µl]) and diluting 2 µl of the DNA with 38 µl of sterile water (to 150 ng/ml). 1 µl of control skeletal muscle cDNA (from the *Rsa* I Digestion) was then mixed with 5 µl of the diluted φX174/ *Hae* III DNA (150 ng/ml) in order to produce the control skeletal muscle tester cDNA.

### Preparation of the adaptor-ligated tester cDNA

A ligation master mix was prepared by combining 3 µl of Sterile water, 2 µl of 5X ligation buffer and 1 µl T4 DNA ligase (400 units/µl) per reaction. 2 µl of adaptor 1 (10 µM) was then added to 2 µl of the diluted tester cDNA. To this, 6 µl of the ligation master mix was also added. The tube was therefore labelled Tester 1-1. In a separate tube, 2 µl of the adaptor 2R (10 µM) was mixed with 2 µl of the diluted tester cDNA and 6 µl of the master mix. This tube was named Tester 1-2.

2 µl of Tester 1-1 and 2 µl of Tester 1-2 were then placed into fresh tubes. These would later be used as the unsubtracted tester control. The remainder of the contents of Tester 1-1 and Tester 1-2 tubes were then centrifuged briefly and incubated at 16°C overnight. The ligation reaction was stopped by adding 1 µl of EDTA/glycogen mix and the samples were heated at 72°C for 5 min in order to inactivate the ligase. In doing so, preparation of the experimental and control skeletal muscle adaptor-ligated tester cDNAs was complete.

1 µl from each Unsubtracted tester control was then removed and diluted into 1 ml of water. These samples were set aside as they were to be used later for PCR (see below). All of the samples were stored at -20°C

### Analysis of Ligation efficiency

1 µl of each ligated cDNA was diluted into 200 µl of water and the following reagents were then combined in four separate tubes:

| **Component** | **Tube:** | **1** | **2** | **3** | **4** |
|---|---|---|---|---|---|
| Tester 1-1 (ligated to Adaptor 1) | | 1 | 1 | - | - |
| Tester 1-2 (ligated to Adaptor 2R) | | - | - | 1 | 1 |
| G3PDH 3' primer (10 µM) | | 1 | 1 | 1 | 1 |
| G3PDH 5' primer (10 µM) | | - | 1 | - | 1 |
| PCR primer 1 (10 µM) | | 1 | - | 1 | - |
| | | | | | |
| Total volume µl | | 3 | 3 | 3 | 3 |

A master mix for all of the reaction tubes plus one additional tube was made up by adding 18.5 µl of sterile H₂O, 2.5 µl of 10X PCR reaction buffer, 0.5 µl of dNTP mix (10 mM), and 0.5 µl of 50X Advantage cDNA Polymerase Mix, per reaction, into a fresh tube. 22 µl of this master mix was then aliquotted into each of the 4 reaction tubes prepared above. The contents of the tubes were overlaid with 50 µl of mineral oil. The reaction mix was incubated in a thermal cycler at 75°C for 5 min in order to extend the adaptors. The following protocol was then carried out immediately in a thermal cycler (Perkin-Elmer GeneAmp PCR Systems 2400): 94°C for 30 sec (1 cycle), 94°C 10 sec, 65°C 30 sec and then 68°C 2.5 min (25 cycles)

### First Hybridisation

1.5 µl of the Adaptor 1-ligated Tester 1-1 was combined with 1.5 µl of the *Rsa* I-digested driver cDNA, prepared earlier and 1 µl of 4X Hybridisation buffer. This process was then repeated combining the Adaptor 2R-ligated Tester 1-2 with the *Rsa* I-digested driver cDNA and 4X Hybridisation buffer. The samples were incubated in a thermal cycler at 98°C for 1.5 min followed by incubation at 68°C for 8 hr.

### Second Hybridisation

1 µl of Driver cDNA (i.e. the *Rsa* I-digested cDNA (see above)), 1 µl 4X Hybridisation buffer and 2 µl Sterile H₂O were all combined in a fresh tube. 1 µl of this mix was then removed and placed in a new tube, overlaid with 1 drop of mineral oil and incubated at 98°C for 1.5 min in order to denature the driver. The following procedure was used to simultaneously mix the driver with hybridisation samples 1 and 2 (prepared in the first hybridisation), thus ensuring that the two hybridisation samples were mixed together only in the presence of freshly denatured driver: A micropipettor was set at 15 µl. The pipette tip was then touched onto the mineral oil/sample interface of the tube containing hybridisation sample 2. The entire sample was drawn partway into the tip before it was removed from the tube in order to draw a small amount of air into the tip. The pipette tip was then touched onto the interface of the tube containing the freshly denatured driver (i.e. the tip contained both samples separated by a small pocket of air) before the entire mixture was transferred to the tube containing hybridisation sample 1. The reaction was then incubated at 68°C overnight. 200 µl of dilution buffer was added to the tube, which was then heated in a thermal cycler at 68°C for 7 min. The product of this second hybridisation was stored at -20°C.

### PCR Amplification

Seven PCR reactions were set up: (1) The forward-subtracted experimental cDNA, (2) the unsubtracted tester control (see preparation of the adaptor ligated tester cDNA), (3) the reverse-subtracted experimental cDNA, (4) the unsubtracted tester control for the reverse subtraction, (5) the subtracted control skeletal muscle cDNA, (6) the unsubtracted tester control for the control subtraction, and (7) the PCR control subtracted cDNA (provided in the kit). The PCR control subtracted cDNA was required to provide a positive PCR control as it contained a successfully subtracted mixture of *Hae* III-digested φX174 DNA.

The PCR templates were prepared by aliquotting 1 µl of each diluted cDNA (i.e., each subtracted sample from the second hybridisation and the corresponding diluted unsubtracted tester control produced by the adaptor ligation, see above) into an appropriately labelled tube. 1 µl of the PCR control subtracted cDNA was placed into a fresh tube. A master mix for all of the primary PCR tubes, plus one additional reaction, was then prepared by combining 19.5 µl of sterile water, 2.5 µl of 10X PCR reaction buffer, 0.5 µl of dNTP Mix (10 mM), 1.0 µl of PCR primer 1 (10 µM) and 0.5 µl of 50X Advantage cDNA Polymerase Mix. 24 µl of Master Mix was then aliquotted into each of the 7 reaction tubes prepared above and the mixture was overlaid with 50 µl of mineral oil, before being incubated in a thermal cycler at 75°C for 5 min in order to extend the adaptors. Thermal cycling was then immediately started using the following protocol: 94°C 25 sec (1 cycle), 94°C 10 sec, 66°C 30 sec and 72°C 1.5 min (32 cycles).

3 µl of each primary PCR mixture was then diluted in 27 µl of H₂O, 1 µl of each of these dilutions was then placed into a fresh tube.

A master mix for the secondary PCRs, (plus an additional reaction) was set up by combining 18.5 µl of sterile water, 2.5 µl of 10X PCR reaction buffer, 1.0 µl of Nested PCR primer 1 (10 µM), 1.0 µl of Nested PCR primer 2R (10 µM), 0.5 µl of dNTP Mix (10 mM) and 0.5 µl of 50X Advantage cDNA Polymerase Mix per reaction. 24 µl of this Master Mix was then added into each reaction tube containing the 1 µl diluted primary PCR mixture. The following PCR protocol was then carried out: 94°C 10 sec, 68°C 30 sec and 72°C 1.5 min (12 cycles). The reaction products were then stored at -20°C.

### Ligation into a Vector/ Transformation & PCR

The products of the PCR amplification (enriched for differentially expressed cDNAs) were ligated into the pCR2.1-TOPO vector using a T/A cloning kit (Invitrogen), transformed into TOPO One Shot competent cells according to the manufacturers protocol and grown up on LB (Luria-Bertani) Agar plates overnight at 37°C. 1,000 colonies were then individually picked (using fresh sterile tips) and dipped into 5 µl of sterile water which had been aliquotted previously into 96 well PCR plates. The water/colonies were heated in a thermal cycler at 100°C for 10 minutes in order to burst the cells, thus releasing the plasmids containing a differentially expressed cDNA insert. The 5 µl of water/plasmid was then used as a template in a PCR reaction (see below) using M13 Forward and Reverse primers (10 ng/µl), complementary to the M13 site present on either side of the cloning site on the vector. 5 µl of the PCR product was then run on a 2% agarose gel and stained by ethidium bromide. PCR products of an amplified insert were identified and 5 µl of the remainder of the PCR product (i.e. from the 15 µl that had not been run on the gel) was diluted 1/10 with water. 5 µl of the diluted PCR product was then used as a template in a sequencing reaction.

### Sequencing

A sequencing reaction containing M13 primer (3.2 pmol/µl), 'BigDye' reaction mix (i.e. AmpliTaq® DNA polymerase, MgCl₂, buffer and fluorescent dNTPs [each of the four deoxynucleoside triphosphates is linked to a specific fluorescent donor dye which in turn is attached to a specific acceptor dye]) and cDNA template (diluted PCR product) was set up. The reaction was carried out on a thermal cycler for 25 cycles of 10 seconds at 96°C, 20 seconds at 50°C and 4 minutes at 60°C. Each reaction product was then purified through a hydrated Centri-Sep column, and lyophilised. The pellets were resuspended in Template Supression Reagent and sequenced on an ABI Prism 310 Genetic Analyser. The analyser uses an ion laser to excite the specific donor dye that transfers its energy to the acceptor dye, which emits a specific energy spectrum that can be read by the sequencer.

The first 150 potentially upregulated genes were sequenced at Parke-Davis, Cambridge. The remaining 877 from both the forward and back subtractions were sequenced at the applicant's core sequencing facility in Ann Arbor, MI, USA.

### Bioinformatics

The sequencing results were analysed using the computer program CHROMAS in which the vector and adaptor sequences were clipped off, leaving only the nucleotide sequence of the differentially expressed gene. Each sequence was then checked for homology to known genes, Expressed Sequence Tags (ESTs) and Proteins using various Basic Local Alignment Search Tool (BLAST) searches against the Genbank sequence database at the National Centre for Biotechnology Information, Bethesda, Maryland, USA (NCBI).

Two lists were derived called STZup and STZdown that contain the nucleic acid sequences from the forward and back subtracted libraries respectively. In each list there are given accession numbers and descriptions for the known rat genes identified, and where available corresponding mouse or human genes. Sequences that are considered to be of interest are listed in Tables I - X.
References have been given where available for the sequences that have been found, and sequence listings have been given in the form in which they currently appear in publicly searchable databases e.g. the NCBI databaase (National Center for Biotechnology Information, National Library of Medicine, National Institutes of Health, Bethesda, Maryland, MD 20894, USA, www.ncbi.nlm.nih.gov). These sequence listings are given for the purposes of identification only. The invention includes the use of subsequently revised versions of the above sequences (which may incorporate small differences to the version set out herein) and homologous sequences or similar proteins in other species as determined by a high percentage identity (e.g. above 50%, preferably above 90%), length of alignment and functional equivalence.

### REFERENCES (see Tables I - X)

1. Araki,R., Fujimori,A., Hamatani,K., Mita,K., Saito,T., Mori,M. Fukumura,R., Morimyo,M., Muto,M., Itoh,M. and Tatsumi,K., Nonsense mutation at Tyr-4046 in the DNA-dependent protein kinase catalytic subunit of severe combined immune deficiency mice Proc. Natl. Acad. Sci. U.S.A. 94 (6), 2438-2443 (1997)
2. Barradeau,S., Imaizumi-Scherrer,T., Weiss,M.C. and Faust,D.M., Alternative 5'-exons of the mouse cAMP-dependent protein kinase subunit RIalpha gene are conserved and expressed in both a ubiquitous and tissue-restricted fashion, FEBS Lett. 476 (3), 272-276 (2000)
3. Reddy,U.R., Phatak,S. and Pleasure,D., Human neural tissues express a truncated Ror1 receptor tyrosine kinase, lacking both extracellular and transmembrane domains Oncogene 13 (7), 1555-1559 (1996).
4. Goto,K. and Kondo,H., A 104-kDa diacylglycerol kinase containing ankyrin-like repeats localizes in the cell nucleus Proc. Natl. Acad. Sci. U.S.A. 93 (20), 11196-11201 (1996)
5. Letwin,K., Yee,S.P. and Pawson,T., Novel protein-tyrosine kinase cDNAs related to fps/fes and eph cloned using anti-phosphotyrosine antibody, Oncogene 3 (6), 621-627 (1988)
6. Kitamura,K., Mizuno,Y., Sasaki,A., Yasui,A., Tsuiki,S. and Kikuchi,K., Molecular cloning and sequence analysis of cDNA for the catalytic subunit 1 alpha of rat kidney type 1 protein phosphatase, and detection of the gene expression at high levels in hepatoma cells and regenerating livers as compared to rat livers, J. Biochem. 109 (2), 307-310 (1991)
7. Lombroso,P.J., Murdoch,G. and Lerner,M., Molecular characterization of a protein-tyrosine-phosphatase, enriched in striatum, Proc. Natl. Acad. Sci. U.S.A. 88 (16), 7242-7246 (1991)
8. Hara,Y., Urayama,O., Kawakami,K., Nojima,H., Nagamune,H., Kojima,T., Ohta,T., Nagano,K. and Nakao,M., Primary structures of two types of alpha-subunit of rat brain Na+,K+,-ATPase deduced from cDNA sequences J. Biochem. 102 (1), 43-58 (1987).
9. Akagi,H., Hirai,K. and Hishinuma,F., Cloning of a glycine receptor subtype expressed in rat brain and spinal cord during a specific period of neuronal development FEBS Lett. 281 (1-2), 160-166 (1991)
10. Advani,R.J., Bae,H.R., Bock,J.B., Chao,D.S., Doung,Y.C., Prekeris,R., Yoo,J.S. and Scheller,R.H., Seven novel mammalian SNARE proteins localize to distinct membrane compartments, J. Biol. Chem. 273 (17), 10317-10324 (1998)
11. Shull,G.E. and Greeb,J., Molecular cloning of two isoforms of the plasma membrane Ca2+-transporting ATPase from rat brain. Structural and functional domains exhibit similarity to Na+,K+- and other cation transport ATPases, J. Biol. Chem. 263 (18), 8646-8657 (1988)
12. Peng,J.B., Chen,X.Z., Berger,U.V., Vassilev,P.M., Brown,E.M. and Hediger,M.A., A rat kidney-specific calcium transporter in the distal nephron, J. Biol. Chem. 275 (36), 28186-28194 (2000)
13. Indiveri,C., Iacobazzi,V., Giangregorio,N. and Palmieri,F., The mitochondrial carnitine carrier protein: cDNA cloning, primary structure and comparison with other mitochondrial transport proteins, Biochem. J. 321 (Pt 3), 713-719 (1997).
14. Ishida,N., Miura,N., Yoshioka,S. and Kawakita,M., Molecular cloning and characterization of a novel isoform of the human UDP-galactose transporter, and of related complementary DNAs belonging to the nucleotide-sugar transporter gene family J. Biochem. 120 (6), 1074-1078 (1996)
15. Mayser,W., Schloss,P. and Betz,H. Primary structure and functional expression of a choline transporter expressed in the rat nervous system, FEBS Lett. 305 (1), 31-36 (1992)
16. Nishimura,N., Nakamura,H., Takai,Y. and Sano,K., Molecular cloning and characterization of two rab GDI species from rat brain: brain-specific and ubiquitous types, J. Biol. Chem. 269 (19), 14191-14198 (1994)
17. Pehrson,J.R. and Fried,V.A. MacroH2A, a core histone containing a large nonhistone region Science 257, 1398-1400 (1992)
18. Zhong,Z., Wen,Z. and Darnell,J.E., Stat3: a STAT family member activated by tyrosine phosphorylation in response to epidermal growth factor and interleukin-6 Science 264, 95-98 (1994)
19. Lagna,G., Kovelman,R., Sukegawa,J. and Roeder,R.G., Cloning and characterization of an evolutionarily divergent DNA-binding subunit of mammalian TFIIIC Mol. Cell. Biol. 14, 3053-3064 (1994)
20. Amerik,A.Yu., Petukhova,G.V., Grigorenko,V.G., Lykov,I.P., Yarovoi,S.V., Lipkin,V.M. and Gorbalenya,A.E., Cloning and sequence analysis of cDNA for a human homolog of eubacterial ATP-dependent Lon proteases, FEBS Lett. 340 (1-2), 25-28 (1994)
21. Sakimura,K., Kushiya,E., Obinata,M., Odani,S. and Takahashi,Y. Molecular cloning and the nucleotide sequence of cDNA for neuron-specific enolase messenger RNA of rat brain, Proc. Natl. Acad. Sci. U.S.A. 82, 7453-7457 (1985)
22. Mori,K., Ogawa,Y., Ebihara,K., Tamura,N., Tashiro,K., Kuwahara,T., Mukoyama,M., Sugawara,A., Ozaki,S., Tanaka,I. and Nakao,K., Isolation and characterization of CA XIV, a novel membrane-bound carbonic anhydrase from mouse kidney, J. Biol. Chem. 274 (22), 15701-15705 (1999)
23. Iwahara,S., Satoh,H., Song,D.X., Webb,J., Burlingame,A.L., Nagae,Y., and Muller-Eberhard,U., Purification, characterization, and cloning of a heme-binding protein (23 kDa) in rat liver cytosol Biochemistry 34 (41), 13398-13406 (1995)
24. Sakakibara,J., Watanabe,R., Kanai,Y. and Ono,T., Molecular cloning and expression of rat squalene epoxidase, J. Biol. Chem. 270 (1), 17-20 (1995)
25. Puga,A. and Oates,E., Isolation and nucleotide sequence of rat Cu/Zn superoxide dismutase cDNA clones, Free Radic. Res. Commun. 3, 337-347 (1987)
26. Pave-Preux,M., Ferry,N., Bouguet,J., Hanoune,J. and Barouki,R., Nucleotide sequence and glucocorticoid regulation of the mRNAs for the isoenzymes of rat aspartate aminotransferase, J. Biol. Chem. 263, 17459-17466 (1988)
27. Kholodilov,N.G., Neystat,M., Oo,T.F., Hutson,S.M. and Burke,R.E., TITLE Upregulation of cytosolic branched chain aminotransferase in substantia nigra following developmental striatal target injury, Brain Res. Mol. Brain Res. 75 (2), 281-286 (2000)
28. Nakano,K., Matuda,S., Yamanaka,T., Tsubouchi,H., Nakagawa,S., Titani,K., Ohta,S. and Miyata,T. Purification and molecular cloning of succinyltransferase of the rat alpha-ketoglutarate dehydrogenase complex. Absence of a sequence motif of the putative E3 and/or E1 binding site, J. Biol. Chem. 266 (28), 19013-19017 (1991)
29. Kayukawa,K., Makino,Y., Yogosawa,S. and Tamura,T., A serine residue in the N-terminal acidic region of rat RPB6, one of the common subunits of RNA polymerases, is exclusively phosphorylated by casein kinase II in vitro, Gene 234 (1), 139-147 (1999)
30. Shechter,I., Klinger,E., Rucker,M.L., Engstrom,R.G., Spirito,J.A., Islam,M.A., Boettcher,B.R. and Wienstein,D.B., Solubilization, purification and characterization of a truncated form of rat hepatic squalene synthetase, J. Biol. Chem. 267, 8628-8635 (1992).
31. Nomura,T., Takizawa,M., Aoki,J., Arai,H., Inoue,K., Wakisaka,E., Yoshizuka,N., Imokawa,G., Dohmae,N., Takio,K., Hattori,M. and Matsuo,N., Purification, cDNA cloning, and expression of UDP-gal: glucosylceramide beta-1,4-galactosyltransferase from rat brain, J. Biol. Chem. 273 (22), 13570-13577 (1998)
32. Mann,S.S. and Hammarback,J.A., Molecular characterization of light chain 3. A microtubule binding subunit of MAP1A and MAP1B, J. Biol. Chem. 269 (15), 11492-11497 (1994).
33. Kuwano,Y. and Wool,I.G., The primary structure of rat ribosomal protein L3, Biochem. Biophys. Res. Commun. 187 (1), 58-64 (1992)
34. Devi,K.R., Chan,Y.L. and Wool,I.G., The primary structure of rat ribosomal protein S4, Biochim. Biophys. Acta 1008 (2), 258-262 (1989)
35. Janz,R., Hofmann,K. and Sudhof,T.C., SVOP, an evolutionarily conserved synaptic vesicle protein, suggests novel transport functions of synaptic vesicles, J. Neurosci. 18 (22), 9269-9281 (1998)
36. Pettersson,M., Bessonova,M., Gu,H.F., Groop,L.C. and Jonsson,J.I., Characterization, chromosomal localization, and expression during hematopoietic differentiation of the gene encoding Arl6ip, ADP-ribosylation-like factor-6 interacting protein (ARL6), Genomics 68 (3), 351-354 (2000)
37. Chan,Y.L., Olvera,J. and Wool,I.G., The primary structure of rat ribosomal protein L14, Biochem. Biophys. Res. Commun. 222 (2), 427-431 (1996)
38. Pfister,K.K., Benashski,S.E., Dillman,J.F. III, Patel-King,R.S. and King,S.M., Identification and molecular characterization of the p24 dynactin light chain, Cell Motil. Cytoskeleton 41 (2), 154-167 (1998)
39. Chan,Y.L., Suzuki,K. and Wool,I.G., The carboxyl extensions of two rat ubiquitin fusion proteins are ribosomal proteins S27a and L40, Biochem. Biophys. Res. Commun. 215 (2), 682-690 (1995)
40. Sugimoto,K., Honda,S., Yamamoto,T., Ueki,T., Monden,M., Kaji,A., Matsumoto,K. and Nakamura,T., Molecular cloning and characterization of a newly identified member of the cadherin family, PB-cadherin, J. Biol. Chem. 271 (19), 11548-11556(1996)
41. Betty,M., Harnish,S.W., Rhodes,K.J. and Cockett,M.I. Distribution of heterotrimeric G-protein beta and gamma subunits in the rat brain, Neuroscience 85 (2), 475-486 (1998)
42. Brown,C.W., McHugh,K.M. and Lessard,J.L., cDNA sequence encoding cytoskeletal gamma-actin from rat, Nucleic Acids Res. 18 (17), 5312 (1990)
43. Leung,T., How,B.E., Manser,E. and Lim,L., Germ cell beta-chimaerin, a new GTPase-activating protein for p21rac, is specifically expressed during the acrosomal assembly stage in rat testis, J. Biol. Chem. 268, 3813-3816 (1993)
44. Epstein,P., Means,A.R. and Berchtold,M.W., Isolation of a rat parvalbumin gene and full length cDNA, J. Biol. Chem. 261, 5886-5891 (1986)
45. Adachi,T., Schamel,W.W., Kim,K.M., Watanabe,T., Becker,B., Nielsen,P.J. and Reth,M., The specificity of association of the IgD molecule with the accessory proteins BAP31/BAP29 lies in the IgD transmembrane sequence, EMBO J. 15 (7), 1534-1541 (1996)
46. Hirano,S., Ono,T., Yan,Q., Wang,X., Sonta,S. and Suzuki,S.T., Protocadherin 2C: a new member of the protocadherin 2 subfamily expressed in a redundant manner with OL-protocadherin in the developing brain, Biochem. Biophys. Res. Commun. 260 (3), 641-645 (1999)
47. Suzuki,K., Olvera,J. and Wool,I.G., The primary structure of rat ribosomal protein L12, Biochem. Biophys. Res. Commun. 172 (1), 35-41 (1990)
48. Li,L. and Cohen,S.N., Tsg101: a novel tumor susceptibility gene isolated by controlled homozygous functional knockout of allelic loci in mammalian cells, Cell 85 (3), 319-329 (1996)
49. Atkinson,M.J., Freeman,M.W. and Kronenberg,H.M., Thymosin beta-4 is expressed in ROS 17/2.8 osteosarcoma cells in a regulated manner, Mol. Endocrinol. 4, 69-74 (1990)
50. Devi,K.R.G., Chan,Y.-L. and Wool,I.G., The primary structure of rat ribosomal protein L18, DNA 7, 157-162 (1988)
51. Thevananther,S., Kolli,A.H. and Devarajan,P., Identification of a novel ankyrin isoform (AnkG190) in kidney and lung that associates with the plasma membrane and binds alpha-Na, K-ATPase, J. Biol. Chem. 273 (37), 23952-23958 (1998).
52. Liao,Y.-C., Tokes,Z., Lim,E., Lackey,A., Woo,C.H., Button,J.D. and Clawson,G.A., Cloning of rat 'prion-related protein' cDNA, Lab. Invest. 57, 370-374 (1987)
53. Nakamura,H., Tanaka,T. and Ishikawa,K., Nucleotide sequence of cloned cDNA specific for rat ribosomal protein L7a, Nucleic Acids Res. 17 (12), 4875 (1989).
54. Schaar,D.G., Varia,M.R., Elkabes,S., Ramakrishnan,L., Dreyfus,C.F. and Black,I.B. The identification of a novel cDNA preferentially expressed in the olfactory-limbic system of the adult rat, Brain Res. 721 (1-2), 217-228 (1996)
55. Wieczorek,D.F. and Hughes,S.R., Developmentally regulated cDNA expressed exclusively in neural tissue, Brain Res. Mol. Brain Res. 10 (1), 33-41 (1991)
56. Tjoelker,L.W., Seyfried,C.E., Eddy,R.L. Jr., Byers,M.G., Shows,T.B., Calderon,J., Schreiber,R.B. and Gray,P.W., Human, mouse, and rat calnexin cDNA cloning: identification of potential calcium binding motifs and gene localization to human chromosome 5, Biochemistry 33 (11), 3229-3236 (1994)
57. Yoshikawa,K., Maruyama,K., Aizawa,T. and Yamamoto,A., A new species of enkephalin precursor mRNA with a distinct 5'-untranslated region in haploid germ cells, FEBS Lett. 246 (1-2), 193-196 (1989)
58. Klar,A., Baldassare,M. and Jessell,T.M., F-spondin: A gene expressed at high levels in the floor plate encodes a secreted protein that promotes neural cell adhesion and neurite extension, Cell 69, 95-110 (1992)
59. Reeben,M., Neuman,T., Palgi,J., Palm,K., Paalme,V. and Saarma,M., Characterization of the rat light neurofilament (NF-L) gene promoter and identification of NGF and cAMP responsive regions, J. Neurosci. Res. 40 (2), 177-188 (1995).
60. Rees,W.D., Harries,D.N. and Fleming,J.V., Partial sequences of two genes regulated by amino acid supply identified by the use of RNA fingerprinting by arbitrarily primed PCR, J. Nutritional Biochem. 9, 136-141 (1998)
61. Hart,M.C., Korshunova,Y.O. and Cooper,J.A., Vertebrates have conserved capping protein alpha isoforms with specific expression patterns, Cell Motil. Cytoskeleton 38 (2), 120-132 (1997)
62. Seki,N., Ohira,M., Nagase,T., Ishikawa,K., Miyajima,N., Nakajima,D., Nomura,N. and Ohara,O. Characterization of cDNA clones in size-fractionated cDNA libraries from human brain, DNA Res. 4 (5), 345-349 (1997)
63. DeGregori,J., Russ,A., von Melchner,H., Rayburn,H., Priyaranjan,P., Jenkins,N.A., Copeland,N.G. and Ruley,H.E., A murine homolog of the yeast RNA1 gene is required for postimplantation development, Genes Dev. 8, 265-276 (1994)
64. Meyer,H.A., Grau,H., Kraft,R., Kostka,S., Prehn,S., Kalies,K.U. and Hartmann,E., Mammalian Sec61 is associated with Sec62 and Sec63, J. Biol. Chem. 275 (19), 14550-14557 (2000)
65. Gao,Y., Melki,R., Walden,P.D., Lewis,S.A., Ampe,C., Rommelaere,H., Vandekerckhove,J. and Cowan,N.J., A novel cochaperonin that modulates the ATPase activity of cytoplasmic chaperonin, J. Cell Biol. 125 (5), 989-996 (1994)
66. Edelmann,W., Yang,K., Umar,A., Heyer,J., Lau,K., Fan,K., Liedtke,W., Cohen,P., Kane,M.K., Lipford,J.R., Yu,N., Crouse,G.F., Pollard,J.W., Kunkel,T., Lipkin,M., Kolodner,R. and Kucherlapati,R., Mutation in the mismatch repair gene Msh6 causes cancer susceptibility, Cell 91 (4), 467-477 (1997)
67. Karthikeyan,L., Flad,M., Engel,M., Meyer-Puttlitz,B., Margolis,R.U. and Margolis,R.K., Immunocytochemical and in situ hybridization studies of the heparan sulfate proteoglycan, glypican, in nervous tissue, J. Cell. Sci. 107 (Pt 11), 3213-3222 (1994)
68. Shimono,A., Okuda,T. and Kondoh,H., N-myc-dependent repression of ndr1, a gene identified by direct subtraction of whole mouse embryo cDNAs between wild type and N-myc mutant, Mech. Dev. 83 (1-2), 39-52 (1999)
69. Kitaoka,Y., Olvera,J. and Wool,I.G., The primary structure of rat ribosomal protein S23, Biochem. Biophys. Res. Commun. 202 (1), 314-320 (1994)
70. Takasawa,S., Yamamoto,H., Terazono,K. and Okamoto,H., Novel gene activated in rat insulinomas, Diabetes 35, 1178-1180(1986)
71. Kaprielian,Z., Cho,K.O., Hadjiargyrou,M. and Patterson,P.H., CD9, a major platelet cell surface glycoprotein, is a ROCA antigen and is expressed in the nervous system, J. Neurosci. 15 (1 Pt 2), 562-573 (1995)
72. Collard,M.W. and Griswold,M.D., Biosynthesis and molecular cloning of sulfated glycoprotein 2 secreted by Rat Sertoli cells, Biochemistry 26, 3297-3303 (1987)
73. Nagai,Y., Kojima,T., Muro,Y., Hachiya,T., Nishizawa,Y., Wakabayashi,T. and Hagiwara,M. Identification of a novel nuclear speckle-type protein, SPOP, FEBS Lett. 418 (1-2), 23-26 (1997)
74. Brown,K.A., Leek,J.P., Lench,N.J., Moynihan,L.M., Markham,A.F. and Mueller,R.F., Human sequences homologous to the gene for the cochlear protein Ocp-II do not map to currently known non-syndromic hearing loss loci, Ann. Hum. Genet. 60 (Pt 5), 385-389 (1996)
75. Wolff,T., O'Neill,R.E. and Palese,P., NS1-Binding protein (NS1-BP): a novel human protein that interacts with the influenza A virus nonstructural NS1 protein is relocalized in the nuclei of infected cells, J. Virol. 72 (9), 7170-7180 (1998)
76. Lemischka,I.R., Farmer,S., Racaniello,V.R. and Sharp,P.A., Nucleotide sequence and evolution of a mammalian alpha-tubulin messenger RNA, J. Mol. Biol. 151 (1), 101-120 (1981).
77. Morales,C.R., El-Alfy,M., Zhao,Q. and Igdoura,S.A., Expression and tissue distribution of rat sulfated glycoprotein-1 (prosaposin), J. Histochem. Cytochem. 44 (4), 327-337 (1996).

## Claims

1. Use of:
(a) an isolated gene sequence that is down-regulated in the spinal cord in response to streptozocin-induced diabetes;
(b) an isolated gene sequence comprising a nucleic acid sequence of Tables I to X.
(c) an isolated gene sequence having at least 80% sequence identity with a nucleic acid sequence of Tables I to X;
(d) an isolated nucleic acid sequence that is hybridizable to any of the gene sequences according to (a), (b) or (c) under stringent hybridisation conditions;
(e) a recombinant vector comprising a gene sequence or nucleic acid sequence according to any one of (a) to (d);
(f) a host cell containing the vector according to (e);
(g) a non-human animal having in its genome an introduced gene sequence or nucleic acid sequence or a removed or down-regulated gene sequence or nucleic acid sequence according to any one of (a) to (d);
(h) an isolated polypeptide comprising an amino acid sequence at least 90% identical to an amino acid sequence encoded by a nucleotide sequence according to any one of (a) to (d), or a polypeptide variant thereof with sequential amino acid deletions from the C terminus and/or the N-terminus; or
(i) an isolated polypeptide encoded by a nucleotide sequence according to any one of (a) to (d); or
(j) an isolated antibody that binds specifically to a polypeptide according to (h) or (i);
in the screening of compounds for the treatment of pain, or for the diagnosis of pain.

2. Use according to claim 1 wherein the isolated gene sequence encodes a kinase.

3. Use according to claim 1 or 2, wherein the isolated gene sequence encodes putative DNA dependent protein kinase catalytic subunit (D87521), putative cAMP-dependent protein kinase regulatory subunit RI alpha (AJ278429), putative protein tyrosine kinase t-Ror1 (U38894), diacylglycerol kinase (DGK-IV) (D78588; U51477) or elk protein tyrosine kinase (X13411).

4. Use according to claim 1 wherein the isolated gene sequence encodes a phosphatase.

5. Use according to claim 1 or 4, wherein the isolated gene sequence encodes an expression product or fragment thereof of PP-1 alpha for catalytic subunit of protein phosphatase type 1 alpha (D00859; U25809; X07848) or protein tyrosine phosphatase, striatum enriched (S49499, U28216, U27831).

6. Use according to claim 1 wherein the isolated gene sequence encodes an ion channel protein.

7. Use according to claim 1 or 6, wherein the isolated gene sequence encodes an expression product or fragment thereof of Na⁺, K⁺-ATPase alpha-subunit (D00189) or voltage dependent anion channel 2 (AF268468; U30838; L06328).

8. Use according to claim 1 wherein the isolated gene sequence encodes a receptor protein.

9. Use according to claim 1 or 8, wherein the isolated gene sequence encodes an expression product or fragment thereof of neonatal glycine receptor NglyR (X57281) or Syntaxin 13 (AF04458).

10. Use according to claim 1 wherein the isolated gene sequence encodes a transporter protein.

11. Use according to claim 1 or 10, wherein the isolated gene sequence encodes an expression product or fragment thereof of ATPase, calcium, plasma membrane, isoform 1 (J03753), calcium transporter CaT2 (AF209196), carnitine/acyl-carnitine carrier protein (X97831), putative potent brain type organic ion transporter (AB012808; ABO40056), UDP-galactose transporter (D87991) or choline transporter CHOT1 (X66494).

12. Use according to claim 1 wherein the isolated gene sequence encodes a G-protein coupled receptor protein.

13. Use according to claim 1 or 12, wherein the isolated gene sequence encodes an expression product or fragment thereof of rab GDI, alpha species, ras related GTPase (X74402).

14. Use according to claim 1 wherein the isolated gene sequence encodes a DNA binding protein.

15. Use according to claim 1 or 14, wherein the isolated gene sequence encodes an expression product or fragment thereof of histone, core, H2A.1 (M99065), putative signal transducer and regulator transcription 1 (Stat1) (U06924), transcription factor IIIC or alpha subunit (L28801).

16. Use according to claim 1 wherein the isolated gene sequence encodes a protease protein.

17. Use according to claim 1 or 16, wherein the isolated gene sequence encodes an expression product or fragment thereof of putative Lon protease (X76040)

18. Use according to claim 1 wherein the isolated gene sequence encodes a ligase, lyase, oxidoreductase or transferase.

19. Use according to claim 1 or 18, wherein the isolated gene sequence encodes an expression product or fragment thereof of GM3 synthase.

20. Use according to claim 1 or 18, wherein the isolated gene sequence encodes an expression product or fragment thereof of enolase, neuron-specific (M11931) or putative carbonic anhydrase XIV (AB005450).

21. Use according to claim 1 or 18, wherein the isolated gene sequence encodes an expression product or fragment thereof of cytosolic malate dehydrogenase (Mdh) (AF093773; M29462; D55654), heme-binding protein (HBP23) (D30035), squalene epoxidase (D37920) or superoxide dismutase, copper-zinc (SOD-1) (M21060; X05634; K00065).

22. Use according to claim 1 or 18, wherein the isolated gene sequence encodes an expression product or fragment thereof of aspartate aminotransferase, cytosolic (J04171), branched-chain aminotransferase, cytosolic, brain (AF165887), dihydrolipoamide succinyl-transferase (D90401), RNA polymerase II (AB017711), squalene synthetase, hepatic (M95591) or UDP-Gal: glucosylceramide beta-1,4-galactosyl transferase (AF048687).

23. A non-human animal having in its genome an introduced gene sequence or a removed or down-regulated gene sequence , said gene sequence being down-regulated in the spinal cord of a mammal in response to streptozocin-induced diabetes.

24. A non-human animal according to claim 23, wherein the introduced gene sequence is according to any of claims 1 to 22

25. A non-human animal according to claim 23 or 24 which is *C. elegans.*

26. A kit comprising;
(a) affinity peptide and/or ligand and/or substrate for an expression product of a gene sequence that is down-regulated in the spinal cord of a mammal in response to streptozocin-induced diabetes; and
(b) a defined quantity of an expression product of a gene sequence that is down-regulated in the spinal cord of a mammal in response to streptozocin-induced diabetes, for simultaneous, separate or sequential use in detecting and/or quantifying down-regulation of a gene sequence in the spinal cord of a mammal in response to streptozocin-induced diabetes.

27. A kit according to claim 26, wherein the gene sequence is defined in any of claims 1 to 22.

28. A kit comprising:
(a) nucleic acid sequences capable of hybridization to a nucleic acid sequence that is down-regulated in the spinal cord of a mammal in response to streptozocin-induced diabetes; and
(b) a defined quantity of one or more nucleic acid sequences capable of hybridization to a nucleic acid sequence that is down-regulated in the spinal cord of a mammal in response to streptozocin-induced diabetes, for simultaneous, separate or sequential use in detecting and/or quantifying down-regulation of a gene sequence in the spinal cord of a mammal in response to streptozocin-induced diabetes.

29. The kit of claim 28, wherein the gene sequence is according to any of claims 1 to 22.

30. A compound that modulates the action of an expression product of a gene sequence that is down-regulated in the spinal cord of a mammal in response to streptozocin-induced diabetes.

31. A compound according to claim 30 wherein the gene sequence is listed in Tables I to X.

32. A compound according to claim 30 or 31 wherein the nucleotide sequence is according to any one of claims 1 to 22.

33. A compound according to any one of claims 30 to 32 for use as a medicament.

34. A compound according to any one of claims 30 to 33 for the treatment or diagnosis of pain.

35. A pharmaceutical composition comprising a compound according to any one of claims 30 to 34 and a pharmaceutically acceptable carrier or diluent.

36. Use of a compound according to any one of claims 30 to 34 in the manufacture of a medicament for the treatment or diagnosis of pain.

37. Use of a compound according to any one of claims 30 to 34 in the manufacture of a medicament for the treatment or diagnosis of chronic pain.

38. A method of treatment of pain, which comprises administering to a patient an effective amount of a compound according to any one of claims 30 to 34.
